# EUROPÄISCHE PATENTANMELDUNG

(11) **EP 1 491 639 A2**
(43) Veröffentlichungstag der Anmeldung: **29.12.2004**
(21) Anmeldenummer: 04090238.9
(22) Anmeldetag: 17.06.2004
(51) Int. Cl.: C12Q 1/68

(54) **Verfahren zur Analyse des Cytosin-Methylieruntgsstatus von Cancer-Testis-Antigenen für die individualisierte Immuntherapie**

(30) Priorität: 24.06.2003 DE 10329240
(71) Anmelder: Epigenomics AG, 10435 Berlin (DE)
(72) Erfinder: Eichler-Mertens, Mathias, 10119 Berlin (DE); Piepenbrock, Christian, 10115 Berlin (DE); Olek, Alexander, 10407 Berlin (DE)
(74) Vertreter: Schubert, Klemens, Dr.

(57) **Zusammenfassung**

Beschrieben wird ein Verfahren zur Bestimmung der Angriffspunkte einer Tumor-Immuntherapie, wobei man:
a) eine DNA-Probe entnimmt,
b) den Methylierungszustand mindestens jeweils einer CpG-Position innerhalb mehrerer Tumor-assoziierter Antigene identifiziert, wobei die Methylierungszustände zusammen ein Methylierungsmuster bilden,
c) aus dem gewonnenen Methylierungsmuster folgert, welche der Tumor-assoziierten Antigene die geeignetsten Angriffspunkte für eine Immuntherapie sind.

Beschrieben ist auch ein Verfahren zur Behandlung von Tumorerkrankungen, wobei man die Angriffspunkte für eine Tumor-Immuntherapie bestimmt und anschließend eine Vakzinierung gegen die geeignetesten Tumor-assoziierten Tumorantigene erfolgt.

Sequenzprotokoll

<110> Epigenomics AG

<120> Verfahren zur Analyse des Cytosin-Methylierungsstatus von Cancer-
      Testis-Antigenen für eine individualisierte Immuntherapie

<160> 235

<210> 1
<211> 2501
<212> DNA
<213> Homo Sapiens

<400> 1

gattaatatg caacaggagg tgagaactaa tactacttta atgcagacaa agataatatt      60
ggctaagtgt taacactggt ctctaaaaat gggcaactta tttcattttc attttgctta     120
tgtacttgag gtattatgtg cacgttggta gtacctttat tatccttaaa gtaatgatgc     180
tgtctaggaa tattaaatac cgtctttctg aaacacctat gaaatttaaa atattttatt     240
ctagatgtag caatatgtgc cacagagtaa cattttaatc taataaattc ggttttaaaa     300
taaatcactt tgggtcgggt gcagtggctc acgcctgtaa tcccagcact ttgggaggtc     360
gaggagggtg gatcacctga ggtcaggagt tccagaccag cttggccaat gtagtgaaac     420
cctgcctgta ctaaaaatac aaaaattagc tgggcgtggt ggcaggtgcc tgtaatccca     480
gctactcagg aagctgaggc aggagaatca cttgaagcca gcaggcggaa gttgcagtga     540
gtggaaattg tgccactgca ctccagtctg ggcggtagag tgagactcca tctcaaaaaa     600
taaaaacaga aaaataaaat aaaatcactt tggctgggca tagtggctca aggctgtaat     660
ctcagcacct tgggaggctg aggtgtgtgg atcacctgag gtcaggtttt tgagaccagc     720
cgggccaaca tgttgaaacc ctgtctctac aaaaaaatac aaaaattagc tgcatggtgt     780
catgcatctg tagtcgcagc tacttgggag gctgaggcta gagaatcgct tgaacctggg     840
aggcaaacat tacagtgggc cgagatcgcg ccattgcact ccagcctggg caacagaatg     900
agactccatc tcagagagag agagagacac cataaattaa tcactttttt caaattagcc     960
agaacatttt tttactttgc ttttctctct tcattgaatt aacaattgta gaatacttac    1020
acactgatca tatctttaac attctctgtt atttcttccg actaggaatt tatagacttt    1080
tcctgctaag acctcaattt gtgtttttgt gaaagacagg aagactgaga tgttgaaata    1140
atttaaaaca gccaaggttg gccgggcgcg gtggctcacg cttgtaatcc cagcactttg    1200
ggaggccgag gcgggcggat cacgaggtca ggagatctag accacggtga aaccccatct    1260
ctactaaaaa tacaaaaagt tagccgggcg tagtggaggg cgcctgtagt cccagctact    1320
cgggaggcgg aggcaggaga atggcgtgaa cccgggaggc ggagcttgca gtgagccgag    1380
atggagccac tgcactccag cctgggtgac agagcaagac tccgtctcaa aaaaaaaaaa    1440
aaaaaaaaaa aaaaaaaaaa aaaaaaaaaa aaaaaatcca aggctaaggc atatatagtg    1500
aaacttttac tatttgtata taaaaaattt gtatttgttt aaaaattgat tcctttgcca    1560
tttggctttt tttttttttc atcctgtctt aatcatgatg tgctttaaaa cacagtatgt    1620
gttaactctt ttttgagatg gagtttcatt cttgttgccc aggctgtagt gcaatggcac    1680
gatcttggct cactgtaacc tctgcctccc ggatttaagg gattctccct cctcaccctc    1740
ctaagtagct tagattacag gcatgcacta ccacctctgg ctaatttttg tatttttagt    1800
agagacaggg tttcactaca ttggtcaggc tggtctcgaa ctcctgacct caagtgatcc    1860
acccgccttg gcctcccaaa gtgttgagat tacaggcctg agccactgtg cccggcttct    1920
gttactcttg aataacactt ttcagctcct gtggaaacca ttcctaaagt gcttctgaag    1980
tactcagttt ttgtttttca ggacatgtac ttgtagacag gattcaaagc agttaagaat    2040
gtctctgcca agtcgacaaa cagctattat tgttaaccct cctccaccag aatatataaa    2100
tactaagaaa aatgggcgat tgacaaatca acttcagtat ctacaaaaag ttgtcctaaa    2160
ggatttatgg aagcatagtt tttcatggcc ctttcaacgt cctgtggatg ctgtgaaact    2220
acagttgcct gtatgtatgt cttcaactat gttagtttca aaaaaagaaa actcctgtaa    2280
atttctgtaa gacataacta gtctttaata ttataaaaca tatttggcca ggcgcgttgg    2340
ctcccgccta taatcccagc actttgggag gccgaggtgg ggagatcacc tgaggtcggg    2400
agttcgagac cagcctgacc aacatggaga gaccccgtct ctactaaaaa tacaaaatta    2460
gccgggcgtg gtggcacatg cctgtacttc cagctaccca g                        2501

<210> 2
<211> 2501
<212> DNA
<213> Homo Sapiens

<400> 2

tttctgggct gtagtgtagt ataccagtca ggtgtctgca ctaagttctg catcaacctg 60
gttgcctaag gatggttgaa ctgacccagg tggaaattgg agcaggtcaa aactcaatgc 120
tgatcagtaa cagggtcgca cctgtaaata gccaccgccc tccagcctgg gcaacacagt 180
gagatcccat ctctaaaata aattttaaaa ataattaatt agaaaaaaaa accagcctgt 240
aatcccagca ctttgggagg ccaaggcggg cagatcacct gaggtcagga gttcaagaca 300
agcctggcca acatggcaaa accccaatct ctaccaaaaa tacaaaaatt agctgggcgt 360
ggtggcaggt gcctgtagtc ccagctaccc aggaggctga ggcaagagaa tcgcttgaac 420
ctgggaggtg gagattgcag tgggccgaga tcgcgccact tcactccagc ctgggtagca 480
gagtgcgact ctgtctcaaa aaaaaaaaaa aagaaagaaa agaaaagaaa aagaaaagaa 540
aagaaaaaaa ctgggaggcc taagcccatt cttggtgtcc tcacactctt ctgccccatc 600
tccttgcacc cagtcttccc ctgcaatctg tgctccatac tagccctcaa gccctcaacg 660
tgacctagta tgagaactgg attctgtcac tctcctgctc ataatatttt atgccttccc 720
tttgcccaga atatcattct ccctattgtt tttaccaatg gaactggtat ttcttcaagg 780
acatgatcaa atttgcctac ttctatatta tcttctaaag cagaattcat ctctcttccc 840
tcaatatgat gatattgaca gggtttgccc tcactcacta gattgtgagc tgctcagggc 900
aggtagcgtt ttttgttttt gtttttgttt ttcttttttg agacagggtc ttgctctgtc 960
acccaggcca gagtgcaatg gtacagtctc agctcactgc agcctcaacc gcctcggctc 1020
aaaccatcat cccatttcag cctcctgagt agctgggact acaggcacat gccattacac 1080
ctggctaatt tttttgtatt tctagtagag acagggtttg gccatgttgc ccgggctggt 1140
ctcgaactcc tggactcaag caatccaccc acctcagcct cccaaaatga gggaccgtgt 1200
cttattcatt tccatgtccc tagtccatag cccagtgctg gacctatggt agtactaaat 1260
aaatatttgt tgaatgcaat agtaaatagc atttcaggga gcaagaacta gattaacaaa 1320
ggtggtaaaa ggtttggaga aaaaaataat agtttaattt ggctagagta tgagggagag 1380
tagtaggaga caagatggaa aggtctcttg ggcaaggttt tgaaggaagt tggaagtcag 1440
aagtacacaa tgtgcatatc gtggcaggca gtggggagcc aatgaaggct tttgagcagg 1500
agagtaatgt gttgaaaaat aaatataggt taaacctatc agagcccctc tgacacatac 1560
acttgctttt cattcaagct caagtttgtc tcccacatac ccattactta actcaccctc 1620
gggctcccct agcagcctgc cctacctctt tacctgcttc ctggtggagt cagggatgta 1680
tacatgagct gctttccctc tcagccagag gacatggggg ccccagctcc cctgcctttc 1740
cccttctgtg cctggagctg ggaagcaggc cagggttagc tgaggctggc tggcaagcag 1800
ctgggtggtg ccagggagag cctgcatagt gccaggtggt gccttgggtt ccaagctgag 1860
tccatggccc cgataacctt ctgcctgtgc acacacctgc ccctcactcc acccccatcc 1920
tagctttggt atgggggaga gggcacaggg ccagacaaac ctgtgagact ttggctccat 1980
ctctgcaaaa gggcgctctg tgagtcagcc tgctcccctc caggcttgct cctcccccac 2040
ccagctctcg tttccaatgc acgtacagcc cgtacacacc gtgtgctggg acaccccaca 2100
gtcagccgca tggctcccct gtgccccagc ccctggctcc ctctgttgat cccggcccct 2160
gctccaggcc tcactgtgca actgctgctg tcactgctgc ttctggtgcc tgtccatccc 2220
cagaggttgc cccggatgca ggaggattcc cccttgggag gaggctcttc tggggaagat 2280
gacccactgg gcgaggagga tctgcccagt gaagaggatt cacccagaga ggaggatcca 2340
cccggagagg aggatctacc tggagaggag gatctacctg gagaggagga tctacctgaa 2400
gttaagccta aatcagaaga agagggctcc ctgaagttag aggatctacc tactgttgag 2460
gctcctggag atcctcaaga accccagaat aatgcccaca g 2501

<210> 3
<211> 2501
<212> DNA
<213> Homo Sapiens

<400> 3

gcccgctgga ccatccaaat ctgtcctgct cccggctgga gtttgatgcc atctagtggc 60
agacgttgct ctggctgaaa tattgatgga gatttccggt ccagtggctg gctgagactt 120
ccaggacagt ggcagtatct ccaggaatct ctcaatcagt taggacttcc tttcttgact 180
tgatctgagg ataataggcc gggaaaataa tggggacccg gagaccctcc ctctgtcttc 240
ctgcttccta agacagggtc acatgcactt tactctccct tccctgacct gttgaaggta 300
agaactttag cctgcctctg tctgagaatg gggagactga gcagaggacg acaggaagga 360
tggcaatcac taaccgggtt atgtctgatt ttcagctatt ccaggtgtta acaagttgtg 420
ttctctggtt tttaaaattc atcatttaat gaaatatatg taatgaaata cagcagtgaa 480
aataataaac tggaggtaca cgcaagagct tgggtgaatt tttcttttct gtaagtaaaa 540
acatttttaa ctgatgtatg ataactctgc atatttatgg gatacatgtg atattctgat 600
acatgcacac gcacacacga tgtaatgatc aagtcggggt atttaggatc tccaccacct 660

caagcattta tcatttcttt ttttggggaa catttcctat cttttagcta ttttgaaata 720
tacaataaat aattacctat agtccctcta cggtgctatc aaacactaga acttaatcct 780
tctatgcaac tatatgtttg tccccattaa cctccccacc ctccccacct ttcccagcgt 840
ctggtaacca tcattctgct ctacctccaa aagatcaacg tttttagttt ccacatgagt 900
gccaatatgc ttttcccagg tgcagaggct gctgattcct ggatttggaa ggtgggggag 960
agtggtttgg atttcagtac ctcccccttc ctcagggcta ggccctgtct ggtcacctcc 1020
tgttgctaca ggtgtgcctg gcacagacat tcagctcccg gggccgtgcc gccctcaccc 1080
ctgcatgcct ctagaggtac ctgtcccgtg gatttgaccc ccttccagac acacaaatag 1140
atcaccactg agcagacaca gggtcacctc ccagctccct accaggcaag agcgaccagg 1200
gcagggactg atactgccga acccaggagc caggcccgac ccagcctcag gtccagcagg 1260
tcccgcctgt ccacctgggc caggcctaga gcccgggagc ccctggctgg tgggaggcca 1320
cccgcaaccc accccacacg cagctccagc tcccccacca ggcggggcga ctaggacagg 1380
gacagaaccc gttgaaccca ggagtgagat ccggccccgg gtcccgctgg gccctcccgt 1440
ccaccttggc tggacctggc gcctgggaga ccttggctgg cgcgaggcca cgcccaccag 1500
acatgcagtt ccagctaccc caccagctgg gcgaccagga cagggacgga ggctgctgag 1560
cccagttaga ggcctgcccc ccggggtctg tcctgggcgc tcccccaagg acggacaggg 1620
caggcagggt ccgggacgat ggccgcacag tcccggcccc gtgttcccag gcccgtcttg 1680
ctcctcgatg tgagggagac ccgggggatg ggacaggctg ggccccgcag tgcctgactc 1740
cctgcagggc tcccgggaca ggggtccggc ggacagccgg ctgctcaggg gtgaggggtc 1800
caagctggca ttgcggccac cttccggccc gggctctctt ggggaggggc ggggttggtg 1860
agaaccggtc acgtgctccg gggctcactc ggggtctccc agggccggaa gtagggcccc 1920
tgtgcgcagg cgccctgagg atcccgggct gcccatctca cgccaggggg cggaacttcc 1980
tgcagcctct ctgcctccgc atcctcgtgg gccctgacct tctctctgag agccgggcag 2040
aggctccgga gccatgcagg ccgaaggccg gggcacaggg ggttcgacgg gcgatgctga 2100
tggcccagga ggccctggca ttcctgatgg cccagggggc aatgctggcg gcccaggaga 2160
ggcgggtgcc acgggcggca gaggtccccg gggcgcaggg gcagcaaggg cctcggggcc 2220
gggaggaggc gccccgcggg gtccgcatgg cggcgcggct tcagggctga atggatgctg 2280
cagatgcggg gccagggggc cggagagccg cctgcttgag ttgtattctg ttctgttctg 2340
ttctgttctg acagttctgg tggcgaggtg gggccgggga gatggggagg gcagggccag 2400
gtgggggagg aggcggggga gatgcgagta agtggttggg tgggggtggg ggtgggggtt 2460
gggaggtggg gatatgagag gccagctgcg aggaggggag g 2501

<210> 4
<211> 2501
<212> DNA
<213> Homo Sapiens

<400> 4

gcattatctc ctttaacccc agcatagtat gtcagatgct gttttacaga tgagccaact 60
gagaccagag atgctcagtc acttgcccaa ggtgacatga ctgatatgga atagagtcaa 120
gatttttttt tttttttttg acacggagtc tcactctgtc tcccaggctg gagtgcagag 180
gcgcaatctc agctcactgc aagctctgcc tcccaggttc acgccattct cctgcctcag 240
cctcctgagt agctgggact acaggcaccc gccaccacac ctggctaatt ttttgtattt 300
ttagcagaga cagggtttca ccgtgttagc caggatggtc tcgatctcct gacctcgtga 360
tctgcctgcc tcggcctccc aaagtgctgg aattacaggt gtgagccacc gcgactggcc 420
agattcaaga tttgaaccca ggtcctcttg gtcccagagg cccctgtttc tcaactccct 480
aggatggcat agcaacctgt cccacaagag gtgcctgctt taagtgtgct cagcacatgg 540
aagcaagttt agaaatgcaa gtgtatacct gtaaagaggt gtgggagatg ggggggaggg 600
aagagagaaa gagatgctgg tgtccttcat tctccagtcc ctgataggtg cctttgatcc 660
cttcttgacc agtatagctg cattcttggc tggggcattc caactagaac tgccaaattt 720
agcacataaa aataaggagg cccagttaaa tttgaatttc agataaacaa tgaataattt 780
gttagtataa atatgtccca tgcaatatct tgttgaaatt aaaaaaaaaa aaaaaagtct 840
tccttccatc cccaccccta ccactaggcc taaggaatag ggtcaggggc tccaaataga 900
atgtggttga gaagtggaat taagcaggct aatagaaggc aaggggcaaa gaagaaacct 960
tgaatgcatt gggtgctggg tgcctcctta aataagcaag aagggtgcat tttgaagaat 1020
tgagatagaa gtctttttgg gctgggtgca gttgctcgtg gttgtaattc cagcactttg 1080
ggaggctgag gcgggaggat cacctgaggt tgggagttca agaccagcct caccaacgtg 1140
gagaaaccct gtctttacta aaaatacaaa aaattagctg gtcatggtgg cacatgcctg 1200
taatcccagc tgctcgggag gctgaggcag gagaatcact tgaaccaggg aggcagaggt 1260
tgtggtgagc agagatcgcg ccattgctct ccagcctggg caacaagagc aaaagttcgt 1320
ttaaaaaaaa aaaaaagtcc tttcgatgtg actgtctcct cccaaatttg tagaccctct 1380
taagatcatg cttttcagat acttcaaaga ttccagaaga tatgccccgg gggtcctgga 1440
agccacaagg taaacacaac acatccccct ccttgactat caattttact agaggatgtg 1500

gtgggaaaac cattatttga tattaaaaca aataggcttg ggatggagta ggatgcaagc 1560
tccccaggaa agtttaagat aaaacctgag acttaaaagg gtgttaagag tggcagccta 1620
gggaatttat cccggactcc gggggagggg gcagagtcac cagcctctgc atttagggat 1680
tctccgagga aaagtgtgag aacggctgca ggcaacccag gcgtcccggc gctaggaggg 1740
acgcacccag gcctgcgcga agagagggag aaagtgaagc tgggagttgc cactcccaga 1800
cttgttggaa tgcagttgga gggggcgagc tgggagcgcg cttgctccca atcacaggag 1860
aaggaggagg tggaggagga gggctgcttg aggaagtata agaatgaagt tgtgaagctg 1920
agattcccct ccattgggac cggagaaacc aggggagccc cccgggcagc cgcgcgcccc 1980
ttcccacggg gccctttact gcgccgcgcg cccggccccc acccctcgca gcaccccgcg 2040
ccccgcgccc tcccagccgg gtccagccgg agccatgggg ccggagccgc agtgagcacc 2100
atggagctgg cggccttgtg ccgctggggg ctcctcctcg ccctcttgcc ccccggagcc 2160
gcgagcaccc aaggtgggtc tggtgtgggg aggggacgga gcagcggcgg gaccctgccc 2220
tgtggatgcc ccgccgaggt cccgcggccg gcggggccag aggggcccgg acgagctctc 2280
ctatcccgaa gttgtggaca gtcgagacgc tcagggcagc cgggccctgg ggccctcggg 2340
cgggaggggg cagttacacg gcagcggctc gagatggccc atccaagaga ctggcgcttt 2400
ccaggctccg aggggctccg ggaacttgtc aaagaagttc tctgaaattg ttcagaaagt 2460
tttcccgcaa agggtgtatt gcgtagagcg cgcgcgcgcg t 2501

<210> 5
<211> 2501
<212> DNA
<213> Homo Sapiens

<400> 5

atctctgctc actgcaaccc cgcctcccgg attcaagcaa ttgtctgcct cagcctcccg 60
agtagctggg attataggca cccaccagca cgcctggcta agttttgtgt ttttactaga 120
gatgggcttt cgccatcttg gccggggtgc tcttgaactc ctgacctcgt gattcacctg 180
ccttggcctc ccaaagtgct gggattacag gcatgagcct ccgcgcccgg tggagacata 240
attcttacat attggttttc tatccagtgg ccttgtgaaa tatgcttgtg aattctaaag 300
tttacttcta ggtcgttttc agtcttcaat atacagaaac atatcatcct ggaataagag 360
cagttttgtt tccgccattt tttttctttt tccctttgt atttttttgt agagacgggg 420
ttttgccatg tttcccgggc tgttgttgaa cttttgagtg caagtgatgc acccacgtca 480
tctcccacag tgctgggatt actggcgtgg gccaccgtgg cgggcccgtc gttgccattg 540
taaagagttt tatttccttt tctgatttta tggcattgcg cagacccacc cgttacaatg 600
gtgacagtgg acatccttgt cttatccctg atgagaaacc gaaaaatttc aacatttcac 660
catcctattc actctccttt ttttgtagat ggactttatc agagtgagtc attccattct 720
gttccaaatt tgctgagagt attcatttga atatatgttg attttcatca aacagtgcat 780
ctatttcgat taccacagcg ttttttccca ttcatgtgtt aatatagtga attcgattga 840
taaatttgta cgtttttagg ttcgattatt aaaacttgag acagcgtctc actctgtcac 900
cgaggctgga gtgcagtggt gttatcagag ctcgctgcag ccttgacctc ctgggctcag 960
gcgctcctcc cacctcagcc tcctgagtag ctgtgagtat aggtacatgc caccatgccc 1020
agctaatttt tcgatggttt tttgtttgtt ttttgtagtg atgagatttt ctgatgttgc 1080
ttaggctggt ctcgaagtcc tgagctcagg tgatctggcc agctcagcct cccaaaatac 1140
taggattaca ggcgtgagcc ttggcctggt ctggtttttc ttatataggg gtcttatcta 1200
tataaagact aaagttaatc tgtgcctttg tgcgggtggg ctaagagcat gatgactttt 1260
atcattctat tgatttaaag aaaactatcc ttgacttacc agtgtgtaag gccatgaaag 1320
cataattctg ttgaaagcat atattgttaa tgggtgttgg gaaccgtgca ctttccgctg 1380
ctgtgggagc atgtccttgg aggtaccttt catctgtttt ctcaactcca aacatcttag 1440
gaccatgggt tgtgactggt aggactatgt atcttgctgc tttcaagacg gagtatattt 1500
tcacgtggtt tcactctggc tgtcctgttt ccctaatact gtcacttcac cctctgtgat 1560
tctgatgcta caaatgatag atatcgtttt agcattttct tacgggtcct agcgattcta 1620
ttcatttttc tttcagtctc tttctctgac ttgttcacaa tgaacaattt cctttttggga 1680
taggttgcta tttctgtttt cgcaggtggt ttacctgtct tcccagccag tcacagtggt 1740
ccttgtcccc atggtgggtc cggggcaaga gagggccctg ggttgggggt ggggttcagt 1800
tgaagatggg gtgagttttg aggggagcac tacttgagtc ccagaggcat aggaaacagc 1860
agagggaggt gggattccct tatcctcaat gaggatgggc atggagggtt tggggcgtgg 1920
cgctgggaac ggcagccctc cccagcccac agccgcgcat gctccctggg ctcccgcctc 1980
agtgcgcatg ttcactgggc gtcttctgcc cggccccttc gcccacgtga agaacgccag 2040
ggagctgtga ggcagtgctg tgtggttcct gccgtccgga ctctttttcc tctactgaga 2100
ttcatctggt aggtctgcag gccagtcatc ccgggggctg aagtgtgagt gagggtggag 2160
agggccttgg gtgggtcagg cgggtcccgc ttcctggtct gtggcctccg agggagaagg 2220
gccacgaggt cgtcctcctt cccttcacag gctgcgaggc caccggcggc ttcgtggtcg 2280
tgaaggggcc tggacgggga ggaaggtggg ccgtggaggg gaggcggtca ggggctcagg 2340

tgaagatggg gtgagtgctg ttggggggat ggaagtcccg aggtgccggg aacccccgac 2400
gacacagggc agattccctg aatggggcct ccggcggggg cgaggcgggc ggtgaagaag 2460
gggcctggca cctgggaagg ctgcggcctg gtgagcgccc c 2501

<210> 6
<211> 2500
<212> DNA
<213> Homo Sapiens

<400> 6

caagcaattg tctgcctcag cctcccgagt agctgggatt ataggcaccc accagcacgc 60
ctggctaagt tttgtgtttt tactagagat gggctttcgc catcttggcc ggggtgctct 120
tgaactcctg acctcgtgat tcacctgcct tggcctccca aagtgctggg attacaggca 180
tgagcctccg cgcccggtgg agacataatt cttacatatt ggttttctat ccagtggcct 240
tgtgaaatat gcttgtgaat tctaaagttt acttctaggt cgttttcagt cttcaatata 300
cagaaacata tcatcctgga ataagagcag ttttgtttcc gccatttttt tttcttttcc 360
cttttgtatt tttttgtaga gacggggttt tgccatgttt cccgggctgt tgttgaactt 420
ttgagtgcaa gtgatgcacc cacgtcatct cccacagtgc tgggattact ggcgtgggcc 480
accgtggcgg gcccgtcgtt gccattgtaa agagttttat ttccttttct gattttatgg 540
cattgcgcag acccacccgt tacaatggtg acagtggaca tccttgtctt atccctgatg 600
agaaaccgaa aaatttcaac atttcaccat cctattcact ctcctttttt tgtagatgga 660
ctttatcaga gtgagtcatt ccattctgtt ccaaatttgc tgagagtatt catttgaata 720
tatgttgatt ttcatcaaac agtgcatcta tttcgattac cacagcgttt tttcccattc 780
atgtgttaat atagtgaatt cgattgataa atttgtacgt ttttaggttc gattattaaa 840
acttgagaca gcgtctcact ctgtcaccga ggctggagtg cagtggtgtt atcagagctc 900
gctgcagcct tgacctcctg ggctcaggcg ctcctcccac ctcagcctcc tgagtagctg 960
tgagtatagg tacatgccac catgcccagc taatttttcg atggtttttt gtttgttttt 1020
tgtagtgatg agattttctg atgttgctta ggctggtctc gaagtcctga gctcaggtga 1080
tctggccagc tcagcctccc aaaatactag gattacaggc gtgagccttg gcctggtctg 1140
gtttttctta tataggggtc ttatctatat aaagactaaa gttaatctgt gcctttgtgc 1200
gggtgggcta agagcatgat gacttttatc attctattga tttaaagaaa actatccttg 1260
acttaccagt gtgtaaggcc atgaaagcat aattctgttg aaagcatata ttgttaatgg 1320
gtgttgggaa ccgtgcactt tccgctgctg tgggagcatg tccttggagg tacctttcat 1380
ctgttttctc aactccaaac atcttaggac catgggttgt gactggtagg actatgtatc 1440
ttgctgcttt caagacggag tatattttca cgtggtttca ctctggctgt cctgtttccc 1500
taatactgtc acttcaccct ctgtgattct gatgctacaa atgatagata tcgttttagc 1560
attttcttac gggtcctagc gattctattc atttttcttt cagtctcttt ctctgacttg 1620
ttcacaatga acaatttcct tttgggatag gttgctattt ctgttttcgc aggtggttta 1680
cctgtcttcc cagccagtca cagtggtcct tgtccccatg gtgggtccgg ggcaagagag 1740
ggccctgggt tgggggtggg gttcagttga agatggggtg agttttgagg ggagcactac 1800
ttgagtccca gaggcatagg aaacagcaga gggaggtggg attcccttat cctcaatgag 1860
gatgggcatg gagggtttgg ggcgtggcgc tgggaacggc agccctcccc agcccacagc 1920
cgcgcatgct ccctgggctc ccgcctcagt gcgcatgttc actgggcgtc ttctgcccgg 1980
ccccttcgcc cacgtgaaga acgccaggga gctgtgaggc agtgctgtgt ggttcctgcc 2040
gtccggactc tttttcctct actgagattc atctggtagg tctgcaggcc agtcatcccg 2100
ggggctgaag tgtgagtgag ggtggagagg gccttgggtg ggtcaggcgg gtcccgcttc 2160
ctggtctgtg gcctccgagg gagaagggcc acgaggtcgt cctccttccc ttcacaggct 2220
gcgaggccac cggcggcttc gtggtcgtga aggggcctgg acggggagga aggtgggccg 2280
tggaggggag gcggtcaggg gctcaggtga agatggggtg agtgctgttg gggggatgga 2340
agtcccgagg tgccgggaac ccccgacgac acagggcaga ttccctgaat ggggcctccg 2400
gcgggggcga ggcgggcggt gaagaagggg cctggcacct gggaaggctg cggcctggtg 2460
agcgcccccc ccagcggtgt ggagtgcgga gcgcctgagt 2500

<210> 7
<211> 2500
<212> DNA
<213> Homo Sapiens

<400> 7

taaacttatc tgtatctttg tgagggtgtg ctaagggcat gatgaaattt atcattctat 60
tgatttaaag aaaactatcc ttgactttcc agtgtgtaag tccatgaaag cataattatg 120
ttgaaagcat atattgttat gggtgttgag aaccctgcac tttctgctgc tgtgggagca 180

tgtccttgga ggtacctttc gtctgttttc tcaactccaa acatcttagg accatgggtt 240
gtgactggta aggaatgtgt cttgcgagtt tcaagatgga gttgattttc acatggtgtc 300
actctggctc tcctgtttct ctaatactgg cacttctctt tctgtgattc tgatgctaca 360
aatgatagat atcgttttag tattttctta tgggtcctag agattgtatt catttttctt 420
tcagtctctt tctctgactt gttcacattt aacaatttct ttttggggta ggttgctatt 480
tctgttttcg caggtggttt acctgtcttc tcaggcagtc accgtggtcc ttgtccccat 540
ggtggggccg gggcaagaga gggccctgga ggggaggggg gtttagttga agatggagtg 600
agttttgagg ggagcactac ttgagtccca gaggcatagg aaacagcaga gggaggtcag 660
attccattat cctcagtggg gatgggaatc gagggttggg ggcgtggggc tgggaacggc 720
agccttcccc aacccgcagc tgtacatgct ccttggctcc cgcctcagtg cgcatgtcca 780
ctgggcgtct tctgctcagc cgctttaccc acgtggagaa cgccagggag ctgtgagggt 840
gtgtggtctc gttcctgtcg tctggaatat ttttcctcta ctgagattca tctggtaggt 900
ctgcaggcca gtcctcccgg ggtctgaagt gtgagtgagg gtgaagagca ggcagtgtgc 960
ttcgggtgag tccgttgggt cctgcttcgt ggtctgtggc ctctgaggga gaagggcctc 1020
gaggcttctg aaagggaagg ggctcctggc ctctgaaaga gaagggcctt gaggtcgtcc 1080
tccttctctc aaaggctgtg aggccaccat ctgctttgtg gtcgtgaagg ggccaggaca 1140
aggaggaagg tgggccatgg aggggaggcg gtcaggggct caggtgaaga aggggcgatt 1200
gctgggtgtg ctgtcagagg gatggaagtc cggaggtgcc aggaataccc gatacagggg 1260
agatccctga atgaggtccc ggacaggtgc gaggagggcg ataaagaagg gacctggcac 1320
ctgggaagac tgcgggctgg tgagtgcccc tgagctttgt ggagtgggga gccccgagtg 1380
agaagcatcg caagatctca cctccgccat ggaaggtctg gcaacagtgg gaaggactgg 1440
gagaggctgt gcggtccaat caaacttgat ttgagagaag tgaatggctc tagtaagtgg 1500
gagtgtgccc aaagtagcaa tcacgagaat tatgattcac taatgttttc atgtggagtc 1560
cacttgtgaa actaaacctc atcagaaatg acctctgcca gtggggcgcc atggcctgcg 1620
cctgtagtcc cagttactcg ggacgctgag gtgggaggat cccttgagca ggaggtcgat 1680
gctgcagtga gctgtgatca cgccgctgca ctctgccaga gcaacacagg gagactgcgg 1740
gaccaaaaga aatttagaaa aaaaatgtcc tctgcgtttt gtcacacgcc ttaagatgat 1800
tgctctgcca gcttggccag cataagtggc tttgtaggca ctcagaaaag gtacacacat 1860
atgcttaact ctgggactta ttttgaaagt attttcaaaa ttaaaacgac aagttaacat 1920
ttatccatgg aggtgatgga atatagcagc cctttcgagg gcatgctccc aatcacggtt 1980
gtctgttttc agtgtgaaat atgagttggc gaggaagatc gacctatcgg tctagaccaa 2040
gactctatgt agagccccct gaaatgattg ggcctatgct ggtgagtgct taaacgttaa 2100
ttcgttgttt tctattagca gaaattaatt tttgtgacag tattgttgca ttagtatgga 2160
aatgctgata aaggtctttc ctgctcataa aaaatgatga tggcgtctca tgaaggaaac 2220
attgattctg gagaattttt tttcctctag tgttcttcag cttttgccca tgacttcttt 2280
ctcaggcttt gtttgttaat gacagattgt acacatgtat tccaacactg agtataatag 2340
cctccaaagt cctcgtgcgt cacttttctc atagtaacct tcctgtgggt cgagtaacct 2400
tattgggcat agagcataga gttggagaaa tgtctttagg cttagttagg accagaaata 2460
gctatgtatt ctgtgtatat atgtaaaatt ttgtatcaat 2500

<210> 8
<211> 2500
<212> DNA
<213> Homo Sapiens

<400> 8

aagcaattgt ctgcctcagc ctcccgagta gctgggatta taggcaccca ccagcacgcc 60
tggctaagtt ttgtgttttt actagagatg ggctttcgcc atcttggccg ggtgctcttt 120
gaactcctga cctcgtgatt cacctgcctt ggcctcccaa agtgctggga ttacaggcat 180
gagcctccgc gcccggtgga gacataattc ttacatattg gttttctatc cagtggcctt 240
gtgaaatatg cttgtgaatt ctaaagttta cttctaggtc gttttcagtc ttcaatatac 300
agaaacatat catcctggaa taagagcagt tttgtttccg ccattttttt ttcttttccc 360
ttttgtattt ttttgtagag acggggtttt gccatgtttc ccgggctgtt gttgaacttt 420
tgagtgcaag tgatgcaccc acgtcatctc ccacagtgct gggattactg gcgtgggcca 480
ccgtggcggg cccgtcgttg ccattgtaaa gagttttatt tccttttctg attttatggc 540
attgcgcaga cccacccgtt acaatggtga cagtggacat ccttgtctta tccctgatga 600
gaaaccgaaa aatttcaaca tttcaccatc ctattcactc tccttttttt gtagatggac 660
tttatcagag tgagtcattc cattctgttc caaatttgct gagagtattc atttgaatat 720
atgttgattt tcatcaaaca gtgcatctat ttcgattacc acagcgtttt ttcccattca 780
tgtgttaata tagtgaattc gattgataaa tttgtacgtt tttaggttcg attattaaaa 840
cttgagacag cgtctcactc tgtcaccgag gctggagtgc agtggtgtta tcagagctcg 900
ctgcagcctt gacctcctgg gctcaggcgc tcctcccacc tcagcctcct gagtagctgt 960
gagtataggt acatgccacc atgcccagct aatttttcga tggttttttg tttgtttttt 1020

gtagtgatga gattttctga tgttgcttag gctggtctcg aagtcctgag ctcaggtgat 1080
ctggccagct cagcctccca aaatactagg attacaggcg tgagccttgg cctggtctgg 1140
tttttcttat ataggggtct tatctatata aagactaaag ttaatctgtg cctttgtgcg 1200
ggtgggctaa gagcatgatg acttttatca ttctattgat ttaaagaaaa ctatccttga 1260
cttaccagtg tgtaaggcca tgaaagcata attctgttga aagcatatat tgttaatggg 1320
tgttgggaac cgtgcacttt ccgctgctgt gggagcatgt ccttggaggt acctttcatc 1380
tgttttctca actccaaaca tcttaggacc atgggttgtg actggtagga ctatgtatct 1440
tgctgctttc aagacggagt atattttcac gtggtttcac tctggctgtc ctgtttccct 1500
aatactgtca cttcaccctc tgtgattctg atgctacaaa tgatagatat cgttttagca 1560
ttttcttacg ggtcctagcg attctattca tttttctttc agtctctttc tctgacttgt 1620
tcacaatgaa caatttcctt ttgggatagg ttgctatttc tgttttcgca ggtggtttac 1680
ctgtcttccc agccagtcac agtggtcctt gtccccatgg tgggtccggg gcaagagagg 1740
gccctgggtt gggggtgggg ttcagttgaa gatggggtga gttttgaggg gagcactact 1800
tgagtcccag aggcatagga aacagcagag ggaggtggga ttcccttatc ctcaatgagg 1860
atgggcatgg agggtttggg gcgtggcgct gggaacggca gccctcccca gcccacagcc 1920
gcgcatgctc cctgggctcc cgcctcagtg cgcatgttca ctgggcgtct tctgcccggc 1980
cccttcgccc acgtgaagaa cgccagggag ctgtgaggca gtgctgtgtg gttcctgccg 2040
tccggactct tttcctctca ctgagattca tctggtaggt ctgcaggcca gtcatcccgg 2100
gggctgaagt gtgagtgagg gtggagaggg ccttgggtgg gtcaggcggg tcccgcttcc 2160
tggtctgtgg cctccgaggg agaagggcca cgaggtcgtc ctccttccct tcacaggctg 2220
cgaggccacc ggcggcttcg tggtcgtgaa ggggcctgga cggggaggaa ggtgggccgt 2280
ggaggggagg cggtcagggg ctcaggtgaa gatggggtga gtgctgttgg ggggatggaa 2340
gtcccgaggt gccgggaacc cccgacgaca cagggcagat tccctgaatg gggcctccgg 2400
cgggggcgag gcgggcggtg aagaaggggc ctggcacctg ggaaggctgc ggcctggtga 2460
gcgccccccc cagcggtgtg gagtgcggag cgcctgagtg 2500

<210> 9
<211> 2500
<212> DNA
<213> Homo Sapiens

<400> 9

gtctgcctca gcctcccgag tagctgggat tataggcacc caccagcacg cctggctaag 60
ttttgtgttt ttactagaga tgggctttcg ccatcttggc cggggtgctc ttgaactcct 120
gacctcgtga ttcacctgcc ttggcctccc aaagtgctgg gattacaggc atgagcctcc 180
gcgcccggtg gagacataat tcttacatat tggttttcta tccagtggcc ttgtgaaata 240
tgcttgtgaa ttctaaagtt tacttctagg tcgttttcag tcttcaatat acagaaacat 300
atcatcctgg aataagagca gttttgtttc cgccattttt ttttcttttc ccttttgtat 360
ttttttgtag agacggggtt ttgccatgtt tcccggctgg ttgttgaact tttgagtgca 420
agtgatgcac ccacgtcatc tcccacagtg ctgggattac tggcgtgggc caccgtggcg 480
ggcccgtcgt tgccattgta aagagtttta tttccttttc tgattttatg gcattgcgca 540
gacccacccg ttacaatggt gacagtggac atccttgtct tatccctgat gagaaaccga 600
aaaatttcaa catttcacca tcctattcac tctccttttt ttgtagatgg actttatcag 660
agtgagtcat tccattctgt tccaaatttg ctgagagtat tcatttgaat atatgttgat 720
tttcatcaaa cagtgcatct atttcgatta ccacagcgtt ttttcccatt catgtgttaa 780
tatagtgaat tcgattgata aatttgtacg tttttaggtt cgattattaa aacttgagac 840
agcgtctcac tctgtcaccg aggctggagt gcagtggtgt tatcagagct cgctgcagcc 900
ttgacctcct gggctcaggc gctcctccca cctcagcctc ctgagtagct gtgagtatag 960
gtacatgcca ccatgcccag ctaatttttc gatggttttt tgtttgtttt ttgtagtgat 1020
gagattttct gatgttgctt aggctggtct cgaagtcctg agctcaggtg atctggccag 1080
ctcagcctcc caaaatacta ggattacagg cgtgagcctt ggcctggtct ggtttttctt 1140
atataggggt cttatctata taaagactaa agttaatctg tgcctttgtg cgggtgggct 1200
aagagcatga tgacttttat cattctattg atttaaagaa aactatcctt gacttaccag 1260
tgtgtaaggc catgaaagca taattctgtt gaaagcatat attgttaatg ggtgttggga 1320
accgtgcact ttccgctgct gtgggagcat gtccttggag gtacctttca tctgttttct 1380
caactccaaa catcttagga ccatgggttg tgactggtag gactatgtat cttgctgctt 1440
tcaagacgga gtatattttc acgtggtttc actctggctg tcctgtttcc ctaatactgt 1500
cacttcaccc tctgtgattc tgatgctaca aatgatagat atcgttttag cattttctta 1560
cgggtcctag cgattctatt catttttctt tcagtctctt tctctgactt gttcacaatg 1620
aacaatttcc ttttgggata ggttgctatt tctgttttcg caggtggttt acctgtcttc 1680
ccagccagtc acagtggtcc ttgtccccat ggtgggtccg gggcaagaga gggccctggg 1740
ttgggggtgg ggttcagttg aagatggggt gagttttgag gggagcacta cttgagtccc 1800
agaggcatag gaaacagcag agggaggtgg gattccctta tcctcaatga ggatgggcat 1860

ggagggtttg gggcgtggcg ctgggaacgg cagccctccc cagcccacag ccgcgcatgc 1920
tccctgggct cccgcctcag tgcgcatgtt cactgggcgt cttctgcccg gccccttcgc 1980
ccacgtgaag aacgccaggg agctgtgagg cagtgctgtg tggttcctgc cgtccggact 2040
ctttttcctc tactgagatt catctggtag gtctgcaggc cagtcatccc gggggctgaa 2100
gtgtgagtga gggtggagag ggccttgggt gggtcaggcg ggtcccgctt cctggtctgt 2160
ggcctccgag ggagaagggc cacgaggtcg tcctccttcc cttcacaggc tgcgaggcca 2220
ccggcggctt cgtggtcgtg aaggggcctg gacggggagg aaggtgggcc gtggagggga 2280
ggcggtcagg ggctcaggtg aagatggggt gagtgctgtt ggggggatgg aagtcccgag 2340
gtgccgggaa cccccgacga cacagggcag attccctgaa tggggcctcc ggcgggggcg 2400
aggcgggcgg tgaagaaggg gcctggcacc tgggaaggct gcggcctggt gagcgccccc 2460
cccagcggtg tggagtgcgg agcgcctgag tgagaagcac 2500

<210> 10
<211> 2500
<212> DNA
<213> Homo Sapiens

<400> 10

agcaattgtc tgcctcagcc tcccgagtag ctgggattat aggcacccac cagcacgcct 60
ggctaagttt tgtgttttta ctagagatgg gctttcgcca tcttggccgg ggtgctcttg 120
aactcctgac ctcgtgattc acctgccttg gcctcccaaa gtgctgggat tacaggcatg 180
agcctccgcg cccggtggag acataattct tacatattgg ttttctatcc agtggccttg 240
tgaaatatgc ttgtgaattc taaagtttac ttctaggtcg ttttcagtct tcaatataca 300
gaaacatatc atcctggaat aagagcagtt ttgtttccgc catttttttt tcttttccct 360
tttgtatttt tttgtagaga cggggttttg ccatgtttcc cgggctgttg ttgaactttt 420
gagtgcaagt gatgcaccca cgtcatctcc cacagtgctg ggattactgg cgtgggccac 480
cgtggcgggc ccgtcgttgc cattgtaaag agttttattt ccttttctga ttttatggca 540
ttgcgcagac ccacccgtta caatggtgac agtggacatc cttgtcttat ccctgatgag 600
aaaccgaaaa atttcaacat ttcaccatcc tattcactct cctttttttg tagatggact 660
ttatcagagt gagtcattcc attctgttcc aaatttgctg agagtattca tttgaatata 720
tgttgatttt catcaaacag tgcatctatt tcgattacca cagcgttttt tcccattcat 780
gtgttaatat agtgaattcg attgataaat ttgtacgttt ttaggttcga ttattaaaac 840
ttgagacagc gtctcactct gtcaccgagg ctggagtgca gtggtgttat cagagctcgc 900
tgcagccttg acctcctggg ctcaggcgct cctcccacct cagcctcctg agtagctgtg 960
agtataggta catgccacca tgcccagcta atttttcgat ggttttttgt ttgttttttg 1020
tagtgatgag attttctgat gttgcttagg ctggtctcga agtcctgagc tcaggtgatc 1080
tggccagctc agcctcccaa aatactagga ttacaggcgt gagccttggc ctggtctggt 1140
ttttcttata taggggtctt atctatataa agactaaagt taatctgtgc ctttgtgcgg 1200
gtgggctaag agcatgatga cttttatcat tctattgatt taaagaaaac tatccttgac 1260
ttaccagtgt gtaaggccat gaaagcataa ttctgttgaa agcatatatt gttaatgggt 1320
gttgggaacc gtgcactttc cgctgctgtg ggagcatgtc cttggaggta cctttcatct 1380
gttttctcaa ctccaaacat cttaggacca tgggttgtga ctggtaggac tatgtatctt 1440
gctgctttca agacggagta tattttcacg tggtttcact ctggctgtcc tgtttcccta 1500
atactgtcac ttcaccctct gtgattctga tgctacaaat gatagatatc gttttagcat 1560
tttcttacgg gtcctagcga ttctattcat ttttctttca gtctctttct ctgacttgtt 1620
cacaatgaac aatttccttt tgggataggt tgctatttct gttttcgcag gtggtttacc 1680
tgtcttccca gccagtcaca gtggtccttg tccccatggt gggtccgggg caagagaggg 1740
ccctgggttg ggggtggggt tcagttgaag atggggtgag ttttgagggg agcactactt 1800
gagtcccaga ggcataggaa acagcagagg gaggtgggat tcccttatcc tcaatgagga 1860
tgggcatgga gggtttgggg cgtggcgctg ggaacggcag ccctccccag cccacagccg 1920
cgcatgctcc ctgggctccc gcctcagtgc gcatgttcac tgggcgtctt ctgcccggcc 1980
ccttcgccca cgtgaagaac gccagggagc tgtgaggcag tgctgtgtgg ttcctgccgt 2040
ccggactctt tttcctctac tgagattcat ctggtaggtc tgcaggccag tcatcccggg 2100
ggctgaagtg tgagtgaggg tggagagggc cttgggtggg tcaggcgggt cccgcttcct 2160
ggtctgtggc ctccgaggga gaagggccac gaggtcgtcc tccttccctt cacaggctgc 2220
gaggccaccg gcggcttcgt ggtcgtgaag gggcctggac ggggaggaag gtgggccgtg 2280
gaggggaggc ggtcaggggc tcaggtgaag atggggtgag tgctgttggg gggatggaag 2340
tcccgaggtg ccgggaaccc ccgacgacac agggcagatt ccctgaatgg ggcctccggc 2400
gggggcgagg cgggcggtga agaaggggcc tggcacctgg gaaggctgcg gcctggtgag 2460
cgcccccccc agcggtgtgg agtgcggagc gcctgagtga 2500

<210> 11
<211> 2500

<212> DNA
<213> Homo Sapiens

<400> 11
   caattgtctg cctcagcctc ccgagtagct gggattatag gcacccacca gcacgcctgg 60
   ctaagttttg tgtttttact agagatgggc tttcgccatc ttggccgggg tgctcttgaa 120
   ctcctgacct cgtgattcac ctgccttggc ctcccaaagt gctgggatta caggcatgag 180
   cctccgcgcc cggtggagac ataattctta catattggtt ttctatccag tggccttgtg 240
   aaatatgctt gtgaattcta aagtttactt ctaggtcgtt ttcagtcttc aatatacaga 300
   aacatatcat cctggaataa gagcagtttt gtttccgcca tttttttttc ttttcccttt 360
   tgtatttttt tgtagagacg gggttttgcc atgtttcccg ggctgttgtt gaacttttga 420
   gtgcaagtga tgcacccacg tcatctccca cagtgctggg attactggcg tgggccaccg 480
   tggcgggccc gtcgttgcca ttgtaaagag ttttatttcc ttttctgatt ttatggcatt 540
   gcgcagaccc acccgttaca atggtgacag tggacatcct tgtcttatcc ctgatgagaa 600
   accgaaaaat ttcaacattt caccatccta ttcactctcc tttttttgta gatggacttt 660
   atcagagtga gtcattccat tctgttccaa atttgctgag agtattcatt tgaatatatg 720
   ttgattttca tcaaacagtg catctatttc gattaccaca gcgttttttc ccattcatgt 780
   gttaatatag tgaattcgat tgataaattt gtacgttttt aggttcgatt attaaaactt 840
   gagacagcgt ctcactctgt caccgaggct ggagtgcagt ggtgttatca gagctcgctg 900
   cagccttgac ctcctgggct caggcgctcc tcccacctca gcctcctgag tagctgtgag 960
   tataggtaca tgccaccatg cccagctaat ttttcgatgg ttttttgttt gttttttgta 1020
   gtgatgagat tttctgatgt tgcttaggct ggtctcgaag tcctgagctc aggtgatctg 1080
   gccagctcag cctcccaaaa tactaggatt acaggcgtga gccttggcct ggtctggttt 1140
   ttcttatata ggggtcttat ctatataaag actaaagtta atctgtgcct ttgtgcgggt 1200
   gggctaagag catgatgact tttatcattc tattgattta aagaaaacta tccttgactt 1260
   accagtgtgt aaggccatga aagcataatt ctgttgaaag catatattgt taatgggtgt 1320
   tgggaaccgt gcactttccg ctgctgtggg agcatgtcct tggaggtacc tttcatctgt 1380
   tttctcaact ccaaacatct taggaccatg ggttgtgact ggtaggacta tgtatcttgc 1440
   tgctttcaag acggagtata ttttcacgtg gtttcactct ggctgtcctg tttccctaat 1500
   actgtcactt caccctctgt gattctgatg ctacaaatga tagatatcgt tttagcattt 1560
   tcttacgggt cctagcgatt ctattcattt ttctttcagt ctctttctct gacttgttca 1620
   caatgaacaa tttccttttg ggataggttg ctatttctgt tttcgcaggt ggtttacctg 1680
   tcttcccagc cagtcacagt ggtccttgtc cccatggtgg gtccggggca agagagggcc 1740
   ctgggttggg ggtggggttc agttgaagat ggggtgagtt ttgaggggag cactacttga 1800
   gtcccagagg cataggaaac agcagaggga ggtgggattc ccttatcctc aatgaggatg 1860
   ggcatggagg gtttggggcg tggcgctggg aacggcagcc ctccccagcc cacagccgcg 1920
   catgctccct gggctcccgc ctcagtgcgc atgttcactg ggcgtcttct gcccggcccc 1980
   ttcgcccacg tgaagaacgc cagggagctg tgaggcagtg ctgtgtggtt cctgccgtcc 2040
   ggactctttt tcctctactg agattcatct ggtaggtctg caggccagtc atcccggggg 2100
   ctgaagtgtg agtgagggtg gagagggcct tgggtgggtc aggcgggtcc cgcttcctgg 2160
   tctgtggcct ccgagggaga agggccacga ggtcgtcctc cttcccttca caggctgcga 2220
   ggccaccggc ggcttcgtgg tcgtgaaggg gcctggacgg ggaggaaggt gggccgtgga 2280
   ggggaggcgg tcaggggctc aggtgaagat ggggtgagtg ctgttggggg gatggaagtc 2340
   ccgaggtgcc gggaaccccc gacgacacag ggcagattcc ctgaatgggg cctccggcgg 2400
   gggcgaggcg ggcggtgaag aaggggcctg gcacctggga aggctgcggc ctggtgagcg 2460
   ccccccccag cggtgtggag tgcggagcgc ctgagtgaga 2500

<210> 12
<211> 2500
<212> DNA
<213> Homo Sapiens

<400> 12

ctgcctcagc ctcccgagta gctgggatta taggcaccca ccagcacgcc tggctaagtt 60
ttgtgttttt actagagatg ggctttcgcc atcttggccg gggtgctctt gaactcctga 120
cctcgtgatt cacctgcctt ggcctcccaa agtgctggga ttacaggcat gagcctccgc 180
gcccggtgga gacataattc ttacatattg gttttctatc cagtggcctt gtgaaatatg 240
cttgtgaatt ctaaagttta cttctaggtc gttttcagtc ttcaatatac agaaacatat 300
catcctggaa taagagcagt tttgtttccg ccattttttt ttcttttccc ttttgtattt 360
ttttgtagag acggggtttt gccatgtttc ccgggctgtt gttgaacttt tgagtgcaag 420
tgatgcaccc acgtcatctc ccacagtgct gggattactg gcgtgggcca ccgtggcggg 480
cccgtcgttg ccattgtaaa gagttttatt tccttttctg attttatggc attgcgcaga 540

cccacccgtt acaatggtga cagtggacat ccttgtctta tccctgatga gaaaccgaaa 600
aatttcaaca tttcaccatc ctattcactc tccttttttt gtagatggac tttatcagag 660
tgagtcattc cattctgttc caaatttgct gagagtattc atttgaatat atgttgattt 720
tcatcaaaca gtgcatctat ttcgattacc acagcgtttt ttcccattca tgtgttaata 780
tagtgaattc gattgataaa tttgtacgtt tttaggttcg attattaaaa cttgagacag 840
cgtctcactc tgtcaccgag gctggagtgc agtggtgtta tcagagctcg ctgcagcctt 900
gacctcctgg gctcaggcgc tcctcccacc tcagcctcct gagtagctgt gagtataggt 960
acatgccacc atgcccagct aatttttcga tggttttttg tttgtttttt gtagtgatga 1020
gattttctga tgttgcttag gctggtctcg aagtcctgag ctcaggtgat ctggccagct 1080
cagcctccca aaatactagg attacaggcg tgagccttgg cctggtctgg tttttcttat 1140
ataggggtct tatctatata aagactaaag ttaatctgtg cctttgtgcg ggtgggctaa 1200
gagcatgatg acttttatca ttctattgat ttaaagaaaa ctatccttga cttaccagtg 1260
tgtaaggcca tgaaagcata attctgttga aagcatatat tgttaatggg tgttgggaac 1320
cgtgcacttt ccgctgctgt gggagcatgt ccttggaggt acctttcatc tgttttctca 1380
actccaaaca tcttaggacc atgggttgtg actggtagga ctatgtatct tgctgctttc 1440
aagacggagt atattttcac gtggtttcac tctggctgtc ctgtttccct aatactgtca 1500
cttcaccctc tgtgattctg atgctacaaa tgatagatat cgttttagca ttttcttacg 1560
ggtcctagcg attctattca tttttctttc agtctctttc tctgacttgt tcacaatgaa 1620
caatttcctt ttgggatagg ttgctatttc tgttttcgca ggtggtttac ctgtcttccc 1680
agccagtcac agtggtcctt gtccccatgg tgggtccggg gcaagagagg gccctgggtt 1740
gggggtgggg ttcagttgaa gatggggtga gttttgaggg gagcactact tgagtcccag 1800
aggcatagga aacagcagag ggaggtggga ttcccttatc ctcaatgagg atgggcatgg 1860
agggtttggg gcgtggcgct gggaacggca gccctcccca gcccacagcc gcgcatgctc 1920
cctgggctcc cgcctcagtg cgcatgttca ctgggcgtct tctgcccggc cccttcgccc 1980
acgtgaagaa cgccagggag ctgtgaggca gtgctgtgtg gttcctgccg tccggactct 2040
ttttcctcta ctgagattca tctggtaggt ctgcaggcca gtcatcccgg gggctgaagt 2100
gtgagtgagg gtggagaggg ccttgggtgg gtcaggcggg tcccgcttcc tggtctgtgg 2160
cctccgaggg agaaggccca cgaggtcgtc ctccttccct tcacaggctg cgaggccacc 2220
ggcggcttcg tggtcgtgaa ggggcctgga cggggaggaa ggtgggccgt ggaggggagg 2280
cggtcagggg ctcaggtgaa gatggggtga gtgctgttgg ggggatggaa gtcccgaggt 2340
gccgggaacc cccgacgaca cagggcagat tccctgaatg gggcctccgg cgggggcgag 2400
gcgggcggtg aagaaggggc ctggcacctg ggaaggctgc ggcctggtga gcgccccccc 2460
cagcggtgtg gagtgcggag cgcctgagtg agaagcactg 2500

<210> 13
<211> 2501
<212> DNA
<213> Homo Sapiens

<400> 13
   ggggatcttt ctaactctca aagaatgtgc gcttctttaa gggtaacctg cagagtctga 60
   gaacggtcca ggtccaggca tgcacggaca gaaaggatat ttttccccca ggggtttagc 120
   gggggcagga gggggtggac agggatgtgc agggcaagct cctttctgtc ttgcaggcac 180
   ccatctaact tttgcccaaa gcagtaacag tgggcttctg tccccctacc cccaaccccc 240
   cacaccttct ctgacagcag aggatcagac cacctaggtg atggtgaagc tctttgacgt 300
   gcaccaggtg caggagacct atgaccccta gcactgcctt ttatctgctg ggttcccctt 360
   ggagaaccat atatctttgg agccccagaa catgctggtt gcacaagaac aaagcacact 420
   tactgtctga gaggaaggct ggtggtgtgg tagcactatt gaacctctct tttcttttga 480
   gatcatattg tacatacaca gtgctacata actggatgtt tcaagttaat aacttaaaat 540
   gaacagcacc actattacag gaaatattat tcagcactgg ttttggtagt cacccatatt 600
   tcattttcaa gtgtgccata atttacttat atagcaagtc catgaccatg ctattataaa 660
   ttaagctgtt ttggaagtcc catgtatcca aatttttgtt cacatggtta aagaaaaaaa 720
   acattcagtg gtagttgtta aagcacagta aggaaaacct tatttaggac tgtggtgatg 780
   ggtataggga ccattgcaat gggatcttgc agtgggggag agagattggg ctcaatactg 840
   aatacagcgc aggccagtgg gaatgtatag cgaaggagca gtatggcggt ccgtgggtgg 900
   aaaattacta aaaggaaaca tcaggggtaa gggggattct ggctaaaccc acctagcagg 960
   attcttgctg aagacaggcc aaggggaatc agacatcacc tgggggatgg tggaagatga 1020
   agaaccccat gcgatagcaa gtatgatcag atattgagga tggagggttc atgccacatt 1080
   gacttagcag ggctctttgc taaaactggg ttttacaagg aaatgcacag atgggcctag 1140
   gaggagaagt ttcagaagcc tgacttaaaa gtttggccaa gcaaggaata tttgtcaaca 1200
   ttcaagacag tttccttaac attaaagtcc tagaagtaga aatgctgggt cacaggattt 1260
   tttatttttt gagacagggt cttgctctgt tgcccaagct ggagtgcagt ggtgtgatct 1320
   tggctcactg caacctcaac ctcctgggct caagctatcc tcccacctca gcctctcaag 1380
   tagctgggac tacaggtgtg caccaccatg cccagctaat tttgtatttt tttgtaaaga 1440
   cagggtcttg ccatgttgcc caggatggtc tcgaactcct gggctcaaga aatccaccca 1500
   cctcagcatc ccaaagttct aggattacag gcatgagctg ccgcacctgg cctggatttt 1560
   tgtgcaaatg tttaggctta tttatgtatg tgggtttatc atccttctcc tttacatttt 1620
   tttctggatt cctatcatgc ttaaaatggc tttgcactct ctgagaacct aagctttcac 1680
   gcatctctgg gaagactgaa taccctctta agggccactg aggtagcagc actgagaaaa 1740
   aattaaaggg taaatttgca ggtgaaatgc agagctggga acattcctag atccagaagg 1800
   acagaacatt ttgcaggagg ggccagaagc cagaaacaag cccttctcag caccgagcag 1860
   gcccttcctc caggtggcac agagcctcta ggaggcggga gagggctgga aagacaaccc 1920
   acgtgacaca cccccgaggt ttgggattgg ctggtggggt tgacactgct gaggtctaaa 1980
   caaggaggca gaagtaggta ggtgccactt ctcttccctt cattcttcgc caggctctct 2040
   gctgactcaa gtgtaagtga gaacaggtgt gagtgagtgg gccggcgggc ttctggtggg 2100
   tcgggcactg tggtccctgg cctcagagaa ggaagggcct cgaggtcatc atccttctcc 2160
   tagcaggcag ctatgcggcg gggcagggac aggggaggaa ggagggccat gaaggggagg 2220
   agggtcagcc aagatgctgt gagttttgga gcgggacact atctgagtcc tggaggcata 2280
   tgaaactgtc cataaaggac ggatgctggg gtcctcagtg gggccagggg aaagggtggg 2340
   acagcccgcg cttgacagcg cctgcctcag tgctcgtgtt cactgggggt cttcccatca 2400
   gccccttcac ccacgaggtg aactgccgcg gagctgtgag ggtgccgttt gcattccaat 2460
   tgtcgggact ctttcacctg agactgagac tcagtgggtg g 2501

<210> 14
<211> 2501
<212> DNA
<213> Homo Sapiens

<400> 14

ttttactaat acaggaatct cttaccaagg aaaataaaag tttcaaaaaa actttattgt 60
tctttccagc aattgcctga aagatccatc aactcctcaa taaaaagtgt caaatgacat 120
ttttcaatcc caaaactctt tgaacccatt gcctaaaaag tcttgttgca tccaccaatc 180
ctaaatcatt gtcattccta cccaattcta accaaaccat cccccacttt aaaataccca 240
ctttataccg gacatgaaaa gcacaacaaa tatacccaaa gcccttccac atctgacata 300
ctagtgcctc tgtggaggta gtgctctacc ttatagcttt atttaaataa tgtcagcgct 360
gtcttatgaa caggttattt ggctgctatt tggggagtag agtatatgat gagcacagtc 420
accactgctg tacatcatgt cacaataaac taagtttaaa tcctaggacc aatgttgtat 480
tagtccgttc tcatgctgct ataaggacat acctgagagt gggtaattta taaaggaaag 540
aggtttaatt gactcatagt tctgcagggc tggggaggcc tctggaaact taacaatcat 600
ggaagaagtg gaagcaaaca catccttctt cacatggcag caggagagag aagagtgagc 660
aaaaggggaa aagcccctta taaaaccatt agatcttgtg aaaacttact cactatcagg 720
ggaacagcat gaaggtaacc accccatgat tacattacct cccacagggt cctttccatg 780
acatgtgggg attatgggaa ctacaattca aggagagatt tgggtgggga cagagtcaaa 840
ccacatcata tgtatggata tttctgaacc atacactgtc tgaatgacct acaggtatat 900
tcagggaaat cagttaccag tccagagatt aaaatgtgca ataataccac ctaaaaaagg 960
aagaggtgtg aatttattta gtctacagaa gtggttctat tcattggtag catggcagaa 1020
aacctcagac cagagaagga agtgattaat caaagtatga ataaaaattg gccaaggtgg 1080
gaggatcact cgatcccaga aattgagacc agcctggaca atgtagtgag attctgtctc 1140
taagtaaata aataaataaa atagctgggc atggtggcac acacctgtag tcccaactac 1200
ttcggaggct gcgtcttgag tattgcttat gcccaggaga tccaggctgc agtgaggtaa 1260
agtcatgcca ttgccctcca tccagggcga caaagtaaca ccctgtctgg aaaaaaaaag 1320
catgtataaa tataaacatt aaaataattt atttctgtca caaaaggtct ttctgagaca 1380
catcaaccac taagggtgac atgggactct ctgttcccaa agtttctgga tgcatgagaa 1440
tggtaggaca agagagagag aaaaaaaaat atctcttaag tccctttctt tctactacaa 1500
acatactggg gattctggtt ttgggacagt gggaaaaccc tggctggcct tgcatgtgcc 1560
tgactcagtg tgaacgcccc ttactgccca agtcccttgt tgcccatgcg ccttactgtc 1620
tgccttctgt cccagcagag ctgtgcaaag agacatcgtg acttccttct gtcttgccga 1680
ctaggagtca gccagtaggt ctgcagttca gtagtcctag aagtttgagt gtgagtgagt 1740
gtgaggagga gcctgcgggc acgatccatg gcctcctagg tggaagggct ttgaggtccc 1800
cgtccttcta acacgcagcg atgcagccat ggccttgctg tggtgggggt gccggacaag 1860
ggagggagaa ggaccataga tgggaggagg gtcaggtaaa caagcagtga gttttgaagg 1920
gggaaactat ttgtggtatt tgagtccgcg aggcatatga aactgcttac agacgatgca 1980
ttccagagtc ctcagtggga accagggagg ggagggagga ctgcatgacg cgggatgggg 2040
tggggcgagg cggggcactg cagcacacaa cgcaggcacc gacttcagtg tgcatgttcc 2100
ttggacacct gcctcagtgt gcatgttcac tgggcatctt cccttcgacc cctttgccca 2160

cgtggtgacc gctggggagc tgtgagagtg tgaggggcac gttccagccg tctggactct 2220
ttctctccta ctgagacgca gcctataggt ccgcaggcca gtcctcccag gaactgaaat 2280
gtgagtgaat gtgaggagga gtgagcgggc ttccagaggg tcaggcggcg tggtctgtgg 2340
cctcagagag taaaaagctt ccaggtcata aggaaggtgg gccgcggagt gaaggggagc 2400
cagatgaaga tagggtgaat gctcggggtg ctgttggagg tatctgagtc ccgaaagtgc 2460
ctggaactcc caatgggaca gattccagac ttcttggtag g 2501

<210> 15
<211> 2500
<212> DNA
<213> Homo Sapiens

<400> 15
   ccttctttac cgccatcttc accacccacc tcccgcctcg gggccccatt caggactcag 60
   gaatgtgccc tgtgtcatgg gttcctacca cctcgggact ttgacccctc caacagcacc 120
   cgcagcactc gcctcatttt cacctgagcc cctgaccgct tccccttcat ggcccacttt 180
   cctccctgtc ctggcccctt cacgaccaca aagccgatgg cggcgtcgca gcctgtgagg 240
   agaaggagga cgagctcgag gcccttctct tttggaggcc actgaggccc ttctccctca 300
   aaagccacac accaccagac cgagggcccc accgaacctg cccgaagccc actggctccg 360
   cctgaagccc actggctcct cctcaccctc actcacactt cagcccctgg gaggactggc 420
   ctgcggatct accagatgag tctcagtaga ggaaaaaagt gtccggacag caggaacgcg 480
   actgcacact ctcacagctc cctggcgttc tccacgtggg cgaaggggcc gacgggaagg 540
   cacccagtga acatgcgcac tgaggctagc acccaaaggt ggggagggct gcagttccca 600
   gccccacacc ccacactccc gattttcatc cccgtgaaga taatggaatc cgacctccct 660
   ctgctgtttc atatgcctcg ggtactcaaa tacctcaaat agtgctcccc tcaaaactca 720
   ttccatcttc aactgagccc ccctcccctc cagggccctc tcttgctctg gccctgccat 780
   agggacaagg accatggtgg ctgcctgtga agtgaggtag acgacctgct aaaacagaaa 840
   tagcaacata cctcatagca ttatacaaga ctaagagtct cataactaaa tattaaaaat 900
   gtctagcata taattcaaaa ttactcagaa tatgaagaac caggaacata gggccactga 960
   aatagggaaa gacaatcagc agacatcact gctaagatag agatgttggg attatgtggg 1020
   aaagccttta aagatgctat tttataaacg cttaggctgg gcgcggttgc tcatgcctgt 1080
   aatcccagca ctttgggagg ccgaggcggg tggatctctt gaggtcagga gttcgagacc 1140
   agcctgacca acatggtgaa acctcgtctt tactaaaaat gcaaaaaatt agctgggcat 1200
   ggtggcgcgc acctgtagtc ttagctactc gggagtctga aacaggagaa ttgcttgaac 1260
   ccaggaggca gaggttgcag tgaaccgaga tcacgcgact gcactctggc ctggacaaca 1320
   gtgagactcc gtctaaaaaa aaaatgctta aacaagcaat catgaacact gttgaaacaa 1380
   gtaaaaacaa agtcctggca cacacacaaa aaaagaatat ttaaagaaga agcaaatgga 1440
   aattttagaa gtggaaaata tgataacgaa ggaaaataat tctccccaaa tggattcaat 1500
   agcagaatgg agacaagaaa agagtttgtg aactcaaaga tagagaaata gaaattgttc 1560
   tgtgtggcca agagcagtga cttacaccaa taatcctagc acttttgcgt gctgaagtaa 1620
   gaagatagat ggcttgaggc taggagtttc caacctgggc aacatagtga gaccttgtct 1680
   ctacaaaaaa acaattaaaa atagctgagt gtgatgacac atgcctgtag tcctagctac 1740
   tgtgaaggct gaggcaagag gatcccttgg gcccaggagt tcaaggctgc agtgagctat 1800
   gatcacacac tgcagtccag cctgggcaac agagtgagac cctgtgtcta aaacaaaaca 1860
   aaaagaaatt gttgaagtgt ctcataacct gatattcaaa atgtcaagca tataattcaa 1920
   aattactcag aatacgaaga agcatgaaag ttaggcatct tggccaggtg cagtggctca 1980
   tacctgtaat cccagcattt tgggaggctg aggcaggtag atcacgaggt caggagttca 2040
   agaccagcct ggccaagatg gtgaaacccc atgtctacca aaaatacaaa aattagctgg 2100
   gtatggtggc gggcacctgt aatcccagct actcgggagg ctgaggcaga gaattccttg 2160
   aacccgggag gcagagtttg cagtgagcca agatcgtgcc actgcactcc agcctgggtg 2220
   acagagtgag gctccatttc aaaaaaaaaa aaggaagtta tggaatttca aatggagaaa 2280
   gacagacaac tgacaccaat gcaaagacag agatgttgag attatctggg aaagccttta 2340
   aagatgctag tttaaaaaag ctataaacat ccatgtccag acacaagttt tcaactcctt 2400
   tgggtagata ccaagaagta caactactgg actgaatagt aagagtttgt ttagttttgt 2460
   aagaaatact gccaaactgc ctttcaaatt ggctttacca 2500

<210> 16
<211> 2500
<212> DNA
<213> Homo Sapiens

<400> 16

cccaccccac ccggcgcctt acccccatct cccaaacccc accttccgca cctggccctg 60
ccctcaccca cctcctggcc ctgcccccca cctcttgacc ctgttgtctc cgtctcacgg 120
cccccacctc gccaccagaa cctcctagaa cagaacagaa caggatacaa ctgaagcagg 180
cggctgtccg gcctcctggc cccgcagggg caccttccat cctgcgcaga agcggcaccg 240
ccatgcggac cccgcggggc gcctcctctc ggccccgagg cccttgctgc ccctgcgccc 300
cggggacctc tgccgcccgt ggcacccgcc tctcctgggc cgccagcatt gccccctggg 360
ccatcaggaa tgccagggcc tcctgggcca tcagcatcgc ccgtcgaacc ccctgtgccc 420
tggccttcgg cctgcatggc tccggagcct ctgcccggct ctcagagaga aggtcagggc 480
ccacgaggat gcggaggcag agaggctgca ggaagttccg ccccctggcg tgagatgggc 540
agcccgggat cctcagggcg cctgcgcaca ggggccctac ttccggccct gggagacccc 600
gagtgagccc cggagcacgt gaccggttct caccaacccc gcccctcccc aagagagccc 660
gggccggaag gtggccgcaa tgccagcttg gacccctcac ccgtgagcag ccggctgtcc 720
gccggacccc tgtcccggga gccctgcagg gagtcaggca ctgcggggcc cagcctgtcc 780
catcccccgg gtctccctca catcgaggag caagacgggc ctgggaacac ggggccggga 840
ctgtgcggcc atcgtcccgg accctgcctg ccctgtccgt ccttggggga gcgcccagga 900
cagaccccgg ggggcaggcc tctaactggg ctcagcagcc tccgtccctg tcctggtcgc 960
ccagctggtg gggtagctgg aactgcatgt ctggtgggcg tggcctcgcg ccagccaagg 1020
tctcccaggc gccaggtcca gccaaggtgg acgggagggc ccagcgggac ccggggccgg 1080
atctcactcc tgggttcaac gggttctgtc cctgtcctag tcgccccgcc tggtggggga 1140
gctggagctg cgtgtggggt gggttgcggg tggcctccca ccagccaggg gctcccgggc 1200
tctaggcctg gcccaggtgg acaggcggga cctgctggac ctgaggctgg gtcgggcctg 1260
gctcctgggt tcggcagtat cagtccctgc cctggtcgct cttgcctggt agggagctgg 1320
gaggtgaccc tgtgtctgct gggtgctggc ttacttctgt gtctggaagg gggtcagatc 1380
cacaggaaag gtgctgcgag aggcgggcag gggtgggggt ggcatggccc taggagctgg 1440
gtgtctgtcc ccacgccctg ttcacaagtc tgtgggcgga gaggctgcaa tactccatcc 1500
cgggtgtctg ggctgctcac caggccggct caggcttccc acatcctgta ccctgggttg 1560
ggcatgaaac cgatccaatc gtcccatagg ctgttctttt tgatacacat agaaactgac 1620
ccttctggtc ttcaagcttg aaccttacag ttgttttatc tgaattcctt cctcaggaaa 1680
tgaccttcgg gcctctcaaa atatcaaaga actgaaactc accagataac cacatccaga 1740
caatgagatg ctggaccccc caaattcatc aggattgctt tctgggccct cccgagttcc 1800
tgttttccta cacattggtg catttcttcc tggctatata aacccctagt tttagtcagg 1860
gggatggatt tgagacttaa ctcccatctc cccggctgca gcacccaatt aaagccgtct 1920
tccctggtaa tgctcttcgt ctcagtcagt cattggcttt ctgtgcggcc agcagcagga 1980
cgtagaccaa acccttggta tttcggtaac aggcacaccc ccaggaaggg accccggaca 2040
cggctaccac ctctaggctt aaagcgaatg cacttcccgc atctccagtc ttccgtgtag 2100
cagctttaac ctatgtatgt ctgtgccaca tccagtcctc agacggctga gtgaccccaa 2160
gaaaggcttc cccgacaccc agacagaggc tgcagggctg gggctgggtg agggtggcgg 2220
gcctgcgggg acattctact gtgctaaaaa gccactgcag acatagcaat aaaaacatgt 2280
cattttccaa agcaggctcc tgcttctgcc tctgctgctc taagaagggg tcagggtgca 2340
ggaggcaggg ggaacctcct ccagctggag ctgctgcggt gagcaaggct ctgctctgga 2400
ggcctctgag gctggcaccc ttctggggac tgggaagggg gcagggaagg cagcagccca 2460
gggaaagcct cgtccccccg gagccgaggc agttggggag 2500

<210> 17
<211> 2501
<212> DNA
<213> Homo Sapiens

<400> 17
   tgagggtgag gtggcagaag ggttggggct ggtgctgggg ctggagggtg gtggtcaggt 60
   gacggagtag gagtagggat gggggtggag gtgtagctga ggagatgggg gttgggtggg 120
   gaatgggaat gcgaatgggg tgggagtggg gattggagtg cagtcttggg ggcccacagc 180
   cctccctctt gggtgctgat cttgatgcct ggcagagcct gtgccctgcc ctctcctaac 240
   cagtcctgcc ctggtcacag caacctctga ttccagagct tggggagaag aacgtggtgt 300
   ctctcaaggc ttttggagca ggggtctggg ggcagcccag gctgagacgt cttcccgcgg 360
   gtgggccagg gctggcagca agggcggcgc tggaatattt ggggctctct atttggagtg 420
   gcggggtcct cagtcctccc tcagcctctc accttgcctc ctcacagagc ctgggtcagg 480
   ttccttcggc cgaactctac gccgtccctc agaatggaaa cctcgcttct ctctgcccca 540
   caatgcgcaa gtcagtggcg tcacatccgg gcacccggat gtttcctggc gggaaccgga 600
   tcctgcctgg gtgggggtgg gagtgggcgt gggggtgggg gtgggggtga gggtggaatg 660
   gctgggcgga tgggcatcag ggttgaggga tggggtgggg gatgtggggt gggggttgga 720
   atgacaatga agagggggtt gggtcacggg agtgagcatt ggagtggggg ttgggtcacg 780
   ggagtggggt cttaggaccc ctcagccctc cttgagaatc ctgagcatga tgtctggcag 840
   agcctggacg ctgccctctc ctaagcagcc cttccctgat cacacgagct ctgacttcag 900
   agtccccagt ggtttaagca gcaagggggc tgggggtggc ccagcctgag aagtccacca 960
   ccagatggtc caggactggc atgaagggat tatttggggt cctctatctg gggtaagtgg 1020
   gtcctcagtc ctccctcagc atgtcacctt gcctccttac agagcctggg cccgcttccc 1080
   tccaccgatc tcaagccgcc cctcagaccg aaaacctcgc ttctctctaa ccactaatgc 1140
   tcaagtcagt ggcgtcacat ctgggcacct gggcttccct gggttgagag caggggtgga 1200
   accggattct gcctggcttg ggctgggggt gagcgtgaag atgtttggat gggtggcgta 1260
   aagggaggga ttgggggtgg tagggggagg gatgccagtg gtgccccggg gcttaagtcg 1320
   tcgtgttggt ggtagtgggg ttgggcgtgg ggttggggga ccctcggtca cccctcaggg 1380
   tcctgacttt catgcctggc agaatctgga cacccatatt ctctgccgat tgtgtctgct 1440
   tctctcccac caagtctctg gcccccagtc ccatgaggtg gcaatgggag ggggggtgtg 1500
   gtcgtggtaa cattgccagg gtcttccagg gctgacagga ggggctgagc tgcattctgt 1560
   ggcccatcta cgtcgggaga gtggaccctc tgtccccact caggaaggtc ctcctcccag 1620
   ctcctggcac tttgttgaag tcatgggcgg gagatgctgt ttctgccatc tctgagcatt 1680
   tgcccagctg cacccattgc agagaatggc tgcaggtccc aggccaggtg ctccctgcag 1740
   agtggcagct cccgcgattg tagtgacctc tgggagaaga cgcacacctt cccacggggg 1800
   cttctctcca gccagagcta cacttggcaa accttttgtc ctcaaagtaa tatgttttac 1860
   tcccaggcac ggtggttcac gcctgtaatc ccaacagttt gagaggccga ggcgggcaga 1920
   tcacttgagg tcaggggttc aagaccagcc tggccaacat agtgaaaccc cgtctctaca 1980
   caaaatacaa aaattagctg ggtgtggtag tgctcaggag gctgaggcgt gagaatcgct 2040
   tgcacctgga ggcggagttt gcagtgaggc gagatcgtgc cattgaactc tagcgtgggc 2100
   ctcgcgacag agtgacactc tgtctcaaaa aatttaaaaa atgaaaaaga aatacacttt 2160
   tcgtatcttc caaggatata tgtaggtagc tgtatatata tatgtattta tttctaggaa 2220
   agaataccta ccagtagtat gcatgattca ctcttatatc ctattctact tcttttccat 2280
   tctttacgtt ctgttgaaat aatttttttt gaaacacaat ttctcaccca ctatgttggt 2340
   gtctggttgt tattagtgga actcttgatc catactgtct taaccagccc tgtttaactg 2400
   gtgtcctgct gttgccctag ggctactagc atcatgtttt gaggagctgc ctcagactgt 2460
   gtaatgatgt gaagtggaaa tttgaaaact ttcattgtaa c 2501

<210> 18
<211> 2501
<212> DNA
<213> Homo Sapiens

<400> 18

gcagggcccg ggccttccct ctgctcccct gaagccgcat tcttggtacc aatatcccct 60
tccctacgtc agcctcagat gccgacgtca gaatttgcgt tcctggtcgc catgaccagg 120
gcttcccagg gccgacatca ggggcagagc cctccctgag cgtcccgaca ttgatgctgg 180
cagaacctag gcgcggctcc cgccgaacca gccgggccct ggtcacacca agctctgact 240
cccagagtcc cctgcggctt aagcggcggg agagagtggg ggcgggggtg gggggggggt 300
gacagcccag cctgagaagt ctgccctcag gtggtccaga gctggcagca gaggcagcgc 360
tgtattattt agggctttct atctgggttg ggggcgtcct cagtcctccc tcagcctctc 420
accttgcctc ctcacagagc ctgggcccgc tctcttcagc cgatctcaag cctgtccctc 480
agaacgagaa cctcgcttct ctctgatccc cgaggcgtaa gttagtggcg tcacatccgg 540
tccccggggc ttccctgggt tgacagcagg ggcggagtgg attctgccgg aactggggtg 600
ggagttgggg tgggagtgag ggtgggagtc cgggtggggt ggggtacggg tgaggatggg 660
gttggggcag gggtggtggt gaagatgagt gtggggaggg gcgtggaggt gggaactggg 720
atggggatct tggggctgtg ggtcgtggtg ggggtggggg tggggtcttt cagtcatccc 780
tcagtgtctc gaccttgatg cctggaaaag cctggacccc tgttttgtgc tgaggatgtc 840
ttctcccctt aaaccaggtc tcttgactcg gagtcttcca aggtggcagt gaggggaggg 900
ttgtggtagg ggtgacaaca ttgccagggt cttccagggc tgacaagagg ggctcagctg 960
gattctgtgg cccctttgtg aggggtgggt ggaccctcgg tcctcactca ggagggtctt 1020
cctcctggct tttgtctctt tgatgaagtg atgggtggga gatgctcagt ttccgtcacc 1080
tctgatcatt tgcccagctg catcccttgc agagcatggg tgcggttccc aggccagatg 1140
gtccctgggg agctgcaaca cccgtgattg taatgaccta tgggagaaga cgcacacctt 1200
cccaacgggg ctcttcccaa gccacaagta gacttggcaa actttttgtc ctaaagggta 1260
tattttctac aagaaagagg ctgagcatcg gggtgtggag aggtcctgct gttttgagtt 1320
tagctgatat tttatagtcc aatgcagtga tgtctggttt tctaaccctc attcctagta 1380
aaaatatgtg ttttaaatgt ctgccaagtg tatattgaat acatatataa catttcatga 1440
aagaatacct accttacgct gtgtggttca ctgcttattc tatttctggt ctattcttta 1500
tattctatta taataatttt tctctttttt tctgagcaag aaaatttaat ataaagaatt 1560
attaacttgt agcagaggat caactaagac cagtcgtcac tgtggtggtt ttaggtggtt 1620
agcagcatag actccggagt catactccct caatcaaccc cgtctttcct gctgtcctga 1680

tatcactcca gagctactgg gctactggca tcgtggtatg aagtgttgcc tcagattgtg 1740
caataaagca gggccagaaa ttcaaatatt tcttcctact cgtcctctta tttgccgtct 1800
atgcaggctg cggacctccg caaatgcact gttgtcctcc actcccccta ctccaaacac 1860
aaggtccaat tcgaaattct gcagtctgct tagggttcta tcatgttttg ctgtgtagaa 1920
tttgttgagt gttgataatg ttttttccct tctcccctcc aggattgatt ctggagtgta 1980
agtcagcaat atgaacttga tgctcatttt ggcctaaagg taaggcacta attctgtaaa 2040
taatttaagg aaaattcacg cctttaaaat attctgttct ccattaatgg atatggtata 2100
atgctttatt tatttgggac ttctttcacc tgaatctatc agcaaacttt tacagttgac 2160
tccagtaaga tcctgctcat ttttgttagg attatcttta aggtaatttt aggagttttt 2220
tgctgttttt aatgggatat tttctattgt atgttctatt tactgttttt ttatgaccag 2280
tttcttgatt ttgctataat gagcttccat aaaacatttt tcttctgaag tcttttattc 2340
atttaaatat tccacatgct tattcctttg actattcgtt aaagataaca ttgtctccaa 2400
acatctctct tttcaaaatt catgcatctt atttagtttg atacccatag ctctggctat 2460
gtgtttcagt gcagttttga acagtagaga aggtagcata c 2501

<210> 19
<211> 2501
<212> DNA
<213> Homo Sapiens

<400> 19

caggggcaaa gctccgattc tgccaggatg ggtggggatg ggaatcgggg tttgggggtc 60
ccgttgatgt ttgagcgatg gtggtgggtt tggtgttggg ggatgggggt ggggttggtg 120
ttgggggatg ggaatggtgg tgggggtgcg ggggggttac cctcagtccc ctcttggggt 180
cctgaccttg attcctggct gagtctgggc cctgagcact tctaaccagc cctgccctgg 240
tcacacaaag ctcttacacc agagttccct gcggttaaag cggcgggggg tggcggggtg 300
aggtggtggc gactgagcct gagaagtctt cccctgcagg gtggtccagg cctggcagca 360
gaggcagcgc tggattattg gggcccctct gtctggggtg aggcccgcct cagtcctccc 420
tcggcatctt accttgcctc ctcacggagc ctgcgcctgc ttccctccac cgacgtcagg 480
ccgttgctca gggcgagaat ctcgctgttc tctgacctcc aacgcgcaag tcagtggcgt 540
cacatccggc cccgtgactt ccctgggttc acagcagggg tggaactgga ttcttcctgg 600
atggggatcc agatggaggt ggaggtaagc atgaagatgg tgttgggggt aggggtaggg 660
atagggacgg ggatcctgag gctgtagatc ctggtggggt tgggggagtt gggggtggga 720
ggggcctttg gtcatccctc aaggtcctga cttggatgcc aggctgagcc tggaccgcca 780
tcctctgccg attgtgtctg ctcccctcag accaagtctc tgactctgag tccccctgaa 840
gtggcactgg gggaggggtg cggtcggggt gacattattg cctaagttta tcagggctga 900
caagagggac tgagctggat tctgtggtcc cactacgtgg gggagggtgg accctcagtc 960
ctcactcaag agggtcctct tcctcactcc tggctgttgg atgaagtgat tggcaggaga 1020
tactctgttt ctgtaacgtc tgtgcatttg cacagctgca ccccttgtag agaatggctg 1080
cgggtcccag gccaggagct ccctgcaggg cgggagctcc cacgatcgta ttgacctctg 1140
gaagaagaca gacactttcc cacgggagct cctctccagc cagagctaca cttggcaaac 1200
ctttggtcct aaatgattta ttcactgaat tgaagaaata cggtttacat atcttccaag 1260
tatatatgta ggtagcgata tatatattta tttctacgaa ctaacaccta cccttagtat 1320
gcatcgttca ctcttactat cctaatctac ttctattcca gtctttgtgt tctgttgaag 1380
gaattttttt ttaaacgtaa ttcctctccc actatgttgg tttcaggttg ttattagcag 1440
aactctggag ccatgctgcc ttaacaaacc caattttcct ggtgtcctgc tattacctag 1500
ggctcttggc atcgtgtttt caggagctgc ctcagattgt gcaatgaagt gaagtggaaa 1560
ttttaaaact tttattctac ccattctgtt atctgccgtc taggcaggct acaaacctcc 1620
ccaacatccc tgccgctaac cccctctaac cgacacggca agtgtcacac acaagtacca 1680
gctcaacaag gtcttcagtc tcaccctgga ctggccctgt tttgttgtgt agattttctt 1740
gctaatactg gttttttact ttctgtttcc attctataaa tttacagggt tgatttggga 1800
agcagagcac agcagcccaa atttgcttgt aatgtcggcg actacaggta aggcactaaa 1860
ttgtaaataa tttaagctga gtttacattt ttacactgtt gtgttcccca tgaatgaata 1920
tgttatactc ctctatgtat gtcaagctat tttttcctga atatgtcagt atacttcgat 1980
agttgcctct ataagcagct tatacatttt tattagactt ctattcaggt aattcttgga 2040
ttttattgct attgcaaatg gcatattgtc tattaattgc tgtagttagt tattgctttc 2100
gtgaggtgaa gattttgatt ttgctgtagt aatctgttgc acacctcatt ctgaaggccg 2160
atgcacttga atattccaca tgccttttcc tttgaatttt caagatcgat aatggtatta 2220
tcactaaaca ttacatgttt ttccaatatt cataccactt actcattttg atacttatag 2280
ctttttgtgt ctcaatcctg atttcaaacg tagagataat aggcttcatc tacttctcgt 2340
tcctgactgg aatgatagtt cttcgaacat ttgacattta agtatgtttg ctgttgattt 2400
taggaaatgg aagtgctctt tgaatatagg ttaaataaat attttctttt ttccataaac 2460
tcattaaatg tctttaaaaa gctttttctg taccctttga c 2501

<210> 20
<211> 2501
<212> DNA
<213> Homo Sapiens

<400> 20

gccgtacatg tgaacgtcag gatccgcgtg cttggccgcc attcccggag cttccgtggg 60
ttgattgcag gggcaaagcc cggattctgc caggatggtg gtgggggagg agatgggaat 120
ggggtttggg ggtggggttg gtgtttgagg gatgggggtg gggttggtgt tgggggatgg 180
gattggtagt gagggtaggg ggtgggttgc cctcagtccc ctcttggggt cctgaccttg 240
attcctggct gagtctgggc cctgagcact tctaaccagc cctgccctgg tcacacaaag 300
ctcttacgcc agagttccct gcggttaaag cggcgggggg tggcggggtg aggtggtggc 360
gactgagcct gagaagtctt cccctgcagg gtggtccagg cccggcagca gaggcagcgc 420
tggattattg ggggccctct gtctggggtg aggcccgcct cagtcctccc tcggcgtctt 480
accttgcctc ctcacggagc ctgcgcctgc ttccctccgc cgacgtcagg ccgttgctca 540
gggcgagaat ctcgctgtcc tctgacctcc aatgcgcaag tcagtggcgt cacatccggg 600
cccgtgactt ccctgggttc acggcaaggg tggaaccgga ttctgcccgg atggggatct 660
ggatgggggg gtgggggtaa gcatgaagat ggtgttggtg taggggtagg gattgggacg 720
gggatcctga ggctgtagat cctggtgggg tcgggggagt tgggggtggg aggggccttt 780
ggtcatccct caaggtcctg acttggatgc cagactgagc ctggaccgcc atcctctgcc 840
gattgtgtct gctcccctca gaccaagtct ctgactctga gtcggcccga agtggcattg 900
gggggagggg tgtgctcggg gtgagatcat tgccagtgtg taccagggct gacaagaggg 960
gctgagctgg attctgtggt cccactatgt gggtgagggt ggatcctcag tcctcactca 1020
ggagggtcct cttcctcact cctggctgtt tgatgaagga atgggtggga gatgctctgt 1080
ttctgtcacc tctgagcatc tgcgcatctg cgcatctgca ccccttgtag agaatggctg 1140
cgggtgccag gccaggtgct ccctgcaggg caggagctcc cgcgatgata gtgacctctg 1200
ggagaagacg cacaccttcc cacgggggct cctccccagc cagagctaca cttggcaaac 1260
ctttggtcct aaatgattta ttctctgaat tgaagaaata cggtttacat atcttctaag 1320
tatatatgta ggtagcaata tatatattta tttctatgaa ctaacaccta cccttagtat 1380
gcatgattca ctctgatatc ctagtctact tccattccag tctttgtgtt ctgttgaaag 1440
aattttttta aaaaacataa ttcctctccc actctgttgg tttcaggttg ttattagcag 1500
aactctggag ccatgctgcc ttaacaaacc caattttcct ggtggacact gctattacct 1560
agggctcttg gcatcgtgtt ttcaggggct gcctcagatt gtgcaatgaa gtgaagtgga 1620
aaattcaaaa cttttattct acccattctt tcatctgccg tctaggcagg ctacaaacct 1680
ccccaacatc tctgccgcta accccctcca acagacactg caagtgtcac acacaagtac 1740
cagctcaaca cagtctccag tctcactctg ggctttccct gttttgttgt gtagattttc 1800
ttgctgatac tggtgtttta ctttctgttt ccattctata aatttacagg gttgatttgg 1860
gaagcagagc acagcagccc aaatttgctt gtaatgtcgg cggctacagg taaggcacta 1920
aattgtaaat aatttaagct gagtttacat ttttacactg ttgtgttccc cgtgaatgaa 1980
tatgttatac tcctctatgg atttcaagct cttttttcct gaatatgtca gtatacttcg 2040
gtagttgcct ctataaacaa cttatacatt tttattaggc ttctattcag gtaattcttg 2100
gattttgttg ctattgcaag tggcatattg tctatcaatt gttgtagtta gttattgctt 2160
tcgtgaggtg aagattttga ttttgctgta gtaatctgtt gcacacctcg ttctgaaggc 2220
cgatgcactt gaatattccg catacctttt cctttgaatt ttcaagatcg ataatggtat 2280

tatcactaaa cactacatgt ttttccaatg ttcataccac tcactcattt cggtacttac 2340
agctctttgt gtctcaatcc tgatttcaaa tgtagagata ataggctaca tctacttctc 2400
gttcctgact tgaatgataa ttcttcgaac atttgacatt taagtatgtt tgctgttgat 2460
tttaggaaat ggaagttctc tttcagtata ggttgcctaa a 2501

<210> 21
<211> 2501
<212> DNA
<213> Homo Sapiens

<400> 21

cattcccgga gcttccgtga cttgatacca ggggcaaagc ccggattctg ccaggatagg 60
ggtgggggtg gggatgggaa tgggggatgg gggatggggg tggggttggt gttgggggac 120
gggaatggtg gtgagggtgg tggttaggct gccctcagtt ccctctcggg gtcctgacct 180
tgattcctgc cacagcgtgg gccctgacct cttctaacca gccctgccct ggccacatta 240
agctctgtcc ccagagttcc ccgtggctaa agcggcgggg gtcggcgggg tgaggtagtg 300

gcgacccagc ctggaagtct tcccctgcgg ggtggcccag gcccggcagc agaggcagca 360
ctggattatt tgaggccctc tgtctgaggt gaggcccgcc tcagtcctcc ctcagcgtct 420
taccttgcct cctcaccgag cctgggccgg cttccctccg ccgacgtcag gccgtcgctc 480
gttgctcagg gcgagaatct cgcggtcttc tgacctccaa tgcgcaagtc agtggcgtca 540
catcctggca acggtactac cctgggttcc gggcaggggt ggaactggat tctgcctgga 600
tggggatcgg atgggggtgg aggtaagcat gaagatggtg atggggcaag gggtaaggat 660
tgggacgggg gtcctgaggc tgtagatcct ggtggggtcg gggagttggg gggtgggtgg 720
gcggggcctt cggtcatccc tcagggtcct gaagttgatg cctggctgag cctggaccgc 780
catcctctgc agattgcttc tgctcccctc agaccaagtc tctgactctg agtccccctg 840
aagtggcatt gggggaaggg gtgtggtctg ggtgacatca ctgcctgcgt ttaccagggc 900
tgacaagagg ggctgagctg gattctgtgg tccctctatg gtgtgtgggg tgggggggcg 960
gtgtggaccc tcagtcctca ctcaggaggg tcgtcttcgt cacctctggc tgttggatga 1020
agtgatgggc aggagatgct ctgtttctgt cacctgtgag catttgcaca gctgcacccc 1080
ttgtagagaa tgcctgcggg tcccaggcca ggtgctccct gcagggcggg gcggcagctc 1140
ccgcggttgt agtgacctcc tatttcagtc tttgtgttct gttgaaagaa tttttttttt 1200
taaaaaatgt aattcctctg ccactatggt ggtttcaggt tgttattagc agaactctgg 1260
agtcatactg ccttaaccaa ccaaattttc ctggtgtcct gctattacct agggctcttg 1320
gcatcgtgtt ttcagaggct gcctcagatt gtgcaaagaa gtgaagtgga aaatttcaaa 1380
cttttattct acccattctg tcatctaccg tgtaggccgg ctacaaacct ccccaacatc 1440
cctgctgcta accccctcca accaacactg caagtgtcaa acacaagtac cagctcaaca 1500
cagccttgag tctcaccctg ggctttccct gttttgttgc gtagattttg ttgctgatac 1560
tggtttttta tttactgttt ccattctgta attttccagg tttgatttgg gaagcagagc 1620
acagcagccc aaatttgttg taatggcggc ggctacaggt aaggcactaa attgtaaata 1680
atttaagctg agtttacatt tttacagtgt tgtgctcccc atgaatgaat atattatact 1740
cctctatgta tttcaagctt ttttttacct gaatatgtca gtatacttcg acagttgtct 1800
ctataaagac cttatacatt tttattagac ttcttttcag gcaattcatg aattttgttg 1860
ctattgcaaa tggcatattg tctattaatt gttgtagtta gttattgctt tcgtgaggtg 1920
aagattttga ttttgctgta gtaatctgtt gcactcctcg ttctgaaggc cgatgcactt 1980
gaatattcca catacctttt cctttgaatt ttcaagatcg ataatggtat tatcactaaa 2040
cattacatat tcttccagta ttcataccac ttactcattt tggtacttat agctctttgt 2100
gtctcaatcc tgatttcaat cgtagagata atagcctaca tctacttctc gttcctgacg 2160
tgaatgataa ttattcgaac gttttgcatt taagtatgtt tgctgttgat tttaggaagt 2220
gcaatttctc tttgaatata ggttaaatac tttttttttt ttttaccatg aactcatatt 2280
aaatttcttt aaaaagcttt ttctgtaccc gttgacacaa tcaaattttt ttctcctcca 2340
ttttgtatgt aaggaattac aatgaaaccg tttttttttt ttcccctaat gttaaaccgt 2400
atgttatttc cttgggcaga gcctgtttat ttaatatgct agtaatttac gtaggatatt 2460
tgctgctatg taagggagct tacttaatgt gtgctttttt t 2501

<210> 22
<211> 2501
<212> DNA
<213> Homo Sapiens

<400> 22
   gggaagcggg ccaggccctg tgaggagtca aggtgagacg ctgagggagg actcaggagg 60
   cccccacccc agatagatga ccccaaataa tcccgcacca ctcctgctac cagccgtggg 120
   ccacccgtgg gcggacttct gagtctgggg cgcccaccac cccactgcct ctgaagttgc 180
   aggggactct ggagtcagag cttgggttga ttagtgtaag actagtgagg gcaggctctg 240
   ccaggcatgg acctcagcac cctaagagag ggctaagcgt accccacccc tattcccatc 300
   ccccaccacg tcccctttcc gattaccatt tgcactccca aaccatccac gcccccatcc 360
   cccaccagca ctcctctcct cttcaacccc ccacctctct cataccgcca tctcccaccc 420
   caacaacccg ggcccttcta ccaacctcac ccctcccacc cccatccaca ctgaatcacg 480
   ttccgcttcc gctttcaacc cacccccaaa aacccgcccc ctccatcgac ctcacccctc 540
   ccacccccat ccacgctgaa tctgatttcc gcttcctctt tcaacccaag aaagccccag 600
   gggcccggat gtgatgccac tgacttgcgc attgggggtt agagagaagc gagctgctct 660
   gtctgaccag cagcttggga ttggcggagg gaagcgggcc aggccctgtg aggagtcaag 720
   gtgagacgct gagggaggac tcaggaggcc cccaccccag atagacgacc ccaaataatc 780
   ccgcaccact cctgctacca gccgtgggcc acccgtgggc ggacttctga gtctggggcg 840
   ccctccaccc cactgcgtct gaagtcgcat gggactctgg agtccgagct tggggtggtt 900
   agtgtaagac tagtgagggc aggctctacg aggcatcaac ctcaggaccc taagagaggg 960
   ccaagcgtac cccaccccta tttctatccc ccaccgcctc ccctttccca ttactatttg 1020
   cactcccaaa cccatccgcg cccctatccc ccaccagcac tcctatcctc aaccccgcac 1080
   ctctctcaca ccgccatctc ccaccccaaa aactggggcc cctccaccaa cctcacccct 1140
   cccaccccca tggatgctga attgggttgc gcttccgctt tcaacccacc cccaaaaacc 1200
   cgccccctcc accgacctca cccctcccac ccccttccac gctgaatcgg gtttccgctt 1260
   ccgctttcaa cccaagaaag tcccaggtgc ccggatgtga tgccactgac ttgcgcattg 1320
   ggggttagag agaagcgagc tgctctgacc agccgcttgg gattggcgga gggaagcggg 1380
   ccaggccctg tgaggagtca aggtgagatg ctgagggagg actcaggagg cccccacccc 1440
   agatagacga ccccaaataa tcccgcacca ctcctgctac cagccgtggg ccacctgtgg 1500
   gcggacttct gagtctgggg cgcccaccac cccactgcct ctgaagtcgc agaggactct 1560
   ggagtcagag cttagggtgt ttagtgtaag actagtgagg ccaggctctg ccaggcatca 1620
   atctcaggac cgtaagagag ggctaagcgt accccacccc tattcccatc ccccatcacg 1680
   tcccctttcc cattaccatt tgcactccta aacccatccg cgcccccatc ccccaccagc 1740
   actcctcctc gaccccccac ctctgtcata ccgccatctg ccaccccaaa aaccggggcc 1800
   cctccaccaa cctcacccct cccaccccca tccacgctga atcgggttcc gcttccgctt 1860
   tcaacccact cccaaaaacc cgccccctcc accgacctcc cccccacccc catccacgct 1920
   gaatcgtgtt tccgcttccg ctttcaaccc aagaaagcct caggggcccg gatgtgatgc 1980
   cactgacttg cgcattgggg gttagagaga agcgagctgc tgtctgacca gcagcttggg 2040
   attggtggaa ggaagcaggc caggccctgt gaggagtcaa ggtgagacgc tgaggaggac 2100
   tcaggaggcc accaccccag atagacgacc ccaaataatc ccgcaccact cctacctgcc 2160
   gtgggccacc tgtgggcgga cttctgagct tggggcgccc accaccccac tgcctctgaa 2220
   gtcgcagggg actctggagt cagagcttgg ggtgtttagt gtaagactag tgaggccagg 2280
   ctctgccggg catcaatctc aggaccctaa gagagggcta agcgtacccc acccctattc 2340
   ccatccccca ccacgtcccc tttcccatta ccatttgcac tcccaaacca tccacgcccc 2400
   catcccccac cagcactcct ctcctcttca accccccacc tctctcatac cgccatctcc 2460
   caccccaaca acccgggccc ttgtaccaac ctcacccctc c 2501

<210> 23
<211> 2501
<212> DNA
<213> Homo Sapiens

<400> 23

cttcacgtca atgccaaaga ccagcgccaa ggactcggag gctttgccga agatcacagg 60
aaagcagcgc ttgtaatttt tgatgacgct ctccagcatt tctgcctttg tgaccggctc 120
cttgacttaa tacttgagga gcagaaaatg aatcaagtca gccaccttct tactgagtgc 180
tgctcggaac acagactctg ggtcagggga ggtgcttggc ccctcctctt cttggttgct 240
ggagccctta atggattgcc tccatagagt gaaatcgatg gcagtgggga tggcggaggc 300
tccctgagga ctcttgagag gacctggtga cccagcagca ggcacctccc ccagggtgcc 360
tgggaccaga ggagaggagg aggacacagc ctcctgctcc tcagtagtgg cagcctgcac 420
acccaccagg cccagggcct cttcttgggt gtcaaggcct tcctcaggct tgcagtgctg 480
actcttctgc tcaagagaca tgacgactct ggtcaccgca acaggcagga gtgtgggcag 540
gagctgggca atggagaccc actggcctgg agagagaggg agcatgtgag aagcctcagc 600
taaaaactga accttggagg ctctaacaaa ggcttactta cagatcttct cctttggtgc 660
tcctctaggg cctcctgggg atcctctgga gcttcctggt gatcctggtc agcctgtccc 720
ctgagaacct gaaggaaaaa gtgagagggc acctcagggt acacaagcaa gcagaggctg 780
attctgcagg actgacagta gagagggtga ggccaggtgc tctggagtcc catatgttct 840
ggggcgatgg gggccattag tgtgcattca aggcaaacgt tcactgctgg cactagacgt 900
ctgcatctcc tctgctctgt gacctgaggg cacggcctca gaccaaggcc tcagtgtctg 960
tttcctggag ctcctggaag aggaatgcat gggccctcag ggtgcagact gcaagcacag 1020
cctcaggccc ccagtgctaa caggagggta ggctggaatc tgtgaggtct ccaccctctt 1080
ggggtgaatg gtcccctctg tgctcacaca gggccctcac ctttctcctg gcaaggcctg 1140
gatcccaccc ttttgctggc ctgagaaact cagattaaga gctcacatcc ctgataggga 1200
acaggacatg aggagactca catctggcca cacgtgccca ggtcctcttg ggaaggacag 1260
gaagagatgc tgaaatgcat tggaattcat gtgtcctggg tgaggaacct gcttcgttct 1320
taccttgtct cctggcaggg cctaagacgc tccctctact gacctgagtg cagacccctc 1380
gacaaagacc tccccatccc tgacaccaat gatcccaggg taagtcgggg ggactgcagc 1440
agacagccct gcccatgctc tctggagtga cagcaggggc agggcagatt tccgtgaggt 1500
cctcatctgt gttctggccc aagggtgccc tcaatcgtcc ctcatgttcc tcaccggaac 1560
tactgacaga tcctgggacc actctgtcta tccaccagat gggggcccct gtgttgacct 1620
gagtcatcct cggagaacaa gttcctcacc tccccgagat ctgaaaacag gaatgagatg 1680
gagctacatc ctgtcacccc cacatggggt gtccaggtat gacccctttg gttctggggt 1740
gagtgtttct atggcctcct gtgggttact cttcatcgtg atggggcctg ccattcctca 1800
acctgaaacc ccctgagatg agcagatagc tcccctttac tgcaagaacc catcaccctg 1860
agggctcttc aacttctttc ctgtggcaca aatgaggttg gcacataggg ctatccttga 1920
tttggtcccc tctgagcaaa aatcagggga cagccagact ctgtggggtc ccttctttct 1980

gagccaaggt atccccaatc ttcatgaatg gggcacacat tggggcctgc agatgctggg 2040
actcctccct ctgctgacct gagtcaccac tgctctgacc acagccctct cctctttgtc 2100
acccagaagg tgcagcaagc atgggctaca ggtggccact gtgcactgga cctcccaggg 2160
caaaggtccc cattgttctg gactcaaagg taccctgagt cctccctctt cttcctctcc 2220
ttgatcctgg cagggttggg ccccacccta tgctgacgtg agtctccatc cctctgattc 2280
ccgaggatga aagaggaggc gcctcagtct cagacagggt tggagtggac cccctatcct 2340
gaggacctgg gttccctgag gcctcccttt actcccagca gggcttgagg agcagagact 2400
cccctgaggc cctgctcatg atactaagcc ctttccctcc ttcagccctg gatgtggacg 2460
tcagaatggg tgtgcctgtc tgtcattcct ggggcttctg t 2501

<210> 24
<211> 2501
<212> DNA
<213> Homo Sapiens

<400> 24

aaaaagcaca cattaagtaa gctcccttac atagcagcaa atatcctacg taaattacta 60
gcatattaaa taaacaggct ctgcccaagg aaataacata cggtttaaca ttaggggaaa 120
aaaaaaaaac ggtttcattg taattcctta catacaaaat ggaggagaaa aaaatttgat 180
tgcgtcaacg ggtacagaaa aagcttttta aagaaattta atatgagttc atggtaaaaa 240
aaaaaaaaaa gtatttaacc tatattcaaa gagaaattgc acttcctaaa atcaacagca 300
aacatactta aatgcaaaac gttcgaataa ttatcattca cgtcaggaac gagaagtaga 360
tgtaggctat tatctctacg attgaaatca ggattgagac acaaagagct ataagtacca 420
aaatgagtaa gtggtatgaa tactggaaga atatgtaatg tttagtgata ataccattat 480
cgatcttgaa aattcaaagg aaaaggtatg tggaatattc aagtgcatcg gccttcagaa 540
cgaggtgtgc aacagattac tacagcaaaa tcaaaatctt cacctcacga aagcaataac 600
taactacaac aattaataga caatatgcca tttgcaatag caacaaaatt catgaattgc 660
ctgaaaagaa gtctaataaa aatgtataag gtctttatag agacaactgt cgaagtatac 720
tgacatattc aggtaaaaaa aaccttgaaa ttacatagag gagtataata tattcattca 780
tggggagcac aacactgtaa aaatgtaaac tcagcttaaa ttatttacaa tttagtgcct 840
tacctgtagc cgccgccatt acaacaaatt tgggctgctg tgctctgctt cccaaatcaa 900
acctggaaaa ttacagaatg gaaacagtaa ataaaaaacc agtatcagca acaaaatcta 960
cgcaacaaaa cagggaaagc ccagggtgag actcaaggct gtgttgagct ggtacttgtg 1020
tttgacactt gcagtgttgg ttggaggggg ttagcagcag ggatgttggg gaggtttgta 1080
gccggcctac acggtagatg acagaatggg tagaataaaa gtttgaaatt ttccacttca 1140
cttctttgca caatctgagg cagcctctga aaacacgatg ccaagagccc taggtaatag 1200
caggacacca ggaaaatttg gttggttaag gcagtatgac tccagagttc tgctaataac 1260
aacctgaaac caccatagtg gcagaggaat tacatttttt aaaaaaaaaa attctttcaa 1320
cagaacacaa agactgaaat aggaggtcac tacaaccgcg ggagctgccg ccccgccctg 1380
cagggagcac ctggcctggg acccgcaggc attctctaca aggggtgcag ctgtgcaaat 1440
gctcacaggt gacagaaaca gagcatctcc tgcccatcac ttcatccaac agccagaggt 1500
gacgaagacg accctcctga gtgaggactg agggtccaca ccgccccccc accccacaca 1560
ccatagaggg accacagaat ccagctcagc ccctcttgtc agccctggta aacgcaggca 1620
gtgatgtcac ccagaccaca ccccttcccc caatgccact tcagggggac tcagagtcag 1680
agacttggtc tgaggggagc agaagcaatc tgcagaggat ggcggtccag gctcagccag 1740
gcatcaactt caggaccctg agggatgacc gaaggccccg cccacccacc cccaactccc 1800
ccgaccccac caggatctac agcctcagga cccccgtccc aatccttacc ccttgcccca 1860
tcaccatctt catgcttacc tccaccccca tccgatcccc atccaggcag aatccagttc 1920
cacccctgcc cggaacccag ggtagtaccg ttgccaggat gtgacgccac tgacttgcgc 1980
attggaggtc agaagaccgc gagattctcg ccctgagcaa cgagcgacgg cctgacgtcg 2040
gcggagggaa gccggcccag gctcggtgag gaggcaaggt aagacgctga gggaggactg 2100
aggcgggcct cacctcagac agagggcctc aaataatcca gtgctgcctc tgctgccggg 2160
cctgggccac cccgcagggg aagacttcca ggctgggtcg ccactacctc accccgccga 2220
cccccgccgc tttagccacg gggaactctg gggacagagc ttaatgtggc cagggcaggg 2280
ctggttagaa gaggtcaggg cccacgctgt ggcaggaatc aaggtcagga ccccgagagg 2340
gaactgaggg cagcctaacc accaccctca ccaccattcc cgtcccccaa caccaacccc 2400
acccccatcc cccattcccc attcccatcc ccacccccac ccctatcctg gcagaatccg 2460
ggctttgccc ctggtatcaa gtcacggaag ctccgggaat g 2501

<210> 25
<211> 2501
<212> DNA
<213> Homo Sapiens

<400> 25

ttcaaattaa catttttagc aaatgaagat taaaaggcac tttaattttc taaaactgac 60
aggaaacata cttgaatgtg aaatgttaga agcattccca ttctcgtcag gagcaagtca 120
ttgatgtatg ctttcactgc taccgttcac agctgcagtg aatgctgaaa tgaaaaagag 180
agctatggat atcaagatga aaaagaggta tgaagattga aaagaaatag atcatatttt 240
tcacaatatg accatcaaga aaattcaaag gaagagacac atggaacatt caaatgaata 300
agagacttca gaagacagca tacaaagacc actgcagcga atacaagaga cttgtcacag 360
atgagcaact tagaacataa aatgaaaaat ataccatccc caacagcatt taaatccaaa 420
atataaagaa gaagaagcct aaccaaaata tgcaagacat ttacagaagc aactgtacaa 480
gtttgctgac agattcagag aaaaaattcc caaataaatg gagtagtata tgatgttcac 540
tgatgaggaa tagaatacag caaaggtgta aatcttactt aaattaaata aacagttaat 600
gcctacctgt agctgccaaa gtgacaatca agcccaggct gctgtgctct actcccaaaa 660
tcaactgtgt aaaaatatag aagggaaaaa gaaaaataaa acaacaatat cagcaacaaa 720
gtctaaacaa caaaacatag aaaaccctgg ggaaaaaact gtgttgaact ggtacttgtg 780
ttctgggcag ttggagaggc agggaggttg aggaggtctg cagccactgc agacgacagg 840
gtgagttgca ggaaaagttt taaaggacca ccccacatca atgtaggatc cgagtctgca 900
cctcaaaaca cgaagccagt agcccagggg taatatcagg acaccaggaa aattggattg 960
attgagacac catggctcca gaattctgct aataacaact agaaacaaag agtaggttag 1020
gactaccttc caaaatacta ttattttaat caaatatata taatgaacta gaaatagaat 1080
agtatgagtg aatcacacat tgtatgagta gatattgttt gataaaataa gtacgtatag 1140
tcgacataca tatatgtata aatatatgta aagatatata cacatacatt catataatta 1200
cacatatata atataaacat atacatacat atgtgtttat gcatataaac ataaatataa 1260
tgcacatata caattggcat agatttaaaa ttcgtttctt actatacgaa tgcgaattaa 1320
attatgaaaa tcactaaagt ggaataggaa ttgttaggta acttcaaaac tcaggacttc 1380
cacaaattta ttgcagctac tcagtttctt cagtgtaaaa ataaatcatt tagtacaaaa 1440
gttttgcaaa atcctcttct agctggggaa gagcccccgt gggaaggtgt gcctcttctc 1500
ccagaggtca ctacaatctt aggtgctgca aacccacagg gagaatctgg cctgggaccc 1560
gcagccattc tctgcaaggg gtgcagctgt gcaaatgctc agaggtgaca gaaacagagt 1620
atctcctaaa agagccaaga gtcaagaaga ggaccctcct gagtgaggac tgagggtcca 1680
cccacaccac atagaggggc cacataattc agctcagacc ctcatgtcag ccctgaaaga 1740
ccccagcagc cttgtccccc caccacaccc ttccccccac tgccacctca ggggactcgg 1800
aatcagaacc ttggtctgag ggaagcagac accatcagca gagtacggag ctccaggctc 1860
tgccagatct caaagtgaga accttgaggg atgactgaac cgctcctccc tcatcccccg 1920
ccccactaga atccggttcc gcccctgttg tccacccagg tgagccctgg gtggacggat 1980
gtgacgccac tgacgtggcc gcggggatca gagagaagcg agggcctggt tctgaggggt 2040
cggcttgaga tcggctgagg gaagcgggct cagggtctgt gaggaggcaa ggtgagaccc 2100
tgagggagga ctgaggaggc ccccacccct catataggac ccaaaataat ctagcgccgc 2160
ccctgctgcc agccctggac caccaggggg cagacttgtc tggctgggcc acccctcgcc 2220
agccctgcgc ttaagcccca ggggaatctg gagtgagagc ttggtgtgac cagagcaggg 2280
ctggttagca gagggcaggg cccaggttct gccaggcatc aagggcagga ccctgaggga 2340
gggctgaggc tccacagagc acatctctgc ccctactgtc agccctggga agccctaggc 2400
atggcggccg ggcatggcgg ccgggcatgg cggccgggca tggcggccgg gctcgctgat 2460
tctgacgtcc gcatccgggg ctgatgcagc aaaggggctt c 2501

<210> 26
<211> 2501
<212> DNA
<213> Homo Sapiens

<400> 26

gcggaaccag attctgccgc gggagagttg gggggtgggg gtggagtttg gggtggggtg 60
gggtggggtg gggtggggtg tagagtggag ctgaggatgg ggctggggaa cctggggctg 120
taggtcatgg ttgggttggg ggggcccagc ctccctcagg gtcctggcct tgatgcccgg 180
cagagcctgg gccctgccct ctcctaacca gccctgccct ggtcacacca agctctcact 240
ccagagtgcc cagcagcttg agcggcggag ggagggggtc ggcccagcct cacaagtcag 300
ccccagatcg gcccccgggt ggtccagggc tgggagcaga gacggcactt ggttctttcg 360
ggtcctctat ctggggtggg gacatcctca gtcttccctc agcacctcac cttgactcct 420
cacagagcct cggccagctt acctccaccg gtatcaagcc gcccctcaga aggaggcctc 480
tcccttctct ctgaccccca gtgcgcacgt cagtgacgtc acatccggcc cccaagcctg 540
gagcttccct gggttgccag taggggctga accgggttct ggcggagtag gggtggggat 600
ggggataggg gccctcagtc atccctcagg gggtcctgac cttgatgcct gggtctcgat 660

ccccattctc tgcccattgg gtctgctcct ctcggaccaa gtctctgact cccagtcaca 720
tgaggtggca gtgaggggag gggtgtggtc ggggtgacaa cattgccagg gtcttccagg 780
gctgacagga agggctgagc tggattctgt ggcccctcta tgtggggagg ttggaccttc 840
agtcccctct caggagggtt ctcctcctgg ctcctggctc tttgatgaag tgatgtgtgg 900
gagatgatct gtttatgtca cctttgagca tttgcccagc tgcacgcgtt gcagagaatg 960

gctgcgggtc ccaggctaga tgctccctgg ggggctgcag cacctgtgat tgtaggacct 1020
gtgagagaag atgcacacca tcccatgggg gctcctcccc agccagagct agactttgca 1080
aaccttttgt cctaaaagat ttatttttac acggaagagg ctgagcagca gcaagagatt 1140
tggagaagcc atgtgttttg aaggtatcga acacagcaga gtccagatta gcgattttca 1200
tattttaatt ctcattcaca tggtaataaa ttttacactg tatgtgtaca ttatatatgg 1260
tattgtacat tttgtttata tctacgttta tatacatacc tgttttagac attatataca 1320
tctatgctta tatgtataca tacacgcata tatatcttta tatcttatat ttacttacat 1380
gtatatatct ttagctacat attcgtttgt atttctagac tttatactat atatatgcac 1440
acatatttta ttaatatcta ctcatagtat gtggccttca cacatactta ctattctatt 1500
cctagttcat tctttatatt ccattaaaat aataattttt tggaaggctg gtcttctcct 1560
actaagtttg tttcaggtgg ttgttagcag aactctggag ccatagtgtc tcaatcaact 1620
caaatttcct ggtgtcctga tattactcca gggctactgg catcagattt tgtggtgctg 1680
cctcagattg cgcgatgatg tggggtggaa tgttaaacac tttctcctat ccatcctgta 1740
atctgccatc tatgcagggt acagacctcc tcgatttccc tgctgctccc tggacccaaa 1800
acacaagtac tagtttagca cagtcttcag tcatccccag ggctttctct gttttgatgg 1860
gttttttttt ttaatctttt ccctcctata ttttttccgg ggttgatttg gggagcagag 1920
cacagcaacc tgagcttggt tgtcactttg gcaatgacag ttaaggcact cactctgtat 1980
ttaatttaag tagcatttac atctttgcca tattctgttc cccatcaagg aatatggtat 2040
acagatccat ttacttgaga ttatttcctg agtctatcag caactttgca cagttgcttc 2100
cataaaggtc ttgtcaggct cttcttattt acattttgga ttttaatgct gctgtgaatg 2160
gtgcattttc tattttctat tttatatact aagttgcttt tgtgtggcaa ggctcttgat 2220
tttgctttaa tgaccttctg tatgatgttt tctgaagtct tttattcatt tgaatattca 2280
atgtgtctat tcctttgaat tttctatgta gataatcata tagttaacaa atactctatt 2340
tctttgcagt cttcatatct cattttcatc ttgacatcca tagctctggc tctttttcat 2400
tttgatattc actgcagatg taggtggtag tggtgacagc atatgtcaac gacttgtcac 2460
tgccgtgaat ggcagagctt ctgacatttc acatttatgt a 2501

<210> 27
<211> 2501
<212> DNA
<213> Homo Sapiens

<400> 27

ttcctcctat ctgatctcag ggaggtgcag gccttttaaa tggagaggtc ttcaggtcaa 60
aaaagagggg agccctagga tctgctagga gccaaatcaa ggaaaccgag tgagaactga 120
ggggactgct cacctctgaa tagagagagc aacagagtcc acccccctgc ataactctta 180
aattgccagg gcaacatgta aggctgagga ctcaccatca cttttttttt ttaataattg 240
accttctttt ttagaatagt tttacgttca cagcaaaact gagcagaaag gagagtctca 300
caggcatcct gtcctcccac agacacagcc tccttcatat caacctccca agccagggtg 360
atacatttgt tacaattgag aatacatgga cacatcatta tcacccaatg tccgtagttt 420
aggattcatt cctgttgctg cacattctgt ggttttaata tttaaaaatg tataatgaca 480
tatgtttacc accatagaat catccagaat tgttttactg ccttaaaagt tctctgtgcc 540
tgatctattt atctctcact ctccccaacc tttggaaacc actgaacttt gtactgtttc 600
catacttttg ccttttccag aatgtcatat agatggaatt atgcaatgta tagacttttc 660
aaattggctt ctttcacttt gtaatatgta tctaagtttc atccatgtct ctttgtggct 720
tgatagttca ttccctttta gtgctgaata atattttatt ctgttaattt atccactctc 780
cttctgaagg acatattgct tgcttcccag tttgggcaat tatgaataaa gctactataa 840
acatttgtgt gcaggttttt tttgtggaca taccgttttc aactcatctg gctaaataac 900
aaggagcgca cttgctagat catatgatga aagtatgcat agctttgtaa gaaacttaca 960
aactgtcttc aaaagtggct gtatcatttt tcatgtccat cagcaatgac agtgtgctcc 1020
tgttgctcca catcctcatc agtatttggg gttatcaggg tttttgattt tgaccattct 1080
tataggtatg tagtggtatc acattattgt tttaatttgc aagtctctaa tgatatgtga 1140
tgttgaccat catttcatgt acgtatttgc cttccatata tattatttgt tgaggtgcct 1200
gtttagatct tttgtccatt tttaataggg ttggtcagtt cttattgttc agttttaaga 1260
catttttgta tgttttggtt aacagtccct tatcagatat gtcttttgaa aataattttt 1320
tcccaacctg ggagttatct tattctcttt gtggtatctt tagcagagca gaagttttaa 1380
tttagcgaag tccagattat caattatttt ctttcataga tgcccatcac attttatacc 1440

agaactagaa aagatgaaat tcttggtctg aagttgcagt tatcagtcag cagaagagac 1500
agtccacaac cctgcttgga gtccagatga ggatcctgag tgcaaacttg ggacctaaag 1560
agcccaggac agagagagca ctaaatgctt ctaggcaggg gtggtgggtt gaggggcccc 1620
tagacttccc tcatctgggt cccagaaaac taaagagtca atttcacaac accaatagag 1680
ggaggctcag gccctgccaa gagctgacat gacaattcta aaggtaatca gagtggatcc 1740
tctccaagcc agaacacaga aagccccact gcgagccttg ttgtcaccca gtcagcccca 1800
ggcagggttg gcaagctgca gcctaaggca cattgtaact tcctcagctg gcttctcagg 1860
ggacagaatg actaagaaca atagcccagt gaatacttag agcagtgttc tcaaggaatc 1920
ctgcagaggc ggcttctgaa aagccaaggt agtatctgcc tgctgaaggt gttctcagga 1980
tttcatttgc tcttctccag gaaccacatc acctgccctt ctgcctacac tcctgcctgc 2040
tgtgcctaac cacagccatc atgcctcggg gtcagaagag taagctccgt gcccgtgaga 2100
aacgccagcg gacccgtggt cagacccagg atctcaaggt tggtcagcct actgcagcag 2160
agaaagaaga gtctccttcc tcttcctcat ctgttttgag ggatactgcc tccagctccc 2220
ttgcttttgg cattccccag gagcctcaga gagagccacc caccacctct gctgctgcag 2280
ctatgtcatg cactggatct gataaaggcg acgagagcca agatgaggaa aatgcaagtt 2340
cctcccaggc ctcaacatcc actgagagat cactcaaaga ttctctaacc aggaagacga 2400
agatgttagt gcagttcctg ctgtacaagt ataaaatgaa agagcccact acaaaggcag 2460
aaatgctgaa gatcatcagc aaaaagtaca aggagcactt c 2501

<210> 28
<211> 2501
<212> DNA
<213> Homo Sapiens

<400> 28

ccctatctca catcactccc ggaacacggc tccttctctg caaggtttgc agtagaaaaa 60
ctggtcatgt ttgcagggat gtgacatttc ccagaaagcc tttaatgaaa gatgatcccc 120
agttcagagg acatgggaaa ccaatatctc agaatatatg aggttatgca tagactgact 180
ctcatctgct ctcagaccta gagagctcga gacagaactg tcaggctgag catccactca 240
tttaatcttc agacctctca tggacatggc ctaagaaggt caacttcatg tcagtagatg 300
cagttcagcc tctgatagat atgaacgtga ggaccctgaa tgaagatgat ggaaggaccc 360
agccagaact gtggggtccc aaaaagtcca gctcaggctg tcagcccttc aaagtccaga 420
acagccctgg ctaaatgtag ctcaccacaa cttccagttc agaggtcggc agagggtgag 480
taccttgatc tgatgggaac agcctcaggt cagtggagga tggagtgccc caagactggc 540
caggtgtcat ggtgaggacc ctgaagagaa ataagagaac tgttatagga ccaacgagtt 600
tctgtgtcag ctgcaaagta acagacccac tataataaaa cagcaaagtt tgcagcaaag 660
agtttaatga ttgcagggtg ccaagtgagg agatgagaag agactctcaa attcatctcc 720
ccaaggagtt ctgggctggg atttgcaagg agatcatgga gggcgagggg ctggagaact 780
gaggttgttg attcgttggg gtaaagggga tgaaatcatc agaatgtgga aactgcgttt 840
tttttttttt agtcagctcc tgtggggtct tttggaccag ctgatgtcag tagggtcctt 900
tagagcagct ggcatcagtg gggtccttca gaccagctga gtcattagaa tcatcagtat 960
gcaggacctt aaggaatatc tcaaagaaaa aaagtttcat tatgttaaaa ttgttatcta 1020
tagagcaatt aagggcaact ataatctagt aacaggatct acatgattct gggataatag 1080
gcaccaaact actatgagga agcaggtcag agagcagctg acttcatgat taatgctgaa 1140
tgttctgcaa gcttggcgta tcttcattta tctcccttcc ctcttccctg attaatttta 1200
tacagttcat aggggcagtt tcaaaaccac ccaccccaga acagaggagc ccacaaattc 1260
caccaatgct gtcacccttg gggagcccag aagagaatta tgaggtggag aaatcccatc 1320
aatatgcctc gtgtgtttca cagaggtgaa gaccttggac tagtggaaat ggccctaggt 1380
caaagagaca aaacccacgc cctaacagga atcaaggtga ggatcctaag tgttacgagg 1440
gggctctccc ccttgcaaca ccagcagagg gggccaaaca gagcccctcg ccattgttag 1500
caccgagagg ccccaaaata agtatctctg aatccccttc attccacctg tatagtctcg 1560
gggaaggaag ggcctttgtc tgaagagggt gaccccagtt gtgcagagag agagtcttgg 1620
ctttcacggg aatcaaggtg aggactctga gggcggatga gaagacctct ccccaaaaaa 1680
ggcacattca cagagccctg ccgctgctgt caggcctgtg aggcccaggc aggggtggcc 1740
tgtttggcac gcttagattt ccacagtggg ggctgaggga ggtgggggta ttgtttggag 1800
gctggcggat ttgggtcagc acgcatattc gtcccaggct gctagatact gaggtgagga 1860
cccctagtgg agacgaaggg accagcaacg ctagaacagt gacgtccggt agcgtccagc 1920
cgtcagccct cagacgccac gggctgccgg atgtgagtca tcctgacttc cgctttgaaa 1980
aaaaagaccc gagcggatgt ggctcatcct gacttccgct ttggaggcga ggacccgagc 2040
gagtgtaggg ggtgcggcgt ctggtcagcc aggggtgaat tctcaggact ggtcggcagt 2100
caaggtgagg accctgagtg taaactgaag agaccacccc cacctgtaac aaagagggcc 2160
ccactaagtc ccgcttctgc atttggtcct gagaggctcc ggtaaagccg tccggcaatg 2220
ttccacctgg aaagttccag ggcaggggaa gggtgggggg aggggcagtc gcgggggaag 2280

gaggtttgga cgcagggaat aggcctcatt ctgcacgtag ggtgggtgta ggccctaact 2340
gaaatcaatt tgagggccct aaatgtggac tgaagagaac atctcctacc cttaacaaag 2400
gtggcctcac taagtctcgt ccctgaagac ggctctggga ggccccagca aagctgtgcc 2460
tggaaagtct cagggagggg agggccttga acctaggaag c 2501

<210> 29
<211> 2501
<212> DNA
<213> Homo Sapiens

<400> 29
   atcattaatt gagcatcagc tctttggaag tcttcatgtt agtactggga atgttttccc 60
   caaaacaaat caatttcttg cccatttaat tttagagtct gggagcagct gtcataaaaa 120
   aaaaaaagat gatgaggtgc tctctaaaac ttaaagaaca agtacaaagg aggaatgaga 180
   aaaagaggag gtttgagatg agagcaatca agtgtaaatg ccctgaggca aggcagtttg 240
   gagtgttagg aaacctcaag tcccccattg ggaggtaatt ttaagggaaa ctacatggtg 300
   ggctggatga gactgtggga ggggccaggc cctcagatgg tgcctctcag aggtgtgaga 360
   caaagcctgg aatgggaagt agttcttaac agttattttg tgctcacgga taaaccagag 420
   agaatctgca cctggggcag gaatggaatg tgccctgtgc tcttgtccca gtgcagcaga 480
   acatagtcac agtgcacaaa ctaggtgctt tatacagttg gtgcatttca gtgttgagag 540
   actaagcccg gaatgggaaa ctatccctaa catttacttt cctgttgtgg gaaaaccaga 600
   gagaacccct acctaggaca aaagtgaaaa gtgttctatg tccctatcct agcacagtct 660
   aatacagtgc acaacctagg tgttctatgt acatcatctc tagtgagttt ctgagaaata 720
   agggagatga cagcttcagg ggaggtaaga tgcccagaag ccaccgtgct ggcactcttt 780
   gtcctgggtt ggagaatcaa gagcccgctc tattaaaagg actttcaaca ggggtgcagc 840
   caggcatggt ggttcatgcc tgtaatccca gcactttggg aggcgaggca ggtggatctc 900
   ttgagatcag gagttcaaga ttagtctggc caacgtggtg aaaccccgtc tctaccaaaa 960
   atacaaaaat tagttgggcg tggtggtgga catctgtaat cccagctact agggaggctg 1020
   aggcacgaga atcacttgag ccctgaggca caggttgcaa tgagccgaga tagtgcctct 1080
   gcactccagc ctgggtgaca gagtgagact ccatctcaaa aaaaaaaaat aaataaataa 1140
   taaaaatcaa aacaggagtg gttaacatag aagggtgcct aggagttaga aaaaaatatt 1200
   ggtcattgac aaaaattttg agacttgagt tgtatccaat tggagaaggc tcttccacaa 1260
   acactttata aattatataa ttttccttgc taagcagcat tttgtttgat tataatttct 1320
   ttgtttggag tttggtaata tgctcaagtt cacagggtga gtaagtgaca gggctgggac 1380
   tagactcctg ttgtgaattc ttctagagcc cacactcttc attctgttcc tctctgcctg 1440
   gagcagactt tgtgttcttt aattcacttt tcttctcaat acttccaaat atatctcagg 1500
   ggttaaaaag aaggattctg tgcccaaaga attggattgg aatacatagc caaagcaata 1560
   aagaatatca aaataaatga aagctacaaa taaagaggag aaagagatat tattcagtga 1620
   caatatgatc atctacatat gcacagtcag ataacctcaa aatcagggac cacacaatca 1680
   tcttgaacag ctccttcccc ttaagaagta aatcaattaa aacagaagtc acataagagg 1740
   ctagtgctgg ctattgaaaa gagtaaatca gcatcatttt tctctgacaa cccaccagct 1800
   accttggact ccctgctttg tgttataatt tctttacctc acattagccc tggggcacag 1860
   acttgcaagg tttgcagtag gaacgctgcc caagagtttg cagggatgtg gcatttccca 1920
   ggaagccttt attcagagag gcaagagaaa ataaggagga cacccataaa taaatactgg 1980
   aaagacaacc acaccaaaag ataacggggc aatctaccca aacctcttct cagcctagaa 2040
   tgccccaggc agtgcagtca gactgagcat cctctctttt acccttcaga gttctcagtg 2100
   aagtgagagt tttgacctaa ggtgacctca gttaactaga gggaggatcc agggtctggc 2160
   agatatcagg gaggaccttg aatgaagaac aggaggagcc agaacagtga tgtcccatag 2220
   aaacccgact ccgctgtctg ccctcacagg acccaaacat ccctggccta atgtggctca 2280
   tcaaaacttc agcctagatc tcggaggacc ttgatctgag caggattcat ggcatggaat 2340
   gtggagtccc aggactggcc agcagcgaag tgaggttctt gagtgagtaa aaaagggaaa 2400
   actaaaccca caaataaggg gacttctcgg agcccaatcc atatttttaa ccctggaaag 2460
   ccctaggcag agctataaaa ctggcttgcc tctcatttca g 2501

<210> 30
<211> 2501
<212> DNA
<213> Homo Sapiens

<400> 30

aaaggagaag ccttaaaaaa ccctcaatac cagtgtatga tagtaacttt tagcaaactt 60
acattaggag agaatttcca tcttgtaaaa tctataattg acatactaaa tgtgaaacat 120

tacaaacatc ccgaatagag tccagaagaa tatacacctg tatagtataa accctactgc 180
tgacaactgg atgaaaatac atctatagtt cacaactata ataataatac cgagtaaatt 240
agaggtaaca ataacaaaag gagataattg tgataatata atctcctaat ttaaaaataa 300
aacaacgtaa ttggtgcaaa aatatttaaa tgtataagga aataaagaaa acgtgtgtac 360
aaaagatcac catagttaag tcaggagtat agtcacacaa aatcaataac taattataat 420
atgtaatgtg aaatatgcct ttcacaacag caacaatatc caaaatatat ctaacagaaa 480
accaaataga aatgtacatg ttcattaagg agacagctat aaaaatttac tgaaagactt 540
aggaaaaata aatctccaat aaagcagtgt actatgttta tttactgtgg aggagatatt 600
gtaaagttat tgattctcct taaatttttt acagattcat tgccttagcc atagctgtga 660
agatgacaac caaacccggg tggttctttc ccatgaccaa actcaaccat agaaaagtta 720
aaggaagaat ttttttttaa aaaaaaagca gtattaacaa cagcaaaaca acaaaaatgc 780
gaaccttgag agatggtcag tttgtattga acaagaatgg gtgtgtgggc aggtaggggc 840
atgagctgaa gtagagacag gtctgtggcc tacaacagac gcagataacg gaattggcta 900
gaggaaaagc tttaaaactt tggtctttgc ttctttcact gtgccattgg acaatgcatt 960
tttttttctg cttcactaaa ggaaattcag tcagaaactt ggaccctatg tgccagttcc 1020
atggggtaat attgccacag caaaaacatc tgggtaggct gaagaagtat gaatccagac 1080
actgtgctaa taactacctg aaacttcttt agtggaagtg gactggcctt taaattcgta 1140
ttattttaat ataatataaa gaatggacta aattaggata gcaaatattg cagtgagtca 1200
cacaagataa tggtagaaat tcattcatga aggtgttgaa tacagacact gatgtcagcc 1260
aagagagaag tcccagacat agctatcagg ctaagaagtc ttctcacttt ctccttatga 1320
attttacagg atctagtcag aggaagggga tgaatttaca tcagaaaatg gagaaaactg 1380
agaccctgcc agcagtcaat gtgaggaccc tgagtgagga ctgaggggct ccacatatca 1440
gaagagacaa gagtgccccc caaatctccc ttcattcccc gatcttgcat cacaacttaa 1500
tagcagcagc caggtttagg aggccccagg cagtagtggc caggtgaggt gtaactgaat 1560
tgcacatgag gagtctcagg aagttgaagt ctagtctgag agggtgtcct caagtcagta 1620
gaggggacat ggcaagaatg aagcgacgac actgagtgag ggctgagggc aaagtccatt 1680
ccaaaaaggg ggtctcacag agctccaccc ctactgtcag tcttggaagg tcccggcatg 1740
gctcccgagc tcaggctgca ccttacttcc aactctggac tctcagggaa tgagagcctc 1800
tatctccggt tgactgattc agctcaacag atagagaagt ccaagggtcc gcgaagagcc 1860
cctgaaagaa ctttacgtga gttccctgga atctgctgca cccacagaac acaaggggga 1920
gtaacagagc cctgcagcta ctgttacgca gggagcgcca ggtgggcgtg gccggaagtg 1980
gttcctcttg acttccacct aataaagggg tctgagccgg tcgcctgagc ctgaaaagtg 2040
ctgtcacgtc agcggaagga ggcgtcccag atcttctcag ctgtcttggt gggtatcctg 2100
acctagaacg gggggcattc cccgacaact tcaaccccct tcccccgaag aaaggcggct 2160
ccacagagcc ctgccccaac ctgccccgac ctggtccctg aggagtcccc aaacatggct 2220
gttaggccca aagtccattc atttttttcc gtcgtgttga agaacatgaa ggcctcggtc 2280
caaggcttgt ggacccaggt cagtagaggg agaaatctca ggccctacat agttaagagg 2340
accctcctta gaattgaggg aggggattcc atgtcccgta acgggttgca ccgaactctg 2400
cccttgacgt tagcctgggg gtccgggcag gtctctagag actgaggtgc attctcactt 2460
tgtccctgtg agagtcagag aggtaggccc ctggttgtaa g 2501

<210> 31
<211> 2501
<212> DNA
<213> Homo Sapiens

<400> 31

cccatcaggg tccccagatt tacaaatttc tgacttccat tttgggagtc agccagaggg 60
gagattttct ctgaggaggg cagcttcagt taaacagagg gagtagcccc agggctggcc 120
actcaacaag aagagaactc tgtcgaagga cagtgacctc cgaacacggg ggaggcacag 180
gctgcaccct gccactccct gctgtcagcc ctggaaaaag gtggggtgtg agtgtcaagg 240
actggggagg tgggagggaa gaggtcagca gggcttaggg agtgagcaga aaggtttgga 300
gcacatcctg gaggtgggaa ggaagggatg gaaaggaggc tgacaagcgg gcggcggggg 360
gggggcgcct tcctcctctg agaggaggcc ccgccccacg ctatccgggc tgtcagccct 420
gatcaaccct gacagagttc ccggatgtgc tttgccggat gtgctttccc ggcggccatc 480
ttgggagtct gaaggacctg aggcattttg tgacgaggat cgtctcaggt cagcggaggg 540
aggagactta tagacctatc cagtcttcaa ggtgagggcc ccgagggaga agtgagggac 600
aaccccacca cagagagaag cagccctggc ctgccctatc cctgcggtgg cacttgtagg 660
tggcggaaac gaagatggta gccgagggct gagggacgtg tcctcacggc agaagaaggg 720
aggaaatgcc ggccctctag agaataaata ggaaggcatt gaggaggacc gggggaaccc 780
ccacctcaga ggacagattc ccagagattc ccaccctgct cctcaaatca gcccccatag 840
agctccccag gtggctgagg ctgagcggcc accctctcac tgctgggtga ggggtgtagg 900
ggagggggag gaggaggcct tgttctgggg gtcccatggc aagtcagcat ggggagctgc 960

ctccagttgg cagagggaag attcccaggc cctgctggga acaagagtga ggactgaggg 1020
gtcacatgtg cgtcagaaca gacgtgaggc cacagggacc gcccccgtgg tagagtgctg 1080
ggaggtggct gccacctcac tacctcccac tgctctcagt gatgtggagt tttcctgagg 1140
tttttcctca ggccagcaga gtggtggggg cctggccctg tctgagaaaa tgtgacaatg 1200
ctaattgaat tctgaggggg tcacgaacct cagaattgtg ggactctggg agtctggcca 1260
gccccagctg ccgtcttagc tggaggggct ccctcccttc ctcttgcagg tgctccagaa 1320
agcaggagtt gaagacctgg gtgtgaggga cacatacatc ctaaaagcac cacagcagag 1380
gaggcccagg cagtgccagg agtcaaggtg agtgcacgac ctgactgtgt accaaggccc 1440
ctacccccag aaacagggca gacctggcag cacccggcct gtagccaccc actgtcattc 1500
ctggtgcctc aggctctgcc tgccagctgt gccccgaggt gctttctcgc gtccttctac 1560
aggttcccag aagacaaacc ccctaggaag acaggcgacc tgtgaggccc tagagcacca 1620
ccttaagaga agaagagctg taagccggcc tttgtcagag ccatcatggg tgagtttctc 1680
agctgaggcc actggcactg tccctctctc cctcagtcct gtgggatccc atcataccta 1740
ttcgtgttca cacgtttacc tgctgctcct gaacaatatt catcatgcct ctctttctaa 1800
accttccacg ccccagcttt gagcaaggct tccagaaggc aattttcata ctggagttgg 1860
tagatgcaga ggatccccca gatgaggaag aggaggaagc ttcctccatt ttctcttcct 1920
ctttccactt tttattcccc tcgtcctcct cctgtttttc tttctcatcc tcatcctcct 1980
ctctgcttct gcgttctcca ggggacaagg atatgcctac tgctgggatg ccgagtcttc 2040
tccagagttc ctctgagagt cctcagagtt gtcctgaggg ggaggactcc cagtctcctc 2100
tccagattcc ccagagttct cctgagagcg acgacaccct gtatcctctc cagagtcctc 2160
agagtcgttc tgagggggag gactcctcgg atcctctcca gagacctcct gaggggaagg 2220
actcccagtc tcctctccag attccccaga gttctcctga gggcgacgac acccagtctc 2280
ctctccagaa ttctcagagt tctcctgagg ggaaggactc cctgtctcct ctagagattt 2340
ctcagagccc tcctgagggt gaggatgtcc agtctcctct gcagaatcct gcgagttcct 2400
tcttctcctc tgctttattg agtattttcc agagttcccc tgagagtact caaagtcctt 2460
ttgagggttt tccccagtct gttctccaga ttcctgtgag c 2501

<210> 32
<211> 2501
<212> DNA
<213> Homo Sapiens

<400> 32

ccccagcagg gcctgggaat cttccctctg ccaaccagag gcagctcccc gtgctgactt 60
gccatgggac cctcagacca aggcctccgc ctcccctacg ccactcaccc aggaataaga 120
tggctgccac cccgcctgag ctgactgggg agctctacga gggctgatac ttgaggagca 180
gggtgggaat ctctgggaat ctgccctctg aggtgggggt tcccccagcc ctcctcagtg 240
tcttcctatt tattccctag agggccggca cctcctccct tcttctgggg ccatttccct 300
gtctgggacc taccagtctc acggcagcgc agtacaggcc ggggtttctt ctctctatgg 360
tgggaggtcc ctcagttctt cctcagggcc ctcaccctga ggactggcca ggtctaagac 420
tcttcccccc gctaacttga ttcgcgcctc ctcacaaatg ccttcgatcc gtcagattcc 480
caagatggcc gtcgggaaag cacatccggg aaccctgccg tggttgtcca gggctgacgc 540
agcgtcaatg tggggcgggg cctcctctct gaggaggaac caccccccac ctctctgctt 600
tttttccatc ccttcctccc cacctcccag ctgccctcca acccttcctg ctcactccct 660
aagccttgcg gacctcttca ctcccacctc cccagtcctt gacaccccac tctccttttt 720
ccaggggtgt cagcagggag tggcttggtg cagtctgcgc ctcccctccg tgttgggagg 780
tcaccgtcct tccacatctc tcttgttttt tggccagttc tgggacttct ttctctgctt 840
aactgaagct gccctcctca gagacaacag cctccccttc tgctgactct caaaatggaa 900
gtcagaaaat tgtacatctg gggaccctga tggggtcctc cagagctggc agcagggaca 960
ggggaggggc actaccagtc atgggtgttc ccttccagcc ttcctgagag gctgtgcctg 1020
gactcctgat actgcctggg atgcctgacc ctgtgcatct gagtctttca cctccgaata 1080
agcccctccc atccctgaga cctgcaaggt gcaagagagg gagaatcaca tccatccaca 1140
gtgggatagg gcctcctagc cctaaggcag gcctccactg ccctctgaaa tggggtggga 1200
atgcccctct gtctttctca aggtatgact cctaaaagag cctatactcc ttccttctcc 1260
tgtccagaca gcaacatttg ttccttcgac ctctaaattc cattccaagt ttttccactt 1320
tactgctgtg agtgaaatta gtgtcatctc tcccataccc tgtgttaaag gcttccttat 1380
tgttgcttct tttcctatcc tagtccctga aaaaaaccac tgtcttttca gcaacccgtg 1440
accctttaaa aaaccaatac gtgattaaag ttttaaatac acacatcaga tttaactaga 1500
atgtgaagtt atatgtattt tggaaaatgc atggactccc atatctacca accaagtatc 1560
atactgacca gttccacctg cctaaacttc tcctgtgcat cccttttgag gtcaatcacc 1620
gcacttctac aaagctccta gcaactgtct tctgttttca gttcctatac ttttaacttt 1680
tccaatttgt catatgatta gcttcataaa atactgactt aaaagtgtgc ctgtactatt 1740
ttttatcaca actagcaagg aatgagaatt tctgttattc tatatcctct cttacattga 1800

gtattttaga ttttcaggtt ttagccattc tcataagcat gtattggaac cttctttggt 1860
ttttgtttgc ctcttcttaa tgacatatga cattggtcat ctttttatat gctttttttc 1920
cgtttgctat attccctttc gtaaagtgtc tattcaaatc atttgcatcg ttttatagat 1980
gaataaactt catttttaga gcattgctaa ttcaaaaata caaagagaaa tcttcatcag 2040
aattagagtt cctacataat ctacaccacc cgccctgccc tgattgctgc acacacacac 2100
aaagtctatc tttttattta cattttgtat tattatgaca tgtttgctac aactgatgga 2160
actgtattga tacctcacac ttaagtccat ggtttacatc aggactcact cttaacgatg 2220
gggatattat acatttcgat aagggtgtaa tggcaagtat tcatcattgt attgtcccac 2280
agaattgttt caccatctta gagctcctgt gtgttccacc taatgaatca attccccttg 2340
ccccaacccc agccctcagc ccctggaaac cagtaatatt tttagtgtat tcatcattgc 2400
atgttccaga atgtggtatg ctaggaagcc tttttagatt ttcttctttc atttagcaat 2460
atgctttcat gattcctcca ggtctatccc actaacaatg a 2501

<210> 33
<211> 2501
<212> DNA
<213> Homo Sapiens

<400> 33

agtgggatgc ggggtgcagg ggccggttcc aggtcccctg agctcaagtc tcgccccacc 60
ccgccccgca agtctagaaa agatgcccct gcccttggct gggtaatctc tggatttacc 120
tactgtctgt aaccccccgg gctgaagaga ccaccccccg ggagtcaatt aacttactct 180
ctggagcgcg cgttcctccc tgctcccagg agtcggtttc ccaaaacttt ctgaggcccg 240
cgcatgctcc cctcgactcc ccgtgtttcc actctccaca gaaatccacg cattcacgcc 300
cctcccctcc cccgagcctg cagaggactc cgccctgctt tccctacatt cagggctgct 360
ccttttgtcg ccaatacaaa cctgttgaca ggtcacgcct gggaagcggg tggggtgtcc 420
cggagcggtg ctgaggcgct gcaggcccgg cttctggctg cgggggagct gtaccctgaa 480
gcctcgccgg aactcgcgtc tggggccagc agggggcact agagtcaaca gggactgtat 540
gcaaaaccca cttcctccca ggccctctag ggggatggtc aggcttctgc agagatgggg 600
aggatccttt cctggtaagg ggagagggac gggctaggag ccaagcggaa ggaccccgtg 660
ttcaaggccc ttcaagggac gggacaggcg aggaatctct ttgagtcttt tgaattgttt 720
tatcagtcag gcctgggaag tactccgcct ccacaaactc tgttttcctg aaaggggtcg 780
ggggggggac cccaacatct cctgccacag gctatgatgg gcatggtggc taagagcaac 840
aggccgcgtg gtgaggctgg ctttttgtgg ctctgccctg ccattccccg aggcatcccc 900
atgccagtga acagagctgc gggttcccag ctgcacagat gaatgcacga acaaaacata 960
ggtccataca aaggaagtca ttgcattgat ccagattcga cctgggattg tcgcataaaa 1020
cagggctgat aaaaaccgtc gtgttttcac cctggaaaca gagactcagc ctccccctct 1080
ttgcttgagg cattcagcaa aggtttgtcc ttgagtggct gtggatgctc aaggacttac 1140
gatttctgac tttataaagt ttagggtgaa aacaaaaaac aaaataagga gaccattgtt 1200
ttaaagtggc cgttgtagct aattatctca agaaagcaaa caggctggta ctgaaagtta 1260
cacaggcggg cggctccctg ggagggttgc ccagctctcc ttttctgaga cctgagcagg 1320
gagcagccag aggagcttga agggatggtg ttcagggcat gggaacaggc tgcacacatc 1380
ttcacggagt acggtgcttg tccagtttga gaagggaaag aaacgggagc agagggctga 1440
tgagaaggca gcagatggat agtgcgggat tgaaaagggg atccatttga agactggcca 1500
ccattattta ttttcatcat ctgattgctg ttgctccatt gagatcgatg cgtggagaac 1560
aagttgggga gtggaacaaa tggcagggag gggagagaag tgagtgaaca tgaccctggt 1620
ccagcctcat aggacagagc gtggtcatga agtccacaga ggaggacagg tagggaggaa 1680
tcatggcatg gccaggttgt agggcaagag gcagagagga gagaggattg tggaggatgc 1740
ctgcggagtg ccgactgtca gtcgaggctg gtcctttatt tatttatttt ttttttttga 1800
gacggagtct cgctcttgcc caggctggag tgcagtggcg cgatctcggc tcactgcaag 1860
ctccgcctcc cgggttcatg ccattctctc gcctcagcct cccaagtact gggactacag 1920
gtgcccgcta ccatgcctgg ctaatttttt tgtttttaat agagacaggg tttcacgtgt 1980

tagccaggat ggtcttgatc tcctgaactc atgatccgcc cacctcggcc tcccaaagtg 2040
ctgggattgc aggcatcagc cactgtgcct ggccaaggct ggtcctttct tttatggcga 2100
tgggtgtttg aaagttgcac ataccaaaat aaactcacta tttgttgacc caaagtaaaa 2160
ccctgagggt atgaaaggga agggctcatg cttacatggc tgagataaaa gctgtctcag 2220
gaagctgggt gtggtggctc acccctgtaa tcccagcact ttgggaggcc aaggttggag 2280
gatcccctga ggccaggaat tgaagactag cctgagtgat atagcaagac cccatctata 2340
tttttaaaaa gggttggctg tctcgggact tttcgaaata gccctgcaag acattcctgt 2400
tttggaatca caacaattta gataagatgc ttttgaaaga acacctgcct aggcacagcg 2460
tctccaccaa tgaactgatg ccaactgtgg ttttgagtct t 2501

<210> 34
<211> 2501
<212> DNA
<213> Homo Sapiens

<400> 34

tgctactaga gtgcactcca cgtgcacgtt cctccctaag agccactgca aggatcctgg 60
gcctgctgcg tggcccaaga atccacgttg cttctgaggc tagatctagc cacagcgatt 120
agggtttctt cgccatggcc ttggtggtgg ctggccctaa actggtcaca gtctgaagac 180
ccac[?]ctgaa agggactaag gttagagaca gcgcccctcc cctcccaccc ttagctcagc 240
atccccacta agcacacccc ttggggacca caaacgccct ctttcccgaa gaccccagta 300
gtgacctgtg ggattctggc tgggtgtccg tgacttaccc tcaaagagaa cctgcacgcc 360
tggggtccca ggatggctgg cttgcccctc ccgtccgaag atgacctcgt ccttctcttt 420
cagtcccggt gggtcttcgg actgccgccg ccgcgctttc ctcggcctgg gcacgctggg 480
tcggcgtggt cccggctcgc gtccctgttg ggcggccgcg gcgcgggtgg cggcgggaca 540
agtcgtgagc gcgcgctggg cgcgcgcccg gccggggcag tcgcggggga ggagcgcccg 600
cccgccgctc gcgcgcccaa cggaccaggc tggggccgtg aggtaactgt tgcagccagc 660
ggaggtggga ggcgacactg agtctccagt cccgagaggt gcccgaggga aaaggaggcg 720
gcagctaaac tggtcctgga gagaagcccc ttccgcccct ctcctcagcc agcatgtccc 780
ggactccgcc gctcctcagt ccgcgcggtg gggaccccgg gccgtggcgg ccggcgcagc 840
cctgacgggt tgcgaaccag gggcgccccc gaacgcgggg gttggggtct gggagcgcga 900
gcggccgcta cggtacgagc ggggtgtgct gagtcccgtg gccacccccg gccccagcca 960
tgaggaggga cgagcgagac gccaaagcca tgcggtccct gcagccgccg gatggggccg 1020
gctcgccccc cgagagtctg aggaacggct acgtgaagag ctgcgtgagc cccttgcggc 1080
aggaccctcc gcgcggcttc ttcttccacc tctgccgctt ctgcaacgtg gagctgcggc 1140
cgccgccggc ctctccccag cagccgcggc gctgctcccc cttctgccgg gcgcgcctct 1200
cgctgggcgc cctggctgcc tttgtcctcg ccctgctgct gggcgcggaa cccgagagct 1260
gggctgccgg ggccgcctgg ctgcggacgc tgctgagcgt gtgttcgcac agcttgagcc 1320
ccctcttcag catcgcctgt gccttcttct tcctcacctg cttcctcacc cggaccaagc 1380
ggggacccgg cccgggccgg agctgcggct cctggtggct gctggcgctg cccgcctgct 1440
gttacctggg ggacttcttg gtgtggcagt ggtggtcttg gccttggggg gatggcgacg 1500
cagggtccgc ggccccgcac acgcccccgg aggcggcagc gggcaggttg ctgctggtgc 1560
tgagctgcgt agggctgctg ctgacgctcg cgcacccgct gcggctccgg cactgcgttc 1620
tggtgctgct cctggccagc ttcgtctggt gggtctcctt caccagcctc gggtcgctgc 1680
cctccgccct caggccgctg ctctccggcc tggtgggggg cgctggctgc ctgctggccc 1740
tggggttgga tcacttcttt caaatcaggg aagcgcctct tcatcctcga ctgtccagtg 1800
ccgccgaaga aaaagtgcct gtgatccgac cccggaggag gtccagctgc gtgtcgttag 1860
gagaaactgc agccagttac tatggcagtt gcaaaatatt caggagaccg tcgttgcctt 1920
gtatttccag agaacaggta tgttagctgg aaggcgaggt ctgggacgcg agcgggttcg 1980
ggtttaccgc tgcagtttcc gagttgaatt cgcttatttc aaagcatgtt aactttcaga 2040
atggaaaaag ggtgtgttgc gggggggggg ggggaggaaa taatgtttta ttctggaaag 2100
gattcttaaa atacaggaag ccttttaaaa atgcagaagc aagtagcttt tgtcattgaa 2160
tagaaatcaa atagcccgct aatcacatgg cactttttaa ggactgtata tgcaatagat 2220
gagttcgcag aagaatgagg tgaatcattt gtggttgaat agattgacta ctcccgcttt 2280
tggcatcctc tgatatgtgt ctttttggtg ccatttatct gtttgctgtt ataatgatgc 2340
ctacccaaaa ataacataca gcctttagaa tgagaggata catctatgag ttttaatcag 2400
tgtggtttcg ttattgaaaa tcgagaggaa aaatctgaga ttttatcgtt acaagacatg 2460
agctttgcaa atgagagtaa tttagatgaa aagacagcag t 2501

<210> 35
<211> 2501
<212> DNA
<213> Homo Sapiens

<400> 35

aagccacatt gtcagtggtt tccttgggca tgggggtggg agctgcagag tcaggggcct 60
ctatctgtgt cttgaaggag ccacaggtca atgggggtga gaatggagca ctctcctcag 120
aggcccccaa ggacagaacc caccggcagc cagcctgaca ggcactacaa ccccagcctg 180
gacccggagt gcagtccctg agataaaatt aaaggcttta tggcaaacac agtatgagat 240
ttctggtggt gggggtggtg gaagctacgg gttggaagtt ggaggtggca ggtgggtggg 300
cagcaggctt agctctgtcc ccgcaccccc tccccaccag gagctttggc tcttttggtt 360
ccccccaccc agtacacaca gccccttctc tgctcctgag gagtggggac atctcaaaga 420

gcaagagctg tgacttctcc accccatctc caatgtatat gttgggtcct ataaattcaa 480
gcatgaataa gatgaaatga ctcctgcctc tgaggacgcc cagtgagtgg gagagggggt 540
ggatccttaa aggggcgcat acaacatagc ctaacacatg ctgagttgtg ccacacaggg 600
agccacagac tgagcaacca ctgggtgagg ggaggggttt caggagagat tgggattgtg 660
tgcactgcgc tttttttttt tttttttaag acagagtctc gctctgtcac caggccggag 720
tgcaatggtg tgatctcaac tcactgcaac ctctgcctcc caggttcaag cgattctcct 780
gcctcagcct cctgagtagc tgggactaca ggtgtgcgcc accacaccca gctaattttt 840
gtatttttag tagagacggg gttttaccat gtaggccagg atggtctcga tctcttgacc 900
tcgtgattcg cctgccttgg cctcccaaag tgctgggatt acaggcgtga gccaccgcgc 960
ccggcctgca ctggggtttt aagtcccaat agttttaagt tagggctggt ctcctcaaga 1020
aaaaaactca tacacacaga tgtgccacat tttgcattca atttcagggg ttccagggat 1080
acactgacgc ccatgtggag ctcctaaaca atcttaacag ttcacggggc caggttttaa 1140
aaatccaatc cagggccagg cgcagtggcg catgcctgta atctcagcac tttgggaggc 1200
caaggcgggt gtatagcctg agctcaggag ttcgagacca gcctggccaa catagtgacc 1260
ccacccccta cccccgctcc ccaccaacca tctacaaaaa aatacaaaaa ttaaaggggc 1320
gtggtagcgt actctcgtag tcccagctac tgggaaggct gaggtgggag gatcacttga 1380
gcccaggagg tcgaggctgc agtgcgctgt gatcacgcca ctgcactcca gcctgagcaa 1440
cagagtgaga tcctatctca agggaaaaaa caaaacaaaa caaaacaggg aacgtgggaa 1500
ggaagcgttc cctgacgata ctggttatag cgggtctgaa gctgggctgt ggaattggag 1560
atctcgggaa aacaacaccc gttggagcca cgaccctagg agtccactaa gcgagcgccc 1620
attgtaggag ggaggtgtct acggaaagac cgtgaggtag tgaacacccc gttgccggtg 1680
aagtgcaagc gcagggaggc cggggaggaa cttaaagcca cgaagacact gacttaatgg 1740
gctctaagtc cttccagttc tgggatccca cctccgggtt aattccgctt cccttgagga 1800
gcgcatgcgc aatggaccca ctcgctcaaa gaacccaccc gacccgagac ggcgcgaatg 1860
tgcagtcgag cgctgatccc ggccttccca tgagcctctg ggcaggctcc caaggcgagc 1920
gacggaagca gccgctctta gtttccggca acacgacagc ggctgccgag cgacccggaa 1980
gtattcccat tttgcgttgt ctgggctcgg cggcagccgg gctcggagtg gacgtgccac 2040
tatggggtcg tccaagaagc atcgcggaga gaaggaggcg gccgggacga cggcggcggc 2100
cggcaccggg ggtgccaccg agcagccgcc gcggcaccgg gaacacaaaa aacacaagca 2160
ccggagtggc ggcagtggcg gtagcggtgg cgaacgacgg aagcggagcc gggaacgtgg 2220
gggcgagcgc gggagcgggc ggcgcggggc cgaagctgag gcccggagca gcacgcacgg 2280
gcgggagcgc agccaggcag agccctccga gcggcgcgtg aagcgggaga agcgcgatga 2340
cggctacgag gccggtgagg aggcggggcc tgcgcagggg gcgggtcggg cgggggtccc 2400
ggaacgcgtg ggtctgggtg tgcgcgcagt gtcagcgaag ctcttcaggc caggaaaccc 2460
ccaagctgtt taccagctct attaaatagt ctttctacgg g 2501

<210> 36
<211> 2501
<212> DNA
<213> Homo Sapiens

<400> 36
   ccactcgtac tccccggggg agctctccgc ggaggaagcc atggcgtact catccccatc 60
   gctctcgctc acgccttcct cctgctgatc ccacgctggc cccatggtct tgtatgtcgc 120
   agcggccaca gcccgcgata acaccttcct ccttgtccta gccgccttaa cctcatcctc 180
   ctctccgtca gccttgggcc cagccttgga ctcagcctcg ggttctgaag ccgaggtttc 240
   ggccgcagtc gccatcttgc gcttctaatg actctcgggt cttcccgcgg ccgccgaaga 300
   cccgctgagg cagaagctgc ccgcggcgcg cgccttatca aaaacccgtt ttcttgccat 360
   tgaaagagaa actgggaact tagataacag gaaaatctcc acatacacta aattcacatt 420
   attttatacc ctgtaaaagc tcccaccaga tataaaaact cattataatg gcatgcgttc 480
   cgtcacaact tcggacagaa gttgggtctt tgggcaggaa gtggaagcca tagaggaagt 540
   catggagcga gagtgatgtc tgtagtgaag aatttggcgc catcttgaga aggtcaaagt 600
   gattcttcga ttaaatgcct tacttatcgc ctagtgtgac ctggcgcttg ggtttactct 660
   ccccagcaac tgcgcctcaa acgcaaagaa aactgcaggt cctcggggaa ggatcgattt 720
   caccattcaa gagaaaaatc catttcccag attcctcccc tttatgtaaa cgtctggtac 780
   ctagtagacg ttaaatacac gtgagttttc cggttttata agatcacata ctgcaaacca 840
   acagtaaatt acattaatac gcatttcttt agaaattctt aattgctagg cactgacata 900
   taaattgagt ttgctgttgt tttagagaga aaaatagaca tattcatttt ttttcaatca 960
   ggacatggta ttcgtttcct tcttaagctt cccatttttg aggtaggaaa gtattggtgt 1020
   gtgtctctat ctctaccttg ctccctcatt agcaggctta atggcaggaa attatgtctg 1080
   ttttgctttc cgttggatcc ccgggtctag tagagcgcct tgacacatag taggtactcg 1140
   acacagatgc tggcttcatg atgaagcaat tgtcctcgaa ttaatacaaa agggaagatt 1200
   cagactcagc gagccggtac cattcgtcta gggacacaca gccaggaagg aaccgaacca 1260
   ggattctaac ccaggattac ccaagttcga attctaattg aacataagtc cttagcgacc 1320
   ttggacggac ttactaaact gccctgaatc tcggcttcct tagctgcaaa atagggttag 1380
   gaaaagtgga ctcggtgctc ctctgcgccg cattcccgca gcgggatttg tgaaagcgaa 1440
   cttgagcccc acactgcaga agccctgagg tcctgtctca accagaatcc agcacccagg 1500
   acaagccctc cagcgacttc agaccttccc gctcccgacc ccgcccgccc ccgagaggcg 1560
   tggtcaaggc ggggccagaa ggcggacgcg tgcacggagc ggtaaagcgc aggcgcaaag 1620
   cttcggtgac gtcagagagg cgcgctccca gctcggagcc gactcgcaga cgcgccccgc 1680
   ccctcggcgt cgctctggac tggcgcaggc gcaagccggc aagatggcgg cggctggggc 1740
   tttccgtctg aggcgggcgg catcggctct gctgctgcgg agcccccgcc tgcccgcccg 1800
   gagctgtcgg ccccggcccg actctatcac aagaaggtag ggacaaaaga gggacgcgcg 1860
   gaatgccgac tcagcggagg cctgggctgg aggggcggcc gcggggttct gcgcagctag 1920
   gactgggagc tgtcccctcc cacgtctttg ccctgactcg ctttcccttg ctgcgcagtg 1980
   aggctcactg caactgataa acaacagtta ccgctcatcg ggcggcgact tccagggggc 2040
   cccgccgctg gccgcgactt cgtgcgtccc aattttaaat tcgccaacag cccaggaggc 2100
   agggtcctgt tgggacttgt ctttctgagt ccagggacag acacaccccc ggagcgggct 2160
   ccggcttcag ccactccgct gccctggcca gatgaccttg ggctagtcac tgcgcctctc 2220
   tgaacctgtt tccccaggtg taaatggggg gctctcagct gtcccttaca aaggatactg 2280
   tgcgtggagt cctggcatgg ttcctggcac ataggcccca gcacgaggag accgtttctg 2340
   ttactgcttt aggagtgcat aggggagtca ggcttgtaac caacatgcgt aatgtttttg 2400
   ttttgtttgg ggggtttttt ggcggggaag gagggataga ttcatagtct gcagcccctt 2460
   gctgtctggt ggcacaaagt cccccagacg tgagactccc a 2501

<210> 37
<211> 2500
<212> DNA
<213> Homo Sapiens

<400> 37

ccgtgagaaa catcctgcct aaccacctgt ccgcaaggcg gacaaaggcc caactgaaga 60
aacatcccta tcatatcttg cttggcagcg ttctaaggaa caccacaatg atattccacc 120
agaaaaagga ccaaaccacc tgatcataag aacatcttat caatatcccg ccgggcagca 180
agccatactg cccaggcccc tcccacccct acctacaagc accccagcct gtaagcggcg 240
gtgggctctg gcattaagcg ggccccccac ttccacaatg gtctgcaata ttcctgtgtt 300
gttgtttgag ccgcccccgc tctgtgtgtc tttctttcac tctcgccttc acttcagaac 360
ctaacaatct taccgtctca ttcacctcgt tggattcaca ggggacgctc ctcttcaccc 420
cgccggcact ggattgtttg tccattgtat atattggttc ttcaatgtct gcggcaggct 480
tttgtaggtt ttgaatcttc gcagtggccc ggagctcttg ccctccctgt atataccctc 540
ctggtgacaa ggcaaagcca cacccttgag ctttgtttga tcatacaggc agtgtcccag 600
ccaatggcag ccctagggtg gcttcacatc acaaagcccc actgctgacc actccctggg 660
cttgcgggcg aggggtgaca ggggtgtaga gcaaaccaaa tgctgttgtt gtttcagcat 720
cccctgaaga tgcatcccaa accgatctgc cgccgctcct catttctcca tgtttaatgt 780
tcacggttca catggaagtc agaggatgat tccttcaagc ccttccccac agccattcct 840
attaagtgat tcattctttt gtcttccagc cctcaccatg acttagtatt ttcgatgtct 900
cacctcaaat cccccacgct agtgtggcta catttattta ttggcggaga tgtgaattat 960
cccatttccc tcctacagtt ccttcacatg cacctcgaag accatttact tttgggctac 1020
tgccaggcaa atttcaaccc atgttctttt acccaatgtc catgtagttc aagtttccta 1080
tttccaatct gaaataatgg ccttcagtct gtcaaaactt tagtgaataa acattcttta 1140
ccgtgtatcc tagtcttgtt tcaaatcagg ggtgtatatt tttttctctt tttgaaatgg 1200
agtttcactg ttgttgccca ggctggagtg cagtggcatg atctcagctc actgcaacct 1260
ctgcctcctg ggtttaagtg attctctggc tcagcttcct gagtagctgg gattacaggc 1320
ctgtgccacc acgcccggct aatttttgta tttttaggag agacggggtc tcaccatctt 1380
ggccaagctg gtcttgaact cctgacctcg tgatccaccc gccttggcct ccaaagtgct 1440
gggattacag gcgtgagcca ccacacccag ccaggacggc cattattaaa accaccattg 1500
acaagtaaat tttggttact tctgtagcat acaacaattt aacataaagt ttataattaa 1560
taacatacag aagttatttc agaattataa gagtttccca taattttgga gcacatatca 1620
ataacatttc tataaataca gcccaaagaa agtcaaacac cattttatat ttgacgatgc 1680
ttcttgtatg atttttatac caaataagac caatatgtct cttttggact tcagaggact 1740
catatcaaaa aattaatgag gaatcaagtt agaatttgac tttggaaagt tcgtcaaaga 1800
taaaaagttt aaaatgcttg atatcacgaa atagggtcat gggtcattgt aaaataagtc 1860
attcatttaa ccaaagcagt aactcaaaga tttcaaaaaa gaaaaaaaaa aggtgaaaat 1920
ctttattctt tgagagagga ggcttaatat cccaaacaat aagccctaat taaaaacagg 1980
atgcggcaaa tgaaatttgt ttttttgaaa ttttataata aaagtttaat catcttgagt 2040

ataaaatata atttctataa accttgtaac ctttatcatt ttgcattaag gagtgagtta 2100
atgctccaag aaagccttgt taaactgaca cagcaaccta gatactggtt ttgctttagt 2160
gtgcctttga tattaatggt taattcctag agaaactgag ctacttttct cttaatatca 2220
gcccttacaa tctcacatgc ccacctgttc cacgatagtc cttgggcctt gaggagttga 2280
atagttttaa tttctgaccc tgcatctcat gaacgcagtt tactttgatt agcatcttct 2340
actgtgcctg aagatgaggc tgtaattgat gtcagtgttt aagatatagc agaacttggt 2400
gtccttttta gacccagaag tcaaagccct atagctcaat ggcacaagga ctttaaaagc 2460
acatacagaa agttacacag atggaagaaa acactttttt 2500

<210> 38
<211> 2501
<212> DNA
<213> Homo Sapiens

<400> 38

gatgttttca tatagattag tctgtttctt gacttctatt ttgtttcact aataaatgtt 60
tctatgcgta ttcatttttt attgctgctg taacaaattt tgaatagctt agacactgct 120
aggcgcggtg gctcacgcct gtaattccag cactttggga ggccgagaca agtggatcgc 180
tcaaggagtt caagaccagc ctggccaaca aggtgaaacc ccatctctac taggggttgg 240
tgatgggcgc ctgaaatcgc agctatttgg gagttcagaa cagttcccac taacattatt 300
ccctcataca caccagggct gagtgaaaaa acaaattgca cacaaaaagt taactacaga 360
ggccattcat ataaaatgct aaaacatgca aaacaagagt atatattttt atggataata 420
agtaaatggt aaagtataaa aacatgaatg agaataaaaa accatcaaat tgagaagaat 480
ggctgtaagt ggagaaggag ggagggcagg gattgcacag aaagcttcaa ggtgacttgt 540
tgatagtggt tttctttgtt tgtttgtttg tttgtttgtt tgtttgtttg tttttgatgg 600
agtttcgctc ttgtcgccca ggctggagtg caatagcata atatcagctc actgcaacct 660
tcaactcccg ggttcaaatc attctgctgc ccacgcctcc cgagtagctg gaattacagg 720
cgcccgaccc acacccagct atttttttta tttttggtag caacaaggtt tcaccatgtt 780
tgccaggcag gtctcaaact tcctcatctc agatgatcca cctgcctcag cctcccaaag 840
tgctcagatt acaggcatga gctaccacac ccagcgcgtt tatagtattt ctaatactct 900
gaataaataa atcagatcta acatagctgt ggggtaatgt tgagatccga ctggactcaa 960
tagtattcca catacttttc cacgtgtttg aaatatttca tttttaaagg acatgttgtt 1020
cttgctaagc actgttaatg aatcaaagga cagttaagaa aatgttacaa gtgaaaatat 1080
aaaaaaagaa aatattaaaa gcaaagatct gcaaaaaaaa tccagagttt ctataattaa 1140
cagcatgtaa gtattggata agtatcttat gagagaggat aatgaacaca ttatgtaata 1200
aagatggatg tttctctgta ttttatcaaa accaaatagt cttctcattc ccaaataacc 1260
ccaattctcc gtgatgagct tggaagggat tttgaaagag tgatccctga tccaaaacac 1320
agagagcttt cccacttgtc agtgagcaga gataacatag agtgaaaaaa agacaagttc 1380
ttgcgaagag atttttgggc atttcaggac tattaatggg acatatgtat ttacttgctc 1440
ttctgctctg acaacacaat tataagacaa ggtcagaaag tccaaactgt ctccaataga 1500
cctattactc gccaactaaa caggccagat tctacaatct cccacaatca ctataaggga 1560
cctgaaaagc cagtgcttga gtatctgcca agtttttgac gttaaaggac tgtttttata 1620
ctgaaaatat ttcagagcca ctggaccaaa tcatccatgg ttcatcacac atttaacagc 1680
ttaattcaca taccataaga ttcacccatt tgaagtgtac aatgattttc agttgttgca 1740
catcttgagt ggatacagtt cagattccca accaatcaat ttaattattt gggaaaaagt 1800
aaaagatatg taatggaata agatgagact gtgatggggt ttagtcccca tgtgataaac 1860
catgagatgg aaaattctga attgaagcca cagataaatg aaccaaccat gactaaacat 1920
aatttagaag caaatctcaa ataactcctc aacaatcagt ggactcataa cctctgctgc 1980
agaatgccct catgcaacag aagtctctct agagtttgga aatcttcacc aacaaagaaa 2040
aattctgatg tattctcttt cagttgaaca tcctcagatg actttcggca ggctccatag 2100
aatcttcccg aaggtgagtg tctctcaaat ctaaaggacc agagaacctt tgtccctcca 2160
cggatatgaa cactggtaag agtggaagaa tatcaaaaat accctcactg cctcctcccc 2220
atgtctatca caacacctga tgtagcatca atggcttgat aatgctaaca gttgtgatcc 2280
ttaatatttc ttttgttttc atagagatgc catatattct aaacagttta catggattaa 2340
tttatttaat ccttaagaat acctctacga cgacgtttct attactatct ccaaataata 2400
atgagtcaca cacttcagtt atcatccata taaaagccat gtgagttggc gcaaatcttc 2460
taagttctct gagatccaga cttctggtca atgaaatggc a 2501

<210> 39
<211> 2501
<212> DNA
<213> Homo Sapiens

<400> 39

ggactatatc aggactgaca ttttcatgat caacagccag ccatctacca gttctggcca 60
atcctatctg tatttcatca acaatgaaca gaaccaagct gggagcatga gatgggacga 120
gggcaagtaa aaataccaca aagctcactg ttcttacgga gattcagctg gtctctctct 180
cgctctttct ttctttcttt ctttcttttt cagtcttgct ctgtcgccca agctggagtg 240
cagtggtgtg atctcagctc aatgcaacct ccacttcccg ggttcaaatc attgagtgag 300
cccaggaggt caagaccaac ctgggaaaca tagcaaaagg cagggtggtg catgcctgta 360
gtcccaaggc cgaggcggga gaatcacttg agtcccagag gtagagccca gcctggacaa 420
catagcgaaa ctgtctctaa tagaaaaatt aaaaatatta gtgggggcgg ggtggtgtga 480
gcccatagtc ccgaggccga ggcgggagga ttgcttgagc ctaggaggtc gaggccagcc 540
tggccaacat agcgaaaccc catttctgct aacaacaaga acaacagaaa aatagcgtgg 600
gcggggtggc tcactcctgt agttttgagg ccaaggtggg agcattgctt gagcccagga 660

gtttgatacc agcctggcca acaaagcgaa agcctgcctc tcctaaaaaa taaataaata 720
aataaataaa taaaaatgag cggggtagaa tggcacacgc ctatagttct gaggccgagg 780
tggaagcatc atctgagcct gagaggttga ggccagcctg ggcaacatat cgaaacccgg 840
tttccactaa aacgaaacaa aacaaaacaa aaaatagctt gggcagggtg gtgcatggct 900
gtagtcctga gatgggagga tcccttgagt ccaggagctt gatgccggcc tggccaacat 960
agggaaacac ggtttctact aaaaaaaaaa aaaaaaaaaa aagcgtgggc agggtggtgc 1020
atgcctgtcg tctcgaggcc atggcggaag gatcccttga gcccaggacg atgagaccag 1080
cctggccaac atagcaaaac ctggtttcta cttaaaaaaa aaaaaaaaaa aaagcgtggg 1140
ctgggtggtg aatgcctgta gtcccgaggc cgaggtggga ggatcgcttg agcccacgac 1200
gtcaagacca gcctggccaa cacagcgaaa ccttctctat taaaaaatca aaaaataaaa 1260
aatattagtg ggggtagggt ggttcgcgcc tgtagtccca agacggaggc tggaggattg 1320
gccaaggcag atgtaaccaa taccacaatc acatcccata agtaaataca ttttcctctc 1380
ttcagggcta caggtaaagg atggtaattg tgcgcaccat acttaagatt ccctttcaaa 1440
aatgtaagca gaggttgcgg ggccttggac tgttttttca gtggcaacag acatagaagc 1500
cactgaagaa tgaaagccac gactaagtat agcaaacctc cgcaaatgtg ctagtttgga 1560
aaacatcgtg tctttcaagt agaaaaatca cagattgact atttttttct tcccacagtt 1620
cagactagaa tccagatttt taacccaaga tccaggaacg gtcttcagag agttcaaaat 1680
ctgacggcgc ctgaggacca cccacttttt cgcagtggca acaaagtgtg gctggaggag 1740
gagacattat tctgctatgt cactgcccaa ggatgatgga ccaatcaggg cagttagtga 1800
actccatctg gccaattaga agtcagaaca gtaggcggaa caagcgaagc ggatgtggct 1860
tctatcagtc ccggctccag ggacggaacc ttctcaaagt gggggtggag actctaattt 1920
tcccgcctaa agcatcccct gggattggct actttaagtt cagagtacgc atgctctgac 1980
tttctctctc tttcgattct tccatactca gagtacgcac ggtctgattt tctctttgga 2040
ttcttccaaa atcagagtaa gcatacgccg atttcttttt tccattcttc ctacccctcc 2100
cctcctccgc ggtgcatttg ctatctagtt ttaataagga gtgtatatga ggcaggccgc 2160
catctcgaat ctttcctgtc agtttctaac tttttcaggt acgggatttt tcctaggaac 2220
tctgtagtaa cttaagaaat ttgggccggg cgtggtggct tacacttgta atccctgcac 2280
ttttggaagc cacagctggt ggatcgcctg aacctaagag gcggaggttg cagtgagcca 2340
tgatcacgcc agtgcactcc cgactgggca acagagcgag accctgtctg aaaaaacaaa 2400
aaccaaaaaa gctcactcaa atctttcctc ctgggctcaa gtgagcctct cgcctaggcc 2460
ttggggctac aggcgcgcac caccccgctt ctgctaaatt t 2501

<210> 40
<211> 2501
<212> DNA
<213> Homo Sapiens

<400> 40

aaacagcctg agtctttcca gcctcaggac ctttggtgct ctgggcgggg tggtgcacgc 60
ctgtagtccc ggggccgagg cgagaggatc gcttgagccc aggagttttt tttttttttt 120
tttttttttg agacagggtc tcgctctgtt gcccaggctg tgttgcagtg gcgcgatcgt 180
ggctcactgc aacctccgcc tcccgggttc aagcgatcca ccacctgtgg gcctccaaag 240
tgctgggatt acaagcgtga gccaccacgc ccagcccaga ttttttaagt tactacagag 300
ctcctaggaa aaatcccata cctgaaaaag ttagaaactg acaggaagaa tttgagatgg 360
cgacctgcct cacataaata cattattaaa attagataag aagtgcacca cggcggaggg 420
gaggggtagg aagaatggaa agagaaaatc agcgcatgct taccctgatt gtggaagaat 480
caaaagggca aatcggcgtg tgcgtactct gaatttaaag tagccaatcc aaggggacgc 540
tttcggcggg aaaatcagag tctcccctcc cccccgcctt gggaaggttc tgtccctaga 600
gcctggactg acagacgcca catcagcttc ccttgttccg cctactgttc tgacttctaa 660

ttggccagat ggagttcact aactgccctg attggtccat catccttggg cagtgacatt 720
gcagaatgtt gtcgcctcct ccagccacac tttgtgcgac aaagtgggtg gtcctcaggc 780
gccatcagga gattttgaac tctctgaaga ccgtccctgg atcttgggtt aaacatctgg 840
attctagtct gaaccgtggg aagaaaaaat agtcgatctg tgatttttct atttgaaaga 900
cacaatgttt tccaaactag cacatttgcg gaggtttgct gtacttagtc gtggagttca 960
ttcttcagtg gcttctacat ctgttggaac taaaaaaaca gtccaaggcc ctccaacctc 1020
ttttattctt attttttttt cttttttttt tttttttgta gttttctttt tttttctttt 1080
tattattata ctttaagttt tagggtacat gtgcacattg tgcaggttag ttacatatgt 1140
atacatgtgc cacgctggtg cgctgcaccc actaactcgt catctagcat tgggtatatc 1200
tcccaatgct atccctcccc cctcccccca ccccacaaca gtccccagag tgtgatgttc 1260
cccttcctgt gtccatgtga tctcattgtt caattcccac ctatgagtga gaatattcgg 1320
tgtttggttt tttgttcttg cgatagttta ctgagaatga tgatttccaa tttcatccat 1380
gtccctacaa aggacatgaa ctcatcaatt tttatggctg catagtattc catggtgtat 1440
atgtgccaca ttttcttaat ccagtctatc attgttggac atttgggttg gttccaagtc 1500
tttgctattg tgaatagtgc cgcatttttg gaagggaatg taagtgtggt gcgcacaatt 1560
accatccttt acctgtagcc ctggagagag gaaaatgtat ttacttatgg gatgtgattg 1620
tggtattggt tacatccgcc ttggtcaatc ctcccatctc attctcggga ctacaggcat 1680
gcaccaccct gcccaggttt gtttttgtta ttattattat tattttttag ttgaaactgg 1740
gtttctctat gtaggccagg ctggcctcaa cctccagggc tcaaggatcc ctcccgcctc 1800
ctcagcctgg ggaccacagg catgcaccac cctgcccacg gtattttttt tgtttttcag 1860
tagaaaccgg gttttgctat gttgcacaga ctggcctcaa cctcctgggc tcaagcaatg 1920
cttccacctc agcctcggga ccataggcgt gtgccattct gccctgataa ttttttttag 1980
gagagacagg ctttcgctat gttgaccagg ctggtatcga ccttctgggc tcaagcaatc 2040
ctcccacctc agcctgggaa ctacaggggt gagccacccc gcccatgcta tttttggttg 2100
ttcttcttat tagtagaaat ggggtttcac tatgttggcc aggctggcct tgacctcctg 2160
ggctcaagca atcctcctgc ctcagcctcg ggaccactgg cacacaccac ccagccccca 2220
ctaatatctt taatttttct agtagagaca gtttcgctat gttgtccagg ctggtctcta 2280
cctcctgggt tcaagtgatc ctcctgcctc ggcctcggga ctgcaggcct gcaccaccct 2340
gccttttgct atgtttccca ggctggtctc aacctcctgg gttcactcaa tgatttgaac 2400
ccgggaaggg gaggttgcat tgagctgaga tcccaccact gcactccagc ctgggtgaca 2460
gagaaaaact gtcgaaaaga aaaggaaggg aagggagaag g 2501

<210> 41
<211> 2501
<212> DNA
<213> Homo Sapiens

<400> 41

ccttctccct tcccttcctt ttcttttcga cagtttttct ctgtcaccca ggctggagtg 60
cagtggtggg atctcagctc aatgcaacct ccccttcccg ggttcaaatc attgagtgaa 120
cccaggaggt tgagaccagc ctgggaaaca tagcaaaagg cagggtggtg caggcctgca 180
gtcccgaggc cgaggcagga ggatcacttg aacccaggag gtagagacca gcctggacaa 240
catagcgaaa ctgtctctac tagaaaaatt aaagatatta gtgggggctg ggtggtgtgt 300
gccagtggtc ccgaggccga ggcaggagga ttgcttgagc ccaggaggtc aaggccagcc 360
tggccaacat agtgaaaccc catttctact aataagaaga acaaccaaaa atagcatggg 420
cggggtggct cacccctgta gttcccaggc tgaggtggga ggattgcttg agcccagaag 480
gtcgatacca gcctggtcaa catagcgaaa gcctgtctct cctaaaaaaa attatcaggg 540
cagaatggca cacgcctatg gtcccgaggc tgaggtggaa gcattgcttg agcccaggag 600
gttgaggcca gtctgtgcaa catagcaaaa cccggtttct actgaaaaac aaaaaaaata 660
ccgtgggcag ggtggtgcat gcctgtggtc cccaggctga ggaggtggga ggatcccttg 720
agccctggag gttgaggcca gcctggccta catagagaaa cccagtttca actaaaaaat 780
aataataata ataacaaaaa caaacctggg cagggtggtg catgcctgta gtcccgagaa 840
tgagatggga ggattgacca aggcggatgt aaccaatacc acaatcacat cccataagta 900
aatacatttt cctctctcca gggctacagg taaaggatgg taattgtgcg caccacactt 960
acattccctt ccaaaaatgc ggcactattc acaatagcaa agacttggaa ccaacccaaa 1020
tgtccaacaa tgatagactg gattaagaaa atgtggcaca tatacaccat ggaatactat 1080
gcagccataa aaattgatga gttcatgtcc tttgtaggga catggatgaa attggaaatc 1140
atcattctca gtaaactatc gcaagaacaa aaaaccaaac accgcatatt ctcactcata 1200
ggtgggaatt gaacaatgag atcacatgga cacaggaagg ggaacatcac actctgggga 1260
ctgttgtggg gtggggggag gggggaggga tagcattggg agatataccc aatgctagat 1320
gacgagttag tgggtgcagc gcaccagcgt ggcacatgta tacatatgta actaacctgc 1380
acaatgtgca catgtaccct aaaacttaaa gtataataat aaaagaaaaa aaaaagaaaa 1440
ctacaaaaaa aaaaaaaaaa gaaaaaaaaa taagaataaa agaggttgga gggccttgga 1500

ctgttttttt agttccaaca gatgtagaag ccactgaaga atgaactcca cgactaagta 1560
cagcaaacct ccgcaaatgt gctagtttgg aaaacattgt gtctttcaaa tagaaaaatc 1620
acagatcgac tatttttctt tcccacggtt cagactagaa tccagatgtt taacccaaga 1680
tccagggacg gtcttcagag agttcaaaat ctcctgatgg cgcctgagga ccacccactt 1740
tgtcgcacaa agtgtggctg gaggaggcga caacattctg caatgtcact gcccaaggat 1800
gatggaccaa tcagggcagt tagtgaactc catctggcca attagaagtc agaacagtag 1860
gcggaacaag cgaagctgat gtggcgtctg tcagtccagg ctctagggac agaaccttcc 1920
caaggcgggg gggaggggag actctgattt tcccgccgaa agcgtcccct tggattggct 1980
actttaaatt cagagtacgc acacgccgat ttgccctttt gattcttcca caatcagggt 2040
aagcatgcgc tgattttctc tttccattct tcctacccct cccctccgcc gtggtgcact 2100
tcttatctaa ttttaataat gtatttatgt gaggcaggtc gccatctcaa attcttcctg 2160
tcagtttcta actttttcag gtatgggatt tttcctagga gctctgtagt aacttaaaaa 2220
atctgggctg ggcgtggtgg ctcacgcttg taatcccagc actttggagg cccacaggtg 2280
gtggatcgct tgaacccggg aggcggaggt tgcagtgagc cacgatcgcg ccactgcaac 2340
acagcctggg caacagagcg agaccctgtc tcaaaaaaaa aaaaaaaaaa aaactcctgg 2400
gctcaagcga tcctctcgcc tcggccccgg gactacaggc gtgcaccacc ccgcccagag 2460
caccaaaggt cctgaggctg gaaagactca ggctgtttct c 2501

<210> 42
<211> 2501
<212> DNA
<213> Homo Sapiens

<400> 42

ttgcactact gcactctagc ctaggtgaca gactgagact ctgactcaaa aaaaaagaaa 60
aaaaaagaaa gagagataca agccgagaga aggaaggtag ggacggtgtg gaggtgccgg 120
gatgccacag agacacttgg actcatcaga acagaagcct aagggagaga aacgtgcagg 180
atccaggtat gagctccact gtggccagtc cctgccctca gccctgacag gatacagaag 240
agcagaacac ccagaagctg ccttgcgatt tttccctgca caaaaggaaa atgtggggta 300
ctttctgcag cctaagaagt agccaaagca ggaaaaggga tgctcatgtg tccccagact 360
tgtctgtacc tagaactttc tgttacctag tttagtcatg gcctcatact ttctcttcat 420
atacacatag atgattttct ccgaggattt catcttttcc cactctttct tagagaagta 480
tttggcaata tcatcgaagg cctaggaaaa gataaaaaag gaattttgtc agtgactcag 540
ctagacacgt ctgccattca gctggagccg cctcctgtgt gctggatctg ggaagtgggg 600
atgatattcc gtcctggttg atgccatggc taactgacag aacatgaggg acctttccta 660
gcttcacccc tgccacacag tagggcttta atgctgctgg ctggctctct tcccactttc 720
cagaatggac tgagattcac caaatgtagt gcacggtcac agacttgtct ccaaggatgc 780
tagttgatga tagagcgagg atgggaggct ctcaagggtc cagatctccc ccaagaccct 840
gctccttgtc tccagtatct ctatcctccc ctcctcagaa accggtcacc ccacactgtc 900
ccctgggcca ctactttgcc ccctccaggt cacctcacct tttggatctt ctctggtatt 960
tgagaaccag ccctaggtct ccttgcaaag gcatcgtctc cgttcatggc accgggagca 1020
gtctgtcctg caagagaaac agaccgagtc tttccagcca cagcagcttt ggtcttgtgg 1080
agggagaaat cagtgaaggc cggccaccct cagtcacctg gaatcaggtg cttcatttct 1140
ccatctgggg cttatctgtc cctgagtaat gacatggggc gaagtcagat gaaaataggg 1200
aaccaggggt ctctgggaga agtattgatt tgggatgaca ggcttcctgt gggcaaagca 1260
gccttgagtc tttgggaggg ggttggctaa tgttgttagt agtttccctg gggccaggct 1320
taccctgaaa gatgtacaga cccttgttgg gaaggcagag atgtgactgt gtaattttat 1380
tcagtggggg catgctgaca cccccactca ataaataaag gaagggaaat gagtcccaga 1440
gataacatgg tctctctggt gatggatctg atcaggcaga gggatggggg gttctgttct 1500
gttgaagaga aatgagcatg gctaatatga acggatttag aggctattac tgggtgattt 1560
gtaaattatt agaagaagag agctagaatt tctgagacta caagagcccg ccatcactta 1620
aagagaatgt agggagtttc aaggtacagc aatcagccag gcgcagtggc tcatgcctgt 1680
aatcccagca ctttgggagg acaaggaggg cgggtcgctt gagtccatga gcttgagacc 1740
agcctggcca acataccgaa accatctcta ctaaaacaac aacaacaaca aaacacacac 1800
acacacacac acacacacac acacattaac agaagcggag tggtgtgcgc ctgtagtccc 1860
aaggcctacg tgagaggatc acttgagccc aggaggaaag atttgagtga gattttttgt 1920
ttgtttgttt gtttgtttgt tttgagtcat ggtctctctc tgttgcccag gctgggatgc 1980
agtggtgtga tcatggctca ctgcaacctc cgcctcgtgg gttcaagtga tccaccagct 2040
gtggcctcca aaactggagt tacaagcgtg agccaccgta cccggcccaa atttgttaag 2100
ttactagagt tcccaggaaa aatcccatac ctgaaaaagt tagaaactga caggaaggat 2160
ttgagatggc gacctgcctc acatacactc cttattaaaa ctagataaca aatgcaccgc 2220
ggaggagggg aggggtagga agaatggaaa aagaaaatca gcgcatgctt actctgattt 2280
tggaagaatc gaaagagaaa atcagaccgt gcgtactctg agtatggaag aatcgaaaga 2340

gagagaaagt cagagcatgc atactctgaa cttaaagtag ccaatcccgg gggatgcttt 2400
aggcgggaaa atcagggtct ccacccccag ttagaaagtt ctgtccctgg aggctggact 2460
gatagacgcc acatcagctt tgcttatccc gcctactgtt c 2501

<210> 43
<211> 2501
<212> DNA
<213> Homo Sapiens

<400> 43

gaaaacaaag tcaatggtat attcaaaatg gagctcatta ttttattgct caaaattaag 60
taacttagca ctaggatgat gataatagct agttactcaa gcctgaaacc aggtagtcat 120
cttagactgc tccctttctt tcacttctta ctactaaaaa gttataaaat tctactaatt 180
ttacttctta aatatctttt acatttgaca cctcctcttc actccactgt ccgtgtcata 240
gctcaagtcc tcatgtctca cccaggctat taaaactgtc tcgctactct ggtctcttaa 300
gcctaaacca ttcttcatac tacaagtttt cttctgaact cgatcgtgta atcctttctg 360
ctcaaaattc ttcaagatct ccgtccacaa agtcaaaaat ccagacactt ttccaaggcc 420
tttctataat acttgctttc tctttctctc tttttttttt tttttttttt tgtgagacgg 480
agtctcactc tgtggcccag gctggaatgc aatggggcaa tctcggctca ctgcgacgtc 540
tgcctccccg ggggttcagg cgattctcct gcctcagcct cccgagtcac tgggactaca 600
ggcgtgtgcc acgtgcctgg ctaattttgg atgtttactg gagacagggt ttcgtcatgt 660
tggtcaggct agtctcaaac tcttgacctc aagtgatccg cccttcttgg cctcccaaag 720
tgctgggatt acagaagtga gccaccgcgc tcggctaata cttgctcttg aaatctctag 780
tgtcatctct tgccattttc cccacttaat cctcacacag aacgcttctt ggacttagtt 840
gtaataatct ctctcacaac tccaatgcct ttgctcgcct tgcgtcagcc agcaatactc 900
tttccccatt ccctctccct ctccctctcc cccttccccc gctctcccct ctctcccctc 960
tttccctact ctcctccgtt tcctccctct gcctcctttc tctcctctgt ctcctctctc 1020
tctctctttg agacagaatc tggctctgtt gcccagactg gagccagtgg caggattatt 1080
agctcactgc agcctctgtc tccaagactc aagtgatctt cccgcctcag ccttttaagt 1140
agctgaggac acaggtgcgc accaccacac ccggctattt taggagaggc agggtctcac 1200
tacgttgccc agactggtct tgaactcctt cctgggctca agcagtcctc ccgccttggc 1260
ctcccaaagt gttgggatta caggcgtgag ccaccggccc gcctagccaa ctctccattc 1320
actcattctt atgtgaaaca gtaacagaac tataccacat ctgtggagct tctcctgact 1380
ccccaagcca ccctaagagc cactttgcct acgtccttat cataggaatt accctaaatg 1440
attataggac attttaattg tttgcccatc tgtttccttc ctaagattgt gacctcttgg 1500
agggcagaaa tggatttttc actctcagct tcagtctgtc atagagcctg acagtttccc 1560
tagcattctt tgacaatagc agaagcctaa agaacttcca ctccaaggaa atacagaatc 1620
cgggaaacaa ttgttcctag cgattaggga aatctcgcga gattcaggtc ctgggagcct 1680
ggcaagccca cacagcgacg gttatttagc cgttgaactg ccagccgccc ggggcccacg 1740
cgctccgggc cgctcaggct gagcgatttc ccgccttttc tgaggttctg aggcgggagc 1800
cattggttct ttctgttgcc ctcatagacc gtatgtagca gttcgcgtgg gcacagaacc 1860
cacggtttcc cgctagttct tcaaaggtga gggcaggtgc cccgagttat tttcctgggg 1920
actgagccca gagcggggcg atgttgtgct actgcacctc cccgccgcag ccctccgctg 1980
ttttcttttg ggtagtggtc caggaactta agacagttcc tcctggcgat gtgatggaat 2040
ttaatgggac aggagaaggg aacgggcttt cttttcaggc cagcgtggca gcgggcggta 2100
gggcgaaagg gagaaggaaa cgagggttta ttccgttgcc cactccgcgg taagcgacgt 2160
tgtagggctc cactgtagcg agagccccgt ggattccttt ttttttagcc atttagtttg 2220
taaacatcac tttaaagaat acatagtgta ttcatgacac tcggtgaaaa aaaactttcc 2280
ttcccctccc gcccccccgg ggcagtagat atttacaacc gtaacagaga aaatggaaaa 2340
gcaaaagccc tttgcattgt tcgtaccacc gagatcaagc agcagtcagg tgtctgcggt 2400
gaaacctcag accctgggag gcgattccac tttcttcaag gtaaatttcc atgtgactct 2460
taattggact cttcttaact ttagggaaat gaagaaaagt g 2501

<210> 44
<211> 2501
<212> DNA
<213> Homo Sapiens

<400> 44

cccgcagtgc gtcggtcacc gtgttgggca ggtagctgcg cacgctggtg gtgaaggcct 60
cgggggggcc cccgcgggcc ccgtccagca gcgcgaagcc gaaggccagc acgttcttcg 120
cgccgcgctc gcacagcctc tgcagcactc gggccaccag ctccttcagg caggacacct 180

gcgggggaag cgccctgagt cgcctgcgct gctctccgca tgtcgctggt tccccccggc 240
cgccctcaac cccagccgga cgccgacccc ggggaggccc acctggcgga aggagggggc 300
ggcggggggc ggccgtgcgt cccagggcac gcacaccagg cactgggcca ccagcgcgcg 360
gaaagccgcc gggtccccgc gctgcaccag ccgccagccc tggggcccca ggcgccgcac 420
gaacgtggcc agcggcagca cctcgcggta gtggctgcgc agcagggagc gcacggctcg 480
gcagcgggga gcgcgcggca tcgcgggggt ggccgggccc agggcttccc acgtgcgcag 540
caggacgcag cgctgcctga aactcgcgcc gcgaggagag ggcggggccg cggaaaggaa 600
ggggaggggc tgggagggcc cggagggggc tgggccgggg acccgggagg ggtcgggacg 660
gggcggggtc cgcgcggagg aggcggagct ggaaggtgaa ggggcaggac gggtgcccgg 720
gtccccagtc cctccgccac gtgggaagcg cggtcctggg cgtctgtgcc cgcgaatcca 780
ctgggagccc ggcctggccc cgacagcgca gctgctccgg gcggacccgg gggtctgggc 840
cgcgcttccc cgcccgcgcg ccgctcgcgc tcccagggtg cagggacgcc agcgagggcc 900
ccagcggaga gaggtcgaat cggcctaggc tgtggggtaa cccgagggag gggccatgat 960
gtggaggccc tgggaacagg tgcgtgcggc gaccctttgg ccgctggcct gatccggaga 1020
cccagggctg cctccaggtc cggacgcggg gcgtcgggct ccgggcacca cgaatgccgg 1080
acgtgaaggg gaggacggag gcgcgtagac gcggctgggg acgaacccga ggacgcattg 1140
ctccctggac gggcacgcgg gacctcccgg agtgcctccc tgcaacactt ccccgcgact 1200
tgggctcctt gacacaggcc cgtcatttct ctttgcaggt tctcaggcgg cgaggggtcc 1260
ccaccatgag caaaccaccc caaatctgtt aatcacccac cggggcggtc ccgtcgagaa 1320
agggtgggaa atggagccag gcgctcctgc tggccgcgca ccgggcgcct cacaccagcc 1380
acaacggcct tgaccctggg ccccggcact ctgtctggca gatgaggcca acatctggtc 1440
acatcccgcc cgcacagggt ggagggcagc ctcggggtcc aggcacctgg ctccaagcct 1500
cggactgcag agctaggagg cccgacttcc agcccagcag tagaagccac acggccactg 1560
gtcccctcca gacctggggc cccggcacaa ccgcaggaca gctgaggact tcccaggaat 1620
ccagactccg ggttgctcaa gtttggatct aaggggcgag aaacttctgg gtctcccgag 1680
gccttgcagg gatgctgtag ctgaggtcgg caaacactga aatgctaaca aacgcaacct 1740
taaatgtaac ctttcctact ttcagaaact gccggaggaa attgctttat ttatggagct 1800
agcatttgaa caggcctcgc accctccctg ggctgtcacg ctcgctggag gttagcctcg 1860
tcttgtaaat acttaggatt acgggtcgct cttctagaaa tccccttagt gatccctaag 1920
cctttttaaa gggctgtgtt tgtgaattgt ctctgccact agggcaaagg ggcggtttgg 1980
aaaatttgtt ccaacaaaag ttaagttgta gcttacactg gttctctgca gagaagccaa 2040
catagaaaac acaattttaa aagagggaag agaagaaatg gaagcagaag attatgctgg 2100
agtaattaac accatgtgca tggcgaggaa acgcctcccg gcattcaatg aagatcgctg 2160
atacccagaa gacaccccag tattatgggt gcagttagtg tgtctttgaa aagctgatga 2220
tgtcttagtc atcacagtgt aaaacatcaa gagtgttcta acaacaataa aaaaattcta 2280
tcattggctt aaaacaccac aacacttgag tggggtgagc ttcctacctc agacccagat 2340
gtttctaaac agagtaaatt ctgagctggg catggtggct cacacctgta atcccagcac 2400
tttaggaggc agaggcaggt ggatcatctg aggtcagaag ttcgagacca gcctggccaa 2460
catggtgaac cccaccccca cccccgtctc tactaaaaat a 2501

<210> 45

<211> 2501
<212> DNA
<213> Homo Sapiens

<400> 45

agaagaaggc atccaaaggc ttagggagat tgagatggtg gagtggatta gtcactttag 60
atctactcat cccagctgag agggtccaga agatataccc ttgatcactg ccttgtgaaa 120
taggtttgtg agggcagtac ctgcatcttt gaagggtcct gtaattgttc ttctctgtat 180
ttcagatcta acagtgggaa ctgcagtcac taaactacaa aatttaaata cattgggaat 240
aattggatcc caaggtggca ggggccaagt ggtggcactc aaccatcaaa ggcaaggtgg 300
gcatagctac tgtaatgggc agcagaggca aagcagcaat cagaatagtc tgactcgtgt 360
agagctctgg cattgggtaa ttaatcaagg tgtttctaga aatgaaattg ataggaagcc 420
cactgcattc ctacttaatt tatataagca gaaaacttct agatcggatg gacaacagac 480
taatttgaat tataaacata gagaatcacg gctcctcagt caatttccaa acttgagcca 540
gtttacagac ctagaacctc ttgaatgaag gggaggctgg gtccccttga ggaaggaccc 600
cattacacta ccgacaactt atgctgttaa tatttctccc atccttcccc aaggagacct 660
atggcctttt accagggtaa ctgtgcactg gggaaaggga aatggtcaga tattttgggg 720
actactggac actggttctg agctgacatt gattccaggg tacccaaaat gtcactgtgg 780
tcttccagtt aaagtaggga cttatggagg tcaggtaatt aatagagttt tagctcaggt 840
ctgacctcgg tccagtgggt ccctggactc gtcctgtggt catttcccca gtgccagaat 900
gcataattgg catatacata cttagcagct ggcagaactc ccacattggc tccctgactg 960

gtagggtgag ggctattatg gtgggaaagg ccaaatagaa gcccttagag ctgcctctat 1020
gtagaaaaat agtaaatcaa aaataatatt gcatcccggg agggattaca gagatcagtg 1080
ccaccatcaa ggacttgaag atgcaggggt ggtgattccc accacatccc tgttcaactc 1140
tcccattcgg cctgtgcaga aggcagatgg atctaggaga atgacagtgg attatcataa 1200
gtttaaccaa gtggtgactc caattgcagc tgctgtacca gtttttctag tcctagatgg 1260
aaataaattt catctgcctc aggtttctgg tataaaaggt gcaatcccag ttacctcaca 1320
ggcctgggtg aggaaaatgc caagagctgt aaaagggctt ggaaaagtaa aaaaggtgat 1380
gtataaatgt aatgattatc attttgtgct caagaataag ccttggaaca caaaatagga 1440
aagtaagtct acagccctaa aatatatgca tataaaactt taaaataatg cagttccaac 1500
tctacagatg gtatctgggg attgtctact gctttagagt ctcaatgccc cttttctcac 1560
gtgggtgttg atttccaaaa aggtagcagt gtcgtctcta atgtgggtgt tgattttcaa 1620
aaaggtagca gtatcgtctc taacgcagtg gttagtaatg ttaagtgcaa catgcacgtt 1680
aaagagtttt aatttttatt gcaaacaagt aagggaagga gactctaaag ccctggctgg 1740
aggaagctct gccgtagagt agtggtcgca tttttgtgtg aacaaagggc tagacagctg 1800
ccactctgag gcgagggccc taggccagga gacgctgagt gcctgggagg ctcccgatga 1860
ggtggagggc cctgagtcca cggagagctg cgaagagaga gaggactcat ctgagtcagg 1920
actgcacggc gcgaccccgc ccaggccccg cccctcggac ggcctggtgg ccccgccccg 1980
ccccctcgtc cctcccgggc gctccaagtt gccgccagaa cccatggccg gcgtggaaac 2040
cgctgcgagg cggcgccacg acggaggctg aggtcgccgg cagtatccat ggcgtcccag 2100
agcggcagct ccctggggct cctggcctgg ttcttgcttc ttcagccatg gctcgaagag 2160
gcccgggcag gcagggtggg tgcgcaggga ggcgtagcac tgctcttccc ctccgcgctc 2220
ccctcagggc caggcggcca ggaccccgga gcgagcggat gggagccgcc acctgtaggg 2280
gctccaggat ccccagcggc cccccagtcc agggggaacg cagtgcgccc cgcttcggtg 2340
ttacttccct caggtgagtc tgattcttac caggctctgg caatcccaca cacactatca 2400
tcaacatgga ccaaagggac agtagcatta aacgtcaggt gacgcttctg ggccatgccc 2460
agccgcagag aactggcctg gtttgagggg gaggggacct g 2501

<210> 46
<211> 2500
<212> DNA
<213> Homo Sapiens

<400> 46

caccggcgcc cactgcgccg cgccgctcac aggcagggca cagccgccgc cgccacccag 60
ggaggggacg gcgggcagca gcgcgttcag gtcccgcacg tcggagccca tttgctgcgg 120
ctcagacccg gacgccccgc ggctcctccg gccctggaga cgttcagcgc tggcctcggc 180
ggcgcctaac ttggcccaga tgccgcccgg gtcccggact ccctgctgct ctggctgctg 240
taggcaccca ggcccggagc ggagcgtgtg ctgagacgcc ggctccggga cacacgtgga 300
agccgggtcc tgcagcaaga ggaagtccag gatcgcggcg aggagacggc ggggcccggg 360
cgcctgggct gccgtcccgg ctctgggtgg gtgggtgggt gaatgagtag gtgggaggga 420
gggcgggaag tgggggagcg gacaggcggt cgggttgcgg agagcccccg ggtgtgggcg 480
ctgccttgaa ctccttaccc cagctgcctg gctgccctca gcttcccaaa gctcaaataa 540
gaggggccgg cggcgcgggg aagaggagga gccaggaggc tcggccgctc cattcactca 600
gcagcccaag cccggccagg cagcgcgcgc tgctccttgg gggcgccctc gcagctgggg 660
tgagggcggg tccgagggag cgtccctctc ggagacaccc tcctcttcaa cccaacacgc 720
gctctcagct ggccggtgtg gcacaaccct ccccccacct ccccttcttg gccttcgcca 780
gtgtgggagc ctcgtctcac tggagagtcc gttcaggtaa gcagtgagtc cgggcagggt 840
cgaatgcggt gggagtagag atggggttgt taatctaaaa gagtggtttg gagggagggg 900
caggtaggct ccaagagggc gggaacttca ggaaagcgca gaaagaactc agtcaagctt 960
caaagaagcg tatacccttg ctttgcaccc ttgagtctgg ctcttgcttc tagaactgtc 1020
gggtagggga gtggccatga tcttggggtc actggggaaa gaggtggccc tttgtgggtt 1080
ccagaggtcg ggactgaaag cgaacgtccc tcagttgggt ggccgggagt tgcaccagtt 1140
ataagccggg cgtatttcga tcggctagta gttgtttact tggtgaggtt tgcgccaggc 1200
ggaaggcggt tgccgcctac ctaaaattgg agtcagcagc acttagtttc cctttccagt 1260
gaggaatatc taattcttct acggttaagc cgggaaaaca gcggctagtt taggagggaa 1320
gaaaaccctt gggcaaacgc ccaaagcctg cgtatattcc ggccggagca tcctggcgcc 1380
cagtttgggg aggaggggtg aaggggaggg tttggatacg aagcccggca gggagcgggt 1440
aactcctgga gccactccgc cgagcactgc ctgtggccct ggtcgaggat accattctgg 1500
ttgctcagtg attgaccagg aggcggaagc cagttaaggg gaccaaggag tggagtaggg 1560
agaacctcca tcggcccacg ggcgcgagtt agtgttcgaa tctttgggag ctcgaagagc 1620
ttcgctaaat ctgactccct tcgtctagtc tctgtttggc agcccccgcg ccccagagca 1680

ggtgtttggg gcgggtaggg ggtgttttgg tttggttttt ggatcttcta aaggagtcag 1740

tatctattgg gaaacaaccc ctaggcaagg agaagaaccg ggggccggag cggagcgtgt 1800
ttcagaggtg cgtataaaat cccaggggcg ccaggaggca gcagcgacgg cgcagcgtgg 1860
gacggccagg cgcgacggga gggaagcaac cggttgcagt ccggcctcct gtacctggct 1920
ggcccgggct ggggctgcaa gctttccagc caaacttatg tgaagcaaag tagttgcagt 1980
ttgacaaaga atggcgtttc aataaatgac atagtaattt tttacgcttt ggtttttagc 2040
ttgcattgca taaacgttgt cgccattaaa ttctggctgc ggacccgaag tgtgtgcggg 2100
cccaggactt actcgaaggg cgcacttctt gggaatgcgc atcccgtagg cgcaggagac 2160
cctgagggta aagatgcggc acttgagccc tccgcttgcc tcccaggcca cctttgctgc 2220
ttgactttgc gtcgtctcca ctcagaagct tttaaaatta ttattaagaa aagaaagatt 2280
ctacaagaca ctgcccccac gctctctgct cagtcctctc ccacacgatc tatccctaaa 2340
cctttcgcgg ggctcgcgcg tttggttttt tgagtggaga aaggactctc gagacaaaat 2400
tcactgccag ttgccctaag aaccccccag atccctgtat ctcccttcct tctccccgcc 2460
tattatttct acttggaagt cgtttttctt aactaggact 2500

<210> 47
<211> 2500
<212> DNA
<213> Homo Sapiens

<400> 47

tggtagaact ctcctttgtt aatcttttga ctactggtac aaaaatacag cattcaaata 60
tgtgaagtaa aagataatcc aggggctagc tgactatggc ttgagatcta aatacagccc 120
atggcttgtt ttgcatagcc attgagataa ggattttttt ccatttaaaa gtattattgc 180
taaaaaacac aacactacgc cacagaaact acatgaccca caaagactga aataattacg 240
atcagatctg ttaaagcaaa atttggtgat gccgtattaa tcaaatacct gttattctgc 300
tttgcattat tgcatacatc ttttcatcca ttaattaatt tatttacaca cggattgaaa 360
atatggatgt tactctatcc cctcaagaaa gcgtccagct ttatgatacc atgatttcag 420
tctgggaaac ttgtcccagg gatcaggtca atacacaaac atacatttga ggtaggagac 480
tggcaggact tgttttctgg tcataactga gcctagagcc tacagtcaga atctatggct 540
atttctgaaa catatactgt ctgaatgacc tacaggtata ttcagggaaa tcagttacca 600
gtccagagat ttaaatgtga agtaacacaa cctaaaaaga aaagagtaaa caggtgtgaa 660
tgtatttatt ctacagaaga atttattcaa ttacgtttat tggaagcatg gcagaaaacc 720
cagaaccaga gaacaaagtg attaataaaa gcatttataa aactggccaa ggtgggagga 780
tcacttgagc ccaggaaatt gagaccagca tgggcaacac agtgagacca ctatctaaaa 840
aataaataaa taataaataa accaattacc caggtgtggt gtctgcacct gtagtagcag 900
ggactgggga ggctacatcg ggaggattgt ttgagcccag gagatggagg cttcagtgag 960
gtatggtcat gccactgccc tccagcctga gcaacagagt gacactctgt ctggaaaaaa 1020
aaataaataa aaacggcttt ataaataaag atatgaaaat aatttatttc ttccacaaaa 1080
ggtcttcctg agacacatcc tccagtaagg gactgtctgt tcccaacgtt tctgaatgca 1140
tgagattgga caaagagaga cgaaagctct caagtccctt tttctactac aaacgccatg 1200
gggatgcggg tctgggaaca gcggaaaacc ctaccctgcc ctgaaaagtc cctggctcaa 1260
tgtgcatgtc cctttctatg aaagttcctt gctgcctatg cgccttgctc tctaacttct 1320
gtccctccac aactgtgaaa ggagacgtcg tgacttcctt ctttcgtgct gactatgact 1380
tagccagtag gtctgcagat cagtcctccc agaaagtgaa gtgtgagtga gtgtgagggg 1440
gagcgtgggg ggcttcctgt gggttgtgcc acttggtcca tggcctctga ggtcgtcatc 1500
tttctactaa aactcagcga tactgccatg gccttgctgt tgtggggtcc cgggcatggg 1560
acgaaggagg gccatagcgg ggaggagggt caggtgagca tggagtgagt tttttttttt 1620
ttttcacgga gtcttgctct gtcaccaggc tggagtgcag tggcgcaatc tcggctcact 1680
gtaacctccg cctcccgggt tcaagtgatt ctcctgcctc agcctcccga gtagctggga 1740
gtacaggcgc gcacaaccac acccagctaa tttttgtatt tttcagtaga gacgggtttt 1800
aaccatgttg gcgaggatgg tctcgatctc ttggccacat gatccacccg cctccgcccc 1860
ccaaagtgct gggattacag gcgtgagcca ccgcgcccgg tcatggagtg agttttgaag 1920
gctgacttta tttgaggtat ttgagtcctg gtggggggga acccacacac agagaggagg 1980
gattccaaag tcgttaatgg ggacctggga aggagcatag gacagggcaa ggcgggataa 2040
ggaggggcac cacagccctt aaggcacgag ggaacctcac tgcgcatgct cctttggtgc 2100
ccacctcagt gcgcatgttc actgggcgtc ttcccatcgg ccccttcgcc agtgtgggga 2160
acgcggcgga gctgtgagcc ggcgactcgg gtccctgagg tctggattct ttctccgcta 2220
ctgagacacg gcgggtaggt ccacaggcag atccaactgg gagttgaagt gtgagtgaga 2280
gtgaagagga accagcaggc ttccggaggg ttgtgtggtc agtgactcag agtgagaagg 2340
ccctcgaagt cgtcgtccct ctcatgcggt gccacgccca tggaccttct tgtctcgtca 2400
cggccataac tagggaggaa ggagggccga ggagtggagg ggctcaggcg aagctggggt 2460
gctgttgggg gtatccgagt cccagaagca cctggaaccc 2500

<210> 48
<211> 2501
<212> DNA
<213> Artificial Sequence

<220>
<223> chemically treated genomic DNA (Homo sapiens)

<400> 48

gattaatatg taataggagg tgagaattaa tattatttta atgtagataa agataatatt 60
ggttaagtgt taatattggt ttttaaaaat gggtaattta ttttattttt attttgttta 120
tgtatttgag gtattatgtg tacgttggta gtatttttat tatttttaaa gtaatgatgt 180
tgtttaggaa tattaaatat cgtttttttg aaatatttat gaaatttaaa atattttatt 240
ttagatgtag taatatgtgt tatagagtaa tattttaatt taataaattc ggttttaaaa 300
taaattattt tgggtcgggt gtagtggttt acgtttgtaa ttttagtatt ttgggaggtc 360
gaggagggtg gattatttga ggttaggagt tttagattag tttggttaat gtagtgaaat 420
tttgtttgta ttaaaaatat aaaaattagt tgggcgtggt ggtaggtgtt tgtaatttta 480
gttatttagg aagttgaggt aggagaatta tttgaagtta gtaggcggaa gttgtagtga 540
gtggaaattg tgttattgta ttttagtttg ggcggtagag tgagatttta ttttaaaaaa 600
taaaaataga aaaataaaat aaaattattt tggttgggta tagtggttta aggttgtaat 660
tttagtattt tgggaggttg aggtgtgtgg attatttgag gttaggtttt tgagattagt 720
cgggttaata tgttgaaatt ttgtttttat aaaaaaatat aaaaattagt tgtatggtgt 780
tatgtatttg tagtcgtagt tatttgggag gttgaggtta gagaatcgtt tgaatttggg 840
aggtaaatat tatagtgggt cgagatcgcg ttattgtatt ttagtttggg taatagaatg 900
agattttatt ttagagagag agagagatat tataaattaa ttattttttt taaattagtt 960
agaatatttt tttattttgt tttttttttt ttattgaatt aataattgta gaatatttat 1020
atattgatta tatttttaat attttttgtt atttttttcg attaggaatt tatagatttt 1080
ttttgttaag attttaattt gtgtttttgt gaaagatagg aagattgaga tgttgaaata 1140
atttaaaata gttaaggttg gtcgggcgcg gtggtttacg tttgtaattt tagtattttg 1200
ggaggtcgag gcgggcggat tacgaggtta ggagatttag attacggtga aattttattt 1260
ttattaaaaa tataaaaagt tagtcgggcg tagtggaggg cgtttgtagt tttagttatt 1320
cgggaggcgg aggtaggaga atggcgtgaa ttcgggaggc ggagtttgta gtgagtcgag 1380
atggagttat tgtattttag tttgggtgat agagtaagat ttcgttttaa aaaaaaaaaa 1440
aaaaaaaaaa aaaaaaaaaa aaaaaaaaaa aaaaaattta aggttaaggt atatatagtg 1500
aaatttttat tatttgtata taaaaaattt gtatttgttt aaaaattgat ttttttgtta 1560
tttggttttt tttttttttt attttgtttt aattatgatg tgttttaaaa tatagtatgt 1620
gttaattttt ttttgagatg gagttttatt tttgttgttt aggttgtagt gtaatggtac 1680
gattttggtt tattgtaatt tttgtttttc ggatttaagg gatttttttt ttttattttt 1740
ttaagtagtt tagattatag gtatgtatta ttatttttgg ttaatttttg tatttttagt 1800
agagataggg ttttattata ttggttaggt tggtttcgaa tttttgattt taagtgattt 1860
attcgttttg gttttttaaa gtgttgagat tataggtttg agttattgtg ttcggttttt 1920
gttatttttg aataatattt tttagttttt gtggaaatta tttttaaagt gtttttgaag 1980
tatttagttt ttgtttttta ggatatgtat ttgtagatag gatttaaagt agttaagaat 2040
gtttttgtta agtcgataaa tagttattat tgttaatttt tttttattag aatatataaa 2100
tattaagaaa aatgggcgat tgataaatta attttagtat ttataaaaag ttgttttaaa 2160
ggatttatgg aagtatagtt ttttatggtt tttttaacgt tttgtggatg ttgtgaaatt 2220
atagttgttt gtatgtatgt ttttaattat gttagtttta aaaaaagaaa atttttgtaa 2280
atttttgtaa gatataatta gtttttaata ttataaaata tatttggtta ggcgcgttgg 2340
ttttcgttta taattttagt attttgggag gtcgaggtgg ggagattatt tgaggtcggg 2400
agttcgagat tagtttgatt aatatggaga gatttcgttt ttattaaaaa tataaaatta 2460
gtcgggcgtg gtggtatatg tttgtatttt tagttattta g 2501

<210> 49
<211> 2501
<212> DNA
<213> Artificial Sequence

<220>
<223> chemically treated genomic DNA (Homo sapiens)

<400> 49

ttgggtagtt ggaagtatag gtatgtgtta ttacgttcgg ttaattttgt atttttagta 60

gagacggggt ttttttatgt tggttaggtt ggtttcgaat tttcgatttt aggtgatttt 120
tttatttcgg ttttttaaag tgttgggatt ataggcggga gttaacgcgt ttggttaaat 180
atgttttata atattaaaga ttagttatgt tttatagaaa tttataggag tttttttttt 240
ttgaaattaa tatagttgaa gatatatata taggtaattg tagttttata gtatttatag 300
gacgttgaaa gggttatgaa aaattatgtt tttataaatt ttttaggata attttttgta 360
gatattgaag ttgatttgtt aatcgtttat tttttttagt atttatatat tttggtggag 420
gagggttaat aataatagtt gtttgtcgat ttggtagaga tatttttaat tgttttgaat 480
tttgtttata agtatatgtt ttgaaaaata aaaattgagt attttagaag tattttagga 540
atggttttta taggagttga aaagtgttat ttaagagtaa tagaagtcgg gtatagtggt 600
ttaggtttgt aattttaata ttttgggagg ttaaggcggg tggattattt gaggttagga 660
gttcgagatt agtttgatta atgtagtgaa attttgtttt tattaaaaat ataaaaatta 720
gttagaggtg gtagtgtatg tttgtaattt aagttattta ggagggtgag gagggagaat 780
tttttaaatt cgggaggtag aggttatagt gagttaagat cgtgttattg tattatagtt 840
tgggtaataa gaatgaaatt ttattttaaa aaagagttaa tatatattgt gttttaaagt 900
atattatgat taagatagga tgaaaaaaaa aaaaagttaa atggtaaagg aattaatttt 960
taaataaata taaatttttt atatataaat agtaaaagtt ttattatata tgttttagtt 1020
ttggattttt tttttttttt tttttttttt tttttttttt tttttttttt tttgagacgg 1080
agttttgttt tgttatttag gttggagtgt agtggtttta tttcggttta ttgtaagttt 1140
cgtttttcgg gtttacgtta tttttttgtt ttcgtttttc gagtagttgg gattataggc 1200
gttttttatt acgttcggtt aattttttgt atttttagta gagatggggt tttatcgtgg 1260
tttagatttt ttgatttcgt gattcgttcg tttcggtttt ttaaagtgtt gggattataa 1320
gcgtgagtta tcgcgttcgg ttaattttgg ttgttttaaa ttattttaat attttagttt 1380
ttttgttttt tataaaaata taaattgagg ttttagtagg aaaagtttat aaatttttag 1440
tcggaagaaa taatagagaa tgttaaagat atgattagtg tgtaagtatt ttataattgt 1500
taatttaatg aagagagaaa agtaaagtaa aaaaatgttt tggttaattt gaaaaaagtg 1560
attaatttat ggtgtttttt tttttttttg agatggagtt ttattttgtt gtttaggttg 1620
gagtgtaatg gcgcgatttc ggtttattgt aatgtttgtt ttttaggttt aagcgatttt 1680
ttagttttag ttttttaagt agttgcgatt atagatgtat gatattatgt agttaatttt 1740
tgtatttttt tgtagagata gggttttaat atgttggttc ggttggtttt aaaaatttga 1800
ttttaggtga tttatatatt ttagtttttt aaggtgttga gattatagtt ttgagttatt 1860
atgtttagtt aaagtgattt tattttattt ttttgttttt attttttgag atggagtttt 1920
attttatcgt ttagattgga gtgtagtggt ataattttta tttattgtaa ttttcgtttg 1980
ttggttttaa gtgatttttt tgttttagtt ttttgagtag ttgggattat aggtatttgt 2040
tattacgttt agttaatttt tgtattttta gtataggtag ggttttatta tattggttaa 2100
gttggtttgg aatttttgat tttaggtgat ttattttttt cgatttttta aagtgttggg 2160
attataggcg tgagttattg tattcgattt aaagtgattt attttaaaat cgaatttatt 2220
agattaaaat gttattttgt ggtatatatt gttatattta gaataaaata ttttaaattt 2280
tataggtgtt ttagaaagac ggtatttaat atttttagat agtattatta ttttaaggat 2340
aataaaggta ttattaacgt gtatataata ttttaagtat ataagtaaaa tgaaaatgaa 2400
ataagttgtt tatttttaga gattagtgtt aatatttagt taatattatt tttgtttgta 2460
ttaaagtagt attagttttt attttttgtt gtatattaat t 2501

<210> 50
<211> 2501
<212> DNA
<213> Artificial Sequence

<220>
<223> chemically treated genomic DNA (Homo sapiens)

<400> 50
   tttttgggtt gtagtgtagt atattagtta ggtgtttgta ttaagttttg tattaatttg 60
   gttgtttaag gatggttgaa ttgatttagg tggaaattgg agtaggttaa aatttaatgt 120
   tgattagtaa tagggtcgta tttgtaaata gttatcgttt tttagtttgg gtaatatagt 180
   gagattttat ttttaaaata aattttaaaa ataattaatt agaaaaaaaa attagtttgt 240
   aattttagta ttttgggagg ttaaggcggg tagattattt gaggttagga gtttaagata 300
   agtttggtta atatggtaaa attttaattt ttattaaaaa tataaaaatt agttgggcgt 360
   ggtggtaggt gtttgtagtt ttagttattt aggaggttga ggtaagagaa tcgtttgaat 420
   ttgggaggtg gagattgtag tgggtcgaga tcgcgttatt ttattttagt ttgggtagta 480
   gagtgcgatt ttgttttaaa aaaaaaaaaa aagaaagaaa agaaaagaaa aagaaaagaa 540
   aagaaaaaaa ttgggaggtt taagtttatt tttggtgttt ttatattttt ttgttttatt 600
   tttttgtatt tagttttttt ttgtaatttg tgttttatat tagtttttaa gtttttaacg 660
   tgatttagta tgagaattgg attttgttat tttttgtttt ataatatttt atgttttttt 720
   tttgtttaga atattatttt ttttattgtt tttattaatg gaattggtat ttttttaagg 780
   atatgattaa atttgtttat ttttatatta ttttttaaag tagaatttat tttttttttt 840
   ttaatatgat gatattgata gggtttgttt ttatttatta gattgtgagt tgtttagggt 900
   aggtagcgtt ttttgttttt gtttttgttt tttttttttg agatagggtt ttgttttgtt 960
   atttaggtta gagtgtaatg gtatagtttt agtttattgt agttttaatc gtttcggttt 1020
   aaattattat tttattttag ttttttgagt agttgggatt ataggtatat gttattatat 1080
   ttggttaatt tttttgtatt tttagtagag atagggtttg gttatgttgt tcgggttggt 1140
   ttcgaatttt tggatttaag taatttattt attttagttt tttaaaatga gggatcgtgt 1200
   tttatttatt tttatgtttt tagtttatag tttagtgttg gatttatggt agtattaaat 1260
   aaatatttgt tgaatgtaat agtaaatagt attttaggga gtaagaatta gattaataaa 1320
   ggtggtaaaa ggtttggaga aaaaaataat agtttaattt ggttagagta tgagggagag 1380
   tagtaggaga taagatggaa aggttttttg ggtaaggttt tgaaggaagt tggaagttag 1440
   aagtatataa tgtgtatatc gtggtaggta gtggggagtt aatgaaggtt tttgagtagg 1500
   agagtaatgt gttgaaaaat aaatataggt taaatttatt agagtttttt tgatatatat 1560
   atttgttttt tatttaagtt taagtttgtt ttttatatat ttattattta atttattttc 1620
   gggttttttt agtagtttgt tttatttttt tatttgtttt ttggtggagt tagggatgta 1680
   tatatgagtt gttttttttt ttagttagag gatatggggg ttttagtttt tttgtttttt 1740
   tttttttgtg tttggagttg ggaagtaggt tagggttagt tgaggttggt tggtaagtag 1800
   ttgggtggtg ttagggagag tttgtatagt gttaggtggt gttttgggtt ttaagttgag 1860
   tttatggttt cgataatttt ttgtttgtgt atatatttgt tttttatttt atttttattt 1920
   tagttttggt atgggggaga gggtataggg ttagataaat ttgtgagatt ttggttttat 1980
   ttttgtaaaa gggcgttttg tgagttagtt tgtttttttt taggtttgtt ttttttttat 2040
   ttagttttcg tttttaatgt acgtatagtt cgtatatatc gtgtgttggg atattttata 2100
   gttagtcgta tggttttttt gtgttttagt ttttggtttt ttttgttgat ttcggttttt 2160
   gttttaggtt ttattgtgta attgttgttg ttattgttgt ttttggtgtt tgtttatttt 2220
   tagaggttgt ttcggatgta ggaggatttt tttttgggag gaggtttttt tggggaagat 2280
   gatttattgg gcgaggagga tttgtttagt gaagaggatt tatttagaga ggaggattta 2340
   ttcggagagg aggatttatt tggagaggag gatttatttg gagaggagga tttatttgaa 2400
   gttaagttta aattagaaga agagggtttt ttgaagttag aggatttatt tattgttgag 2460
   gtttttggag atttttaaga attttagaat aatgtttata g 2501

<210> 51
<211> 2501
<212> DNA
<213> Artificial Sequence

<220>
<223> chemically treated genomic DNA (Homo sapiens)

<400> 51

ttgtgggtat tattttgggg tttttgagga tttttaggag ttttaatagt aggtagattt 60
tttaatttta gggagttttt tttttttgat ttaggtttaa ttttaggtag attttttttt 120
ttaggtagat tttttttttt aggtagattt tttttttcgg gtggattttt ttttttgggt 180
gaattttttt tattgggtag atttttttcg tttagtgggt tatttttttt agaagagttt 240
ttttttaagg gggaattttt ttgtattcgg ggtaattttt ggggatggat aggtattaga 300
agtagtagtg atagtagtag ttgtatagtg aggtttggag taggggtcgg gattaataga 360
gggagttagg ggttggggta taggggagtt atgcggttga ttgtggggtg ttttagtata 420
cggtgtgtac gggttgtacg tgtattggaa acgagagttg ggtgggggag gagtaagttt 480
ggaggggagt aggttgattt atagagcgtt tttttgtaga gatggagtta aagttttata 540
ggtttgtttg gttttgtgtt ttttttttta tattaaagtt aggatggggg tggagtgagg 600
ggtaggtgtg tgtataggta gaaggttatc ggggttatgg atttagtttg gaatttaagg 660
tattatttgg tattatgtag gttttttttg gtattattta gttgtttgtt agttagtttt 720
agttaatttt ggtttgtttt ttagttttag gtatagaagg ggaaaggtag gggagttggg 780
gtttttatgt tttttggttg agagggaaag tagtttatgt atatattttt gattttatta 840
ggaagtaggt aaagaggtag ggtaggttgt taggggagtt cgagggtgag ttaagtaatg 900
ggtatgtggg agataaattt gagtttgaat gaaaagtaag tgtatgtgtt agaggggttt 960
tgataggttt aatttatatt tattttttaa tatattattt ttttgtttaa aagtttttat 1020
tggtttttta ttgtttgtta cgatatgtat attgtgtatt tttgattttt aatttttttt 1080
aaaattttgt ttaagagatt tttttatttt gttttttatt attttttttt atattttagt 1140
taaattaaat tattattttt ttttttaaat tttttattat ttttgttaat ttagtttttg 1200
ttttttgaaa tgttatttat tattgtattt aataaatatt tatttagtat tattataggt 1260
ttagtattgg gttatggatt agggatatgg aaatgaataa gatacggttt tttattttgg 1320
gaggttgagg tgggtggatt gtttgagttt aggagttcga gattagttcg ggtaatatgg 1380

ttaaattttg tttttattag aaatataaaa aaattagtta ggtgtaatgg tatgtgtttg 1440
tagttttagt tatttaggag gttgaaatgg gatgatggtt tgagtcgagg cggttgaggt 1500
tgtagtgagt tgagattgta ttattgtatt ttggtttggg tgatagagta agattttgtt 1560
ttaaaaaaga aaaataaaaa taaaaataaa aaacgttatt tgttttgagt agtttataat 1620
ttagtgagtg agggtaaatt ttgttaatat tattatattg agggaagaga gatgaatttt 1680
gttttagaag ataatataga agtaggtaaa tttgattatg tttttgaaga aatattagtt 1740
ttattggtaa aaataatagg gagaatgata ttttgggtaa agggaaggta taaaatatta 1800
tgagtaggag agtgatagaa tttagttttt atattaggtt acgttgaggg tttgagggtt 1860
agtatggagt atagattgta ggggaagatt gggtgtaagg agatggggta gaagagtgtg 1920
aggatattaa gaatgggttt aggtttttta gttttttttt tttttttttt tttttttttt 1980
tttttttttt tttttttttt ttttgagata gagtcgtatt ttgttattta ggttggagtg 2040
aagtggcgcg atttcggttt attgtaattt ttatttttta ggtttaagcg atttttttgt 2100
tttagttttt tgggtagttg ggattatagg tatttgttat tacgtttagt taatttttgt 2160
atttttggta gagattgggg ttttgttatg ttggttaggt ttgttttgaa tttttgattt 2220
taggtgattt gttcgttttg gttttttaaa gtgttgggat tataggttgg tttttttttt 2280
taattaatta tttttaaaat ttattttaga gatgggattt tattgtgttg tttaggttgg 2340
agggcggtgg ttatttatag gtgcgatttt gttattgatt agtattgagt tttgatttgt 2400
tttaattttt atttgggtta gtttaattat ttttaggtaa ttaggttgat gtagaattta 2460
gtgtagatat ttgattggta tattatatta tagtttagaa a 2501

<210> 52
<211> 2501
<212> DNA
<213> Artificial Sequence

<220>
<223> chemically treated genomic DNA (Homo sapiens)

<400> 52

gttcgttgga ttatttaaat ttgttttgtt ttcggttgga gtttgatgtt atttagtggt 60
agacgttgtt ttggttgaaa tattgatgga gattttcggt ttagtggttg gttgagattt 120
ttaggatagt ggtagtattt ttaggaattt tttaattagt taggattttt ttttttgatt 180
tgatttgagg ataataggtc gggaaaataa tggggattcg gagatttttt ttttgttttt 240
ttgtttttta agatagggtt atatgtattt tatttttttt tttttgattt gttgaaggta 300
agaattttag tttgtttttg tttgagaatg gggagattga gtagaggacg ataggaagga 360
tggtaattat taatcgggtt atgtttgatt tttagttatt ttaggtgtta ataagttgtg 420
ttttttggtt tttaaaattt attatttaat gaaatatatg taatgaaata tagtagtgaa 480
aataataaat tggaggtata cgtaagagtt tgggtgaatt tttttttttt gtaagtaaaa 540
atatttttaa ttgatgtatg ataattttgt atatttatgg gatatatgtg atattttgat 600
atatgtatac gtatatacga tgtaatgatt aagtcggggt atttaggatt tttattattt 660
taagtattta ttattttttt ttttggggaa tattttttat tttttagtta ttttgaaata 720
tataataaat aattatttat agttttttta cggtgttatt aaatattaga atttaatttt 780
tttatgtaat tatatgtttg tttttattaa tttttttatt ttttttattt tttttagcgt 840
ttggtaatta ttattttgtt ttatttttaa aagattaacg tttttagttt ttatatgagt 900
gttaatatgt tttttttagg tgtagaggtt gttgattttt ggatttggaa ggtgggggag 960
agtggtttgg attttagtat tttttttttt tttagggtta ggttttgttt ggttattttt 1020
tgttgttata ggtgtgtttg gtatagatat ttagttttcg gggtcgtgtc gtttttattt 1080
ttgtatgttt ttagaggtat ttgtttcgtg gatttgattt ttttttagat atataaatag 1140
attattattg agtagatata gggttatttt ttagtttttt attaggtaag agcgattagg 1200
gtagggattg atattgtcga atttaggagt taggttcgat ttagttttag gtttagtagg 1260
tttcgtttgt ttatttgggt taggtttaga gttcgggagt ttttggttgg tgggaggtta 1320
ttcgtaattt attttatacg tagttttagt ttttttatta ggcggggcga ttaggatagg 1380
gatagaattc gttgaattta ggagtgagat tcggtttcgg gtttcgttgg gttttttcgt 1440
ttattttggt tggatttggc gtttgggaga ttttggttgg cgcgaggtta cgtttattag 1500
atatgtagtt ttagttattt tattagttgg gcgattagga tagggacgga ggttgttgag 1560
tttagttaga ggtttgtttt tcgggttttg ttttgggcgt ttttttaagg acggataggg 1620
taggtagggt tcgggacgat ggtcgtatag tttcggtttc gtgtttttag gttcgttttg 1680
tttttcgatg tgagggagat tcgggggatg ggataggttg ggtttcgtag tgtttgattt 1740
tttgtagggt tttcgggata ggggttcggc ggatagtcgg ttgtttaggg gtgaggggtt 1800
taagttggta ttgcggttat ttttcggttc gggttttttt ggggaggggc ggggttggtg 1860
agaatcggtt acgtgtttcg gggtttattc ggggtttttt agggtcggaa gtagggtttt 1920
tgtgcgtagg cgttttgagg atttcgggtt gtttatttta cgttaggggg cggaattttt 1980
tgtagttttt ttgttttcgt attttcgtgg gttttgattt tttttttgag agtcgggtag 2040

aggtttcgga gttatgtagg tcgaaggtcg gggtataggg ggttcgacgg gcgatgttga 2100
tggtttagga ggttttggta tttttgatgg tttagggggt aatgttggcg gtttaggaga 2160
ggcgggtgtt acgggcggta gaggttttcg ggcgtagggg gtagtaaggg tttcggggtc 2220
gggaggaggc gtttcgcggg gttcgtatgg cggcgcggtt ttagggttga atggatgttg 2280
tagatgcggg gttagggggt cggagagtcg tttgtttgag ttgtattttg ttttgttttg 2340
ttttgttttg atagttttgg tggcgaggtg ggggtcggga gatggggagg gtagggttag 2400
gtgggggagg aggcggggga gatgcgagta agtggttggg tgggggtggg ggtgggggtt 2460
gggaggtggg gatatgagag gttagttgcg aggaggggag g 2501

<210> 53
<211> 2501
<212> DNA
<213> Artificial Sequence

<220>
<223> chemically treated genomic DNA (Homo sapiens)

<400> 53

tttttttttt tcgtagttgg ttttttatat ttttattttt taatttttat ttttattttt 60
atttaattat ttattcgtat ttttttcgtt ttttttttta tttggttttg ttttttttat 120
ttttcggttt ttatttcgtt attagaattg ttagaataga atagaataga atagaatata 180
atttaagtag gcggtttttc ggttttttgg tttcgtattt gtagtattta tttagttttg 240
aagtcgcgtc gttatgcgga tttcgcgggg cgtttttttt cggtttcgag gtttttgttg 300
tttttgcgtt tcggggattt ttgtcgttcg tggtattcgt ttttttgggg tcgttagtat 360
tgttttttgg gttattagga atgttagggt tttttgggtt attagtatcg ttcgtcgaat 420
tttttgtgtt tcggttttcg gtttgtatgg tttcggagtt tttgttcggt ttttagagag 480
aaggttaggg tttacgagga tgcggaggta gagaggttgt aggaagtttc gttttttggc 540
gtgagatggg tagttcggga tttttagggc gtttgcgtat aggggtttta ttttcggttt 600
tgggagattt cgagtgagtt tcggagtacg tgatcggttt ttattaattt cgtttttttt 660
taagagagtt cgggtcggaa ggtggtcgta atgttagttt ggatttttta tttttgagta 720
gtcggttgtt cgtcggattt ttgtttcggg agttttgtag ggagttaggt attgcggggt 780
ttagtttgtt ttatttttcg ggtttttttt atatcgagga gtaagacggg tttgggaata 840
cggggtcggg attgtgcggt tatcgtttcg gattttgttt gttttgttcg tttttggggg 900
agcgtttagg atagatttcg gggggtaggt ttttaattgg gtttagtagt tttcgttttt 960
gttttggtcg tttagttggt ggggtagttg gaattgtatg tttggtgggc gtggtttcgc 1020
gttagttaag gttttttagg cgttaggttt agttaaggtg gacgggaggg tttagcggga 1080
ttcggggtcg gattttattt ttgggtttaa cgggttttgt ttttgtttta gtcgtttcgt 1140
ttggtggggg agttggagtt gcgtgtgggg tgggttgcgg gtggtttttt attagttagg 1200
ggttttcggg ttttaggttt ggtttaggtg gataggcggg atttgttgga tttgaggttg 1260
ggtcgggttt ggtttttggg ttcggtagta ttagtttttg ttttggtcgt ttttgtttgg 1320
tagggagttg ggaggtgatt ttgtgtttgt ttagtggtga tttatttgtg tgtttggaag 1380
ggggttaaat ttacgggata ggtattttta gaggtatgta ggggtgaggg cggtacggtt 1440
tcgggagttg aatgtttgtg ttaggtatat ttgtagtaat aggaggtgat tagatagggt 1500
ttagttttga ggaaggggga ggtattgaaa tttaaattat ttttttttat tttttaaatt 1560
taggaattag tagtttttgt atttgggaaa agtatattgg tatttatgtg gaaattaaaa 1620
acgttgattt tttggaggta gagtagaatg atggttatta gacgttggga aaggtgggga 1680
gggtggggag gttaatgggg ataaatatat agttgtatag aaggattaag ttttagtgtt 1740
tgatagtatc gtagagggat tataggtaat tatttattgt atattttaaa atagttaaaa 1800
gataggaaat gttttttaaa aaaagaaatg ataaatgttt gaggtggtgg agattttaaa 1860
tatttcgatt tgattattat atcgtgtgtg cgtgtgtatg tattagaata ttatatgtat 1920
tttataaata tgtagagtta ttatatatta gttaaaaatg tttttattta tagaaaagaa 1980
aaatttattt aagtttttgc gtgtattttt agtttattat ttttattgtt gtattttatt 2040
atatatattt tattaaatga tgaattttaa aaattagaga atataatttg ttaatatttg 2100
gaatagttga aaattagata taattcggtt agtgattgtt attttttttg tcgttttttg 2160
tttagttttt ttatttttag atagaggtag gttaaagttt ttatttttaa taggttaggg 2220
aagggagagt aaagtgtatg tgattttgtt ttaggaagta ggaagataga gggagggttt 2280
tcgggttttt attatttttt cggtttatta tttttagatt aagttaagaa aggaagtttt 2340
aattgattga gagatttttg gagatattgt tattgttttg gaagttttag ttagttattg 2400
gatcggaaat ttttattaat attttagtta gagtaacgtt tgttattaga tggtattaaa 2460
ttttagtcgg gagtaggata gatttggatg gtttagcggg t 2501

<210> 54
<211> 2501

<212> DNA
<213> Artificial Sequence

<220>
<223> chemically treated genomic DNA (Homo sapiens)

<400> 54

gtattatttt ttttaatttt agtatagtat gttagatgtt gttttataga tgagttaatt 60
gagattagag atgtttagtt atttgtttaa ggtgatatga ttgatatgga atagagttaa 120
gatttttttt tttttttttg atacggagtt ttattttgtt ttttaggttg gagtgtagag 180
gcgtaatttt agtttattgt aagttttgtt ttttaggttt acgttatttt tttgttttag 240
ttttttgagt agttgggatt ataggtattc gttattatat ttggttaatt ttttgtattt 300
ttagtagaga tagggtttta tcgtgttagt taggatggtt tcgatttttt gatttcgtga 360
tttgtttgtt tcggtttttt aaagtgttgg aattataggt gtgagttatc gcgattggtt 420
agatttaaga tttgaattta ggtttttttg gttttagagg tttttgtttt ttaatttttt 480
aggatggtat agtaatttgt tttataagag gtgtttgttt taagtgtgtt tagtatatgg 540
aagtaagttt agaaatgtaa gtgtatattt gtaaagaggt gtgggagatg ggggggaggg 600
aagagagaaa gagatgttgg tgttttttat tttttagttt ttgataggtg tttttgattt 660
ttttttgatt agtatagttg tatttttggt tggggtattt taattagaat tgttaaattt 720
agtatataaa aataaggagg tttagttaaa tttgaatttt agataaataa tgaataattt 780
gttagtataa atatgtttta tgtaatattt tgttgaaatt aaaaaaaaaa aaaaaagttt 840
tttttttatt tttattttta ttattaggtt taaggaatag ggttaggggt tttaaataga 900
atgtggttga gaagtggaat taagtaggtt aatagaaggt aaggggtaaa gaagaaattt 960
tgaatgtatt gggtgttggg tgttttttta aataagtaag aagggtgtat tttgaagaat 1020
tgagatagaa gtttttttgg gttgggtgta gttgttcgtg gttgtaattt tagtattttg 1080
ggaggttgag gcgggaggat tatttgaggt tgggagttta agattagttt tattaacgtg 1140
gagaaatttt gtttttatta aaaatataaa aaattagttg gttatggtgg tatatgtttg 1200
taattttagt tgttcgggag gttgaggtag gagaattatt tgaattaggg aggtagaggt 1260
tgtggtgagt agagatcgcg ttattgtttt ttagtttggg taataagagt aaaagttcgt 1320
ttaaaaaaaa aaaaaagttt tttcgatgtg attgtttttt tttaaatttg tagatttttt 1380
taagattatg ttttttagat attttaaaga ttttagaaga tatgtttcgg gggttttgga 1440
agttataagg taaatataat atattttttt ttttgattat taattttatt agaggatgtg 1500
gtgggaaaat tattatttga tattaaaata aataggtttg ggatggagta ggatgtaagt 1560
tttttaggaa agtttaagat aaaatttgag atttaaaagg gtgttaagag tggtagttta 1620
gggaatttat ttcggatttc gggggagggg gtagagttat tagtttttgt atttagggat 1680
ttttcgagga aaagtgtgag aacggttgta ggtaatttag gcgtttcggc gttaggaggg 1740
acgtatttag gtttgcgcga agagagggag aaagtgaagt tgggagttgt tatttttaga 1800
tttgttggaa tgtagttgga gggggcgagt tgggagcgcg tttgttttta attataggag 1860
aaggaggagg tggaggagga gggttgtttg aggaagtata agaatgaagt tgtgaagttg 1920
agattttttt ttattgggat cggagaaatt aggggagttt ttcgggtagt cgcgcgtttt 1980
tttttacggg gttttttatt gcgtcgcgcg ttcggttttt atttttcgta gtatttcgcg 2040
tttcgcgttt ttttagtcgg gtttagtcgg agttatgggg tcggagtcgt agtgagtatt 2100
atggagttgg cggttttgtg tcgttggggg ttttttttcg tttttttgtt tttcggagtc 2160
gcgagtattt aaggtgggtt tggtgtgggg aggggacgga gtagcggcgg gattttgttt 2220
tgtggatgtt tcgtcgaggt ttcgcggtcg gcggggttag aggggttcgg acgagttttt 2280
ttatttcgaa gttgtggata gtcgagacgt ttagggtagt cgggttttgg ggttttcggg 2340
cgggaggggg tagttatacg gtagcggttc gagatggttt atttaagaga ttggcgtttt 2400
ttaggtttcg aggggtttcg ggaatttgtt aaagaagttt tttgaaattg tttagaaagt 2460
tttttcgtaa agggtgtatt gcgtagagcg cgcgcgcgcg t 2501

<210> 55
<211> 2501
<212> DNA
<213> Artificial Sequence

<220>
<223> chemically treated genomic DNA (Homo sapiens)

<400> 55

acgcgcgcgc gcgttttacg taatatattt tttgcgggaa aattttttga ataattttag 60
agaatttttt tgataagttt tcggagtttt tcggagtttg gaaagcgtta gttttttgga 120
tgggttattt cgagtcgttg tcgtgtaatt gttttttttc gttcgagggt tttagggttc 180

ggttgttttg agcgtttcga ttgtttataa tttcgggata ggagagttcg ttcgggtttt 240
tttggtttcg tcggtcgcgg gatttcggcg gggtatttat agggtagggt ttcgtcgttg 300
tttcgttttt tttttatatt agatttattt tgggtgttcg cggtttcggg gggtaagagg 360
gcgaggagga gtttttagcg gtataaggtc gttagtttta tggtgtttat tgcggtttcg 420
gttttatggt ttcggttgga ttcggttggg agggcgcggg gcgcggggtg ttgcgagggg 480
tgggggtcgg gcgcgcggcg tagtaaaggg tttcgtggga aggggcgcgc ggttgttcgg 540
ggggtttttt tggttttttc ggttttaatg gaggggaatt ttagttttat aattttattt 600
ttatattttt ttaagtagtt tttttttttt attttttttt ttttttgtga ttgggagtaa 660
gcgcgttttt agttcgtttt ttttaattgt attttaataa gtttgggagt ggtaattttt 720
agttttattt tttttttttt ttcgcgtagg tttgggtgcg ttttttttag cgtcgggacg 780
tttgggttgt ttgtagtcgt ttttatattt tttttcggag aatttttaaa tgtagaggtt 840
ggtgattttg tttttttttt cggagttcgg gataaatttt ttaggttgtt atttttaata 900
tttttttaag ttttaggttt tattttaaat ttttttgggg agtttgtatt ttattttatt 960
ttaagtttat ttgttttaat attaaataat ggttttttta ttatattttt tagtaaaatt 1020
gatagttaag gagggggatg tgttgtgttt attttgtggt ttttaggatt ttcggggtat 1080
attttttgga atttttgaag tatttgaaaa gtatgatttt aagagggttt ataaatttgg 1140
gaggagatag ttatatcgaa aggatttttt ttttttttta aacgaatttt tgtttttgtt 1200
gtttaggttg gagagtaatg gcgcgatttt tgtttattat aatttttgtt tttttggttt 1260
aagtgatttt tttgttttag tttttcgagt agttgggatt ataggtatgt gttattatga 1320
ttagttaatt ttttgtattt ttagtaaaga tagggttttt ttacgttggt gaggttggtt 1380
ttgaattttt aattttaggt gattttttcg ttttagtttt ttaaagtgtt ggaattataa 1440
ttacgagtaa ttgtatttag tttaaaaaga tttttatttt aattttttaa aatgtatttt 1500
ttttgtttat ttaaggaggt atttagtatt taatgtattt aaggtttttt ttttgttttt 1560
tgttttttat tagtttgttt aattttattt tttaattata ttttatttgg agtttttgat 1620
tttatttttt aggtttagtg gtaggggtgg ggatggaagg aagatttttt tttttttttt 1680
taattttaat aagatattgt atgggatata tttatattaa taaattattt attgtttatt 1740
tgaaatttaa atttaattgg gtttttttat ttttatgtgt taaatttggt agttttagtt 1800
ggaatgtttt agttaagaat gtagttatat tggttaagaa gggattaaag gtatttatta 1860
gggattggag aatgaaggat attagtattt tttttttttt tttttttttt ttatttttta 1920
tattttttta taggtatata tttgtatttt taaatttgtt tttatgtgtt gagtatattt 1980
aaagtaggta ttttttgtgg gataggttgt tatgttattt tagggagttg agaaataggg 2040
gtttttggga ttaagaggat ttgggtttaa attttgaatt tggttagtcg cggtggttta 2100
tatttgtaat tttagtattt tgggaggtcg aggtaggtag attacgaggt taggagatcg 2160
agattatttt ggttaatacg gtgaaatttt gtttttgtta aaaatataaa aaattagtta 2220
ggtgtggtgg cgggtgtttg tagttttagt tatttaggag gttgaggtag gagaatggcg 2280
tgaatttggg aggtagagtt tgtagtgagt tgagattgcg tttttgtatt ttagtttggg 2340
agatagagtg agatttcgtg ttaaaaaaaa aaaaaaaaat tttgatttta ttttatatta 2400
gttatgttat tttgggtaag tgattgagta tttttggttt tagttggttt atttgtaaaa 2460
tagtatttga tatattatgt tggggttaaa ggagataatg t 2501

<210> 56
<211> 2501
<212> DNA
<213> Artificial Sequence

<220>
<223> chemically treated genomic DNA (Homo sapiens)

<400> 56

attttttgttt attgtaattt cgttttttcgg atttaagtaa ttgtttgttt tagtttttcg 60
agtagttggg attataggta tttattagta cgtttggtta agttttgtgt ttttattaga 120
gatgggtttt cgttattttg gtcggggtgt ttttgaattt ttgatttcgt gatttatttg 180
ttttggtttt ttaaagtgtt gggattatag gtatgagttt tcgcgttcgg tggagatata 240
atttttatat attggttttt tatttagtgg ttttgtgaaa tatgtttgtg aattttaaag 300
tttattttta ggtcgttttt agtttttaat atatagaaat atattatttt ggaataagag 360
tagttttgtt ttcgttattt tttttttttt ttttttttgt atttttttgt agagacgggg 420
ttttgttatg tttttcgggt tgttgttgaa tttttgagtg taagtgatgt atttacgtta 480
ttttttatag tgttgggatt attggcgtgg gttatcgtgg cgggttcgtc gttgttattg 540
taaagagttt tatttttttt tttgatttta tggtattgcg tagatttatt cgttataatg 600
gtgatagtgg atatttttgt tttatttttg atgagaaatc gaaaaatttt aatattttat 660
tattttattt attttttttt ttttgtagat ggattttatt agagtgagtt attttatttt 720
gttttaaatt tgttgagagt atttatttga atatatgttg atttttatta aatagtgtat 780

ttatttcgat tattatagcg ttttttttta tttatgtgtt aatatagtga attcgattga 840
taaatttgta cgtttttagg ttcgattatt aaaatttgag atagcgtttt attttgttat 900
cgaggttgga gtgtagtggt gttattagag ttcgttgtag ttttgatttt ttgggtttag 960
gcgttttttt tattttagtt ttttgagtag ttgtgagtat aggtatatgt tattatgttt 1020
agttaatttt tcgatggttt tttgtttgtt ttttgtagtg atgagatttt ttgatgttgt 1080
ttaggttggt ttcgaagttt tgagtttagg tgatttggtt agtttagttt tttaaaatat 1140
taggattata ggcgtgagtt ttggtttggt ttggtttttt ttatataggg gttttattta 1200
tataaagatt aaagttaatt tgtgtttttg tgcgggtggg ttaagagtat gatgattttt 1260
attattttat tgatttaaag aaaattattt ttgatttatt agtgtgtaag gttatgaaag 1320
tataattttg ttgaaagtat atattgttaa tgggtgttgg gaatcgtgta tttttcgttg 1380
ttgtgggagt atgtttttgg aggtattttt tatttgtttt tttaatttta aatattttag 1440
gattatgggt tgtgattggt aggattatgt attttgttgt ttttaagacg gagtatattt 1500
ttacgtggtt ttattttggt tgttttgttt ttttaatatt gttattttat tttttgtgat 1560
tttgatgtta taaatgatag atatcgtttt agtatttttt tacgggtttt agcgatttta 1620
tttatttttt ttttagtttt tttttttgat ttgtttataa tgaataattt ttttttggga 1680
taggttgtta tttttgtttt cgtaggtggt ttatttgttt ttttagttag ttatagtggt 1740
ttttgttttt atggtgggtt cggggtaaga gagggttttg ggttgggggt ggggtttagt 1800
tgaagatggg gtgagttttg aggggagtat tatttgagtt ttagaggtat aggaaatagt 1860
agagggaggt gggatttttt tatttttaat gaggatgggt atggagggtt tggggcgtgg 1920
cgttgggaac ggtagttttt tttagtttat agtcgcgtat gttttttggg ttttcgtttt 1980
agtgcgtatg tttattgggc gttttttgtt cggttttttc gtttacgtga agaacgttag 2040
ggagttgtga ggtagtgttg tgtggttttt gtcgttcgga tttttttttt tttattgaga 2100
tttatttggt aggtttgtag gttagttatt tcgggggttg aagtgtgagt gagggtggag 2160
agggttttgg gtgggttagg cgggtttcgt tttttggttt gtggttttcg agggagaagg 2220
gttacgaggt cgtttttttt ttttttatag gttgcgaggt tatcggcggt ttcgtggtcg 2280
tgaaggggtt tggacgggga ggaaggtggg tcgtggaggg gaggcggtta ggggtttagg 2340
tgaagatggg gtgagtgttg ttggggggat ggaagtttcg aggtgtcggg aattttcgac 2400
gatatagggt agattttttg aatggggttt tcggcggggg cgaggcgggc ggtgaagaag 2460
gggtttggta tttgggaagg ttgcggtttg gtgagcgttt t 2501

<210> 57
<211> 2501
<212> DNA
<213> Artificial Sequence

<220>
<223> chemically treated genomic DNA (Homo sapiens)

<400> 57

ggggcgttta ttaggtcgta gtttttttag gtgttaggtt ttttttttat cgttcgtttc 60
gttttcgtcg gaggttttat ttagggaatt tgttttgtgt cgtcgggggt tttcggtatt 120
tcgggatttt tattttttta atagtattta ttttattttt atttgagttt ttgatcgttt 180
tttttttacg gtttattttt tttttcgttt aggttttttt acgattacga agtcgtcggt 240
ggtttcgtag tttgtgaagg gaaggaggac gatttcgtgg tttttttttt tcggaggtta 300
tagattagga agcgggattc gtttgattta tttaaggttt tttttatttt tatttatatt 360
ttagttttcg ggatgattgg tttgtagatt tattagatga attttagtag aggaaaaaga 420
gttcggacgg taggaattat atagtattgt tttatagttt tttggcgttt tttacgtggg 480
cgaaggggtc gggtagaaga cgtttagtga atatgcgtat tgaggcggga gtttagggag 540
tatgcgcggt tgtgggttgg ggagggttgt cgtttttagc gttacgtttt aaatttttta 600
tgtttatttt tattgaggat aagggaattt tatttttttt tgttgttttt tatgtttttg 660
ggatttaagt agtgtttttt ttaaaattta ttttattttt aattgaattt tatttttaat 720
ttagggtttt tttttgtttc ggatttatta tggggataag gattattgtg attggttggg 780
aagataggta aattatttgc gaaaatagaa atagtaattt attttaaaag gaaattgttt 840
attgtgaata agttagagaa agagattgaa agaaaaatga atagaatcgt taggattcgt 900
aagaaaatgt taaaacgata tttattattt gtagtattag aattatagag ggtgaagtga 960
tagtattagg gaaataggat agttagagtg aaattacgtg aaaatatatt tcgttttgaa 1020
agtagtaaga tatatagttt tattagttat aatttatggt tttaagatgt ttggagttga 1080
gaaaatagat gaaaggtatt tttaaggata tgtttttata gtagcggaaa gtgtacggtt 1140
tttaatattt attaataata tatgttttta atagaattat gtttttatgg ttttatatat 1200
tggtaagtta aggatagttt tttttaaatt aatagaatga taaaagttat tatgttttta 1260

gtttattcgt ataaaggtat agattaattt tagtttttat atagataaga tttttatata 1320
agaaaaatta gattaggtta aggtttacgt ttgtaatttt agtattttgg gaggttgagt 1380

tggttagatt atttgagttt aggatttcga gattagttta agtaatatta gaaaatttta 1440
ttattataaa aaataaataa aaaattatcg aaaaattagt tgggtatggt ggtatgtatt 1500
tatatttata gttatttagg aggttgaggt gggaggagcg tttgagttta ggaggttaag 1560
gttgtagcga gttttgataa tattattgta ttttagtttc ggtgatagag tgagacgttg 1620
ttttaagttt taataatcga atttaaaaac gtataaattt attaatcgaa tttattatat 1680
taatatatga atgggaaaaa acgttgtggt aatcgaaata gatgtattgt ttgatgaaaa 1740
ttaatatata tttaaatgaa tatttttagt aaatttggaa tagaatggaa tgatttattt 1800
tgataaagtt tatttataaa aaaaggagag tgaataggat ggtgaaatgt tgaaattttt 1860
cggtttttta ttagggataa gataaggatg tttattgtta ttattgtaac gggtgggttt 1920
gcgtaatgtt ataaaattag aaaaggaaat aaaatttttt ataatggtaa cgacgggttc 1980
gttacggtgg tttacgttag taattttagt attgtgggag atgacgtggg tgtattattt 2040
gtatttaaaa gtttaataat agttcgggaa atatggtaaa atttcgtttt tataaaaaaa 2100
tataaaaggg aaaagaaaaa aaaatggcgg aaataaaatt gtttttattt taggatgata 2160
tgtttttgta tattgaagat tgaaaacgat ttagaagtaa attttagaat ttataagtat 2220
attttataag gttattggat agaaaattaa tatgtaagaa ttatgttttt atcgggcgcg 2280
gaggtttatg tttgtaattt tagtattttg ggaggttaag gtaggtgaat tacgaggtta 2340
ggagtttaag agtatttcgg ttaagatggc gaaagtttat ttttagtaaa aatataaaat 2400
ttagttaggc gtgttggtgg gtgtttataa ttttagttat tcgggaggtt gaggtagata 2460
attgtttgaa ttcgggaggc ggggttgtag tgagtagaga t 2501

<210> 58
<211> 2500
<212> DNA
<213> Artificial Sequence

<220>
<223> chemically treated genomic DNA (Homo sapiens)

<400> 58

taagtaattg tttgttttag tttttcgagt agttgggatt ataggtattt attagtacgt 60
ttggttaagt tttgtgtttt tattagagat gggttttcgt tattttggtc ggggtgtttt 120
tgaatttttg atttcgtgat ttatttgttt tggtttttta aagtgttggg attataggta 180
tgagttttcg cgttcggtgg agatataatt tttatatatt ggttttttat ttagtggttt 240
tgtgaaatat gtttgtgaat tttaaagttt atttttaggt cgtttttagt ttttaatata 300
tagaaatata ttattttgga ataagagtag ttttgttttc gttatttttt tttttttttt 360
tttttgtatt tttttgtaga gacggggttt tgttatgttt ttcgggttgt tgttgaattt 420
ttgagtgtaa gtgatgtatt tacgttattt tttatagtgt tgggattatt ggcgtgggtt 480
atcgtggcgg gttcgtcgtt gttattgtaa agagttttat tttttttttt gattttatgg 540
tattgcgtag atttattcgt tataatggtg atagtggata tttttgtttt atttttgatg 600
agaaatcgaa aaattttaat attttattat tttatttatt tttttttttt tgtagatgga 660
ttttattaga gtgagttatt ttattttgtt ttaaatttgt tgagagtatt tatttgaata 720
tatgttgatt tttattaaat agtgtattta tttcgattat tatagcgttt ttttttattt 780
atgtgttaat atagtgaatt cgattgataa atttgtacgt ttttaggttc gattattaaa 840
atttgagata gcgttttatt ttgttatcga ggttggagtg tagtggtgtt attagagttc 900
gttgtagttt tgattttttg ggtttaggcg ttttttttat tttagttttt tgagtagttg 960
tgagtatagg tatatgttat tatgtttagt taatttttcg atggtttttt gtttgttttt 1020
tgtagtgatg agattttttg atgttgttta ggttggtttc gaagttttga gtttaggtga 1080
tttggttagt ttagtttttt aaaatattag gattataggc gtgagttttg gtttggtttg 1140
gtttttttta tataggggtt ttatttatat aaagattaaa gttaatttgt gtttttgtgc 1200
gggtgggtta agagtatgat gatttttatt attttattga tttaaagaaa attatttttg 1260
atttattagt gtgtaaggtt atgaaagtat aattttgttg aaagtatata ttgttaatgg 1320
gtgttgggaa tcgtgtattt ttcgttgttg tgggagtatg tttttggagg tattttttat 1380
ttgttttttt aattttaaat attttaggat tatgggttgt gattggtagg attatgtatt 1440
ttgttgtttt taagacggag tatattttta cgtggtttta ttttggttgt tttgtttttt 1500
taatattgtt attttatttt ttgtgatttt gatgttataa atgatagata tcgttttagt 1560
atttttttac gggttttagc gattttattt attttttttt tagttttttt ttttgatttg 1620
tttataatga ataatttttt tttgggatag gttgttattt ttgttttcgt aggtggttta 1680
tttgtttttt tagttagtta tagtggtttt tgtttttatg gtgggttcgg ggtaagagag 1740
ggttttgggt tgggggtggg gtttagttga agatggggtg agttttgagg ggagtattat 1800
ttgagtttta gaggtatagg aaatagtaga gggaggtggg atttttttat ttttaatgag 1860
gatgggtatg gagggtttgg ggcgtggcgt tgggaacggt agtttttttt agtttatagt 1920
cgcgtatgtt ttttgggttt tcgttttagt gcgtatgttt attgggcgtt ttttgttcgg 1980
ttttttcgtt tacgtgaaga acgttaggga gttgtgaggt agtgttgtgt ggtttttgtc 2040

gttcggattt tttttttttt attgagattt atttggtagg tttgtaggtt agttatttcg 2100
ggggttgaag tgtgagtgag ggtggagagg gttttgggtg ggttaggcgg gtttcgtttt 2160
ttggtttgtg gttttcgagg gagaagggtt acgaggtcgt tttttttttt tttataggtt 2220
gcgaggttat cggcggtttc gtggtcgtga aggggtttgg acggggagga aggtgggtcg 2280
tggaggggag gcggttaggg gtttaggtga agatggggtg agtgttgttg gggggatgga 2340
agtttcgagg tgtcgggaat tttcgacgat atagggtaga ttttttgaat ggggttttcg 2400
gcgggggcga ggcgggcggt gaagaagggg tttggtattt gggaaggttg cggtttggtg 2460
agcgtttttt ttagcggtgt ggagtgcgga gcgtttgagt 2500

<210> 59
<211> 2500
<212> DNA
<213> Artificial Sequence

<220>
<223> chemically treated genomic DNA (Homo sapiens)

<400> 59

atttaggcgt ttcgtatttt atatcgttgg gggggcgttt tattaggtcg tagttttttt 60
aggtgttagg tttttttttt atcgttcgtt tcgttttcgt cggaggtttt atttagggaa 120
tttgttttgt gtcgtcgggg gttttcggta tttcgggatt tttatttttt taatagtatt 180
tattttattt ttatttgagt ttttgatcgt ttttttttta cggtttattt tttttttcgt 240
ttaggttttt ttacgattac gaagtcgtcg gtggtttcgt agtttgtgaa gggaaggagg 300
acgatttcgt ggtttttttt tttcggaggt tatagattag gaagcgggat tcgtttgatt 360
tatttaaggt tttttttatt tttatttata ttttagtttt cgggatgatt ggtttgtaga 420
tttattagat gaattttagt agaggaaaaa gagttcggac ggtaggaatt atatagtatt 480
gttttatagt tttttggcgt tttttacgtg ggcgaagggg tcgggtagaa gacgtttagt 540
gaatatgcgt attgaggcgg gagtttaggg agtatgcgcg gttgtgggtt ggggagggtt 600
gtcgttttta gcgttacgtt ttaaattttt tatgtttatt tttattgagg ataagggaat 660
tttatttttt tttgttgttt tttatgtttt tgggatttaa gtagtgtttt ttttaaaatt 720
tattttattt ttaattgaat tttattttta atttagggtt tttttttgtt tcggatttat 780
tatggggata aggattattg tgattggttg ggaagatagg taaattattt gcgaaaatag 840
aaatagtaat ttattttaaa aggaaattgt ttattgtgaa taagttagag aaagagattg 900
aaagaaaaat gaatagaatc gttaggattc gtaagaaaat gttaaaacga tatttattat 960
ttgtagtatt agaattatag agggtgaagt gatagtatta gggaaatagg atagttagag 1020
tgaaattacg tgaaaatata tttcgttttg aaagtagtaa gatatatagt tttattagtt 1080
ataatttatg gttttaagat gtttggagtt gagaaaatag atgaaaggta tttttaagga 1140
tatgttttta tagtagcgga aagtgtacgg tttttaatat ttattaataa tatatgtttt 1200
taatagaatt atgtttttat ggttttatat attggtaagt taaggatagt tttttttaaa 1260
ttaatagaat gataaaagtt attatgtttt tagtttattc gtataaaggt atagattaat 1320
tttagttttt atatagataa gatttttata taagaaaaat tagattaggt taaggtttac 1380
gtttgtaatt ttagtatttt gggaggttga gttggttaga ttatttgagt ttaggatttc 1440
gagattagtt taagtaatat tagaaaattt tattattata aaaaataaat aaaaaattat 1500
cgaaaaatta gttgggtatg gtggtatgta tttatattta tagttattta ggaggttgag 1560
gtgggaggag cgtttgagtt taggaggtta aggttgtagc gagttttgat aatattattg 1620
tattttagtt tcggtgatag agtgagacgt tgttttaagt tttaataatc gaatttaaaa 1680
acgtataaat ttattaatcg aatttattat attaatatat gaatgggaaa aaacgttgtg 1740
gtaatcgaaa tagatgtatt gtttgatgaa aattaatata tatttaaatg aatattttta 1800
gtaaatttgg aatagaatgg aatgatttat tttgataaag tttatttata aaaaaaggag 1860
agtgaatagg atggtgaaat gttgaaattt ttcggttttt tattagggat aagataagga 1920
tgtttattgt tattattgta acgggtgggt ttgcgtaatg ttataaaatt agaaaaggaa 1980
ataaaatttt ttataatggt aacgacgggt tcgttacggt ggtttacgtt agtaatttta 2040
gtattgtggg agatgacgtg ggtgtattat ttgtatttaa aagtttaata atagttcggg 2100
aaatatggta aaatttcgtt tttataaaaa aatataaaag ggaaaagaaa aaaaaatggc 2160
ggaaataaaa ttgtttttat tttaggatga tatgtttttg tatattgaag attgaaaacg 2220
atttagaagt aaattttaga atttataagt atattttata aggttattgg atagaaaatt 2280
aatatgtaag aattatgttt ttatcgggcg cggaggttta tgtttgtaat tttagtattt 2340
tgggaggtta aggtaggtga attacgaggt taggagttta agagtatttc ggttaagatg 2400
gcgaaagttt atttttagta aaaatataaa atttagttag gcgtgttggt gggtgtttat 2460
aattttagtt attcgggagg ttgaggtaga taattgtttg 2500

<210> 60

<211> 2500
<212> DNA
<213> Artificial Sequence

<220>
<223> chemically treated genomic DNA (Homo sapiens)

<400> 60

taaatttatt tgtatttttg tgagggtgtg ttaagggtat gatgaaattt attattttat 60
tgatttaaag aaaattattt ttgatttttt agtgtgtaag tttatgaaag tataattatg 120
ttgaaagtat atattgttat gggtgttgag aattttgtat tttttgttgt tgtgggagta 180
tgtttttgga ggtatttttc gtttgttttt ttaattttaa atattttagg attatgggtt 240
gtgattggta aggaatgtgt tttgcgagtt ttaagatgga gttgattttt atatggtgtt 300
attttggttt ttttgttttt ttaatattgg tatttttttt tttgtgattt tgatgttata 360
aatgatagat atcgttttag tattttttta tgggttttag agattgtatt tatttttttt 420
ttagtttttt tttttgattt gtttatattt aataattttt ttttggggta ggttgttatt 480
tttgttttcg taggtggttt atttgttttt ttaggtagtt atcgtggttt ttgtttttat 540
ggtggggtcg gggtaagaga gggttttgga ggggaggggg gtttagttga agatggagtg 600
agttttgagg ggagtattat ttgagtttta gaggtatagg aaatagtaga gggaggttag 660
attttattat ttttagtggg gatgggaatc gagggttggg ggcgtggggt tgggaacggt 720
agtttttttt aattcgtagt tgtatatgtt ttttggtttt cgttttagtg cgtatgttta 780
ttgggcgttt tttgtttagt cgttttattt acgtggagaa cgttagggag ttgtgagggt 840
gtgtggtttc gtttttgtcg tttggaatat ttttttttta ttgagattta tttggtaggt 900
ttgtaggtta gttttttcgg ggtttgaagt gtgagtgagg gtgaagagta ggtagtgtgt 960
ttcgggtgag ttcgttgggt tttgtttcgt ggtttgtggt ttttgaggga gaagggtttc 1020
gaggtttttg aaagggaagg ggtttttggt ttttgaaaga gaagggtttt gaggtcgttt 1080
tttttttttt aaaggttgtg aggttattat ttgttttgtg gtcgtgaagg ggttaggata 1140
aggaggaagg tgggttatgg aggggaggcg gttaggggtt taggtgaaga aggggcgatt 1200
gttgggtgtg ttgttagagg gatggaagtt cggaggtgtt aggaatattc gatatagggg 1260
agatttttga atgaggtttc ggataggtgc gaggagggcg ataaagaagg gatttggtat 1320
ttgggaagat tgcgggttgg tgagtgtttt tgagttttgt ggagtgggga gtttcgagtg 1380
agaagtatcg taagatttta ttttcgttat ggaaggtttg gtaatagtgg gaaggattgg 1440
gagaggttgt gcggtttaat taaatttgat ttgagagaag tgaatggttt tagtaagtgg 1500
gagtgtgttt aaagtagtaa ttacgagaat tatgatttat taatgttttt atgtggagtt 1560
tatttgtgaa attaaatttt attagaaatg atttttgtta gtggggcgtt atggtttgcg 1620
tttgtagttt tagttattcg ggacgttgag gtgggaggat tttttgagta ggaggtcgat 1680
gttgtagtga gttgtgatta cgtcgttgta ttttgttaga gtaatatagg gagattgcgg 1740
gattaaaaga aatttagaaa aaaaatgttt tttgcgtttt gttatacgtt ttaagatgat 1800
tgttttgtta gtttggttag tataagtggt tttgtaggta tttagaaaag gtatatatat 1860
atgtttaatt ttgggattta ttttgaaagt atttttaaaa ttaaaacgat aagttaatat 1920
ttatttatgg aggtgatgga atatagtagt tttttcgagg gtatgttttt aattacggtt 1980
gtttgttttt agtgtgaaat atgagttggc gaggaagatc gatttatcgg tttagattaa 2040
gattttatgt agagtttttt gaaatgattg ggtttatgtt ggtgagtgtt taaacgttaa 2100
ttcgttgttt tttattagta gaaattaatt tttgtgatag tattgttgta ttagtatgga 2160
aatgttgata aaggtttttt ttgtttataa aaaatgatga tggcgtttta tgaaggaaat 2220
attgattttg gagaattttt ttttttttag tgttttttag tttttgttta tgattttttt 2280
tttaggtttt gtttgttaat gatagattgt atatatgtat tttaatattg agtataatag 2340
tttttaaagt tttcgtgcgt tatttttttt atagtaattt ttttgtgggt cgagtaattt 2400
tattgggtat agagtataga gttggagaaa tgtttttagg tttagttagg attagaaata 2460
gttatgtatt ttgtgtatat atgtaaaatt ttgtattaat 2500

<210> 61
<211> 2500
<212> DNA
<213> Artificial Sequence

<220>
<223> chemically treated genomic DNA (Homo sapiens)

<400> 61
   attgatataa aattttatat atatatatag aatatatagt tatttttggt tttaattaag 60
   tttaaagata tttttttaat tttatgtttt atgtttaata aggttattcg atttatagga 120
   aggttattat gagaaaagtg acgtacgagg attttggagg ttattatatt tagtgttgga 180
   atatatgtgt ataatttgtt attaataaat aaagtttgag aaagaagtta tgggtaaaag 240
   ttgaagaata ttagaggaaa aaaaattttt tagaattaat gtttttttta tgagacgtta 300
   ttattatttt ttatgagtag gaaagatttt tattagtatt tttatattaa tgtaataata 360
   ttgttataaa aattaatttt tgttaataga aaataacgaa ttaacgttta agtatttatt 420
   agtataggtt taattatttt agggggtttt atatagagtt ttggtttaga tcgataggtc 480
   gatttttttc gttaatttat attttatatt gaaaatagat aatcgtgatt gggagtatgt 540
   tttcgaaagg gttgttatat tttattattt ttatggataa atgttaattt gtcgttttaa 600
   ttttgaaaat atttttaaaa taagttttag agttaagtat atgtgtgtat tttttttgag 660
   tgtttataaa gttatttatg ttggttaagt tggtagagta attattttaa ggcgtgtgat 720
   aaaacgtaga ggatattttt tttttaaatt ttttttggtt tcgtagtttt tttgtgttgt 780
   tttggtagag tgtagcggcg tgattatagt ttattgtagt atcgattttt tgtttaaggg 840
   atttttttat tttagcgttt cgagtaattg ggattatagg cgtaggttat ggcgttttat 900
   tggtagaggt tatttttgat gaggtttagt tttataagtg gattttatat gaaaatatta 960
   gtgaattata attttcgtga ttgttatttt gggtatattt ttatttatta gagttattta 1020
   ttttttttaa attaagtttg attggatcgt atagtttttt ttagtttttt ttattgttgt 1080
   tagatttttt atggcggagg tgagattttg cgatgttttt tattcggggt tttttatttt 1140
   ataaagttta ggggtattta ttagttcgta gtttttttag gtgttaggtt ttttttttat 1200
   cgtttttttc gtatttgttc gggattttat ttagggattt tttttgtatc gggtattttt 1260
   ggtattttcg gatttttatt tttttgatag tatatttagt aatcgttttt tttttatttg 1320
   agtttttgat cgtttttttt ttatggttta tttttttttt tgttttggtt tttttacgat 1380
   tataaagtag atggtggttt tatagttttt gagagaagga ggacgatttt aaggtttttt 1440
   ttttttagag gttaggagtt tttttttttt tagaagtttc gaggtttttt ttttttagag 1500
   gttatagatt acgaagtagg atttaacgga tttattcgaa gtatattgtt tgttttttat 1560
   ttttatttat attttagatt tcgggaggat tggtttgtag atttattaga tgaattttag 1620
   tagaggaaaa atattttaga cgataggaac gagattatat atttttatag ttttttggcg 1680
   ttttttacgt gggtaaagcg gttgagtaga agacgtttag tggatatgcg tattgaggcg 1740
   ggagttaagg agtatgtata gttgcgggtt ggggaaggtt gtcgttttta gttttacgtt 1800
   tttaattttc gatttttatt tttattgagg ataatggaat ttgatttttt tttgttgttt 1860
   tttatgtttt tgggatttaa gtagtgtttt ttttaaaatt tattttattt ttaattaaat 1920
   tttttttttt tttagggttt ttttttgttt cggttttatt atggggataa ggattacggt 1980
   gattgtttga gaagataggt aaattatttg cgaaaataga aatagtaatt tattttaaaa 2040
   agaaattgtt aaatgtgaat aagttagaga aagagattga aagaaaaatg aatataattt 2100
   ttaggattta taagaaaata ttaaaacgat atttattatt tgtagtatta gaattataga 2160
   aagagaagtg ttagtattag agaaatagga gagttagagt gatattatgt gaaaattaat 2220
   tttattttga aattcgtaag atatattttt tattagttat aatttatggt tttaagatgt 2280
   ttggagttga gaaaatagac gaaaggtatt tttaaggata tgtttttata gtagtagaaa 2340
   gtgtagggtt tttaatattt ataataatat atgtttttaa tataattatg tttttatgga 2400
   tttatatatt ggaaagttaa ggatagtttt ttttaaatta atagaatgat aaattttatt 2460
   atgtttttag tatattttta taaagatata gataagttta 2500

<210> 62
<211> 2500
<212> DNA
<213> Artificial Sequence

<220>
<223> chemically treated genomic DNA (Homo sapiens)

<400> 62

aagtaattgt ttgttttagt ttttcgagta gttgggatta taggtattta ttagtacgtt 60
tggttaagtt ttgtgttttt attagagatg ggttttcgtt attttggtcg ggtgtttttt 120
gaatttttga tttcgtgatt tatttgtttt ggttttttaa agtgttggga ttataggtat 180
gagttttcgc gttcggtgga gatataattt ttatatattg gttttttatt tagtggtttt 240
gtgaaatatg tttgtgaatt ttaaagttta tttttaggtc gtttttagtt tttaatatat 300
agaaatatat tattttggaa taagagtagt tttgttttcg ttattttttt tttttttttt 360
ttttgtattt ttttgtagag acggggtttt gttatgtttt tcgggttgtt gttgaatttt 420
tgagtgtaag tgatgtattt acgttatttt ttatagtgtt gggattattg gcgtgggtta 480
tcgtggcggg ttcgtcgttg ttattgtaaa gagttttatt tttttttttg attttatggt 540
attgcgtaga tttattcgtt ataatggtga tagtggatat ttttgtttta tttttgatga 600
gaaatcgaaa aattttaata ttttattatt ttatttattt tttttttttt gtagatggat 660
tttattagag tgagttattt tattttgttt taaatttgtt gagagtattt atttgaatat 720
atgttgattt ttattaaata gtgtatttat ttcgattatt atagcgtttt tttttattta 780

tgtgttaata tagtgaattc gattgataaa tttgtacgtt tttaggttcg attattaaaa 840
tttgagatag cgttttattt tgttatcgag gttggagtgt agtggtgtta ttagagttcg 900
ttgtagtttt gatttttttgg gtttaggcgt ttttttattt ttagtttttt gagtagttgt 960
gagtataggt atatgttatt atgtttagtt aatttttcga tggttttttg tttgtttttt 1020
gtagtgatga gattttttga tgttgtttag gttggtttcg aagttttgag tttaggtgat 1080
ttggttagtt tagtttttta aaatattagg attataggcg tgagttttgg tttggtttgg 1140
ttttttttat ataggggttt tatttatata aagattaaag ttaatttgtg tttttgtgcg 1200
ggtgggttaa gagtatgatg atttttatta ttttattgat ttaaagaaaa ttatttttga 1260
tttattagtg tgtaaggtta tgaaagtata attttgttga aagtatatat tgttaatggg 1320
tgttgggaat cgtgtatttt tcgttgttgt gggagtatgt ttttggaggt attttttatt 1380
tgttttttta attttaaata ttttaggatt atgggttgtg attggtagga ttatgtattt 1440
tgttgttttt aagacggagt atatttttac gtggttttat tttggttgtt ttgttttttt 1500
aatattgtta ttttattttt tgtgattttg atgttataaa tgatagatat cgttttagta 1560
tttttttacg ggttttagcg atttatttta ttttttttttt agtttttttt tttgatttgt 1620
ttataatgaa taattttttt ttgggatagg ttgttatttt tgttttcgta ggtggtttat 1680
ttgttttttt agttagttat agtggttttt gtttttatgg tgggttcggg gtaagagagg 1740
gttttgggtt gggggtgggg tttagttgaa gatggggtga gttttgaggg gagtattatt 1800
tgagttttag aggtatagga aatagtagag ggaggtggga tttttttatt tttaatgagg 1860
atgggtatgg agggtttggg gcgtggcgtt gggaacggta gtttttttta gtttatagtc 1920
gcgtatgttt tttgggtttt cgttttagtg cgtatgttta ttgggcgttt tttgttcggt 1980
tttttcgttt acgtgaagaa cgttagggag ttgtgaggta gtgttgtgtg gtttttgtcg 2040
ttcggatttt ttttttttta ttgagattta tttggtaggt ttgtaggtta gttatttcgg 2100
gggttgaagt gtgagtgagg gtggagaggg ttttgggtgg gttaggcggg tttcgttttt 2160
tggtttgtgg ttttcgaggg agaagggtta cgaggtcgtt tttttttttt ttataggttg 2220
cgaggttatc ggcggtttcg tggtcgtgaa ggggtttgga cggggaggaa ggtgggtcgt 2280
ggaggggagg cggttagggg tttaggtgaa gatggggtga gtgttgttgg ggggatggaa 2340
gtttcgaggt gtcgggaatt ttcgacgata tagggtagat tttttgaatg gggttttcgg 2400
cgggggcgag gcgggcggtg aagaaggggt ttggtatttg ggaaggttgc ggtttggtga 2460
gcgttttttt tagcggtgtg gagtgcggag cgtttgagtg 2500

<210> 63
<211> 2500
<212> DNA
<213> Artificial Sequence

<220>
<223> chemically treated genomic DNA (Homo sapiens)

<400> 63

tatttaggcg tttcgtattt tatatcgttg gggggggcgt ttattaggtc gtagtttttt 60
taggtgttag gttttttttt tatcgttcgt ttcgttttcg tcggaggttt tatttaggga 120
atttgttttg tgtcgtcggg ggttttcggt atttcgggat ttttattttt ttaatagtat 180
ttattttatt tttatttgag tttttgatcg tttttttttt acggtttatt ttttttttcg 240
tttaggtttt tttacgatta cgaagtcgtc ggtggtttcg tagtttgtga agggaaggag 300
gacgatttcg tggttttttt ttttcggagg ttatagatta ggaagcggga ttcgtttgat 360
ttatttaagg ttttttttat ttttatttat attttagttt tcgggatgat tggtttgtag 420
atttattaga tgaattttag tagaggaaaa agagttcgga cggtaggaat tatatagtat 480
tgttttatag ttttttggcg ttttttacgt gggcgaaggg gtcgggtaga agacgtttag 540
tgaatatgcg tattgaggcg ggagtttagg gagtatgcgc ggttgtgggt tggggagggt 600
tgtcgttttt agcgttacgt tttaaatttt ttatgtttat ttttattgag gataagggaa 660
ttttattttt ttttgttgtt ttttatgttt ttgggattta agtagtgttt tttttaaaat 720
ttattttatt tttaattgaa ttttattttt aatttagggt ttttttttgt ttcggattta 780
ttatggggat aaggattatt gtgattggtt gggaagatag gtaaattatt tgcgaaaata 840
gaaatagtaa tttattttaa aaggaaattg tttattgtga ataagttaga gaaagagatt 900
gaaagaaaaa tgaatagaat cgttaggatt cgtaagaaaa tgttaaaacg atatttatta 960
tttgtagtat tagaattata gagggtgaag tgatagtatt agggaaatag gatagttaga 1020
gtgaaattac gtgaaaatat atttcgtttt gaaagtagta agatatatag ttttattagt 1080
tataatttat ggttttaaga tgtttggagt tgagaaaata gatgaaaggt atttttaagg 1140
atatgttttt atagtagcgg aaagtgtacg gtttttaata tttattaata atatatgttt 1200
ttaatagaat tatgttttta tggttttata tattggtaag ttaaggatag ttttttttaa 1260
attaatagaa tgataaaagt tattatgttt ttagtttatt cgtataaagg tatagattaa 1320
ttttagtttt tatatagata agatttttat ataagaaaaa ttagattagg ttaaggttta 1380
cgtttgtaat tttagtattt tgggaggttg agttggttag attatttgag tttaggattt 1440

cgagattagt ttaagtaata ttagaaaatt ttattattat aaaaaataaa taaaaaatta 1500
tcgaaaaatt agttgggtat ggtggtatgt atttatattt atagttattt aggaggttga 1560
ggtgggagga gcgtttgagt ttaggaggtt aaggttgtag cgagttttga taatattatt 1620
gtattttagt ttcggtgata gagtgagacg ttgttttaag ttttaataat cgaatttaaa 1680
aa[?][?]tataaa tttattaatc gaatttatta tattaatata tgaatgggaa aaaacgttgt 1740
gg[?]aatcgaa atagatgtat tgtttgatga aaattaatat atatttaaat gaatattttt 1800
ag[?]aaatttg gaatagaatg gaatgattta ttttgataaa gtttatttat aaaaaaagga 1860
gagtgaatag gatggtgaaa tgttgaaatt tttcggtttt ttattaggga taagataagg 1920
atgtttattg ttattattgt aacgggtggg tttgcgtaat gttataaaat tagaaaagga 1980
aataaaattt tttataatgg taacgacggg ttcgttacgg tggtttacgt tagtaatttt 2040
agtattgtgg gagatgacgt gggtgtatta tttgtattta aaagtttaat aatagttcgg 2100
gaaatatggt aaaatttcgt ttttataaaa aaatataaaa gggaaaagaa aaaaaaatgg 2160
cggaaataaa attgttttta ttttaggatg atatgttttt gtatattgaa gattgaaaac 2220
gatttagaag taaattttag aatttataag tatattttat aaggttattg gatagaaaat 2280
taatatgtaa gaattatgtt tttatcgggc gcggaggttt atgtttgtaa ttttagtatt 2340
ttgggaggtt aaggtaggtg aattacgagg ttaggagttt aagagtattt cggttaagat 2400
ggcgaaagtt tatttttagt aaaaatataa aatttagtta ggcgtgttgg tgggtgttta 2460
taattttagt tattcgggag gttgaggtag ataattgttt 2500

<210> 64
<211> 2500
<212> DNA
<213> Artificial Sequence

<220>
<223> chemically treated genomic DNA (Homo sapiens)

<400> 64

gtttgtttta gtttttcgag tagttgggat tataggtatt tattagtacg tttggttaag 60
ttttgtgttt ttattagaga tgggttttcg ttattttggt cggggtgttt ttgaattttt 120
gatttcgtga tttatttgtt ttggtttttt aaagtgttgg gattataggt atgagttttc 180
gcgttcggtg gagatataat ttttatatat tggtttttta tttagtggtt ttgtgaaata 240
tgtttgtgaa ttttaaagtt tatttttagg tcgtttttag tttttaatat atagaaatat 300
attattttgg aataagagta gttttgtttt cgttattttt tttttttttt ttttttgtat 360
ttttttgtag agacggggtt ttgttatgtt tttcgggttg ttgttgaatt tttgagtgta 420
agtgatgtat ttacgttatt ttttatagtg ttgggattat tggcgtgggt tatcgtggcg 480
ggttcgtcgt tgttattgta aagagtttta tttttttttt tgattttatg gtattgcgta 540
gatttattcg ttataatggt gatagtggat atttttgttt tatttttgat gagaaatcga 600
aaaattttaa tattttatta ttttatttat tttttttttt ttgtagatgg attttattag 660
agtgagttat tttattttgt tttaaatttg ttgagagtat ttatttgaat atatgttgat 720
ttttattaaa tagtgtattt atttcgatta ttatagcgtt tttttttatt tatgtgttaa 780
tatagtgaat tcgattgata aatttgtacg tttttaggtt cgattattaa aatttgagat 840
agcgttttat tttgttatcg aggttggagt gtagtggtgt tattagagtt cgttgtagtt 900
ttgatttttt gggtttaggc gtttttttta ttttagtttt ttgagtagtt gtgagtatag 960
gtatatgtta ttatgtttag ttaatttttc gatggttttt tgtttgtttt ttgtagtgat 1020
gagatttttt gatgttgttt aggttggttt cgaagttttg agtttaggtg atttggttag 1080
tttagttttt taaaatatta ggattatagg cgtgagtttt ggtttggttt ggtttttttt 1140
atataggggt tttatttata taaagattaa agttaatttg tgtttttgtg cgggtgggtt 1200
aagagtatga tgatttttat tattttattg atttaaagaa aattattttt gatttattag 1260
tgtgtaaggt tatgaaagta taattttgtt gaaagtatat attgttaatg ggtgttggga 1320
atcgtgtatt tttcgttgtt gtgggagtat gtttttggag gtatttttta tttgtttttt 1380
taattttaaa tattttagga ttatgggttg tgattggtag gattatgtat tttgttgttt 1440
ttaagacgga gtatattttt acgtggtttt attttggttg ttttgttttt ttaatattgt 1500
tattttattt tttgtgattt tgatgttata aatgatagat atcgttttag tattttttta 1560
cgggttttag cgattttatt tatttttttt ttagtttttt tttttgattt gtttataatg 1620
aataattttt ttttgggata ggttgttatt tttgttttcg taggtggttt atttgttttt 1680
ttagttagtt atagtggttt ttgtttttat ggtgggttcg gggtaagaga gggttttggg 1740
ttgggggtgg ggtttagttg aagatggggt gagttttgag gggagtatta tttgagtttt 1800
agaggtatag gaaatagtag agggaggtgg gattttttta tttttaatga ggatgggtat 1860
ggagggtttg gggcgtggcg ttgggaacgg tagttttttt tagtttatag tcgcgtatgt 1920
tttttgggtt ttcgttttag tgcgtatgtt tattgggcgt tttttgttcg gttttttcgt 1980
ttacgtgaag aacgttaggg agttgtgagg tagtgttgtg tggtttttgt cgttcggatt 2040
tttttttttt tattgagatt tatttggtag gtttgtaggt tagttatttc gggggttgaa 2100

gtgtgagtga gggtggagag ggttttgggt gggttaggcg ggtttcgttt tttggtttgt 2160
ggttttcgag ggagaagggt tacgaggtcg tttttttttt ttttataggt tgcgaggtta 2220
tcggcggttt cgtggtcgtg aaggggtttg gacggggagg aaggtgggtc gtggagggga 2280
ggcggttagg ggtttaggtg aagatggggt gagtgttgtt ggggggatgg aagtttcgag 2340
gtgtcgggaa ttttcgacga tatagggtag attttttgaa tggggttttc ggcgggggcg 2400
aggcgggcgg tgaagaaggg gtttggtatt tgggaaggtt gcggtttggt gagcgttttt 2460
tttagcggtg tggagtgcgg agcgtttgag tgagaagtat 2500

<210> 65
<211> 2500
<212> DNA
<213> Artificial Sequence

<220>
<223> chemically treated genomic DNA (Homo sapiens)

<400> 65

gtgtttttta tttaggcgtt tcgtatttta tatcgttggg gggggcgttt attaggtcgt 60
agttttttta ggtgttaggt ttttttttta tcgttcgttt cgttttcgtc ggaggtttta 120
tttagggaat ttgttttgtg tcgtcggggg ttttcggtat ttcgggattt ttattttttt 180
aatagtattt attttatttt tatttgagtt tttgatcgtt ttttttttac ggtttatttt 240
ttttttcgtt taggtttttt tacgattacg aagtcgtcgg tggtttcgta gtttgtgaag 300
ggaaggagga cgatttcgtg gttttttttt ttcggaggtt atagattagg aagcgggatt 360
cgtttgattt atttaaggtt ttttttattt ttatttatat tttagttttc gggatgattg 420
gtttgtagat ttattagatg aattttagta gaggaaaaag agttcggacg gtaggaatta 480
tatagtattg ttttatagtt ttttggcgtt ttttacgtgg gcgaaggggt cgggtagaag 540
acgtttagtg aatatgcgta ttgaggcggg agtttaggga gtatgcgcgg ttgtgggttg 600
gggagggttg tcgtttttag cgttacgttt taaatttttt atgtttattt ttattgagga 660
taagggaatt ttattttttt ttgttgtttt ttatgttttt gggatttaag tagtgttttt 720
tttaaaattt attttatttt taattgaatt ttatttttaa tttagggttt ttttttgttt 780
cggatttatt atggggataa ggattattgt gattggttgg gaagataggt aaattatttg 840
cgaaaataga aatagtaatt tattttaaaa ggaaattgtt tattgtgaat aagttagaga 900
aagagattga aagaaaaatg aatagaatcg ttaggattcg taagaaaatg ttaaaacgat 960
atttattatt tgtagtatta gaattataga gggtgaagtg atagtattag ggaaatagga 1020
tagttagagt gaaattacgt gaaaatatat ttcgttttga aagtagtaag atatatagtt 1080
ttattagtta taatttatgg ttttaagatg tttggagttg agaaaataga tgaaaggtat 1140
ttttaaggat atgtttttat agtagcggaa agtgtacggt ttttaatatt tattaataat 1200
atatgttttt aatagaatta tgtttttatg gttttatata ttggtaagtt aaggatagtt 1260
ttttttaaat taatagaatg ataaaagtta ttatgttttt agtttattcg tataaaggta 1320
tagattaatt ttagttttta tatagataag atttttatat aagaaaaatt agattaggtt 1380
aaggtttacg tttgtaattt tagtattttg ggaggttgag ttggttagat tatttgagtt 1440
taggatttcg agattagttt aagtaatatt agaaaatttt attattataa aaaataaata 1500
aaaaattatc gaaaaattag ttgggtatgg tggtatgtat ttatatttat agttatttag 1560
gaggttgagg tgggaggagc gtttgagttt aggaggttaa ggttgtagcg agttttgata 1620
atattattgt attttagttt cggtgataga gtgagacgtt gttttaagtt ttaataatcg 1680
aatttaaaaa cgtataaatt tattaatcga atttattata ttaatatatg aatgggaaaa 1740
aacgttgtgg taatcgaaat agatgtattg tttgatgaaa attaatatat atttaaatga 1800
atatttttag taaatttgga atagaatgga atgatttatt ttgataaagt ttatttataa 1860
aaaaaggaga gtgaatagga tggtgaaatg ttgaaatttt tcggtttttt attagggata 1920
agataaggat gtttattgtt attattgtaa cgggtgggtt tgcgtaatgt tataaaatta 1980
gaaaaggaaa taaaattttt tataatggta acgacgggtt cgttacggtg gtttacgtta 2040
gtaattttag tattgtggga gatgacgtgg gtgtattatt tgtatttaaa agtttaataa 2100
tagttcggga aatatggtaa aatttcgttt ttataaaaaa atataaaagg gaaaagaaaa 2160
aaaaatggcg gaaataaaat tgtttttatt ttaggatgat atgtttttgt atattgaaga 2220
ttgaaaacga tttagaagta aattttagaa tttataagta tattttataa ggttattgga 2280
tagaaaatta atatgtaaga attatgtttt tatcgggcgc ggaggtttat gtttgtaatt 2340
ttagtatttt gggaggttaa ggtaggtgaa ttacgaggtt aggagtttaa gagtatttcg 2400
gttaagatgg cgaaagttta tttttagtaa aaatataaaa tttagttagg cgtgttggtg 2460
ggtgtttata attttagtta ttcgggaggt tgaggtagat 2500

<210> 66
<211> 2500
<212> DNA

<213> Artificial Sequence

<220>
<223> chemically treated genomic DNA (Homo sapiens)

<400> 66

agtaattgtt tgttttagtt tttcgagtag ttgggattat aggtatttat tagtacgttt 60
ggttaagttt tgtgttttta ttagagatgg gtttcgtta ttttggtcgg ggtgtttttg 120
aatttttgat ttcgtgattt atttgttttg gttttttaaa gtgttgggat tataggtatg 180
agttttcgcg ttcggtggag atataatttt tatatattgg ttttttattt agtggttttg 240
tgaaatatgt ttgtgaattt taaagtttat ttttaggtcg tttttagttt ttaatatata 300
gaaatatatt attttggaat aagagtagtt ttgttttcgt tatttttttt tttttttttt 360
tttgtatttt tttgtagaga cggggttttg ttatgttttt cgggttgttg ttgaattttt 420
gagtgtaagt gatgtattta cgttattttt tatagtgttg ggattattgg cgtgggttat 480
cgtggcgggt tcgtcgttgt tattgtaaag agtttatttt ttttttttga ttttatggta 540
ttgcgtagat ttattcgtta taatggtgat agtggatatt tttgttttat ttttgatgag 600
aaatcgaaaa attttaatat tttattattt tatttatttt tttttttttg tagatggatt 660
ttattagagt gagttatttt attttgtttt aaatttgttg agagtattta tttgaatata 720
tgttgatttt tattaaatag tgtatttatt tcgattatta tagcgttttt ttttatttat 780
gtgttaatat agtgaattcg attgataaat ttgtacgttt ttaggttcga ttattaaaat 840
ttgagatagc gttttatttt gttatcgagg ttggagtgta gtggtgttat tagagttcgt 900
tgtagttttg atttttgggg tttaggcgtt ttttttattt tagttttttg agtagttgtg 960
agtataggta tatgttatta tgtttagtta atttttcgat ggttttttgt ttgttttttg 1020
tagtgatgag attttttgat gttgtttagg ttggtttcga agttttgagt ttaggtgatt 1080
tggttagttt agttttttaa aatattagga ttataggcgt gagttttggt ttggtttggt 1140
tttttttata taggggtttt atttatataa agattaaagt taatttgtgt ttttgtgcgg 1200
gtgggttaag agtatgatga tttttattat tttattgatt taaagaaaat tatttttgat 1260
ttattagtgt gtaaggttat gaaagtataa ttttgttgaa agtatatatt gttaatgggt 1320
gttgggaatc gtgtattttt cgttgttgtg ggagtatgtt tttggaggta ttttttattt 1380
gtttttttaa ttttaaatat tttaggatta tgggttgtga ttggtaggat tatgtatttt 1440
gttgttttta agacggagta tatttttacg tggttttatt ttggttgttt tgttttttta 1500
atattgttat tttatttttt gtgattttga tgttataaat gatagatatc gttttagtat 1560
ttttttacgg gttttagcga ttttatttat ttttttttta gttttttttt ttgatttgtt 1620
tataatgaat aatttttttt tgggataggt tgttattttt gttttcgtag gtggtttatt 1680
tgttttttta gttagttata gtggtttttg tttttatggt gggttcgggg taagagaggg 1740
ttttgggttg ggggtggggt ttagttgaag atggggtgag ttttgagggg agtattattt 1800
gagttttaga ggtataggaa atagtagagg gaggtgggat ttttttattt ttaatgagga 1860
tgggtatgga gggtttgggg cgtggcgttg ggaacggtag ttttttttag tttatagtcg 1920
cgtatgtttt ttgggttttc gttttagtgc gtatgtttat tgggcgtttt ttgttcggtt 1980
ttttcgttta cgtgaagaac gttagggagt tgtgaggtag tgttgtgtgg tttttgtcgt 2040
tcggattttt ttttttttat tgagatttat ttggtaggtt tgtaggttag ttatttcggg 2100
ggttgaagtg tgagtgaggg tggagagggt tttgggtggg ttaggcgggt ttcgtttttt 2160
ggtttgtggt tttcgaggga gaagggttac gaggtcgttt tttttttttt tataggttgc 2220
gaggttatcg gcggtttcgt ggtcgtgaag gggtttggac ggggaggaag gtgggtcgtg 2280
gaggggaggc ggttaggggt ttaggtgaag atggggtgag tgttgttggg gggatggaag 2340
tttcgaggtg tcgggaattt tcgacgatat agggtagatt ttttgaatgg ggttttcggc 2400
gggggcgagg cgggcggtga agaaggggtt tggtatttgg gaaggttgcg gtttggtgag 2460
cgtttttttt agcggtgtgg agtgcggagc gtttgagtga 2500

<210> 67
<211> 2500
<212> DNA
<213> Artificial Sequence

<220>
<223> chemically treated genomic DNA (Homo sapiens)

<400> 67

ttatttaggc gtttcgtatt ttatatcgtt ggggggggcg tttattaggt cgtagttttt 60
ttaggtgtta ggtttttttt ttatcgttcg tttcgttttc gtcggaggtt ttatttaggg 120
aatttgtttt gtgtcgtcgg gggttttcgg tatttcggga tttttatttt tttaatagta 180
tttattttat ttttatttga gtttttgatc gttttttttt tacggtttat tttttttttc 240

gtttaggttt ttttacgatt acgaagtcgt cggtggtttc gtagtttgtg aagggaagga 300
ggacgatttc gtggtttttt tttttcggag gttatagatt aggaagcggg attcgtttga 360
tttatttaag gtttttttta tttttattta tatttagttt ttcgggatga ttggtttgta 420
gatttattag atgaatttta gtagaggaaa aagagttcgg acggtaggaa ttatatagta 480
ttgttttata gttttttggc gttttttacg tgggcgaagg ggtcgggtag aagacgttta 540
gtgaatatgc gtattgaggc gggagtttag ggagtatgcg cggttgtggg ttggggaggg 600
ttgtcgtttt tagcgttacg ttttaaattt tttatgttta tttttattga ggataaggga 660
attttatttt tttttgttgt tttttatgtt tttgggattt aagtagtgtt ttttttaaaa 720
tttattttat ttttaattga attttatttt taatttaggg tttttttttg tttcggattt 780
attatgggga taaggattat tgtgattggt tgggaagata ggtaaattat ttgcgaaaat 840
agaaatagta atttatttta aaaggaaatt gtttattgtg aataagttag agaaagagat 900
tgaaagaaaa atgaatagaa tcgttaggat tcgtaagaaa atgttaaaac gatatttatt 960
atttgtagta ttagaattat agagggtgaa gtgatagtat tagggaaata ggatagttag 1020
agtgaaatta cgtgaaaata tatttcgttt tgaaagtagt aagatatata gttttattag 1080
ttataattta tggttttaag atgtttggag ttgagaaaat agatgaaagg tatttttaag 1140
gatatgtttt tatagtagcg gaaagtgtac ggtttttaat atttattaat aatatatgtt 1200
tttaatagaa ttatgttttt atggttttat atattggtaa gttaaggata gtttttttta 1260
aattaataga atgataaaag ttattatgtt tttagtttat tcgtataaag gtatagatta 1320
attttagttt ttatatagat aagattttta tataagaaaa attagattag gttaaggttt 1380
acgtttgtaa ttttagtatt ttgggaggtt gagttggtta gattatttga gtttaggatt 1440
tcgagattag tttaagtaat attagaaaat tttattatta taaaaaataa ataaaaaatt 1500
atcgaaaaat tagttgggta tggtggtatg tatttatatt tatagttatt taggaggttg 1560
aggtgggagg agcgtttgag tttaggaggt taaggttgta gcgagttttg ataatattat 1620
tgtattttag tttcggtgat agagtgagac gttgttttaa gttttaataa tcgaatttaa 1680
aaacgtataa atttattaat cgaatttatt atattaatat atgaatggga aaaaacgttg 1740
tggtaatcga aatagatgta ttgtttgatg aaaattaata tatatttaaa tgaatatttt 1800
tagtaaattt ggaatagaat ggaatgattt attttgataa agtttattta taaaaaaagg 1860
agagtgaata ggatggtgaa atgttgaaat ttttcggttt tttattaggg ataagataag 1920
gatgtttatt gttattattg taacgggtgg gtttgcgtaa tgttataaaa ttagaaaagg 1980
aaataaaatt ttttataatg gtaacgacgg gttcgttacg gtggtttacg ttagtaattt 2040
tagtattgtg ggagatgacg tgggtgtatt atttgtattt aaaagtttaa taatagttcg 2100
ggaaatatgg taaaatttcg tttttataaa aaaatataaa agggaaaaga aaaaaaaatg 2160
gcggaaataa aattgttttt attttaggat gatatgtttt tgtatattga agattgaaaa 2220
cgatttagaa gtaaatttta gaatttataa gtatatttta taaggttatt ggatagaaaa 2280
ttaatatgta agaattatgt ttttatcggg cgcggaggtt tatgtttgta attttagtat 2340
tttgggaggt taaggtaggt gaattacgag gttaggagtt taagagtatt tcggttaaga 2400
tggcgaaagt ttatttttag taaaaatata aaatttagtt aggcgtgttg gtgggtgttt 2460
ataattttag ttattcggga ggttgaggta gataattgtt 2500

<210> 68
<211> 2500
<212> DNA
<213> Artificial Sequence

<220>
<223> chemically treated genomic DNA (Homo sapiens)

<400> 68

taattgtttg ttttagtttt tcgagtagtt gggattatag gtatttatta gtacgtttgg 60
ttaagttttg tgtttttatt agagatgggt tttcgttatt ttggtcgggg tgtttttgaa 120
tttttgattt cgtgatttat ttgttttggt tttttaaagt gttgggatta taggtatgag 180
ttttcgcgtt cggtggagat ataattttta tatattggtt ttttatttag tggttttgtg 240
aaatatgttt gtgaatttta aagtttattt ttaggtcgtt tttagttttt aatatataga 300
aatatattat tttggaataa gagtagtttt gttttcgtta tttttttttt tttttttttt 360
tgtatttttt tgtagagacg gggttttgtt atgttttcgg ggttgttgtt gaatttttga 420
gtgtaagtga tgtatttacg ttatttttta tagtgttggg attattggcg tgggttatcg 480
tggcgggttc gtcgttgtta ttgtaaagag ttttattttt ttttttgatt ttatggtatt 540
gcgtagattt attcgttata atggtgatag tggatatttt tgttttattt ttgatgagaa 600
atcgaaaaat tttaatattt tattatttta tttatttttt tttttttgta gatggatttt 660
attagagtga gttattttat tttgttttaa atttgttgag agtatttatt tgaatatatg 720
ttgattttta ttaaatagtg tatttatttc gattattata gcgttttttt ttatttatgt 780
gttaatatag tgaattcgat tgataaattt gtacgttttt aggttcgatt attaaaattt 840
gagatagcgt tttattttgt tatcgaggtt ggagtgtagt ggtgttatta gagttcgttg 900

tagttttgat tttttgggtt taggcgtttt ttttatttta gttttttgag tagttgtgag 960
tataggtata tgttattatg tttagttaat ttttcgatgg ttttttgttt gttttttgta 1020
gtgatgagat tttttgatgt tgtttaggtt ggtttcgaag ttttgagttt aggtgatttg 1080
gttagtttag ttttttaaaa tattaggatt ataggcgtga gttttggttt ggtttggttt 1140
tttttatata ggggttttat ttatataaag attaaagtta atttgtgttt ttgtgcgggt 1200
gggttaagag tatgatgatt tttattattt tattgattta aagaaaatta tttttgattt 1260
attagtgtgt aaggttatga aagtataatt ttgttgaaag tatatattgt taatgggtgt 1320
tgggaatcgt gtatttttcg ttgttgtggg agtatgtttt tggaggtatt ttttatttgt 1380
ttttttaatt ttaaatattt taggattatg ggttgtgatt ggtaggatta tgtattttgt 1440
tgtttttaag acggagtata tttttacgtg gttttatttt ggttgttttg tttttttaat 1500
attgttattt tattttttgt gattttgatg ttataaatga tagatatcgt tttagtattt 1560
ttttacgggt tttagcgatt ttatttattt tttttttagt tttttttttt gatttgttta 1620
taatgaataa tttttttttg ggataggttg ttatttttgt tttcgtaggt ggtttatttg 1680
tttttttagt tagttatagt ggtttttgtt tttatggtgg gttcggggta agagagggtt 1740
ttgggttggg ggtggggttt agttgaagat ggggtgagtt ttgaggggag tattatttga 1800
gttttagagg tataggaaat agtagaggga ggtgggattt ttttattttt aatgaggatg 1860
ggtatggagg gtttggggcg tggcgttggg aacggtagtt ttttttagtt tatagtcgcg 1920
tatgtttttt gggttttcgt tttagtgcgt atgtttattg ggcgtttttt gttcggtttt 1980
ttcgtttacg tgaagaacgt tagggagttg tgaggtagtg ttgtgtggtt tttgtcgttc 2040
ggattttttt ttttttattg agatttattt ggtaggtttg taggttagtt atttcggggg 2100
ttgaagtgtg agtgagggtg gagagggttt tgggtgggtt aggcgggttt cgtttttggg 2160
tttgtggttt tcgagggaga agggttacga ggtcgttttt ttttttttta taggttgcga 2220
ggttatcggc ggtttcgtgg tcgtgaaggg gtttggacgg ggaggaaggt gggtcgtgga 2280
ggggaggcgg ttaggggttt aggtgaagat ggggtgagtg ttgttggggg gatggaagtt 2340
tcgaggtgtc gggaattttc gacgatatag ggtagatttt ttgaatgggg ttttcggcgg 2400
gggcgaggcg ggcggtgaag aaggggtttg gtatttggga aggttgcggt ttggtgagcg 2460
ttttttttag cggtgtggag tgcggagcgt ttgagtgaga 2500

<210> 69
<211> 2500
<212> DNA
<213> Artificial Sequence

<220>
<223> chemically treated genomic DNA (Homo sapiens)

<400> 69

ttttatttag gcgtttcgta ttttatatcg ttgggggggg cgtttattag gtcgtagttt 60
ttttaggtgt taggtttttt ttttatcgtt cgtttcgttt tcgtcggagg ttttatttag 120
ggaatttgtt ttgtgtcgtc gggggttttc ggtatttcgg gatttttatt tttttaatag 180
tatttatttt atttttattt gagtttttga tcgttttttt tttacggttt attttttttt 240
tcgtttaggt ttttttacga ttacgaagtc gtcggtggtt tcgtagtttg tgaagggaag 300
gaggacgatt tcgtggtttt tttttttcgg aggttataga ttaggaagcg ggattcgttt 360
gatttattta aggttttttt tatttttatt tatattttag ttttcgggat gattggtttg 420
tagatttatt agatgaattt tagtagagga aaaagagttc ggacggtagg aattatatag 480
tattgtttta tagttttttg gcgtttttta cgtgggcgaa ggggtcgggt agaagacgtt 540
tagtgaatat gcgtattgag gcgggagttt agggagtatg cgcggttgtg ggttggggag 600
ggttgtcgtt tttagcgtta cgttttaaat tttttatgtt tatttttatt gaggataagg 660
gaattttatt tttttttgtt gttttttatg tttttgggat ttaagtagtg ttttttttaa 720
aatttatttt atttttaatt gaattttatt tttaatttag ggtttttttt tgtttcggat 780
ttattatggg gataaggatt attgtgattg gttgggaaga taggtaaatt atttgcgaaa 840
atagaaatag taatttattt taaaaggaaa ttgtttattg tgaataagtt agagaaagag 900
attgaaagaa aaatgaatag aatcgttagg attcgtaaga aaatgttaaa acgatattta 960
ttatttgtag tattagaatt atagagggtg aagtgatagt attagggaaa taggatagtt 1020

agagtgaaat tacgtgaaaa tatatttcgt tttgaaagta gtaagatata tagttttatt 1080
agttataatt tatggtttta agatgtttgg agttgagaaa atagatgaaa ggtattttta 1140
aggatatgtt tttatagtag cggaaagtgt acggttttta atatttatta ataatatatg 1200
tttttaatag aattatgttt ttatggtttt atatattggt aagttaagga tagttttttt 1260
taaattaata gaatgataaa agttattatg tttttagttt attcgtataa aggtatagat 1320
taattttagt ttttatatag ataagatttt tatataagaa aaattagatt aggttaaggt 1380
ttacgtttgt aattttagta ttttgggagg ttgagttggt tagattattt gagtttagga 1440
tttcgagatt agtttaagta atattagaaa attttattat tataaaaaat aaataaaaaa 1500

ttatcgaaaa attagttggg tatggtggta tgtatttata tttatagtta tttaggaggt 1560
tgaggtggga ggagcgtttg agtttaggag gttaaggttg tagcgagttt tgataatatt 1620
attgtatttt agtttcggtg atagagtgag acgttgtttt aagttttaat aatcgaattt 1680
aaaaacgtat aaatttatta atcgaattta ttatattaat atatgaatgg gaaaaaacgt 1740
tgtggtaatc gaaatagatg tattgtttga tgaaaattaa tatatattta aatgaatatt 1800
tttagtaaat ttggaataga atggaatgat ttattttgat aaagtttatt tataaaaaaa 1860
ggagagtgaa taggatggtg aaatgttgaa atttttcggt tttttattag ggataagata 1920
aggatgttta ttgttattat tgtaacgggt gggtttgcgt aatgttataa aattagaaaa 1980
ggaaataaaa ttttttataa tggtaacgac gggttcgtta cggtggttta cgttagtaat 2040
tttagtattg tgggagatga cgtgggtgta ttatttgtat ttaaaagttt aataatagtt 2100
cgggaaatat ggtaaaattt cgtttttata aaaaaatata aaagggaaaa gaaaaaaaaa 2160
tggcggaaat aaaattgttt ttattttagg atgatatgtt tttgtatatt gaagattgaa 2220
aacgatttag aagtaaattt tagaatttat aagtatattt tataaggtta ttggatagaa 2280
aattaatatg taagaattat gtttttatcg ggcgcggagg tttatgtttg taattttagt 2340
attttgggag gttaaggtag gtgaattacg aggttaggag tttaagagta tttcggttaa 2400
gatggcgaaa gtttattttt agtaaaaata taaaatttag ttaggcgtgt tggtgggtgt 2460
ttataatttt agttattcgg gaggttgagg tagataattg 2500

<210> 70
<211> 2500
<212> DNA
<213> Artificial Sequence

<220>
<223> chemically treated genomic DNA (Homo sapiens)

<400> 70

ttgttttagt ttttcgagta gttgggatta taggtattta ttagtacgtt tggttaagtt 60
ttgtgttttt attagagatg ggttttcgtt attttggtcg gggtgttttt gaatttttga 120
tttcgtgatt tatttgtttt ggttttttaa agtgttggga ttataggtat gagttttcgc 180
gttcggtgga gatataattt ttatatattg gtttttattt tagtggtttt gtgaaatatg 240
tttgtgaatt ttaaagttta tttttaggtc gtttttagtt tttaatatat agaaatatat 300
tattttggaa taagagtagt tttgttttcg ttattttttt tttttttttt ttttgtattt 360
ttttgtagag acggggtttt gttatgtttt tcgggttgtt gttgaatttt tgagtgtaag 420
tgatgtattt acgttatttt ttatagtgtt gggattattg gcgtgggtta tcgtggcggg 480
ttcgtcgttg ttattgtaaa gagttttatt tttttttttg attttatggt attgcgtaga 540
tttattcgtt ataatggtga tagtggatat ttttgtttta tttttgatga gaaatcgaaa 600
aattttaata ttttattatt ttatttattt tttttttttt gtagatggat tttattagag 660
tgagttattt tattttgttt taaatttgtt gagagtattt atttgaatat atgttgattt 720
ttattaaata gtgtatttat ttcgattatt atagcgtttt tttttattta tgtgttaata 780
tagtgaattc gattgataaa tttgtacgtt tttaggttcg attattaaaa tttgagatag 840
cgttttattt tgttatcgag gttggagtgt agtggtgtta ttagagttcg ttgtagtttt 900
gattttttgg gtttaggcgt tttttttatt ttagtttttt gagtagttgt gagtataggt 960
atatgttatt atgtttagtt aatttttcga tggttttttg tttgtttttt gtagtgatga 1020
gattttttga tgttgtttag gttggtttcg aagttttgag tttaggtgat ttggttagtt 1080
tagtttttta aaatattagg attataggcg tgagttttgg tttggtttgg ttttttttat 1140
ataggggttt tatttatata aagattaaag ttaatttgtg tttttgtgcg ggtgggttaa 1200
gagtatgatg atttttatta ttttattgat ttaaagaaaa ttatttttga tttattagtg 1260
tgtaaggtta tgaaagtata attttgttga aagtatatat tgttaatggg tgttgggaat 1320
cgtgtatttt tcgttgttgt gggagtatgt ttttggaggt attttttatt tgttttttta 1380
attttaaata ttttaggatt atgggttgtg attggtagga ttatgtattt tgttgttttt 1440
aagacggagt atatttttac gtggttttat tttggttgtt ttgttttttt aatattgtta 1500
ttttattttt tgtgattttg atgttataaa tgatagatat cgttttagta tttttttacg 1560
ggttttagcg attttattta tttttttttt agtttttttt tttgatttgt ttataatgaa 1620
taattttttt ttgggatagg ttgttatttt tgttttcgta ggtggtttat ttgttttttt 1680
agttagttat agtggttttt gtttttatgg tgggttcggg gtaagagagg gttttgggtt 1740
gggggtgggg tttagttgaa gatggggtga gttttgaggg gagtattatt tgagttttag 1800
aggtatagga aatagtagag ggaggtggga tttttttatt tttaatgagg atgggtatgg 1860
agggtttggg gcgtggcgtt gggaacggta gtttttttta gtttatagtc gcgtatgttt 1920
tttgggtttt cgttttagtg cgtatgttta ttgggcgttt tttgttcggt tttttcgttt 1980
acgtgaagaa cgttagggag ttgtgaggta gtgttgtgtg gtttttgtcg ttcggatttt 2040
ttttttttta ttgagattta tttggtaggt ttgtaggtta gttatttcgg gggttgaagt 2100
gtgagtgagg gtggagaggg ttttgggtgg gttaggcggg tttcgttttt tggtttgtgg 2160

ttttcgaggg agaagggtta cgaggtcgtt tttttttttt ttataggttg cgaggttatc 2220
ggcggtttcg tggtcgtgaa ggggtttgga cggggaggaa ggtgggtcgt ggaggggagg 2280

cggttagggg tttaggtgaa gatggggtga gtgttgttgg ggggatggaa gtttcgaggt 2340
gtcgggaatt ttcgacgata tagggtagat tttttgaatg gggttttcgg cgggggcgag 2400
gcgggcggtg aagaaggggt ttggtatttg ggaaggttgc ggtttggtga gcgttttttt 2460
tagcggtgtg gagtgcggag cgtttgagtg agaagtattg 2500

<210> 71
<211> 2500
<212> DNA
<213> Artificial Sequence

<220>
<223> chemically treated genomic DNA (Homo sapiens)

<400> 71

tagtgttttt tatttaggcg tttcgtattt tatatcgttg gggggggcgt ttattaggtc 60
gtagtttttt taggtgttag gttttttttt tatcgttcgt ttcgttttcg tcggaggttt 120
tatttaggga atttgttttg tgtcgtcggg ggttttcggt atttcgggat ttttattttt 180
ttaatagtat ttattttatt tttatttgag tttttgatcg tttttttttt acggtttatt 240
ttttttttcg tttaggtttt tttacgatta cgaagtcgtc ggtggtttcg tagtttgtga 300
agggaaggag gacgatttcg tggttttttt ttttcggagg ttatagatta ggaagcggga 360
ttcgtttgat ttatttaagg ttttttttat ttttatttat attttagttt tcgggatgat 420
tggtttgtag atttattaga tgaattttag tagaggaaaa agagttcgga cggtaggaat 480
tatatagtat tgttttatag ttttttggcg ttttttacgt gggcgaaggg gtcgggtaga 540
agacgtttag tgaatatgcg tattgaggcg ggagtttagg gagtatgcgc ggttgtgggt 600
tggggagggt tgtcgttttt agcgttacgt tttaaatttt ttatgtttat ttttattgag 660
gataagggaa ttttattttt ttttgttgtt ttttatgttt ttgggattta agtagtgttt 720
tttttaaaat ttattttatt tttaattgaa ttttattttt aatttagggt ttttttttgt 780
ttcggattta ttatggggat aaggattatt gtgattggtt gggaagatag gtaaattatt 840
tgcgaaaata gaaatagtaa tttattttaa aaggaaattg tttattgtga ataagttaga 900
gaaagagatt gaaagaaaaa tgaatagaat cgttaggatt cgtaagaaaa tgttaaaacg 960
atatttatta tttgtagtat tagaattata gagggtgaag tgatagtatt agggaaatag 1020
gatagttaga gtgaaattac gtgaaaatat atttcgtttt gaaagtagta agatatatag 1080
ttttattagt tataatttat ggttttaaga tgtttggagt tgagaaaata gatgaaaggt 1140
atttttaagg atatgttttt atagtagcgg aaagtgtacg gtttttaata tttattaata 1200
atatatgttt ttaatagaat tatgttttta tggttttata tattggtaag ttaaggatag 1260
ttttttttaa attaatagaa tgataaaagt tattatgttt ttagtttatt cgtataaagg 1320
tatagattaa ttttagtttt tatatagata agatttttat ataagaaaaa ttagattagg 1380
ttaaggttta cgtttgtaat tttagtattt tgggaggttg agttggttag attatttgag 1440
tttaggattt cgagattagt ttaagtaata ttagaaaatt ttattattat aaaaaataaa 1500
taaaaaatta tcgaaaaatt agttgggtat ggtggtatgt atttatattt atagttattt 1560
aggaggttga ggtgggagga gcgtttgagt ttaggaggtt aaggttgtag cgagttttga 1620
taatattatt gtattttagt ttcggtgata gagtgagacg ttgttttaag ttttaataat 1680
cgaatttaaa aacgtataaa tttattaatc gaatttatta tattaatata tgaatgggaa 1740
aaaacgttgt ggtaatcgaa atagatgtat tgtttgatga aaattaatat atatttaaat 1800
gaatattttt agtaaatttg gaatagaatg gaatgattta ttttgataaa gtttatttat 1860
aaaaaaagga gagtgaatag gatggtgaaa tgttgaaatt tttcggtttt ttattaggga 1920
taagataagg atgtttattg ttattattgt aacgggtggg tttgcgtaat gttataaaat 1980
tagaaaagga aataaaattt tttataatgg taacgacggg ttcgttacgg tggtttacgt 2040
tagtaatttt agtattgtgg gagatgacgt gggtgtatta tttgtattta aaagtttaat 2100
aatagttcgg gaaatatggt aaaatttcgt ttttataaaa aaatataaaa gggaaaagaa 2160
aaaaaaatgg cggaaataaa attgttttta ttttaggatg atatgttttt gtatattgaa 2220
gattgaaaac gatttagaag taaattttag aatttataag tatattttat aaggttattg 2280
gatagaaaat taatatgtaa gaattatgtt tttatcgggc gcggaggttt atgtttgtaa 2340
ttttagtatt ttgggaggtt aaggtaggtg aattacgagg ttaggagttt aagagtattt 2400
cggttaagat ggcgaaagtt tatttttagt aaaaatataa aatttagtta ggcgtgttgg 2460
tgggtgttta taattttagt tattcgggag gttgaggtag 2500

<210> 72
<211> 2501
<212> DNA

<213> Artificial Sequence

<220>
<223> chemically treated genomic DNA (Homo sapiens)

<400> 72
   ggggattttt ttaattttta aagaatgtgc gtttttttaa gggtaatttg tagagtttga 60
   gaacggttta ggtttaggta tgtacggata gaaaggatat ttttttttta ggggtttagc 120
   gggggtagga gggggtggat agggatgtgt agggtaagtt tttttttgtt ttgtaggtat 180
   ttatttaatt tttgtttaaa gtagtaatag tgggtttttg tttttttatt tttaattttt 240
   tatatttttt ttgatagtag aggattagat tatttaggtg atggtgaagt tttttgacgt 300
   gtattaggtg taggagattt atgattttta gtattgtttt ttatttgttg ggtttttttt 360
   ggagaattat atatttttgg agttttagaa tatgttggtt gtataagaat aaagtatatt 420
   tattgtttga gaggaaggtt ggtggtgtgg tagtattatt gaattttttt ttttttttga 480
   gattatattg tatatatata gtgttatata attggatgtt ttaagttaat aatttaaaat 540
   gaatagtatt attattatag gaaatattat ttagtattgg ttttggtagt tatttatatt 600
   ttatttttaa gtgtgttata atttatttat atagtaagtt tatgattatg ttattataaa 660
   ttaagttgtt ttggaagttt tatgtattta aatttttgtt tatatggtta aagaaaaaaa 720
   atatttagtg gtagttgtta aagtatagta aggaaaattt tatttaggat tgtggtgatg 780
   ggtataggga ttattgtaat gggattttgt agtgggggag agagattggg tttaatattg 840
   aatatagcgt aggttagtgg gaatgtatag cgaaggagta gtatggcggt tcgtgggtgg 900
   aaaattatta aaaggaaata ttaggggtaa gggggatttt ggttaaattt atttagtagg 960
   atttttgttg aagataggtt aaggggaatt agatattatt tgggggatgg tggaagatga 1020
   agaattttat gcgatagtaa gtatgattag atattgagga tggagggttt atgttatatt 1080
   gatttagtag ggttttttgt taaaattggg ttttataagg aaatgtatag atgggtttag 1140
   gaggagaagt tttagaagtt tgatttaaaa gtttggttaa gtaaggaata tttgttaata 1200
   tttaagatag tttttttaat attaaagttt tagaagtaga aatgttgggt tataggattt 1260
   tttatttttt gagatagggt tttgttttgt tgtttaagtt ggagtgtagt ggtgtgattt 1320
   tggtttattg taattttaat tttttgggtt taagttattt ttttatttta gttttttaag 1380
   tagttgggat tataggtgtg tattattatg tttagttaat ttttgtattt tttgtaaaga 1440
   tagggttttg ttatgttgtt taggatggtt tcgaattttt gggtttaaga aatttattta 1500
   ttttagtatt ttaaagtttt aggattatag gtatgagttg tcgtatttgg tttggatttt 1560
   tgtgtaaatg tttaggttta tttatgtatg tgggtttatt attttttttt tttatatttt 1620
   tttttggatt tttattatgt ttaaaatggt tttgtatttt ttgagaattt aagtttttac 1680
   gtatttttgg gaagattgaa tattttttta agggttattg aggtagtagt attgagaaaa 1740
   aattaaaggg taaatttgta ggtgaaatgt agagttggga atatttttag atttagaagg 1800
   atagaatatt ttgtaggagg ggttagaagt tagaaataag ttttttttag tatcgagtag 1860
   gttttttttt taggtggtat agagttttta ggaggcggga gagggttgga aagataattt 1920
   acgtgatata ttttcgaggt ttgggattgg ttggtggggt tgatattgtt gaggtttaaa 1980
   taaggaggta gaagtaggta ggtgttattt tttttttttt tatttttcgt taggtttttt 2040
   gttgatttaa gtgtaagtga gaataggtgt gagtgagtgg gtcggcgggt ttttggtggg 2100
   tcgggtattg tggtttttgg ttttagagaa ggaagggttt cgaggttatt attttttttt 2160
   tagtaggtag ttatgcggcg gggtagggat aggggaggaa ggagggttat gaaggggagg 2220
   agggttagtt aagatgttgt gagttttgga gcgggatatt atttgagttt tggaggtata 2280
   tgaaattgtt tataaaggac ggatgttggg gtttttagtg ggggttaggg aaagggtggg 2340
   atagttcgcg tttgatagcg tttgttttag tgttcgtgtt tattgggggt ttttttatta 2400
   gtttttttat ttacgaggtg aattgtcgcg gagttgtgag ggtgtcgttt gtattttaat 2460
   tgtcgggatt tttttatttg agattgagat ttagtgggtg g 2501

<210> 73
<211> 2501
<212> DNA
<213> Artificial Sequence

<220>
<223> chemically treated genomic DNA (Homo sapiens)

<400> 73
   ttatttattg agttttagtt ttaggtgaaa gagtttcgat aattggaatg taaacggtat 60
   ttttatagtt tcgcggtagt ttatttcgtg ggtgaagggg ttgatgggaa gatttttagt 120
   gaatacgagt attgaggtag gcgttgttaa gcgcgggttg ttttattttt tttttggttt 180
   ttattgagga ttttagtatt cgttttttat ggatagtttt atatgttttt aggatttaga 240
   tagtgtttcg ttttaaaatt tatagtattt tggttgattt tttttttttt tatggttttt 300
   tttttttttt tgttttgttt tcgtcgtata gttgtttgtt aggagaagga tgatgatttc 360
   gaggtttttt tttttttgag gttagggatt atagtgttcg atttattaga agttcgtcgg 420
   tttatttatt tatatttgtt tttatttata tttgagttag tagagagttt ggcgaagaat 480
   gaagggaaga gaagtggtat ttatttattt ttgttttttt gtttagattt tagtagtgtt 540
   aattttatta gttaatttta aatttcgggg gtgtgttacg tgggttgttt ttttagtttt 600
   ttttcgtttt ttagaggttt tgtgttattt ggaggaaggg tttgttcggt gttgagaagg 660
   gtttgttttt ggtttttggt tttttttgta aaatgttttg tttttttgga tttaggaatg 720
   tttttagttt tgtattttat ttgtaaattt attttttaat ttttttttag tgttgttatt 780
   ttagtggttt ttaagagggt atttagtttt tttagagatg cgtgaaagtt taggttttta 840
   gagagtgtaa agttatttta agtatgatag gaatttagaa aaaaatgtaa aggagaagga 900
   tgataaattt atatatataa ataagtttaa atatttgtat aaaaatttag gttaggtgcg 960
   gtagtttatg tttgtaattt tagaattttg ggatgttgag gtgggtggat tttttgagtt 1020
   taggagttcg agattatttt gggtaatatg gtaagatttt gtttttataa aaaatataaa 1080
   aattagttgg gtatggtggt gtatatttgt agttttagtt atttgagagg ttgaggtggg 1140
   aggatagttt gagtttagga ggttgaggtt gtagtgagtt aagattatat tattgtattt 1200
   tagtttgggt aatagagtaa gattttgttt taaaaaataa aaaattttgt gatttagtat 1260
   ttttattttt aggattttaa tgttaaggaa attgttttga atgttgataa atattttttg 1320
   tttggttaaa tttttaagtt aggtttttga aatttttttt tttaggttta tttgtgtatt 1380
   tttttgtaaa atttagtttt agtaaagagt tttgttaagt taatgtggta tgaatttttt 1440
   atttttaata tttgattata tttgttatcg tatggggttt tttatttttt attatttttt 1500
   aggtgatgtt tgattttttt tggtttgttt ttagtaagaa ttttgttagg tgggtttagt 1560
   tagaattttt tttatttttg atgttttttt ttagtaattt tttatttacg gatcgttata 1620
   ttgttttttc gttatatatt tttattggtt tgcgttgtat ttagtattga gtttaatttt 1680
   ttttttttat tgtaagattt tattgtaatg gtttttatat ttattattat agttttaaat 1740
   aaggtttttt ttattgtgtt ttaataatta ttattgaatg tttttttttt ttaattatgt 1800
   gaataaaaat ttggatatat gggattttta aaatagttta atttataata gtatggttat 1860
   ggatttgtta tataagtaaa ttatggtata tttgaaaatg aaatatgggt gattattaaa 1920
   attagtgttg aataatattt tttgtaatag tggtgttgtt tattttaagt tattaatttg 1980
   aaatatttag ttatgtagta ttgtgtatgt ataatatgat tttaaaagaa aagagaggtt 2040
   taatagtgtt attatattat tagttttttt tttagatagt aagtgtgttt tgtttttgtg 2100
   taattagtat gttttggggt tttaaagata tatggttttt taaggggaat ttagtagata 2160
   aaaggtagtg ttaggggtta taggtttttt gtatttggtg tacgttaaag agttttatta 2220
   ttatttaggt ggtttgattt tttgttgtta gagaaggtgt ggggggttgg gggtaggggg 2280
   atagaagttt attgttattg ttttgggtaa aagttagatg ggtgtttgta agatagaaag 2340
   gagtttgttt tgtatatttt tgtttatttt tttttgtttt cgttaaattt ttgggggaaa 2400
   aatatttttt ttgttcgtgt atgtttggat ttggatcgtt tttagatttt gtaggttatt 2460
   tttaaagaag cgtatatttt ttgagagtta gaaagatttt t 2501

<210> 74
<211> 2501
<212> DNA
<213> Artificial Sequence

<220>
<223> chemically treated genomic DNA (Homo sapiens)

<400> 74

ttttattaat ataggaattt tttattaagg aaaataaaag ttttaaaaaa attttattgt 60
tttttttagt aattgtttga aagatttatt aattttttaa taaaaagtgt taaatgatat 120
tttttaattt taaaattttt tgaatttatt gtttaaaaag ttttgttgta tttattaatt 180
ttaaattatt gttattttta tttaatttta attaaattat tttttatttt aaaatattta 240
ttttatatcg gatatgaaaa gtataataaa tatatttaaa gtttttttat atttgatata 300
ttagtgtttt tgtggaggta gtgttttatt tttatagtttt atttaaataa tgttagcgtt 360
gttttatgaa taggttattt ggttgttatt tggggagtag agtatatgat gagtatagtt 420
attattgttg tatattatgt tataataaat taagtttaaa ttttaggatt aatgttgtat 480
tagttcgttt ttatgttgtt ataaggatat atttgagagt gggtaattta taaaggaaag 540
aggtttaatt gatttatagt tttgtagggt tggggaggtt tttggaaatt taataattat 600
ggaagaagtg gaagtaaata tatttttttt tatatggtag taggagagag aagagtgagt 660
aaaaggggaa aagtttttta taaaattatt agattttgtg aaaatttatt tattattagg 720
ggaatagtat gaaggtaatt attttatgat tatattattt tttatagggt tttttttatg 780
atatgtgggg attatgggaa ttataattta aggagagatt tgggtgggga tagagttaaa 840
ttatattata tgtatggata tttttgaatt atatattgtt tgaatgattt ataggtatat 900

ttagggaaat tagttattag tttagagatt aaaatgtgta ataatattat ttaaaaaagg 960
aagaggtgtg aatttattta gtttatagaa gtggttttat ttattggtag tatggtagaa 1020
aattttagat tagagaagga agtgattaat taaagtatga ataaaaattg gttaaggtgg 1080
gaggattatt cgattttaga aattgagatt agtttggata atgtagtgag attttgtttt 1140
taagtaaata aataaataaa atagttgggt atggtggtat atatttgtag ttttaattat 1200
ttcggaggtt gcgttttgag tattgtttat gtttaggaga tttaggttgt agtgaggtaa 1260
agttatgtta ttgtttttta tttagggcga taaagtaata ttttgtttgg aaaaaaaaag 1320
tatgtataaa tataaatatt aaaataattt atttttgtta taaaaggttt ttttgagata 1380
tattaattat taagggtgat atgggatttt ttgtttttaa agtttttgga tgtatgagaa 1440
tggtaggata agagagagag aaaaaaaaat attttttaag tttttttttt tttattataa 1500
atatattggg gattttggtt ttgggatagt gggaaaattt tggttggttt tgtatgtgtt 1560
tgatttagtg tgaacgtttt ttattgttta agttttttgt tgtttatgcg ttttattgtt 1620
tgttttttgt tttagtagag ttgtgtaaag agatatcgtg attttttttt gttttgtcga 1680
ttaggagtta gttagtaggt ttgtagttta gtagttttag aagtttgagt gtgagtgagt 1740
gtgaggagga gtttgcgggt acgatttatg gttttttagg tggaagggtt ttgaggtttt 1800
cgttttttta atacgtagcg atgtagttat ggttttgttg tggtgggggt gtcggataag 1860
ggagggagaa ggattataga tgggaggagg gttaggtaaa taagtagtga gttttgaagg 1920
gggaaattat ttgtggtatt tgagttcgcg aggtatatga aattgtttat agacgatgta 1980
ttttagagtt tttagtggga attagggagg ggagggagga ttgtatgacg cgggatgggg 2040
tggggcgagg cggggtattg tagtatataa cgtaggtatc gattttagtg tgtatgtttt 2100
ttggatattt gttttagtgt gtatgtttat tgggtatttt tttttcgatt tttttgttta 2160
cgtggtgatc gttggggagt tgtgagagtg tgaggggtac gttttagtcg tttggatttt 2220
ttttttttta ttgagacgta gtttataggt tcgtaggtta gtttttttag gaattgaaat 2280
gtgagtgaat gtgaggagga gtgagcgggt ttttagaggg ttaggcggcg tggtttgtgg 2340
ttttagagag taaaaagttt ttaggttata aggaaggtgg gtcgcggagt gaaggggagt 2400
tagatgaaga tagggtgaat gttcggggtg ttgttggagg tatttgagtt tcgaaagtgt 2460
ttggaatttt taatgggata gattttagat tttttggtag g 2501

<210> 75
<211> 2501
<212> DNA
<213> Artificial Sequence

<220>
<223> chemically treated genomic DNA (Homo sapiens)

<400> 75

tttattaaga agtttggaat ttgttttatt gggagtttta ggtattttcg ggatttagat 60
atttttaata gtatttcgag tatttatttt atttttattt ggtttttttt tatttcgcgg 120
tttatttttt ttatgatttg gaagtttttt attttttgag gttatagatt acgtcgtttg 180
attttttgga agttcgttta ttttttttta tatttattta tattttagtt tttgggagga 240
ttggtttgcg gatttatagg ttgcgtttta gtaggagaga aagagtttag acggttggaa 300
cgtgtttttt atatttttat agttttttag cggttattac gtgggtaaag gggtcgaagg 360
gaagatgttt agtgaatatg tatattgagg taggtgttta aggaatatgt atattgaagt 420
cggtgtttgc gttgtgtgtt gtagtgtttc gtttcgtttt attttatttc gcgttatgta 480
gttttttttt ttttttttgg tttttattga ggattttgga atgtatcgtt tgtaagtagt 540
tttatatgtt tcgcggattt aaatattata aatagttttt ttttttaaaa tttattgttt 600
gtttatttga tttttttttt atttatggtt tttttttttt ttttgttcgg tatttttatt 660
atagtaaggt tatggttgta tcgttgcgtg ttagaaggac ggggatttta aagttttttt 720
atttaggagg ttatggatcg tgttcgtagg ttttttttta tatttattta tatttaaatt 780
tttaggatta ttgaattgta gatttattgg ttgattttta gtcggtaaga tagaaggaag 840
ttacgatgtt tttttgtata gttttgttgg gatagaaggt agatagtaag gcgtatgggt 900
aataagggat ttgggtagta aggggcgttt atattgagtt aggtatatgt aaggttagtt 960
agggtttttt tattgtttta aaattagaat ttttagtatg tttgtagtag aaagaaaggg 1020
atttaagaga tatttttttt tttttttttt ttgttttatt atttttatgt atttagaaat 1080
tttgggaata gagagtttta tgttattttt agtggttgat gtgttttaga aagatttttt 1140
gtgatagaaa taaattattt taatgtttat atttatatat gttttttttt tttagatagg 1200
gtgttatttt gtcgttttgg atggagggta atggtatgat tttattttat tgtagtttgg 1260
attttttggg tataagtaat atttaagacg tagttttcga agtagttggg attataggtg 1320
tgtgttatta tgtttagtta ttttatttat ttatttattt agagatagaa ttttattata 1380
ttgtttaggt tggttttaat ttttgggatc gagtgatttt tttattttgg ttaattttta 1440
tttatatttt gattaattat tttttttttt ggtttgaggt tttttgttat gttattaatg 1500
aatagaatta tttttgtaga ttaaataaat ttatattttt ttttttttta ggtggtatta 1560

ttgtatattt taatttttgg attggtaatt gatttttttg aatatatttg taggttattt 1620
agatagtgta tggtttagaa atatttatat atatgatgtg gtttgatttt gtttttattt 1680
aaattttttt ttgaattgta gtttttataa tttttatatg ttatggaaag gattttgtgg 1740
gaggtaatgt aattatgggg tggttatttt tatgttgttt ttttgatagt gagtaagttt 1800
ttataagatt taatggtttt ataaggggtt tttttttttt tgtttatttt tttttttttt 1860
gttgttatgt gaagaaggat gtgtttgttt ttattttttt tatgattgtt aagtttttag 1920
aggttttttt agttttgtag aattatgagt taattaaatt tttttttttt ataaattatt 1980
tatttttagg tatgttttta tagtagtatg agaacggatt aatataatat tggttttagg 2040
atttaaattt agtttattgt gatatgatgt atagtagtgg tgattgtgtt tattatatat 2100
tttatttttt aaatagtagt taaataattt gtttataaga tagcgttgat attatttaaa 2160
taaagttata aggtagagta ttatttttat agaggtatta gtatgttaga tgtggaaggg 2220
ttttgggtat atttgttgtg tttttatgtt tcggtataaa gtgggtattt taaagtgggg 2280
gatggtttgg ttagaattgg gtaggaatga taatgattta ggattggtgg atgtaataag 2340
attttttagg taatgggttt aaagagtttt gggattgaaa aatgttattt gatatttttt 2400
attgaggagt tgatggattt tttaggtaat tgttggaaag aataataaag tttttttgaa 2460
atttttattt tttttggtaa gagatttttg tattagtaaa a 2501

<210> 76
<211> 2500
<212> DNA
<213> Artificial Sequence

<220>
<223> chemically treated genomic DNA (Homo sapiens)

<400> 76

ttttttttat cgttattttt attatttatt tttcgtttcg gggttttatt taggatttag 60
gaatgtgttt tgtgttatgg gtttttatta tttcgggatt ttgatttttt taatagtatt 120
cgtagtattc gttttatttt tatttgagtt tttgatcgtt ttttttttat ggtttatttt 180
tttttttgtt ttggtttttt tacgattata aagtcgatgg cggcgtcgta gtttgtgagg 240
agaaggagga cgagttcgag gttttttttt tttggaggtt attgaggttt ttttttttta 300
aaagttatat attattagat cgaggggttt atcgaatttg ttcgaagttt attggtttcg 360
tttgaagttt attggttttt ttttattttt atttatattt tagtttttgg gaggattggt 420
ttgcggattt attagatgag ttttagtaga ggaaaaaagt gttcggatag taggaacgcg 480
attgtatatt tttatagttt tttggcgttt tttacgtggg cgaaggggtc gacgggaagg 540
tatttagtga atatgcgtat tgaggttagt atttaaaggt ggggagggtt gtagttttta 600
gttttatatt ttatattttc gatttttatt ttcgtgaaga taatggaatt cgattttttt 660
ttgttgtttt atatgtttcg ggtatttaaa tattttaaat agtgtttttt ttaaaattta 720
ttttattttt aattgagttt tttttttttt tagggttttt ttttgttttg gttttgttat 780
agggataagg attatggtgg ttgtttgtga agtgaggtag acgatttgtt aaaatagaaa 840
tagtaatata ttttatagta ttatataaga ttaagagttt tataattaaa tattaaaaat 900
gtttagtata taatttaaaa ttatttagaa tatgaagaat taggaatata gggttattga 960
aatagggaaa gataattagt agatattatt gttaagatag agatgttggg attatgtggg 1020
aaagttttta aagatgttat tttataaacg tttaggttgg gcgcggttgt ttatgtttgt 1080
aattttagta ttttgggagg tcgaggcggg tggatttttt gaggttagga gttcgagatt 1140
agtttgatta atatggtgaa atttcgtttt tattaaaaat gtaaaaaatt agttgggtat 1200
ggtggcgcgt atttgtagtt ttagttattc gggagtttga aataggagaa ttgtttgaat 1260
ttaggaggta gaggttgtag tgaatcgaga ttacgcgatt gtattttggt ttggataata 1320
gtgagatttc gtttaaaaaa aaaatgttta aataagtaat tatgaatatt gttgaaataa 1380
gtaaaaataa agttttggta tatatataaa aaaagaatat ttaaagaaga agtaaatgga 1440
aattttagaa gtggaaaata tgataacgaa ggaaaataat tttttttaaa tggatttaat 1500
agtagaatgg agataagaaa agagtttgtg aatttaaaga tagagaaata gaaattgttt 1560
tgtgtggtta agagtagtga tttatattaa taattttagt atttttgcgt gttgaagtaa 1620
gaagatagat ggtttgaggt taggagtttt taatttgggt aatatagtga gattttgttt 1680
ttataaaaaa ataattaaaa atagttgagt gtgatgatat atgtttgtag ttttagttat 1740
tgtgaaggtt gaggtaagag gatttttggg gtttaggagt ttaaggttgt agtgagttat 1800
gattatatat tgtagtttag tttgggtaat agagtgagat tttgtgttta aaataaaata 1860
aaaagaaatt gttgaagtgt tttataattt gatatttaaa atgttaagta tataatttaa 1920
aattatttag aatacgaaga agtatgaaag ttaggtattt tggttaggtg tagtggttta 1980
tatttgtaat tttagtattt tgggaggttg aggtaggtag attacgaggt taggagttta 2040
agattagttt ggttaagatg gtgaaatttt atgtttatta aaaatataaa aattagttgg 2100
gtatggtggc gggtatttgt aattttagtt attcgggagg ttgaggtaga gaattttttg 2160
aattcgggag gtagagtttg tagtgagtta agatcgtgtt attgtatttt agtttgggtg 2220

atagagtgag gttttatttt aaaaaaaaaa aaggaagtta tggaatttta aatggagaaa 2280
gatagataat tgatattaat gtaaagatag agatgttgag attatttggg aaagttttta 2340
aagatgttag tttaaaaaag ttataaatat ttatgtttag atataagttt ttaatttttt 2400
tgggtagata ttaagaagta taattattgg attgaatagt aagagtttgt ttagttttgt 2460
aagaaatatt gttaaattgt tttttaaatt ggttttatta 2500

<210> 77
<211> 2500
<212> DNA
<213> Artificial Sequence

<220>
<223> chemically treated genomic DNA (Homo sapiens)

<400> 77
   tggtaaagtt aatttgaaag gtagtttggt agtatttttt ataaaattaa ataaattttt 60
   attatttagt ttagtagttg tatttttggg tatttattta aaggagttga aaatttgtgt 120
   ttggatatgg atgtttatag tttttttaaa ttagtatttt taaaggtttt tttagataat 180
   tttaatattt ttgtttttgt attggtgtta gttgtttgtt tttttttatt tgaaatttta 240
   taattttttt tttttttttt gaaatggagt tttattttgt tatttaggtt ggagtgtagt 300
   ggtacgattt tggtttattg taaattttgt ttttcgggtt taaggaattt tttgttttag 360
   tttttcgagt agttgggatt ataggtgttc gttattatat ttagttaatt tttgtatttt 420
   tggtagatat ggggttttat tattttggtt aggttggttt tgaattttgg atttcgtgat 480
   ttatttgttt tagtttttta aaatgttggg attataggta tgagttattg tatttggtta 540
   agatgtttaa tttttatgtt ttttcgtatt ttgagtaatt ttgaattata tgtttgatat 600
   tttgaatatt aggttatgag atattttaat aatttttttt tgttttgttt tagatatagg 660
   gttttatttt gttgtttagg ttggattgta gtgtgtgatt atagtttatt gtagttttga 720
   atttttgggt ttaagggatt tttttgtttt agtttttata gtagttagga ttataggtat 780
   gtgttattat atttagttat ttttaattgt ttttttgtag agataaggtt ttattatgtt 840
   gtttaggttg gaaattttta gttttaagtt atttattttt ttattttagt acgtaaaagt 900
   gttaggatta ttggtgtaag ttattgtttt tggttatata gaataatttt tattttttta 960
   tttttgagtt tataaatttt ttttttgttt ttattttgtt attgaattta tttggggaga 1020
   attatttttt ttcgttatta tattttttat ttttaaaatt tttatttgtt ttttttttaa 1080
   atattttttt tttgtgtgtg tgttaggatt ttgtttttat ttgttttaat agtgtttatg 1140
   attgtttgtt taagtatttt ttttttagac ggagttttat tgttgtttag gttagagtgt 1200
   agtcgcgtga tttcggttta ttgtaatttt tgtttttggg gtttaagtaa tttttttgtt 1260
   ttagattttc gagtagttaa gattataggt gcgcgttatt atgtttagtt aattttttgt 1320
   atttttagta aagacgaggt tttattatgt tggttaggtt ggtttcgaat ttttgatttt 1380
   aagagattta ttcgtttcgg ttttttaaag tgttgggatt ataggtatga gtaatcgcgt 1440
   ttagtttaag cgtttataaa atagtatttt taaaggtttt tttatataat tttaatattt 1500
   ttattttagt agtgatgttt gttgattgtt tttttttatt ttagtggttt tatgtttttg 1560
   gttttttata ttttgagtaa ttttgaatta tatgttagat atttttaata tttagttatg 1620
   agatttttag ttttgtataa tgttatgagg tatgttgtta tttttgtttt agtaggtcgt 1680
   ttattttatt ttataggtag ttattatggt ttttgttttt atggtagggt tagagtaaga 1740
   gagggttttg gaggggaggg gggtttagtt gaagatggaa tgagttttga ggggagtatt 1800
   atttgaggta tttgagtatt cgaggtatat gaaatagtag agggaggtcg gattttatta 1860
   tttttacggg gatgaaaatc gggagtgtgg ggtgtggggt tgggaattgt agtttttttt 1920
   atttttgggt gttagtttta gtgcgtatgt ttattgggtg ttttttcgtc ggttttttcg 1980
   tttacgtgga gaacgttagg gagttgtgag agtgtgtagt cgcgtttttg ttgttcggat 2040
   attttttttt tttattgaga tttatttggt agattcgtag gttagttttt ttaggggttg 2100
   aagtgtgagt gagggtgagg aggagttagt gggttttagg cggagttagt gggtttcggg 2160
   taggttcggt gggtttttcg gtttggtggt gtgtggtttt tgagggagaa gggttttagt 2220
   ggtttttaaa agagaagggt ttcgagttcg tttttttttt ttttataggt tgcgacgtcg 2280
   ttatcggttt tgtggtcgtg aaggggttag gatagggagg aaagtgggtt atgaagggga 2340
   agcggttagg ggtttaggtg aaaatgaggc gagtgttgcg ggtgttgttg gaggggttaa 2400
   agtttcgagg tggtaggaat ttatgatata gggtatattt ttgagttttg aatggggttt 2460
   cgaggcggga ggtgggtggt gaagatggcg gtaaagaagg 2500

<210> 78
<211> 2500
<212> DNA
<213> Artificial Sequence

<220>
<223> chemically treated genomic DNA (Homo sapiens)

<400> 78
   tttattttat tcggcgtttt atttttattt tttaaatttt atttttcgta tttggttttg 60
   tttttattta ttttttggtt ttgtttttta ttttttgatt ttgttgtttt cgttttacgg 120
   tttttatttc gttattagaa ttttttagaa tagaatagaa taggatataa ttgaagtagg 180
   cggttgttcg gttttttggt ttcgtagggg tattttttat tttgcgtaga agcggtatcg 240
   ttatgcggat ttcgcggggc gttttttttc ggtttcgagg tttttgttgt ttttgcgttt 300
   cggggatttt tgtcgttcgt ggtattcgtt ttttttgggt cgttagtatt gttttttggg 360
   ttattaggaa tgttagggtt ttttgggtta ttagtatcgt tcgtcgaatt ttttgtgttt 420
   tggttttcgg tttgtatggt ttcggagttt ttgttcggtt tttagagaga aggttagggt 480
   ttacgaggat gcggaggtag agaggttgta ggaagtttcg ttttttggcg tgagatgggt 540
   agttcgggat ttttagggcg tttgcgtata ggggttttat tttcggtttt gggagatttc 600
   gagtgagttt cggagtacgt gatcggtttt tattaatttc gttttttttt aagagagttc 660
   gggtcggaag gtggtcgtaa tgttagtttg gattttttat tcgtgagtag tcggttgttc 720
   gtcggatttt tgtttcggga gttttgtagg gagttaggta ttgcggggtt tagtttgttt 780
   tatttttcgg gtttttttta tatcgaggag taagacgggt ttgggaatac ggggtcggga 840
   ttgtgcggtt atcgtttcgg attttgtttg ttttgttcgt ttttggggga gcgtttagga 900
   tagatttcgg ggggtaggtt tttaattggg tttagtagtt ttcgtttttg ttttggtcgt 960
   ttagttggtg gggtagttgg aattgtatgt ttggtgggcg tggtttcgcg ttagttaagg 1020
   ttttttaggc gttaggttta gttaaggtgg acgggagggt ttagcgggat tcggggtcgg 1080
   attttatttt tgggtttaac gggttttgtt tttgttttag tcgtttcgtt tggtggggga 1140
   gttggagttg cgtgtggggt gggttgcggg tggtttttta ttagttaggg gttttcgggt 1200
   tttaggtttg gtttaggtgg ataggcggga tttgttggat ttgaggttgg gtcgggtttg 1260
   gtttttgggt tcggtagtat tagtttttgt tttggtcgtt tttgtttggt agggagttgg 1320
   gaggtgattt tgtgtttgtt gggtgttggt ttatttttgt gtttggaagg gggttagatt 1380
   tataggaaag gtgttgcgag aggcgggtag gggtgggggt ggtatggttt taggagttgg 1440
   gtgtttgttt ttacgttttg tttataagtt tgtggggcga gaggttgtaa tattttattt 1500
   cgggtgtttg ggttgtttat taggtcggtt taggtttttt atattttgta ttttgggttg 1560
   ggtatgaaat cgatttaatc gttttatagg ttgttttttt tgatatatat agaaattgat 1620
   ttttttggtt tttaagtttg aattttatag ttgttttatt tgaatttttt ttttaggaaa 1680
   tgattttcgg gttttttaaa atattaaaga attgaaattt attagataat tatatttaga 1740
   taatgagatg ttggattttt taaatttatt aggattgttt tttgggtttt ttcgagtttt 1800
   tgttttttta tatattggtg tatttttttt tggttatata aatttttagt tttagttagg 1860
   gggatggatt tgagatttaa tttttatttt ttcggttgta gtatttaatt aaagtcgttt 1920
   tttttggtaa tgtttttcgt tttagttagt tattggtttt ttgtgcggtt agtagtagga 1980
   cgtagattaa atttttggta tttcggtaat aggtatattt ttaggaaggg atttcggata 2040
   cggttattat ttttaggttt aaagcgaatg tatttttcgt atttttagtt tttcgtgtag 2100
   tagttttaat ttatgtatgt ttgtgttata tttagttttt agacggttga gtgattttaa 2160
   gaaaggtttt ttcgatattt agatagaggt tgtagggttg gggttgggtg agggtggcgg 2220
   gtttgcgggg atattttatt gtgttaaaaa gttattgtag atatagtaat aaaaatatgt 2280
   tattttttaa agtaggtttt tgtttttgtt tttgttgttt taagaagggg ttagggtgta 2340
   ggaggtaggg ggaatttttt ttagttggag ttgttgcggt gagtaaggtt ttgttttgga 2400
   ggtttttgag gttggtattt ttttggggat tgggaagggg gtagggaagg tagtagttta 2460
   gggaaagttt cgttttttcg gagtcgaggt agttggggag 2500

<210> 79

<211> 2500
<212> DNA
<213> Artificial Sequence

<220>
<223> chemically treated genomic DNA (Homo sapiens)

<400> 79

ttttttaatt gtttcggttt cggggggacg aggttttttt tgggttgttg ttttttttgt 60
ttttttttta gtttttagaa gggtgttagt tttagaggtt tttagagtag agttttgttt 120
atcgtagtag ttttagttgg aggaggtttt ttttgttttt tgtattttga ttttttttta 180
gagtagtaga ggtagaagta ggagtttgtt ttggaaaatg atatgttttt attgttatgt 240
ttgtagtggt tttttagtat agtagaatgt tttcgtaggt tcgttatttt tatttagttt 300

tagttttgta gtttttgttt gggtgtcggg gaagtttttt ttggggttat ttagtcgttt 360
gaggattgga tgtggtatag atatatatag gttaaagttg ttatacggaa gattggagat 420
gcgggaagtg tattcgtttt aagtttagag gtggtagtcg tgttcggggt ttttttttgg 480
gggtgtgttt gttatcgaaa tattaagggt ttggtttacg ttttgttgtt ggtcgtatag 540
aaagttaatg attgattgag acgaagagta ttattaggga agacggtttt aattgggtgt 600
tgtagtcggg gagatgggag ttaagtttta aatttatttt tttgattaaa attaggggtt 660
tatatagtta ggaagaaatg tattaatgtg taggaaaata ggaattcggg agggtttaga 720
aagtaatttt gatgaatttg gggggtttag tattttattg tttggatgtg gttatttggt 780
gagttttagt tttttgatat tttgagaggt tcgaaggtta ttttttgagg aaggaattta 840
gataaaataa ttgtaaggtt taagtttgaa gattagaagg gttagttttt atgtgtatta 900
aaaagaatag tttatgggac gattggatcg gttttatgtt taatttaggg tataggatgt 960
gggaagtttg agtcggtttg gtgagtagtt tagatattcg ggatggagta ttgtagtttt 1020
tcgttttata gatttgtgaa tagggcgtgg ggatagatat ttagttttta gggttatgtt 1080
atttttattt ttgttcgttt ttcgtagtat tttttttgtg gatttgattt ttttttagat 1140
atagaagtaa gttagtattt agtagatata gggttatttt ttagtttttt attaggtaag 1200
agcgattagg gtagggattg atattgtcga atttaggagt taggttcgat ttagttttag 1260
gtttagtagg tttcgtttgt ttatttgggt taggtttaga gttcgggagt ttttggttgg 1320
tgggaggtta ttcgtaattt attttatacg tagttttagt ttttttatta ggcggggcga 1380
ttaggatagg gatagaattc gttgaattta ggagtgagat tcggtttcgg gtttcgttgg 1440
gttttttcgt ttattttggt tggatttggc gtttgggaga ttttggttgg cgcgaggtta 1500
cgtttattag atatgtagtt ttagttattt tattagttgg gcgattagga tagggacgga 1560
ggttgttgag tttagttaga ggtttgtttt tcggggtttg ttttgggcgt ttttttaagg 1620
acggataggg taggtagggt tcgggacgat ggtcgtatag tttcggtttc gtgtttttag 1680
gttcgttttg tttttcgatg tgagggagat tcgggggatg ggataggttg ggtttcgtag 1740
tgtttgattt tttgtagggt tttcgggata ggggttcggc ggatagtcgg ttgtttacgg 1800
gtgaggggtt taagttggta ttgcggttat ttttcggttc gggttttttt ggggaggggc 1860
ggggttggtg agaatcggtt acgtgtttcg gggtttattc ggggtttttt agggtcggaa 1920
gtagggtttt tgtgcgtagg cgttttgagg atttcgggtt gtttatttta cgttaggggg 1980
cggaattttt tgtagttttt ttgttttcgt attttcgtgg gttttgattt tttttttgag 2040
agtcgggtag aggtttcgga gttatgtagg tcgaaggtta gggtataggg ggttcgacgg 2100
gcgatgttga tggtttagga ggttttggta tttttgatgg tttagggggt aatgttggcg 2160
gtttaggaga ggcgggtgtt acgggcggta gaggttttcg ggcgtagggg gtagtaaggg 2220
tttcggggtc gagaggaggc gtttcgcggg gttcgtatgg cggtgtcgtt tttgcgtagg 2280
atggaaggtg tttttgcggg gttaggaggt cggatagtcg tttgttttag ttgtattttg 2340
ttttgttttg ttttaggagg ttttggtggc gaggtggggg tcgtgagacg gagataatag 2400
ggttaagagg tggggggtag ggttaggagg tgggtgaggg tagggttagg tgcggaaggt 2460
ggggtttggg agatgggggt aaggcgtcgg gtggggtggg 2500

<210> 80
<211> 2501
<212> DNA
<213> Artificial Sequence

<220>
<223> chemically treated genomic DNA (Homo sapiens)

<400> 80

tgagggtgag gtggtagaag ggttggggtt ggtgttgggg ttggagggtg gtggttaggt 60
gacggagtag gagtagggat gggggtggag gtgtagttga ggagatgggg gttgggtggg 120
gaatgggaat gcgaatgggg tgggagtggg gattggagtg tagttttggg ggtttatagt 180
tttttttttt gggtgttgat tttgatgttt ggtagagttt gtgttttgtt tttttttaat 240
tagttttgtt ttggttatag taatttttga ttttagagtt tggggagaag aacgtggtgt 300
tttttaaggt ttttggagta ggggtttggg ggtagtttag gttgagacgt tttttcgcgg 360
gtgggttagg gttggtagta agggcggcgt tggaatattt ggggtttttt atttggagtg 420
gcggggtttt tagttttttt ttagtttttt attttgtttt tttatagagt ttgggttagg 480
ttttttcggt cgaattttac gtcgtttttt agaatggaaa tttcgttttt ttttgtttta 540
taatgcgtaa gttagtggcg ttatattcgg gtattcggat gttttttggc gggaatcgga 600
ttttgtttgg gtgggggtgg gagtgggcgt gggggtgggg gtgggggtga gggtggaatg 660
gttgggcgga tgggtattag ggttgaggga tggggtgggg gatgtggggt gggggttgga 720
atgataatga agagggggtt gggttacggg agtgagtatt ggagtggggg ttgggttacg 780
ggagtggggt tttaggattt tttagttttt tttgagaatt ttgagtatga tgtttggtag 840
agtttggacg ttgttttttt ttaagtagtt tttttttgat tatacgagtt ttgattttag 900
agtttttagt ggtttaagta gtaagggggt tgggggtggt ttagtttgag aagtttatta 960

ttagatggtt taggattggt atgaagggat tatttggggt tttttatttg gggtaagtgg 1020
gtttttagtt tttttttagt atgttatttt gtttttttat agagtttggg ttcgtttttt 1080
tttatcgatt ttaagtcgtt ttttagatcg aaaatttcgt ttttttttaa ttattaatgt 1140
ttaagttagt ggcgttatat ttgggtattt gggttttttt gggttgagag taggggtgga 1200
atcggatttt gtttggtttg ggttgggggt gagcgtgaag atgtttggat gggtggcgta 1260
aagggaggga ttgggggtgg tagggggagg gatgttagtg gtgtttcggg gtttaagtcg 1320
tcgtgttggt ggtagtgggg ttgggcgtgg ggttggggga ttttcggtta ttttttaggg 1380
ttttgatttt tatgtttggt agaatttgga tatttatatt ttttgtcgat tgtgtttgtt 1440
ttttttttat taagtttttg gtttttagtt ttatgaggtg gtaatgggag ggggggtgtg 1500
gtcgtggtaa tattgttagg gttttttagg gttgatagga ggggttgagt tgtattttgt 1560
ggtttattta cgtcgggaga gtggattttt tgtttttatt taggaaggtt ttttttttag 1620
tttttggtat tttgttgaag ttatgggcgg gagatgttgt ttttgttatt tttgagtatt 1680
tgtttagttg tatttattgt agagaatggt tgtaggtttt aggttaggtg ttttttgtag 1740
agtggtagtt ttcgcgattg tagtgatttt tgggagaaga cgtatatttt tttacggggg 1800
ttttttttta gttagagtta tatttggtaa attttttgtt tttaaagtaa tatgttttat 1860
ttttaggtac ggtggtttac gtttgtaatt ttaatagttt gagaggtcga ggcgggtaga 1920
ttatttgagg ttaggggttt aagattagtt tggttaatat agtgaaattt cgtttttata 1980
taaaatataa aaattagttg ggtgtggtag tgtttaggag gttgaggcgt gagaatcgtt 2040
tgtatttgga ggcggagttt gtagtgaggc gagatcgtgt tattgaattt tagcgtgggt 2100
ttcgcgatag agtgatattt tgttttaaaa aatttaaaaa atgaaaaaga aatatatttt 2160
tcgtattttt taaggatata tgtaggtagt tgtatatata tatgtattta tttttaggaa 2220
agaatattta ttagtagtat gtatgattta tttttatatt ttattttatt ttttttttat 2280
tttttacgtt ttgttgaaat aatttttttt gaaatataat tttttattta ttatgttggt 2340
gtttggttgt tattagtgga atttttgatt tatattgttt taattagttt tgtttaattg 2400
gtgttttgtt gttgttttag ggttattagt attatgtttt gaggagttgt tttagattgt 2460
gtaatgatgt gaagtggaaa tttgaaaatt tttattgtaa t 2501

<210> 81
<211> 2501
<212> DNA
<213> Artificial Sequence

<220>
<223> chemically treated genomic DNA (Homo sapiens)

<400> 81
   gttataatga aagtttttaa atttttattt tatattatta tatagtttga ggtagttttt 60
   taaaatatga tgttagtagt tttagggtaa tagtaggata ttagttaaat agggttggtt 120
   aagatagtat ggattaagag ttttattaat aataattaga tattaatata gtgggtgaga 180
   aattgtgttt taaaaaaaat tattttaata gaacgtaaag aatggaaaag aagtagaata 240
   ggatataaga gtgaattatg tatattattg gtaggtattt ttttttagaa ataaatatat 300
   atatatatat agttatttat atatattttt ggaagatacg aaaagtgtat ttttttttta 360
   ttttttaaat tttttgagat agagtgttat tttgtcgcga ggtttacgtt agagtttaat 420
   ggtacgattt cgttttattg taaatttcgt ttttaggtgt aagcgatttt tacgttttag 480
   ttttttgagt attattatat ttagttaatt tttgtatttt gtgtagagac ggggttttat 540
   tatgttggtt aggttggttt tgaatttttg attttaagtg atttgttcgt ttcggttttt 600
   taaattgttg ggattatagg cgtgaattat cgtgtttggg agtaaaatat attattttga 660
   ggataaaagg tttgttaagt gtagttttgg ttggagagaa gttttcgtgg gaaggtgtgc 720
   gttttttttt agaggttatt ataatcgcgg gagttgttat tttgtaggga gtatttggtt 780
   tgggatttgt agttattttt tgtaatgggt gtagttgggt aaatgtttag agatggtaga 840
   aatagtattt ttcgtttatg attttaataa agtgttagga gttgggagga ggattttttt 900
   gagtggggat agagggttta ttttttcgac gtagatgggt tatagaatgt agtttagttt 960
   tttttgttag ttttggaaga ttttggtaat gttattacga ttatattttt ttttttattg 1020
   ttattttatg ggattggggg ttagagattt ggtgggagag aagtagatat aatcggtaga 1080
   gaatatgggt gtttagattt tgttaggtat gaaagttagg attttgaggg gtgatcgagg 1140
   gttttttaat tttacgttta attttattat tattaatacg acgatttaag tttcggggta 1200
   ttattggtat tttttttttt attattttta attttttttt ttacgttatt tatttaaata 1260
   tttttacgtt tatttttagt ttaagttagg tagaattcgg ttttattttt gtttttaatt 1320
   tagggaagtt taggtgttta gatgtgacgt tattgatttg agtattagtg gttagagaga 1380
   agcgaggttt tcggtttgag gggcggtttg agatcggtgg agggaagcgg gtttaggttt 1440
   tgtaaggagg taaggtgata tgttgaggga ggattgagga tttatttatt ttagatagag 1500
   gattttaaat aattttttta tgttagtttt ggattatttg gtggtggatt ttttaggttg 1560
   ggttattttt agtttttttg ttgtttaaat tattggggat tttgaagtta gagttcgtgt 1620
   gattagggaa gggttgttta ggagagggta gcgtttaggt tttgttagat attatgttta 1680
   ggatttttaa ggagggttga gggtttttaa gattttattt tcgtgattta atttttattt 1740
   taatgtttat tttcgtgatt taattttttt tttattgtta ttttaatttt tattttatat 1800
   tttttatttt attttttaat tttgatgttt attcgtttag ttattttatt tttattttta 1860
   tttttatttt tacgtttatt tttattttta tttaggtagg attcggtttt cgttaggaaa 1920
   tattcgggtg ttcggatgtg acgttattga tttgcgtatt gtggggtaga gagaagcgag 1980
   gtttttattt tgagggacgg cgtagagttc ggtcgaagga atttgattta ggttttgtga 2040
   ggaggtaagg tgagaggttg agggaggatt gaggatttcg ttattttaaa tagagagttt 2100
   taaatatttt agcgtcgttt ttgttgttag ttttggttta ttcgcgggaa gacgttttag 2160
   tttgggttgt ttttagattt ttgttttaaa agttttgaga gatattacgt tttttttttt 2220
   aagttttgga attagaggtt gttgtgatta gggtaggatt ggttaggaga gggtagggta 2280
   taggttttgt taggtattaa gattagtatt taagagggag ggttgtgggt ttttaagatt 2340
   gtattttaat ttttattttt attttattcg tatttttatt ttttatttaa tttttatttt 2400
   tttagttata tttttatttt tatttttatt tttatttcgt tatttgatta ttatttttta 2460
   gttttagtat tagttttaat ttttttgtta ttttattttt a 2501

<210> 82
<211> 2501
<212> DNA
<213> Artificial Sequence

<220>
<223> chemically treated genomic DNA (Homo sapiens)

<400> 82
   gtagggttcg ggtttttttt ttgttttttt gaagtcgtat ttttggtatt aatatttttt 60
   tttttacgtt agttttagat gtcgacgtta gaatttgcgt ttttggtcgt tatgattagg 120
   gttttttagg gtcgatatta ggggtagagt tttttttgag cgtttcgata ttgatgttgg 180
   tagaatttag gcgcggtttt cgtcgaatta gtcgggtttt ggttatatta agttttgatt 240
   tttagagttt tttgcggttt aagcggcggg agagagtggg ggcgggggtg gggggggggt 300
   gatagtttag tttgagaagt ttgtttttag gtggtttaga gttggtagta gaggtagcgt 360
   tgtattattt agggtttttt atttgggttg ggggcgtttt tagttttttt ttagtttttt 420
   attttgtttt tttatagagt ttgggttcgt tttttttagt cgattttaag tttgtttttt 480
   agaacgagaa tttcgttttt ttttgatttt cgaggcgtaa gttagtggcg ttatattcgg 540
   ttttcggggt ttttttgggt tgatagtagg ggcggagtgg attttgtcgg aattggggtg 600
   ggagttgggg tgggagtgag ggtgggagtt cgggtggggt ggggtacggg tgaggatggg 660
   gttggggtag gggtggtggt gaagatgagt gtggggaggg gcgtggaggt gggaattggg 720
   atggggattt tggggttgtg ggtcgtggtg ggggtggggg tggggttttt tagttatttt 780
   ttagtgtttc gattttgatg tttggaaaag tttggatttt tgttttgtgt tgaggatgtt 840
   tttttttttt aaattaggtt ttttgattcg gagtttttta aggtggtagt gaggggaggg 900
   ttgtggtagg ggtgataata ttgttagggt tttttagggt tgataagagg ggtttagttg 960
   gattttgtgg tttttttgtg aggggtgggt ggattttcgg tttttattta ggagggtttt 1020
   ttttttggtt tttgtttttt tgatgaagtg atgggtggga gatgtttagt tttcgttatt 1080
   tttgattatt tgtttagttg tattttttgt agagtatggg tgcggttttt aggttagatg 1140
   gtttttgggg agttgtaata ttcgtgattg taatgattta tgggagaaga cgtatatttt 1200
   tttaacgggg ttttttttaa gttataagta gatttggtaa attttttgtt ttaaagggta 1260
   tattttttat aagaaagagg ttgagtatcg gggtgtggag aggttttgtt gttttgagtt 1320
   tagttgatat tttatagttt aatgtagtga tgtttggttt ttttaatttt atttttagta 1380
   aaaatatgtg ttttaaatgt ttgttaagtg tatattgaat atatatataa tattttatga 1440
   aagaatattt attttacgtt gtgtggttta ttgtttattt tatttttggt ttatttttta 1500
   tattttatta taataatttt tttttttttt tttgagtaag aaaatttaat ataaagaatt 1560
   attaatttgt agtagaggat taattaagat tagtcgttat tgtggtggtt ttaggtggtt 1620
   agtagtatag atttcggagt tatatttttt taattaattt cgtttttttt gttgttttga 1680
   tattatttta gagttattgg gttattggta tcgtggtatg aagtgttgtt ttagattgtg 1740
   taataaagta gggttagaaa tttaaatatt tttttttatt cgttttttta tttgtcgttt 1800
   atgtaggttg cggattttcg taaatgtatt gttgtttttt atttttttta ttttaaatat 1860
   aaggtttaat tcgaaatttt gtagtttgtt tagggtttta ttatgttttg ttgtgtagaa 1920
   tttgttgagt gttgataatg tttttttttt tttttttttt aggattgatt ttggagtgta 1980
   agttagtaat atgaatttga tgtttatttt ggtttaaagg taaggtatta attttgtaaa 2040
   taatttaagg aaaatttacg tttttaaaat attttgtttt ttattaatgg atatggtata 2100
   atgttttatt tatttgggat ttttttattt tgaatttatt agtaaatttt tatagttgat 2160
   tttagtaaga ttttgtttat ttttgttagg attattttta aggtaatttt aggagttttt 2220
   tgttgttttt aatgggatat tttttattgt atgttttatt tattgttttt ttatgattag 2280
   ttttttgatt ttgttataat gagtttttat aaaatatttt ttttttgaag ttttttattt 2340
   atttaaatat tttatatgtt tatttttttg attattcgtt aaagataata ttgtttttaa 2400
   atattttttt ttttaaaatt tatgtatttt atttagtttg atatttatag ttttggttat 2460
   gtgttttagt gtagttttga atagtagaga aggtagtata t 2501

<210> 83
<211> 2501
<212> DNA
<213> Artificial Sequence

<220>
<223> chemically treated genomic DNA (Homo sapiens)

<400> 83

gtatgttatt ttttttattg tttaaaattg tattgaaata tatagttaga gttatgggta 60
ttaaattaaa taagatgtat gaattttgaa aagagagatg tttggagata atgttatttt 120
taacgaatag ttaaaggaat aagtatgtgg aatatttaaa tgaataaaag attttagaag 180
aaaaatgttt tatggaagtt tattatagta aaattaagaa attggttata aaaaaatagt 240
aaatagaata tataatagaa aatattttat taaaaatagt aaaaaatttt taaaattatt 300
ttaaagataa ttttaataaa aatgagtagg attttattgg agttaattgt aaaagtttgt 360
tgatagattt aggtgaaaga agttttaaat aaataaagta ttatattata tttattaatg 420
gagaatagaa tattttaaag gcgtgaattt tttttaaatt atttatagaa ttagtgtttt 480
atttttaggt taaaatgagt attaagttta tattgttgat ttatatttta gaattaattt 540
tggaggggag aagggaaaaa atattattaa tatttaataa attttatata gtaaaatatg 600
atagaatttt aagtagattg tagaatttcg aattggattt tgtgtttgga gtagggggag 660
tggaggataa tagtgtattt gcggaggttc gtagtttgta tagacggtaa ataagaggac 720
gagtaggaag aaatatttga atttttggtt ttgttttatt gtataatttg aggtaatatt 780
ttatattacg atgttagtag tttagtagtt ttggagtgat attaggatag taggaaagac 840
ggggttgatt gagggagtat gatttcggag tttatgttgt taattattta aaattattat 900
agtgacgatt ggttttagtt gattttttgt tataagttaa taatttttta tattaaattt 960
ttttgtttag aaaaaaagag aaaaattatt ataatagaat ataaagaata gattagaaat 1020
agaataagta gtgaattata tagcgtaagg taggtatttt tttatgaaat gttatatatg 1080
tatttaatat atatttggta gatatttaaa atatatattt ttattaggaa tgagggttag 1140
aaaattagat attattgtat tggattataa aatattagtt aaatttaaaa tagtaggatt 1200
tttttatatt tcgatgttta gttttttttt tgtagaaaat atatttttta ggataaaaag 1260
tttgttaagt ttatttgtgg tttgggaaga gtttcgttgg gaaggtgtgc gttttttttt 1320
ataggttatt ataattacgg gtgttgtagt tttttaggga ttatttggtt tgggaatcgt 1380
atttatgttt tgtaagggat gtagttgggt aaatgattag aggtgacgga aattgagtat 1440
tttttattta ttattttatt aaagagataa aagttaggag gaagattttt ttgagtgagg 1500
atcgagggtt tatttatttt ttataaaggg gttatagaat ttagttgagt tttttttgtt 1560
agttttggaa gattttggta atgttgttat ttttattata attttttttt tattgttatt 1620
ttggaagatt tcgagttaag agatttggtt taaggggaga agatattttt agtataaaat 1680
aggggtttag gtttttttag gtattaaggt cgagatattg agggatgatt gaaagatttt 1740
atttttattt ttattacgat ttatagtttt aagattttta ttttagtttt tatttttacg 1800
ttttttttta tatttatttt tattattatt tttgttttaa ttttattttt attcgtattt 1860
tattttattc ggatttttat ttttattttt attttaattt ttattttagt ttcggtagaa 1920
tttatttcgt ttttgttgtt aatttaggga agtttcgggg atcggatgtg acgttattaa 1980
tttacgtttc ggggattaga gagaagcgag gttttcgttt tgagggatag gtttgagatc 2040
ggttgaagag agcgggttta ggttttgtga ggaggtaagg tgagaggttg agggaggatt 2100
gaggacgttt ttaatttaga tagaaagttt taaataatat agcgttgttt ttgttgttag 2160
ttttggatta tttgagggta gattttttag gttgggttgt tatttttttt ttattttcgt 2220
ttttattttt tttcgtcgtt taagtcgtag gggattttgg gagttagagt ttggtgtgat 2280
tagggttcgg ttggttcggc gggagtcgcg tttaggtttt gttagtatta atgtcgggac 2340
gtttagggag ggttttgttt ttgatgtcgg ttttgggaag ttttggttat ggcgattagg 2400
aacgtaaatt ttgacgtcgg tatttgaggt tgacgtaggg aaggggatat tggtattaag 2460
aatgcggttt taggggagta gagggaaggt tcgggttttg t 2501

<210> 84
<211> 2501
<212> DNA
<213> Artificial Sequence

<220>
<223> chemically treated genomic DNA (Homo sapiens)

<400> 84
   taggggtaaa gtttcgattt tgttaggatg ggtggggatg ggaatcgggg tttgggggtt 60
   tcgttgatgt ttgagcgatg gtggtgggtt tggtgttggg ggatgggggt ggggttggtg 120
   ttgggggatg ggaatggtgg tgggggtgcg ggggggttat ttttagtttt tttttggggt 180
   tttgattttg atttttggtt gagtttgggt tttgagtatt tttaattagt tttgttttgg 240
   ttatataaag tttttatatt agagtttttt gcggttaaag cggcgggggg tggcggggtg 300
   aggtggtggc gattgagttt gagaagtttt tttttgtagg gtggtttagg tttggtagta 360
   gaggtagcgt tggattattg ggggtttttt gtttggggtg aggttcgttt tagttttttt 420
   tcggtatttt attttgtttt tttacggagt ttgcgtttgt ttttttttat cgacgttagg 480
   tcgttgttta gggcgagaat ttcgttgttt tttgattttt aacgcgtaag ttagtggcgt 540
   tatattcggt ttcgtgattt ttttgggttt atagtagggg tggaattgga ttttttttgg 600
   atggggattt agatggaggt ggaggtaagt atgaagatgg tgttgggggt aggggtaggg 660
   atagggacgg ggattttgag gttgtagatt ttggtggggt tgggggagtt gggggtggga 720
   ggggtttttg gttatttttt aaggttttga tttggatgtt aggttgagtt tggatcgtta 780
   ttttttgtcg attgtgtttg ttttttttag attaagtttt tgattttgag tttttttgaa 840
   gtggtattgg gggaggggtg cggtcggggt gatattattg tttaagttta ttagggttga 900
   taagagggat tgagttggat tttgtggttt tattacgtgg gggagggtgg atttttagtt 960
   tttatttaag agggtttttt tttttatttt tggttgttgg atgaagtgat tggtaggaga 1020
   tattttgttt ttgtaacgtt tgtgtatttg tatagttgta ttttttgtag agaatggttg 1080
   cgggttttag gttaggagtt ttttgtaggg cgggagtttt tacgatcgta ttgatttttg 1140
   gaagaagata gatatttttt tacgggagtt tttttttagt tagagttata tttggtaaat 1200
   ttttggtttt aaatgattta tttattgaat tgaagaaata cggtttatat attttttaag 1260
   tatatatgta ggtagcgata tatatattta tttttacgaa ttaatattta tttttagtat 1320
   gtatcgttta tttttattat tttaatttat ttttatttta gtttttgtgt tttgttgaag 1380
   gaattttttt ttaaacgtaa tttttttttt attatgttgg ttttaggttg ttattagtag 1440
   aattttggag ttatgttgtt ttaataaatt taattttttt ggtgttttgt tattatttag 1500
   ggtttttggt atcgtgtttt taggagttgt tttagattgt gtaatgaagt gaagtggaaa 1560
   ttttaaaatt tttattttat ttattttgtt atttgtcgtt taggtaggtt ataaattttt 1620
   ttaatatttt tgtcgttaat tttttttaat cgatacggta agtgttatat ataagtatta 1680
   gtttaataag gtttttagtt ttattttgga ttggttttgt tttgttgtgt agattttttt 1740
   gttaatattg gttttttatt ttttgttttt attttataaa tttatagggt tgatttggga 1800
   agtagagtat agtagtttaa atttgtttgt aatgtcggcg attataggta aggtattaaa 1860
   ttgtaaataa tttaagttga gtttatattt ttatattgtt gtgtttttta tgaatgaata 1920
   tgttatattt ttttatgtat gttaagttat ttttttttga atatgttagt atatttcgat 1980
   agttgttttt ataagtagtt tatatatttt tattagattt ttatttaggt aatttttgga 2040
   ttttattgtt attgtaaatg gtatattgtt tattaattgt tgtagttagt tattgttttc 2100
   gtgaggtgaa gattttgatt ttgttgtagt aatttgttgt atattttatt ttgaaggtcg 2160
   atgtatttga atattttata tgtttttttt tttgaatttt taagatcgat aatggtatta 2220
   ttattaaata ttatatgttt ttttaatatt tatattattt atttattttg atatttatag 2280
   ttttttgtgt tttaattttg attttaaacg tagagataat aggttttatt tatttttcgt 2340
   ttttgattgg aatgatagtt tttcgaatat ttgatattta agtatgtttg ttgttgattt 2400
   taggaaatgg aagtgttttt tgaatatagg ttaaataaat attttttttt ttttataaat 2460
   ttattaaatg tttttaaaaa gttttttttg tatttttgga t 2501

<210> 85
<211> 2501
<212> DNA
<213> Artificial Sequence

<220>
<223> chemically treated genomic DNA (Homo sapiens)

<400> 85

gttaaagggt atagaaaaag ttttttaaag atatttaatg agtttatgga aaaaagaaaa 60
tatttattta atttatattt aaagagtatt tttatttttt aaaattaata gtaaatatat 120
ttaaatgtta aatgttcgaa gaattattat tttagttagg aacgagaagt agatgaagtt 180
tattattttt acgtttgaaa ttaggattga gatataaaaa gttataagta ttaaaatgag 240
taagtggtat gaatattgga aaaatatgta atgtttagtg ataatattat tatcgatttt 300

gaaaatttaa aggaaaaggt atgtggaata tttaagtgta tcggttttta gaatgaggtg 360
tgtaatagat tattatagta aaattaaaat ttttatttta cgaaagtaat aattaattat 420
agtaattaat agataatatg ttatttgtaa tagtaataaa atttaagaat tatttgaata 480
gaagtttaat aaaaatgtat aagttgttta tagaggtaat tatcgaagta tattgatata 540
tttaggaaaa aatagtttga tatatataga ggagtataat atatttattt atggggaata 600
taatagtgta aaaatgtaaa tttagtttaa attatttata atttagtgtt ttatttgtag 660
tcgtcgatat tataagtaaa tttgggttgt tgtgttttgt tttttaaatt aattttgtaa 720
atttatagaa tggaaataga aagtaaaaaa ttagtattag taagaaaatt tatataataa 780
aatagggtta gtttagggtg agattgaaga ttttgttgag ttggtatttg tgtgtgatat 840
ttgtcgtgtc ggttagaggg ggttagcggt agggatgttg gggaggtttg tagtttgttt 900
agacggtaga taatagaatg ggtagaataa aagttttaaa atttttattt tattttattg 960
tataatttga ggtagttttt gaaaatacga tgttaagagt tttaggtaat agtaggatat 1020
taggaaaatt gggtttgtta aggtagtatg gttttagagt tttgttaata ataatttgaa 1080
attaatatag tgggagagga attacgttta aaaaaaaatt tttttaatag aatataaaga 1140
ttggaataga agtagattag gatagtaaga gtgaacgatg tatattaagg gtaggtgtta 1200
gttcgtagaa ataaatatat atatcgttat ttatatatat atttggaaga tatgtaaatc 1260
gtattttttt aatttagtga ataaattatt taggattaaa ggtttgttaa gtgtagtttt 1320
ggttggagag gagttttcgt gggaaagtgt ttgttttttt ttagaggtta atacgatcgt 1380
gggagttttc gttttgtagg gagtttttgg tttgggattc gtagttattt tttataaggg 1440
gtgtagttgt gtaaatgtat agacgttata gaaatagagt attttttgtt aattatttta 1500
tttaatagtt aggagtgagg aagaggattt ttttgagtga ggattgaggg tttatttttt 1560
tttacgtagt gggattatag aatttagttt agtttttttt gttagttttg ataaatttag 1620
gtaataatgt tatttcgatc gtattttttt tttagtgtta ttttaggggg atttagagtt 1680
agagatttgg tttgagggga gtagatataa tcggtagagg atggcggttt aggtttagtt 1740
tggtatttaa gttaggattt tgagggatga ttaaaggttt tttttatttt taattttttt 1800
aattttatta ggatttatag ttttaggatt ttcgttttta tttttatttt tatttttaat 1860
attattttta tgtttatttt tatttttatt tggattttta tttaggaaga atttagtttt 1920
atttttgttg tgaatttagg gaagttacgg ggtcggatgt gacgttattg atttgcgcgt 1980
tggaggttag agaatagcga gattttcgtt ttgagtaacg gtttgacgtc ggtggaggga 2040
agtaggcgta ggtttcgtga ggaggtaagg taagatgtcg agggaggatt gaggcgggtt 2100
ttattttaga tagaggtttt ttaataattt agcgttgttt ttgttgttag gtttggatta 2160
ttttgtaggg gaagattttt taggtttagt cgttattatt ttatttcgtt atttttcgtc 2220
gttttaatcg tagggaattt tggtgtaaga gttttgtgtg attagggtag ggttggttag 2280
aagtgtttag ggtttagatt tagttaggaa ttaaggttag gattttaaga ggggattgag 2340
ggtaattttt tcgtattttt attattattt ttatttttta atattaattt tatttttatt 2400
ttttaatatt aaatttatta ttatcgttta aatattaacg ggatttttaa atttcgattt 2460
ttatttttat ttattttggt agaatcggag ttttgttttt g 2501

<210> 86
<211> 2501
<212> DNA
<213> Artificial Sequence

<220>
<223> chemically treated genomic DNA (Homo sapiens)

<400> 86

gtcgtatatg tgaacgttag gattcgcgtg tttggtcgtt attttcggag ttttcgtggg 60
ttgattgtag gggtaaagtt cggattttgt taggatggtg gtgggggagg agatgggaat 120
ggggtttggg ggtggggttg gtgtttgagg gatgggggtg gggttggtgt tgggggatgg 180
gattggtagt gagggtaggg ggtgggttgt ttttagtttt tttttggggt tttgattttg 240
atttttggtt gagtttgggt tttgagtatt tttaattagt tttgttttgg ttatataaag 300
tttttacgtt agagtttttt gcggttaaag cggcgggggg tggcggggtg aggtggtggc 360
gattgagttt gagaagtttt tttttgtagg gtggtttagg ttcggtagta gaggtagcgt 420
tggattattg gggttttttt gtttggggtg aggttcgttt tagttttttt tcggcgtttt 480
attttgtttt tttacggagt ttgcgtttgt tttttttcgt cgacgttagg tcgttgttta 540
gggcgagaat ttcgttgttt tttgattttt aatgcgtaag ttagtggcgt tatattcggg 600
ttcgtgattt ttttgggttt acggtaaggg tggaatcgga ttttgttcgg atggggattt 660
ggatgggggg gtgggggtaa gtatgaagat ggtgttggtg taggggtagg gattgggacg 720
gggatttgga ggttgtagat tttggtgggg tcgggggagt tgggggtggg aggggttttt 780
ggttattttt taaggttttg atttggatgt tagattgagt ttggatcgtt attttttgtc 840
gattgtgttt gtttttttta gattaagttt ttgattttga gtcggttcga agtggtattg 900
gggggagggg tgtgttcggg gtgagattat tgttagtgtg tattagggtt gataagaggg 960

gttgagttgg attttgtggt tttattatgt gggtgagggt ggatttttag tttttattta 1020
ggagggtttt tttttttatt tttggttgtt tgatgaagga atgggtggga gatgttttgt 1080
ttttgttatt tttgagtatt tgcgtatttg cgtatttgta ttttttgtag agaatggttg 1140
cgggtgttag gttaggtgtt ttttgtaggg taggagtttt cgcgatgata gtgatttttg 1200
ggagaagacg tatatttttt tacgggggtt tttttttagt tagagttata tttggtaaat 1260
ttttggtttt aaatgattta ttttttgaat tgaagaaata cggtttatat attttttaag 1320
tatatatgta ggtagtaata tatatattta tttttatgaa ttaatattta tttttagtat 1380
gtatgattta ttttgatatt ttagtttatt tttattttag tttttgtgtt ttgttgaaag 1440
aattttttta aaaaatataa tttttttttt attttgttgg ttttaggttg ttattagtag 1500
aattttggag ttatgttgtt ttaataaatt taattttttt ggtggatatt gttattattt 1560
agggtttttg gtatcgtgtt tttaggggtt gttttagatt gtgtaatgaa gtgaagtgga 1620
aaatttaaaa tttttatttt atttattttt ttatttgtcg tttaggtagg ttataaattt 1680
ttttaatatt tttgtcgtta atttttttta atagatattg taagtgttat atataagtat 1740
tagtttaata tagtttttag ttttattttg ggtttttttt gttttgttgt gtagattttt 1800
ttgttgatat tggtgtttta ttttttgttt ttattttata aatttatagg gttgatttgg 1860
gaagtagagt atagtagttt aaatttgttt gtaatgtcgg cggttatagg taaggtatta 1920
aattgtaaat aatttaagtt gagtttatat ttttatattg ttgtgttttt cgtgaatgaa 1980
tatgttatat ttttttatgg attttaagtt tttttttttt gaatatgtta gtatatttcg 2040
gtagttgttt ttataaataa tttatatatt tttattaggt ttttatttag gtaatttttg 2100
gattttgttg ttattgtaag tggtatattg tttattaatt gttgtagtta gttattgttt 2160
tcgtgaggtg aagattttga ttttgttgta gtaatttgtt gtatatttcg ttttgaaggt 2220
cgatgtattt gaatatttcg tatatttttt tttttgaatt tttaagatcg ataatggtat 2280
tattattaaa tattatatgt ttttttaatg tttatattat ttatttattt cggtatttat 2340
agttttttgt gttttaattt tgattttaaa tgtagagata ataggttata tttatttttc 2400
gtttttgatt tgaatgataa tttttcgaat atttgatatt taagtatgtt tgttgttgat 2460
tttaggaaat ggaagttttt ttttagtata ggttgtttaa a 2501

<210> 87
<211> 2501
<212> DNA
<213> Artificial Sequence

<220>
<223> chemically treated genomic DNA (Homo sapiens)

<400> 87

tttaggtaat ttatattgaa agagaatttt tattttttaa aattaatagt aaatatattt 60
aaatgttaaa tgttcgaaga attattattt aagttaggaa cgagaagtag atgtagttta 120
ttatttttat atttgaaatt aggattgaga tataaagagt tgtaagtatc gaaatgagtg 180
agtggtatga atattggaaa aatatgtagt gtttagtgat aatattatta tcgattttga 240
aaatttaaag gaaaaggtat gcggaatatt taagtgtatc ggtttttaga acgaggtgtg 300
taatagatta ttatagtaaa attaaaattt ttattttacg aaagtaataa ttaattataa 360
taattgatag ataatatgtt atttgtaata gtaataaaat ttaagaatta tttgaataga 420
agtttaataa aaatgtataa gttgtttata gaggtaatta tcgaagtata ttgatatatt 480
taggaaaaaa gagtttgaaa tttatagagg agtataatat atttatttac ggggaatata 540
atagtgtaaa aatgtaaatt tagtttaaat tatttataat ttagtgtttt atttgtagtc 600
gtcgatatta taagtaaatt tgggttgttg tgttttgttt tttaaattaa ttttgtaaat 660
ttatagaatg gaaatagaaa gtaaaatatt agtattagta agaaaattta tataataaaa 720
tagggaaagt ttagagtgag attggagatt gtgttgagtt ggtatttgtg tgtgatattt 780
gtagtgtttg ttggaggggg ttagcggtag agatgttggg gaggtttgta gtttgtttag 840
acggtagatg aaagaatggg tagaataaaa gttttgaatt ttttatttta ttttattgta 900
taatttgagg tagtttttga aaatacgatg ttaagagttt taggtaatag tagtgtttat 960
taggaaaatt gggtttgtta aggtagtatg gttttagagt tttgttaata ataatttgaa 1020
attaatagag tgggagagga attatgtttt ttaaaaaaat ttttttaata gaatataaag 1080
attggaatgg aagtagatta ggatattaga gtgaattatg tatattaagg gtaggtgtta 1140
gtttatagaa ataaatatat atattgttat ttatatatat atttagaaga tatgtaaatc 1200
gtattttttt aatttagaga ataaattatt taggattaaa ggtttgttaa gtgtagtttt 1260
ggttggggag gagttttcgt gggaaggtgt gcgttttttt ttagaggtta ttattatcgc 1320
gggagttttt gttttgtagg gagtatttgg tttggtattc gtagttattt tttataaggg 1380
gtgtagatgc gtagatgcgt agatgtttag aggtgataga aatagagtat tttttattta 1440
tttttttatt aaatagttag gagtgaggaa gaggattttt ttgagtgagg attgaggatt 1500
tatttttatt tatatagtgg gattatagaa tttagtttag ttttttttgt tagttttggt 1560
atatattggt aatgatttta tttcgagtat attttttttt ttaatgttat ttcgggtcga 1620

tttagagtta gagatttggt ttgaggggag tagatataat cggtagagga tggcggttta 1680
ggtttagttt ggtatttaag ttaggatttt gagggatgat taaaggtttt ttttattttt 1740
aattttttcg attttattag gatttatagt tttaggattt tcgttttaat ttttattttt 1800
atattaatat tatttttatg tttattttta tttttttatt tagattttta ttcgggtaga 1860
attcggtttt atttttgtcg tgaatttagg gaagttacgg gttcggatgt gacgttattg 1920
atttgcgtat tggaggttag aggatagcga gattttcgtt ttgagtaacg gtttgacgtc 1980
ggcggaggga agtaggcgta ggtttcgtga ggaggtaagg taagacgtcg agggaggatt 2040
gaggcgggtt ttattttaga tagagggttt ttaataattt agcgttgttt ttgttgtcgg 2100
gtttggatta ttttgtaggg gaagattttt taggtttagt cgttattatt ttatttcgtt 2160
atttttcgtc gttttaatcg tagggaattt tggcgtaaga gttttgtgtg attagggtag 2220
ggttggttag aagtgtttag ggtttagatt tagttaggaa ttaaggttag gattttaaga 2280
ggggattgag ggtaatttat tttttatttt tattattaat tttatttttt aatattaatt 2340
ttatttttat tttttaaata ttaattttat ttttaaattt tatttttatt ttttttttta 2400
ttattatttt ggtagaattc gggttttgtt tttgtaatta atttacggaa gtttcgggaa 2460
tggcggttaa gtacgcggat tttgacgttt atatgtacgg t 2501

<210> 88
<211> 2501
<212> DNA
<213> Artificial Sequence

<220>
<223> chemically treated genomic DNA (Homo sapiens)

<400> 88

tattttcgga gttttcgtga tttgatatta ggggtaaagt tcggattttg ttaggatagg 60
ggtgggggtg gggatgggaa tgggggatgg gggatggggg tggggttggt gttgggggac 120
gggaatggtg gtgagggtgg tggttaggtt gtttttagtt ttttttcggg gttttgattt 180
tgatttttgt tatagcgtgg gttttgattt tttttaatta gttttgtttt ggttatatta 240
agttttgttt ttagagtttt tcgtggttaa agcggcgggg gtcggcgggg tgaggtagtg 300
gcgatttagt ttggaagttt ttttttgcgg ggtggtttag gttcggtagt agaggtagta 360
ttggattatt tgaggttttt tgtttgaggt gaggttcgtt ttagtttttt tttagcgttt 420
tattttgttt ttttatcgag tttgggtcgg ttttttttcg tcgacgttag gtcgtcgttc 480
gttgtttagg gcgagaattt cgcggttttt tgatttttaa tgcgtaagtt agtggcgtta 540
tattttggta acggtattat tttgggtttc gggtaggggt ggaattggat tttgtttgga 600
tggggatcgg atgggggtgg aggtaagtat gaagatggtg atggggtaag gggtaaggat 660
tgggacgggg gttttgaggt tgtagatttt ggtggggtcg ggggagttgg gggtgggtgg 720
gcggggtttt cggttatttt ttagggtttt gaagttgatg tttggttgag tttggatcgt 780
tattttttgt agattgtttt tgtttttttt agattaagtt tttgattttg agtttttttg 840
aagtggtatt gggggaaggg gtgtggtttg ggtgatatta ttgtttgcgt ttattagggt 900
tgataagagg ggttgagttg gattttgtgg tttttttatg gtgtgtgggg tgggggggcg 960
gtgtggattt ttagttttta tttaggaggg tcgttttcgt tatttttggt tgttggatga 1020
agtgatgggt aggagatgtt ttgtttttgt tatttgtgag tatttgtata gttgtatttt 1080
ttgtagagaa tgtttgcggg ttttaggtta ggtgtttttt gtagggcggg gcggtagttt 1140
tcgcggttgt agtgattttt tattttagtt tttgtgtttt gttgaaagaa tttttttttt 1200
taaaaaatgt aatttttttg ttattatggt ggttttaggt tgttattagt agaattttgg 1260
agttatattg ttttaattaa ttaaattttt ttggtgtttt gttattattt agggtttttg 1320
gtatcgtgtt tttagaggtt gttttagatt gtgtaaagaa gtgaagtgga aaattttaaa 1380
tttttatttt atttattttg ttatttatcg tgtaggtcgg ttataaattt ttttaatatt 1440
tttgttgtta atttttttta attaatattg taagtgttaa atataagtat tagtttaata 1500
tagttttgag ttttattttg ggtttttttt gttttgttgc gtagattttg ttgttgatat 1560
tggtttttta tttattgttt ttattttgta attttttagg tttgatttgg gaagtagagt 1620
atagtagttt aaatttgttg taatggcggc ggttataggt aaggtattaa attgtaaata 1680
atttaagttg agtttatatt tttatagtgt tgtgtttttt atgaatgaat atattatatt 1740
tttttatgta ttttaagttt ttttttattt gaatatgtta gtatatttcg atagttgttt 1800
ttataaagat tttatatatt tttattagat ttttttttag gtaatttatg aattttgttg 1860
ttattgtaaa tggtatattg tttattaatt gttgtagtta gttattgttt tcgtgaggtg 1920
aagattttga ttttgttgta gtaatttgtt gtatttttcg ttttgaaggt cgatgtattt 1980
gaatatttta tatatttttt tttttgaatt tttaagatcg ataatggtat tattattaaa 2040
tattatatat ttttttagta tttatattat ttatttattt tggtatttat agttttttgt 2100
gttttaattt tgattttaat cgtagagata atagtttata tttatttttc gtttttgacg 2160
tgaatgataa ttattcgaac gttttgtatt taagtatgtt tgttgttgat tttaggaagt 2220
gtaatttttt tttgaatata ggttaaatat tttttttttt ttttattatg aatttatatt 2280

aaattttttt aaaaagtttt ttttgtattc gttgatataa ttaaattttt ttttttttta 2340
ttttgtatgt aaggaattat aatgaaatcg tttttttttt tttttttaat gttaaatcgt 2400
atgttatttt tttgggtaga gtttgtttat ttaatatgtt agtaatttac gtaggatatt 2460
tgttgttatg taagggagtt tatttaatgt gtgttttttt t 2501

<210> 89
<211> 2501
<212> DNA
<213> Artificial Sequence

<220>
<223> chemically treated genomic DNA (Homo sapiens)

<400> 89

aaaaaaagta tatattaagt aagttttttt atatagtagt aaatatttta cgtaaattat 60
tagtatatta aataaatagg ttttgtttaa ggaaataata tacggtttaa tattagggga 120
aaaaaaaaaa acggttttat tgtaattttt tatatataaa atggaggaga aaaaaatttg 180
attgtgttaa cgggtataga aaaagttttt taaagaaatt taatatgagt ttatggtaaa 240
aaaaaaaaaa agtatttaat ttatatttaa agagaaattg tattttttaa aattaatagt 300
aaatatattt aaatgtaaaa cgttcgaata attattattt acgttaggaa cgagaagtag 360
atgtaggtta ttatttttac gattgaaatt aggattgaga tataaagagt tataagtatt 420
aaaatgagta agtggtatga atattggaag aatatgtaat gtttagtgat aatattatta 480
tcgattttga aaatttaaag gaaaaggtat gtggaatatt taagtgtatc ggtttttaga 540
acgaggagtg taatagatta ttatagtaaa attaaaattt ttattttacg aaagtaataa 600
ttaattataa taattaatag ataatatgtt atttgtaata gtaataaaat ttatgaattg 660
tttgaaaaga agtttaataa aaatgtataa ggtttttata gagataattg tcgaagtata 720
ttgatatatt taggtaaaaa aaagtttgaa atatatagag gagtataata tatttattta 780
tggggagtat aatattgtaa aaatgtaaat ttagtttaaa ttatttataa tttagtgttt 840
tatttgtagt cgtcgttatt ataataaatt tgggttgttg tgttttgttt tttaaattaa 900
atttggaaaa ttatagaatg gaaatagtaa ataaaaaatt agtattagta ataaaattta 960
cgtaataaaa tagggaaagt ttagggtgag atttaaggtt gtgttgagtt ggtatttgtg 1020
tttgatattt gtagtgttgg ttggaggggg ttagtagtag ggatgttggg gaggtttgta 1080
gtcggtttat acggtagatg atagaatggg tagaataaaa gtttgaaatt ttttatttta 1140
tttttttgta taatttgagg tagtttttga aaatacgatg ttaagagttt taggtaatag 1200
taggatatta ggaaaatttg gttggttaag gtagtatgat tttagagttt tgttaataat 1260
aatttgaaat tattatagtg gtagaggaat tatatttttt aaaaaaaaaa atttttttaa 1320
tagaatataa agattgaaat aggaggttat tataatcgcg ggagttgtcg tttcgttttg 1380
tagggagtat ttggtttggg attcgtaggt attttttata aggggtgtag ttgtgtaaat 1440
gtttataggt gatagaaata gagtattttt tgtttattat tttatttaat agttagaggt 1500
gacgaagacg atttttttga gtgaggattg agggtttata tcgttttttt attttatata 1560
ttatagaggg attatagaat ttagtttagt tttttttgtt agttttggta aacgtaggta 1620
gtgatgttat ttagattata tttttttttt taatgttatt ttagggggat ttagagttag 1680
agatttggtt tgaggggagt agaagtaatt tgtagaggat ggcggtttag gtttagttag 1740
gtattaattt taggattttg agggatgatc gaaggtttcg tttatttatt tttaattttt 1800
tcgattttat taggatttat agttttagga ttttcgtttt aatttttatt ttttgtttta 1860
ttattatttt tatgtttatt tttattttta ttcgattttt atttaggtag aatttagttt 1920
tatttttgtt cggaatttag ggtagtatcg ttgttaggat gtgacgttat tgatttgcgt 1980
attggaggtt agaagatcgc gagattttcg ttttgagtaa cgagcgacgg tttgacgtcg 2040
gcggagggaa gtcggtttag gttcggtgag gaggtaaggt aagacgttga gggaggattg 2100
aggcgggttt tattttagat agagggtttt aaataattta gtgttgtttt tgttgtcggg 2160
tttgggttat ttcgtagggg aagattttta ggttgggtcg ttattatttt atttcgtcga 2220
ttttcgtcgt tttagttacg gggaattttg gggatagagt ttaatgtggt tagggtaggg 2280
ttggttagaa gaggttaggg tttacgttgt ggtaggaatt aaggttagga tttcgagagg 2340
gaattgaggg tagtttaatt attattttta ttattatttt cgttttttaa tattaatttt 2400
atttttattt tttatttttt atttttattt ttatttttat ttttattttg gtagaattcg 2460
ggttttgttt ttggtattaa gttacggaag tttcgggaat g 2501

<210> 90
<211> 2501
<212> DNA
<213> Artificial Sequence

<220>

<223> chemically treated genomic DNA (Homo sapiens)

<400> 90

gggaagcggg ttaggttttg tgaggagtta aggtgagacg ttgagggagg atttaggagg 60
tttttatttt agatagatga ttttaaataa tttcgtatta tttttgttat tagtcgtggg 120
ttattcgtgg gcggattttt gagtttgggg cgtttattat tttattgttt ttgaagttgt 180
aggggatttt ggagttagag tttgggttga ttagtgtaag attagtgagg gtaggttttg 240
ttaggtatgg attttagtat tttaagagag ggttaagcgt attttatttt tatttttatt 300
ttttattacg tttttttttc gattattatt tgtattttta aattatttac gtttttattt 360
tttattagta tttttttttt ttttaatttt ttattttttt tatatcgtta ttttttattt 420
taataattcg ggttttttta ttaattttat tttttttatt tttatttata ttgaattacg 480
tttcgttttc gttttaattt tatttttaaa aattcgtttt ttttatcgat tttatttttt 540
ttatttttat ttacgttgaa tttgattttc gttttttttt ttaatttaag aaagttttag 600
gggttcggat gtgatgttat tgatttgcgt attgggggtt agagagaagc gagttgtttt 660
gtttgattag tagtttggga ttggcggagg gaagcgggtt aggttttgtg aggagttaag 720
gtgagacgtt gagggaggat ttaggaggtt tttattttag atagacgatt ttaaataatt 780
tcgtattatt tttgttatta gtcgtgggtt attcgtgggc ggatttttga gtttggggcg 840
ttttttattt tattgcgttt gaagtcgtat gggattttgg agttcgagtt tggggtggtt 900
agtgtaagat tagtgagggt aggttttacg aggtattaat tttaggattt taagagaggg 960
ttaagcgtat tttattttta tttttatttt ttatcgtttt ttttttttta ttattatttg 1020
tatttttaaa tttattcgcg tttttatttt ttattagtat ttttattttt aatttcgtat 1080
tttttttata tcgttatttt ttattttaaa aattggggtt tttttattaa ttttattttt 1140
tttattttta tggatgttga attgggttgc gttttcgttt ttaatttatt tttaaaaatt 1200
cgtttttttt atcgatttta ttttttttat ttttttttac gttgaatcgg gttttcgttt 1260
tcgtttttaa tttaagaaag ttttaggtgt tcggatgtga tgttattgat ttgcgtattg 1320
ggggttagag agaagcgagt tgttttgatt agtcgtttgg gattggcgga gggaagcggg 1380
ttaggttttg tgaggagtta aggtgagatg ttgagggagg atttaggagg tttttatttt 1440
agatagacga ttttaaataa tttcgtatta tttttgttat tagtcgtggg ttatttgtgg 1500
gcggattttt gagtttgggg cgtttattat tttattgttt ttgaagtcgt agaggatttt 1560
ggagttagag tttagggtgt ttagtgtaag attagtgagg ttaggttttg ttaggtatta 1620
attttaggat cgtaagagag ggttaagcgt attttatttt tatttttatt ttttattacg 1680
tttttttttt tattattatt tgtattttta aatttattcg cgtttttatt ttttattagt 1740
attttttttc gattttttat ttttgttata tcgttatttg ttattttaaa aatcggggtt 1800
tttttattaa ttttattttt tttattttta tttacgttga atcgggtttc gttttcgttt 1860
ttaatttatt ttttaaaaatt cgtttttttt atcgattttt tttttatttt tatttacgtt 1920
gaatcgtgtt ttcgttttcg tttttaattt aagaaagttt taggggttcg gatgtgatgt 1980
tattgatttg cgtattgggg gttagagaga agcgagttgt tgtttgatta gtagtttggg 2040
attggtggaa ggaagtaggt taggttttgt gaggagttaa ggtgagacgt tgaggaggat 2100
ttaggaggtt attattttag atagacgatt ttaaataatt tcgtattatt tttatttgtc 2160
gtgggttatt tgtgggcgga tttttgagtt tggggcgttt attattttat tgtttttgaa 2220
gtcgtagggg attttggagt tagagtttgg ggtgtttagt gtaagattag tgaggttagg 2280
ttttgtcggg tattaatttt aggattttaa gagagggtta agcgtatttt atttttattt 2340
ttatttttta ttacgttttt ttttttatta ttatttgtat ttttaaatta tttacgtttt 2400
tattttttat tagtattttt ttttttttta attttttatt ttttttatat cgttattttt 2460
tattttaata attcgggttt ttgtattaat tttatttttt t 2501

<210> 91
<211> 2501
<212> DNA
<213> Artificial Sequence

<220>
<223> chemically treated genomic DNA (Homo sapiens)

<400> 91

ggaggggtga ggttggtata agggttcggg ttgttggggt gggagatggc ggtatgagag 60
aggtgggggg ttgaagagga gaggagtgtt ggtgggggat gggggcgtgg atggtttggg 120
agtgtaaatg gtaatgggaa aggggacgtg gtgggggatg ggaatagggg tggggtacgt 180
ttagtttttt tttagggttt tgagattgat gttcggtaga gtttggtttt attagtttta 240
tattaaatat tttaagtttt gattttagag ttttttgcga ttttagaggt agtggggtgg 300
tgggcgtttt aagtttagaa gttcgtttat aggtggttta cggtaggtag gagtggtgcg 360
ggattatttg ggtcgtttta tttggggtgg tggtttttttg agtttttttt agcgttttat 420

tttgattttt tatagggttt ggtttgtttt tttttattaa ttttaagttg ttggttagat 480
agtagttcgt ttttttttaa tttttaatgc gtaagttagt ggtattatat tcgggttttt 540
gaggtttttt tgggttgaaa gcggaagcgg aaatacgatt tagcgtggat gggggtgggg 600
gggaggtcgg tggagggggc gggtttttgg gagtgggttg aaagcggaag cggaattcga 660
tttagcgtgg atgggggtgg gaggggtgag gttggtggag gggtttcggt ttttggggtg 720
gtagatggcg gtatgataga ggtggggggt cgaggaggag tgttggtggg ggatgggggc 780
gcggatgggt ttaggagtgt aaatggtaat gggaaagggg acgtgatggg ggatgggaat 840
aggggtgggg tacgtttagt ttttttttac ggttttgaga ttgatgtttg gtagagtttg 900
gttttattag ttttatatta aatattttaa gttttgattt tagagttttt tgcgatttta 960
gaggtagtgg ggtggtgggc gttttagatt tagaagttcg tttataggtg gtttacggtt 1020
ggtagtagga gtggtgcggg attatttggg gtcgtttatt tggggtgggg gttttttgag 1080
ttttttttta gtattttatt ttgatttttt atagggtttg gttcgttttt tttcgttaat 1140
tttaagcggt tggttagagt agttcgtttt tttttaattt ttaatgcgta agttagtggt 1200
attatattcg ggtatttggg atttttttgg gttgaaagcg gaagcggaaa ttcgatttag 1260
cgtggaaggg ggtgggaggg gtgaggtcgg tggagggggc gggtttttgg gggtgggttg 1320
aaagcggaag cgtaatttaa tttagtattt atgggggtgg gaggggtgag gttggtggag 1380
gggttttagt ttttggggtg ggagatggcg gtgtgagaga ggtgcggggt tgaggatagg 1440
agtgttggtg gggggatagg gcgcggatgg gtttgggagt gtaaatagta atgggaaagg 1500
ggaggcggtg gggggataga atagggggtgg ggtacgtttg gttttttttt agggttttga 1560
ggttgatgtt tcgtagagtt tgtttttatt agttttatat taattatttt aagttcggat 1620
tttagagttt tatgcgattt tagacgtagt ggggtggagg gcgttttaga tttagaagtt 1680
cgtttacggg tggtttacgg ttggtagtag gagtggtgcg ggattatttg ggtcgtttta 1740
tttggggtgg gggttttttg agtttttttt tagcgtttta ttttgatttt ttatagggtt 1800
tggttcgttt tttttcgtta attttaagtt gttggttaga tagagtagtt cgtttttttt 1860
taatttttaa tgcgtaagtt agtggtatta tattcgggtt tttggggttt ttttgggttg 1920
aaagaggaag cggaaattag atttagcgtg gatgggggtg ggaggggtga ggtcgatgga 1980
gggggcgggt ttttgggggt gggttgaaag cggaagcgga acgtgattta gtgtggatgg 2040
gggtgggagg ggtgaggttg gtagaagggt tcgggttgtt ggggtgggag atggcggtat 2100
gagagaggtg gggggttgaa gaggagagga gtgttggtgg gggatggggg cgtggatggt 2160
ttgggagtgt aaatggtaat cggaaagggg acgtggtggg ggatgggaat aggggtgggg 2220
tacgtttagt ttttttttag ggtgttgagg tttatgtttg gtagagtttg tttttattag 2280
ttttatatta attaatttaa gttttgattt tagagttttt tgtaatttta gaggtagtgg 2340
ggtggtgggc gttttagatt tagaagttcg tttacgggtg gtttacggtt ggtagtagga 2400
gtggtgcggg attatttggg gttatttatt tggggtgggg gttttttgag ttttttttta 2460
gcgttttatt ttgatttttt atagggtttg gttcgttttt t 2501

<210> 92
<211> 2501
<212> DNA
<213> Artificial Sequence

<220>
<223> chemically treated genomic DNA (Homo sapiens)

<400> 92

ttttacgtta atgttaaaga ttagcgttaa ggattcggag gttttgtcga agattatagg 60
aaagtagcgt ttgtaatttt tgatgacgtt ttttagtatt tttgtttttg tgatcggttt 120
tttgatttaa tatttgagga gtagaaaatg aattaagtta gttatttttt tattgagtgt 180
tgttcggaat atagattttg ggttagggga ggtgtttggt tttttttttt tttggttgtt 240
ggagttttta atggattgtt tttatagagt gaaatcgatg gtagtgggga tggcggaggt 300
tttttgagga tttttgagag gatttggtga tttagtagta ggtatttttt ttagggtgtt 360
tgggattaga ggagaggagg aggatatagt tttttgtttt ttagtagtgg tagtttgtat 420
atttattagg tttagggttt ttttttgggt gttaaggttt tttttaggtt tgtagtgttg 480
atttttttgt ttaagagata tgacgatttt ggttatcgta ataggtagga gtgtgggtag 540
gagttgggta atggagattt attggtttgg agagagaggg agtatgtgag aagttttagt 600
taaaaattga attttggagg ttttaataaa ggtttattta tagatttttt tttttggtgt 660
ttttttaggg ttttttgggg atttttggga gttttttggt gattttggtt agtttgtttt 720
ttgagaattt gaaggaaaaa gtgagagggt attttagggt atataagtaa gtagaggttg 780
attttgtagg attgatagta gagagggtga ggttaggtgt tttggagttt tatatgtttt 840
ggggcgatgg gggttattag tgtgtattta aggtaaacgt ttattgttgg tattagacgt 900
ttgtattttt tttgttttgt gatttgaggg tacggtttta gattaaggtt ttagtgtttg 960
ttttttggag tttttggaag aggaatgtat gggtttttag ggtgtagatt gtaagtatag 1020
ttttaggttt ttagtgttaa taggagggta ggttggaatt tgtgaggttt ttattttttt 1080

ggggtgaatg gttttttttg tgtttatata gggtttttat tttttttttg gtaaggtttg 1140
gatttatatt ttttgttggt ttgagaaatt tagattaaga gtttatattt ttgataggga 1200
ataggatatg aggagattta tatttggtta tacgtgttta ggtttttttg ggaaggatag 1260
g[?]agagatgt tgaaatgtat tggaatttat gtgttttggg tgaggaattt gtttcgtttt 1320
tattttgttt tttggtaggg tttaagacgt ttttttattt gatttgagtg tagatttttc 1380
gataaagatt tttttatttt tgatattaat gattttaggg taagtcgggg ggattgtagt 1440
agatagtttt gtttatgttt tttggagtga tagtaggggt agggtagatt ttcgtgaggt 1500
ttttatttgt gttttggttt aagggtgttt ttaatcgttt tttatgtttt ttatcggaat 1560
tattgataga ttttgggatt attttgttta tttattagat gggggttttt gtgttgattt 1620
gagttatttt cggagaataa gttttttatt ttttcgagat ttgaaaatag gaatgagatg 1680
gagttatatt ttgttatttt tatatggggt gtttaggtat gatttttttg gttttggggt 1740
gagtgttttt atggtttttt gtgggttatt ttttatcgtg atggggtttg ttatttttta 1800
atttgaaatt ttttgagatg agtagatagt ttttttttat tgtaagaatt tattattttg 1860
agggtttttt aatttttttt ttgtggtata aatgaggttg gtatataggg ttatttttga 1920
tttggttttt tttgagtaaa aattagggga tagttagatt ttgtggggtt tttttttttt 1980
gagttaaggt atttttaatt tttatgaatg gggtatatat tggggtttgt agatgttggg 2040
attttttttt ttgttgattt gagttattat tgttttgatt atagtttttt tttttttgtt 2100
atttagaagg tgtagtaagt atgggttata ggtggttatt gtgtattgga ttttttaggg 2160
taaaggtttt tattgttttg gatttaaagg tattttgagt tttttttttt tttttttttt 2220
ttgattttgg tagggttggg ttttatttta tgttgacgtg agtttttatt tttttgattt 2280
tcgaggatga aagaggaggc gttttagttt tagatagggt tggagtggat tttttatttt 2340
gaggatttgg gttttttgag gttttttttt atttttagta gggtttgagg agtagagatt 2400
tttttgaggt tttgtttatg atattaagtt tttttttttt tttagttttg gatgtggacg 2460
ttagaatggg tgtgtttgtt tgttattttt ggggtttttg t 2501

<210> 93
<211> 2501
<212> DNA
<213> Artificial Sequence

<220>
<223> chemically treated genomic DNA (Homo sapiens)

<400> 93

atagaagttt taggaatgat agataggtat atttattttg acgtttatat ttagggttga 60
aggagggaaa gggtttagta ttatgagtag ggttttaggg gagtttttgt tttttaagtt 120
ttgttgggag taaagggagg ttttagggaa tttaggtttt taggataggg ggtttatttt 180
aattttgttt gagattgagg cgtttttttt tttattttcg ggaattagag ggatggagat 240
ttacgttagt atagggtggg gtttaatttt gttaggatta aggagaggaa gaagagggag 300
gatttagggt atttttgagt ttagaataat ggggattttt gttttgggag gtttagtgta 360
tagtggttat ttgtagttta tgtttgttgt attttttggg tgataaagag gagagggttg 420
tggttagagt agtggtgatt taggttagta gagggaggag ttttagtatt tgtaggtttt 480
aatgtgtgtt ttatttatga agattgggga tattttggtt tagaaagaag ggattttata 540

gagtttggtt gttttttgat ttttgtttag aggggattaa attaaggata gttttatgtg 600
ttaattttat ttgtgttata ggaaagaagt tgaagagttt ttagggtgat gggtttttgt 660
agtaaagggg agttatttgt ttattttagg gggttttagg ttgaggaatg gtaggtttta 720
ttacgatgaa gagtaattta taggaggtta tagaaatatt tattttagaa ttaaaggggt 780
tatatttgga tattttatgt gggggtgata ggatgtagtt ttattttatt tttgttttta 840
gatttcgggg aggtgaggaa tttgtttttc gaggatgatt taggttaata taggggtttt 900
tatttggtgg atagatagag tggttttagg atttgttagt agtttcggtg aggaatatga 960
gggacgattg agggtatttt tgggttagaa tatagatgag gattttacgg aaatttgttt 1020
tgtttttgtt gttattttag agagtatggg tagggttgtt tgttgtagtt ttttcgattt 1080
attttgggat tattggtgtt agggatgggg aggtttttgt cgaggggttt gtatttaggt 1140
tagtagaggg agcgttttag gttttgttag gagataaggt aagaacgaag taggtttttt 1200
atttaggata tatgaatttt aatgtatttt agtatttttt tttgtttttt ttaagaggat 1260
ttgggtacgt gtggttagat gtgagttttt ttatgttttg ttttttatta gggatgtgag 1320
tttttaattt gagtttttta ggttagtaaa agggtgggat ttaggttttg ttaggagaaa 1380
ggtgagggtt ttgtgtgagt atagagggga ttatttattt taagagggtg gagattttat 1440
agattttagt ttattttttt gttagtattg ggggtttgag gttgtgtttg tagtttgtat 1500
tttgagggtt tatgtatttt ttttttagga gttttaggaa atagatattg aggttttggt 1560
ttgaggtcgt gtttttaggt tatagagtag aggagatgta gacgtttagt gttagtagtg 1620
aacgtttgtt ttgaatgtat attaatggtt tttatcgttt tagaatatat gggattttag 1680

agtatttggt tttatttttt ttattgttag ttttgtagaa ttagtttttg tttgtttgtg 1740
tattttgagg tgttttttta tttttttttt taggttttta ggggataggt tgattaggat 1800
tattaggaag ttttagagga tttttaggag gttttagagg agtattaaag gagaagattt 1860
gtaagtaagt ttttgttaga gtttttaagg tttagttttt agttgaggtt ttttatatgt 1920
tttttttttt tttaggttag tgggttttta ttgtttagtt tttgtttata tttttgtttg 1980
ttgcggtgat tagagtcgtt atgttttttg agtagaagag ttagtattgt aagtttgagg 2040
aaggttttga tatttaagaa gaggttttgg gtttggtggg tgtgtaggtt gttattattg 2100
aggagtagga ggttgtgttt tttttttttt ttttggtttt aggtattttg ggggaggtgt 2160
ttgttgttgg gttattaggt ttttttaaga gtttttaggg agttttcgtt atttttattg 2220
ttatcgattt tattttatgg aggtaattta ttaagggttt tagtaattaa gaagaggagg 2280
ggttaagtat tttttttgat ttagagtttg tgtttcgagt agtatttagt aagaaggtgg 2340
ttgatttgat ttattttttg ttttttaagt attaagttaa ggagtcggtt ataaaggtag 2400
aaatgttgga gagcgttatt aaaaattata agcgttgttt ttttgtgatt ttcggtaaag 2460
ttttcgagtt tttggcgttg gtttttggta ttgacgtgaa g 2501

<210> 94
<211> 2501
<212> DNA
<213> Artificial Sequence

<220>
<223> chemically treated genomic DNA (Homo sapiens)

<400> 94

aaaaagtata tattaagtaa gtttttttat atagtagtaa atattttacg taaattatta 60
gtatattaaa taaataggtt ttgtttaagg aaataatata cggtttaata ttaggggaaa 120
aaaaaaaaac ggttttattg taatttttta tatataaaat ggaggagaaa aaaatttgat 180
tgcgttaacg ggtatagaaa aagtttttta aagaaattta atatgagttt atggtaaaaa 240
aaaaaaaaaa gtatttaatt tatatttaaa gagaaattgt attttttaaa attaatagta 300
aatatattta aatgtaaaac gttcgaataa ttattattta cgttaggaac gagaagtaga 360
tgtaggttat tatttttacg attgaaatta ggattgagat ataaagagtt ataagtatta 420
aaatgagtaa gtggtatgaa tattggaaga atatgtaatg tttagtgata atattattat 480
cgattttgaa aatttaaagg aaaaggtatg tggaatattt aagtgtatcg gtttttagaa 540
cgaggtgtgt aatagattat tatagtaaaa ttaaaatttt tattttacga aagtaataat 600
taattataat aattaataga taatatgtta tttgtaatag taataaaatt tatgaattgt 660
ttgaaaagaa gtttaataaa aatgtataag gtttttatag agataattgt cgaagtatat 720
tgatatattt aggtaaaaaa aattttgaaa ttatatagag gagtataata tatttattta 780
tggggagtat aatattgtaa aaatgtaaat ttagtttaaa ttatttataa tttagtgttt 840
tatttgtagt cgtcgttatt ataataaatt tgggttgttg tgttttgttt tttaaattaa 900
atttggaaaa ttatagaatg gaaatagtaa ataaaaaatt agtattagta ataaaattta 960
cgtaataaaa tagggaaagt ttagggtgag atttaaggtt gtgttgagtt ggtatttgtg 1020
tttgatattt gtagtgttgg ttggaggggg ttagtagtag ggatgttggg gaggtttgta 1080
gtcggtttat acggtagatg atagaatggg tagaataaaa gtttgaaatt ttttatttta 1140
tttttttgta taatttgagg tagtttttga aaatacgatg ttaagagttt taggtaatag 1200
taggatatta ggaaaatttg gttggttaag gtagtatgat tttagagttt tgttaataat 1260
aatttgaaat tattatagtg gtagaggaat tatatttttt aaaaaaaaaa atttttttaa 1320
tagaatataa agattgaaat aggaggttat tataatcgcg ggagttgtcg tttcgttttg 1380
tagggagtat ttggtttggg attcgtaggt attttttata aggggtgtag ttgtgtaaat 1440
gtttataggt gatagaaata gagtattttt tgtttattat tttatttaat agttagaggt 1500
gacgaagacg atttttttga gtgaggattg agggtttata tcgttttttt attttatata 1560
ttatagaggg attatagaat ttagtttagt tttttttgtt agttttggta aacgtaggta 1620
gtgatgttat ttagattata tttttttttt taatgttatt ttagggggat ttagagttag 1680
agatttggtt tgaggggagt agaagtaatt tgtagaggat ggcggtttag gtttagttag 1740
gtattaattt taggattttg agggatgatc gaaggtttcg tttatttatt tttaattttt 1800

tcgattttat taggatttat agttttagga ttttcgtttt aatttttatt ttttgtttta 1860
ttattatttt tatgtttatt tttattttta ttcgattttt atttaggtag aatttagttt 1920
tatttttgtt cggaatttag ggtagtatcg ttgttaggat gtgacgttat tgatttgcgt 1980
attggaggtt agaagatcgc gagattttcg ttttgagtaa cgagcgacgg tttgacgtcg 2040
gcggagggaa gtcggtttag gttcggtgag gaggtaaggt aagacgttga gggaggattg 2100
aggcgggttt tattttagat agagggtttt aaataattta gtgttgtttt tgttgtcggg 2160
tttgggttat ttcgtagggg aagattttta ggttgggtcg ttattatttt atttcgtcga 2220
ttttcgtcgt tttagttacg gggaattttg gggatagagt ttaatgtggt tagggtaggg 2280

ttggttagaa gaggttaggg tttacgttgt ggtaggaatt aaggttagga tttcgagagg 2340
gaattgaggg tagtttaatt attattttta ttattatttt cgttttttaa tattaatttt 2400
atttttattt tttatttttt atttttattt ttatttttat ttttattttg gtagaattcg 2460
ggttttgttt ttggtattaa gttacggaag tttcgggaat g 2501

<210> 95
<211> 2501
<212> DNA
<213> Artificial Sequence

<220>
<223> chemically treated genomic DNA (Homo sapiens)

<400> 95
   tattttcgga gttttcgtga tttgatatta ggggtaaagt tcggattttg ttaggatagg 60
   ggtgggggtg gggatgggaa tggggaatgg gggatggggg tggggttggt gttgggggac 120
   gggaatggtg gtgagggtgg tggttaggtt gtttttagtt ttttttcggg gttttgattt 180
   tgatttttgt tatagcgtgg gttttgattt tttttaatta gttttgtttt ggttatatta 240
   agttttgttt ttagagtttt tcgtggttaa agcggcgggg gtcggcgggg tgaggtagtg 300
   gcgatttagt ttggaagttt ttttttgcgg ggtggtttag gttcggtagt agaggtagta 360
   ttggattatt tgaggttttt tgtttgaggt gaggttcgtt ttagtttttt tttagcgttt 420
   tattttgttt ttttatcgag tttgggtcgg ttttttttcg tcgacgttag gtcgtcgttc 480
   gttgtttagg gcgagaattt cgcggttttt tgatttttaa tgcgtaagtt agtggcgtta 540
   tattttggta acggtattat tttgggtttc gggtaggggt ggaattggat tttgtttgga 600
   tggggatcgg atgggggtgg aggtaagtat gaagatggtg atggggtaag gggtaaggat 660
   tgggacgggg gttttgaggt tgtagatttt ggtggggtcg ggggagttgg gggtgggtgg 720
   gcggggtttt cggttatttt ttagggtttt gaagttgatg tttggttgag tttggatcgt 780
   tattttttgt agattgtttt tgtttttttt agattaagtt tttgattttg agtttttttg 840
   aagtggtatt gggggaaggg gtgtggtttg ggtgatatta ttgtttgcgt ttattagggt 900
   tgataagagg ggttgagttg gattttgtgg tttttttatg gtgtgtgggg tgggggggcg 960
   gtgtggattt ttagttttta tttaggaggg tcgttttcgt tatttttggt tgttggatga 1020
   agtgatgggt aggagatgtt ttgtttttgt tatttgtgag tatttgtata gttgtatttt 1080
   ttgtagagaa tgtttgcggg ttttaggtta ggtgtttttt gtagggcggg gcggtagttt 1140
   tcgcggttgt agtgattttt tattttagtt tttgtgtttt gttgaaagaa tttttttttt 1200
   taaaaaatgt aatttttttg ttattatggt ggttttaggt tgttattagt agaattttgg 1260
   agttatattg ttttaattaa ttaaattttt ttggtgtttt gttattattt agggtttttg 1320
   gtatcgtgtt tttagaggtt gttttagatt gtgtaaagaa gtgaagtgga aaattttaaa 1380
   tttttatttt atttattttg ttatttatcg tgtaggtcgg ttataaattt ttttaatatt 1440
   tttgttgtta atttttttta attaatattg taagtgttaa atataagtat tagtttaata 1500
   tagttttgag ttttattttg ggtttttttt gttttgttgc gtagattttg ttgttgatat 1560
   tggtttttta tttattgttt ttattttgta attttttagg tttgatttgg gaagtagagt 1620
   atagtagttt aaatttgttg taatggcggc ggttataggt aaggtattaa attgtaaata 1680
   atttaagttg agtttatatt tttatagtgt tgtgtttttt atgaatgaat atattatatt 1740
   tttttatgta attttaaggt tttttttatt tgaatatgtt agtatatttc gatagttgtt 1800
   tttataaaga ttttatatat ttttattaga ttttttttta ggtaatttat gaattttgtt 1860
   gttattgtaa atggtatatt gtttattaat tgttgtagtt agttattgtt ttcgtgaggt 1920
   gaagattttg attttgttgt agtaatttgt tgtatatttc gttttgaagg tcgatgtatt 1980
   tgaatatttt atatattttt ttttttgaat ttttaagatc gataatggta ttattattaa 2040
   atattatata tttttttagt atttatatta tttatttatt ttggtattta tagttttttg 2100
   tgttttaatt ttgattttaa tcgtagagat aatagtttat atttattttt cgtttttgac 2160
   gtgaatgata attattcgaa cgttttgtat ttaagtatgt ttgttgttga ttttaggaag 2220
   tgtaattttt ttttgaatat aggttaaata tttttttttt tttttattta tgaatttata 2280
   ttaaattttt ttaaaaagtt ttttttgtat tcgttgacgt aattaaattt tttttttttt 2340
   tattttgtat gtaaggaatt ataatgaaat cgtttttttt ttttttttta atgttaaatc 2400
   gtatgttatt tttttgggta gagtttgttt atttaatatg ttagtaattt acgtaggata 2460
   tttgttgtta tgtaagggag tttatttaat gtgtgttttt t 2501

<210> 96
<211> 2501
<212> DNA
<213> Artificial Sequence

<220>

<223> chemically treated genomic DNA (Homo sapiens)

<400> 96

tttaaattaa tatttttagt aaatgaagat taaaaggtat tttaattttt taaaattgat 60
aggaaatata tttgaatgtg aaatgttaga agtattttta ttttcgttag gagtaagtta 120
ttgatgtatg tttttattgt tatcgtttat agttgtagtg aatgttgaaa tgaaaaagag 180
agttatggat attaagatga aaaagaggta tgaagattga aaagaaatag attatatttt 240
ttataatatg attattaaga aaatttaaag gaagagatat atggaatatt taaatgaata 300
agagatttta gaagatagta tataaagatt attgtagcga atataagaga tttgttatag 360
atgagtaatt tagaatataa aatgaaaaat atattatttt taatagtatt taaatttaaa 420
atataaagaa gaagaagttt aattaaaata tgtaagatat ttatagaagt aattgtataa 480
gtttgttgat agatttagag aaaaaatttt taaataaatg gagtagtata tgatgtttat 540
tgatgaggaa tagaatatag taaaggtgta aattttattt aaattaaata aatagttaat 600
gtttatttgt agttgttaaa gtgataatta agtttaggtt gttgtgtttt atttttaaaa 660
ttaattgtgt aaaaatatag aagggaaaaa gaaaaataaa ataataatat tagtaataaa 720
gtttaaataa taaaatatag aaaattttgg ggaaaaaatt gtgttgaatt ggtatttgtg 780
ttttgggtag ttggagaggt agggaggttg aggaggtttg tagttattgt agacgatagg 840
gtgagttgta ggaaaagttt taaaggatta ttttatatta atgtaggatt cgagtttgta 900
ttttaaaata cgaagttagt agtttagggg taatattagg atattaggaa aattggattg 960
attgagatat tatggtttta gaattttgtt aataataatt agaaataaag agtaggttag 1020
gattattttt taaaatatta ttattttaat taaatatata taatgaatta gaaatagaat 1080
agtatgagtg aattatatat tgtatgagta gatattgttt gataaaataa gtacgtatag 1140
tcgatatata tatatgtata aatatatgta aagatatata tatatatatt tatataatta 1200
tatatatata atataaatat atatatatat atgtgtttat gtatataaat ataaatataa 1260
tgtatatata taattggtat agatttaaaa ttcgtttttt attatacgaa tgcgaattaa 1320
attatgaaaa ttattaaagt ggaataggaa ttgttaggta attttaaaat ttaggatttt 1380
tataaattta ttgtagttat ttagtttttt tagtgtaaaa ataaattatt tagtataaaa 1440
gttttgtaaa attttttttt agttggggaa gagttttcgt gggaaggtgt gttttttttt 1500
ttagaggtta ttataatttt aggtgttgta aatttatagg gagaatttgg tttgggattc 1560
gtagttattt tttgtaaggg gtgtagttgt gtaaatgttt agaggtgata gaaatagagt 1620
attttttaaa agagttaaga gttaagaaga ggattttttt gagtgaggat tgaggtttta 1680
tttatattat atagaggggt tatataattt agtttagatt tttatgttag ttttgaaaga 1740
ttttagtagt tttgtttttt tattatattt ttttttttat tgttatttta ggggattcgg 1800
aattagaatt ttggtttgag ggaagtagat attattagta gagtacggag ttttaggttt 1860
tgttagattt taaagtgaga attttgaggg atgattgaat cgtttttttt ttatttttcg 1920
ttttattaga attcggtttc gtttttgttg tttatttagg tgagttttgg gtggacggat 1980
gtgacgttat tgacgtggtc gcggggatta gagagaagcg agggtttggt tttgaggggt 2040
cggtttgaga tcggttgagg gaagcgggtt tagggtttgt gaggaggtaa ggtgagattt 2100
tgagggagga ttgaggaggt ttttattttt tatataggat ttaaaataat ttagcgtcgt 2160
ttttgttgtt agttttggat tattaggggg tagatttgtt tggttgggtt atttttcgtt 2220
agttttgcgt ttaagtttta ggggaatttg gagtgagagt ttggtgtgat tagagtaggg 2280
ttggttagta gagggtaggg tttaggtttt gttaggtatt aagggtagga ttttgaggga 2340
gggttgaggt tttatagagt atatttttgt ttttattgtt agttttggga agttttaggt 2400
atggcggtcg ggtatggcgg tcgggtatgg cggtcgggta tggcggtcgg gttcgttgat 2460
tttgacgttc gtattcgggg ttgatgtagt aaaggggttt t 2501

<210> 97
<211> 2501
<212> DNA
<213> Artificial Sequence

<220>
<223> chemically treated genomic DNA (Homo sapiens)

<400> 97

gaagtttttt tgttgtatta gtttcggatg cggacgttag aattagcgag ttcggtcgtt 60
atgttcggtc gttatgttcg gtcgttatgt tcggtcgtta tgtttagggt tttttagggt 120
tgatagtagg ggtagagatg tgttttgtgg agttttagtt tttttttagg gttttgtttt 180
tgatgtttgg tagaatttgg gttttgtttt ttgttaatta gttttgtttt ggttatatta 240
agtttttatt tttagatttt ttgggtttta agcgtagggt tggcgagggg tggtttagtt 300
agataagttt gttttttggt ggtttagggt tggtagtagg ggcggcgtta gattattttg 360
ggttttatat gaggggtggg ggttttttta gttttttttt agggttttat tttgtttttt 420

tatagatttt gagttcgttt tttttagtcg attttaagtc gattttttag aattaggttt 480
tcgttttttt ttgattttcg cggttacgtt agtggcgtta tattcgttta tttagggttt 540
atttgggtgg ataatagggg cggaatcgga ttttagtggg gcgggggatg agggaggagc 600
ggtttagtta ttttttaagg tttttatttt gagatttggt agagtttgga gtttcgtatt 660
ttgttgatgg tgtttgtttt ttttagatta aggttttgat ttcgagtttt ttgaggtggt 720
agtgggggga agggtgtggt ggggggataa ggttgttggg gttttttagg gttgatatga 780
gggtttgagt tgaattatgt ggttttttta tgtggtgtgg gtggattttt agtttttatt 840
taggagggtt ttttttttga tttttggttt ttttaggaga tattttgttt ttgttatttt 900
tgagtatttg tatagttgta ttttttgtag agaatggttg cgggttttag gttagatttt 960
ttttgtgggt ttgtagtatt taagattgta gtgatttttg ggagaagagg tatatttttt 1020
tacgggggtt tttttttagt tagaagagga ttttgtaaaa tttttgtatt aaatgattta 1080
tttttatatt gaagaaattg agtagttgta ataaatttgt ggaagttttg agttttgaag 1140
ttatttaata atttttattt tattttagtg atttttataa tttaattcgt attcgtatag 1200
taagaaacga attttaaatt tatgttaatt gtatatgtgt attatattta tgtttatatg 1260
tataaatata tatgtatgta tatgtttata ttatatatgt gtaattatat gaatgtatgt 1320
gtatatattt ttatatatat ttatatatat atgtatgtcg attatacgta tttattttat 1380
taaataatat ttatttatat aatgtgtgat ttatttatat tattttattt ttagtttatt 1440
atatatattt gattaaaata atagtatttt ggaaggtagt tttaatttat tttttgtttt 1500
tagttgttat tagtagaatt ttggagttat ggtgttttaa ttaatttaat ttttttggtg 1560
ttttgatatt atttttgggt tattggtttc gtgttttgag gtgtagattc ggattttata 1620
ttgatgtggg gtggtttttt aaaatttttt ttgtaattta ttttgtcgtt tgtagtggtt 1680
gtagattttt ttaatttttt tgttttttta attgtttaga atataagtat tagtttaata 1740
tagttttttt tttagggttt tttatgtttt gttgtttaga ttttgttgtt gatattgttg 1800
ttttattttt tttttttttt tttatatttt tatatagttg attttgggag tagagtatag 1860
tagtttgggt ttgattgtta ttttggtagt tataggtagg tattaattgt ttatttaatt 1920
taagtaagat ttatattttt gttgtatttt attttttatt agtgaatatt atatattatt 1980
ttatttattt gggaattttt tttttgaatt tgttagtaaa tttgtatagt tgtttttgta 2040
aatgttttgt atattttggt taggtttttt tttttttata ttttggattt aaatgttgtt 2100
ggggatggta tattttttat tttatgtttt aagttgttta tttgtgataa gttttttgta 2160
ttcgttgtag tggtttttgt atgttgtttt ttgaagtttt ttatttattt gaatgtttta 2220
tgtgtttttt tttttgaatt tttttgatgg ttatattgtg aaaaatatga tttatttttt 2280
ttttaatttt atattttttt tttattttga tatttatagt tttttttttt attttagtat 2340
ttattgtagt tgtgaacggt agtagtgaaa gtatatatta atgatttgtt tttgacgaga 2400
atgggaatgt ttttaatatt ttatatttaa gtatgttttt tgttagtttt agaaaattaa 2460
agtgtttttt aatttttatt tgttaaaaat gttaatttga a 2501

<210> 98
<211> 2501
<212> DNA
<213> Artificial Sequence

<220>
<223> chemically treated genomic DNA (Homo sapiens)

<400> 98

gcggaattag attttgtcgc gggagagttg ggggggtggg gtggagtttg gggtggggtg 60
gggtggggtg gggtggggtg tagagtggag ttgaggatgg ggttggggaa tttggggttg 120
taggttatgg ttgggttggg ggggtttagt tttttttagg gttttggttt tgatgttcgg 180
tagagtttgg gttttgtttt tttttaatta gttttgtttt ggttatatta agtttttatt 240
ttagagtgtt tagtagtttg agcggcggag ggagggggtc ggtttagttt tataagttag 300
ttttagatcg gttttcgggt ggtttagggt tgggagtaga gacggtattt ggttttttcg 360
ggttttttat ttggggtggg gatattttta gttttttttt agtattttat tttgattttt 420
tatagagttt cggttagttt atttttatcg gtattaagtc gttttttaga aggaggtttt 480
tttttttttt ttgattttta gtgcgtacgt tagtgacgtt atattcggtt tttaagtttg 540
gagttttttt gggttgttag taggggttga atcgggtttt ggcggagtag gggtggggat 600
ggggataggg gtttttagtt attttttagg gggttttgat tttgatgttt gggtttcgat 660
ttttattttt tgtttattgg gtttgttttt ttcggattaa gtttttgatt tttagttata 720
tgaggtggta gtgaggggag gggtgtggtc ggggtgataa tattgttagg gttttttagg 780
gttgatagga agggttgagt tggattttgt ggttttttta tgtggggagg ttggattttt 840
agtttttttt taggagggtt ttttttttgg tttttggttt tttgatgaag tgatgtgtgg 900
gagatgattt gtttatgtta tttttgagta tttgtttagt tgtacgcgtt gtagagaatg 960
gttgcgggtt ttaggttaga tgttttttgg ggggttgtag tatttgtgat tgtaggattt 1020
gtgagagaag atgtatatta ttttatgggg gttttttttt agttagagtt agattttgta 1080

aattttttgt tttaaaagat ttatttttat acggaagagg ttgagtagta gtaagagatt 1140
tggagaagtt atgtgttttg aaggtatcga atatagtaga gtttagatta gcgattttta 1200
tattttaatt tttatttata tggtaataaa ttttatattg tatgtgtata ttatatatgg 1260
tattgtatat tttgtttata tttacgttta tatatatatt tgttttagat attatatata 1320
tttatgttta tatgtatata tatacgtata tatattttta tattttatat ttatttatat 1380
gtatatattt ttagttatat attcgtttgt atttttagat tttatattat atatatgtat 1440
atatatttta ttaatattta tttatagtat gtggttttta tatatattta ttattttatt 1500
tttagtttat tttttatatt ttattaaaat aataattttt tggaaggttg gttttttttt 1560
attaagtttg ttttaggtgg ttgttagtag aattttggag ttatagtgtt ttaattaatt 1620
taaatttttt ggtgttttga tattatttta gggttattgg tattagattt tgtggtgttg 1680
ttttagattg cgcgatgatg tggggtggaa tgttaaatat ttttttttat ttattttgta 1740
atttgttatt tatgtagggt atagattttt tcgatttttt tgttgttttt tggatttaaa 1800
atataagtat tagtttagta tagtttttag ttatttttag ggtttttttt gttttgatgg 1860
gttttttttt ttaatttttt tttttttata tttttttcgg ggttgatttg gggagtagag 1920
tatagtaatt tgagtttggt tgttattttg gtaatgatag ttaaggtatt tattttgtat 1980
ttaatttaag tagtatttat atttttgtta tattttgttt tttattaagg aatatggtat 2040
atagatttat ttatttgaga ttattttttg agtttattag taattttgta tagttgtttt 2100
tataaaggtt ttgttaggtt ttttttattt atattttgga ttttaatgtt gttgtgaatg 2160
gtgtattttt tattttttat tttatatatt aagttgtttt tgtgtggtaa ggtttttgat 2220
tttgttttaa tgattttttg tatgatgttt tttgaagttt tttatttatt tgaatattta 2280
atgtgtttat ttttttgaat tttttatgta gataattata tagttaataa atattttatt 2340
tttttgtagt ttttatattt tatttttatt ttgatattta tagttttggt ttttttttat 2400
tttgatattt attgtagatg taggtggtag tggtgatagt atatgttaac gatttgttat 2460
tgtcgtgaat ggtagagttt ttgatatttt atatttatgt a 2501

<210> 99
<211> 2501

<212> DNA
<213> Artificial Sequence

<220>
<223> chemically treated genomic DNA (Homo sapiens)

<400> 99

tatataaatg tgaaatgtta gaagttttgt tatttacggt agtgataagt cgttgatata 60
tgttgttatt attattattt atatttgtag tgaatattaa aatgaaaaag agttagagtt 120
atggatgtta agatgaaaat gagatatgaa gattgtaaag aaatagagta tttgttaatt 180
atatgattat ttatatagaa aatttaaagg aatagatata ttgaatattt aaatgaataa 240
aagattttag aaaatattat atagaaggtt attaaagtaa aattaagagt tttgttatat 300
aaaagtaatt tagtatataa aatagaaaat agaaaatgta ttatttatag tagtattaaa 360
atttaaaatg taaataagaa gagtttgata agatttttat ggaagtaatt gtgtaaagtt 420
gttgatagat ttaggaaata attttaagta aatggatttg tatattatat tttttgatgg 480
ggaatagaat atggtaaaga tgtaaatgtt atttaaatta aatatagagt gagtgtttta 540
attgttattg ttaaagtgat aattaagttt aggttgttgt gttttgtttt ttaaattaat 600
ttcggaaaaa atataggagg gaaaagatta aaaaaaaaaa tttattaaaa tagagaaagt 660
tttggggatg attgaagatt gtgttaaatt agtatttgtg ttttgggttt agggagtagt 720
agggaaatcg aggaggtttg tattttgtat agatggtaga ttataggatg gataggagaa 780
agtgtttaat attttatttt atattatcgc gtaatttgag gtagtattat aaaatttgat 840
gttagtagtt ttggagtaat attaggatat taggaaattt gagttgattg agatattatg 900
gttttagagt tttgttaata attatttgaa ataaatttag taggagaaga ttagtttttt 960
aaaaaattat tattttaatg gaatataaag aatgaattag gaatagaata gtaagtatgt 1020
gtgaaggtta tatattatga gtagatatta ataaaatatg tgtgtatata tatagtataa 1080
agtttagaaa tataaacgaa tatgtagtta aagatatata tatgtaagta aatataagat 1140
ataaagatat atatgcgtgt atgtatatat ataagtatag atgtatataa tgtttaaaat 1200
aggtatgtat ataaacgtag atataaataa aatgtataat attatatata atgtatatat 1260
atagtgtaaa atttattatt atgtgaatga gaattaaaat atgaaaatcg ttaatttgga 1320
ttttgttgtg ttcgatattt ttaaaatata tggttttttt aaattttttg ttgttgttta 1380
gtttttttcg tgtaaaaata aattttttag gataaaaggt ttgtaaagtt tagttttggt 1440
tggggaggag tttttatggg atggtgtgta ttttttttta taggttttat aattataggt 1500
gttgtagttt tttagggagt atttagtttg ggattcgtag ttattttttg taacgcgtgt 1560
agttgggtaa atgtttaaag gtgatataaa tagattattt tttatatatt attttattaa 1620
agagttagga gttaggagga gaattttttt gagaggggat tgaaggttta atttttttat 1680

atagaggggt tatagaattt agtttagttt tttttgttag ttttggaaga ttttggtaat 1740
gttgttattt cgattatatt ttttttttta ttgttatttt atgtgattgg gagttagaga 1800
tttggttcga gaggagtaga tttaatgggt agagaatggg gatcgagatt taggtattaa 1860
ggttaggatt ttttgaggga tgattgaggg tttttatttt tatttttatt tttatttcgt 1920
tagaattcgg tttagttttt attggtaatt tagggaagtt ttaggtttgg gggtcggatg 1980
tgacgttatt gacgtgcgta ttgggggtta gagagaaggg agaggttttt ttttgagggg 2040
cggtttgata tcggtggagg taagttggtc gaggttttgt gaggagttaa ggtgaggtgt 2100
tgagggaaga ttgaggatgt ttttatttta gatagaggat tcgaaagaat taagtgtcgt 2160
ttttgttttt agttttggat tattcggggg tcgatttggg gttgatttgt gaggttgggt 2220
cgattttttt ttttcgtcgt ttaagttgtt gggtattttg gagtgagagt ttggtgtgat 2280
tagggtaggg ttggttagga gagggtaggg tttaggtttt gtcgggtatt aaggttagga 2340
ttttgaggga ggttgggttt ttttaattta attatgattt atagttttag gttttttagt 2400
tttattttta gttttatttt atattttatt ttattttatt ttattttatt ttaaatttta 2460
ttttattttt ttaatttttt cgcggtagaa tttggtttcg t 2501

<210> 100
<211> 2501
<212> DNA
<213> Artificial Sequence

<220>
<223> chemically treated genomic DNA (Homo sapiens)

<400> 100

ttttttttat ttgattttag ggaggtgtag gttttttaaa tggagaggtt tttaggttaa 60
aaaagagggg agttttagga tttgttagga gttaaattaa ggaaatcgag tgagaattga 120
ggggattgtt tatttttgaa tagagagagt aatagagttt atttttttgt ataattttta 180
aattgttagg gtaatatgta aggttgagga tttattatta tttttttttt ttaataattg 240
attttttttt ttagaatagt tttacgttta tagtaaaatt gagtagaaag gagagtttta 300
taggtatttt gtttttttat agatatagtt ttttttatat taatttttta agttagggtg 360
atatatttgt tataattgag aatatatgga tatattatta ttatttaatg ttcgtagttt 420
aggatttatt tttgttgttg tatattttgt ggttttaata tttaaaaatg tataatgata 480
tatgtttatt attatagaat tatttagaat tgttttattg ttttaaaagt tttttgtgtt 540
tgatttattt attttttatt ttttttaatt tttggaaatt attgaatttt gtattgtttt 600
tatatttttg ttttttttag aatgttatat agatggaatt atgtaatgta tagatttttt 660
aaattggttt tttttatttt gtaatatgta tttaagtttt atttatgttt ttttgtggtt 720
tgatagttta ttttttttta gtgttgaata atattttatt ttgttaattt atttattttt 780
tttttgaagg atatattgtt tgttttttag tttgggtaat tatgaataaa gttattataa 840
atatttgtgt gtaggttttt tttgtggata tatcgttttt aatttatttg gttaaataat 900
aaggagcgta tttgttagat tatatgatga aagtatgtat agttttgtaa gaaatttata 960
aattgttttt aaaagtggtt gtattatttt ttatgtttat tagtaatgat agtgtgtttt 1020
tgttgtttta tatttttatt agtatttggg gttattaggg tttttgattt tgattatttt 1080
tataggtatg tagtggtatt atattattgt tttaatttgt aagtttttaa tgatatgtga 1140
tgttgattat tattttatgt acgtatttgt tttttatata tattatttgt tgaggtgttt 1200
gtttagattt tttgtttatt ttttaatagg ttggttagtt tttattgttt agttttaaga 1260
tatttttgta tgttttggtt aatagttttt tattagatat gttttttgaa aataattttt 1320
ttttaatttg ggagttattt tatttttttt gtggtatttt tagtagagta gaagttttaa 1380
tttagcgaag tttagattat taattatttt tttttataga tgtttattat attttatatt 1440
agaattagaa aagatgaaat ttttggtttg aagttgtagt tattagttag tagaagagat 1500
agtttataat tttgtttgga gtttagatga ggattttgag tgtaaatttg ggatttaaag 1560
agtttaggat agagagagta ttaaatgttt ttaggtaggg gtggtgggtt gaggggtttt 1620
tagatttttt ttatttgggt tttagaaaat taaagagtta attttataat attaatagag 1680
ggaggtttag gttttgttaa gagttgatat gataatttta aaggtaatta gagtggattt 1740
tttttaagtt agaatataga aagttttatt gcgagttttg ttgttattta gttagtttta 1800
ggtagggttg gtaagttgta gtttaaggta tattgtaatt tttttagttg gttttttagg 1860
ggatagaatg attaagaata atagtttagt gaatatttag agtagtgttt ttaaggaatt 1920
ttgtagaggc ggtttttgaa aagttaaggt agtatttgtt tgttgaaggt gtttttagga 1980
ttttatttgt ttttttttag gaattatatt atttgttttt ttgtttatat ttttgtttgt 2040
tgtgtttaat tatagttatt atgtttcggg gttagaagag taagtttcgt gttcgtgaga 2100
aacgttagcg gattcgtggt tagatttagg attttaaggt tggttagttt attgtagtag 2160
agaaagaaga gttttttttt ttttttttat ttgttttgag ggatattgtt tttagttttt 2220
ttgtttttgg tattttttag gagttttaga gagagttatt tattattttt gttgttgtag 2280

ttatgttatg tattggattt gataaaggcg acgagagtta agatgaggaa aatgtaagtt 2340
ttttttaggt tttaatattt attgagagat tatttaaaga ttttttaatt aggaagacga 2400
agatgttagt gtagtttttg ttgtataagt ataaaatgaa agagtttatt ataaaggtag 2460
aaatgttgaa gattattagt aaaaagtata aggagtattt t 2501

<210> 101
<211> 2501
<212> DNA
<213> Artificial Sequence

<220>
<223> chemically treated genomic DNA (Homo sapiens)

<400> 101

gaagtgtttt ttgtattttt tgttgatgat ttttagtatt tttgtttttg tagtgggttt 60
ttttatttta tatttgtata gtaggaattg tattaatatt ttcgtttttt tggttagaga 120
atttttgagt gattttttag tggatgttga ggtttgggag gaatttgtat tttttttatt 180
ttggttttcg tcgtttttat tagatttagt gtatgatata gttgtagtag tagaggtggt 240
gggtggtttt ttttgaggtt tttggggaat gttaaaagta agggagttgg aggtagtatt 300
ttttaaaata gatgaggaag aggaaggaga tttttttttt tttgttgtag taggttgatt 360
aattttgaga ttttgggttt gattacgggt tcgttggcgt tttttacggg tacggagttt 420
atttttttga tttcgaggta tgatggttgt ggttaggtat agtaggtagg agtgtaggta 480
gaagggtagg tgatgtggtt tttggagaag agtaaatgaa attttgagaa tatttttagt 540
aggtagatat tattttggtt ttttagaagt cgtttttgta ggattttttg agaatattgt 600
tttaagtatt tattgggtta ttgtttttag ttattttgtt ttttgagaag ttagttgagg 660
aagttataat gtgttttagg ttgtagtttg ttaattttgt ttggggttga ttgggtgata 720
ataaggttcg tagtggggtt ttttgtgttt tggtttggag aggatttatt ttgattattt 780
ttagaattgt tatgttagtt tttggtaggg tttgagtttt tttttattgg tgttgtgaaa 840
ttgatttttt agttttttgg gatttagatg agggaagttt aggggttttt taatttatta 900
tttttgttta gaagtattta gtgttttttt tgttttgggt tttttaggtt ttaagtttgt 960
atttaggatt tttatttgga ttttaagtag ggttgtggat tgtttttttt gttgattgat 1020
aattgtaatt ttagattaag aattttattt tttttagttt tggtataaaa tgtgatgggt 1080
atttatgaaa gaaaataatt gataatttgg atttcgttaa attaaaattt ttgttttgtt 1140
aaagatatta taaagagaat aagataattt ttaggttggg aaaaaattat ttttaaaaga 1200
tatatttgat aagggattgt taattaaaat atataaaaat gttttaaaat tgaataataa 1260
gaattgatta attttattaa aaatggataa aagatttaaa taggtatttt aataaataat 1320
atatatggaa ggtaaatacg tatatgaaat gatggttaat attatatatt attagagatt 1380
tgtaaattaa aataataatg tgatattatt atatatttat aagaatggtt aaaattaaaa 1440
attttgataa ttttaaatat tgatgaggat gtggagtaat aggagtatat tgttattgtt 1500
gatggatatg aaaaatgata tagttatttt tgaagatagt ttgtaagttt tttataaagt 1560
tatgtatatt tttattatat gatttagtaa gtgcgttttt tgttatttag ttagatgagt 1620
tgaaaacggt atgtttataa aaaaaatttg tatataaatg tttatagtag ttttatttat 1680
aattgtttaa attgggaagt aagtaatatg ttttttagaa ggagagtgga taaattaata 1740
gaataaaata ttatttagta ttaaaaggga atgaattatt aagttataaa gagatatgga 1800
tgaaatttag atatatatta taaagtgaaa gaagttaatt tgaaaagttt atatattgta 1860
taattttatt tatatgatat tttggaaaag gtaaaagtat ggaaatagta taaagtttag 1920
tggtttttaa aggttgggga gagtgagaga taaatagatt aggtatagag aatttttaag 1980
gtagtaaaat aattttggat gattttatgg tggtaaatat atgttattat atatttttaa 2040
atattaaaat tatagaatgt gtagtaatag gaatgaattt taaattacgg atattgggtg 2100
ataatgatgt gtttatgtat ttttaattgt aataaatgta ttattttggt ttgggaggtt 2160
gatatgaagg aggttgtgtt tgtgggagga taggatgttt gtgagatttt tttttttgtt 2220

tagttttgtt gtgaacgtaa aattatttta aaaaagaagg ttaattatta aaaaaaaaaa 2280
gtgatggtga gtttttagtt ttatatgttg ttttggtaat ttaagagtta tgtagggggg 2340
tggattttgt tgtttttttt atttagaggt gagtagtttt tttagttttt attcggtttt 2400
tttgatttgg tttttagtag attttagggt tttttttttt tttgatttga agattttttt 2460
atttaaaagg tttgtatttt tttgagatta gataggagga a 2501

<210> 102
<211> 2501
<212> DNA
<213> Artificial Sequence

<220>
<223> chemically treated genomic DNA (Homo sapiens)

<400> 102

ttttatttta tattattttc ggaatacggt tttttttttg taaggtttgt agtagaaaaa 60
ttggttatgt ttgtagggat gtgatatttt ttagaaagtt tttaatgaaa gatgattttt 120
agtttagagg atatgggaaa ttaatatttt agaatatatg aggttatgta tagattgatt 180
tttatttgtt tttagattta gagagttcga gatagaattg ttaggttgag tatttattta 240
tttaattttt agatttttta tggatatggt ttaagaaggt taattttatg ttagtagatg 300
tagtttagtt tttgatagat atgaacgtga ggattttgaa tgaagatgat ggaaggattt 360
agttagaatt gtggggtttt aaaaagttta gtttaggttg ttagtttttt aaagtttaga 420
atagttttgg ttaaatgtag tttattataa tttttagttt agaggtcggt agagggtgag 480
tattttgatt tgatgggaat agttttaggt tagtggagga tggagtgttt taagattggt 540
taggtgttat ggtgaggatt ttgaagagaa ataagagaat tgttatagga ttaacgagtt 600
tttgtgttag ttgtaaagta atagatttat tataataaaa tagtaaagtt tgtagtaaag 660
agtttaatga ttgtagggtg ttaagtgagg agatgagaag agatttttaa atttattttt 720
ttaaggagtt ttgggttggg atttgtaagg agattatgga gggcgagggg ttggagaatt 780
gaggttgttg attcgttggg gtaaagggga tgaaattatt agaatgtgga aattgcgttt 840
tttttttttt agttagtttt tgtggggttt tttggattag ttgatgttag tagggttttt 900
tagagtagtt ggtattagtg gggtttttta gattagttga gttattagaa ttattagtat 960
gtaggatttt aaggaatatt ttaaagaaaa aaagttttat tatgttaaaa ttgttattta 1020
tagagtaatt aagggtaatt ataatttagt aataggattt atatgatttt gggataatag 1080
gtattaaatt attatgagga agtaggttag agagtagttg attttatgat taatgttgaa 1140
tgttttgtaa gtttggcgta tttttattta tttttttttt tttttttttg attaatttta 1200
tatagtttat aggggtagtt ttaaaattat ttattttaga atagaggagt ttataaattt 1260
tattaatgtt gttatttttg gggagtttag aagagaatta tgaggtggag aaattttatt 1320
aatatgtttc gtgtgtttta tagaggtgaa gattttggat tagtggaaat ggttttaggt 1380
taaagagata aaatttacgt tttaatagga attaaggtga ggattttaag tgttacgagg 1440
gggttttttt ttttgtaata ttagtagagg gggttaaata gagtttttcg ttattgttag 1500
tatcgagagg ttttaaaata agtatttttg aatttttttt attttatttg tatagtttcg 1560
gggaaggaag ggtttttgtt tgaagagggt gattttagtt gtgtagagag agagttttgg 1620
tttttacggg aattaaggtg aggattttga gggcggatga gaagattttt ttttaaaaaa 1680
ggtatattta tagagttttg tcgttgttgt taggtttgtg aggtttaggt aggggtggtt 1740
tgtttggtac gtttagattt ttatagtggg ggttgaggga ggtgggggta ttgtttggag 1800
gttggcggat ttgggttagt acgtatattc gttttaggtt gttagatatt gaggtgagga 1860
tttttagtgg agacgaaggg attagtaacg ttagaatagt gacgttcggt agcgtttagt 1920
cgttagtttt tagacgttac gggttgtcgg atgtgagtta ttttgatttt cgttttgaaa 1980
aaaaagattc gagcggatgt ggtttatttt gattttcgtt ttggaggcga ggattcgagc 2040
gagtgtaggg ggtgcggcgt ttggttagtt aggggtgaat ttttaggatt ggtcggtagt 2100
taaggtgagg attttgagtg taaattgaag agattatttt tatttgtaat aaagagggtt 2160
ttattaagtt tcgtttttgt atttggtttt gagaggtttc ggtaaagtcg ttcggtaatg 2220
ttttatttgg aaagttttag ggtaggggaa gggtgggggg aggggtagtc gcgggggaag 2280
gaggtttgga cgtagggaat aggttttatt ttgtacgtag ggtgggtgta ggttttaatt 2340
gaaattaatt tgagggtttt aaatgtggat tgaagagaat attttttatt tttaataaag 2400
gtggttttat taagtttcgt ttttgaagac ggttttggga ggttttagta aagttgtgtt 2460
tggaaagttt tagggagggg agggttttga atttaggaag t 2501

<210> 103
<211> 2501
<212> DNA
<213> Artificial Sequence

<220>
<223> chemically treated genomic DNA (Homo sapiens)

<400> 103

gttttttagg tttaaggttt tttttttttt gagatttttt aggtatagtt ttgttggggt 60
tttttagagt cgtttttagg gacgagattt agtgaggtta tttttgttaa gggtaggaga 120
tgtttttttt agtttatatt tagggttttt aaattgattt tagttagggt ttatatttat 180
tttacgtgta gaatgaggtt tattttttgc gtttaaattt ttttttttcg cgattgtttt 240
tttttttatt ttttttttgt tttggaattt tttaggtgga atattgtcgg acggttttat 300
cggagttttt taggattaaa tgtagaagcg ggatttagtg ggtttttttt tgttataggt 360

gggggtggtt tttttagttt atatttaggg tttttatttt gattgtcgat tagttttgag 420
aatttatttt tggttgatta gacgtcgtat tttttatatt cgttcgggtt ttcgttttta 480
aagcggaagt taggatgagt tatattcgtt cgggtttttt tttttaaagc ggaagttagg 540
atgatttata ttcggtagtt cgtggcgttt gagggttgac ggttggacgt tatcggacgt 600
tattgtttta gcgttgttgg ttttttcgtt tttattaggg gtttttattt tagtatttag 660
tagtttggga cgaatatgcg tgttgattta aattcgttag tttttaaata atatttttat 720
tttttttagt ttttattgtg gaaatttaag cgtgttaaat aggttatttt tgtttgggtt 780
ttataggttt gatagtagcg gtagggtttt gtgaatgtgt tttttttggg gagaggtttt 840
tttattcgtt tttagagttt ttattttgat tttcgtgaaa gttaagattt tttttttgta 900
taattggggt tatttttttt agataaaggt tttttttttt tcgagattat ataggtggaa 960
tgaaggggat ttagagatat ttattttggg gtttttcggt gttaataatg gcgaggggtt 1020
ttgtttggtt ttttttgttg gtgttgtaag ggggagagtt ttttcgtaat atttaggatt 1080
tttattttga tttttgttag ggcgtgggtt ttgttttttt gatttagggt tatttttatt 1140
agtttaaggt ttttattttt gtgaaatata cgaggtatat tgatgggatt tttttatttt 1200
ataatttttt tttgggtttt ttaagggtga tagtattggt ggaatttgtg ggtttttttg 1260
ttttggggtg ggtggttttg aaattgtttt tatgaattgt ataaaattaa ttagggaaga 1320
gggaagggag ataaatgaag atacgttaag tttgtagaat atttagtatt aattatgaag 1380
ttagttgttt tttgatttgt ttttttatag tagtttggtg tttattattt tagaattatg 1440
tagattttgt tattagatta tagttgtttt taattgtttt atagataata attttaatat 1500
aatgaaattt tttttttttg agatattttt taaggttttg tatattgatg attttaatga 1560
tttagttggt ttgaaggatt ttattgatgt tagttgtttt aaaggatttt attgatatta 1620
gttggtttaa aagattttat aggagttgat taaaaaaaaa aaaacgtagt ttttatattt 1680
tgatgatttt atttttttta ttttaacgaa ttaataattt tagtttttta gtttttcgtt 1740
ttttatgatt tttttgtaaa ttttagttta gaattttttg gggagatgaa tttgagagtt 1800
tttttttatt tttttatttg gtattttgta attattaaat tttttgttgt aaattttgtt 1860
gttttattat agtgggtttg ttattttgta gttgatatag aaattcgttg gttttataat 1920
agttttttta ttttttttta gggtttttat tatgatattt ggttagtttt ggggtatttt 1980
attttttatt gatttgaggt tgtttttatt agattaaggt atttattttt tgtcgatttt 2040
tgaattggaa gttgtggtga gttatattta gttagggttg ttttggattt tgaagggttg 2100
atagtttgag ttggattttt tgggatttta tagttttggt tgggtttttt tattattttt 2160
atttagggtt tttacgttta tatttattag aggttgaatt gtatttattg atatgaagtt 2220
gattttttta ggttatgttt atgagaggtt tgaagattaa atgagtggat gtttagtttg 2280
atagttttgt ttcgagtttt ttaggtttga gagtagatga gagttagttt atgtataatt 2340
ttatatattt tgagatattg gttttttatg ttttttgaat tggggattat tttttattaa 2400
aggttttttg ggaaatgtta tatttttgta aatatgatta gtttttttat tgtaaatttt 2460
gtagagaagg agtcgtgttt cgggagtgat gtgagatagg g 2501

<210> 104
<211> 2501
<212> DNA
<213> Artificial Sequence

<220>
<223> chemically treated genomic DNA (Homo sapiens)

<400> 104
   attattaatt gagtattagt tttttggaag tttttatgtt agtattggga atgttttttt 60
   taaaataaat taattttttg tttatttaat tttagagttt gggagtagtt gttataaaaa 120
   aaaaaaagat gatgaggtgt tttttaaaat ttaaagaata agtataaagg aggaatgaga 180
   aaaagaggag gtttgagatg agagtaatta agtgtaaatg ttttgaggta aggtagtttg 240
   gagtgttagg aaattttaag ttttttattg ggaggtaatt ttaagggaaa ttatatggtg 300
   ggttggatga gattgtggga ggggttaggt ttttagatgg tgttttttag aggtgtgaga 360
   taaagtttgg aatgggaagt agtttttaat agttattttg tgtttacgga taaattagag 420
   agaatttgta tttggggtag gaatggaatg tgttttgtgt ttttgtttta gtgtagtaga 480
   atatagttat agtgtataaa ttaggtgttt tatatagttg gtgtatttta gtgttgagag 540
   attaagttcg gaatgggaaa ttatttttaa tatttatttt tttgttgtgg gaaaattaga 600
   gagaattttt atttaggata aaagtgaaaa gtgttttatg tttttatttt agtatagttt 660
   aatatagtgt ataatttagg tgttttatgt atattatttt tagtgagttt ttgagaaata 720
   agggagatga tagttttagg ggaggtaaga tgtttagaag ttatcgtgtt ggtatttttt 780
   gttttgggtt ggagaattaa gagttcgttt tattaaaagg atttttaata ggggtgtagt 840
   taggtatggt ggtttatgtt tgtaatttta gtattttggg aggcgaggta ggtggatttt 900
   ttgagattag gagtttaaga ttagtttggt taacgtggtg aaatttcgtt tttattaaaa 960
   atataaaaat tagttgggcg tggtggtgga tatttgtaat tttagttatt agggaggttg 1020
   aggtacgaga attatttgag ttttgaggta taggttgtaa tgagtcgaga tagtgttttt 1080
   gtattttagt ttgggtgata gagtgagatt ttattttaaa aaaaaaaaat aaataaataa 1140
   taaaaattaa aataggagtg gttaatatag aagggtgttt aggagttaga aaaaaatatt 1200
   ggttattgat aaaaattttg agatttgagt tgtatttaat tggagaaggt ttttttataa 1260
   atattttata aattatataa ttttttttgt taagtagtat tttgtttgat tataattttt 1320
   ttgtttggag tttggtaata tgtttaagtt tatagggtga gtaagtgata gggttgggat 1380
   tagatttttg ttgtgaattt ttttagagtt tatatttttt attttgtttt tttttgtttg 1440
   gagtagattt tgtgtttttt aatttatttt tttttttaat atttttaaat atattttagg 1500
   ggttaaaaag aaggattttg tgtttaaaga attggattgg aatatatagt taaagtaata 1560
   aagaatatta aaataaatga aagttataaa taaagaggag aaagagatat tatttagtga 1620
   taatatgatt atttatatat gtatagttag ataattttaa aattagggat tatataatta 1680
   ttttgaatag tttttttttt ttaagaagta aattaattaa aatagaagtt atataagagg 1740
   ttagtgttgg ttattgaaaa gagtaaatta gtattatttt tttttgataa tttattagtt 1800
   attttggatt ttttgttttg tgttataatt tttttatttt atattagttt tggggtatag 1860
   atttgtaagg tttgtagtag gaacgttgtt taagagtttg tagggatgtg gtatttttta 1920
   ggaagttttt atttagagag gtaagagaaa ataaggagga tatttataaa taaatattgg 1980
   aaagataatt atattaaaag ataacggggt aatttattta aatttttttt tagtttagaa 2040
   tgttttaggt agtgtagtta gattgagtat tttttttttt attttttaga gtttttagtg 2100
   aagtgagagt tttgatttaa ggtgatttta gttaattaga gggaggattt agggtttggt 2160
   agatattagg gaggattttg aatgaagaat aggaggagtt agaatagtga tgttttatag 2220
   aaattcgatt tcgttgtttg tttttatagg atttaaatat ttttggttta atgtggttta 2280
   ttaaaatttt agtttagatt tcggaggatt ttgatttgag taggatttat ggtatggaat 2340
   gtggagtttt aggattggtt agtagcgaag tgaggttttt gagtgagtaa aaaagggaaa 2400
   attaaattta taaataaggg gatttttcgg agtttaattt atatttttaa ttttggaaag 2460
   ttttaggtag agttataaaa ttggtttgtt ttttatttta g 2501

<210> 105
<211> 2501
<212> DNA
<213> Artificial Sequence

<220>
<223> chemically treated genomic DNA (Homo sapiens)

<400> 105
   ttgaaatgag aggtaagtta gttttatagt tttgtttagg gttttttagg gttaaaaata 60
   tggattgggt ttcgagaagt ttttttattt gtgggtttag tttttttttt tttatttatt 120
   taagaatttt atttcgttgt tggttagttt tgggatttta tattttatgt tatgaatttt 180
   gtttagatta aggtttttcg agatttaggt tgaagttttg atgagttata ttaggttagg 240
   gatgtttggg ttttgtgagg gtagatagcg gagtcgggtt tttatgggat attattgttt 300
   tggttttttt tgttttttat ttaaggtttt ttttgatatt tgttagattt tggatttttt 360
   ttttagttaa ttgaggttat tttaggttaa aatttttatt ttattgagaa ttttgaaggg 420
   taaaagagag gatgtttagt ttgattgtat tgtttggggt attttaggtt gagaagaggt 480
   ttgggtagat tgtttcgtta ttttttggtg tggttgtttt tttagtattt atttatgggt 540
   gtttttttta tttttttttg tttttttgaa taaaggtttt ttgggaaatg ttatattttt 600
   gtaaattttt gggtagcgtt tttattgtaa attttgtaag tttgtgtttt agggttaatg 660
   tgaggtaaag aaattataat ataaagtagg gagtttaagg tagttggtgg gttgttagag 720
   aaaaatgatg ttgatttatt ttttttaata gttagtatta gttttttatg tgatttttgt 780
   tttaattgat ttatttttta aggggaagga gttgtttaag atgattgtgt ggtttttgat 840
   tttgaggtta tttgattgtg tatatgtaga tgattatatt gttattgaat aatatttttt 900
   tttttttttt atttgtagtt tttatttatt ttgatatttt ttattgtttt ggttatgtat 960
   tttaatttaa ttttttgggt atagaatttt tttttttaat ttttgagata tatttggaag 1020
   tattgagaag aaaagtgaat taaagaatat aaagtttgtt ttaggtagag aggaatagaa 1080
   tgaagagtgt gggttttaga agaatttata ataggagttt agttttagtt ttgttattta 1140
   tttattttgt gaatttgagt atattattaa attttaaata aagaaattat aattaaataa 1200
   aatgttgttt agtaaggaaa attatataat ttataaagtg tttgtggaag agtttttttt 1260
   aattggatat aatttaagtt ttaaaatttt tgttaatgat taatattttt ttttaatttt 1320
   taggtatttt tttatgttaa ttatttttgt tttgattttt attatttatt tatttttttt 1380
   ttttgagatg gagttttatt ttgttattta ggttggagtg tagaggtatt atttcggttt 1440
   attgtaattt gtgttttagg gtttaagtga ttttcgtgtt ttagtttttt tagtagttgg 1500
   gattatagat gtttattatt acgtttaatt aatttttgta tttttggtag agacggggtt 1560
   ttattacgtt ggttagatta attttgaatt tttgatttta agagatttat ttgtttcgtt 1620
   ttttaaagtg ttgggattat aggtatgaat tattatgttt ggttgtattt ttgttgaaag 1680
   tttttttaat agagcgggtt tttgattttt taatttagga taaagagtgt tagtacggtg 1740
   gtttttgggt attttatttt ttttgaagtt gttatttttt ttatttttta gaaatttatt 1800
   agagatgatg tatatagaat atttaggttg tgtattgtat tagattgtgt taggataggg 1860
   atatagaata ttttttattt ttgttttagg taggggtttt ttttggtttt tttataatag 1920
   gaaagtaaat gttagggata gttttttatt tcgggtttag ttttttaata ttgaaatgta 1980
   ttaattgtat aaagtattta gtttgtgtat tgtgattatg ttttgttgta ttgggataag 2040
   agtatagggt atattttatt tttgttttag gtgtagattt tttttggttt attcgtgagt 2100
   ataaaataat tgttaagaat tattttttat tttaggtttt gttttatatt tttgagaggt 2160
   attatttgag ggtttggttt tttttatagt tttatttagt ttattatgta gtttttttta 2220
   aaattatttt ttaatggggg atttgaggtt ttttaatatt ttaaattgtt ttgttttagg 2280
   gtatttatat ttgattgttt ttattttaaa tttttttttt tttttatttt ttttttgtat 2340
   ttgtttttta agttttagag agtattttat tatttttttt ttttttatga tagttgtttt 2400
   tagattttaa aattaaatgg gtaagaaatt gatttgtttt ggggaaaata tttttagtat 2460
   taatatgaag atttttaaag agttgatgtt taattaatga t 2501

<210> 106
<211> 2501
<212> DNA
<213> Artificial Sequence

<220>
<223> chemically treated genomic DNA (Homo sapiens)

<400> 106
   aaaggagaag ttttaaaaaa tttttaatat tagtgtatga tagtaatttt tagtaaattt 60
   atattaggag agaattttta ttttgtaaaa tttataattg atatattaaa tgtgaaatat 120
   tataaatatt tcgaatagag tttagaagaa tatatatttg tatagtataa attttattgt 180
   tgataattgg atgaaaatat atttatagtt tataattata ataataatat cgagtaaatt 240
   agaggtaata ataataaaag gagataattg tgataatata attttttaat ttaaaaataa 300
   aataacgtaa ttggtgtaaa aatatttaaa tgtataagga aataaagaaa acgtgtgtat 360
   aaaagattat tataggtaag ttaggagtat agttatataa aattaataat taattataat 420
   atgtaatgtg aaatatgttt tttataatag taataatatt taaaatatat ttaatagaaa 480
   attaaataga aatgtatatg tttattaagg agatagttat aaaaatttat tgaaagattt 540
   aggaaaaata aatttttaat aaagtagtgt attatgttta tttattgtgg aggagatatt 600
   gtaaagttat tgattttttt taaatttttt atagatttat tgttttagtt atagttgtga 660
   agatgataat taaattcggg tggttttttt ttatgattaa atttaattat agaaaagtta 720
   aaggaagaat ttttttttaa aaaaaaagta gtattaataa tagtaaaata ataaaaatgc 780
   gaattttgag agatggttag tttgtattga ataagaatgg gtgtgtgggt aggtaggggt 840
   atgagttgaa gtagagatag gtttgtggtt tataatagac gtagataacg gaattggtta 900
   gaggaaaagt tttaaaattt tggtttttgt tttttttatt gtgttattgg ataatgtatt 960
   tttttttttg ttttattaaa ggaaatttag ttagaaattt ggattttatg tgttagtttt 1020
   atggggtaat attgttatag taaaaatatt tgggtaggtt gaagaagtat gaatttagat 1080
   attgtgttaa taattatttg aaattttttt agtggaagtg gattggtttt taaattcgta 1140
   ttattttaat ataatataaa gaatggatta aattaggata gtaaatattg tagtgagtta 1200
   tataagataa tggtagaaat ttatttatga aggtgttgaa tatagatatt gatgttagtt 1260
   aagagagaag ttttagatat agttattagg ttaagaagtt tttttatttt ttttttatga 1320
   attttatagg atttagttag aggaagggga tgaatttata ttagaaaatg gagaaaattg 1380
   agattttgtt agtagttaat gtgaggattt tgagtgagga ttgaggggtt ttatatatta 1440
   gaagagataa gagtgttttt taaatttttt tttatttttc gattttgtat tataatttaa 1500
   tagtagtagt taggtttagg aggttttagg tagtagtggt taggtgaggt gtaattgaat 1560
   tgtatatgag gagttttagg aagttgaagt ttagtttgag agggtgtttt taagttagta 1620
   gaggggatat ggtaagaatg aagcgacgat attgagtgag ggttgagggt aaagtttatt 1680
   ttaaaaaggg ggttttatag agttttattt ttattgttag ttttggaagg tttcggtatg 1740
   gttttcgagt ttaggttgta ttttattttt aattttggat ttttagggaa tgagagtttt 1800
   tattttcggt tgattgattt agtttaatag atagagaagt ttaagggttc gcgaagagtt 1860
   tttgaaagaa ttttacgtga gtttttggga atttgttgta tttatagaat ataaggggga 1920
   gtaatagagt tttgtagtta ttgttacgta gggagcgtta ggtgggcgtg gtcggaagtg 1980
   gttttttttg atttttattt aataaagggg tttgagtcgg tcgtttgagt ttgaaaagtg 2040
   ttgttacgtt agcggaagga ggcgttttag atttttttag ttgttttggt gggtattttg 2100
   atttagaacg gggggtattt ttcgataatt ttaatttttt tttttcgaag aaaggcggtt 2160
   ttatagagtt ttgttttaat ttgtttcgat ttggtttttg aggagttttt aaatatggtt 2220
   gttaggttta aagtttattt attttttttc gtcgtgttga agaatatgaa ggtttcggtt 2280
   taaggtttgt ggatttaggt tagtagaggg agaaatttta ggttttatat agttaagagg 2340
   atttttttta gaattgaggg aggggatttt atgtttcgta acgggttgta tcgaattttg 2400
   tttttgacgt tagtttgggg gttcgggtag gtttttagag attgaggtgt atttttattt 2460
   tgtttttgtg agagttagag aggtaggttt ttggttgtaa g 2501

<210> 107
<211> 2501
<212> DNA
<213> Artificial Sequence

<220>
<223> chemically treated genomic DNA (Homo sapiens)

<400> 107
   tttataatta ggggtttatt tttttgattt ttatagggat aaagtgagaa tgtattttag 60
   tttttagaga tttgttcgga tttttaggtt aacgttaagg gtagagttcg gtgtaattcg 120
   ttacgggata tggaattttt ttttttaatt ttaaggaggg tttttttaat tatgtagggt 180
   ttgagatttt ttttttattt gatttgggtt tataagtttt ggatcgaggt ttttatgttt 240
   tttaatacga cggaaaaaaa tgaatggatt ttgggtttaa tagttatgtt tggggatttt 300
   ttagggatta ggtcggggta ggttggggta gggttttgtg gagtcgtttt ttttcggggg 360
   aagggggttg aagttgtcgg ggaatgtttt tcgttttagg ttaggatatt tattaagata 420
   gttgagaaga tttgggacgt tttttttcgt tgacgtgata gtatttttta ggtttaggcg 480
   atcggtttag atttttttat taggtggaag ttaagaggaa ttattttcgg ttacgtttat 540
   ttggcgtttt ttgcgtaata gtagttgtag ggttttgtta tttttttttg tgttttgtgg 600
   gtgtagtaga ttttagggaa tttacgtaaa gtttttttag gggtttttcg cggatttttg 660
   gattttttta tttgttgagt tgaattagtt aatcggagat agaggttttt attttttgag 720
   agtttagagt tggaagtaag gtgtagtttg agttcgggag ttatgtcggg attttttaag 780
   attgatagta ggggtggagt tttgtgagat tttttttttg gaatggattt tgtttttagt 840
   ttttatttag tgtcgtcgtt ttatttttgt tatgtttttt ttattgattt gaggatattt 900
   ttttagatta gattttaatt ttttgagatt ttttatgtgt aatttagtta tattttattt 960
   ggttattatt gtttggggtt ttttaaattt ggttgttgtt attaagttgt gatgtaagat 1020
   cggggaatga aggagatttt gggggtattt tttgtttttt ttgatatgtg gagtttttta 1080
   gtttttattt agggttttta tattgattgt tggtagggtt ttagtttttt ttattttttg 1140
   atgtaaattt attttttttt tttgattaga ttttgtaaaa tttataagga gaaagtgaga 1200
   agatttttta gtttgatagt tatgtttggg attttttttt tggttgatat tagtgtttgt 1260
   atttaatatt tttatgaatg aatttttatt attattttgt gtgatttatt gtaatatttg 1320
   ttattttaat ttagtttatt ttttatatta tattaaaata atacgaattt aaaggttagt 1380
   ttatttttat taaagaagtt ttaggtagtt attagtatag tgtttggatt tatatttttt 1440
   tagtttattt agatgttttt gttgtggtaa tattatttta tggaattggt atatagggtt 1500
   taagtttttg attgaatttt ttttagtgaa gtagaaaaaa aaatgtattg tttaatggta 1560
   tagtgaaaga agtaaagatt aaagttttaa agtttttttt ttagttaatt tcgttatttg 1620
   cgtttgttgt aggttataga tttgttttta ttttagttta tgtttttatt tgtttatata 1680
   tttatttttg tttaatataa attgattatt ttttaaggtt cgtatttttg ttgttttgtt 1740
   gttgttaata ttgttttttt tttaaaaaaa aatttttttt ttaatttttt tatggttgag 1800
   tttggttatg ggaaagaatt attcgggttt ggttgttatt tttatagtta tggttaaggt 1860
   aatgaatttg taaaaaattt aaggagaatt aataatttta taatattttt tttatagtaa 1920
   ataaatatag tatattgttt tattggagat ttattttttt taagtttttt agtaaatttt 1980
   tatagttgtt tttttaatga atatgtatat ttttatttgg ttttttgtta gatatatttt 2040
   ggatattgtt gttgttgtga aaggtatatt ttatattata tattataatt agttattgat 2100
   tttgtgtgat tatatttttg atttatttat ggtgattttt tgtatatacg ttttttttat 2160
   ttttttatat atttaaatat ttttgtatta attacgttgt tttattttta aattaggaga 2220
   ttatattatt ataattattt ttttttgtta ttgttatttt taatttattc ggtattatta 2280
   ttatagttgt gaattataga tgtattttta tttagttgtt agtagtaggg tttatattat 2340
   ataggtgtat atttttttgg attttattcg ggatgtttgt aatgttttat atttagtatg 2400
   ttaattatag attttataag atggaaattt ttttttaatg taagtttgtt aaaagttatt 2460
   attatatatt ggtattgagg gttttttaag gttttttttt t 2501

<210> 108
<211> 2501
<212> DNA
<213> Artificial Sequence

<220>

<223> chemically treated genomic DNA (Homo sapiens)

<400> 108

tttattaggg tttttagatt tataaatttt tgatttttat tttgggagtt agttagaggg 60
gagatttttt ttgaggaggg tagttttagt taaatagagg gagtagtttt agggttggtt 120
atttaataag aagagaattt tgtcgaagga tagtgatttt cgaatacggg ggaggtatag 180
gttgtatttt gttatttttt gttgttagtt ttggaaaaag gtggggtgtg agtgttaagg 240
attggggagg tgggagggaa gaggttagta gggtttaggg agtgagtaga aaggtttgga 300
gtatattttg gaggtgggaa ggaagggatg gaaaggaggt tgataagcgg gcggcggggg 360
gggggcgttt tttttttttg agaggaggtt tcgttttacg ttattcgggt tgttagtttt 420
gattaatttt gatagagttt tcggatgtgt tttgtcggat gtgttttttc ggcggttatt 480
ttgggagttt gaaggatttg aggtattttg tgacgaggat cgttttaggt tagcggaggg 540
aggagattta tagatttatt tagtttttaa ggtgagggtt tcgagggaga agtgagggat 600
aattttatta tagagagaag tagttttggt ttgttttatt tttgcggtgg tatttgtagg 660
tggcggaaac gaagatggta gtcgagggtt gagggacgtg tttttacggt agaagaaggg 720
aggaaatgtc ggttttttag agaataaata ggaaggtatt gaggaggatc gggggaattt 780
ttattttaga ggatagattt ttagagattt ttattttgtt ttttaaatta gtttttatag 840
agttttttag gtggttgagg ttgagcggtt atttttttat tgttgggtga ggggtgtagg 900
ggagggggag gaggaggttt tgttttgggg gttttatggt aagttagtat ggggagttgt 960
ttttagttgg tagagggaag atttttaggt tttgttggga ataagagtga ggattgaggg 1020
gttatatgtg cgttagaata gacgtgaggt tatagggatc gttttcgtgg tagagtgttg 1080
ggaggtggtt gttattttat tattttttat tgtttttagt gatgtggagt ttttttgagg 1140
ttttttttta ggttagtaga gtggtggggg tttggttttg tttgagaaaa tgtgataatg 1200
ttaattgaat tttgaggggg ttacgaattt tagaattgtg ggattttggg agtttggtta 1260
gttttagttg tcgttttagt tggaggggtt tttttttttt tttttgtagg tgttttagaa 1320
agtaggagtt gaagatttgg gtgtgaggga tatatatatt ttaaaagtat tatagtagag 1380
gaggtttagg tagtgttagg agttaaggtg agtgtacgat ttgattgtgt attaagggtt 1440
ttatttttag aaatagggta gatttggtag tattcggttt gtagttattt attgttattt 1500
ttggtgtttt aggttttgtt tgttagttgt gtttcgaggt gttttttcgc gtttttttat 1560
aggtttttag aagataaatt ttttaggaag ataggcgatt tgtgaggttt tagagtatta 1620
ttttaagaga agaagagttg taagtcggtt tttgttagag ttattatggg tgagtttttt 1680
agttgaggtt attggtattg tttttttttt ttttagtttt gtgggatttt attatattta 1740
ttcgtgttta tacgtttatt tgttgttttt gaataatatt tattatgttt ttttttttaa 1800
attttttacg ttttagtttt gagtaaggtt tttagaaggt aatttttata ttggagttgg 1860
tagatgtaga ggatttttta gatgaggaag aggaggaagt tttttttatt tttttttttt 1920
ttttttattt tttatttttt tcgttttttt tttgtttttt ttttttattt ttattttttt 1980
ttttgttttt gcgtttttta ggggataagg atatgtttat tgttgggatg tcgagttttt 2040
tttagagttt ttttgagagt ttttagagtt gttttgaggg ggaggatttt tagttttttt 2100
tttagatttt ttagagtttt tttgagagcg acgatatttt gtattttttt tagagttttt 2160
agagtcgttt tgagggggag gatttttcgg atttttttta gagatttttt gaggggaagg 2220
atttttagtt ttttttttag attttttaga gtttttttga gggcgacgat atttagtttt 2280
ttttttagaa tttttagagt ttttttgagg ggaaggattt tttgtttttt ttagagattt 2340
tttagagttt ttttgagggt gaggatgttt agtttttttt gtagaatttt gcgagttttt 2400
tttttttttt tgttttattg agtatttttt agagtttttt tgagagtatt taaagttttt 2460
ttgagggttt tttttagttt gttttttaga tttttgtgag t 2501

<210> 109
<211> 2501
<212> DNA
<213> Artificial Sequence

<220>
<223> chemically treated genomic DNA (Homo sapiens)

<400> 109

gtttatagga atttggagaa tagattgggg aaaattttta aaaggatttt gagtattttt 60
aggggaattt tggaaaatat ttaataaagt agaggagaag aaggaattcg taggattttg 120
tagaggagat tggatatttt tatttttagg agggttttga gaaattttta gaggagatag 180
ggagtttttt tttttaggag aattttgaga attttggaga ggagattggg tgtcgtcgtt 240
tttaggagaa ttttggggaa tttggagagg agattgggag tttttttttt taggaggttt 300
ttggagagga ttcgaggagt tttttttttt agaacgattt tgaggatttt ggagaggata 360
tagggtgtcg tcgtttttag gagaattttg gggaatttgg agaggagatt gggagttttt 420

ttttttagga taattttgag gatttttaga ggaattttgg agaagattcg gtattttagt 480
agtaggtata tttttgtttt ttggagaacg tagaagtaga gaggaggatg aggatgagaa 540
agaaaaatag gaggaggacg aggggaataa aaagtggaaa gaggaagaga aaatggagga 600
agtttttttt ttttttttat ttgggggatt ttttgtattt attaatttta gtatgaaaat 660
tgttttttgg aagttttgtt taaagttggg gcgtggaagg tttagaaaga gaggtatgat 720
gaatattgtt taggagtagt aggtaaacgt gtgaatacga ataggtatga tgggatttta 780
taggattgag ggagagaggg atagtgttag tggttttagt tgagaaattt atttatgatg 840
gttttgataa aggtcggttt atagtttttt tttttttaag gtggtgtttt agggttttat 900
aggtcgtttg tttttttagg gggtttgttt tttgggaatt tgtagaagga cgcgagaaag 960
tatttcgggg tatagttggt aggtagagtt tgaggtatta ggaatgatag tgggtggtta 1020
taggtcgggt gttgttaggt ttgttttgtt tttgggggta gggtttttgg tatatagtta 1080
ggtcgtgtat ttattttgat ttttggtatt gtttgggttt tttttgttgt ggtgttttta 1140
ggatgtatgt gttttttata tttaggtttt taatttttgt tttttggagt atttgtaaga 1200
ggaagggagg gagttttttt agttaagacg gtagttgggg ttggttagat ttttagagtt 1260
ttataatttt gaggttcgtg atttttttag aatttaatta gtattgttat atttttttag 1320
atagggttag gtttttatta ttttgttggt ttgaggaaaa attttaggaa aattttatat 1380
tattgagagt agtgggaggt agtgaggtgg tagttatttt ttagtatttt attacggggg 1440
cggtttttgt ggttttacgt ttgttttgac gtatatgtga ttttttagtt tttatttttg 1500
tttttagtag ggtttgggaa tttttttttt gttaattgga ggtagttttt tatgttgatt 1560
tgttatggga tttttagaat aaggtttttt tttttttttt ttttatattt tttatttagt 1620
agtgagaggg tggtcgttta gttttagtta tttggggagt tttatggggg ttgatttgag 1680
gagtagggtg ggaatttttg ggaatttgtt ttttgaggtg gggggttttt cggttttttt 1740
taatgttttt ttatttattt tttagagggt cggtattttt tttttttttt tgtcgtgagg 1800
atacgttttt tagttttcgg ttattatttt cgttttcgtt atttataagt gttatcgtag 1860
ggatagggta ggttagggtt gttttttttt gtggtggggt tgttttttat ttttttttcg 1920
gggtttttat tttgaagatt ggataggttt ataagttttt ttttttcgtt gatttgagac 1980
gattttcgtt ataaaatgtt ttaggttttt tagattttta agatggtcgt cgggaaagta 2040
tattcggtaa agtatattcg ggaattttgt tagggttgat tagggttgat agttcggata 2100
gcgtggggcg gggttttttt ttagaggagg aaggcgtttt tttttcgtcg ttcgtttgtt 2160
agtttttttt ttattttttt tttttatttt ttaggatgtg ttttaaattt ttttgtttat 2220
tttttaagtt ttgttgattt tttttttttt atttttttag tttttgatat ttatatttta 2280
ttttttttta gggttgatag tagggagtgg tagggtgtag tttgtgtttt tttcgtgttc 2340
ggaggttatt gtttttcgat agagtttttt ttttgttgag tggttagttt tggggttatt 2400
ttttttgttt aattgaagtt gtttttttta gagaaaattt tttttttggt tgatttttaa 2460
aatggaagtt agaaatttgt aaatttgggg atttgatgg g 2501

<210> 110
<211> 2501
<212> DNA
<213> Artificial Sequence

<220>
<223> chemically treated genomic DNA (Homo sapiens)

<400> 110
   ttttagtagg gtttgggaat tttttttttg ttaattagag gtagtttttc gtgttgattt 60
   gttatgggat ttttagatta aggttttcgt tttttttacg ttatttattt aggaataaga 120
   tggttgttat ttcgtttgag ttgattgggg agttttacga gggttgatat ttgaggagta 180
   gggtgggaat ttttgggaat ttgttttttg aggtgggggt ttttttagtt ttttttagtg 240
   tttttttatt tattttttag agggtcggta tttttttttt ttttttgggg ttattttttt 300
   gtttgggatt tattagtttt acggtagcgt agtataggtc ggggtttttt ttttttatgg 360
   tgggaggttt tttagttttt ttttagggtt tttattttga ggattggtta ggtttaagat 420
   tttttttttc gttaatttga ttcgcgtttt tttataaatg ttttcgattc gttagatttt 480
   taagatggtc gtcgggaaag tatattcggg aattttgtcg tggttgttta gggttgacgt 540
   agcgttaatg tggggcgggg tttttttttt gaggaggaat tattttttat ttttttgttt 600
   tttttttatt tttttttttt tattttttag ttgtttttta attttttttg tttatttttt 660
   aagttttgcg gattttttta tttttatttt tttagttttt gatattttat tttttttttt 720
   ttaggggtgt tagtagggag tggtttggtg tagtttgcgt ttttttttcg tgttgggagg 780
   ttatcgtttt tttatatttt ttttgttttt tggttagttt tgggattttt ttttttgttt 840
   aattgaagtt gtttttttta gagataatag tttttttttt tgttgatttt taaaatggaa 900
   gttagaaaat tgtatatttg gggattttga tggggttttt tagagttggt agtagggata 960
   ggggaggggt attattagtt atgggtgttt ttttttagtt tttttgagag gttgtgtttg 1020
   gatttttgat attgtttggg atgtttgatt ttgtgtattt gagtttttta ttttcgaata 1080
   agtttttttt atttttgaga tttgtaaggt gtaagagagg gagaattata tttatttata 1140
   gtgggatagg gttttttagt tttaaggtag gtttttattg ttttttgaaa tggggtggga 1200
   atgttttttt gtttttttta aggtatgatt tttaaaagag tttatatttt tttttttttt 1260
   tgtttagata gtaatatttg ttttttcgat ttttaaattt tattttaagt ttttttattt 1320
   tattgttgtg agtgaaatta gtgttatttt ttttatattt tgtgttaaag gtttttttat 1380
   tgttgttttt ttttttattt tagtttttga aaaaaattat tgttttttta gtaattcgtg 1440
   attttttaaa aaattaatac gtgattaaag ttttaaatat atatattaga tttaattaga 1500
   atgtgaagtt atatgtattt tggaaaatgt atggattttt atatttatta attaagtatt 1560
   atattgatta gttttatttg tttaaatttt ttttgtgtat tttttttgag gttaattatc 1620
   gtatttttat aaagttttta gtaattgttt tttgttttta gtttttatat ttttaatttt 1680
   tttaatttgt tatatgatta gttttataaa atattgattt aaaagtgtgt ttgtattatt 1740
   ttttattata attagtaagg aatgagaatt tttgttattt tatatttttt tttatattga 1800
   gtattttaga tttttaggtt ttagttattt ttataagtat gtattggaat tttttttggt 1860
   ttttgtttgt ttttttttaa tgatatatga tattggttat ttttttatat gttttttttt 1920
   cgtttgttat attttttttc gtaaagtgtt tatttaaatt atttgtatcg ttttatagat 1980
   gaataaattt tatttttaga gtattgttaa tttaaaaata taaagagaaa tttttattag 2040
   aattagagtt tttatataat ttatattatt cgttttgttt tgattgttgt atatatatat 2100
   aaagtttatt tttttattta tattttgtat tattatgata tgtttgttat aattgatgga 2160
   attgtattga tattttatat ttaagtttat ggtttatatt aggatttatt tttaacgatg 2220
   gggatattat atatttcgat aagggtgtaa tggtaagtat ttattattgt attgttttat 2280
   agaattgttt tattatttta gagtttttgt gtgttttatt taatgaatta attttttttg 2340
   ttttaatttt agtttttagt ttttggaaat tagtaatatt tttagtgtat ttattattgt 2400
   atgttttaga atgtggtatg ttaggaagtt tttttagatt tttttttttt atttagtaat 2460
   atgtttttat gattttttta ggtttatttt attaataatg a 2501

<210> 111
<211> 2501
<212> DNA
<213> Artificial Sequence

<220>
<223> chemically treated genomic DNA (Homo sapiens)

<400> 111

ttattgttag tgggatagat ttggaggaat tatgaaagta tattgttaaa tgaaagaaga 60
aaatttaaaa aggtttttta gtatattata ttttggaata tgtaatgatg aatatattaa 120
aaatattatt ggtttttagg ggttgagggt tggggttggg gtaaggggaa ttgatttatt 180
aggtggaata tataggagtt ttaagatggt gaaataattt tgtgggataa tataatgatg 240
aatatttgtt attatatttt tatcgaaatg tataatattt ttatcgttaa gagtgagttt 300
tgatgtaaat tatggattta agtgtgaggt attaatatag ttttattagt tgtagtaaat 360
atgttataat aatataaaat gtaaataaaa agatagattt tgtgtgtgtg tgtagtaatt 420
agggtagggc gggtggtgta gattatgtag gaattttaat tttgatgaag attttttttt 480
gtatttttga attagtaatg ttttaaaaat gaagtttatt tatttataaa acgatgtaaa 540
tgatttgaat agatatttta cgaaagggaa tatagtaaac ggaaaaaaag tatataaaaa 600
gatgattaat gttatatgtt attaagaaga ggtaaataaa aattaaagaa ggttttaata 660
tatgtttatg agaatggtta aaatttgaaa atttaaaata tttaatgtaa gagaggatat 720
agaataatag aaatttttat tttttgttag ttgtgataaa aaatagtata ggtatatttt 780
taagttagta ttttatgaag ttaattatat gataaattgg aaaagttaaa agtataggaa 840
ttgaaaatag aagatagttg ttaggagttt tgtagaagtg cggtgattga ttttaaaagg 900
gatgtatagg agaagtttag gtaggtggaa ttggttagta tgatatttgg ttggtagata 960
tgggagttta tgtatttttt aaaatatata taattttata ttttagttaa atttgatgtg 1020
tgtatttaaa attttaatta cgtattggtt ttttaaaggg ttacgggttg ttgaaaagat 1080
agtggttttt tttagggatt aggataggaa aagaagtaat aataaggaag tttttaatat 1140
agggtatggg agagatgata ttaattttat ttatagtagt aaagtggaaa aatttggaat 1200
ggaatttaga ggtcgaagga ataaatgttg ttgtttggat aggagaagga aggagtatag 1260
gtttttttag gagttatatt ttgagaaaga tagaggggta tttttatttt attttagagg 1320
gtagtggagg tttgttttag ggttaggagg ttttatttta ttgtggatgg atgtgatttt 1380
tttttttttg tattttgtag gttttaggga tgggaggggt ttattcggag gtgaaagatt 1440
tagatgtata gggttaggta ttttaggtag tattaggagt ttaggtatag ttttttagga 1500
aggttggaag ggaatattta tgattggtag tgtttttttt ttgtttttgt tgttagtttt 1560
ggaggatttt attagggttt ttagatgtat aattttttga tttttatttt gagagttagt 1620
agaaggggag gttgttgttt ttgaggaggg tagttttagt taagtagaga aagaagtttt 1680
agaattggtt aaaaaataag agagatgtgg aaggacggtg attttttaat acggagggga 1740

ggcgtagatt gtattaagtt attttttgtt gatatttttg gaaaaaggag agtggggtgt 1800
taaggattgg ggaggtggga gtgaagaggt tcgtaaggtt tagggagtga gtaggaaggg 1860
ttggagggta gttgggaggt ggggaggaag ggatggaaaa aaagtagaga ggtggggggt 1920
ggtttttttt tagagaggag gtttcgtttt atattgacgt tgcgttagtt ttggataatt 1980
acggtagggt tttcggatgt gttttttcga cggttatttt gggaatttga cggatcgaag 2040
gtatttgtga ggaggcgcga attaagttag cggggggaag agttttagat ttggttagtt 2100
tttagggtga gggttttgag gaagaattga gggatttttt attatagaga gaagaaattt 2160
cggtttgtat tgcgttgtcg tgagattggt aggttttaga tagggaaatg gttttagaag 2220
aagggaggag gtgtcggttt tttagggaat aaataggaag atattgagga gggttggggg 2280
aatttttatt ttagagggta gatttttaga gatttttatt ttgtttttta agtattagtt 2340
ttcgtagagt tttttagtta gtttaggcgg ggtggtagtt attttatttt tgggtgagtg 2400
gcgtagggga ggcggaggtt ttggtttgag ggttttatgg taagttagta cggggagttg 2460
tttttggttg gtagagggaa gatttttagg ttttgttggg g 2501

<210> 112
<211> 2501
<212> DNA
<213> Artificial Sequence

<220>
<223> chemically treated genomic DNA (Homo sapiens)

<400> 112

agtgggatgc ggggtgtagg ggtcggtttt aggttttttg agtttaagtt tcgttttatt 60
tcgtttcgta agtttagaaa agatgttttt gtttttggtt gggtaatttt tggatttatt 120
tattgtttgt aatttttcgg gttgaagaga ttatttttcg ggagttaatt aatttatttt 180
ttggagcgcg cgtttttttt tgtttttagg agtcggtttt ttaaaatttt ttgaggttcg 240
cgtatgtttt tttcgatttt tcgtgttttt attttttata gaaatttacg tatttacgtt 300
tttttttttt ttcgagtttg tagaggattt cgttttgttt tttttatatt tagggttgtt 360
ttttttgtcg ttaatataaa tttgttgata ggttacgttt gggaagcggg tggggtgttt 420
cggagcggtg ttgaggcgtt gtaggttcgg tttttggttg cgggggagtt gtattttgaa 480
gtttcgtcgg aattcgcgtt tggggttagt agggggtatt agagttaata gggattgtat 540
gtaaaattta ttttttttta ggttttttag ggggatggtt aggtttttgt agagatgggg 600
aggatttttt tttggtaagg ggagagggac gggttaggag ttaagcggaa ggatttcgtg 660
tttaaggttt tttaagggac gggataggcg aggaattttt ttgagttttt tgaattgttt 720
tattagttag gtttgggaag tatttcgttt ttataaattt tgtttttttg aaaggggtcg 780
ggggggggat tttaatattt tttgttatag gttatgatgg gtatggtggt taagagtaat 840
aggtcgcgtg gtgaggttgg ttttttgtgg ttttgttttg ttatttttcg aggtattttt 900
atgttagtga atagagttgc gggtttttag ttgtatagat gaatgtacga ataaaatata 960
ggtttatata aaggaagtta ttgtattgat ttagattcga tttgggattg tcgtataaaa 1020
tagggttgat aaaaatcgtc gtgtttttat tttggaaata gagatttagt tttttttttt 1080
ttgtttgagg tatttagtaa aggtttgttt ttgagtggtt gtggatgttt aaggatttac 1140
gatttttgat tttataaagt ttagggtgaa aataaaaaat aaaataagga gattattgtt 1200
ttaaagtggt cgttgtagtt aattatttta agaaagtaaa taggttggta ttgaaagtta 1260
tataggcggg cggttttttg ggagggttgt ttagtttttt ttttttgaga tttgagtagg 1320
gagtagttag aggagtttga agggatggtg tttagggtat gggaataggt tgtatatatt 1380
tttacggagt acggtgtttg tttagtttga gaagggaaag aaacgggagt agagggttga 1440
tgagaaggta gtagatggat agtgcgggat tgaaaagggg atttatttga agattggtta 1500
ttattattta tttttattat ttgattgttg ttgttttatt gagatcgatg cgtggagaat 1560
aagttgggga gtggaataaa tggtagggag gggagagaag tgagtgaata tgattttggt 1620
ttagttttat aggatagagc gtggttatga agtttataga ggaggatagg tagggaggaa 1680
ttatggtatg gttaggttgt agggtaagag gtagagagga gagaggattg tggaggatgt 1740
ttgcggagtg tcgattgtta gtcgaggttg gtttttattt tatttatttt tttttttga 1800
gacggagttt cgtttttgtt taggttggag tgtagtggcg cgatttcggt ttattgtaag 1860
tttcgttttt cgggtttatg ttattttttc gttttagttt tttaagtatt gggattatag 1920
gtgttcgtta ttatgtttgg ttaatttttt tgtttttaat agagataggg ttttacgtgt 1980
tagttaggat ggttttgatt ttttgaattt atgattcgtt tatttcggtt ttttaaagtg 2040
ttgggattgt aggtattagt tattgtgttt ggttaaggtt ggtttttttt tttatggcga 2100
tgggtgtttg aaagttgtat atattaaaat aaatttatta tttgttgatt taaagtaaaa 2160
ttttgagggt atgaaaggga agggtttatg tttatatggt tgagataaaa gttgttttag 2220
gaagttgggt gtggtggttt atttttgtaa ttttagtatt ttgggaggtt aaggttggag 2280
gattttttga ggttaggaat tgaagattag tttgagtgat atagtaagat tttatttata 2340
tttttaaaaa gggttggttg tttcgggatt tttcgaaata gttttgtaag atatttttgt 2400

tttggaatta taataattta gataagatgt ttttgaaaga atatttgttt aggtatagcg 2460
tttttattaa tgaattgatg ttaattgtgg ttttgagttt t 2501

<210> 113
<211> 2501
<212> DNA
<213> Artificial Sequence

<220>
<223> chemically treated genomic DNA (Homo sapiens)

<400> 113

aagatttaaa attatagttg gtattagttt attggtggag acgttgtgtt taggtaggtg 60
tttttttaaa agtattttat ttaaattgtt gtgattttaa aataggaatg ttttgtaggg 120
ttatttcgaa aagtttcgag atagttaatt ttttttaaaa atatagatgg ggttttgtta 180
tattatttag gttagttttt aatttttggt tttaggggat tttttaattt tggtttttta 240
aagtgttggg attatagggg tgagttatta tatttagttt tttgagatag tttttatttt 300
agttatgtaa gtatgagttt ttttttttta tatttttagg gttttatttt gggttaataa 360
atagtgagtt tattttggta tgtgtaattt ttaaatattt atcgttataa aagaaaggat 420
tagttttggt taggtatagt ggttgatgtt tgtaatttta gtattttggg aggtcgaggt 480
gggcggatta tgagtttagg agattaagat tattttggtt aatacgtgaa attttgtttt 540
tattaaaaat aaaaaaatta gttaggtatg gtagcgggta tttgtagttt tagtatttgg 600
gaggttgagg cgagagaatg gtatgaattc gggaggcgga gtttgtagtg agtcgagatc 660
gcgttattgt attttagttt gggtaagagc gagatttcgt tttaaaaaaa aaaaataaat 720
aaataaagga ttagtttcga ttgatagtcg gtatttcgta ggtatttttt ataatttttt 780
tttttttttg tttttgtttt tataatttgg ttatgttatg attttttttt atttgttttt 840
ttttgtggat tttatgatta cgttttgttt tatgaggttg gattagggtt atgtttattt 900
attttttttt tttttttgtt atttgtttta ttttttaatt tgttttttac gtatcgattt 960
taatggagta atagtaatta gatgatgaaa ataaataatg gtggttagtt tttaaatgga 1020
tttttttttt aatttcgtat tatttatttg ttgttttttt attagttttt tgttttcgtt 1080
tttttttttt tttaaattgg ataagtatcg tatttcgtga agatgtgtgt agtttgtttt 1140
tatgttttga atattatttt tttaagtttt tttggttgtt ttttgtttag gttttagaaa 1200
aggagagttg ggtaattttt ttagggagtc gttcgtttgt gtaattttta gtattagttt 1260
gtttgttttt ttgagataat tagttataac ggttatttta aaataatggt ttttttattt 1320
tgttttttgt ttttatttta aattttataa agttagaaat cgtaagtttt tgagtattta 1380
tagttattta aggataaatt tttgttgaat gttttaagta aagaggggga ggttgagttt 1440
ttgtttttag ggtgaaaata cgacggtttt tattagtttt gttttatgcg ataattttag 1500
gtcgaatttg gattaatgta atgatttttt ttgtatggat ttatgttttg ttcgtgtatt 1560
tatttgtgta gttgggaatt cgtagttttg tttattggta tggggatgtt tcggggaatg 1620
gtagggtaga gttataaaaa gttagtttta ttacgcggtt tgttgttttt agttattatg 1680
tttattatag tttgtggtag gagatgttgg ggtttttttt tcgatttttt ttaggaaaat 1740
agagtttgtg gaggcggagt attttttagg tttgattgat aaaataattt aaaagattta 1800
aagagatttt tcgtttgttt cgttttttga agggttttga atacggggtt ttttcgtttg 1860
gtttttagtt cgtttttttt tttttattag gaaaggattt tttttatttt tgtagaagtt 1920
tgattatttt tttagagggt ttgggaggaa gtgggttttg tatatagttt ttgttgattt 1980
tagtgttttt tgttggtttt agacgcgagt ttcggcgagg ttttagggta tagttttttc 2040
gtagttagaa gtcgggtttg tagcgtttta gtatcgtttc gggatatttt attcgttttt 2100
taggcgtgat ttgttaatag gtttgtattg gcgataaaag gagtagtttt gaatgtaggg 2160
aaagtagggc ggagtttttt gtaggttcgg gggaggggag gggcgtgaat gcgtggattt 2220
ttgtggagag tggaaatacg gggagtcgag gggagtatgc gcgggtttta gaaagttttg 2280
ggaaatcgat ttttgggagt agggaggaac gcgcgtttta gagagtaagt taattgattt 2340
tcggggggtg gtttttttag ttcggggggt tatagatagt aggtaaattt agagattatt 2400
tagttaaggg taggggtatt ttttttagat ttgcggggcg gggtggggcg agatttgagt 2460
ttaggggatt tggaatcggt ttttgtattt cgtattttat t 2501

<210> 114
<211> 2501
<212> DNA
<213> Artificial Sequence

<220>
<223> chemically treated genomic DNA (Homo sapiens)

<400> 114

tgttattaga gtgtatttta cgtgtacgtt tttttttaag agttattgta aggattttgg 60
gtttgttgcg tggtttaaga atttacgttg tttttgaggt tagatttagt tatagcgatt 120
agggtttttt cgttatggtt ttggtggtgg ttggttttaa attggttata gtttgaagat 180
ttattttgaa agggattaag gttagagata gcgttttttt ttttttattt ttagtttagt 240
atttttatta agtatatttt ttggggatta taaacgtttt ttttttcgaa gattttagta 300
gtgatttgtg ggattttggt tgggtgttcg tgatttattt ttaaagagaa tttgtacgtt 360
tggggtttta ggatggttgg tttgtttttt tcgttcgaag atgatttcgt tttttttttt 420
tagtttcggt gggttttcgg attgtcgtcg tcgcgttttt ttcggtttgg gtacgttggg 480
tcggcgtggt ttcggttcgc gtttttgttg ggcggtcgcg gcgcgggtgg cggcgggata 540
agtcgtgagc gcgcgttggg cgcgcgttcg gtcggggtag tcgcggggga ggagcgttcg 600
ttcgtcgttc gcgcgtttaa cggattaggt tggggtcgtg aggtaattgt tgtagttagc 660
ggaggtggga ggcgatattg agtttttagt ttcgagaggt gttcgaggga aaaggaggcg 720
gtagttaaat tggttttgga gagaagtttt tttcgttttt ttttttagtt agtatgtttc 780
ggatttcgtc gttttttagt tcgcgcggtg gggatttcgg gtcgtggcgg tcggcgtagt 840
tttgacgggt tgcgaattag ggggcgtttc gaacgcgggg gttggggttt gggagcgcga 900
gcggtcgtta cggtacgagc ggggtgtgtt gagtttcgtg gttattttcg gttttagtta 960
tgaggaggga cgagcgagac gttaaagtta tgcggttttt gtagtcgtcg gatggggtcg 1020
gttcgttttt cgagagtttg aggaacggtt acgtgaagag ttgcgtgagt tttttgcggt 1080
aggatttttc gcgcggtttt tttttttatt tttgtcgttt ttgtaacgtg gagttgcggt 1140
cgtcgtcggt ttttttttag tagtcgcggc gttgtttttt tttttgtcgg gcgcgttttt 1200
cgttgggcgt tttggttgtt tttgttttcg ttttgttgtt gggcgcggaa ttcgagagtt 1260
gggttgtcgg ggtcgtttgg ttgcggacgt tgttgagcgt gtgttcgtat agtttgagtt 1320
ttttttttag tatcgtttgt gttttttttt tttttatttg tttttttatt cggattaagc 1380
ggggattcgg ttcgggtcgg agttgcggtt tttggtggtt gttggcgttg ttcgtttgtt 1440
gttatttggg ggattttttg gtgtggtagt ggtggttttg gttttggggg gatggcgacg 1500
tagggttcgc ggtttcgtat acgttttcgg aggcggtagc gggtaggttg ttgttggtgt 1560
tgagttgcgt agggttgttg ttgacgttcg cgtattcgtt gcggtttcgg tattgcgttt 1620
tggtgttgtt tttggttagt ttcgtttggt gggttttttt tattagtttc gggtcgttgt 1680
ttttcgtttt taggtcgttg tttttcggtt tggtgggggg cgttggttgt ttgttggttt 1740
tggggttgga ttattttttt taaattaggg aagcgttttt ttattttcga ttgtttagtg 1800
tcgtcgaaga aaaagtgttt gtgattcgat ttcggaggag gtttagttgc gtgtcgttag 1860
gagaaattgt agttagttat tatggtagtt gtaaaatatt taggagatcg tcgttgtttt 1920
gtatttttag agaataggta tgttagttgg aaggcgaggt ttgggacgcg agcgggttcg 1980
ggtttatcgt tgtagttttc gagttgaatt cgtttatttt aaagtatgtt aatttttaga 2040
atggaaaaag ggtgtgttgc gggggggggg ggggaggaaa taatgtttta ttttggaaag 2100
gatttttaaa atataggaag ttttttaaaa atgtagaagt aagtagtttt tgttattgaa 2160
tagaaattaa atagttcgtt aattatatgg tattttttaa ggattgtata tgtaatagat 2220
gagttcgtag aagaatgagg tgaattattt gtggttgaat agattgatta ttttcgtttt 2280
tggtattttt tgatatgtgt ttttttggtg ttatttattt gtttgttgtt ataatgatgt 2340
ttatttaaaa ataatatata gtttttagaa tgagaggata tatttatgag ttttaattag 2400

tgtggtttcg ttattgaaaa tcgagaggaa aaatttgaga ttttatcgtt ataagatatg 2460
agttttgtaa atgagagtaa tttagatgaa aagatagtag t 2501

<210> 115
<211> 2501
<212> DNA
<213> Artificial Sequence

<220>
<223> chemically treated genomic DNA (Homo sapiens)

<400> 115
   attgttgttt ttttatttaa attattttta tttgtaaagt ttatgttttg taacgataaa 60
   attttagatt ttttttttcg atttttaata acgaaattat attgattaaa atttatagat 120
   gtattttttt attttaaagg ttgtatgtta tttttgggta ggtattatta taatagtaaa 180
   tagataaatg gtattaaaaa gatatatatt agaggatgtt aaaagcggga gtagttaatt 240
   tatttaatta taaatgattt attttatttt tttgcgaatt tatttattgt atatatagtt 300
   tttaaaaagt gttatgtgat tagcgggtta tttgattttt atttaatgat aaaagttatt 360
   tgtttttgta tttttaaaag gttttttgta ttttaagaat tttttttaga ataaaatatt 420
   attttttttt tttttttttt cgtaatatat ttttttttta ttttgaaagt taatatgttt 480
   tgaaataagc gaatttaatt cggaaattgt agcggtaaat tcgaattcgt tcgcgtttta 540
   gatttcgttt tttagttaat atatttgttt tttggaaata taaggtaacg acggtttttt 600
   gaatattttg taattgttat agtaattggt tgtagttttt tttaacgata cgtagttgga 660
   tttttttcgg ggtcggatta taggtatttt tttttcggcg gtattggata gtcgaggatg 720
   aagaggcgtt tttttgattt gaaagaagtg atttaatttt agggttagta ggtagttagc 780
   gtttttattt aggtcggaga gtagcggttt gagggcggag ggtagcgatt cgaggttggt 840
   gaaggagatt tattagacga agttggttag gagtagtatt agaacgtagt gtcggagtcg 900
   tagcgggtgc gcgagcgtta gtagtagttt tacgtagttt agtattagta gtaatttgtt 960
   cgttgtcgtt ttcgggggcg tgtgcggggt cgcggatttt gcgtcgttat ttttttaagg 1020
   ttaagattat tattgttata ttaagaagtt ttttaggtaa tagtaggcgg gtagcgttag 1080
   tagttattag gagtcgtagt ttcggttcgg gtcggttttt cgtttggttc gggtgaggaa 1140
   gtaggtgagg aagaagaagg tataggcgat gttgaagagg gggtttaagt tgtgcgaata 1200
   tacgtttagt agcgttcgta gttaggcggt ttcggtagtt tagttttcgg gtttcgcgtt 1260
   tagtagtagg gcgaggataa aggtagttag ggcgtttagc gagaggcgcg ttcggtagaa 1320
   gggggagtag cgtcgcggtt gttggggaga ggtcggcggc ggtcgtagtt ttacgttgta 1380
   gaagcggtag aggtggaaga agaagtcgcg cggagggttt tgtcgtaagg ggtttacgta 1440
   gttttttacg tagtcgtttt ttagattttc ggggggcgag tcggttttat tcggcggttg 1500
   tagggatcgt atggttttgg cgtttcgttc gttttttttt atggttgggg tcgggggtgg 1560
   ttacgggatt tagtatattt cgttcgtatc gtagcggtcg ttcgcgtttt tagattttaa 1620
   ttttcgcgtt cggggcgttt tttggttcgt aattcgttag ggttgcgtcg gtcgttacgg 1680
   ttcggggttt ttatcgcgcg gattgaggag cggcggagtt cgggatatgt tggttgagga 1740
   gaggggcgga aggggttttt ttttaggatt agtttagttg tcgttttttt ttttttcggg 1800
   tatttttcgg gattggagat ttagtgtcgt tttttatttt cgttggttgt aatagttatt 1860
   ttacggtttt agtttggttc gttgggcgcg cgagcggcgg gcgggcgttt ttttttcgcg 1920
   attgtttcgg tcgggcgcgc gtttagcgcg cgtttacgat ttgtttcgtc gttattcgcg 1980
   tcgcggtcgt ttaataggga cgcgagtcgg gattacgtcg atttagcgtg tttaggtcga 2040
   ggaaagcgcg gcggcggtag ttcgaagatt tatcgggatt gaaagagaag gacgaggtta 2100
   ttttcggacg ggaggggtaa gttagttatt ttgggatttt aggcgtgtag gttttttttg 2160
   agggtaagtt acggatattt agttagaatt ttataggtta ttattggggt tttcgggaaa 2220
   gagggcgttt gtggttttta aggggtgtgt ttagtgggga tgttgagtta agggtgggag 2280
   gggaggggcg ttgtttttaa ttttagtttt ttttagggtg ggtttttaga ttgtgattag 2340
   tttagggtta gttattatta aggttatggc gaagaaattt taatcgttgt ggttagattt 2400
   agttttagaa gtaacgtgga tttttgggtt acgtagtagg tttaggattt ttgtagtggt 2460
   ttttagggag gaacgtgtac gtggagtgta ttttagtagt a 2501

<210> 116
<211> 2501
<212> DNA
<213> Artificial Sequence

<220>
<223> chemically treated genomic DNA (Homo sapiens)

<400> 116
   aagttatatt gttagtggtt tttttgggta tgggggtggg agttgtagag ttaggggttt 60
   ttatttgtgt tttgaaggag ttataggtta atgggggtga gaatggagta ttttttttag 120
   aggtttttaa ggatagaatt tatcggtagt tagtttgata ggtattataa ttttagtttg 180
   gattcggagt gtagtttttg agataaaatt aaaggtttta tggtaaatat agtatgagat 240
   ttttggtggt gggggtggtg gaagttacgg gttggaagtt ggaggtggta ggtgggtggg 300
   tagtaggttt agttttgttt tcgtattttt tttttattag gagttttggt ttttttggtt 360
   ttttttattt agtatatata gttttttttt tgtttttgag gagtggggat attttaaaga 420
   gtaagagttg tgattttttt attttatttt taatgtatat gttgggtttt ataaatttaa 480
   gtatgaataa gatgaaatga tttttgtttt tgaggacgtt tagtgagtgg gagagggggt 540
   ggatttttaa aggggcgtat ataatatagt ttaatatatg ttgagttgtg ttatataggg 600
   agttatagat tgagtaatta ttgggtgagg ggaggggttt taggagagat tgggattgtg 660
   tgtattgcgt tttttttttt tttttttaag atagagtttc gttttgttat taggtcggag 720
   tgtaatggtg tgattttaat ttattgtaat ttttgttttt taggtttaag cgattttttt 780
   gttttagttt tttgagtagt tgggattata ggtgtgcgtt attatattta gttaattttt 840
   gtatttttag tagagacggg gttttattat gtaggttagg atggtttcga ttttttgatt 900
   tcgtgattcg tttgttttgg ttttttaaag tgttgggatt ataggcgtga gttatcgcgt 960
   tcggtttgta ttggggtttt aagttttaat agttttaagt tagggttggt ttttttaaga 1020
   aaaaaattta tatatataga tgtgttatat tttgtattta atttaggggg ttttagggat 1080
   atattgacgt ttatgtggag tttttaaata attttaatag tttacggggt taggttttaa 1140
   aaatttaatt tagggttagg cgtagtggcg tatgtttgta attttagtat tttgggaggt 1200
   taaggcgggt gtatagtttg agtttaggag ttcgagatta gtttggttaa tatagtgatt 1260
   ttatttttta ttttcgtttt ttattaatta tttataaaaa aatataaaaa ttaaaggggc 1320
   gtggtagcgt attttcgtag ttttagttat tgggaaggtt gaggtgggag gattatttga 1380
   gtttaggagg tcgaggttgt agtgcgttgt gattacgtta ttgtatttta gtttgagtaa 1440
   tagagtgaga ttttatttta agggaaaaaa taaaataaaa taaaataggg aacgtgggaa 1500
   ggaagcgttt tttgacgata ttggttatag cgggtttgaa gttgggttgt ggaattggag 1560
   atttcgggaa aataatattc gttggagtta cgattttagg agtttattaa gcgagcgttt 1620
   attgtaggag ggaggtgttt acggaaagat cgtgaggtag tgaatatttc gttgtcggtg 1680
   aagtgtaagc gtagggaggt cggggaggaa tttaaagtta cgaagatatt gatttaatgg 1740
   gttttaagtt tttttagttt tgggatttta ttttcgggtt aatttcgttt tttttgagga 1800
   gcgtatgcgt aatgggatta ttcgtttaaa gaatttattc gattcgagac ggcgcgaatg 1860
   tgtagtcgag cgttgatttc ggttttttta tgagtttttg gggtaggttt taaggcgagc 1920
   gacggaagta gtcgttttta gttttcggta atacgatagc ggttgtcgag cgattcggaa 1980
   gtatttttat tttgcgttgt ttgggttcgg cggtagtcgg gttcggagtg gacgtgttat 2040
   tatggggtcg tttaagaagt atcgcggaga gaaggaggcg gtcgggacga cggcggcggt 2100
   cggtatcggg ggtgttatcg agtagtcgtc gcggtatcgg gaatataaaa aatataagta 2160
   tcggagtggc ggtagtggcg gtagcggtgg cgaacgacgg aagcggagtc gggaacgtgg 2220
   gggcgagcgc gggagcgggc ggcgcggggt cgaagttgag gttcggagta gtacgtacgg 2280
   gcgggagcgt agttaggtag agtttttcga gcggcgcgtg aagcgggaga agcgcgatga 2340
   cggttacgag gtcggtgagg aggcggggtt tgcgtagggg gcgggtcggg cgggggtttc 2400
   ggaacgcgtg ggtttgggtg tgcgcgtagt gttagcgaag ttttttaggt taggaaattt 2460
   ttaagttgtt tattagtttt attaaatagt ttttttacgg g 2501

<210> 117
<211> 2501
<212> DNA
<213> Artificial Sequence

<220>
<223> chemically treated genomic DNA (Homo sapiens)

<400> 117

ttcgtagaaa gattatttaa tagagttggt aaatagtttg ggggtttttt ggtttgaaga 60
gtttcgttga tattgcgcgt atatttagat ttacgcgttt cgggattttc gttcgattcg 120
ttttttgcgt aggtttcgtt tttttatcgg tttcgtagtc gttatcgcgt ttttttcgtt 180
ttacgcgtcg ttcggagggt tttgtttggt tgcgttttcg ttcgtgcgtg ttgtttcggg 240
ttttagtttc ggtttcgcgt cgttcgtttt cgcgttcgtt tttacgtttt cggtttcgtt 300
ttcgtcgttc gttatcgtta tcgttattgt cgttatttcg gtgtttgtgt tttttgtgtt 360
ttcggtgtcg cggcggttgt tcggtggtat tttcggtgtc ggtcgtcgtc gtcgtttcgg 420
tcgttttttt tttttcgcga tgttttttgg acgattttat agtggtacgt ttatttcgag 480
ttcggttgtc gtcgagttta gataacgtaa aatgggaata ttttcgggtc gttcggtagt 540
cgttgtcgtg ttgtcggaaa ttaagagcgg ttgttttcgt cgttcgtttt ggagtttgtt 600
ttagaggttt atgggaaggt cgggattagc gttcgattgt atattcgcgt cgtttcgggt 660
cgggtgggtt ttttgagcga gtggttttat tgcgtatgcg ttttttaagg gaagcggaat 720
taattcggag gtgggatttt agaattggaa ggatttagag tttattaagt tagtgttttc 780
gtggttttaa gttttttttc ggtttttttg cgtttgtatt ttatcggtaa cggggtgttt 840
attattttac ggttttttcg tagatatttt ttttttataa tgggcgttcg tttagtggat 900
ttttagggtc gtggttttaa cgggtgttgt tttttcgaga tttttaattt tatagtttag 960
ttttagattc gttataatta gtatcgttag ggaacgtttt ttttttacgt tttttgtttt 1020
gttttgtttt gttttttttt ttgagatagg attttatttt gttgtttagg ttggagtgta 1080
gtggcgtgat tatagcgtat tgtagtttcg attttttggg tttaagtgat ttttttattt 1140
tagttttttt agtagttggg attacgagag tacgttatta cgttttttta atttttgtat 1200
ttttttgtag atggttggtg gggagcgggg gtagggggtg gggttattat gttggttagg 1260

ttggtttcga atttttgagt ttaggttata tattcgtttt ggttttttaa agtgttgaga 1320
ttataggtat gcgttattgc gtttggtttt ggattggatt tttaaaattt ggtttcgtga 1380
attgttaaga ttgtttagga gttttatatg ggcgttagtg tatttttgga atttttgaaa 1440
ttgaatgtaa aatgtggtat atttgtgtgt atgagttttt tttttgagga gattagtttt 1500
aatttaaaat tattgggatt taaaatttta gtgtaggtcg ggcgcggtgg tttacgtttg 1560
taattttagt attttgggag gttaaggtag gcgaattacg aggttaagag atcgagatta 1620
ttttggttta tatggtaaaa tttcgttttt attaaaaata taaaaattag ttgggtgtgg 1680
tggcgtatat ttgtagtttt agttatttag gaggttgagg taggagaatc gtttgaattt 1740

gggaggtaga ggttgtagtg agttgagatt atattattgt atttcggttt ggtgatagag 1800
cgagattttg ttttaaaaaa aaaaaaaaaa agcgtagtgt atataatttt aatttttttt 1860
gaaatttttt ttttatttta gtggttgttt agtttgtggt tttttgtgtg gtataattta 1920
gtatgtgtta ggttatgttg tatgcgtttt tttaaggatt tatttttttt tttatttatt 1980
gggcgttttt agaggtagga gttattttat tttatttatg tttgaattta taggatttaa 2040
tatatatatt ggagatgggg tggagaagtt atagtttttg ttttttgaga tgtttttatt 2100
ttttaggagt agagaagggg ttgtgtgtat tgggtggggg gaattaaaag agttaaagtt 2160
tttggtgggg agggggtgcg gggatagagt taagtttgtt gtttatttat ttgttatttt 2220
taatttttaa ttcgtagttt ttattatttt tattattaga aattttatat tgtgtttgtt 2280
ataaagtttt taattttatt ttagggattg tatttcgggt ttaggttggg gttgtagtgt 2340
ttgttaggtt ggttgtcggt gggttttgtt tttgggggtt tttgaggaga gtgttttatt 2400
tttattttta ttgatttgtg gtttttttaa gatatagata gaggtttttg attttgtagt 2460
ttttattttt atgtttaagg aaattattga taatgtggtt t 2501

<210> 118
<211> 2501
<212> DNA
<213> Artificial Sequence

<220>
<223> chemically treated genomic DNA (Homo sapiens)

<400> 118
   ttattcgtat ttttcggggg agtttttcgc ggaggaagtt atggcgtatt tatttttatc 60
   gttttcgttt acgttttttt tttgttgatt ttacgttggt tttatggttt tgtatgtcgt 120
   agcggttata gttcgcgata atattttttt ttttgtttta gtcgttttaa ttttattttt 180
   tttttcgtta gttttgggtt tagttttgga tttagtttcg ggttttgaag tcgaggtttc 240
   ggtcgtagtc gttattttgc gtttttaatg attttcgggt tttttcgcgg tcgtcgaaga 300
   ttcgttgagg tagaagttgt tcgcggcgcg cgttttatta aaaattcgtt tttttgttat 360
   tgaaagagaa attgggaatt tagataatag gaaaattttt atatatatta aatttatatt 420
   attttatatt ttgtaaaagt ttttattaga tataaaaatt tattataatg gtatgcgttt 480
   cgttataatt tcggatagaa gttgggtttt tgggtaggaa gtggaagtta tagaggaagt 540
   tatggagcga gagtgatgtt tgtagtgaag aatttggcgt tattttgaga aggttaaagt 600
   gatttttcga ttaaatgttt tatttatcgt ttagtgtgat ttggcgtttg ggtttatttt 660
   ttttagtaat tgcgttttaa acgtaaagaa aattgtaggt tttcggggaa ggatcgattt 720
   tattatttaa gagaaaaatt tattttttag attttttttt tttatgtaaa cgtttggtat 780
   ttagtagacg ttaaatatac gtgagttttt cggttttata agattatata ttgtaaatta 840
   atagtaaatt atattaatac gtattttttt agaaattttt aattgttagg tattgatata 900
   taaattgagt ttgttgttgt tttagagaga aaaatagata tatttatttt tttttaatta 960
   ggatatggta ttcgtttttt ttttaagttt tttatttttg aggtaggaaa gtattggtgt 1020
   gtgtttttat ttttattttg tttttttatt agtaggttta atgggtagga attatgtttg 1080
   ttttgttttt cgttggattt tcgggtttag tagagcgttt tgatatatag taggtattcg 1140
   atatagatgt tggttttatg atgaagtaat tgttttcgaa ttaatataaa agggaagatt 1200
   tagatttagc gagtcggtat tattcgttta gggatatata gttaggaagg aatcgaatta 1260
   ggattttaat ttaggattat ttaagttcga attttaattg aatataagtt tttagcgatt 1320
   ttggacggat ttattaaatt gttttgaatt tcggtttttt tagttgtaaa atagggttag 1380
   gaaaagtgga ttcggtgttt ttttgcgtcg tattttcgta gcgggatttg tgaaagcgaa 1440
   tttgagtttt atattgtaga agtttgaggg ttttgtttta attagaattt agtatttagg 1500
   ataagttttt tagcgatttt agattttttc gttttcgatt tcgttcgttt tcgagaggcg 1560
   tggttaaggc ggggttagaa ggcggacgcg tgtacggagc ggtaaagcgt aggcgtaaag 1620
   tttcggtgac gttagagagg cgcgttttta gttcggagtc gattcgtaga cgcgtttcgt 1680
   ttttcggcgt cgttttggat tggcgtaggc gtaagtcggt aagatggcgg cggttggggt 1740
   ttttcgtttg aggcgggcgg tatcggtttt gttgttgcgg agttttcgtt tgttcgttcg 1800
   gagttgtcgg tttcggttcg attttattat aagaaggtag ggataaaaga gggacgcgcg 1860
   gaatgtcgat ttagcggagg tttgggttgg aggggcggtc gcggggtttt gcgtagttag 1920
   gattgggagt tgtttttttt tacgtttttg ttttgattcg tttttttttg ttgcgtagtg 1980
   aggtttattg taattgataa ataatagtta tcgtttatcg ggcggcgatt tttagggggt 2040
   ttcgtcgttg gtcgcgattt cgtgcgtttt aattttaaat tcgttaatag tttaggaggt 2100
   agggttttgt tgggatttgt ttttttgagt ttagggatag atatattttc ggagcgggtt 2160
   tcggttttag ttatttcgtt gttttggtta gatgattttg ggttagttat tgcgtttttt 2220
   tgaatttgtt tttttaggtg taaatggggg gtttttagtt gttttttata aaggatattg 2280
   tgcgtggagt tttggtatgg tttttggtat ataggtttta gtacgaggag atcgtttttg 2340
   ttattgtttt aggagtgtat aggggagtta ggtttgtaat taatatgcgt aatgtttttg 2400
   ttttgtttgg ggggtttttt ggcggggaag gagggataga tttatagttt gtagtttttt 2460
   gttgtttggt ggtataaagt tttttagacg tgagattttt a 2501

<210> 119
<211> 2501
<212> DNA
<213> Artificial Sequence

<220>
<223> chemically treated genomic DNA (Homo sapiens)

<400> 119

tgggagtttt acgtttgggg gattttgtgt tattagatag taaggggttg tagattatga 60
atttattttt tttttttcgt taaaaaattt tttaaataaa ataaaaatat tacgtatgtt 120
ggttataagt ttgatttttt tatgtatttt taaagtagta atagaaacgg ttttttcgtg 180
ttggggttta tgtgttagga attatgttag gattttacgt atagtatttt ttgtaaggga 240
tagttgagag ttttttattt atatttgggg aaataggttt agagaggcgt agtgattagt 300
ttaaggttat ttggttaggg tagcggagtg gttgaagtcg gagttcgttt cgggggtgtg 360
tttgtttttg gatttagaaa gataagtttt aataggattt tgttttttgg gttgttggcg 420
aatttaaaat tgggacgtac gaagtcgcgg ttagcggcgg ggttttttgg aagtcgtcgt 480
tcgatgagcg gtaattgttg tttattagtt gtagtgagtt ttattgcgta gtaagggaaa 540
gcgagttagg gtaaagacgt gggaggggat agtttttagt tttagttgcg tagaatttcg 600
cggtcgtttt tttagtttag gttttcgttg agtcggtatt tcgcgcgttt tttttttgtt 660
tttatttttt tgtgatagag tcgggtcggg gtcgatagtt tcgggcgggt aggcgggggt 720
ttcgtagtag tagagtcgat gtcgttcgtt ttagacggaa agttttagtc gtcgttattt 780
tgtcggtttg cgtttgcgtt agtttagagc gacgtcgagg ggcggggcgc gtttgcgagt 840
cggtttcgag ttgggagcgc gtttttttga cgttatcgaa gttttgcgtt tgcgttttat 900
cgtttcgtgt acgcgttcgt tttttggttt cgttttgatt acgtttttcg ggggcgggcg 960
gggtcgggag cgggaaggtt tgaagtcgtt ggagggtttg ttttgggtgt tggattttgg 1020
ttgagatagg attttagggt ttttgtagtg tggggtttaa gttcgttttt ataaatttcg 1080
ttgcgggaat gcggcgtaga ggagtatcga gtttattttt tttaatttta ttttgtagtt 1140
aaggaagtcg agatttaggg tagtttagta agttcgttta aggtcgttaa ggatttatgt 1200
ttaattagaa ttcgaatttg ggtaattttg ggttagaatt ttggttcggt ttttttttgg 1260
ttgtgtgttt ttagacgaat ggtatcggtt cgttgagttt gaattttttt ttttgtatta 1320
attcgaggat aattgtttta ttatgaagtt agtatttgtg tcgagtattt attatgtgtt 1380
aaggcgtttt attagattcg gggatttaac ggaaagtaaa atagatataa tttttgttta 1440
ttaagtttgt taatgaggga gtaaggtaga gatagagata tatattaata tttttttatt 1500
ttaaaaatgg gaagtttaag aaggaaacga atattatgtt ttgattgaaa aaaaatgaat 1560
atgtttattt ttttttttaa aataatagta aatttaattt atatgttagt gtttagtaat 1620
taagaatttt taaagaaatg cgtattaatg taatttattg ttggtttgta gtatgtgatt 1680
ttataaaatc ggaaaattta cgtgtattta acgtttatta ggtattagac gtttatataa 1740
aggggaggaa tttgggaaat ggattttttt tttgaatggt gaaatcgatt ttttttcgag 1800
gatttgtagt tttttttgcg tttgaggcgt agttgttggg gagagtaaat ttaagcgtta 1860
ggttatatta ggcgataagt aaggtattta atcgaagaat tattttgatt tttttaagat 1920
ggcgttaaat tttttattat agatattatt ttcgttttat gatttttttt atggttttta 1980
ttttttgttt aaagatttaa tttttgttcg aagttgtgac ggaacgtatg ttattataat 2040
gagtttttat atttggtggg agtttttata gggtataaaa taatgtgaat ttagtgtatg 2100
tggagatttt tttgttattt aagtttttag tttttttttt aatggtaaga aaacgggttt 2160
ttgataaggc gcgcgtcgcg ggtagttttt gttttagcgg gttttcggcg gtcgcgggaa 2220
gattcgagag ttattagaag cgtaagatgg cgattgcggt cgaaatttcg gttttagaat 2280
tcgaggttga gtttaaggtt gggtttaagg ttgacggaga ggaggatgag gttaaggcgg 2340
ttaggataag gaggaaggtg ttatcgcggg ttgtggtcgt tgcgatatat aagattatgg 2400
ggttagcgtg ggattagtag gaggaaggcg tgagcgagag cgatggggat gagtacgtta 2460
tggttttttt cgcggagagt tttttcgggg agtacgagtg g 2501

<210> 120
<211> 2500
<212> DNA
<213> Artificial Sequence

<220>
<223> chemically treated genomic DNA (Homo sapiens)

<400> 120

tcgtgagaaa tattttgttt aattatttgt tcgtaaggcg gataaaggtt taattgaaga      60
aatattttta ttatattttg tttggtagcg ttttaaggaa tattataatg atattttatt     120

agaaaaagga ttaaattatt tgattataag aatattttat taatatttcg tcgggtagta     180
agttatattg tttaggtttt ttttattttt atttataagt attttagttt gtaagcggcg     240
gtgggttttg gtattaagcg ggttttttat ttttataatg gtttgtaata tttttgtgtt     300
gttgtttgag tcgttttcgt tttgtgtgtt ttttttttat tttcgttttt attttagaat      360
ttaataattt tatcgtttta tttatttcgt tggatttata ggggacgttt tttttttattt    420
cgtcggtatt ggattgtttg tttattgtat atattggttt tttaatgttt gcggtaggtt     480
tttgtaggtt ttgaattttc gtagtggttc ggagtttttg ttttttttgt atatattttt      540
ttggtgataa ggtaaagtta tatttttgag ttttgtttga ttatataggt agtgttttag      600
ttaatggtag ttttagggtg gttttatatt ataaagtttt attgttgatt attttttggg       660
tttgcgggcg aggggtgata ggggtgtaga gtaaattaaa tgttgttgtt gttttagtat      720
tttttgaaga tgtattttaa atcgatttgt cgtcgttttt tattttttta tgtttaatgt     780
ttacggttta tatggaagtt agaggatgat ttttttaagt ttttttttat agttattttt      840
attaagtgat ttattttttt gtttttagt ttttattatg atttagtatt ttcgatgttt      900
tattttaaat tttttacgtt agtgtggtta tatttattta ttggcggaga tgtgaattat     960
tttatttttt ttttatagtt tttttatatg tatttcgaag attatttatt tttgggttat    1020
tgttaggtaa attttaattt atgttttttt atttaatgtt tatgtagttt aagtttttta     1080
tttttaattt gaaataatgg tttttagttt gttaaaattt tagtgaataa atatttttta     1140
tcgtgtattt tagttttgtt ttaaattagg ggtgtatatt tttttttttt tttgaaatgg    1200
agttttattg ttgttgttta ggttggagtg tagtggtatg attttagttt attgtaattt     1260
ttgttttttg ggtttaagtg attttttggt ttagtttttt gagtagttgg gattataggt      1320
ttgtgttatt acgttcggtt aatttttgta tttttaggag agacggggtt ttattatttt     1380
ggttaagttg gttttgaatt tttgatttcg tgatttattc gttttggttt ttaaagtgtt    1440
gggattatag gcgtgagtta ttatatttag ttaggacggt tattattaaa attattattg    1500
ataagtaaat tttggttatt tttgtagtat ataataattt aatataaagt ttataattaa    1560
taatatatag aagttatttt agaattataa gagtttttta taattttgga gtatatatta    1620
ataatatttt tataaatata gtttaaagaa agttaaatat tattttatat ttgacgatgt    1680
tttttgtatg atttttatat taaataagat taatatgttt tttttggatt ttagaggatt    1740
tatattaaaa aattaatgag gaattaagtt agaatttgat tttggaaagt tcgttaaaga    1800
taaaaagttt aaaatgtttg atattacgaa atagggttat gggttattgt aaaataagtt    1860
atttatttaa ttaaagtagt aatttaaaga ttttaaaaaa gaaaaaaaaa aggtgaaaat    1920
ttttattttt tgagagagga ggtttaatat tttaaataat aagttttaat taaaaatagg    1980
atgcggtaaa tgaaatttgt ttttttgaaa ttttataata aaagtttaat tattttgagt    2040
ataaaatata atttttataa attttgtaat ttttattatt ttgtattaag gagtgagtta    2100
atgttttaag aaagttttgt taaattgata tagtaattta gatattggtt ttgttttagt    2160
gtgtttttga tattaatggt taatttttag agaaattgag ttattttttt tttaatatta    2220
gtttttataa ttttatatgt ttatttgttt tacgatagtt tttgggtttt gaggagttga    2280
atagttttaa tttttgattt tgtattttat gaacgtagtt tattttgatt agtatttttt    2340
attgtgtttg aagatgaggt tgtaattgat gttagtgttt aagatatagt agaatttggt    2400
gtttttttta gatttagaag ttaaagtttt atagtttaat ggtataagga ttttaaaagt    2460
atatatagaa agttatatag atggaagaaa atattttttt                          2500

<210> 121
<211> 2500
<212> DNA
<213> Artificial Sequence

<220>
<223> chemically treated genomic DNA (Homo sapiens)

<400> 121

aaaaaagtgt ttttttttat ttgtgtaatt ttttgtatgt gtttttaaag tttttgtgtt      60
attgagttat agggttttga tttttgggtt taaaaaggat attaagtttt gttatatttt     120
aaatattgat attaattata gttttatttt taggtatagt agaagatgtt aattaaagta     180
aattgcgttt atgagatgta gggttagaaa ttaaaattat ttaatttttt aaggtttaag     240
gattatcgtg gaataggtgg gtatgtgaga ttgtaagggt tgatattaag agaaaagtag     300
tttagttttt ttaggaatta attattaata ttaaaggtat attaaagtaa aattagtatt     360
taggttgttg tgttagttta ataaggtttt tttggagtat taatttattt tttaatgtaa     420
aatgataaag gttataaggt ttatagaaat tatattttat atttaagatg attaaatttt     480

tattataaaa ttttaaaaaa ataaatttta tttgtcgtat tttgttttta attagggttt 540
attgtttggg atattaagtt ttttttttta aagaataaag atttttattt tttttttttt 600
ttttttgaaa tttttgagtt attgttttgg ttaaatgaat gatttatttt ataatgattt 660
atgattttat ttcgtgatat taagtatttt aaatttttta tttttgacga attttttaaa 720
gttaaatttt aatttgattt tttattaatt ttttgatatg agttttttga agtttaaaag 780
agatatattg gttttatttg gtataaaaat tatataagaa gtatcgttaa atataaaatg 840
gtgtttgatt ttttttgggt tgtatttata gaaatgttat tgatatgtgt tttaaaatta 900
tgggaaattt ttataatttt gaaataattt ttgtatgtta ttaattataa attttatgtt 960
aaattgttgt atgttataga agtaattaaa atttatttgt taatggtggt tttaataatg 1020
gtcgttttgg ttgggtgtgg tggtttacgt ttgtaatttt agtattttgg aggttaaggc 1080
gggtggatta cgaggttagg agtttaagat tagtttggtt aagatggtga gatttcgttt 1140
tttttaaaaa tataaaaatt agtcgggcgt ggtggtatag gtttgtaatt ttagttattt 1200
aggaagttga gttagagaat tatttaaatt taggaggtag aggttgtagt gagttgagat 1260
tatgttattg tattttagtt tgggtaataa tagtgaaatt ttattttaaa aagagaaaaa 1320
aatatatatt tttgatttga aataagatta ggatatacgg taaagaatgt ttatttatta 1380
aagttttgat agattgaagg ttattatttt agattggaaa taggaaattt gaattatatg 1440
gatattgggt aaaagaatat gggttgaaat ttgtttggta gtagtttaaa agtaaatggt 1500
tttcgaggtg tatgtgaagg aattgtagga gggaaatggg ataatttata ttttcgttaa 1560
taaataaatg tagttatatt agcgtggggg atttgaggtg agatatcgaa aatattaagt 1620
tatggtgagg gttggaagat aaaagaatga attatttaat aggaatggtt gtggggaagg 1680
gtttgaagga attatttttt gatttttatg tgaatcgtga atattaaata tggagaaatg 1740
aggagcggcg gtagatcggt ttgggatgta tttttagggg atgttgaaat aataatagta 1800
tttggtttgt tttatatttt tgttattttt cgttcgtaag tttagggagt ggttagtagt 1860
ggggttttgt gatgtgaagt tattttaggg ttgttattgg ttgggatatt gtttgtatga 1920
ttaaataaag tttaagggtg tggttttgtt ttgttattag gagggtatat atagggaggg 1980
taagagtttc gggttattgc gaagatttaa aatttataaa agtttgtcgt agatattgaa 2040
gaattaatat atataatgga taaataattt agtgtcggcg gggtgaagag gagcgttttt 2100
tgtgaattta acgaggtgaa tgagacggta agattgttag gttttgaagt gaaggcgaga 2160
gtgaaagaaa gatatataga gcgggggcgg tttaaataat aatataggaa tattgtagat 2220
tattgtggaa gtggggggtt cgtttaatgt tagagtttat cgtcgtttat aggttggggt 2280
gtttgtaggt aggggtggga ggggtttggg tagtatggtt tgttgttcgg cgggatattg 2340
ataagatgtt tttatgatta ggtggtttgg tttttttttt ggtggaatat tattgtggtg 2400
ttttttagaa cgttgttaag taagatatga tagggatgtt tttttagttg ggtttttgtt 2460
cgttttgcgg ataggtggtt aggtaggatg ttttttacgg 2500

<210> 122
<211> 2501
<212> DNA
<213> Artificial Sequence

<220>
<223> chemically treated genomic DNA (Homo sapiens)

<400> 122

gatgttttta tatagattag tttgtttttt gatttttatt ttgttttatt aataaatgtt 60
tttatgcgta tttatttttt attgttgttg taataaattt tgaatagttt agatattgtt 120
aggcgcggtg gtttacgttt gtaattttag tattttggga ggtcgagata agtggatcgt 180
ttaaggagtt taagattagt ttggttaata aggtgaaatt ttatttttat taggggttgg 240
tgatgggcgt ttgaaatcgt agttatttgg gagtttagaa tagtttttat taatattatt 300
tttttatata tattagggtt gagtgaaaaa ataaattgta tataaaaagt taattataga 360
ggttatttat ataaaatgtt aaaatatgta aaataagagt atatattttt atggataata 420
agtaaatggt aaagtataaa aatatgaatg agaataaaaa attattaaat tgagaagaat 480
ggttgtaagt ggagaaggag ggagggtagg gattgtatag aaagttttaa ggtgatttgt 540
tgatagtggt tttttttgtt tgtttgtttg tttgtttgtt tgtttgtttg tttttgatgg 600
agtttcgttt ttgtcgttta ggttggagtg taatagtata atattagttt attgtaattt 660
ttaattttcg ggtttaaatt attttgttgt ttacgttttt cgagtagttg gaattatagg 720
cgttcgattt atatttagtt atttttttta tttttggtag taataaggtt ttattatgtt 780
tgttaggtag gttttaaatt tttttatttt agatgattta tttgttttag ttttttaaag 840
tgtttagatt ataggtatga gttattatat ttagcgcgtt tatagtattt ttaatatttt 900
gaataaataa attagattta atatagttgt ggggtaatgt tgagattcga ttggatttaa 960
tagtatttta tatatttttt tacgtgtttg aaatatttta tttttaaagg atatgttgtt 1020
tttgttaagt attgttaatg aattaaagga tagttaagaa aatgttataa gtgaaaatat 1080
aaaaaaagaa aatattaaaa gtaaagattt gtaaaaaaaa tttagagttt ttataattaa 1140

tagtatgtaa gtattggata agtattttat gagagaggat aatgaatata ttatgtaata 1200
aagatggatg tttttttgta ttttattaaa attaaatagt ttttttattt ttaaataatt 1260
ttaatttttc gtgatgagtt tggaagggat tttgaaagag tgatttttga tttaaaatat 1320
agagagtttt tttatttgtt agtgagtaga gataatatag agtgaaaaaa agataagttt 1380
ttgcgaagag atttttgggt attttaggat tattaatggg atatatgtat ttatttgttt 1440
ttttgttttg ataatataat tataagataa ggttagaaag tttaaattgt ttttaataga 1500
tttattattc gttaattaaa taggttagat tttataattt tttataatta ttataaggga 1560
tttgaaaagt tagtgtttga gtatttgtta agtttttgac gttaaaggat tgtttttata 1620
ttgaaaatat tttagagtta ttggattaaa ttatttatgg tttattatat atttaatagt 1680
ttaatttata tattataaga tttatttatt tgaagtgtat aatgattttt agttgttgta 1740
tattttgagt ggatatagtt tagattttta attaattaat ttaattattt gggaaaaagt 1800
aaaagatatg taatggaata agatgagatt gtgatggggt ttagttttta tgtgataaat 1860
tatgagatgg aaaattttga attgaagtta tagataaatg aattaattat gattaaatat 1920
aatttagaag taaattttaa ataatttttt aataattagt ggatttataa tttttgttgt 1980
agaatgtttt tatgtaatag aagttttttt agagtttgga aatttttatt aataaagaaa 2040
aattttgatg tatttttttt tagttgaata tttttagatg attttcggta ggttttatag 2100
aattttttcg aaggtgagtg ttttttaaat ttaaaggatt agagaatttt tgttttttta 2160
cggatatgaa tattggtaag agtggaagaa tattaaaaat atttttattg tttttttttt 2220
atgtttatta taatatttga tgtagtatta atggtttgat aatgttaata gttgtgattt 2280
ttaatatttt ttttgttttt atagagatgt tatatatttt aaatagttta tatggattaa 2340
tttatttaat ttttaagaat atttttacga cgacgttttt attattattt ttaaataata 2400
atgagttata tattttagtt attatttata taaaagttat gtgagttggc gtaaattttt 2460
taagtttttt gagatttaga tttttggtta atgaaatggt a 2501

<210> 123
<211> 2501
<212> DNA
<213> Artificial Sequence

<220>
<223> chemically treated genomic DNA (Homo sapiens)

<400> 123
   tgttatttta ttgattagaa gtttggattt tagagaattt agaagatttg cgttaattta 60
   tatggttttt atatggatga taattgaagt gtgtgattta ttattatttg gagatagtaa 120
   tagaaacgtc gtcgtagagg tatttttaag gattaaataa attaatttat gtaaattgtt 180
   tagaatatat ggtattttta tgaaaataaa agaaatatta aggattataa ttgttagtat 240
   tattaagtta ttgatgttat attaggtgtt gtgatagata tggggaggag gtagtgaggg 300
   tatttttgat atttttttat ttttattagt gtttatattc gtggagggat aaaggttttt 360
   tggtttttta gatttgagag atatttattt tcgggaagat tttatggagt ttgtcgaaag 420
   ttatttgagg atgtttaatt gaaagagaat atattagaat ttttttttgt tggtgaagat 480
   ttttaaattt tagagagatt tttgttgtat gagggtattt tgtagtagag gttatgagtt 540
   tattgattgt tgaggagtta tttgagattt gtttttaaat tatgtttagt tatggttggt 600
   ttatttattt gtggttttaa tttagaattt tttattttat ggtttattat atggggatta 660
   aattttatta tagttttatt ttattttatt atatattttt tatttttttt taaataatta 720
   aattgattgg ttgggaattt gaattgtatt tatttaagat gtgtaataat tgaaaattat 780
   tgtatatttt aaatgggtga attttatggt atgtgaatta agttgttaaa tgtgtgatga 840
   attatggatg atttggttta gtggttttga aatattttta gtataaaaat agttttttaa 900
   cgttaaaaat ttggtagata tttaagtatt ggttttttag gttttttata gtgattgtgg 960
   gagattgtag aatttggttt gtttagttgg cgagtaatag gtttattgga gatagtttgg 1020
   attttttgat tttgttttat aattgtgttg ttagagtaga agagtaagta aatatatatg 1080
   ttttattaat agttttgaaa tgtttaaaaa ttttttcgta agaatttgtt ttttttttat 1140
   tttatgttat ttttgtttat tgataagtgg gaaagttttt tgtgttttgg attaggattt 1200
   atttttttaa aatttttttt aagtttatta cggagaattg gggttatttg ggaatgagaa 1260
   gattatttgg ttttgataaa atatagagaa atatttattt ttattatata atgtgtttat 1320
   tatttttttt tataagatat ttatttaata tttatatgtt gttaattata gaaattttgg 1380
   attttttttg tagatttttg tttttaatat tttttttttt tatattttta tttgtaatat 1440
   ttttttaatt gttttttgat ttattaatag tgtttagtaa gaataatatg ttttttaaaa 1500
   atgaaatatt ttaaatacgt ggaaaagtat gtggaatatt attgagttta gtcggatttt 1560
   aatattattt tatagttatg ttagatttga tttatttatt tagagtatta gaaatattat 1620
   aaacgcgttg ggtgtggtag tttatgtttg taatttgagt attttgggag gttgaggtag 1680
   gtggattatt tgagatgagg aagtttgaga tttgtttggt aaatatggtg aaattttgtt 1740
   gttattaaaa ataaaaaaaa tagttgggtg tgggtcgggc gtttgtaatt ttagttattc 1800
   gggaggcgtg ggtagtagaa tgatttgaat tcgggagttg aaggttgtag tgagttgata 1860
   ttatgttatt gtattttagt ttgggcgata agagcgaaat tttattaaaa ataaataaat 1920
   aaataaataa ataaataaat aaataaagaa aattattatt aataagttat tttgaagttt 1980
   tttgtgtaat ttttgttttt tttttttttt tatttatagt tatttttttt aatttgatgg 2040
   ttttttattt ttatttatgt ttttatattt tattatttat ttattattta taaaaatata 2100
   tatttttgtt ttgtatgttt tagtatttta tatgaatggt ttttgtagtt aattttttgt 2160
   gtgtaatttg tttttttatt tagttttggt gtgtatgagg gaataatgtt agtgggaatt 2220
   gttttgaatt tttaaatagt tgcgatttta ggcgtttatt attaattttt agtagagatg 2280
   gggttttatt ttgttggtta ggttggtttt gaattttttg agcgatttat ttgtttcggt 2340
   tttttaaagt gttggaatta taggcgtgag ttatcgcgtt tagtagtgtt taagttattt 2400
   aaaatttgtt atagtagtaa taaaaaatga atacgtatag aaatatttat tagtgaaata 2460
   aaatagaagt taagaaatag attaatttat atgaaaatat t 2501

<210> 124
<211> 2501
<212> DNA
<213> Artificial Sequence

<220>
<223> chemically treated genomic DNA (Homo sapiens)

<400> 124
   ggattatatt aggattgata tttttatgat taatagttag ttatttatta gttttggtta 60
   attttatttg tattttatta ataatgaata gaattaagtt gggagtatga gatgggacga 120
   gggtaagtaa aaatattata aagtttattg tttttacgga gatttagttg gttttttttt 180
   cgtttttttt tttttttttt tttttttttt tagttttgtt ttgtcgttta agttggagtg 240
   tagtggtgtg attttagttt aatgtaattt ttatttttcg ggtttaaatt attgagtgag 300
   tttaggaggt taagattaat ttgggaaata tagtaaaagg tagggtggtg tatgtttgta 360
   gttttaaggt cgaggcggga gaattatttg agttttagag gtagagttta gtttggataa 420
   tatagcgaaa ttgtttttaa tagaaaaatt aaaaatatta gtgggggcgg ggtggtgtga 480
   gtttatagtt tcgaggtcga ggcgggagga ttgtttgagt ttaggaggtc gaggttagtt 540
   tggttaatat agcgaaattt tatttttgtt aataataaga ataatagaaa aatagcgtgg 600
   gcggggtggt ttatttttgt agttttgagg ttaaggtggg agtattgttt gagtttagga 660
   gtttgatatt agtttggtta ataaagcgaa agtttgtttt ttttaaaaaa taaataaata 720
   aataaataaa taaaaatgag cggggtagaa tggtatacgt ttatagtttt gaggtcgagg 780
   tggaagtatt atttgagttt gagaggttga ggttagtttg ggtaatatat cgaaattcgg 840
   tttttattaa aacgaaataa aataaaataa aaaatagttt gggtagggtg gtgtatggtt 900
   gtagttttga gatgggagga ttttttgagt ttaggagttt gatgtcggtt tggttaatat 960
   agggaaatac ggtttttatt aaaaaaaaaa aaaaaaaaaa aagcgtgggt agggtggtgt 1020
   atgtttgtcg tttcgaggtt atggcggaag gattttttga gtttaggacg atgagattag 1080
   tttggttaat atagtaaaat ttggttttta tttaaaaaaa aaaaaaaaaa aaagcgtggg 1140
   ttgggtggtg aatgtttgta gtttcgaggt cgaggtggga ggatcgtttg agtttacgac 1200
   gttaagatta gtttggttaa tatagcgaaa ttttttttat taaaaaatta aaaaataaaa 1260
   aatattagtg ggggtagggt ggttcgcgtt tgtagtttta agacggaggt tggaggattg 1320
   gttaaggtag atgtaattaa tattataatt atattttata agtaaatata tttttttttt 1380
   tttagggtta taggtaaagg atggtaattg tgcgtattat atttaagatt tttttttaaa 1440
   aatgtaagta gaggttgcgg ggttttggat tgttttttta gtggtaatag atatagaagt 1500
   tattgaagaa tgaaagttac gattaagtat agtaaatttt cgtaaatgtg ttagtttgga 1560
   aaatatcgtg ttttttaagt agaaaaatta tagattgatt attttttttt ttttatagtt 1620
   tagattagaa tttagatttt taatttaaga tttaggaacg gtttttagag agtttaaaat 1680
   ttgacggcgt ttgaggatta tttatttttt cgtagtggta ataaagtgtg gttggaggag 1740
   gagatattat tttgttatgt tattgtttaa ggatgatgga ttaattaggg tagttagtga 1800
   attttatttg gttaattaga agttagaata gtaggcggaa taagcgaagc ggatgtggtt 1860
   tttattagtt tcggttttag ggacggaatt tttttaaagt gggggtggag attttaattt 1920
   tttcgtttaa agtatttttt gggattggtt attttaagtt tagagtacgt atgttttgat 1980
   tttttttttt tttcgatttt tttatattta gagtacgtac ggtttgattt tttttttgga 2040
   tttttttaaa attagagtaa gtatacgtcg attttttttt tttatttttt ttattttttt 2100
   tttttttcgc ggtgtatttg ttatttagtt ttaataagga gtgtatatga ggtaggtcgt 2160
   tatttcgaat tttttttgtt agtttttaat ttttttaggt acggattttt ttttaggaat 2220
   tttgtagtaa tttaagaaat ttgggtcggg cgtggtggtt tatatttgta atttttgtat 2280
   ttttggaagt tatagttggt ggatcgtttg aatttaagag gcggaggttg tagtgagtta 2340
   tgattacgtt agtgtatttt cgattgggta atagagcgag attttgtttg aaaaaataaa 2400
   aattaaaaaa gtttatttaa attttttttt ttgggtttaa gtgagttttt cgtttaggtt 2460
   ttggggttat aggcgcgtat tatttcgttt ttgttaaatt t 2501

<210> 125
<211> 2501
<212> DNA
<213> Artificial Sequence

<220>
<223> chemically treated genomic DNA (Homo sapiens)

<400> 125
   aaatttagta gaagcggggt ggtgcgcgtt tgtagtttta aggtttaggc gagaggttta 60
   tttgagttta ggaggaaaga tttgagtgag tttttttggt ttttgttttt ttagataggg 120
   tttcgttttg ttgtttagtc gggagtgtat tggcgtgatt atggtttatt gtaattttcg 180
   ttttttaggt ttaggcgatt tattagttgt ggtttttaaa agtgtaggga ttataagtgt 240
   aagttattac gttcggttta aattttttaa gttattatag agtttttagg aaaaatttcg 300
   tatttgaaaa agttagaaat tgataggaaa gattcgagat ggcggtttgt tttatatata 360
   ttttttatta aaattagata gtaaatgtat cgcggaggag gggaggggta ggaagaatgg 420
   aaaaagaaaa tcggcgtatg tttattttga ttttggaaga atttaaagag aaaattagat 480
   cgtgcgtatt ttgagtatgg aagaatcgaa agagagagaa agttagagta tgcgtatttt 540
   gaatttaaag tagttaattt taggggatgt tttaggcggg aaaattagag tttttatttt 600
   tattttgaga aggtttcgtt tttggagtcg ggattgatag aagttatatt cgtttcgttt 660
   gtttcgttta ttgttttgat ttttaattgg ttagatggag tttattaatt gttttgattg 720
   gtttattatt tttgggtagt gatatagtag aataatgttt ttttttttag ttatattttg 780
   ttgttattgc gaaaaagtgg gtggttttta ggcgtcgtta gattttgaat tttttgaaga 840
   tcgtttttgg attttgggtt aaaaatttgg atttagtttt gaattgtggg aagaaaaaaa 900
   tagttaattt gtgatttttt tatttgaaag atacgatgtt ttttaaatta gtatatttgc 960
   ggaggtttgt tatatttagt cgtggttttt attttttagt ggtttttatg tttgttgtta 1020
   ttgaaaaaat agtttaaggt ttcgtaattt ttgtttatat ttttgaaagg gaattttaag 1080
   tatggtgcgt ataattatta ttttttattt gtagttttga agagaggaaa atgtatttat 1140
   ttatgggatg tgattgtggt attggttata tttgttttgg ttaatttttt agttttcgtt 1200
   ttgggattat aggcgcgaat tattttattt ttattaatat tttttatttt ttgatttttt 1260
   aatagagaag gtttcgttgt gttggttagg ttggttttga cgtcgtgggt ttaagcgatt 1320
   tttttatttc ggtttcggga ttataggtat ttattattta gtttacgttt tttttttttt 1380
   ttttttttaa gtagaaatta ggttttgtta tgttggttag gttggtttta tcgttttggg 1440
   tttaagggat tttttcgtta tggtttcgag acgataggta tgtattattt tgtttacgtt 1500
   tttttttttt tttttttttt tagtagaaat cgtgtttttt tatgttggtt aggtcggtat 1560
   taagtttttg gatttaaggg atttttttat tttaggatta tagttatgta ttattttgtt 1620
   taagttattt tttgttttgt tttgtttcgt tttagtggaa atcgggtttc gatatgttgt 1680
   ttaggttggt tttaattttt taggtttaga tgatgttttt atttcggttt tagaattata 1740
   ggcgtgtgtt attttatttc gtttattttt atttatttat ttatttattt attttttagg 1800
   agaggtaggt tttcgttttg ttggttaggt tggtattaaa tttttgggtt taagtaatgt 1860
   ttttattttg gttttaaaat tataggagtg agttatttcg tttacgttat ttttttgttg 1920
   tttttgttgt tagtagaaat ggggtttcgt tatgttggtt aggttggttt cgatttttta 1980
   ggtttaagta attttttcgt ttcggtttcg ggattatggg tttatattat ttcgttttta 2040
   ttaatatttt taattttttt attagagata gtttcgttat gttgtttagg ttgggtttta 2100
   tttttgggat ttaagtgatt ttttcgtttc ggttttggga ttataggtat gtattatttt 2160
   gttttttgtt atgtttttta ggttggtttt gattttttgg gtttatttaa tgatttgaat 2220
   tcgggaagtg gaggttgtat tgagttgaga ttatattatt gtattttagt ttgggcgata 2280
   gagtaagatt gaaaaagaaa gaaagaaaga aagaaagagc gagagagaga ttagttgaat 2340
   tttcgtaaga atagtgagtt ttgtggtatt tttatttgtt ttcgttttat tttatgtttt 2400
   tagtttggtt ttgtttattg ttgatgaaat atagatagga ttggttagaa ttggtagatg 2460
   gttggttgtt gattatgaaa atgttagttt tgatatagtt t 2501

<210> 126
<211> 2501
<212> DNA
<213> Artificial Sequence

<220>
<223> chemically treated genomic DNA (Homo sapiens)

<400> 126

aaatagtttg agttttttta gttttaggat ttttggtgtt ttgggcgggg tggtgtacgt 60
ttgtagtttc ggggtcgagg cgagaggatc gtttgagttt aggagttttt tttttttttt 120
tttttttttg agatagggtt tcgttttgtt gtttaggttg tgttgtagtg gcgcgatcgt 180
ggtttattgt aattttcgtt tttcgggttt aagcgattta ttatttgtgg gtttttaaag 240
tgttgggatt ataagcgtga gttattacgt ttagtttaga ttttttaagt tattatagag 300
tttttaggaa aaattttata tttgaaaaag ttagaaattg ataggaagaa tttgagatgg 360
cgatttgttt tatataaata tattattaaa attagataag aagtgtatta cggcggaggg 420
gaggggtagg aagaatggaa agagaaaatt agcgtatgtt tattttgatt gtggaagaat 480
taaaagggta aatcggcgtg tgcgtatttt gaatttaaag tagttaattt aaggggacgt 540
tttcggcggg aaaattagag tttttttttt ttttcgtttt gggaaggttt tgtttttaga 600
gtttggattg atagacgtta tattagtttt ttttgtttcg tttattgttt tgatttttaa 660
ttggttagat ggagtttatt aattgttttg attggtttat tatttttggg tagtgatatt 720
gtagaatgtt gtcgtttttt ttagttatat tttgtgcgat aaagtgggtg gtttttaggc 780
gttattagga gattttgaat tttttgaaga tcgtttttgg attttgggtt aaatatttgg 840
attttagttt gaatcgtggg aagaaaaaat agtcgatttg tgattttttt atttgaaaga 900
tataatgttt tttaaattag tatatttgcg gaggtttgtt gtatttagtc gtggagttta 960
ttttttagtg gtttttatat ttgttggaat taaaaaaata gtttaaggtt ttttaatttt 1020
ttttattttt attttttttt tttttttttt tttttttgta gttttttttt tttttttttt 1080
tattattata ttttaagttt tagggtatat gtgtatattg tgtaggttag ttatatatgt 1140
atatatgtgt tacgttggtg cgttgtattt attaattcgt tatttagtat tgggtatatt 1200
ttttaatgtt attttttttt ttttttttta ttttataata gtttttagag tgtgatgttt 1260
ttttttttgt gtttatgtga ttttattgtt taatttttat ttatgagtga gaatattcgg 1320
tgtttggttt tttgtttttg cgatagttta ttgagaatga tgatttttaa ttttatttat 1380
gtttttataa aggatatgaa tttattaatt tttatggttg tatagtattt tatggtgtat 1440
atgtgttata tttttttaat ttagtttatt attgttggat atttgggttg gttttaagtt 1500
tttgttattg tgaatagtgt cgtatttttg gaagggaatg taagtgtggt gcgtataatt 1560
attatttttt atttgtagtt ttggagagag gaaaatgtat ttatttatgg gatgtgattg 1620
tggtattggt tatattcgtt ttggttaatt tttttatttt attttcggga ttataggtat 1680
gtattatttt gtttaggttt gtttttgtta ttattattat tattttttag ttgaaattgg 1740
gtttttttat gtaggttagg ttggttttaa tttttagggt ttaagggatt ttttcgtttt 1800
tttagtttgg ggattatagg tatgtattat tttgtttacg gtattttttt tgttttttag 1860
tagaaatcgg gttttgttat gttgtataga ttggttttaa ttttttgggt ttaagtaatg 1920
tttttatttt agtttcggga ttataggcgt gtgttatttt gttttgataa ttttttttag 1980
gagagatagg ttttcgttat gttgattagg ttggtatcga ttttttgggt ttaagtaatt 2040
tttttatttt agtttgggaa ttataggggt gagttatttc gtttatgtta tttttggttg 2100
ttttttttat tagtagaaat ggggttttat tatgttggtt aggttggttt tgattttttg 2160
ggtttaagta atttttttgt tttagtttcg ggattattgg tatatattat ttagttttta 2220
ttaatatttt taattttttt agtagagata gtttcgttat gttgtttagg ttggttttta 2280
ttttttgggt ttaagtgatt tttttgtttc ggtttcggga ttgtaggttt gtattatttt 2340
gttttttgtt atgtttttta ggttggtttt aattttttgg gtttatttaa tgatttgaat 2400
tcgggaaggg gaggttgtat tgagttgaga ttttattatt gtattttagt ttgggtgata 2460
gagaaaaatt gtcgaaaaga aaaggaaggg aagggagaag g 2501

<210> 127
<211> 2501
<212> DNA
<213> Artificial Sequence

<220>
<223> chemically treated genomic DNA (Homo sapiens)

<400> 127

tttttttttt tttttttttt tttttttcga tagttttttt ttgttattta ggttggagtg 60
tagtggtggg attttagttt aatgtaattt ttttttttcg ggtttaaatt attgagtgaa 120
tttaggaggt tgagattagt ttgggaaata tagtaaaagg tagggtggtg taggtttgta 180
gtttcgaggt cgaggtagga ggattatttg aatttaggag gtagagatta gtttggataa 240
tatagcgaaa ttgtttttat tagaaaaatt aaagatatta gtgggggttg ggtggtgtgt 300
gttagtggtt tcgaggttga ggtaggagga ttgtttgagt ttaggaggtt aaggttagtt 360
tggttaatat agtgaaattt tatttttatt aataagaaga ataattaaaa atagtatggg 420
cggggtggtt tatttttgta gtttttaggt tgaggtggga ggattgtttg agtttagaag 480
gtcgatatta gtttggttaa tatagcgaaa gtttgttttt tttaaaaaaa attattaggg 540
tagaatggta tacgtttatg gtttcgaggt tgaggtggaa gtattgtttg agtttaggag 600

gttgaggtta gtttgtgtaa tatagtaaaa ttcggttttt attgaaaaat aaaaaaaata 660
tcgtgggtag ggtggtgtat gtttgtggtt tttaggttga ggaggcggga ggattttttg 720
agttttggag gttgaggtta gtttggttta tatagagaaa tttagtttta attaaaaaat 780
aataataata ataataaaaa taaatttggg tagggtggtg tatgtttgta gtttcgagaa 840
tgagatggga ggattgatta aggcggatgt aattaatatt ataattatat tttataagta 900
aatatatttt ttttttttta gggttatagg taaaggatgg taattgtgcg tattatattt 960
atattttttt ttaaaaatgc ggtattattt ataatagtaa agatttggaa ttaatttaaa 1020
tgtttaataa tgatagattg gattaagaaa atgtggtata tatatattat ggaatattat 1080
gtagttataa aaattgatga gtttatgttt tttgtaggga tatggatgaa attggaaatt 1140
attattttta gtaaattatc gtaagaataa aaaattaaat atcgaatatt tttatttata 1200
ggtgggaatt gaataatgag attatatgga tataggaagg ggaatattat attttgggga 1260
ttgttgtggg gtggggggag gggggaggga tagtattggg agatatattt aatgttagat 1320
gacgagttag tgggtgtagc gtattagcgt ggtatatgta tatatatgta attaatttgt 1380
ataatgtgta tatgtatttt aaaatttaaa gtataataat aaaagaaaaa aaaaagaaaa 1440
ttataaaaaa aaaaaaaaaa gaaaaaaaaa taagaataaa agaggttgga gggttttgga 1500
ttgttttttt agttttaata gatgtagaag ttattgaaga atgaatttta cgattaagta 1560
tagtaaattt tcgtaaatgt gttagtttgg aaaatattgt gttttttaaa tagaaaaatt 1620
atagatcgat tatttttttt ttttacggtt tagattagaa tttagatgtt taatttaaga 1680
tttagggacg gtttttagag agtttaaaat tttttgatgg cgtttgagga ttatttattt 1740
tgtcgtataa agtgtggttg gaggaggcga taatattttg taatgttatt gtttaaggat 1800
gatggattaa ttagggtagt tagtgaattt tatttggtta attagaagtt agaatagtag 1860
gcggaataag ggaagttgat gtggcgtttg ttagtttagg ttttagggat agaatttttt 1920
taaggcgggg gggaggggag attttgattt tttcgtcgaa agcgtttttt tggattggtt 1980
attttaaatt tagagtacgt atacgtcgat ttgttttttt gattttttta taattagggt 2040
aagtatgcgt tgattttttt tttttatttt ttttattttt ttttttcgtc gtggtgtatt 2100
ttttatttaa ttttaataat gtatttatgt gaggtaggtc gttattttaa attttttttg 2160
ttagttttta atttttttag gtatgggatt ttttttagga gttttgtagt aatttaaaaa 2220
atttgggttg ggcgtggtgg tttacgtttg taattttagt attttggagg tttataggtg 2280
gtggatcgtt tgaattcggg aggcggaggt tgtagtgagt tacgatcgcg ttattgtaat 2340
atagtttggg taatagagcg agattttgtt ttaaaaaaaa aaaaaaaaaa aaaaaatttt 2400
tgggtttaag cgattttttc gtttcggttt cgggattata ggcgtgtatt atttcgttta 2460
gagtattaaa ggttttgagg ttggaaagat ttaggttgtt t 2501

<210> 128
<211> 2501
<212> DNA
<213> Artificial Sequence

<220>
<223> chemically treated genomic DNA (Homo sapiens)

<400> 128
   tttttttttt tttttttttt tttttttcga tagttttttt ttgttattta ggttggagtg 60
   tagtggtggg attttagttt aatgtaattt ttttttttcg ggtttaaatt attgagtgaa 120
   tttaggaggt tgagattagt ttgggaaata tagtaaaagg tagggtggtg taggtttgta 180
   gtttcgaggt cgaggtagga ggattatttg aatttaggag gtagagatta gtttggataa 240
   tatagcgaaa ttgtttttat tagaaaaatt aaagatatta gtgggggttg ggtggtgtgt 300
   gttagtggtt tcgaggtcga ggtaggagga ttgtttgagt ttaggaggtt aaggttagtt 360
   tggttaatat agtgaaattt tatttttatt aataagaaga ataattaaaa atagtatggg 420
   cggggtggtt tatttttgta gtttttaggt tgaggtggga ggattgtttg agtttagaag 480
   gtcgatatta gtttggttaa tatagcgaaa gtttgttttt tttaaaaaaa attattaggg 540
   tagaatggta tacgtttatg gtttcgaggt tgaggtggaa gtattgtttg agtttaggag 600
   gttgaggtta gtttgtgtaa tatagtaaaa ttcggttttt attgaaaaat aaaaaaaata 660
   tcgtgggtag ggtggtgtat gtttgtggtt tttaggttga ggaggtggga ggattttttg 720
   agttttggag gttgaggtta gtttggttta tatagagaaa tttagtttta attaaaaaat 780
   aataataata ataataaaaa taaatttggg tagggtggtg tatgtttgta gtttcgagaa 840
   tgagatggga ggattgatta aggcggatgt aattaatatt ataattatat tttataagta 900
   aatatatttt ttttttttta gggttatagg taaaggatgg taattgtgcg tattatattt 960
   atattttttt ttaaaaatgc ggtattattt ataatagtaa agatttggaa ttaatttaaa 1020
   tgtttaataa tgatagattg gattaagaaa atgtggtata tatatattat ggaatattat 1080
   gtagttataa aaattgatga gtttatgttt tttgtaggga tatggatgaa attggaaatt 1140
   attattttta gtaaattatc gtaagaataa aaaattaaat atcgtatatt tttatttata 1200
   ggtgggaatt gaataatgag attatatgga tataggaagg ggaatattat attttgggga 1260
   ttgttgtggg gtggggggag gggggaggga tagtattggg agatatattt aatgttagat 1320
   gacgagttag tgggtgtagc gtattagcgt ggtatatgta tatatatgta attaatttgt 1380
   ataatgtgta tatgtatttt aaaatttaaa gtataataat aaaagaaaaa aaaaagaaaa 1440
   ttataaaaaa aaaaaaaaaa gaaaaaaaaa taagaataaa agaggttgga gggttttgga 1500
   ttgttttttt agttttaata gatgtagaag ttattgaaga atgaatttta cgattaagta 1560
   tagtaaattt tcgtaaatgt gttagtttgg aaaatattgt gttttttaaa tagaaaaatt 1620
   atagatcgat tatttttttt ttttacggtt tagattagaa tttagatgtt taatttaaga 1680
   tttagggacg gtttttagag agtttaaaat tttttgatgg cgtttgagga ttatttattt 1740
   tgtcgtataa agtgtggttg gaggaggcga taatattttg taatgttatt gtttaaggat 1800
   gatggattaa ttagggtagt tagtgaattt tatttggtta attagaagtt agaatagtag 1860
   gcggaataag cgaagttgat gtggcgtttg ttagtttagg ttttagggat agaatttttt 1920
   taaggcgggg gggaggggag atttgatttt tttcgtcgaa agcgtttttt tggattggtt 1980
   attttaaatt tagagtacgt atacgtcgat ttgttttttt gattttttta taattagggt 2040
   aagtatgcgt tgattttttt tttttatttt ttttattttt ttttttcgtc gtggtgtatt 2100
   ttttatttaa ttttaataat gtatttatgt gaggtaggtc gttattttaa attttttttg 2160
   ttagttttta atttttttag gtatggattt ttttttagga gttttgtagt aatttaaaaa 2220
   atttgggttg ggcgtggtgg tttacgtttg taattttagt attttggagg tttataggtg 2280
   gtggatcgtt tgaattcggg aggcggaggt tgtagtgagt tacgatcgcg ttattgtaat 2340
   atagtttggg taatagagcg agattttgtt ttaaaaaaaa aaaaaaaaaa aaatttttgg 2400
   gtttaagcga ttttttcgtt tcggtttcgg gattataggc gtgtattatt tcgtttagag 2460
   tattaaaggt tttgaggttg gaaagattta ggttgttttt t 2501

<210> 129
<211> 2501
<212> DNA
<213> Artificial Sequence

<220>
<223> chemically treated genomic DNA (Homo sapiens)

<400> 129

gagaaatagt ttgagttttt ttagttttag gatttttggt gttttgggcg gggtggtgta 60
cgtttgtagt ttcggggtcg aggcgagagg atcgtttgag tttaggagtt tttttttttt 120
tttttttttg agatagggtt tcgttttgtt gtttaggttg tgttgtagtg gcgcgatcgt 180
ggtttattgt aattttcgtt tttcgggttt aagcgattta ttatttgtgg gtttttaaag 240
tgttgggatt ataagcgtga gttattacgt ttagtttaga ttttttaagt tattatagag 300
tttttaggaa aaattttata tttgaaaaag ttagaaattg ataggaagaa tttgagatgg 360
cgatttgttt tatataaata tattattaaa attagataag aagtgtatta cggcggaggg 420
gaggggtagg aagaatggaa agagaaaatt agcgtatgtt tattttgatt gtggaagaat 480
taaaagggta aatcggcgtg tgcgtatttt gaatttaaag tagttaattt aaggggacgt 540
tttcggcggg aaaattagag tttttttttt ttttcgtttt gggaaggttt tgtttttaga 600
gtttggattg atagacgtta tattagtttc gtttgtttcg tttattgttt tgatttttaa 660
ttggttagat ggagtttatt aattgttttg attggtttat tatttttggg tagtgatatt 720
gtagaatgtt gtcgtttttt ttagttatat tttgtgcgat aaagtgggtg gtttttaggc 780
gttattagga gattttgaat tttttgaaga tcgtttttgg attttgggtt aaatatttgg 840
attttagttt gaatcgtggg aagaaaaaat agtcgatttg tgattttttt atttgaaaga 900
tataatgttt tttaaattag tatatttgcg gaggtttgtt gtatttagtc gtggagttta 960
ttttttagtg gtttttatat ttgttggaat taaaaaaata gtttaaggtt ttttaatttt 1020
ttttattttt attttttttt tttttttttt tttttttgta gttttttttt tttttttttt 1080
tattattata ttttaagttt tagggtatat gtgtatattg tgtaggttag ttatatatgt 1140
atatatgtgt tacgttggtg cgttgtattt attaattcgt tatttagtat tgggtatatt 1200
ttttaatgtt attttttttt ttttttttta ttttataata gtttttagag tgtgatgttt 1260
ttttttttgt gtttatgtga ttttattgtt taatttttat ttatgagtga gaatatgcgg 1320
tgtttggttt tttgtttttg cgatagttta ttgagaatga tgatttttaa ttttatttat 1380
gtttttataa aggatatgaa tttattaatt tttatggttg tatagtattt tatggtgtat 1440
atgtgttata tttttttaat ttagtttatt attgttggat atttgggttg gttttaagtt 1500
tttgttattg tgaatagtgt cgtatttttg gaagggaatg taagtgtggt gcgtataatt 1560
attatttttt atttgtagtt ttggagagag gaaaatgtat ttatttatgg gatgtgattg 1620
tggtattggt tatattcgtt ttggttaatt tttttatttt attttcggga ttataggtat 1680
gtattatttt gtttaggttt gtttttgtta ttattattat tattttttag ttgaaattgg 1740
gtttttttat gtaggttagg ttggttttaa tttttagggt ttaagggatt tttttatttt 1800
tttagtttgg ggattatagg tatgtattat tttgtttacg gtattttttt tgttttttag 1860
tagaaatcgg gttttgttat gttgtataga ttggttttaa ttttttgggt ttaagtaatg 1920

tttttatttt agtttcggga ttataggcgt gtgttatttt gttttgataa ttttttttag 1980
gagagatagg ttttcgttat gttgattagg ttggtatcga ttttttgggt ttaagtaatt 2040
tttttatttt agtttgggaa ttataggggt gagttatttc gtttatgtta tttttggttg 2100
ttttttttat tagtagaaat ggggttttat tatgttggtt aggttggttt tgattttttg 2160
ggtttaagta atttttttgt ttcggtttcg ggattattgg tatatattat ttagttttta 2220
ttaatatttt taattttttt agtagagata gtttcgttat gttgtttagg ttggttttta 2280
ttttttgggt ttaagtgatt tttttgtttc ggtttcggga ttgtaggttt gtattatttt 2340
gttttttgtt atgtttttta ggttggtttt aattttttgg gtttatttaa tgatttgaat 2400
tcgggaaggg gaggttgtat tgagttgaga ttttattatt gtattttagt ttgggtgata 2460
gagaaaaatt gtcgaaaaga aaaggaaggg aagggagaag g 2501

<210> 130
<211> 2501
<212> DNA
<213> Artificial Sequence

<220>
<223> chemically treated genomic DNA (Homo sapiens)

<400> 130

ttgtattatt gtattttagt ttaggtgata gattgagatt ttgatttaaa aaaaaagaaa 60
aaaaaagaaa gagagatata agtcgagaga aggaaggtag ggacggtgtg gaggtgtcgg 120
gatgttatag agatatttgg atttattaga atagaagttt aagggagaga aacgtgtagg 180
atttaggtat gagttttatt gtggttagtt tttgttttta gttttgatag gatatagaag 240
agtagaatat ttagaagttg ttttgcgatt tttttttgta taaaaggaaa atgtggggta 300
ttttttgtag tttaagaagt agttaaagta ggaaaaggga tgtttatgtg tttttagatt 360
tgtttgtatt tagaattttt tgttatttag tttagttatg gttttatatt ttttttttat 420
atatatatag atgatttttt tcgaggattt tatttttttt tatttttttt tagagaagta 480
tttggtaata ttatcgaagg tttaggaaaa gataaaaaag gaattttgtt agtgatttag 540
ttagatacgt ttgttattta gttggagtcg ttttttgtgt gttggatttg ggaagtgggg 600
atgatatttc gttttggttg atgttatggt taattgatag aatatgaggg atttttttta 660
gttttatttt tgttatatag tagggtttta atgttgttgg ttggtttttt ttttattttt 720
tagaatggat tgagatttat taaatgtagt gtacggttat agatttgttt ttaaggatgt 780
tagttgatga tagagcgagg atgggaggtt tttaagggtt tagatttttt ttaagatttt 840
gttttttgtt tttagtattt ttattttttt ttttttagaa atcggttatt ttatattgtt 900
ttttgggtta ttattttgtt ttttttaggt tattttattt tttggatttt ttttggtatt 960
tgagaattag ttttaggttt ttttgtaaag gtatcgtttt cgtttatggt atcgggagta 1020
gtttgttttg taagagaaat agatcgagtt tttttagtta tagtagtttt ggttttgtgg 1080
agggagaaat tagtgaaggt cggttatttt tagttatttg gaattaggtg ttttattttt 1140
ttatttgggg tttatttgtt tttgagtaat gatatggggc gaagttagat gaaaataggg 1200
aattaggggt ttttgggaga agtattgatt tgggatgata ggttttttgt gggtaaagta 1260
gttttgagtt tttgggaggg ggttggttaa tgttgttagt agtttttttg gggttaggtt 1320
tattttgaaa gatgtataga tttttgttgg gaaggtagag atgtgattgt gtaattttat 1380
ttagtggggg tatgttgata tttttattta ataaataaag gaagggaaat gagttttaga 1440

gataatatgg tttttttggt gatggatttg attaggtaga gggatggggg gttttgtttt 1500
gttgaagaga aatgagtatg gttaatatga acggatttag aggttattat tgggtgattt 1560
gtaaattatt agaagaagag agttagaatt tttgagatta taagagttcg ttattattta 1620
aagagaatgt agggagtttt aaggtatagt aattagttag gcgtagtggt ttatgtttgt 1680
aattttagta ttttgggagg ataaggaggg cgggtcgttt gagtttatga gtttgagatt 1740
agtttggtta atatatcgaa attattttta ttaaaataat aataataata aaatatatat 1800
atatatatat atatatatat atatattaat agaagcggag tggtgtgcgt ttgtagtttt 1860
aaggtttacg tgagaggatt atttgagttt aggaggaaag atttgagtga gattttttgt 1920
ttgtttgttt gtttgtttgt tttgagttat ggtttttttt tgttgtttag gttgggatgt 1980
agtggtgtga ttatggttta ttgtaatttt cgtttcgtgg gtttaagtga tttattagtt 2040
gtggttttta aaattggagt tataagcgtg agttatcgta ttcggtttaa atttgttaag 2100
ttattagagt ttttaggaaa aattttatat ttgaaaaagt tagaaattga taggaaggat 2160
ttgagatggc gatttgtttt atatatattt tttattaaaa ttagataata aatgtatcgc 2220
ggaggagggg aggggtagga agaatggaaa aagaaaatta gcgtatgttt attttgattt 2280
tggaagaatc gaaagagaaa attagatcgt gcgtattttg agtatggaag aatcgaaaga 2340
gagagaaagt tagagtatgt atattttgaa tttaaagtag ttaatttcgg gggatgtttt 2400
aggcgggaaa attagggttt ttatttttag ttagaaagtt ttgtttttgg aggttggatt 2460
gatagacgtt atattagttt tgtttatttc gtttattgtt t 2501

<210> 131
<211> 2501
<212> DNA
<213> Artificial Sequence

<220>
<223> chemically treated genomic DNA (Homo sapiens)

<400> 131

gaatagtagg cgggataagt aaagttgatg tggcgtttat tagtttagtt tttagggata 60
gaatttttta attgggggtg gagattttga ttttttcgtt taaagtattt ttcgggattg 120
gttattttaa gtttagagta tgtatgtttt gatttttttt ttttttcgat ttttttatat 180
ttagagtacg tacggtttga tttttttttt cgattttttt aaaattagag taagtatgcg 240
ttgatttttt ttttttattt tttttatttt tttttttttt cgcggtgtat ttgttattta 300
gttttaataa ggagtgtatg tgaggtaggt cgttatttta aatttttttt gttagttttt 360
aattttttta ggtatgggat tttttttggg aattttagta atttaataaa tttgggtcgg 420
gtacggtggt ttacgtttgt aattttagtt ttggaggtta tagttggtgg attatttgaa 480
tttacgaggc ggaggttgta gtgagttatg attatattat tgtattttag tttgggtaat 540
agagagagat tatgatttaa aataaataaa taaataaata aataaaaaat tttatttaaa 600
tttttttttt tgggtttaag tgattttttt acgtaggttt tgggattata ggcgtatatt 660
atttcgtttt tgttaatgtg tgtgtgtgtg tgtgtgtgtg tgtgtgtgtt ttgttgttgt 720
tgttgtttta gtagagatgg tttcggtatg ttggttaggt tggttttaag tttatggatt 780
taagcgattc gttttttttg tttttttaaa gtgttgggat tataggtatg agttattgcg 840
tttggttgat tgttgtattt tgaaattttt tatatttttt ttaagtgatg gcgggttttt 900
gtagttttag aaattttagt tttttttttt taataattta taaattattt agtaatagtt 960
tttaaattcg tttatattag ttatgtttat ttttttttaa tagaatagaa ttttttattt 1020
ttttgtttga ttagatttat tattagagag attatgttat ttttgggatt tatttttttt 1080
tttttattta ttgagtgggg gtgttagtat gtttttattg aataaaatta tatagttata 1140
tttttgtttt tttaataagg gtttgtatat tttttagggt aagtttggtt ttagggaaat 1200
tattaataat attagttaat ttttttttaa agatttaagg ttgttttgtt tataggaagt 1260
ttgttatttt aaattaatat tttttttaga gatttttggt tttttatttt tatttgattt 1320
cgttttatgt tattatttag ggatagataa gttttagatg gagaaatgaa gtatttgatt 1380
ttaggtgatt gagggtggtc ggtttttatt gatttttttt tttataagat taaagttgtt 1440
gtggttggaa agattcggtt tgtttttttt gtaggataga ttgttttcgg tgttatgaac 1500
ggagacgatg tttttgtaag gagatttagg gttggttttt aaatattaga gaagatttaa 1560
aaggtgaggt gatttggagg gggtaaagta gtggtttagg ggatagtgtg gggtgatcgg 1620
tttttgagga ggggaggata gagatattgg agataaggag tagggttttg ggggagattt 1680
ggatttttga gagtttttta ttttcgtttt attattaatt agtatttttg gagataagtt 1740
tgtgatcgtg tattatattt ggtgaatttt agtttatttt ggaaagtggg aagagagtta 1800
gttagtagta ttaaagtttt attgtgtggt aggggtgaag ttaggaaagg ttttttatgt 1860
tttgttagtt agttatggta ttaattagga cggaatatta tttttatttt ttagatttag 1920
tatataggag gcggttttag ttgaatggta gacgtgttta gttgagttat tgataaaatt 1980
ttttttttat ttttttttag gttttcgatg atattgttaa atattttttt aagaaagagt 2040
gggaaaagat gaaattttcg gagaaaatta tttatgtgta tatgaagaga aagtatgagg 2100
ttatgattaa attaggtaat agaaagtttt aggtatagat aagtttgggg atatatgagt 2160
attttttttt ttgttttggt tattttttag gttgtagaaa gtattttata tttttttttt 2220
gtgtagggaa aaatcgtaag gtagtttttg ggtgttttgt ttttttgtat tttgttaggg 2280
ttgagggtag ggattggtta tagtggagtt tatatttgga ttttgtacgt tttttttttt 2340
taggtttttg ttttgatgag tttaagtgtt tttgtggtat ttcggtattt ttatatcgtt 2400
tttatttttt ttttttcggt ttgtattttt tttttttttt tttttttttt ttttgagtta 2460
gagttttagt ttgttattta ggttagagtg tagtagtgta a 2501

<210> 132
<211> 2501
<212> DNA
<213> Artificial Sequence

<220>
<223> chemically treated genomic DNA (Homo sapiens)

<400> 132

gaaaataaag ttaatggtat atttaaaatg gagtttatta ttttattgtt taaaattaag 60
taatttagta ttaggatgat gataatagtt agttatttaa gtttgaaatt aggtagttat 120
tttagattgt tttttttttt ttatttttta ttattaaaaa gttataaaat tttattaatt 180
ttatttttta aatatttttt atatttgata tttttttttt attttattgt tcgtgttata 240
gtttaagttt ttatgtttta tttaggttat taaaattgtt tcgttatttt ggttttttaa 300
gtttaaatta ttttttatat tataagtttt tttttgaatt cgatcgtgta attttttttg 360
tttaaaattt tttaagattt tcgtttataa agttaaaaat ttagatattt ttttaaggtt 420
tttttataat atttgttttt tttttttttt tttttttttt tttttttttt tgtgagacgg 480
agtttatttt tgtggtttag gttggaatgt aatggggtaa tttcggttta ttgcgacgtt 540
tgttttttcg ggggtttagg cgattttttt gttttagttt ttcgagttat tgggattata 600
ggcgtgtgtt acgtgtttgg ttaattttgg atgtttattg gagatagggt ttcgttatgt 660
tggttaggtt agttttaaat ttttgatttt aagtgattcg ttttttttgg ttttttaaag 720
tgttgggatt atagaagtga gttatcgcgt tcggttaata tttgtttttg aaatttttag 780
tgttattttt tgttattttt tttatttaat ttttatatag aacgtttttt ggatttagtt 840
gtaataattt tttttataat tttaatgttt ttgttcgttt tgcgttagtt agtaatattt 900
tttttttatt tttttttttt tttttttttt tttttttttc gttttttttt tttttttttt 960
tttttttatt ttttttcgtt tttttttttt gttttttttt ttttttttgt tttttttttt 1020
tttttttttg agatagaatt tggttttgtt gtttagattg gagttagtgg taggattatt 1080
agtttattgt agtttttgtt tttaagattt aagtgatttt ttcgttttag ttttttaagt 1140
agttgaggat ataggtgcgt attattatat tcggttattt taggagaggt agggttttat 1200
tacgttgttt agattggttt tgaatttttt tttgggttta agtagttttt tcgttttggt 1260
tttttaaagt gttgggatta taggcgtgag ttatcggttc gtttagttaa ttttttattt 1320
atttattttt atgtgaaata gtaatagaat tatattatat ttgtggagtt ttttttgatt 1380
ttttaagtta ttttaagagt tattttgttt acgtttttat tataggaatt attttaaatg 1440
attataggat attttaattg tttgtttatt tgtttttttt ttaagattgt gattttttgg 1500
agggtagaaa tggatttttt atttttagtt ttagtttgtt atagagtttg atagtttttt 1560
tagtattttt tgataatagt agaagtttaa agaattttta ttttaaggaa atatagaatt 1620
cgggaaataa ttgtttttag cgattaggga aatttcgcga gatttaggtt ttgggagttt 1680
ggtaagttta tatagcgacg gttatttagt cgttgaattg ttagtcgttc ggggtttacg 1740
cgtttcgggt cgtttaggtt gagcgatttt tcgttttttt tgaggttttg aggcgggagt 1800
tattggtttt ttttgttgtt tttatagatc gtatgtagta gttcgcgtgg gtatagaatt 1860
tacggttttt cgttagtttt ttaaaggtga gggtaggtgt ttcgagttat ttttttgggg 1920
attgagttta gagcggggcg atgttgtgtt attgtatttt ttcgtcgtag tttttcgttg 1980
tttttttttg ggtagtggtt taggaattta agatagtttt ttttggcgat gtgatggaat 2040
ttaatgggat aggagaaggg aacgggtttt ttttttaggt tagcgtggta gcgggcggta 2100
gggcgaaagg gagaaggaaa cgaggtttta tttcgttgtt tatttcgcgg taagcgacgt 2160
tgtagggttt tattgtagcg agagtttcgt ggattttttt ttttttagtt atttagtttg 2220
taaatattat tttaaagaat atatagtgta tttatgatat tcggtgaaaa aaaatttttt 2280
tttttttttc gttttttcgg ggtagtagat atttataatc gtaatagaga aaatggaaaa 2340
gtaaaagttt tttgtattgt tcgtattatc gagattaagt agtagttagg tgtttgcggt 2400
gaaattttag attttgggag gcgattttat tttttttaag gtaaattttt atgtgatttt 2460
taattggatt ttttttaatt ttagggaaat gaagaaaagt g 2501

<210> 133
<211> 2501
<212> DNA
<213> Artificial Sequence

<220>
<223> chemically treated genomic DNA (Homo sapiens)

<400> 133

tatttttttt tattttttta aagttaagaa gagtttaatt aagagttata tggaaattta 60
ttttgaagaa agtggaatcg ttttttaggg tttgaggttt tatcgtagat atttgattgt 120
tgtttgattt cggtggtacg aataatgtaa agggtttttg tttttttatt ttttttgtta 180
cggttgtaaa tatttattgt ttcggggggg cgggagggga aggaaagttt ttttttatcg 240
agtgttatga atatattatg tattttttaa agtgatgttt ataaattaaa tggttaaaaa 300

aaaaggaatt tacggggttt tcgttatagt ggagttttat aacgtcgttt atcgcggagt 360
gggtaacgga ataaattttc gttttttttt ttttttcgtt ttatcgttcg ttgttacgtt 420
ggtttgaaaa gaaagttcgt tttttttttt tgttttatta aattttatta tatcgttagg 480
aggaattgtt ttaagttttt ggattattat ttaaaagaaa atagcggagg gttgcggcgg 540
ggaggtgtag tagtataata tcgtttcgtt ttgggtttag tttttaggaa aataattcgg 600

ggtatttgtt tttatttttg aagaattagc gggaaatcgt gggttttgtg tttacgcgaa 660
ttgttatata cggtttatga gggtaataga aagaattaat ggttttcgtt ttagaatttt 720
agaaaaggcg ggaaatcgtt tagtttgagc ggttcggagc gcgtgggttt cgggcggttg 780
gtagtttaac ggttaaataa tcgtcgttgt gtgggtttgt taggttttta ggatttgaat 840
ttcgcgagat ttttttaatc gttaggaata attgtttttc ggattttgta tttttttgga 900
gtggaagttt tttaggtttt tgttattgtt aaagaatgtt agggaaattg ttaggtttta 960
tgatagattg aagttgagag tgaaaaattt atttttgttt tttaagaggt tataatttta 1020
ggaaggaaat agatgggtaa ataattaaaa tgttttataa ttatttaggg taatttttat 1080
gataaggacg taggtaaagt ggtttttagg gtggtttggg gagttaggag aagttttata 1140
gatgtggtat agttttgtta ttgttttata taagaatgag tgaatggaga gttggttagg 1200
cgggtcggtg gtttacgttt gtaattttaa tattttggga ggttaaggcg ggaggattgt 1260
ttgagtttag gaaggagttt aagattagtt tgggtaacgt agtgagattt tgtttttttt 1320
aaaatagtcg ggtgtggtgg tgcgtatttg tgtttttagt tatttaaaag gttgaggcgg 1380
gaagattatt tgagttttgg agatagaggt tgtagtgagt taataatttt gttattggtt 1440
ttagtttggg taatagagtt agattttgtt ttaaagagag agagagagga gatagaggag 1500
agaaaggagg tagagggagg aaacggagga gagtagggaa agaggggaga gaggggagag 1560
cgggggaagg gggagaggga gagggagagg gaatggggaa agagtattgt tggttgacgt 1620
aaggcgagta aaggtattgg agttgtgaga gagattatta taattaagtt taagaagcgt 1680
tttgtgtgag gattaagtgg ggaaaatggt aagagatgat attagagatt ttaagagtaa 1740
gtattagtcg agcgcggtgg tttatttttg taattttagt attttgggag gttaagaagg 1800
gcggattatt tgaggttaag agtttgagat tagtttgatt aatatgacga aattttgttt 1860
ttagtaaata tttaaaatta gttaggtacg tggtatacgt ttgtagtttt agtgattcgg 1920
gaggttgagg taggagaatc gtttgaattt tcggggaggt agacgtcgta gtgagtcgag 1980
attgttttat tgtattttag tttgggttat agagtgagat ttcgttttat aaaaaaaaaa 2040
aaaaaaaaaa agagagaaag agaaagtaag tattatagaa aggttttgga aaagtgtttg 2100
gatttttgat tttgtggacg gagattttga agaattttga gtagaaagga ttatacgatc 2160
gagtttagaa gaaaatttgt agtatgaaga atggtttagg tttaagagat tagagtagcg 2220
agatagtttt aatagtttgg gtgagatatg aggatttgag ttatgatacg gatagtggag 2280
tgaagaggag gtgttaaatg taaaagatat ttaagaagta aaattagtag aattttataa 2340
ttttttagta gtaagaagtg aaagaaaggg agtagtttaa gatgattatt tggttttagg 2400
tttgagtaat tagttattat tattatttta gtgttaagtt atttaatttt gagtaataaa 2460
ataatgagtt ttattttgaa tatattattg attttgtttt t 2501

<210> 134
<211> 2501
<212> DNA
<213> Artificial Sequence

<220>
<223> chemically treated genomic DNA (Homo sapiens)

<400> 134
   ttcgtagtgc gtcggttatc gtgttgggta ggtagttgcg tacgttggtg gtgaaggttt 60
   cgggggggtt ttcgcgggtt tcgtttagta gcgcgaagtc gaaggttagt acgtttttcg 120
   cgtcgcgttc gtatagtttt tgtagtattc gggttattag tttttttagg taggatattt 180
   gcgggggaag cgttttgagt cgtttgcgtt gtttttcgta tgtcgttggt ttttttcggt 240
   cgtttttaat tttagtcgga cgtcgatttc ggggaggttt atttggcgga aggagggggc 300
   ggcggggggc ggtcgtgcgt tttagggtac gtatattagg tattgggtta ttagcgcgcg 360
   gaaagtcgtc gggttttcgc gttgtattag tcgttagttt tggggtttta ggcgtcgtac 420
   gaacgtggtt agcggtagta tttcgcggta gtggttgcgt agtagggagc gtacggttcg 480
   gtagcgggga gcgcgcggta tcgcgggggt ggtcggggtt agggtttttt acgtgcgtag 540
   taggacgtag cgttgtttga aattcgcgtc gcgaggagag ggcggggtcg cggaaaggaa 600
   ggggaggggt tgggagggtt cggagggggt tgggtcgggg attcgggagg ggtcgggacg 660
   gggcggggtt cgcgcggagg aggcggagtt ggaaggtgaa ggggtaggac gggtgttcgg 720
   gtttttagtt ttttcgttac gtgggaagcg cggttttggg cgtttgtgtt cgcgaattta 780
   ttgggagttc ggtttggttt cgatagcgta gttgtttcgg gcggattcgg gggtttgggt 840
   cgcgtttttt cgttcgcgcg tcgttcgcgt ttttagggtg tagggacgtt agcgagggtt 900
   ttagcggaga gaggtcgaat cggtttaggt tgtggggtaa ttcgagggag gggttatgat 960
   gtggaggttt tgggaatagg tgcgtgcggc gatttttggg tcgttggttt gattcggaga 1020
   tttagggttg tttttaggtt cggacgcggg gcgtcgggtt tcgggtatta cgaatgtcgg 1080
   acgtgaaggg gaggacggag gcgcgtagac gcggttgggg acgaattcga ggacgtattg 1140
   ttttttggac gggtacgcgg gatttttcgg agtgtttttt tgtaatattt tttcgcgatt 1200
   tgggtttttt gatataggtt cgttattttt ttttgtaggt ttttaggcgg cgaggggttt 1260
   ttattatgag taaattattt taaatttgtt aattatttat cggggcggtt tcgtcgagaa 1320
   agggtgggaa atggagttag gcgtttttgt tggtcgcgta tcgggcgttt tatattagtt 1380
   ataacggttt tgattttggg tttcggtatt ttgtttggta gatgaggtta atatttggtt 1440
   atatttcgtt cgtatagggt ggagggtagt ttcggggttt aggtatttgg ttttaagttt 1500
   cggattgtag agttaggagg ttcgattttt agtttagtag tagaagttat acggttattg 1560
   gtttttttta gatttggggt ttcggtataa tcgtaggata gttgaggatt ttttaggaat 1620
   ttagatttcg ggttgtttaa gtttggattt aaggggcgag aaatttttgg gtttttcgag 1680
   gttttgtagg gatgttgtag ttgaggtcgg taaatattga aatgttaata aacgtaattt 1740
   taaatgtaat tttttttatt tttagaaatt gtcggaggaa attgttttat ttatggagtt 1800
   agtatttgaa taggtttcgt attttttttg ggttgttacg ttcgttggag gttagtttcg 1860
   ttttgtaaat atttaggatt acgggtcgtt tttttagaaa tttttttagt gatttttaag 1920
   tttttttaaa gggttgtgtt tgtgaattgt ttttgttatt agggtaaagg ggcggtttgg 1980
   aaaatttgtt ttaataaaag ttaagttgta gtttatattg gttttttgta gagaagttaa 2040
   tatagaaaat ataattttaa aagagggaag agaagaaatg gaagtagaag attatgttgg 2100
   agtaattaat attatgtgta tggcgaggaa acgtttttcg gtatttaatg aagatcgttg 2160
   atatttagaa gatattttag tattatgggt gtagttagtg tgtttttgaa aagttgatga 2220
   tgttttagtt attatagtgt aaaatattaa gagtgtttta ataataataa aaaaatttta 2280
   ttattggttt aaaatattat aatatttgag tggggtgagt tttttatttt agatttagat 2340
   gtttttaaat agagtaaatt ttgagttggg tatggtggtt tatatttgta attttagtat 2400
   tttaggaggt agaggtaggt ggattatttg aggttagaag ttcgagatta gtttggttaa 2460
   tatggtgaat tttattttta ttttcgtttt tattaaaaat a 2501

<210> 135
<211> 2501
<212> DNA
<213> Artificial Sequence

<220>
<223> chemically treated genomic DNA (Homo sapiens)

<400> 135

tatttttagt agagacgggg gtgggggtgg ggtttattat gttggttagg ttggtttcga 60
atttttgatt ttagatgatt tatttgtttt tgttttttaa agtgttggga ttataggtgt 120
gagttattat gtttagttta gaatttattt tgtttagaaa tatttgggtt tgaggtagga 180
agtttatttt atttaagtgt tgtggtgttt taagttaatg atagaatttt tttattgttg 240
ttagaatatt tttgatgttt tatattgtga tgattaagat attattagtt ttttaaagat 300
atattaattg tatttataat attggggtgt tttttgggta ttagcgattt ttattgaatg 360
tcgggaggcg ttttttcgtt atgtatatgg tgttaattat tttagtataa ttttttgttt 420
ttattttttt tttttttttt tttaaaattg tgttttttat gttggttttt ttgtagagaa 480
ttagtgtaag ttataattta atttttgttg gaataaattt tttaaatcgt ttttttgttt 540
tagtggtaga gataatttat aaatatagtt ttttaaaaag gtttagggat tattaagggg 600
atttttagaa gagcgattcg taattttaag tatttataag acgaggttaa tttttagcga 660
gcgtgatagt ttagggaggg tgcgaggttt gtttaaatgt tagttttata aataaagtaa 720
tttttttcgg tagtttttga aagtaggaaa ggttatattt aaggttgcgt ttgttagtat 780
tttagtgttt gtcgatttta gttatagtat ttttgtaagg tttcgggaga tttagaagtt 840
tttcgttttt tagatttaaa tttgagtaat tcggagtttg gatttttggg aagtttttag 900
ttgttttgcg gttgtgtcgg ggttttaggt ttggagggga ttagtggtcg tgtggttttt 960
attgttgggt tggaagtcgg gttttttagt tttgtagttc gaggtttgga gttaggtgtt 1020
tggatttcga ggttgttttt tattttgtgc gggcgggatg tgattagatg ttggttttat 1080
ttgttagata gagtgtcggg gtttagggtt aaggtcgttg tggttggtgt gaggcgttcg 1140
gtgcgcggtt agtaggagcg tttggtttta ttttttattt tttttcgacg ggatcgtttc 1200
ggtgggtgat taatagattt ggggtggttt gtttatggtg gggatttttc gtcgtttgag 1260
aatttgtaaa gagaaatgac gggtttgtgt taaggagttt aagtcgcggg gaagtgttgt 1320
agggaggtat ttcgggaggt ttcgcgtgtt cgtttaggga gtaatgcgtt ttcgggttcg 1380
tttttagtcg cgtttacgcg ttttcgtttt tttttttacg ttcggtattc gtggtgttcg 1440
gagttcgacg tttcgcgttc ggatttggag gtagttttgg gttttcggat taggttagcg 1500
gttaaagggt cgtcgtacgt atttgttttt agggttttta tattatggtt tttttttcgg 1560
gttattttat agtttaggtc gattcgattt tttttcgttg gggttttcgt tggcgttttt 1620
gtattttggg agcgcgagcg gcgcgcgggc ggggaagcgc ggtttagatt ttcgggttcg 1680
ttcggagtag ttgcgttgtc ggggttaggt cgggttttta gtggattcgc gggtatagac 1740
gtttaggatc gcgtttttta cgtggcggag ggattgggga ttcgggtatt cgttttgttt 1800
ttttattttt tagtttcgtt tttttcgcgc ggatttcgtt tcgtttcgat ttttttcggg 1860
ttttcggttt agtttttttc gggttttttt agtttttttt tttttttttc gcggtttcgt 1920

ttttttttcg cggcgcgagt tttaggtagc gttgcgtttt gttgcgtacg tgggaagttt 1980
tggtttcggt tattttcgcg atgtcgcgcg tttttcgttg tcgagtcgtg cgttttttgt 2040
tgcgtagtta ttatcgcgag gtgttgtcgt tggttacgtt cgtgcggcgt ttggggtttt 2100
agggttggcg gttggtgtag cgcggggatt cggcggtttt tcgcgcgttg gtggtttagt 2160
gtttggtgtg cgtgttttgg gacgtacggt cgtttttcgt cgtttttttt tttcgttagg 2220
tgggtttttt cggggtcggc gttcggttgg ggttgagggc ggtcgggggg aattagcgat 2280
atgcggagag tagcgtaggc gatttagggc gtttttttcg taggtgtttt gtttgaagga 2340
gttggtggtt cgagtgttgt agaggttgtg cgagcgcggc gcgaagaacg tgttggtttt 2400
cggtttcgcg ttgttggacg gggttcgcgg gggttttttc gaggttttta ttattagcgt 2460
gcgtagttat ttgtttaata cggtgatcga cgtattgcgg g 2501

<210> 136
<211> 2501
<212> DNA
<213> Artificial Sequence

<220>
<223> chemically treated genomic DNA (Homo sapiens)

<400> 136

agaagaaggt atttaaaggt ttagggagat tgagatggtg gagtggatta gttattttag 60
atttatttat tttagttgag agggtttaga agatatattt ttgattattg ttttgtgaaa 120
taggtttgtg agggtagtat ttgtattttt gaagggtttt gtaattgttt ttttttgtat 180
tttagattta atagtgggaa ttgtagttat taaattataa aatttaaata tattgggaat 240
aattggattt taaggtggta ggggttaagt ggtggtattt aattattaaa ggtaaggtgg 300
gtatagttat tgtaatgggt agtagaggta aagtagtaat tagaatagtt tgattcgtgt 360
agagttttgg tattgggtaa ttaattaagg tgtttttaga aatgaaattg ataggaagtt 420
tattgtattt ttatttaatt tatataagta gaaaattttt agatcggatg gataatagat 480
taatttgaat tataaatata gagaattacg gttttttagt taatttttaa atttgagtta 540
gtttatagat ttagaatttt ttgaatgaag gggaggttgg gtttttttga ggaaggattt 600
tattatatta tcgataattt atgttgttaa tatttttttt attttttttt aaggagattt 660
atggtttttt attagggtaa ttgtgtattg gggaaaggga aatggttaga tattttgggg 720
attattggat attggttttg agttgatatt gattttaggg tatttaaaat gttattgtgg 780
ttttttagtt aaagtaggga tttatggagg ttaggtaatt aatagagttt tagtttaggt 840
ttgatttcgg tttagtgggt ttttggattc gttttgtggt tattttttta gtgttagaat 900
gtataattgg tatatatata tttagtagtt ggtagaattt ttatattggt tttttgattg 960
gtagggtgag ggttattatg gtgggaaagg ttaaatagaa gtttttagag ttgtttttat 1020
gtagaaaaat agtaaattaa aaataatatt gtatttcggg agggattata gagattagtg 1080
ttattattaa ggatttgaag atgtaggggt ggtgattttt attatatttt tgtttaattt 1140
ttttattcgg tttgtgtaga aggtagatgg atttaggaga atgatagtgg attattataa 1200
gtttaattaa gtggtgattt taattgtagt tgttgtatta gtttttttag ttttagatgg 1260
aaataaattt tatttgtttt aggtttttgg tataaaaggt gtaattttag ttattttata 1320
ggtttgggtg aggaaaatgt taagagttgt aaaagggttt ggaaaagtaa aaaaggtgat 1380
gtataaatgt aatgattatt attttgtgtt taagaataag ttttggaata taaaatagga 1440
aagtaagttt atagttttaa aatatatgta tataaaattt taaaataatg tagttttaat 1500
tttatagatg gtatttgggg attgtttatt gttttagagt tttaatgttt ttttttttac 1560
gtgggtgttg atttttaaaa aggtagtagt gtcgttttta atgtgggtgt tgatttttaa 1620
aaaggtagta gtatcgtttt taacgtagtg gttagtaatg ttaagtgtaa tatgtacgtt 1680
aaagagtttt aatttttatt gtaaataagt aagggaagga gattttaaag ttttggttgg 1740
aggaagtttt gtcgtagagt agtggtcgta tttttgtgtg aataaagggt tagatagttg 1800
ttattttgag gcgagggttt taggttagga gacgttgagt gtttgggagg ttttcgatga 1860
ggtggagggt tttgagttta cggagagttg cgaagagaga gaggatttat ttgagttagg 1920
attgtacggc gcgatttcgt ttaggtttcg tttttcggac ggtttggtgg tttcgtttcg 1980
ttttttcgtt tttttcgggc gttttaagtt gtcgttagaa tttatggtcg gcgtggaaat 2040
cgttgcgagg cggcgttacg acggaggttg aggtcgtcgg tagtatttat ggcgttttag 2100
agcggtagtt ttttggggtt tttggtttgg tttttgtttt tttagttatg gttcgaagag 2160
gttcgggtag gtagggtggg tgcgtaggga ggcgtagtat tgtttttttt tttcgcgttt 2220
tttttagggt taggcggtta ggatttcgga gcgagcggat gggagtcgtt atttgtaggg 2280
gttttaggat ttttagcggt tttttagttt agggggaacg tagtgcgttt cgtttcggtg 2340
ttattttttt taggtgagtt tgatttttat taggttttgg taattttata tatattatta 2400
ttaatatgga ttaaagggat agtagtatta aacgttaggt gacgtttttg ggttatgttt 2460
agtcgtagag aattggtttg gtttgagggg gaggggattt g 2501

<210> 137
<211> 2501
<212> DNA
<213> Artificial Sequence

<220>
<223> chemically treated genomic DNA (Homo sapiens)

<400> 137
   taggtttttt tttttttaaa ttaggttagt tttttgcggt tgggtatggt ttagaagcgt 60
   tatttgacgt ttaatgttat tgtttttttg gtttatgttg atgatagtgt gtgtgggatt 120
   gttagagttt ggtaagaatt agatttattt gagggaagta atatcgaagc ggggcgtatt 180
   gcgttttttt tggattgggg ggtcgttggg gattttggag tttttatagg tggcggtttt 240
   tattcgttcg tttcggggtt ttggtcgttt ggttttgagg ggagcgcgga ggggaagagt 300
   agtgttacgt ttttttgcgt atttattttg tttgttcggg ttttttcgag ttatggttga 360
   agaagtaaga attaggttag gagttttagg gagttgtcgt tttgggacgt tatggatatt 420
   gtcggcgatt ttagttttcg tcgtggcgtc gtttcgtagc ggtttttacg tcggttatgg 480
   gttttggcgg taatttggag cgttcgggag ggacgagggg gcggggcggg gttattaggt 540
   cgttcgaggg gcggggtttg ggcggggtcg cgtcgtgtag ttttgattta gatgagtttt 600
   tttttttttc gtagtttttc gtggatttag ggttttttat tttatcggga gttttttagg 660
   tatttagcgt tttttggttt agggttttcg ttttagagtg gtagttgttt agttttttgt 720
   ttatataaaa atgcgattat tattttacgg tagagttttt tttagttagg gttttagagt 780
   tttttttttt tatttgtttg taataaaaat taaaattttt taacgtgtat gttgtattta 840
   atattattaa ttattgcgtt agagacgata ttgttatttt tttgaaaatt aatatttata 900
   ttagagacga tattgttatt tttttggaaa ttaatattta cgtgagaaaa ggggtattga 960
   gattttaaag tagtagataa tttttagata ttatttgtag agttggaatt gtattatttt 1020
   aaagttttat atgtatatat tttaggtttg tagatttatt tttttatttt gtgttttaag 1080
   gtttattttt gagtataaaa tgataattat tatatttata tattattttt tttatttttt 1140
   taagtttttt tatagttttt ggtatttttt ttatttaggt ttgtgaggta attgggattg 1200
   tattttttat attagaaatt tgaggtagat gaaatttatt tttatttagg attagaaaaa 1260
   ttggtatagt agttgtaatt ggagttatta tttggttaaa tttatgataa tttattgtta 1320
   tttttttaga tttatttgtt ttttgtatag gtcgaatggg agagttgaat agggatgtgg 1380
   tgggaattat tatttttgta tttttaagtt tttgatggtg gtattgattt ttgtaatttt 1440
   tttcgggatg taatattatt tttgatttat tattttttta tatagaggta gttttaaggg 1500
   tttttatttg gtttttttta ttataatagt ttttatttta ttagttaggg agttaatgtg 1560
   ggagttttgt tagttgttaa gtatgtatat gttaattatg tattttggta ttggggaaat 1620
   gattatagga cgagtttagg gatttattgg atcgaggtta gatttgagtt aaaattttat 1680
   taattatttg atttttataa gttttatttt taattggaag attatagtga tattttgggt 1740
   attttggaat taatgttagt ttagaattag tgtttagtag tttttaaaat atttgattat 1800
   tttttttttt ttagtgtata gttattttgg taaaaggtta taggtttttt tggggaagga 1860
   tgggagaaat attaatagta taagttgtcg gtagtgtaat ggggtttttt tttaagggga 1920
   tttagttttt tttttattta agaggtttta ggtttgtaaa ttggtttaag tttggaaatt 1980
   gattgaggag tcgtgatttt ttatgtttat aatttaaatt agtttgttgt ttattcgatt 2040
   tagaagtttt ttgtttatat aaattaagta ggaatgtagt gggtttttta ttaattttat 2100
   ttttagaaat attttgatta attatttaat gttagagttt tatacgagtt agattatttt 2160
   gattgttgtt ttgtttttgt tgtttattat agtagttatg tttattttgt ttttgatggt 2220
   tgagtgttat tatttggttt ttgttatttt gggatttaat tatttttaat gtatttaaat 2280
   tttgtagttt agtgattgta gtttttattg ttagatttga aatatagaga agaataatta 2340
   taggattttt taaagatgta ggtattgttt ttataaattt attttataag gtagtgatta 2400
   agggtatatt ttttggattt ttttagttgg gatgagtaga tttaaagtga ttaatttatt 2460
   ttattatttt aattttttta agtttttgga tgtttttttt t 2501

<210> 138
<211> 2500
<212> DNA
<213> Artificial Sequence

<220>
<223> chemically treated genomic DNA (Homo sapiens)

<400> 138

tatcggcgtt tattgcgtcg cgtcgtttat aggtagggta tagtcgtcgt cgttatttag 60

ggaggggacg gcgggtagta gcgcgtttag gtttcgtacg tcggagttta tttgttgcgg 120
tttagattcg gacgtttcgc ggttttttcg gttttggaga cgtttagcgt tggtttcggc 180
ggcgtttaat ttggtttaga tgtcgttcgg gtttcggatt ttttgttgtt ttggttgttg 240
taggtattta ggttcggagc ggagcgtgtg ttgagacgtc ggtttcggga tatacgtgga 300
agtcgggttt tgtagtaaga ggaagtttag gatcgcggcg aggagacggc ggggttcggg 360
cgtttgggtt gtcgtttcgg ttttgggtgg gtgggtgggt gaatgagtag gtgggaggga 420
gggcgggaag tgggggagcg gataggcggt cgggttgcgg agagttttcg ggtgtgggcg 480
ttgttttgaa ttttttattt tagttgtttg gttgttttta gttttttaaa gtttaaataa 540
gaggggtcgg cggcgcgggg aagaggagga gttaggaggt tcggtcgttt tatttattta 600
gtagtttaag ttcggttagg tagcgcgcgt tgttttttgg gggcgttttc gtagttgggg 660
tgagggcggg ttcgagggag cgtttttttc ggagatattt ttttttttaa tttaatacgc 720
gtttttagtt ggtcggtgtg gtataatttt ttttttattt tttttttttg gttttcgtta 780
gtgtgggagt ttcgttttat tggagagttc gtttaggtaa gtagtgagtt cgggtagggt 840
cgaatgcggt gggagtagag atggggttgt taatttaaaa gagtggtttg gagggagggg 900
taggtaggtt ttaagagggc gggaatttta ggaaagcgta gaaagaattt agttaagttt 960
taaagaagcg tatatttttg ttttgtattt ttgagtttgg tttttgtttt tagaattgtc 1020
gggtagggga gtggttatga ttttggggtt attggggaaa gaggtggttt tttgtgggtt 1080
ttagaggtcg ggattgaaag cgaacgtttt ttagttgggt ggtcgggagt tgtattagtt 1140
ataagtcggg cgtatttcga tcggttagta gttgtttatt tggtgaggtt tgcgttaggc 1200
ggaaggcggt tgtcgtttat ttaaaattgg agttagtagt atttagtttt tttttttagt 1260
gaggaatatt taattttttt acggttaagt cgggaaaata gcggttagtt taggagggaa 1320
gaaaattttt gggtaaacgt ttaaagtttg cgtatatttc ggtcggagta ttttggcgtt 1380
tagtttgggg aggaggggtg aaggggaggg tttggatacg aagttcggta gggagcgggt 1440
aatttttgga gttatttcgt cgagtattgt ttgtggtttt ggtcgaggat attattttgg 1500
ttgtttagtg attgattagg aggcggaagt tagttaaggg gattaaggag tggagtaggg 1560
agaattttta tcggtttacg ggcgcgagtt agtgttcgaa tttttgggag ttcgaagagt 1620
ttcgttaaat ttgatttttt tcgtttagtt tttgtttggt agttttcgcg ttttagagta 1680
ggtgtttggg gcgggtaggg ggtgttttgg tttggttttt ggatttttta aaggagttag 1740
tatttattgg gaaataattt ttaggtaagg agaagaatcg ggggtcggag cggagcgtgt 1800
tttagaggtg cgtataaaat tttaggggcg ttaggaggta gtagcgacgg cgtagcgtgg 1860
gacggttagg cgcgacggga gggaagtaat cggttgtagt tcggtttttt gtatttggtt 1920
ggttcgggtt ggggttgtaa gttttttagt taaatttatg tgaagtaaag tagttgtagt 1980
ttgataaaga atggcgtttt aataaatgat atagtaattt tttacgtttt ggtttttagt 2040
ttgtattgta taaacgttgt cgttattaaa ttttggttgc ggattcgaag tgtgtgcggg 2100
tttaggattt attcgaaggg cgtatttttt gggaatgcgt atttcgtagg cgtaggagat 2160
tttgagggta aagatgcggt atttgagttt ttcgtttgtt ttttaggtta tttttgttgt 2220
ttgattttgc gtcgttttta tttagaagtt tttaaaatta ttattaagaa aagaaagatt 2280
ttataagata ttgtttttac gttttttgtt tagttttttt ttatacgatt tatttttaaa 2340
tttttcgcgg ggttcgcgcg tttggttttt tgagtggaga aaggattttc gagataaaat 2400
ttattgttag ttgttttaag aattttttag atttttgtat tttttttttt ttttttcgtt 2460
tattattttt atttggaagt cgtttttttt aattaggatt 2500

<210> 139
<211> 2500
<212> DNA
<213> Artificial Sequence

<220>
<223> chemically treated genomic DNA (Homo sapiens)

<400> 139

agttttagtt aagaaaaacg atttttaagt agaaataata ggcggggaga aggaagggag 60
atatagggat ttggggggtt tttagggtaa ttggtagtga attttgtttc gagagttttt 120
tttttattta aaaaattaaa cgcgcgagtt tcgcgaaagg tttagggata gatcgtgtgg 180
gagaggattg agtagagagc gtgggggtag tgttttgtag aatttttttt tttttaataa 240
taattttaaa agtttttgag tggagacgac gtaaagttaa gtagtaaagg tggtttggga 300
ggtaagcgga gggtttaagt gtcgtatttt tatttttagg gttttttgcg tttacgggat 360
gcgtattttt aagaagtgcg tttttcgagt aagttttggg ttcgtatata tttcgggttc 420
gtagttagaa tttaatggcg ataacgttta tgtaatgtaa gttaaaaatt aaagcgtaaa 480
aaattattat gttatttatt gaaacgttat tttttgttaa attgtaatta ttttgtttta 540
tataagtttg gttggaaagt ttgtagtttt agttcgggtt agttaggtat aggaggtcgg 600
attgtaatcg gttgtttttt tttcgtcgcg tttggtcgtt ttacgttgcg tcgtcgttgt 660
tgttttttgg cgtttttggg attttatacg tatttttgaa atacgtttcg tttcggtttt 720

cggttttttt ttttgtttag gggttgtttt ttaatagata ttgatttttt tagaagattt 780
aaaaattaaa ttaaaatatt ttttattcgt tttaaatatt tgttttgggg cgcgggggtt 840
gttaaataga gattagacga agggagttag atttagcgaa gtttttcgag tttttaaaga 900
ttcgaatatt aattcgcgtt cgtgggtcga tggaggtttt ttttatttta ttttttggtt 960
tttttaattg gttttcgttt tttggttaat tattgagtaa ttagaatggt attttcgatt 1020
agggttatag gtagtgttcg gcggagtggt tttaggagtt attcgttttt tgtcgggttt 1080
cgtatttaaa tttttttttt tatttttttt ttttaaattg ggcgttagga tgtttcggtc 1140
ggaatatacg taggttttgg gcgtttgttt aagggttttt ttttttttta aattagtcgt 1200
tgttttttcg gtttaatcgt agaagaatta gatatttttt attggaaagg gaaattaagt 1260
gttgttgatt ttaattttag gtaggcggta atcgtttttc gtttggcgta aattttatta 1320
agtaaataat tattagtcga tcgaaatacg ttcggtttat aattggtgta attttcggtt 1380
atttaattga gggacgttcg tttttagttt cgatttttgg aatttataaa gggttatttt 1440
tttttttagt gattttaaga ttatggttat ttttttattc gatagtttta gaagtaagag 1500
ttagatttaa gggtgtaaag taagggtata cgtttttttg aagtttgatt gagttttttt 1560
tgcgtttttt tgaagttttc gtttttttgg agtttatttg tttttttttt taaattattt 1620
ttttagatta ataattttat ttttattttt atcgtattcg attttgttcg gatttattgt 1680
ttatttgaac ggatttttta gtgagacgag gtttttatat tggcgaaggt taagaagggg 1740
aggtgggggg agggttgtgt tatatcggtt agttgagagc gcgtgttggg ttgaagagga 1800
gggtgttttc gagagggacg ttttttcgga ttcgttttta ttttagttgc gagggcgttt 1860
ttaaggagta gcgcgcgttg tttggtcggg tttgggttgt tgagtgaatg gagcggtcga 1920
gttttttggt tttttttttt tttcgcgtcg tcggtttttt ttatttgagt tttgggaagt 1980
tgagggtagt taggtagttg gggtaaggag tttaaggtag cgtttatatt cggggttttt 2040
tcgtaattcg atcgtttgtt cgttttttta tttttcgttt ttttttttat ttatttattt 2100
atttatttat ttatttagag tcgggacggt agtttaggcg ttcgggtttc gtcgtttttt 2160
cgtcgcgatt ttggattttt ttttgttgta ggattcggtt tttacgtgtg tttcggagtc 2220
ggcgttttag tatacgtttc gtttcgggtt tgggtgttta tagtagttag agtagtaggg 2280
agttcgggat tcgggcggta tttgggttaa gttaggcgtc gtcgaggtta gcgttgaacg 2340
tttttagggt cggaggagtc gcggggcgtt cgggtttgag tcgtagtaaa tgggtttcga 2400
cgtgcgggat ttgaacgcgt tgttgttcgt cgtttttttt ttgggtggcg gcggcggttg 2460
tgttttgttt gtgagcggcg cggcgtagtg ggcgtcggtg 2500

<210> 140
<211> 2500
<212> DNA
<213> Artificial Sequence

<220>
<223> chemically treated genomic DNA (Homo sapiens)

<400> 140
   tggtagaatt tttttttgtt aattttttga ttattggtat aaaaatatag tatttaaata 60
   tgtgaagtaa aagataattt aggggttagt tgattatggt ttgagattta aatatagttt 120
   atggtttgtt ttgtatagtt attgagataa ggattttttt ttatttaaaa gtattattgt 180
   taaaaaatat aatattacgt tatagaaatt atatgattta taaagattga aataattacg 240
   attagatttg ttaaagtaaa atttggtgat gtcgtattaa ttaaatattt gttattttgt 300
   tttgtattat tgtatatatt tttttattta ttaattaatt tatttatata cggattgaaa 360
   atatggatgt tattttattt ttttaagaaa gcgtttagtt ttatgatatt atgattttag 420
   tttgggaaat ttgttttagg gattaggtta atatataaat atatatttga ggtaggagat 480
   tggtaggatt tgttttttgg ttataattga gtttagagtt tatagttaga atttatggtt 540
   atttttgaaa tatatattgt ttgaatgatt tataggtata tttagggaaa ttagttatta 600
   gtttagagat ttaaatgtga agtaatataa tttaaaaaga aaagagtaaa taggtgtgaa 660
   tgtatttatt ttatagaaga atttatttaa ttacgtttat tggaagtatg gtagaaaatt 720
   tagaattaga gaataaagtg attaataaaa gtatttataa aattggttaa ggtgggagga 780
   ttatttgagt ttaggaaatt gagattagta tgggtaatat agtgagatta ttatttaaaa 840
   aataaataaa taataaataa attaattatt taggtgtggt gtttgtattt gtagtagtag 900
   ggattgggga ggttatatcg ggaggattgt ttgagtttag gagatggagg ttttagtgag 960
   gtatggttat gttattgttt tttagtttga gtaatagagt gatattttgt ttggaaaaaa 1020
   aaataaataa aaacggtttt ataaataaag atatgaaaat aatttatttt ttttataaaa 1080
   ggtttttttg agatatattt tttagtaagg gattgtttgt ttttaacgtt tttgaatgta 1140
   tgagattgga taaagagaga cgaaagtttt taagtttttt tttttattat aaacgttatg 1200
   gggatgcggg tttgggaata gcggaaaatt ttattttgtt ttgaaaagtt tttggtttaa 1260
   tgtgtatgtt tttttttatg aaagtttttt gttgtttatg cgttttgttt ttttaatttt 1320
   gtttttttat aattgtgaaa ggagacgtcg tgattttttt ttttcgtgtt gattatgatt 1380
   tagttagtag gtttgtagat tagttttttt agaaagtgaa gtgtgagtga gtgtgagggg 1440
   gagcgtgggg ggttttttgt gggttgtgtt atttggttta tggtttttga ggtcgttatt 1500
   tttttattaa aatttagcga tattgttatg gttttgttgt tgtggggttt cgggtatggg 1560
   acgaaggagg gttatagcgg ggaggagggt taggtgagta tggagtgagt tttttttttt 1620
   tttttacgga gttttgtttt gttattaggt tggagtgtag tggcgtaatt tcggtttatt 1680
   gtaattttcg tttttcgggt ttaagtgatt tttttgtttt agtttttcga gtagttggga 1740
   gtataggcgc gtataattat atttagttaa tttttgtatt ttttagtaga gacgggtttt 1800
   aattatgttg gcgaggatgg tttcgatttt ttggttatat gatttattcg ttttcgtttt 1860
   ttaaagtgtt gggattatag gcgtgagtta tcgcgttcgg ttatggagtg agttttgaag 1920
   gttgatttta tttgaggtat ttgagttttg gtggggggga atttatatat agagaggagg 1980
   gattttaaag tcgttaatgg ggatttggga aggagtatag gatagggtaa ggcgggataa 2040
   ggaggggtat tatagttttt aaggtacgag ggaattttat tgcgtatgtt tttttggtgt 2100
   ttattttagt gcgtatgttt attgggcgtt tttttatcgg ttttttcgtt agtgtgggga 2160
   acgcggcgga gttgtgagtc ggcgattcgg gtttttgagg tttggatttt tttttcgtta 2220
   ttgagatacg gcgggtaggt ttataggtag atttaattgg gagttgaagt gtgagtgaga 2280
   gtgaagagga attagtaggt tttcggaggg ttgtgtggtt agtgatttag agtgagaagg 2340
   ttttcgaagt cgtcgttttt tttatgcggt gttacgttta tggatttttt tgtttcgtta 2400
   cggttataat tagggaggaa ggagggtcga ggagtggagg ggtttaggcg aagttggggt 2460
   gttgttgggg gtattcgagt tttagaagta tttggaattt 2500

<210> 141
<211> 2500
<212> DNA
<213> Artificial Sequence

<220>
<223> chemically treated genomic DNA (Homo sapiens)

<400> 141
   gggttttagg tgtttttggg attcggatat ttttaatagt atttagtttt cgtttgagtt 60
   tttttatttt tcggtttttt ttttttttta gttatggtcg tgacgagata agaaggttta 120
   tgggcgtggt atcgtatgag agggacgacg atttcgaggg tttttttatt ttgagttatt 180
   gattatataa tttttcggaa gtttgttggt ttttttttat ttttatttat attttaattt 240
   ttagttggat ttgtttgtgg atttattcgt cgtgttttag tagcggagaa agaatttaga 300
   ttttagggat tcgagtcgtc ggtttatagt ttcgtcgcgt tttttatatt ggcgaagggg 360
   tcgatgggaa gacgtttagt gaatatgcgt attgaggtgg gtattaaagg agtatgcgta 420
   gtgaggtttt ttcgtgtttt aagggttgtg gtgttttttt ttatttcgtt ttgttttgtt 480
   ttatgttttt ttttaggttt ttattaacga ttttggaatt tttttttttt gtgtgtgggt 540
   ttttttttat taggatttaa atattttaaa taaagttagt ttttaaaatt tattttatga 600
   tcgggcgcgg tggtttacgt ttgtaatttt agtatttggg gggcggaggc cgggtggatt 660
   atgtggttaa gagatcgaga ttattttcgt taatatggtt aaaattcgtt tttattgaaa 720
   aatataaaaa ttagttgggt gtggttgtgc gcgtttgtat ttttagttat tcgggaggtt 780
   gaggtaggag aattatttga attcgggagg cggaggttat agtgagtcga gattgcgtta 840
   ttgtatttta gtttggtgat agagtaagat ttcgtgaaaa aaaaaaaaaa atttatttta 900
   tgtttatttg attttttttt tcgttatggt tttttttcgt tttatgttcg ggattttata 960
   atagtaaggt tatggtagta tcgttgagtt ttagtagaaa gatgacgatt ttagaggtta 1020
   tggattaagt ggtataattt ataggaagtt ttttacgttt ttttttatat ttatttatat 1080
   tttatttttt gggaggattg atttgtagat ttattggtta agttatagtt agtacgaaag 1140
   aaggaagtta cgacgttttt ttttatagtt gtggagggat agaagttaga gagtaaggcg 1200
   tataggtagt aaggaatttt tatagaaagg gatatgtata ttgagttagg gattttttag 1260
   ggtagggtag ggtttttcgt tgtttttaga ttcgtatttt tatggcgttt gtagtagaaa 1320
   aagggatttg agagttttcg tttttttttg tttaatttta tgtatttaga aacgttggga 1380
   atagatagtt ttttattgga ggatgtgttt taggaagatt ttttgtggaa gaaataaatt 1440
   atttttatat ttttatttat aaagtcgttt ttatttattt ttttttttag atagagtgtt 1500
   attttgttgt ttaggttgga gggtagtggt atgattatat tttattgaag tttttatttt 1560
   ttgggtttaa ataatttttt cgatgtagtt tttttagttt ttgttattat aggtgtagat 1620
   attatatttg ggtaattggt ttatttatta tttatttatt ttttagatag tggttttatt 1680
   gtgttgttta tgttggtttt aattttttgg gtttaagtga tttttttatt ttggttagtt 1740
   ttataaatgt ttttattaat tattttgttt tttggttttg ggttttttgt tatgttttta 1800
   ataaacgtaa ttgaataaat ttttttgtag aataaatata tttatatttg tttatttttt 1860
   tttttttagg ttgtgttatt ttatatttaa atttttggat tggtaattga tttttttgaa 1920
   tatatttgta ggttatttag atagtatatg ttttagaaat agttatagat tttgattgta 1980
   ggttttaggt ttagttatga ttagaaaata agttttgtta gtttttattt ttaaatgtat 2040
   gtttgtgtat tgatttgatt tttgggataa gttttttaga ttgaaattat ggtattataa 2100
   agttggacgt ttttttgagg ggatagagta atatttatat ttttaattcg tgtgtaaata 2160
   aattaattaa tggatgaaaa gatgtatgta ataatgtaaa gtagaataat aggtatttga 2220
   ttaatacggt attattaaat tttgttttaa tagatttgat cgtaattatt ttagtttttg 2280
   tgggttatgt agtttttgtg gcgtagtgtt gtgtttttta gtaataatat ttttaaatgg 2340
   aaaaaaattt ttattttaat ggttatgtaa aataagttat gggttgtatt tagattttaa 2400
   gttatagtta gttagttttt ggattatttt ttattttata tatttgaatg ttgtattttt 2460
   gtattagtag ttaaaagatt aataaaggag agttttatta 2500

<210> 142
<211> 2501
<212> DNA
<213> Artificial Sequence

<220>
<223> chemically treated genomic DNA (Homo sapiens)

<400> 142

gattaatatg taataggagg tgagaattaa tattatttta atgtagataa agataatatt 60
ggttaagtgt taatattggt ttttaaaaat gggtaattta ttttattttt attttgttta 120
tgtatttgag gtattatgtg tatgttggta gtatttttat tatttttaaa gtaatgatgt 180
tgtttaggaa tattaaatat tgttttttttg aaatatttat gaatttaaaa atattttatt 240
ttagatgtag taatatgtgt tatagagtaa tattttaatt taataaattt ggttttaaaa 300
taaattattt tgggttgggt gtagtggttt atgtttgtaa ttttagtatt ttgggaggtt 360
gaggagggtg gattatttga ggttaggagt tttagattag tttggttaat gtagtgaaat 420
tttgtttgta ttaaaaatat aaaaattagt tgggtgtggt ggtaggtgtt tgtaatttta 480
gttatttagg aagttgaggt aggagaatta tttgaagtta gtaggtggaa gttgtagtga 540
gtggaaattg tgttattgta ttttagtttg ggtggtagag tgagatttta ttttaaaaaa 600
taaaaataga aaaataaaat aaaattattt tggttgggta tagtggttta aggttgtaat 660
tttagtattt tgggaggttg aggtgtgtgg attatttgag gttaggtttt tgagattagt 720
tgggttaata tgttgaaatt ttgtttttat aaaaaaatat aaaaattagt tgtatggtgt 780
tatgtatttg tagttgtagt tatttgggag gttgaggtta gagaattgtt tgaatttggg 840
aggtaaatat tatagtgggt tgagattgtg ttattgtatt ttagtttggg taatagaatg 900
agattttatt ttagagagag agagagatat tataaattaa ttattttttt taaattagtt 960
agaatatttt tttattttgt tttttttttt ttattgaatt aataattgta gaatatttat 1020
atattgatta tatttttaat attttttgtt attttttttg attaggaatt tatagatttt 1080
ttttgttaag attttaattt gtgtttttgt gaaagatagg aagattgaga tgttgaaata 1140
atttaaaata gttaaggttg gttgggtgtg gtggtttatg tttgtaattt tagtattttg 1200
ggaggttgag gtgggtggat tatgaggtta ggagatttag attatggtga aattttattt 1260
ttattaaaaa tataaaaagt tagttgggtg tagtggaggg tgtttgtagt tttagttatt 1320
tgggaggtgg aggtaggaga atggtgtgaa tttgggaggt ggagtttgta gtgagttgag 1380
atggagttat tgtattttag tttgggtgat agagtaagat tttgttttaa aaaaaaaaaa 1440
aaaaaaaaaa aaaaaaaaaa aaaaaaaaaa aaaaaattta aggttaaggt atatatagtg 1500
aaatttttat tatttgtata taaaaaattt gtatttgttt aaaaattgat ttttttgtta 1560
tttggttttt tttttttttt attttgtttt aattatgatg tgttttaaaa tatagtatgt 1620
gttaattttt ttttgagatg gagttttatt tttgttgttt aggttgtagt gtaatggtat 1680
gattttggtt tattgtaatt tttgtttttt ggatttaagg gatttttttt ttttattttt 1740
ttaagtagtt tagattatag gtatgtatta ttatttttgg ttaatttttg tatttttagt 1800
agagataggg ttttattata ttggttaggt tggttttgaa tttttgattt taagtgattt 1860
atttgttttg gttttttaaa gtgttgagat tataggtttg agttattgtg tttggttttt 1920
gttatttttg aataatattt tttagttttt gtggaaatta tttttaaagt gtttttgaag 1980
tatttagttt ttgtttttta ggatatgtat ttgtagatag gatttaaagt agttaagaat 2040
gtttttgtta agttgataaa tagttattat tgttaatttt tttttattag aatatataaa 2100
tattaagaaa aatgggtgat tgataaatta attttagtat ttataaaaag ttgttttaaa 2160
ggatttatgg aagtatagtt ttttatggtt tttttaatgt tttgtggatg ttgtgaaatt 2220
atagttgttt gtatgtatgt ttttaattat gttagtttta aaaaaagaaa atttttgtaa 2280
atttttgtaa gatataatta gtttttaata ttataaaata tatttggtta ggtgtgttgg 2340
tttttgttta taattttagt attttgggag gttgaggtgg ggagattatt tgaggttggg 2400
agtttgagat tagtttgatt aatatggaga gattttgttt ttattaaaaa tataaaatta 2460
gttgggtgtg gtggtatatg tttgtatttt tagttattta g 2501

<210> 143
<211> 2501

<212> DNA
<213> Artificial Sequence

<220>
<223> chemically treated genomic DNA (Homo sapiens)

<400> 143

ttgggtagtt ggaagtatag gtatgtgtta ttatgtttgg ttaattttgt atttttagta      60
gagatggggt ttttttatgt tggttaggtt ggttttgaat tttgattttt aggtgatttt      120
tttattttgg ttttttaaag tgttgggatt ataggtggga gttaatgtgt ttggttaaat      180
atgttttata atattaaaga ttagttatgt tttatagaaa tttataggag tttttttttt      240
ttgaaattaa tatagttgaa gatatatata taggtaattg tagttttata gtatttatag      300
gatgttgaaa gggttatgaa aaattatgtt tttataaatt ttttaggata attttttgta      360
gatattgaag ttgatttgtt aattgtttat tttttttagt atttatatat tttggtggag      420
gagggttaat aataatagtt gtttgttgat ttggtagaga tatttttaat tgttttgaat      480
tttgtttata agtatatgtt ttgaaaaata aaaattgagt attttagaag tattttagga      540
atggttttta taggagttga aaagtgttat ttaagagtaa tagaagttgg gtatagtggt      600
ttaggtttgt aattttaata ttttgggagg ttaaggtggg tggattattt gaggttagga      660
gtttgagatt agtttgatta atgtagtgaa attttgtttt tattaaaaat ataaaaatta      720
gttagaggtg gtagtgtatg tttgtaattt aagttattta ggagggtgag gagggagaat      780
tttttaaatt tgggaggtag aggttatagt gagttaagat tgtgttattg tattatagtt      840
tgggtaataa gaatgaaatt ttattttaaa aaagagttaa tatatattgt gttttaaagt      900
atattatgat taagatagga tgaaaaaaaa aaaaagttaa atggtaaagg aattaatttt      960
taaataaata taaatttttt atatataaat agtaaaagtt ttattatata tgttttagtt      1020
ttggattttt tttttttttt tttttttttt tttttttttt tttttttttt tttgagatgg      1080
agttttgttt tgttatttag gttggagtgt agtggtttta ttttggttta ttgtaagttt      1140
tgttttttgg gtttatgtta tttttttgtt tttgtttttt gagtagttgg gattataggt      1200
gttttttatt atgtttggtt aattttttgt atttttagta gagatggggt tttattgtgg      1260
tttagatttt ttgattttgt gatttgtttg ttttggtttt ttaaagtgtt gggattataa      1320
gtgtgagtta ttgtgtttgg ttaattttgg ttgttttaaa ttattttaat attttagttt      1380
ttttgttttt tataaaaata taaattgagg ttttagtagg aaaagtttat aaatttttag      1440
ttggaagaaa taatagagaa tgttaaagat atgattagtg tgtaagtatt ttataattgt      1500
taatttaatg aagagagaaa agtaaagtaa aaaaatgttt tggttaattt gaaaaaagtg      1560
attaatttat ggtgtttttt tttttttttg agatggagtt ttattttgtt gtttaggttg      1620

gagtgtaatg gtgtgatttt ggtttattgt aatgtttgtt ttttaggttt aagtgatttt      1680
ttagttttag ttttttaagt agttgtgatt atagatgtat gatattatgt agttaatttt      1740
tgtatttttt tgtagagata gggttttaat atgttggttt ggttggtttt aaaaatttga      1800
ttttaggtga tttatatatt ttagtttttt aaggtgttga gattatagtt ttgagttatt      1860
atgtttagtt aaagtgattt tattttattt ttttgttttt attttttgag atggagtttt      1920
attttattgt ttagattgga gtgtagtggt ataattttta tttattgtaa tttttgtttg      1980
ttggttttaa gtgatttttt tgttttagtt ttttgagtag ttgggattat aggtatttgt      2040
tattatgttt agttaatttt tgtattttta gtataggtag ggttttatta tattggttaa      2100
gttggtttgg aatttttgat tttaggtgat ttattttttt tgatttttta aagtgttggg      2160
attataggtg tgagttattg tatttgattt aaagtgattt attttaaaat tgaatttatt      2220
agattaaaat gttattttgt ggtatatatt gttatattta gaataaaata ttttaaattt      2280
tataggtgtt ttagaaagat ggtatttaat atttttagat agtattatta ttttaaggat      2340
aataaaggta ttattaatgt gtatataata ttttaagtat ataagtaaaa tgaaaatgaa      2400
ataagttgtt tatttttaga gattagtgtt aatatttagt taatattatt tttgtttgta      2460
ttaaagtagt attagttttt attttttgtt gtatattaat t      2501

<210> 144
<211> 2501
<212> DNA
<213> Artificial Sequence

<220>
<223> chemically treated genomic DNA (Homo sapiens)

<400> 144

tttttgggtt gtagtgtagt atattagtta ggtgtttgta ttaagttttg tattaatttg      60
gttgtttaag gatggttgaa ttgatttagg tggaaattgg agtaggttaa aatttaatgt      120

tgattagtaa tagggttgta tttgtaaata gttattgttt tttagtttgg gtaatatagt 180
gagattttat ttttaaaata aattttaaaa ataattaatt agaaaaaaaa attagtttgt 240
aattttagta ttttgggagg ttaaggtggg tagattattt gaggttagga gtttaagata 300
agtttggtta atatggtaaa atttaatttt ttattaaaaa tataaaaatt agttgggtgt 360
ggtggtaggt gtttgtagtt ttagttattt aggaggttga ggtaagagaa ttgtttgaat 420
ttgggaggtg gagattgtag tgggttgaga ttgtgttatt ttattttagt ttgggtagta 480
gagtgtgatt ttgttttaaa aaaaaaaaaa aagaaagaaa agaaaagaaa aagaaaagaa 540
aagaaaaaaa ttgggaggtt taagtttatt tttggtgttt ttatattttt ttgttttatt 600
tttttgtatt tagttttttt ttgtaatttg tgttttatat tagtttttaa gtttttaatg 660
tgatttagta tgagaattgg attttgttat tttttgtttt ataatatttt atgttttttt 720
tttgtttaga atattatttt ttttattgtt tttattaatg gaattggtat ttttttaagg 780
atatgattaa atttgtttat ttttatatta ttttttaaag tagaatttat tttttttttt 840
ttaatatgat gatattgata gggtttgttt ttatttatta gattgtgagt tgtttagggt 900
aggtagtgtt ttttgttttt gtttttgttt tttttttttg agatagggtt ttgttttgtt 960
atttaggtta gagtgtaatg gtatagtttt agtttattgt agttttaatt gttttggttt 1020
aaattattat tttattttag ttttttgagt agttgggatt ataggtatat gttattatat 1080
ttggttaatt tttttgtatt tttagtagag atagggtttg gttatgttgt ttgggttggt 1140
tttgaatttt tggatttaag taatttattt attttagttt tttaaaatga gggattgtgt 1200
tttatttatt tttatgtttt tagtttatag tttagtgttg gatttatggt agtattaaat 1260
aaatatttgt tgaatgtaat agtaaatagt attttaggga gtaagaatta gattaataaa 1320
ggtggtaaaa ggtttggaga aaaaaataat agtttaattt ggttagagta tgagggagag 1380
tagtaggaga taagatggaa aggttttttg ggtaaggttt tgaaggaagt tggaagttag 1440
aagtatataa tgtgtatatt gtggtaggta gtggggagtt aatgaaggtt tttgagtagg 1500
agagtaatgt gttgaaaaat aaatataggt taaatttatt agagtttttt tgatatatat 1560
atttgttttt tatttaagtt taagtttgtt ttttatatat ttattattta atttattttt 1620
gggttttttt agtagtttgt tttatttttt tatttgtttt ttggtggagt tagggatgta 1680
tatatgagtt gttttttttt ttagttagag gatatggggg ttttagtttt tttgtttttt 1740
tttttttgtg tttggagttg ggaagtaggt tagggttagt tgaggttggt tggtaagtag 1800
ttgggtggtg ttagggagag tttgtatagt gttaggtggt gttttgggtt ttaagttgag 1860
tttatggttt tgataatttt ttgtttgtgt atatatttgt tttttatttt atttttattt 1920
tagttttggt atgggggaga gggtataggg ttagataaat ttgtgagatt ttggttttat 1980
ttttgtaaaa gggtgttttg tgagttagtt tgtttttttt taggtttgtt ttttttttat 2040
ttagtttttg tttttaatgt atgtatagtt tgtatatatt gtgtgttggg atattttata 2100
gttagttgta tggttttttt gtgttttagt ttttggtttt ttttgttgat tttggttttt 2160
gttttaggtt ttattgtgta attgttgttg ttattgttgt ttttggtgtt tgtttatttt 2220
tagaggttgt tttggatgta ggaggatttt tttttgggag gaggtttttt tggggaagat 2280
gatttattgg gtgaggagga tttgtttagt gaagaggatt tatttagaga ggaggattta 2340
tttggagagg aggatttatt tggagaggag gatttatttg gagaggagga tttatttgaa 2400
gttaagttta aattagaaga agaggttttt ttgaagttag aggatttatt tattgttgag 2460
gtttttggag atttttaaga attttagaat aatgtttata g 2501

<210> 145
<211> 2501
<212> DNA
<213> Artificial Sequence

<220>
<223> chemically treated genomic DNA (Homo sapiens)

<400> 145
   ttgtgggtat tattttgggg tttttgagga tttttaggag ttttaatagt aggtagattt 60
   tttaatttta gggagttttt tttttttgat ttaggtttaa ttttaggtag attttttttt 120
   ttaggtagat tttttttttt aggtagattt ttttttttgg gtggattttt ttttttgggt 180
   gaattttttt tattgggtag attttttttg tttagtgggt tatttttttt agaagagttt 240
   ttttttaagg gggaattttt ttgtatttgg ggtaattttt ggggatggat aggtattaga 300
   agtagtagtg atagtagtag ttgtatagtg aggtttggag taggggttgg gattaataga 360
   gggagttagg ggttggggta taggggagtt atgtggttga ttgtggggtg ttttagtata 420
   tggtgtgtat gggttgtatg tgtattggaa atgagagttg ggtgggggag gagtaagttt 480
   ggaggggagt aggttgattt atagagtgtt tttttgtaga gatggagtta aagttttata 540
   ggtttgtttg gttttgtgtt ttttttttta tattaaagtt aggatggggg tggagtgagg 600
   ggtaggtgtg tgtataggta gaaggttatt ggggttatgg atttagtttg gaatttaagg 660
   tattatttgg tattatgtag gttttttttg gtattattta gttgtttgtt agttagtttt 720
   agttaatttt ggtttgtttt ttagttttag gtatagaagg ggaaaggtag gggagttggg 780
   gtttttatgt tttttggttg agagggaaag tagtttatgt atatattttt gattttatta 840
   ggaagtaggt aaagaggtag ggtaggttgt taggggagtt tgagggtgag ttaagtaatg 900
   ggtatgtggg agataaattt gagtttgaat gaaaagtaag tgtatgtgtt agaggggttt 960
   tgataggttt aatttatatt tattttttaa tatattattt ttttgtttaa aagtttttat 1020
   tggtttttta ttgtttgtta tgatatgtat attgtgtatt tttgattttt aatttttttt 1080
   aaaattttgt ttaagagatt tttttatttt gttttttatt attttttttt atattttagt 1140
   taaattaaat tattattttt ttttttaaat tttttattat ttttgttaat ttagtttttg 1200
   ttttttgaaa tgttatttat tattgtattt aataaatatt tatttagtat tattataggt 1260
   ttagtattgg gttatggatt agggatatgg aaatgaataa gatatggttt tttattttgg 1320
   gaggttgagg tgggtggatt gtttgagttt aggagtttga gattagtttg ggtaatatgg 1380
   ttaaattttg tttttattag aaatataaaa aaattagtta ggtgtaatgg tatgtgtttg 1440
   tagttttagt tatttaggag gttgaaatgg gatgatggtt tgagttgagg tggttgaggt 1500
   tgtagtgagt tgagattgta ttattgtatt ttggtttggg tgatagagta agattttgtt 1560
   ttaaaaaaga aaaataaaaa taaaaataaa aaatgttatt tgttttgagt agtttataat 1620
   ttagtgagtg agggtaaatt ttgttaatat tattatattg agggaagaga gatgaatttt 1680
   gttttagaag ataatataga agtaggtaaa tttgattatg tttttgaaga aatattagtt 1740
   ttattggtaa aaataatagg gagaatgata ttttgggtaa agggaaggta taaaatatta 1800
   tgagtaggag agtgatagaa tttagttttt atattaggtt atgttgaggg tttgagggtt 1860
   agtatggagt atagattgta ggggaagatt gggtgtaagg agatggggta gaagagtgtg 1920
   aggatattaa gaatgggttt aggtttttta gttttttttt tttttttttt tttttttttt 1980
   tttttttttt tttttttttt ttttgagata gagttgtatt ttgttattta ggttggagtg 2040
   aagtggtgtg attttggttt attgtaattt ttatttttta ggtttaagtg atttttttgt 2100
   tttagttttt tgggtagttg ggattatagg tatttgttat tatgtttagt taatttttgt 2160
   atttttggta gagattgggg ttttgttatg ttggttaggt ttgttttgaa tttttgattt 2220
   taggtgattt gtttgttttg gttttttaaa gtgttgggat tataggttgg tttttttttt 2280
   taattaatta tttttaaaat ttattttaga gatgggattt tattgtgttg tttaggttgg 2340
   agggtggtgg ttatttatag gtgtgatttt gttattgatt agtattgagt tttgatttgt 2400
   tttaattttt atttgggtta gtttaattat ttttaggtaa ttaggttgat gtagaattta 2460
   gtgtagatat ttgattggta tattatatta tagtttagaa a 2501

<210> 146
<211> 2501
<212> DNA
<213> Artificial Sequence

<220>
<223> chemically treated genomic DNA (Homo sapiens)

<400> 146
   gtttgttgga ttatttaaat ttgttttgtt tttggttgga gtttgatgtt atttagtggt 60
   agatgttgtt ttggttgaaa tattgatgga gatttttggt ttagtggttg gttgagattt 120
   ttaggatagt ggtagtattt ttaggaattt tttaattagt taggattttt ttttttgatt 180
   tgatttgagg ataataggtt gggaaaataa tggggatttg gagatttttt ttttgttttt 240
   ttgtttttta agatagggtt atatgtattt tatttttttt tttttgattt gttgaaggta 300
   agaattttag tttgtttttg tttgagaatg gggagattga gtagaggatg ataggaagga 360
   tggtaattat taattgggtt atgtttgatt tttagttatt ttaggtgtta ataagttgtg 420
   ttttttggtt tttaaaattt attatttaat gaaatatatg taatgaaata tagtagtgaa 480
   aataataaat tggaggtata tgtaagagtt tgggtgaatt tttttttttt gtaagtaaaa 540
   atatttttaa ttgatgtatg ataattttgt atatttatgg gatatatgtg atattttgat 600
   atatgtatat gtatatatga tgtaatgatt aagttggggt atttaggatt tttattattt 660
   taagtattta ttattttttt ttttggggaa tattttttat tttttagtta ttttgaaata 720
   tataataaat aattatttat agttttttta tggtgttatt aaatattaga atttaatttt 780
   tttatgtaat tatatgtttg tttttattaa tttttttatt ttttttattt tttttagtgt 840
   ttggtaatta ttattttgtt ttatttttaa aagattaatg tttttagttt ttatatgagt 900
   gttaatatgt tttttttagg tgtagaggtt gttgattttt ggatttggaa ggtgggggag 960
   agtggtttgg atttagtatt tttttttttt tttagggtta ggttttgttt ggttattttt 1020
   tgttgttata ggtgtgtttg gtatagatat ttagtttttg gggttgtgtt gtttttattt 1080
   ttgtatgttt ttagaggtat ttgttttgtg gatttgattt ttttttagat atataaatag 1140
   attattattg agtagatata gggttatttt ttagtttttt attaggtaag agtgattagg 1200
   gtagggattg atattgttga atttaggagt taggtttgat ttagttttag gtttagtagg 1260
   ttttgtttgt ttatttgggt taggtttaga gtttgggagt ttttggttgg tgggaggtta 1320
   tttgtaattt attttatatg tagttttagt ttttttatta ggtggggtga ttaggatagg 1380
   gatagaattt gttgaattta ggagtgagat ttggttttgg gttttgttgg gtttttttgt 1440
   ttattttggt tggatttggt gtttgggaga ttttggttgg tgtgaggtta tgtttattag 1500
   atatgtagtt ttagttattt tattagttgg gtgattagga tagggatgga ggttgttgag 1560
   tttagttaga ggtttgtttt ttggggtttg ttttgggtgt ttttttaagg atggataggg 1620
   taggtagggt ttgggatgat ggttgtatag ttttggtttt gtgtttttag gtttgttttg 1680
   ttttttgatg tgagggagat ttgggggatg ggataggttg ggttttgtag tgtttgattt 1740
   tttgtagggt ttttgggata ggggtttggt ggatagttgg ttgtttaggg gtgaggggtt 1800
   taagttggta ttgtggttat tttttggttt gggttttttt ggggaggggt ggggttggtg 1860
   agaattggtt atgtgttttg gggtttattt ggggtttttt agggttggaa gtagggtttt 1920
   tgtgtgtagg tgttttgagg attttgggtt gtttatttta tgttaggggg tggaattttt 1980
   tgtagttttt ttgtttttgt atttttgtgg gttttgattt tttttttgag agttgggtag 2040
   aggttttgga gttatgtagg ttgaaggttg gggtataggg ggtttgatgg gtgatgttga 2100
   tggtttagga ggttttggta tttttgatgg tttagggggt aatgttggtg gtttaggaga 2160
   ggtgggtgtt atgggtggta gaggtttttg gggtgtaggg gtagtaaggg ttttggggtt 2220
   gggaggaggt gttttgtggg gtttgtatgg tggtgtggtt ttagggttga atggatgttg 2280
   tagatgtggg gttagggggt tggagagttg tttgtttgag ttgtattttg ttttgttttg 2340
   ttttgttttg atagttttgg tggtgaggtg ggggttggga gatggggagg gtagggttag 2400
   gtgggggagg aggtggggga gatgtgagta agtggttggg tgggggtggg ggtgggggtt 2460
   gggaggtggg gatatgagag gttagttgtg aggaggggag g 2501

<210> 147
<211> 2501
<212> DNA
<213> Artificial Sequence

<220>
<223> chemically treated genomic DNA (Homo sapiens)

<400> 147

tttttttttt ttgtagttgg ttttttatat ttttattttt taatttttat ttttattttt 60
atttaattat ttatttgtat tttttttgtt ttttttttta tttggttttg ttttttttat 120
tttttggttt ttattttgtt attagaattg ttagaataga atagaataga atagaatata 180
atttaagtag gtggtttttt ggttttttgg ttttgtattt gtagtattta tttagttttg 240
aagttgtgtt gttatgtgga ttttgtgggg tgtttttttt tggttttgag gtttttgttg 300
tttttgtgtt ttggggattt ttgttgtttg tggtatttgt tttttttggg ttgttagtat 360
tgttttttgg gttattagga atgttagggt tttttgggtt attagtattg tttgttgaat 420
tttttgtgtt ttggtttttg gtttgtatgg ttttggagtt tttgtttggt ttttagagag 480
aaggttaggg tttatgagga tgtggaggta gagaggttgt aggaagtttt gttttttggt 540
gtgagatggg tagtttggga tttttagggt gtttgtgtat aggggtttta tttttggttt 600
tgggagattt tgagtgagtt ttggagtatg tgattggttt ttattaattt tgtttttttt 660
taagagagtt tgggttggaa ggtggttgta atgttagttt ggatttttta tttttgagta 720
gttggttgtt tgttggattt ttgttttggg agttttgtag ggagttaggt attgtggggt 780
ttagtttgtt ttattttttg ggtttttttt atattgagga gtaagatggg tttgggaata 840
tggggttggg attgtgtggt tattgttttg gattttgttt gttttgtttg tttttggggg 900
agtgtttagg atagattttg gggggtaggt ttttaattgg gtttagtagt ttttgttttt 960
gttttggttg tttagttggt ggggtagttg gaattgtatg tttggtgggt gtggttttgt 1020
gttagttaag gttttttagg tgttaggttt agttaaggtg gatgggaggg tttagtggga 1080
tttggggttg gattttattt ttgggtttaa tgggttttgt ttttgtttta gttgttttgt 1140
ttggtggggg agttggagtt gtgtgtgggg tgggttgtgg gtggtttttt attagttagg 1200
ggtttttggg ttttaggttt ggtttaggtg gataggtggg atttgttgga tttgaggttg 1260
ggttgggttt ggtttttggg tttggtagta ttagtttttg ttttggttgt ttttgtttgg 1320
tagggagttg ggaggtgatt ttgtgtttgt ttagtggtga tttatttgtg tgtttggaag 1380
ggggttaaat ttatgggata ggtattttta gaggtatgta ggggtgaggg tggtatggtt 1440
ttgggagttg aatgtttgtg ttaggtatat ttgtagtaat aggaggtgat tagatagggt 1500
ttagttttga ggaaggggga ggtattgaaa tttaaattat ttttttttat tttttaaatt 1560
taggaattag tagtttttgt atttgggaaa agtatattgg tatttatgtg gaaattaaaa 1620
atgttgattt tttggaggta gagtagaatg atggttatta gatgttggga aaggtgggga 1680
gggtggggag gttaatgggg ataaatatat agttgtatag aaggattaag ttttagtgtt 1740
tgatagtatt gtagagggat tataggtaat tatttattgt atattttaaa atagttaaaa 1800
gataggaaat gttttttaaa aaaagaaatg ataaatgttt gaggtggtgg agattttaaa 1860
tattttgatt tgattattat attgtgtgtg tgtgtgtatg tattagaata ttatatgtat 1920
tttataaata tgtagagtta ttatatatta gttaaaaatg tttttattta tagaaaagaa 1980
aaatttattt aagtttttgt gtgtattttt agtttattat ttttattgtt gtattttatt 2040

atatatattt tattaaatga tgaattttaa aaattagaga atataatttg ttaatatttg 2100
gaatagttga aaattagata taatttggtt agtgattgtt attttttttg ttgttttttg 2160
tttagttttt ttatttttag atagaggtag gttaaagttt ttatttttaa taggttaggg 2220
aagggagagt aaagtgtatg tgattttgtt ttaggaagta ggaagataga gggagggttt 2280
ttgggttttt attatttttt tggtttatta tttttagatt aagttaagaa aggaagtttt 2340
aattgattga gagatttttg gagatattgt tattgttttg gaagttttag ttagttattg 2400
gattggaaat ttttattaat attttagtta gagtaatgtt tgttattaga tggtattaaa 2460
ttttagttgg gagtaggata gatttggatg gtttagtggg t 2501

<210> 148
<211> 2501
<212> DNA
<213> Artificial Sequence

<220>
<223> chemically treated genomic DNA (Homo sapiens)

<400> 148

gtattatttt ttttaatttt agtatagtat gttagatgtt gttttataga tgagttaatt 60
gagattagag atgtttagtt atttgtttaa ggtgatatga ttgatatgga atagagttaa 120
gatttttttt tttttttttg atatggagtt ttattttgtt ttttaggttg gagtgtagag 180
gtgtaatttt agtttattgt aagttttgtt ttttaggttt atgttatttt tttgttttag 240
ttttttgagt agttgggatt ataggtattt gttattatat ttggttaatt ttttgtattt 300
ttagtagaga tagggtttta ttgtgttagt taggatggtt ttgatttttt gattttgtga 360
tttgtttgtt ttggtttttt aaagtgttgg aattataggt gtgagttatt gtgattggtt 420
agatttaaga tttgaattta ggtttttttg gttttagagg tttttgtttt ttaatttttt 480
aggatggtat agtaatttgt tttataagag gtgtttgttt taagtgtgtt tagtatatgg 540
aagtaagttt agaaatgtaa gtgtatattt gtaaagaggt gtgggagatg ggggggaggg 600
aagagagaaa gagatgttgg tgttttttat tttttagttt ttgataggtg tttttgattt 660
ttttttgatt agtatagttg tatttttggt tggggtattt taattagaat tgttaaattt 720
agtatataaa aataaggagg tttagttaaa tttgaatttt agataaataa tgaataattt 780
gttagtataa atatgtttta tgtaatattt tgttgaaatt aaaaaaaaaa aaaaaagttt 840
tttttttatt tttattttta ttattaggtt taaggaatag ggttaggggt tttaaataga 900
atgtggttga gaagtggaat taagtaggtt aatagaaggt aaggggtaaa gaagaaattt 960
tgaatgtatt gggtgttggg tgttttttta aataagtaag aagggtgtat tttgaagaat 1020
tgagatagaa gtttttttgg gttgggtgta gttgtttgtg gttgtaattt tagtattttg 1080
ggaggttgag gtgggaggat tatttgaggt tgggagttta agattagttt tattaatgtg 1140
gagaaatttt gtttttatta aaaatataaa aattagttgg gttatggtgg tatatgtttg 1200
taattttagt tgtttgggag gttgaggtag gagaattatt tgaattaggg aggtagaggt 1260
tgtggtgagt agagattgtg ttattgtttt ttagtttggg taataagagt aaaagtttgt 1320
ttaaaaaaaa aaaaaagttt ttttgatgtg attgtttttt tttaaatttg tagatttttt 1380
taagattatg ttttttagat attttaaaga ttttagaaga tatgttttgg gggttttgga 1440
agttataagg taaatataat atattttttt ttttgattat taattttatt agaggatgtg 1500
gtgggaaaat tattatttga tattaaaata aataggtttg ggatggagta ggatgtaagt 1560
tttttaggaa agtttaagat aaaatttgag atttaaaagg gtgttaagag tggtagttta 1620
gggaatttat tttggatttt gggggagggg gtagagttat tagtttttgt atttagggat 1680
tttttgagga aaagtgtgag aatggttgta ggtaatttag gtgttttggt gttaggaggg 1740
atgtatttag gtttgtgtga agagagggag aaagtgaagt tgggagttgt tatttttaga 1800
tttgttggaa tgtagttgga gggggtgagt tgggagtgtg tttgttttta attataggag 1860
aaggaggagg tggaggagga gggttgtttg aggaagtata agaatgaagt tgtgaagttg 1920
agattttttt ttattgggat tggagaaatt aggggagttt tttgggtagt tgtgtgtttt 1980
tttttatggg gttttttatt gtgttgtgtg tttggttttt attttttgta gtattttgtg 2040
ttttgtgttt ttttagttgg gtttagttgg agttatgggg ttggagttgt agtgagtatt 2100
atggagttgg tggttttgtg ttgttggggg tttttttttg tttttttgtt ttttggagtt 2160
gtgagtattt aaggtgggtt tggtgtgggg aggggatgga gtagtggtgg gattttgttt 2220
tgtggatgtt ttgttgaggt tttgtggttg gtggggttag aggggtttgg atgagttttt 2280
ttattttgaa gttgtggata gttgagatgt ttagggtagt tgggttttgg ggtttttggg 2340
tgggaggggg tagttatatg gtagtggttt gagatggttt atttaagaga ttggtgtttt 2400
ttaggttttg aggggttttg ggaatttgtt aaagaagttt tttgaaattg tttagaaagt 2460
ttttttgtaa agggtgtatt gtgtagagtg tgtgtgtgtg t 2501

<210> 149
<211> 2501

<212> DNA
<213> Artificial Sequence

<220>
<223> chemically treated genomic DNA (Homo sapiens)

<400> 149

atgtgtgtgt gtgttttatg taatatattt tttgtgggaa aattttttga ataattttag 60
agaatttttt tgataagttt ttggagtttt ttggagtttg gaaagtgtta gttttttgga 120
tgggttattt tgagttgttg ttgtgtaatt gttttttttt gtttgagggt tttagggttt 180
ggttgttttg agtgttttga ttgtttataa ttttgggata ggagagtttg tttgggtttt 240
tttggttttg ttggttgtgg gattttggtg gggtatttat agggtagggt tttgttgttg 300
ttttgttttt tttttatatt agatttattt tgggtgtttg tggttttggg gggtaagagg 360
gtgaggagga gtttttagtg gtataaggtt gttagtttta tggtgtttat tgtggttttg 420
gttttatggt tttggttgga tttggttggg agggtgtggg gtgtggggtg ttgtgagggg 480
tgggggttgg gtgtgtggtg tagtaaaggg ttttgtggga aggggtgtgt ggttgtttgg 540
ggggtttttt tggttttttt ggttttaatg gaggggaatt ttagttttat aattttattt 600
ttatattttt ttaagtagtt tttttttttt attttttttt ttttttgtga ttgggagtaa 660
gtgtgttttt agtttgtttt ttttaattgt attttaataa gtttgggagt ggtaattttt 720
agttttattt tttttttttt tttgtgtagg tttgggtgtg ttttttttag tgttgggatg 780
tttgggttgt ttgtagttgt ttttatattt ttttttggag aatttttaaa tgtagaggtt 840
ggtgattttg tttttttttt tggagtttgg gataaatttt ttaggttgtt atttttaata 900
tttttttaag ttttaggttt tattttaaat ttttttgggg agtttgtatt ttattttatt 960
ttaagtttat ttgttttaat attaaataat ggttttttta ttatattttt tagtaaaatt 1020
gatagttaag gagggggatg tgttgtgttt attttgtggt ttttaggatt tttggggtat 1080
attttttgga atttttgaag tatttgaaaa gtatgatttt aagagggttt ataaatttgg 1140
gaggagatag ttatattgaa aggatttttt ttttttttta aatgaatttt tgtttttgtt 1200
gtttaggttg gagagtaatg gtgtgatttt tgtttattat aatttttgtt tttttggttt 1260
aagtgatttt tttgttttag ttttttgagt agttgggatt ataggtatgt gttattatga 1320
ttagttaatt ttttgtattt ttagtaaaga tagggttttt ttatgttggt gaggttggtt 1380
ttgaattttt aattttaggt gatttttttg ttttagtttt ttaaagtgtt ggaattataa 1440
ttatgagtaa ttgtatttag tttaaaaaga tttttatttt aattttttaa aatgtatttt 1500
ttttgtttat ttaaggaggt atttagtatt taatgtattt aaggtttttt ttttgttttt 1560
tgttttttat tagtttgttt aattttattt tttaattata ttttatttgg agtttttgat 1620
tttatttttt aggtttagtg gtaggggtgg ggatggaagg aagatttttt tttttttttt 1680
taattttaat aagatattgt atgggatata tttatattaa taaattattt attgtttatt 1740
tgaaatttaa atttaattgg gtttttttat ttttatgtgt taaatttggt agttttagtt 1800
ggaatgtttt agttaagaat gtagttatat tggttaagaa gggattaaag gtatttatta 1860
gggattggag aatgaaggat attagtattt tttttttttt tttttttttt ttatttttta 1920
tattttttta taggtatata tttgtatttt taaatttgtt tttatgtgtt gagtatattt 1980
aaagtaggta ttttttgtgg gataggttgt tatgttattt tagggagttg agaaataggg 2040
gtttttggga ttaagaggat ttgggtttaa attttgaatt tggttagttg tggtggttta 2100
tatttgtaat tttagtattt tgggaggttg aggtaggtag attatgaggt taggagattg 2160
agattatttt ggttaatatg gtgaaatttt gtttttgtta aaaatataaa aaattagtta 2220
ggtgtggtgg tgggtgtttg tagttttagt tatttaggag gttgaggtag gagaatggtg 2280
tgaatttggg aggtagagtt tgtagtgagt tgagattgtg tttttgtatt ttagtttggg 2340
agatagagtg agattttgtg ttaaaaaaaa aaaaaaaaat tttgatttta ttttatatta 2400
gttatgttat tttgggtaag tgattgagta tttttggttt tagttggttt atttgtaaaa 2460
tagtatttga tatattatgt tggggttaaa ggagataatg t 2501

<210> 150
<211> 2501
<212> DNA
<213> Artificial Sequence

<220>
<223> chemically treated genomic DNA (Homo sapiens)

<400> 150

atttttgttt attgtaattt tgttttttgg atttaagtaa ttgtttgttt tagttttttg 60
agtagttggg attataggta tttattagta tgtttggtta agttttgtgt ttttattaga 120
gatgggtttt tgttattttg gttggggtgt ttttgaattt ttgattttgt gatttatttg 180

ttttggtttt ttaaagtgtt gggattatag gtatgagttt ttgtgtttgg tggagatata 240
atttttatat attggttttt tatttagtgg ttttgtgaaa tatgtttgtg aattttaaag 300
tttattttta ggttgttttt agtttttaat atatagaaat atattatttt ggaataagag 360
tagttttgtt tttgttattt tttttttttt ttttttttgt atttttttgt agagatgggg 420
ttttgttatg ttttttgggt tgttgttgaa tttttgagtg taagtgatgt atttatgtta 480
ttttttatag tgttgggatt attggtgtgg gttattgtgg tgggtttgtt gttgttattg 540
taaagagttt tatttttttt tttgatttta tggtattgtg tagatttatt tgttataatg 600
gtgatagtgg atatttttgt tttatttttg atgagaaatt gaaaaatttt aatattttat 660
tattttattt attttttttt ttttgtagat ggattttatt agagtgagtt attttatttt 720
gttttaaatt tgttgagagt atttatttga atatatgttg atttttatta aatagtgtat 780
ttattttgat tattatagtg ttttttttta tttatgtgtt aatatagtga atttgattga 840
taaatttgta tgtttttagg tttgattatt aaaatttgag atagtgtttt attttgttat 900
tgaggttgga gtgtagtggt gttattagag tttgttgtag ttttgatttt ttgggtttag 960
gtgttttttt tattttagtt ttttgagtag ttgtgagtat aggtatatgt tattatgttt 1020
agttaatttt ttgatggttt tttgtttgtt ttttgtagtg atgagatttt ttgatgttgt 1080
ttaggttggt tttgaagttt tgagtttagg tgatttggtt agtttagttt tttaaaatat 1140
taggattata ggtgtgagtt ttggtttggt ttggtttttt ttatataggg gttttattta 1200
tataaagatt aaagttaatt tgtgtttttg tgtgggtggg ttaagagtat gatgattttt 1260
attattttat tgatttaaag aaaattattt ttgatttatt agtgtgtaag gttatgaaag 1320
tataattttg ttgaaagtat atattgttaa tgggtgttgg gaattgtgta ttttttgttg 1380
ttgtgggagt atgtttttgg aggtattttt tatttgtttt tttaatttta aatattttag 1440
gattatgggt tgtgattggt aggattatgt attttgttgt ttttaagatg gagtatattt 1500
ttatgtggtt ttattttggt tgttttgttt ttttaatatt gttattttat tttttgtgat 1560
tttgatgtta taaatgatag atattgtttt agtatttttt tatgggtttt agtgatttta 1620
tttatttttt ttttagtttt tttttttgat ttgtttataa tgaataattt ttttttggga 1680
taggttgtta tttttgtttt tgtaggtggt ttatttgttt ttttagttag ttatagtggt 1740
ttttgttttt atggtgggtt tggggtaaga gagggttttg ggttgggggt ggggtttagt 1800
tgaagatggg gtgagttttg aggggagtat tatttgagtt ttagaggtat aggaaatagt 1860
agagggaggt gggatttttt tatttttaat gaggatgggt atggagggtt tggggtgtgg 1920
tgttgggaat ggtagttttt tttagtttat agttgtgtat gttttttggg tttttgtttt 1980
agtgtgtatg tttattgggt gttttttgtt tggttttttt gtttatgtga agaatgttag 2040
ggagttgtga ggtagtgttg tgtggttttt gttgtttgga tttttttttt tttattgaga 2100
tttatttggt aggtttgtag gttagttatt ttgggggttg aagtgtgagt gagggtggag 2160
agggttttgg gtgggttagg tgggttttgt tttttggttt gtggtttttg agggagaagg 2220
gttatgaggt tgtttttttt ttttttatag gttgtgaggt tattggtggt tttgtggttg 2280
tgaaggggtt tggatgggga ggaaggtggg ttgtggaggg gaggtggtta ggggtttagg 2340
tgaagatggg gtgagtgttg ttggggggat ggaagttttg aggtgttggg aatttttgat 2400
gatatagggt agattttttg aatggggttt ttggtggggg tgaggtgggt ggtgaagaag 2460
gggtttggta tttgggaagg ttgtggtttg gtgagtgttt t 2501

<210> 151
<211> 2501
<212> DNA
<213> Artificial Sequence

<220>
<223> chemically treated genomic DNA (Homo sapiens)

<400> 151
   ggggtgttta ttaggttgta gtttttttag gtgttaggtt ttttttttat tgtttgtttt 60
   gtttttgttg gaggttttat ttagggaatt tgttttgtgt tgttgggggt ttttggtatt 120
   ttgggatttt tattttttta atagtattta ttttattttt atttgagttt ttgattgttt 180
   tttttttatg gtttattttt ttttttgttt aggttttttt atgattatga agttgttggt 240
   ggttttgtag tttgtgaagg gaaggaggat gattttgtgg tttttttttt ttggaggtta 300
   tagattagga agtgggattt gtttgattta tttaaggttt tttttatttt tatttatatt 360
   ttagtttttg ggatgattgg tttgtagatt tattagatga attttagtag aggaaaaaga 420
   gtttggatgg taggaattat atagtattgt tttatagttt tttggtgttt tttatgtggg 480
   tgaaggggtt gggtagaaga tgtttagtga atatgtgtat tgaggtggga gtttagggag 540
   tatgtgtggt tgtgggttgg ggagggttgt tgtttttagt gttatgtttt aaatttttta 600
   tgtttatttt tattgaggat aagggaattt tatttttttt tgttgttttt tatgtttttg 660
   ggatttaagt agtgtttttt ttaaaattta ttttattttt aattgaattt tatttttaat 720
   ttagggtttt tttttgtttt ggatttatta tggggataag gattattgtg attggttggg 780
   aagataggta aattatttgt gaaaatagaa atagtaattt attttaaaag gaaattgttt 840
   attgtgaata agttagagaa agagattgaa agaaaaatga atagaattgt taggatttgt 900
   aagaaaatgt taaaatgata tttattattt gtagtattag aattatagag ggtgaagtga 960
   tagtattagg gaaataggat agttagagtg aaattatgtg aaaatatatt ttgttttgaa 1020
   agtagtaaga tatatagttt tattagttat aatttatggt tttaagatgt ttggagttga 1080
   gaaaatagat gaaaggtatt tttaaggata tgtttttata gtagtggaaa gtgtatggtt 1140
   tttaatattt attaataata tatgttttta atagaattat gtttttatgg ttttatatat 1200
   tggtaagtta aggatagttt tttttaaatt aatagaatga taaaagttat tatgttttta 1260
   gtttatttgt ataaaggtat agattaattt tagtttttat atagataaga tttttatata 1320
   agaaaaatta gattaggtta aggtttatgt ttgtaatttt agtattttgg gaggttgagt 1380
   tggttagatt atttgagttt aggattttga gattagttta agtaatatta gaaaatttta 1440
   ttattataaa aaataaataa aaaattattg aaaaattagt tgggtatggt ggtatgtatt 1500
   tatatttata gttatttagg aggttgaggt gggaggagtg tttgagttta ggaggttaag 1560
   gttgtagtga gttttgataa tattattgta ttttagtttt ggtgatagag tgagatgttg 1620
   ttttaagttt taataattga atttaaaaat gtataaattt attaattgaa tttattatat 1680
   taatatatga atgggaaaaa atgttgtggt aattgaaata gatgtattgt ttgatgaaaa 1740
   ttaatatata tttaaatgaa tatttttagt aaatttggaa tagaatggaa tgatttattt 1800
   tgataaagtt tatttataaa aaaaggagag tgaataggat ggtgaaatgt tgaaattttt 1860
   tggtttttta ttagggataa gataaggatg tttattgtta ttattgtaat gggtgggttt 1920
   gtgtaatgtt ataaaattag aaaaggaaat aaaatttttt ataatggtaa tgatgggttt 1980
   gttatggtgg tttatgttag taattttagt attgtgggag atgatgtggg tgtattattt 2040
   gtatttaaaa gtttaataat agtttgggaa atatggtaaa attttgtttt tataaaaaaa 2100
   tataaaaggg aaaagaaaaa aaaatggtgg aaataaaatt gtttttattt taggatgata 2160
   tgtttttgta tattgaagat tgaaaatgat ttagaagtaa atttagaatt ttataagtat 2220
   attttataag gttattggat agaaaattaa tatgtaagaa ttatgttttt attgggtgtg 2280
   gaggtttatg tttgtaattt tagtattttg ggaggttaag gtaggtgaat tatgaggtta 2340
   ggagtttaag agtattttgg ttaagatggt gaaagtttat ttttagtaaa aatataaaat 2400
   ttagttaggt gtgttggtgg gtgtttataa ttttagttat ttgggaggtt gaggtagata 2460
   attgtttgaa tttgggaggt ggggttgtag tgagtagaga t 2501

<210> 152
<211> 2500
<212> DNA
<213> Artificial Sequence

<220>
<223> chemically treated genomic DNA (Homo sapiens)

<400> 152

taagtaattg tttgttttag ttttttgagt agttgggatt ataggtattt attagtatgt 60
ttggttaagt tttgtgtttt tattagagat gggtttttgt tattttggtt ggggtgtttt 120
tgaatttttg attttgtgat ttatttgttt tggtttttta aagtgttggg attataggta 180
tgagtttttg tgtttggtgg agatataatt tttatatatt ggttttttat ttagtggttt 240
tgtgaaatat gtttgtgaat tttaaagttt atttttaggt tgtttttagt ttttaatata 300
tagaaatata ttattttgga ataagagtag ttttgttttt gttatttttt tttttttttt 360
tttttgtatt tttttgtaga gatggggttt tgttatgttt tttgggttgt tgttgaattt 420
ttgagtgtaa gtgatgtatt tatgttattt tttatagtgt tgggattatt ggtgtgggtt 480
attgtggtgg gtttgttgtt gttattgtaa agagttttat tttttttttt gattttatgg 540
tattgtgtag atttatttgt tataatggtg atagtggata tttttgtttt atttttgatg 600
agaaattgaa aaattttaat atttattatt ttatttattt tttttttttt tgtagatgga 660
ttttattaga gtgagttatt ttattttgtt ttaaatttgt tgagagtatt tatttgaata 720
tatgttgatt ttttattaaat agtgtattta ttttgattat tatagtgttt ttttttattt 780
atgtgttaat atagtgaatt tgattgataa atttgtatgt ttttaggttt gattattaaa 840
atttgagata gtgttttatt ttgttattga ggttggagtg tagtggtgtt attagagttt 900
gttgtagttt tgattttttg ggtttaggtg ttttttttat tttagttttt tgagtagttg 960
tgagtatagg tatatgttat tatgtttagt taattttttg atggtttttt gtttgttttt 1020
tgtagtgatg agattttttg atgttgttta ggttggtttt gaagttttga gtttaggtga 1080
tttggttagt ttagtttttt aaaatattag gattataggt gtgagttttg gtttggtttg 1140
gtttttttta tataggggtt ttatttatat aaagattaaa gttaatttgt gtttttgtgt 1200
gggtgggtta agagtatgat gatttttatt attttattga tttaaagaaa attatttttg 1260
atttattagt gtgtaaggtt atgaaagtat aattttgttg aaagtatata ttgttaatgg 1320
gtgttgggaa ttgtgtattt tttgttgttg tgggagtatg tttttggagg tattttttat 1380
ttgttttttt aattttaaat attttaggat tatgggttgt gattggtagg attatgtatt 1440
ttgttgtttt taagatggag tatattttta tgtggtttta ttttggttgt tttgtttttt 1500

taatattgtt attttatttt ttgtgatttt gatgttataa atgatagata ttgttttagt 1560
atttttttat gggttttagt gattttattt attttttttt tagttttttt ttttgatttg 1620
tttataatga ataatttttt tttgggatag gttgttattt ttgtttttgt aggtggttta 1680
tttgtttttt tagttagtta tagtggtttt tgtttttatg gtgggtttgg ggtaagagag 1740
ggttttgggt tgggggtggg gtttagttga agatggggtg agttttgagg ggagtattat 1800
ttgagtttta gaggtatagg aaatagtaga gggaggtggg atttttttat ttttaatgag 1860
gatgggtatg gagggtttgg ggtgtggtgt tgggaatggt agtttttttt agtttatagt 1920
tgtgtatgtt ttttgggttt ttgttttagt gtgtatgttt attgggtgtt ttttgtttgg 1980
tttttttgtt tatgtgaaga atgttaggga gttgtgaggt agtgttgtgt ggtttttgtt 2040
gtttggattt tttttttttt attgagattt atttggtagg tttgtaggtt agttattttg 2100
ggggttgaag tgtgagtgag ggtggagagg gttttgggtg ggttaggtgg gttttgtttt 2160
ttggtttgtg gtttttgagg gagaagggtt atgaggttgt tttttttttt tttataggtt 2220
gtgaggttat tggtggtttt gtggttgtga aggggtttgg atggggagga aggtgggttg 2280
tggaggggag gtggttaggg gtttaggtga agatggggtg agtgttgttg gggggatgga 2340
agtttgagg tgttgggaat tttgatgat ataggtaga tttttgaat ggggtttttg 2400
gtgggggtga ggtgggtggt gaagaagggg tttggtattt gggaaggttg tggtttggtg 2460
agtgtttttt ttagtggtgt ggagtgtgga gtgtttgagt 2500

<210> 153
<211> 2500
<212> DNA
<213> Artificial Sequence

<220>
<223> chemically treated genomic DNA (Homo sapiens)

<400> 153
   atttaggtgt tttgtatttt atattgttgg ggggggtgtt tattaggttg tagttttttt 60
   aggtgttagg tttttttttt attgtttgtt ttgtttttgt tggaggtttt atttagggaa 120
   tttgttttgt gttgttgggg gtttttggta ttttgggatt tttatttttt taatagtatt 180
   tattttattt ttatttgagt ttttgattgt ttttttttta tggtttattt ttttttttgt 240
   ttaggttttt ttatgattat gaagttgttg gtggttttgt agtttgtgaa gggaaggagg 300
   atgattttgt ggtttttttt ttttggaggt tatagattag gaagtgggat ttgtttgatt 360
   tatttaaggt tttttttatt tttatttata ttttagtttt tgggatgatt ggtttgtaga 420
   tttattagat gaattttagt agaggaaaaa gagtttggat ggtaggaatt atatagtatt 480
   gttttatagt tttttggtgt tttttatgtg ggtgaagggg ttgggtagaa gatgtttagt 540
   gaatatgtgt attgaggtgg gagtttaggg agtatgtgtg gttgtgggtt ggggagggtt 600
   gttgttttta gtgttatgtt ttaaattttt tatgtttatt tttattgagg ataagggaat 660
   tttatttttt tttgttgttt tttatgtttt tgggatttaa gtagtgtttt ttttaaaatt 720
   tattttattt ttaattgaat tttattttta atttagggtt tttttttgtt ttggatttat 780
   tatggggata aggattattg tgattggttg ggaagatagg taaattattt gtgaaaatag 840
   aaatagtaat ttattttaaa aggaaattgt ttattgtgaa taagttagag aaagagattg 900
   aaagaaaaat gaatagaatt gttaggattt gtaagaaaat gttaaaatga tatttattat 960
   ttgtagtatt agaattatag agggtgaagt gatagtatta gggaaatagg atagttagag 1020
   tgaaattatg tgaaaatata ttttgttttg aaagtagtaa gatatatagt tttattagtt 1080
   ataatttatg gttttaagat gtttggagtt gagaaaatag atgaaaggta tttttaagga 1140
   tatgttttta tagtagtgga aagtgtatgg tttttaatat ttattaataa tatatgtttt 1200
   taatagaatt atgtttttat ggttttatat attggtaagt taaggatagt tttttttaaa 1260
   ttaatagaat gataaaagtt attatgtttt tagtttattt gtataaaggt atagattaat 1320
   tttagttttt atatagataa gatttttata taagaaaaat tagattaggt taaggtttat 1380
   gtttgtaatt ttagtatttt gggaggttga gttggttaga ttatttgagt ttaggatttt 1440
   gagattagtt taagtaatat tagaaaattt tattattata aaaaataaat aaaaaattat 1500
   tgaaaaatta gttgggtatg gtggtatgta tttatattta tagttattta ggaggttgag 1560
   gtgggaggag tgtttgagtt taggaggtta aggttgtagt gagttttgat aatattattg 1620
   tattttagtt ttggtgatag agtgagatgt tgttttaagt tttaataatt gaatttaaaa 1680
   atgtataaat ttattaattg aatttattat attaatatat gaatgggaaa aaatgttgtg 1740
   gtaattgaaa tagatgtatt gtttgatgaa aattaatata tatttaaatg aatattttta 1800
   gtaaatttgg aatagaatgg aatgatttat tttgataaag tttatttata aaaaaaggag 1860
   agtgaatagg atggtgaaat gttgaaattt tttggttttt tattagggat aagataagga 1920
   tgtttattgt tattattgta atgggtgggt ttgtgtaatg ttataaaatt agaaaaggaa 1980
   ataaaatttt ttataatggt aatgatgggt ttgttatggt ggtttatgtt agtaatttta 2040
   gtattgtggg agatgatgtg ggtgtattat ttgtatttaa aagtttaata atagtttggg 2100
   aaatatggta aaattttgtt tttataaaaa aatataaaag ggaaaagaaa aaaaaatggt 2160
   ggaaataaaa ttgtttttat tttaggatga tatgtttttg tatattgaag attgaaaatg 2220
   atttagaagt aaattttaga atttataagt atattttata aggttattgg atagaaaatt 2280
   aatatgtaag aattatgttt ttattgggtg tggaggttta tgtttgtaat tttagtattt 2340
   tgggaggtta aggtaggtga attatgaggt taggagttta agagtatttt ggttaagatg 2400
   gtgaaagttt atttttagta aaaatataaa atttagttag gtgtgttggt gggtgtttat 2460
   aattttagtt atttgggagg ttgaggtaga taattgtttg 2500

<210> 154

<211> 2500
<212> DNA
<213> Artificial Sequence

<220>
<223> chemically treated genomic DNA (Homo sapiens)

<400> 154

taaatttatt tgtatttttg tgagggtgtg ttaagggtat gatgaaattt attattttat 60
tgatttaaag aaaattattt ttgatttttt agtgtgtaag tttatgaaag tataattatg 120
ttgaaagtat atattgttat gggtgttgag aattttgtat tttttgttgt tgtgggagta 180
tgtttttgga ggtatttttt gtttgttttt ttaattttaa atattttagg attatgggtt 240
gtgattggta aggaatgtgt tttgtgagtt ttaagatgga gttgattttt atatggtgtt 300
attttggttt ttttgttttt ttaatattgg tatttttttt tttgtgattt tgatgttata 360
aatgatagat attgttttag tattttttta tgggttttag agattgtatt tatttttttt 420
ttagtttttt tttttgattt gtttatattt aataattttt ttttggggta ggttgttatt 480
tttgtttttg taggtggttt atttgttttt ttaggtagtt attgtggttt ttgtttttat 540
ggtggggttg gggtaagaga gggttttgga ggggaggggg gtttagttga agatggagtg 600
agttttgagg ggagtattat ttgagtttta gaggtatagg aaatagtaga gggaggttag 660
attttattat ttttagtggg gatgggaatt gagggttggg ggtgtggggt tgggaatggt 720
agtttttttt aatttgtagt tgtatatgtt ttttggtttt tgttttagtg tgtatgttta 780
ttgggtgttt tttgtttagt tgttttattt atgtggagaa tgttagggag ttgtgagggt 840
gtgtggtttt gtttttgttg tttggaatat ttttttttta ttgagattta tttggtaggt 900
ttgtaggtta gtttttttgg ggtttgaagt gtgagtgagg gtgaagagta ggtagtgtgt 960
tttgggtgag tttgttgggt tttgttttgt ggtttgtggt ttttgaggga gaagggtttt 1020
gaggtttttg aaagggaagg ggtttttggt ttttgaaaga gaagggtttt gaggttgttt 1080
tttttttttt aaaggttgtg aggttattat ttgttttgtg gttgtgaagg ggttaggata 1140
aggaggaagg tgggttatgg aggggaggtg gttaggggtt taggtgaaga aggggtgatt 1200
gttgggtgtg ttgttagagg gatggaagtt tggaggtgtt aggaatattt gatatagggg 1260
agatttttga atgaggtttt ggataggtgt gaggagggtg ataaagaagg gatttggtat 1320
ttgggaagat tgtgggttgg tgagtgtttt tgagttttgt ggagtgggga gttttgagtg 1380
agaagtattg taagatttta tttttgttat ggaaggtttg gtaatagtgg gaaggattgg 1440
gagaggttgt gtggtttaat taaatttgat ttgagagaag tgaatggttt tagtaagtgg 1500
gagtgtgttt aaagtagtaa ttatgagaat tatgatttat taatgttttt atgtggagtt 1560
tatttgtgaa attaaatttt attagaaatg atttttgtta gtggggtgtt atggtttgtg 1620
tttgtagttt tagttatttg ggatgttgag gtgggaggat tttttgagta ggaggttgat 1680
gttgtagtga gttgtgatta tgttgttgta ttttgttaga gtaatatagg gagattgtgg 1740
gattaaaaga aatttagaaa aaaaatgttt tttgtgtttt gttatatgtt ttaagatgat 1800
tgttttgtta gtttggttag tataagtggt tttgtaggta tttagaaaag gtatatatat 1860
atgtttaatt ttgggattta ttttgaaagt atttttaaaa ttaaaatgat aagttaatat 1920
ttatttatgg aggtgatgga atatagtagt ttttttgagg gtatgttttt aattatggtt 1980
gtttgttttt agtgtgaaat atgagttggt gaggaagatt gatttattgg tttagattaa 2040
gattttatgt agagtttttt gaaatgattg ggtttatgtt ggtgagtgtt taaatgttaa 2100
tttgttgttt tttattagta gaaattaatt tttgtgatag tattgttgta ttagtatgga 2160
aatgttgata aaggtttttt ttgtttataa aaaatgatga tggtgtttta tgaaggaaat 2220
attgattttg gagaattttt ttttttttag tgttttttag tttttgttta tgattttttt 2280
tttaggtttt gtttgttaat gatagattgt atatatgtat tttaatattg agtataatag 2340
tttttaaagt ttttgtgtgt tatttttttt atagtaattt ttttgtgggt tgagtaattt 2400
tattgggtat agagtataga gttggagaaa tgtttttagg tttagttagg attagaaata 2460
gttatgtatt ttgtgtatat atgtaaaatt ttgtattaat 2500

<210> 155
<211> 2500
<212> DNA

<213> Artificial Sequence

<220>
<223> chemically treated genomic DNA (Homo sapiens)

<400> 155

attgatataa aattttatat atatatatag aatatatagt tatttttggt tttaattaag 60
tttaaagata tttttttaat tttatgtttt atgtttaata aggttatttg atttatagga 120
aggttattat gagaaaagtg atgtatgagg attttggagg ttattatatt tagtgttgga 180
atatatgtgt ataatttgtt attaataaat aaagtttgag aaagaagtta tgggtaaaag 240
ttgaagaata ttagaggaaa aaaaattttt tagaattaat gtttttttta tgagatgtta 300
ttattatttt ttatgagtag gaaagatttt tattagtatt tttatattaa tgtaataata 360
ttgttataaa aattaatttt tgttaataga aaataatgaa ttaatgttta agtatttatt 420
agtataggtt taattatttt agggggtttt atatagagtt ttggtttaga ttgataggtt 480
gatttttttt gttaatttat attttatatt gaaaatagat aattgtgatt gggagtatgt 540
ttttgaaagg gttgttatat tttattattt ttatggataa atgttaattt gttgttttaa 600
ttttgaaaat atttttaaaa taagttttag agttaagtat atgtgtgtat tttttttgag 660
tgtttataaa gttatttatg ttggttaagt tggtagagta attattttaa ggtgtgtgat 720
aaaatgtaga ggatattttt tttttaaatt ttttttggtt ttgtagtttt tttgtgttgt 780
tttggtagag tgtagtggtg tgattatagt ttattgtagt attgattttt tgtttaaggg 840
atttttttat tttagtgttt tgagtaattg ggattatagg tgtaggttat ggtgttttat 900
tggtagaggt tatttttgat gaggtttagt tttataagtg gattttatat gaaaatatta 960
gtgaattata atttttgtga ttgttatttt gggtatattt ttatttatta gagttattta 1020
ttttttttaa attaagtttg attggattgt atagtttttt ttagtttttt ttattgttgt 1080
tagatttttt atggtggagg tgagattttg tgatgttttt tatttggggt tttttatttt 1140
ataaagttta ggggtattta ttagtttgta gtttttttag gtgttaggtt ttttttttat 1200
tgtttttttt gtatttgttt gggattttat ttagggattt tttttgtatt gggtattttt 1260
ggtatttttg gatttttatt tttttgatag tatatttagt aattgttttt tttttatttg 1320
agtttttgat tgtttttttt ttatggttta tttttttttt tgttttggtt tttttatgat 1380
tataaagtag atggtggttt tatagttttt gagagaagga ggatgatttt aaggtttttt 1440
ttttttagag gttaggagtt tttttttttt tagaagtttt gaggtttttt ttttttagag 1500
gttatagatt atgaagtagg atttaatgga tttatttgaa gtatattgtt tgttttttat 1560
ttttatttat attttagatt ttgggaggat tggtttgtag atttattaga tgaattttag 1620
tagaggaaaa atattttaga tgataggaat gagattatat atttttatag ttttttggtg 1680
ttttttatgt gggtaaagtg gttgagtaga agatgtttag tggatatgtg tattgaggtg 1740
ggagttaagg agtatgtata gttgtgggtt ggggaaggtt gttgttttta gttttatgtt 1800
tttaattttt gatttttatt tttattgagg ataatggaat ttgatttttt tttgttgttt 1860
tttatgtttt tgggatttaa gtagtgtttt ttttaaaatt tattttattt ttaattaaat 1920
tttttttttt tttagggttt ttttttgttt tggttttatt atggggataa ggattatggt 1980
gattgtttga gaagataggt aaattatttg tgaaaataga aatagtaatt tattttaaaa 2040
agaaattgtt aaatgtgaat aagttagaga aagagattga aagaaaaatg aatataattt 2100
ttaggattta taagaaaata ttaaaatgat atttattatt tgtagtatta gaattataga 2160
aagagaagtg ttagtattag agaaatagga gagttagagt gatattatgt gaaaattaat 2220
tttattttga aatttgtaag atatattttt tattagttat aatttatggt tttaagatgt 2280
ttggagttga gaaaatagat gaaaggtatt tttaaggata tgtttttata gtagtagaaa 2340
gtgtagggtt tttaatattt ataataatat atgtttttaa tataattatg tttttatgga 2400
tttatatatt ggaaagttaa ggatagtttt ttttaaatta atagaatgat aaattttatt 2460
atgtttttag tatattttta taaagatata gataagttta 2500

<210> 156
<211> 2500
<212> DNA
<213> Artificial Sequence

<220>
<223> chemically treated genomic DNA (Homo sapiens)

<400> 156

aagtaattgt ttgttttagt tttttgagta gttgggatta taggtattta ttagtatgtt 60
tggttaagtt ttgtgttttt attagagatg ggtttttgtt attttggttg gggtgttttt 120
gaatttttga ttttgtgatt tatttgtttt ggttttttaa agtgttggga ttataggtat 180
gagtttttgt gtttggtgga gatataattt ttatatattg gttttttatt tagtggtttt 240

gtgaaatatg tttgtgaatt ttaaagttta tttttaggtt gtttttagtt tttaatatat 300
agaaatatat tattttggaa taagagtagt tttgtttttg ttattttttt tttttttttt 360
ttttgtattt ttttgtagag atggggtttt gttatgtttt ttgggttgtt gttgaatttt 420
tgagtgtaag tgatgtattt atgttatttt ttatagtgtt gggattattg gtgtgggtta 480
ttgtggtggg tttgttgttg ttattgtaaa gagtttattt tttttttttg attttatggt 540
attgtgtaga tttatttgtt ataatggtga tagtggatat ttttgtttta tttttgatga 600
gaaattgaaa aattttaata ttttattatt ttatttattt tttttttttt gtagatggat 660
tttattagag tgagttattt tattttgttt taaatttgtt gagagtattt atttgaatat 720
atgttgattt ttattaaata gtgtatttat tttgattatt atagtgtttt tttttattta 780
tgtgttaata tagtgaattt gattgataaa tttgtatgtt tttaggtttg attattaaaa 840
tttgagatag tgttttattt tgttattgag gttggagtgt agtggtgtta ttagagtttg 900
ttgtagtttt gatttttggg gtttaggtgt ttttttattt ttagtttttt gagtagttgt 960
gagtataggt atatgttatt atgtttagtt aattttttga tggttttttg tttgtttttt 1020
gtagtgatga gattttttga tgttgtttag gttggttttg aagttttgag tttaggtgat 1080
ttggttagtt tagtttttta aaatattagg attataggtg tgagttttgg tttggtttgg 1140
ttttttttat ataggggttt tatttatata aagattaaag ttaatttgtg tttttgtgtg 1200
ggtgggttaa gagtatgatg atttttatta ttttattgat ttaaagaaaa ttatttttga 1260
tttattagtg tgtaaggtta tgaaagtata attttgttga aagtatatat tgttaatggg 1320
tgttgggaat tgtgtatttt ttgttgttgt gggagtatgt ttttggaggt attttttatt 1380
tgttttttta attttaaata ttttaggatt atgggttgtg attggtagga ttatgtattt 1440
tgttgttttt aagatggagt atatttttat gtggttttat tttggttgtt ttgttttttt 1500
aatattgtta ttttattttt tgtgattttg atgttataaa tgatagatat tgttttagta 1560
tttttttatg ggttttagtg atttatttta tttttttttt agtttttttt tttgatttgt 1620
ttataatgaa taattttttt ttgggatagg ttgttatttt tgtttttgta ggtggtttat 1680
ttgttttttt agttagttat agtggttttt gtttttatgg tgggtttggg gtaagagagg 1740
gttttgggtt gggggtgggg tttagttgaa gatggggtga gttttgaggg gagtattatt 1800

tgagttttag aggtatagga aatagtagag ggaggtggga tttttttatt tttaatgagg 1860
atgggtatgg agggtttggg gtgtggtgtt gggaatggta gtttttttta gtttatagtt 1920
gtgtatgttt tttgggtttt tgttttagtg tgtatgttta ttgggtgttt tttgtttggt 1980
ttttttgttt atgtgaagaa tgttagggag ttgtgaggta gtgttgtgtg gtttttgttg 2040
tttggatttt ttttttttta ttgagattta tttggtaggt ttgtaggtta gttattttgg 2100
gggttgaagt gtgagtgagg gtggagaggg ttttgggtgg gttaggtggg ttttgttttt 2160
tggtttgtgg tttttgaggg agaagggtta tgaggttgtt tttttttttt ttataggttg 2220
tgaggttatt ggtggttttg tggttgtgaa ggggtttgga tggggaggaa ggtgggttgt 2280
ggaggggagg tggttagggg tttaggtgaa gatggggtga gtgttgttgg ggggatggaa 2340
gttttgaggt gttgggaatt tttgatgata tagggtagat tttttgaatg gggtttttgg 2400
tgggggtgag gtgggtggtg aagaaggggt ttggtatttg ggaaggttgt ggtttggtga 2460
gtgttttttt tagtggtgtg gagtgtggag tgtttgagtg 2500

<210> 157
<211> 2500
<212> DNA
<213> Artificial Sequence

<220>
<223> chemically treated genomic DNA (Homo sapiens)

<400> 157

tatttaggtg ttttgtattt tatattgttg gggggggtgt ttattaggtt gtagtttttt 60
taggtgttag gttttttttt tattgtttgt tttgtttttg ttggaggttt tatttaggga 120
atttgttttg tgttgttggg ggtttttggt attttgggat ttttattttt ttaatagtat 180
ttattttatt tttatttgag tttttgattg tttttttttt atggtttatt tttttttttg 240
tttaggtttt tttatgatta tgaagttgtt ggtggttttg tagtttgtga agggaaggag 300
gatgattttg tggttttttt tttttggagg ttatagatta ggaagtggga tttgtttgat 360
ttatttaagg ttttttttat ttttatttat attttagttt ttgggatgat tggtttgtag 420
atttattaga tgaattttag tagaggaaaa agagtttgga tggtaggaat tatatagtat 480
tgttttatag ttttttggtg ttttttatgt gggtgaaggg gttgggtaga agatgtttag 540
tgaatatgtg tattgaggtg ggagtttagg gagtatgtgt ggttgtgggt tggggagggt 600
tgttgttttt agtgttatgt tttaaatttt ttatgtttat ttttattgag gataagggaa 660
ttttattttt ttttgttgtt ttttatgttt ttgggattta agtagtgttt tttttaaaat 720
ttattttatt tttaattgaa ttttattttt aatttagggt ttttttttgt tttggattta 780
ttatggggat aaggattatt gtgattggtt gggaagatag gtaaattatt tgtgaaaata 840

gaaatagtaa tttattttaa aaggaaattg tttattgtga ataagttaga gaaagagatt 900
gaaagaaaaa tgaatagaat tgttaggatt tgtaagaaaa tgttaaaatg atatttatta 960
tttgtagtat tagaattata gagggtgaag tgatagtatt agggaaatag gatagttaga 1020
gtgaaattat gtgaaaatat attttgtttt gaaagtagta agatatatag ttttattagt 1080
tataatttat ggttttaaga tgtttggagt tgagaaaata gatgaaaggt atttttaagg 1140
atatgttttt atagtagtgg aaagtgtatg gtttttaata tttattaata atatatgttt 1200
ttaatagaat tatgttttta tggttttata tattggtaag ttaaggatag ttttttttaa 1260
attaatagaa tgataaaagt tattatgttt ttagtttatt tgtataaagg tatagattaa 1320
ttttagtttt tatatagata agatttttat ataagaaaaa ttagattagg ttaaggttta 1380
tgtttgtaat tttagtattt tgggaggttg agttggttag attatttgag tttaggattt 1440
tgagattagt ttaagtaata ttagaaaatt ttattattat aaaaaataaa taaaaaatta 1500
ttgaaaaatt agttgggtat ggtggtatgt atttatattt atagttattt aggaggttga 1560
ggtgggagga gtgtttgagt ttaggaggtt aaggttgtag tgagttttga taatattatt 1620
gtattttagt tttggtgata gagtgagatg ttgttttaag ttttaataat tgaatttaaa 1680
aatgtataaa tttattaatt gaatttatta tattaatata tgaatgggaa aaaatgttgt 1740
ggtaattgaa atagatgtat tgtttgatga aaattaatat atatttaaat gaatattttt 1800
agtaaatttg gaatagaatg gaatgattta ttttgataaa gtttatttat aaaaaaagga 1860
gagtgaatag gatggtgaaa tgttgaaatt ttttggtttt ttattaggga taagataagg 1920
atgtttattg ttattattgt aatgggtggg tttgtgtaat gttataaaat tagaaaagga 1980
aataaaattt tttataatgg taatgatggg tttgttatgg tggtttatgt tagtaatttt 2040
agtattgtgg gagatgatgt gggtgtatta tttgtattta aaagtttaat aatagtttgg 2100
gaaatatggt aaaattttgt ttttataaaa aaatataaaa gggaaaagaa aaaaaaatgg 2160
tggaaataaa attgttttta ttttaggatg atatgttttt gtatattgaa gattgaaaat 2220
gatttagaag taaattttag aatttataag tatattttat aaggttattg gatagaaaat 2280
taatatgtaa gaattatgtt tttattgggt gtggaggttt atgtttgtaa ttttagtatt 2340
ttgggaggtt aaggtaggtg aattatgagg ttaggagttt aagagtattt tggttaagat 2400
ggtgaaagtt tatttttagt aaaaatataa aatttagtta ggtgtgttgg tgggtgttta 2460
taattttagt tatttgggag gttgaggtag ataattgttt 2500

<210> 158
<211> 2500
<212> DNA
<213> Artificial Sequence

<220>
<223> chemically treated genomic DNA (Homo sapiens)

<400> 158

gtttgtttta gttttttgag tagttgggat tataggtatt tattagtatg tttggttaag 60
ttttgtgttt ttattagaga tgggtttttg ttattttggt tggggtgttt ttgaattttt 120
gattttgtga tttatttgtt ttggtttttt aaagtgttgg gattataggt atgagttttt 180
gtgtttggtg gagatataat ttttatatat tggtttttta tttagtggtt ttgtgaaata 240
tgtttgtgaa ttttaaagtt tatttttagg ttgtttttag tttttaatat atagaaatat 300
attattttgg aataagagta gttttgtttt tgttattttt tttttttttt ttttttgtat 360
ttttttgtag agatggggtt ttgttatgtt ttttgggttg ttgttgaatt tttgagtgta 420
agtgatgtat ttatgttatt ttttatagtg ttgggattat tggtgtgggt tattgtggtg 480
ggtttgttgt tgttattgta aagagtttta tttttttttt tgattttatg gtattgtgta 540
gatttatttg ttataatggt gatagtggat atttttgttt tatttttgat gagaaattga 600
aaaattttaa tattttatta ttttatttat tttttttttt ttgtagatgg attttattag 660
agtgagttat tttattttgt tttaaatttg ttgagagtat ttatttgaat atatgttgat 720
ttttattaaa tagtgtattt attttgatta ttatagtgtt tttttttatt tatgtgttaa 780
tatagtgaat ttgattgata aatttgtatg tttttaggtt tgattattaa aatttgagat 840
agtgttttat tttgttattg aggttggagt gtagtggtgt tattagagtt tgttgtagtt 900
ttgatttttt gggtttaggt gtttttttta ttttagtttt ttgagtagtt gtgagtatag 960
gtatatgtta ttatgtttag ttaatttttt gatggttttt tgtttgtttt ttgtagtgat 1020
gagatttttt gatgttgttt aggttggttt tgaagttttg agtttaggtg atttggttag 1080
tttagttttt taaaatatta ggattatagg tgtgagtttt ggtttggttt ggtttttttt 1140
atataggggt tttatttata taaagattaa agttaatttg tgtttttgtg tgggtgggtt 1200
aagagtatga tgatttttat tattttattg atttaaagaa aattattttt gatttattag 1260
tgtgtaaggt tatgaaagta taattttgtt gaaagtatat attgttaatg ggtgttggga 1320
attgtgtatt ttttgttgtt gtgggagtat gtttttggag gtatttttta tttgtttttt 1380
taattttaaa tattttagga ttatgggttg tgattggtag gattatgtat tttgttgttt 1440
ttaagatgga gtatattttt atgtggtttt attttggttg ttttgttttt ttaatattgt 1500

tattttattt tttgtgattt tgatgttata aatgatagat attgttttag tattttttta 1560
tgggttttag tgattttatt tatttttttt ttagtttttt tttttgattt gtttataatg 1620
aataattttt ttttgggata ggttgttatt tttgtttttg taggtggttt atttgttttt 1680
ttagttagtt atagtggttt ttgtttttat ggtgggtttg gggtaagaga gggttttggg 1740
ttgggggtgg ggtttagttg aagatggggt gagttttgag gggagtatta tttgagtttt 1800
agaggtatag gaaatagtag agggaggtgg gattttttta tttttaatga ggatgggtat 1860
ggagggtttg gggtgtggtg ttgggaatgg tagttttttt tagtttatag ttgtgtatgt 1920
tttttgggtt tttgttttag tgtgtatgtt tattgggtgt tttttgtttg gtttttttgt 1980
ttatgtgaag aatgttaggg agttgtgagg tagtgttgtg tggtttttgt tgtttggatt 2040
tttttttttt tattgagatt tatttggtag gtttgtaggt tagttatttt gggggttgaa 2100
gtgtgagtga gggtggagag ggttttgggt gggttaggtg ggttttgttt tttggtttgt 2160
ggtttttgag ggagaagggt tatgaggttg tttttttttt ttttataggt tgtgaggtta 2220
ttggtggttt tgtggttgtg aaggggtttg gatggggagg aaggtgggtt gtggagggga 2280
ggtggttagg ggtttaggtg aagatggggt gagtgttgtt ggggggatgg aagttttgag 2340
gtgttgggaa tttttgatga tatagggtag attttttgaa tggggttttt ggtgggggtg 2400
aggtgggtgg tgaagaaggg gtttggtatt tgggaaggtt gtggtttggt gagtgttttt 2460
tttagtggtg tggagtgtgg agtgtttgag tgagaagtat 2500

<210> 159
<211> 2500
<212> DNA
<213> Artificial Sequence

<220>
<223> chemically treated genomic DNA (Homo sapiens)

<400> 159

gtgtttttta tttaggtgtt ttgtatttta tattgttggg gggggtgttt attaggttgt 60
agttttttta ggtgttaggt ttttttttta ttgtttgttt tgtttttgtt ggaggtttta 120
tttagggaat ttgttttgtg ttgttggggg tttttggtat tttgggattt ttattttttt 180
aatagtattt atttttattt tatttgagtt tttgattgtt ttttttttat ggtttatttt 240
tttttttgtt taggtttttt tatgattatg aagttgttgg tggttttgta gtttgtgaag 300
ggaaggagga tgattttgtg gttttttttt tttggaggtt atagattagg aagtgggatt 360
tgtttgattt atttaaggtt ttttttattt ttatttatat tttagttttt gggatgattg 420
gtttgtagat ttattagatg aattttagta gaggaaaaag agtttggatg gtaggaatta 480
tatagtattg ttttatagtt ttttggtgtt ttttatgtgg gtgaaggggt tgggtagaag 540
atgtttagtg aatatgtgta ttgaggtggg agtttaggga gtatgtgtgg ttgtgggttg 600
gggagggttg ttgtttttag tgttatgttt taaatttttt atgtttattt ttattgagga 660

taagggaatt ttattttttt ttgttgtttt ttatgttttt gggatttaag tagtgttttt 720
tttaaaattt attttatttt taattgaatt ttatttttaa tttagggttt ttttttgttt 780
tggatttatt atggggataa ggattattgt gattggttgg gaagataggt aaattatttg 840
tgaaaataga aatagtaatt tattttaaaa ggaaattgtt tattgtgaat aagttagaga 900
aagagattga aagaaaaatg aatagaattg ttaggatttg taagaaaatg ttaaaatgat 960
atttattatt tgtagtatta gaattataga gggtgaagtg atagtattag ggaaatagga 1020
tagttagagt gaaattatgt gaaaatatat tttgttttga aagtagtaag atatatagtt 1080
ttattagtta taatttatgg ttttaagatg tttggagttg agaaaataga tgaaaggtat 1140
ttttaaggat atgtttttat agtagtggaa agtgtatggt ttttaatatt tattaataat 1200
atatgttttt aatagaatta tgtttttatg gttttatata ttggtaagtt aaggatagtt 1260
ttttttaaat taatagaatg ataaaagtta ttatgttttt agtttatttg tataaaggta 1320
tagattaatt ttagttttta tatagataag atttttatat aagaaaaatt agattaggtt 1380
aaggtttatg tttgtaattt tagtattttg ggaggttgag ttggttagat tatttgagtt 1440
taggattttg agattagttt aagtaatatt agaaaatttt attattataa aaaataaata 1500
aaaaattatt gaaaaattag ttgggtatgg tggtatgtat ttatatttat agttatttag 1560
gaggttgagg tgggaggagt gtttgagttt aggaggttaa ggttgtagtg agttttgata 1620
atattattgt attttagttt tggtgataga gtgagatgtt gttttaagtt ttaataattg 1680
aatttaaaaa tgtataaatt tattaattga atttattata ttaatatatg aatggaaaaa 1740
aatgttgtgg taattgaaat agatgtattg tttgatgaaa attaatatat atttaaatga 1800
atatttttag taaatttgga atagaatgga atgatttatt ttgataaagt ttatttataa 1860
aaaaaggaga gtgaatagga tggtgaaatg ttgaaatttt ttggtttttt attagggata 1920
agataaggat gtttattgtt attattgtaa tgggtgggtt tgtgtaatgt tataaaatta 1980
gaaaaggaaa taaaattttt tataatggta atgatgggtt tgttatggtg gtttatgtta 2040
gtaattttag tattgtggga gatgatgtgg gtgtattatt tgtatttaaa agtttaataa 2100

tagtttggga aatatggtaa aattttgttt ttataaaaaa atataaaagg gaaaagaaaa 2160
aaaaatggtg gaaataaaat tgtttttatt ttaggatgat atgtttttgt atattgaaga 2220
ttgaaaatga tttagaagta aattttagaa tttataagta tattttataa ggttattgga 2280
tagaaaatta atatgtaaga attatgtttt tattgggtgt ggaggtttat gtttgtaatt 2340
ttagtatttt gggaggttaa ggtaggtgaa ttatgaggtt aggagtttaa gagtattttg 2400
gttaagatgg tgaaagttta tttttagtaa aaatataaaa tttagttagg tgtgttggtg 2460
ggtgtttata attttagtta tttgggaggt tgaggtagat 2500

<210> 160
<211> 2500
<212> DNA
<213> Artificial Sequence

<220>
<223> chemically treated genomic DNA (Homo sapiens)

<400> 160

agtaattgtt tgttttagtt ttttgagtag ttgggattat aggtatttat tagtatgttt 60
ggttaagttt tgtgttttta ttagagatgg gtttttgtta ttttggttgg ggtgtttttg 120
aatttttgat tttgtgattt atttgttttg gttttttaaa gtgttgggat tataggtatg 180
agtttttgtg tttggtggag atataatttt tatatattgg ttttttattt agtggttttg 240
tgaaatatgt ttgtgaattt taaagtttat ttttaggttg tttttagttt ttaatatata 300
gaaatatatt attttggaat aagagtagtt ttgtttttgt tatttttttt tttttttttt 360
tttgtatttt tttgtagaga tggggttttg ttatgttttt tgggttgttg ttgaattttt 420
gagtgtaagt gatgtattta tgttattttt tatagtgttg ggattattgg tgtgggttat 480
tgtggtgggt ttgttgttgt tattgtaaag agttttattt ttttttttga ttttatggta 540
ttgtgtagat ttatttgtta taatggtgat agtggatatt tttgttttat ttttgatgag 600
aaattgaaaa attttaatat tttattattt tatttatttt tttttttttg tagatggatt 660
ttattagagt gagttatttt attttgtttt aaatttgttg agagtattta tttgaatata 720
tgttgatttt tattaaatag tgtatttatt ttgattatta tagtgttttt ttttatttat 780
gtgttaatat agtgaatttg attgataaat ttgtatgttt ttaggtttga ttattaaaat 840
ttgagatagt gttttatttt gttattgagg ttggagtgta gtggtgttat tagagtttgt 900
tgtagttttg atttttttggg tttaggtgtt ttttttattt tagtttttttg agtagttgtg 960
agtataggta tatgttatta tgtttagtta attttttgat ggttttttgt ttgttttttg 1020
tagtgatgag attttttgat gttgtttagg ttggttttga agttttgagt ttaggtgatt 1080
tggttagttt agttttttaa aatattagga ttataggtgt gagttttggt ttggtttggt 1140
tttttttata taggggtttt atttatataa agattaaagt taatttgtgt ttttgtgtgg 1200
gtgggttaag agtatgatga tttttattat tttattgatt taaagaaaat tatttttgat 1260
ttattagtgt gtaaggttat gaaagtataa ttttgttgaa agtatatatt gttaatgggt 1320
gttgggaatt gtgtattttt tgttgttgtg ggagtatgtt tttggaggta ttttttattt 1380
gtttttttaa ttttaaatat tttaggatta tgggttgtga ttggtaggat tatgtatttt 1440
gttgttttta agatggagta tatttttatg tggttttatt ttggttgttt tgttttttta 1500
atattgttat tttatttttt gtgattttga tgttataaat gatagatatt gttttagtat 1560
ttttttatgg gttttagtga ttttatttat ttttttttta gttttttttt ttgatttgtt 1620
tataatgaat aatttttttt tgggataggt tgttattttt gtttttgtag gtggtttatt 1680
tgttttttta gttagttata gtggtttttg tttttatggt gggtttgggg taagagaggg 1740
ttttgggttg ggggtggggt ttagttgaag atggggtgag ttttgagggg agtattattt 1800
gagttttaga ggtataggaa atagtagagg gaggtgggat ttttttattt ttaatgagga 1860
tgggtatgga gggtttgggg tgtggtgttg ggaatggtag ttttttttag tttatagttg 1920
tgtatgtttt ttgggttttt gttttagtgt gtatgtttat tgggtgtttt ttgtttggtt 1980
tttttgttta tgtgaagaat gttagggagt tgtgaggtag tgttgtgtgg tttttgttgt 2040
ttggattttt ttttttttat tgagatttat ttggtaggtt tgtaggttag ttattttggg 2100
ggttgaagtg tgagtgaggg tggagagggt tttgggtggg ttaggtgggt tttgtttttt 2160
ggtttgtggt ttttgaggga gaagggttat gaggttgttt tttttttttt tataggttgt 2220
gaggttattg gtggttttgt ggttgtgaag gggtttggat ggggaggaag gtgggttgtg 2280
gaggggaggt ggttaggggt ttaggtgaag atggggtgag tgttgttggg gggatggaag 2340
ttttgaggtg ttgggaattt ttgatgatat agggtagatt ttttgaatgg ggtttttggt 2400
gggggtgagg tgggtggtga agaaggggtt tggtatttgg gaaggttgtg gtttggtgag 2460
tgtttttttt agtggtgtgg agtgtggagt gtttgagtga 2500

<210> 161
<211> 2500
<212> DNA

<213> Artificial Sequence

<220>
<223> chemically treated genomic DNA (Homo sapiens)

<400> 161

ttatttaggt gttttgtatt ttatattgtt ggggggggtg tttattaggt tgtagttttt 60
ttaggtgtta ggtttttttt ttattgtttg ttttgttttt gttggaggtt ttatttaggg 120
aatttgtttt gtgttgttgg gggtttttgg tattttggga tttttatttt tttaatagta 180
tttattttat ttttatttga gtttttgatt gttttttttt tatggtttat tttttttttt 240
gtttaggttt ttttatgatt atgaagttgt tggtggtttt gtagtttgtg aagggaagga 300
ggatgatttt gtggtttttt ttttttggag gttatagatt aggaagtggg atttgtttga 360
tttatttaag gtttttttta tttttattta tattttagtt tttgggatga ttggtttgta 420
gatttattag atgaatttta gtagaggaaa aagagtttgg atggtaggaa ttatatagta 480
ttgttttata gttttttggt gttttttatg tgggtgaagg ggttgggtag aagatgttta 540
gtgaatatgt gtattgaggt gggagtttag ggagtatgtg tggttgtggg ttggggaggg 600
ttgttgtttt tagtgttatg ttttaaattt tttatgttta tttttattga ggataaggga 660
attttatttt tttttgttgt tttttatgtt tttgggattt aagtagtgtt ttttttaaaa 720
tttattttat ttttaattga attttatttt taatttaggg tttttttttg ttttggattt 780
attatgggga taaggattat tgtgattggt tgggaagata ggtaaattat ttgtgaaaat 840
agaaatagta atttatttta aaaggaaatt gtttattgtg aataagttag agaaagagat 900
tgaaagaaaa atgaatagaa ttgttaggat ttgtaagaaa atgttaaaat gatatttatt 960
atttgtagta ttagaattat agagggtgaa gtgatagtat tagggaaata ggatagttag 1020
agtgaaatta tgtgaaaata tattttgttt tgaaagtagt aagatatata gttttattag 1080
ttataattta tggttttaag atgtttggag ttgagaaaat agatgaaagg tatttttaag 1140
gatatgtttt tatagtagtg gaaagtgtat ggtttttaat atttattaat aatatatgtt 1200
tttaatagaa ttatgttttt atggttttat atattggtaa gttaaggata gtttttttta 1260
aattaataga atgataaaag ttattatgtt tttagtttat ttgtataaag gtatagatta 1320
attttagttt ttatatagat aagattttta tataagaaaa attagattag gttaaggttt 1380
atgtttgtaa ttttagtatt ttgggaggtt gagttggtta gattatttga gtttaggatt 1440
ttgagattag tttaagtaat attagaaaat tttattatta taaaaaataa ataaaaaatt 1500
attgaaaaat tagttgggta tggtggtatg tatttatatt tatagttatt taggaggttg 1560
aggtgggagg agtgtttgag tttaggaggt taaggttgta gtgagttttg ataatattat 1620
tgtattttag ttttggtgat agagtgagat gttgttttaa gttttaataa ttgaatttaa 1680
aaatgtataa atttattaat tgaatttatt atattaatat atgaatggga aaaaatgttg 1740
tggtaattga aatagatgta ttgtttgatg aaaattaata tatatttaaa tgaatatttt 1800
tagtaaattt ggaatagaat ggaatgattt attttgataa agtttattta taaaaaaagg 1860
agagtgaata ggatggtgaa atgttgaaat tttttggttt tttattaggg ataagataag 1920
gatgtttatt gttattattg taatgggtgg gtttgtgtaa tgttataaaa ttagaaaagg 1980
aaataaaatt ttttataatg gtaatgatgg gtttgttatg gtggtttatg ttagtaattt 2040
tagtattgtg ggagatgatg tgggtgtatt atttgtattt aaaagtttaa taatagtttg 2100
ggaaatatgg taaaattttg tttttataaa aaaatataaa agggaaaaga aaaaaaaatg 2160
gtggaaataa aattgttttt attttaggat gatatgtttt tgtatattga agattgaaaa 2220
tgatttagaa gtaaatttta gaatttataa gtatatttta taaggttatt ggatagaaaa 2280
ttaatatgta agaattatgt ttttattggg tgtggaggtt tatgtttgta attttagtat 2340
tttgggaggt taaggtaggt gaattatgag gttaggagtt taagagtatt ttggttaaga 2400
tggtgaaagt ttatttttag taaaaatata aaatttagtt aggtgtgttg gtgggtgttt 2460
ataattttag ttatttggga ggttgaggta gataattgtt 2500

<210> 162
<211> 2500
<212> DNA
<213> Artificial Sequence

<220>
<223> chemically treated genomic DNA (Homo sapiens)

<400> 162
   taattgtttg ttttagtttt ttgagtagtt gggattatag gtatttatta gtatgtttgg 60
   ttaagttttg tgtttttatt agagatgggt tttgttattt ttggttgggg tgtttttgaa 120
   tttttgattt tgtgatttat ttgttttggt tttttaaagt gttgggatta taggtatgag 180
   tttttgtgtt tggtggagat ataattttta tatattggtt ttttatttag tggttttgtg 240
   aaatatgttt gtgaatttta aagtttattt ttaggttgtt tttagttttt aatatataga 300
   aatatattat tttggaataa gagtagtttt gtttttgtta tttttttttt tttttttttt 360
   tgtatttttt tgtagagatg gggttttgtt atgtttttgg ggttgttgtt gaatttttga 420
   gtgtaagtga tgtatttatg ttatttttta tagtgttggg attattggtg tgggttattg 480
   tggtgggttt gttgttgtta ttgtaaagag ttttattttt ttttttgatt ttatggtatt 540
   gtgtagattt atttgttata atggtgatag tggatatttt tgttttattt ttgatgagaa 600
   attgaaaaat tttaatattt tattatttta tttatttttt tttttttgta gatggatttt 660
   attagagtga gttattttat tttgttttaa atttgttgag agtatttatt tgaatatatg 720
   ttgattttta ttaaatagtg tatttatttt gattattata gtgttttttt ttatttatgt 780
   gttaatatag tgaatttgat tgataaattt gtatgttttt aggtttgatt attaaaattt 840
   gagatagtgt tttattttgt tattgaggtt ggagtgtagt ggtgttatta gagtttgttg 900
   tagttttgat tttttgggtt taggtgtttt ttttatttta gttttttgag tagttgtgag 960
   tataggtata tgttattatg tttagttaat tttttgatgg ttttttgttt gttttttgta 1020
   gtgatgagat tttttgatgt tgtttaggtt ggttttgaag ttttgagttt aggtgatttg 1080
   gttagtttag ttttttaaaa tattaggatt ataggtgtga gttttggttt ggtttggttt 1140
   tttttatata ggggttttat ttatataaag attaaagtta atttgtgttt ttgtgtgggt 1200
   gggttaagag tatgatgatt tttattattt tattgattta aagaaaatta tttttgattt 1260
   attagtgtgt aaggttatga aagtataatt ttgttgaaag tatatattgt taatgggtgt 1320
   tgggaattgt gtattttttg ttgttgtggg agtatgtttt tggaggtatt ttttatttgt 1380
   ttttttaatt ttaaatattt taggattatg ggttgtgatt ggtaggatta tgtattttgt 1440
   tgtttttaag atggagtata tttttatgtg gttttatttt ggttgttttg tttttttaat 1500
   attgttattt tattttttgt gattttgatg ttataaatga tagatattgt tttagtattt 1560
   ttttatgggt tttagtgatt ttatttattt tttttttagt tttttttttt gatttgttta 1620
   taatgaataa tttttttttg ggataggttg ttatttttgt ttttgtaggt ggtttatttg 1680
   tttttttagt tagttatagt ggtttttgtt tttatggtgg gtttggggta agagagggtt 1740
   ttgggttggg ggtggggttt agttgaagat ggggtgagtt ttgaggggag tattatttga 1800
   gttttagagg tataggaaat agtagaggga ggtgggattt ttttattttt aatgaggatg 1860
   ggtatggagg gtttggggtg tggtgttggg aatggtagtt ttttttagtt tatagttgtg 1920
   tatgtttttt gggtttttgt tttagtgtgt atgtttattg ggtgtttttt gtttggtttt 1980
   tttgtttatg tgaagaatgt tagggagttg tgaggtagtg ttgtgtggtt tttgttgttt 2040
   ggattttttt ttttttattg agatttattt ggtaggtttg taggttagtt attttggggg 2100
   ttgaagtgtg agtgagggtg gagagggttt tgggtgggtt aggtgggttt tgttttttgg 2160
   tttgtggttt ttgagggaga agggttatga ggttgttttt ttttttttta taggttgtga 2220
   ggttattggt ggttttgtgg ttgtgaaggg gtttggatgg ggaggaaggt gggttgtgga 2280
   ggggaggtgg ttaggggttt aggtgaagat ggggtgagtg ttgttggggg gatggaagtt 2340
   ttgaggtgtt gggaattttt gatgatatag ggtagatttt ttgaatgggg tttttggtgg 2400
   gggtgaggtg ggtggtgaag aaggggtttg gtatttggga aggttgtggt ttggtgagtg 2460
   ttttttttag tggtgtggag tgtggagtgt ttgagtgaga 2500

<210> 163
<211> 2500
<212> DNA
<213> Artificial Sequence

<220>
<223> chemically treated genomic DNA (Homo sapiens)

<400> 163

ttttatttag gtgttttgta ttttatattg ttgggggggg tgtttattag gttgtagttt 60
ttttaggtgt taggtttttt ttttattgtt tgttttgttt ttgttggagg ttttatttag 120
ggaatttgtt ttgtgttgtt gggggttttt ggtattttgg gatttttatt tttttaatag 180
tatttatttt atttttattt gagtttttga ttgttttttt tttatggttt attttttttt 240
ttgtttaggt ttttttatga ttatgaagtt gttggtggtt ttgtagtttg tgaagggaag 300
gaggatgatt ttgtggtttt ttttttttgg aggttataga ttaggaagtg ggatttgttt 360
gatttattta aggttttttt tatttttatt tatattttag tttttgggat gattggtttg 420
tagatttatt agatgaattt tagtagagga aaaagagttt ggatggtagg aattatatag 480
tattgtttta tagttttttg gtgtttttta tgtgggtgaa ggggttgggt agaagatgtt 540
tagtgaatat gtgtattgag gtgggagttt agggagtatg tgtggttgtg ggttggggag 600
ggttgttgtt tttagtgtta tgttttaaat tttttatgtt tatttttatt gaggataagg 660
gaattttatt tttttttgtt gttttttatg tttttgggat ttaagtagtg ttttttttaa 720
aatttatttt atttttaatt gaattttatt tttaatttag ggtttttttt tgttttggat 780
ttattatggg gataaggatt attgtgattg gttgggaaga taggtaaatt atttgtgaaa 840
atagaaatag taatttattt taaaaggaaa ttgtttattg tgaataagtt agagaaagag 900

attgaaagaa aaatgaatag aattgttagg atttgtaaga aaatgttaaa atgatattta 960
ttatttgtag tattagaatt atagagggtg aagtgatagt attagggaaa taggatagtt 1020
agagtgaaat tatgtgaaaa tatattttgt tttgaaagta gtaagatata tagttttatt 1080
agttataatt tatggtttta agatgtttgg agttgagaaa atagatgaaa ggtattttta 1140
aggatatgtt tttatagtag tggaaagtgt atggttttta atatttatta ataatatatg 1200
tttttaatag aattatgttt ttatggtttt atatattggt aagttaagga tagttttttt 1260
taaattaata gaatgataaa agttattatg tttttagttt atttgtataa aggtatagat 1320
taattttagt ttttatatag ataagatttt tatataagaa aaattagatt aggttaaggt 1380
ttatgtttgt aattttagta ttttgggagg ttgagttggt tagattattt gagtttagga 1440
ttttgagatt agtttaagta atattagaaa attttattat tataaaaaat aaataaaaaa 1500
ttattgaaaa attagttggg tatggtggta tgtatttata tttatagtta tttaggaggt 1560
tgaggtggga ggagtgtttg agtttaggag gttaaggttg tagtgagttt tgataatatt 1620
attgtatttt agttttggtg atagagtgag atgttgtttt aagttttaat aattgaattt 1680
aaaaatgtat aaatttatta attgaattta ttatattaat atatgaatgg gaaaaaatgt 1740
tgtggtaatt gaaatagatg tattgtttga tgaaaattaa tatatattta aatgaatatt 1800
tttagtaaat ttggaataga atggaatgat ttattttgat aaagtttatt tataaaaaaa 1860
ggagagtgaa taggatggtg aaatgttgaa attttttggt tttttattag ggataagata 1920
aggatgttta ttgttattat tgtaatgggt gggtttgtgt aatgttataa aattagaaaa 1980
ggaaataaaa ttttttataa tggtaatgat gggtttgtta tggtggttta tgttagtaat 2040
tttagtattg tgggagatga tgtgggtgta ttatttgtat ttaaaagttt aataatagtt 2100
tgggaaatat ggtaaaattt tgtttttata aaaaaatata aaagggaaaa gaaaaaaaaa 2160
tggtggaaat aaaattgttt ttattttagg atgatatgtt tttgtatatt gaagattgaa 2220
aatgatttag aagtaaattt tagaatttat aagtatattt tataaggtta ttggatagaa 2280
aattaatatg taagaattat gtttttattg ggtgtggagg tttatgtttg taattttagt 2340
attttgggag gttaaggtag gtgaattatg aggttaggag tttaagagta ttttggttaa 2400
gatggtgaaa gtttattttt agtaaaaata taaaatttag ttaggtgtgt tggtgggtgt 2460
ttataatttt agttatttgg gaggttgagg tagataattg 2500

<210> 164
<211> 2500
<212> DNA
<213> Artificial Sequence

<220>
<223> chemically treated genomic DNA (Homo sapiens)

<400> 164
   ttgttttagt tttttgagta gttgggatta taggtattta ttagtatgtt tggttaagtt 60
   ttgtgttttt attagagatg ggtttttgtt attttggttg gggtgttttt gaatttttga 120
   ttttgtgatt tatttgtttt ggttttttaa agtgttggga ttataggtat gagtttttgt 180
   gtttggtgga gatataattt ttatatattg gtttttattt tagtggtttt gtgaaatatg 240
   tttgtgaatt ttaaagttta tttttaggtt gtttttagtt tttaatatat agaaatatat 300
   tattttggaa taagagtagt tttgtttttg ttattttttt tttttttttt ttttgtattt 360
   ttttgtagag atggggtttt gttatgtttt ttgggttgtt gttgaatttt tgagtgtaag 420
   tgatgtattt atgttatttt ttatagtgtt gggattattg gtgtgggtta ttgtggtggg 480
   tttgttgttg ttattgtaaa gagttttatt tttttttttg attttatggt attgtgtaga 540
   tttatttgtt ataatggtga tagtggatat ttttgtttta tttttgatga gaaattgaaa 600
   aattttaata ttttattatt ttatttattt tttttttttt gtagatggat tttattagag 660
   tgagttattt tattttgttt taaatttgtt gagagtattt atttgaatat atgttgattt 720
   ttattaaata gtgtatttat tttgattatt atagtgtttt tttttattta tgtgttaata 780
   tagtgaattt gattgataaa tttgtatgtt tttaggtttg attattaaaa tttgagatag 840
   tgttttattt tgttattgag gttggagtgt agtggtgtta ttagagtttg ttgtagtttt 900
   gatttttggg gtttaggtgt tttttttatt ttagtttttt gagtagttgt gagtataggt 960
   atatgttatt atgtttagtt aattttttga tggttttttg tttgtttttt gtagtgatga 1020
   gattttttga tgttgtttag gttggttttg aagttttgag tttaggtgat ttggttagtt 1080
   tagtttttta aaatattagg attataggtg tgagttttgg tttggtttgg ttttttttat 1140
   ataggggttt tatttatata aagattaaag ttaatttgtg tttttgtgtg ggtgggttaa 1200
   gagtatgatg atttttatta ttttattgat ttaaagaaaa ttatttttga tttattagtg 1260
   tgtaaggtta tgaaagtata attttgttga aagtatatat tgttaatggg tgttgggaat 1320
   tgtgtatttt ttgttgttgt gggagtatgt ttttggaggt attttttatt tgttttttta 1380
   attttaaata ttttaggatt atgggttgtg attggtagga ttatgtattt tgttgttttt 1440
   aagatggagt atatttttat gtggttttat tttggttgtt ttgttttttt aatattgtta 1500
   ttttattttt tgtgattttg atgttataaa tgatagatat tgttttagta tttttttatg 1560
   ggttttagtg attttattta tttttttttt agtttttttt tttgatttgt ttataatgaa 1620
   taattttttt ttgggatagg ttgttatttt tgtttttgta ggtggtttat ttgttttttt 1680
   agttagttat agtggttttt gtttttatgg tgggtttggg gtaagagagg gttttgggtt 1740
   gggggtgggg tttagttgaa gatggggtga gttttgaggg gagtattatt tgagttttag 1800
   aggtatagga aatagtagag ggaggtggga tttttttatt tttaatgagg atgggtatgg 1860
   agggtttggg gtgtggtgtt gggaatggta gtttttttta gtttatagtt gtgtatgttt 1920
   tttgggtttt tgttttagtg tgtatgttta ttgggtgttt tttgtttggt ttttttgttt 1980
   atgtgaagaa tgttagggag ttgtgaggta gtgttgtgtg gtttttgttg tttggatttt 2040
   ttttttttta ttgagattta tttggtaggt ttgtaggtta gttattttgg gggttgaagt 2100
   gtgagtgagg gtggagaggg ttttgggtgg gttaggtggg ttttgttttt tggtttgtgg 2160
   tttttgaggg agaaggttta tgaggttgtt tttttttttt ttataggttg tgaggttatt 2220
   ggtggttttg tggttgtgaa ggggtttgga tggggaggaa ggtgggttgt ggaggggagg 2280
   tggttagggg tttaggtgaa gatggggtga gtgttgttgg ggggatggaa gttttgaggt 2340
   gttgggaatt tttgatgata tagggtagat tttttgaatg gggtttttgg tgggggtgag 2400
   gtgggtggtg aagaaggggt ttggtatttg ggaaggttgt ggtttggtga gtgttttttt 2460
   tagtggtgtg gagtgtggag tgtttgagtg agaagtattg 2500

<210> 165
<211> 2500
<212> DNA
<213> Artificial Sequence

<220>
<223> chemically treated genomic DNA (Homo sapiens)

<400> 165

tagtgttttt tatttaggtg ttttgtattt tatattgttg gggggggtgt ttattaggtt 60
gtagtttttt taggtgttag gttttttttt tattgtttgt tttgtttttg ttggaggttt 120
tatttaggga atttgttttg tgttgttggg ggtttttggt attttgggat ttttattttt 180
ttaatagtat ttattttatt tttatttgag tttttgattg tttttttttt atggtttatt 240
tttttttttg tttaggtttt tttatgatta tgaagttgtt ggtggttttg tagtttgtga 300
agggaaggag gatgattttg tggttttttt tttttggagg ttatagatta ggaagtggga 360
tttgtttgat ttatttaagg ttttttttat ttttatttat atttagtttt ttgggatgat 420
tggtttgtag atttattaga tgaattttag tagaggaaaa agagtttgga tggtaggaat 480
tatatagtat tgttttatag ttttttggtg ttttttatgt gggtgaaggg gttgggtaga 540
agatgtttag tgaatatgtg tattgaggtg ggagtttagg gagtatgtgt ggttgtgggt 600
tggggagggt tgttgttttt agtgttatgt tttaaatttt ttatgtttat ttttattgag 660
gataagggaa ttttattttt ttttgttgtt ttttatgttt ttgggattta agtagtgttt 720
tttttaaaat ttattttatt tttaattgaa ttttattttt aatttagggt ttttttttgt 780
tttggattta ttatggggat aaggattatt gtgattggtt gggaagatag gtaaattatt 840
tgtgaaaata gaaatagtaa tttattttaa aaggaaattg tttattgtga ataagttaga 900
gaaagagatt gaaagaaaaa tgaatagaat tgttaggatt tgtaagaaaa tgttaaaatg 960
atatttatta tttgtagtat tagaattata gagggtgaag tgatagtatt agggaaatag 1020
gatagttaga gtgaaattat gtgaaaatat attttgtttt gaaagtagta agatatatag 1080
ttttattagt tataatttat ggttttaaga tgtttggagt tgagaaaata gatgaaaggt 1140
atttttaagg atatgttttt atagtagtgg aaagtgtatg gtttttaata tttattaata 1200
atatatgttt ttaatagaat tatgttttta tggttttata tattggtaag ttaaggatag 1260
ttttttttaa attaatagaa tgataaaagt tattatgttt ttagtttatt tgtataaagg 1320
tatagattaa ttttagtttt tatatagata agatttttat ataagaaaaa ttagattagg 1380
ttaaggttta tgtttgtaat tttagtattt tgggaggttg agttggttag attatttgag 1440
tttaggattt tgagattagt ttaagtaata ttagaaaatt ttattattat aaaaaataaa 1500
taaaaaatta ttgaaaaatt agttgggtat ggtggtatgt atttatattt atagttattt 1560
aggaggttga ggtgggagga gtgtttgagt ttaggaggtt aaggttgtag tgagttttga 1620
taatattatt gtattttagt tttggtgata gagtgagatg ttgttttaag ttttaataat 1680
tgaatttaaa aatgtataaa tttattaatt gaatttatta tattaatata tgaatgggaa 1740
aaaatgttgt ggtaattgaa atagatgtat tgtttgatga aaattaatat atatttaaat 1800
gaatattttt agtaaatttg gaatagaatg gaatgattta ttttgataaa gtttatttat 1860
aaaaaaagga gagtgaatag gatggtgaaa tgttgaaatt ttttggtttt ttattaggga 1920
taagataagg atgtttattg ttattattgt aatgggtggg tttgtgtaat gttataaaat 1980
tagaaaagga aataaaattt tttataatgg taatgatggg tttgttatgg tggtttatgt 2040
tagtaatttt agtattgtgg gagatgatgt gggtgtatta tttgtattta aaagtttaat 2100
aatagtttgg gaaatatggt aaaattttgt ttttataaaa aaatataaaa gggaaaagaa 2160
aaaaaaatgg tggaaataaa attgttttta ttttaggatg atatgttttt gtatattgaa 2220

gattgaaaat gatttagaag taaattttag aatttataag tatattttat aaggttattg 2280
gatagaaaat taatatgtaa gaattatgtt tttattgggt gtggaggttt atgtttgtaa 2340
ttttagtatt ttgggaggtt aaggtaggtg aattatgagg ttaggagttt aagagtattt 2400
tggttaagat ggtgaaagtt tatttttagt aaaaatataa aatttagtta ggtgtgttgg 2460
tgggtgttta taattttagt tatttgggag gttgaggtag 2500

<210> 166
<211> 2501
<212> DNA
<213> Artificial Sequence

<220>
<223> chemically treated genomic DNA (Homo sapiens)

<400> 166

ggggattttt ttaattttta aagaatgtgt gtttttttaa gggtaatttg tagagtttga 60
gaatggttta ggtttaggta tgtatggata gaaaggatat ttttttttta ggggtttagt 120
gggggtagga gggggtggat agggatgtgt agggtaagtt tttttttgtt ttgtaggtat 180
ttatttaatt tttgtttaaa gtagtaatag tgggtttttg tttttttatt tttaattttt 240
tatatttttt ttgatagtag aggattagat tatttaggtg atggtgaagt tttttgatgt 300
gtattaggtg taggagattt atgattttta gtattgtttt ttatttgttg ggtttttttt 360
ggagaattat atatttttgg agttttagaa tatgttggtt gtataagaat aaagtatatt 420
tattgtttga gaggaaggtt ggtggtgtgg tagtattatt gaattttttt ttttttttga 480
gattatattg tatatatata gtgttatata attggatgtt ttaagttaat aatttaaaat 540
gaatagtatt attattatag gaaatattat ttagtattgg ttttggtagt tatttatatt 600
ttatttttaa gtgtgttata atttatttat atagtaagtt tatgattatg ttattataaa 660
ttaagttgtt ttggaagttt tatgtattta aatttttgtt tatatggtta aagaaaaaaa 720
atatttagtg gtagttgtta aagtatagta aggaaaattt tatttaggat tgtggtgatg 780
ggtataggga ttattgtaat gggattttgt agtgggggag agagattggg tttaatattg 840
aatatagtgt aggttagtgg gaatgtatag tgaaggagta gtatggtggt ttgtgggtgg 900
aaaattatta aaaggaaata ttaggggtaa gggggatttt ggttaaattt atttagtagg 960
atttttgttg aagataggtt aaggggaatt agatattatt tgggggatgg tggaagatga 1020
agaattttat gtgatagtaa gtatgattag atattgagga tggagggttt atgttatatt 1080
gatttagtag ggttttttgt taaaattggg ttttataagg aaatgtatag atgggtttag 1140
gaggagaagt tttagaagtt tgatttaaaa gtttggttaa gtaaggaata tttgttaata 1200
tttaagatag tttttttaat attaaagttt tagaagtaga aatgttgggt tataggattt 1260
tttatttttt gagatagggt tttgttttgt tgtttaagtt ggagtgtagt ggtgtgattt 1320
tggtttattg taattttaat tttttgggtt taagttattt ttttatttta gttttttaag 1380
tagttgggat tataggtgtg tattattatg tttagttaat ttttgtattt tttgtaaaga 1440
tagggttttg ttatgttgtt taggatggtt ttgaattttt gggtttaaga aatttattta 1500
ttttagtatt ttaaagtttt aggattatag gtatgagttg ttgtatttgg tttggatttt 1560
tgtgtaaatg tttaggttta tttatgtatg tgggtttatt attttttttt tttatatttt 1620
tttttggatt tttattatgt ttaaaatggt tttgtatttt ttgagaattt aagtttttat 1680
gtatttttgg gaagattgaa tattttttta agggttattg aggtagtagt attgagaaaa 1740
aattaaaggg taaatttgta ggtgaaatgt agagttggga atatttttag atttagaagg 1800
atagaatatt ttgtaggagg ggttagaagt tagaaataag ttttttttag tattgagtag 1860
gttttttttt taggtggtat agagttttta ggaggtggga gagggttgga aagataattt 1920
atgtgatata tttttgaggt ttgggattgg ttggtggggt tgatattgtt gaggtttaaa 1980
taaggaggta gaagtaggta ggtgttattt tttttttttt tattttttgt taggtttttt 2040
gttgatttaa gtgtaagtga gaataggtgt gagtgagtgg gttggtgggt ttttggtggg 2100
ttgggtattg tggtttttgg ttttagagaa ggaagggttt tgaggttatt attttttttt 2160
tagtaggtag ttatgtggtg gggtagggat aggggaggaa ggagggttat gaaggggagg 2220
agggttagtt aagatgttgt gagttttgga gtgggatatt atttgagttt tggaggtata 2280
tgaaattgtt tataaaggat ggatgttggg gtttttagtg ggggttaggg aaagggtggg 2340
atagtttgtg tttgatagtg tttgttttag tgtttgtgtt tattgggggt ttttttatta 2400
gtttttttat ttatgaggtg aattgttgtg gagttgtgag ggtgttgttt gtattttaat 2460
tgttgggatt tttttatttg agattgagat ttagtgggtg g 2501

<210> 167
<211> 2501
<212> DNA
<213> Artificial Sequence

<220>
<223> chemically treated genomic DNA (Homo sapiens)

<400> 167

ttatttattg agttttagtt ttaggtgaaa gagttttgat aattggaatg taaatggtat 60
ttttatagtt ttgtggtagt ttattttgtg ggtgaagggg ttgatgggaa gatttttagt 120
gaatatgagt attgaggtag gtgttgttaa gtgtgggttg ttttattttt tttttggttt 180
ttattgagga ttttagtatt tgttttttat ggatagtttt atatgttttt aggatttaga 240
tagtgttttg ttttaaaatt tatagtattt tggttgattt tttttttttt tatggttttt 300
tttttttttt tgtttttgtt ttgttgtata gttgtttgtt aggagaagga tgatgatttt 360
gaggtttttt tttttttgag gttagggatt atagtgtttg atttattaga agtttgttgg 420
tttatttatt tatatttgtt tttatttata tttgagttag tagagagttt ggtgaagaat 480
gaagggaaga gaagtggtat ttatttattt ttgttttttt gtttagattt tagtagtgtt 540
aattttatta gttaatttta aattttgggg gtgtgttatg tgggttgttt ttttagtttt 600
tttttgtttt ttagaggttt tgtgttattt ggaggaaggg tttgtttggt gttgagaagg 660
gtttgttttt ggtttttggt tttttttgta aaatgttttg tttttttgga tttaggaatg 720
tttttagttt tgtattttat ttgtaaattt attttttaat ttttttttag tgttgttatt 780
ttagtggttt ttaagagggt atttagtttt tttagagatg tgtgaaagtt taggttttta 840
gagagtgtaa agttatttta agtatgatag gaatttagaa aaaaatgtaa aggagaagga 900
tgataaattt atatatataa ataagtttaa atatttgtat aaaaatttag gttaggtgtg 960
gtagtttatg tttgtaattt tagaattttg ggatgttgag gtgggtggat tttttgagtt 1020
taggagtttg agattatttt gggtaatatg gtaagatttt gtttttataa aaaatataaa 1080
aattagttgg gtatggtggt gtatatttgt agttttagtt atttgagagg ttgaggtggg 1140
aggatagttt gagtttagga ggttgaggtt gtagtgagtt aagattatat tattgtattt 1200
tagtttgggt aatagagtaa gattttgttt taaaaaataa aaaattttgt gatttagtat 1260
ttttattttt aggattttaa tgttaaggaa attgttttga atgttgataa atattttttg 1320
tttggttaaa tttttaagtt aggtttttga aatttttttt tttaggttta tttgtgtatt 1380
tttttgtaaa atttagtttt agtaaagagt tttgttaagt taatgtggta tgaatttttt 1440
atttttaata tttgattata tttgttattg tatggggttt ttttattttt attatttttt 1500
aggtgatgtt tgattttttt tggtttgttt ttagtaagaa ttttgttagg tgggtttagt 1560
tagaattttt tttatttttg atgttttttt ttagtaattt tttatttatg gattgttata 1620
ttgttttttt gttatatatt tttattggtt tgtgttgtat ttagtattga gtttaatttt 1680
ttttttttat tgtaagattt tattgtaatg gtttttatat ttattattat agttttaaat 1740
aaggtttttt ttattgtgtt ttaataatta ttattgaatg tttttttttt ttaattatgt 1800
gaataaaaat ttggatatat gggattttta aaatagttta atttataata gtatggttat 1860
ggatttgtta tataagtaaa ttatggtata tttgaaaatg aaatatgggt gattattaaa 1920
attagtgttg aataatattt tttgtaatag tggtgttgtt tattttaagt tattaatttg 1980
aaatatttag ttatgtagta ttgtgtatgt ataatatgat tttaaaagaa aagagaggtt 2040
taatagtgtt attatattat tagttttttt tttagatagt aagtgtgttt tgtttttgtg 2100
taattagtat gttttggggt tttaaagata tatggttttt taaggggaat ttagtagata 2160
aaaggtagtg ttaggggtta taggtttttt gtatttggtg tatgttaaag agttttatta 2220
ttatttaggt ggtttgattt tttgttgtta gagaaggtgt ggggggttgg gggtaggggg 2280
atagaagttt attgttattg ttttgggtaa aagttagatg ggtgtttgta agatagaaag 2340
gagtttgttt tgtatatttt tgtttatttt tttttgtttt tgttaaattt ttgggggaaa 2400
aatatttttt ttgtttgtgt atgtttggat ttggattgtt tttagatttt gtaggttatt 2460
tttaaagaag tgtatatttt ttgagagtta gaaagatttt t 2501

<210> 168
<211> 2501
<212> DNA
<213> Artificial Sequence

<220>
<223> chemically treated genomic DNA (Homo sapiens)

<400> 168

ttttattaat ataggaattt tttattaagg aaaataaaag ttttaaaaaa attttattgt 60
tttttttagt aattgtttga aagatttatt aattttttaa taaaaagtgt taaatgatat 120
tttttaattt taaaattttt tgaatttatt gtttaaaaag ttttgttgta tttattaatt 180
ttaaattatt gttattttta tttaatttta attaaattat tttttatttt aaaatattta 240
ttttatattg gatatgaaaa gtataataaa tatatttaaa gtttttttat atttgatata 300
ttagtgtttt tgtggaggta gtgttttatt ttatagtttt atttaaataa tgttagtgtt 360

gttttatgaa taggttattt ggttgttatt tggggagtag agtatatgat gagtatagtt 420
attattgttg tatattatgt tataataaat taagtttaaa ttttaggatt aatgttgtat 480
tagtttgttt ttatgttgtt ataaggatat atttgagagt gggtaattta taaaggaaag 540
aggtttaatt gatttatagt tttgtagggt tggggaggtt tttggaaatt taataattat 600
ggaagaagtg gaagtaaata tatttttttt tatatggtag taggagagag aagagtgagt 660
aaaaggggaa aagtttttta taaaattatt agattttgtg aaaatttatt tattattagg 720
ggaatagtat gaaggtaatt attttatgat tatattattt tttatagggt tttttttatg 780
atatgtgggg attatgggaa ttataattta aggagagatt tgggtgggga tagagttaaa 840
ttatattata tgtatggata tttttgaatt atatattgtt tgaatgattt ataggtatat 900
ttagggaaat tagttattag tttagagatt aaaatgtgta ataatattat ttaaaaaagg 960
aagaggtgtg aatttattta gtttatagaa gtggttttat ttattggtag tatggtagaa 1020
aattttagat tagagaagga agtgattaat taaagtatga ataaaaattg gttaaggtgg 1080
gaggattatt tgattttaga aattgagatt agtttggata atgtagtgag attttgtttt 1140
taagtaaata aataaataaa atagttgggt atggtggtat atatttgtag ttttaattat 1200
tttggaggtt gtgttttgag tattgtttat gtttaggaga tttaggttgt agtgaggtaa 1260
agttatgtta ttgtttttta tttagggtga taaagtaata ttttgtttgg aaaaaaaaag 1320
tatgtataaa tataaatatt aaaataattt attttgttta taaaaggttt ttttgagata 1380
tattaattat taagggtgat atgggatttt ttgtttttaa agtttttgga tgtatgagaa 1440
tggtaggata agagagagag aaaaaaaaat atttttaagt tttttttttt tttattataa 1500
atatattggg gattttggtt ttgggatagt gggaaaattt tggttggttt tgtatgtgtt 1560
tgatttagtg tgaatgtttt ttattgttta agtttttgtt tgtttatgtg ttttattgtt 1620
tgttttttgt tttagtagag ttgtgtaaag agatattgtg attttttttt gttttgttga 1680
ttaggagtta gttagtaggt ttgtagttta gtagttttag aagtttgagt gtgagtgagt 1740
gtgaggagga gtttgtgggt atgatttatg gtttttaggt tggaagggtt ttgaggtttt 1800
tgttttttta atatgtagtg atgtagttat ggttttgttg tggtgggggt gttggataag 1860
ggagggagaa ggattataga tgggaggagg gttaggtaaa taagtagtga gttttgaagg 1920
gggaaattat ttgtggtatt tgagtttgtg aggtatatga aattgtttat agatgatgta 1980
ttttagagtt tttagtggga attagggagg ggagggagga ttgtatgatg tgggatgggg 2040
tggggtgagg tggggtattg tagtatataa tgtaggtatt gattttagtg tgtatgtttt 2100
ttggatattt gttttagtgt gtatgtttat tgggtatttt tttttgattt tttttgttta 2160
tgtggtgatt gttggggagt tgtgagagtg tgaggggtat gttttagttg tttggatttt 2220
ttttttttta ttgagatgta gtttataggt ttgtaggtta gtttttttag gaattgaaat 2280
gtgagtgaat gtgaggagga gtgagtgggt ttttagaggg ttaggtggtg tggtttgtgg 2340
ttttagagag taaaaagttt ttaggttata aggaaggtgg gttgtggagt gaaggggagt 2400
tagatgaaga tagggtgaat gtttggggtg ttgttggagg tatttgagtt ttgaaagtgt 2460
ttggaatttt taatgggata gattttagat tttttggtag g 2501

<210> 169
<211> 2501
<212> DNA
<213> Artificial Sequence

<220>
<223> chemically treated genomic DNA (Homo sapiens)

<400> 169
   tttattaaga agtttggaat ttgttttatt gggagtttta ggtatttttg ggatttagat 60
   atttttaata gtattttgag tatttatttt atttttattt ggtttttttt tattttgtgg 120
   tttatttttt ttatgatttg gaagtttttt attttttgag gttatagatt atgttgtttg 180
   attttttgga agtttgttta ttttttttta tatttattta tattttagtt tttgggagga 240
   ttggtttgtg gatttatagg ttgtgtttta gtaggagaga aagagtttag atggttggaa 300
   tgtgtttttt atatttttat agttttttag tggttattat gtgggtaaag gggttgaagg 360
   gaagatgttt agtgaatatg tatattgagg taggtgttta aggaatatgt atattgaagt 420
   tggtgtttgt gttgtgtgtt gtagtgtttt gttttgtttt attttatttt gtgttatgta 480
   gttttttttt ttttttttgg tttttattga ggattttgga atgtattgtt tgtaagtagt 540
   tttatatgtt ttgtggattt aaatattata aatagttttt ttttttaaaa tttattgttt 600
   gtttatttga tttttttttt atttatggtt tttttttttt ttttgtttgg tatttttatt 660
   atagtaaggt tatggttgta ttgttgtgtg ttagaaggat ggggatttta aagttttttt 720
   atttaggagg ttatggattg tgtttgtagg ttttttttta tatttattta tatttaaatt 780
   tttaggatta ttgaattgta gatttattgg ttgattttta gttggtaaga tagaaggaag 840
   ttatgatgtt tttttgtata gttttgttgg gatagaaggt agatagtaag gtgtatgggt 900
   aataagggat ttgggtagta aggggtgttt atattgagtt aggtatatgt aaggttagtt 960
   agggtttttt tattgtttta aaattagaat ttttagtatg tttgtagtag aaagaaaggg 1020
   atttaagaga tatttttttt tttttttttt ttgttttatt atttttatgt atttagaaat 1080
   tttgggaata gagagtttta tgttattttt agtggttgat gtgttttaga aagatttttt 1140
   gtgatagaaa taaattattt taatgtttat atttatatat gttttttttt tttagatagg 1200
   gtgttatttt gttgttttgg atggagggta atggtatgat tttattttat tgtagtttgg 1260
   atttttgggg tataagtaat atttaagatg tagtttttga agtagttggg attataggtg 1320
   tgtgttatta tgtttagtta ttttatttat ttatttattt agagatagaa ttttattata 1380
   ttgtttaggt tggttttaat ttttgggatt gagtgatttt tttattttgg ttaattttta 1440
   tttatatttt gattaattat tttttttttt ggtttgaggt tttttgttat gttattaatg 1500
   aatagaatta tttttgtaga ttaaataaat ttatattttt ttttttttta ggtggtatta 1560
   ttgtatattt taatttttgg attggtaatt gatttttttg aatatatttg taggttattt 1620
   agatagtgta tggtttagaa atatttatat atatgatgtg gtttgatttt gtttttattt 1680
   aaattttttt ttgaattgta gtttttataa tttttatatg ttatggaaag gattttgtgg 1740
   gaggtaatgt aattatgggg tggttatttt tatgttgttt ttttgatagt gagtaagttt 1800
   ttataagatt taatggtttt ataaggggtt tttttttttt tgtttatttt tttttttttt 1860
   gttgttatgt gaagaaggat gtgtttgttt ttattttttt tatgattgtt aagtttttag 1920
   aggttttttt agttttgtag aattatgagt taattaaatt tttttttttt ataaattatt 1980
   tatttttagg tatgttttta tagtagtatg agaatggatt aatataatat tggttttagg 2040
   atttaaattt agtttattgt gatatgatgt atagtagtgg tgattgtgtt tattatatat 2100
   tttatttttt aaatagtagt taaataattt gtttataaga tagtgttgat attatttaaa 2160
   taaagttata aggtagagta ttatttttat agaggtatta gtatgttaga tgtggaaggg 2220
   ttttgggtat atttgttgtg ttttttatgt ttggtataaa gtgggtattt taaagtgggg 2280
   gatggtttgg ttagaattgg gtaggaatga taatgattta ggattggtgg atgtaataag 2340
   attttttagg taatgggttt aaagagtttt gggattgaaa aatgttattt gatatttttt 2400
   attgaggagt tgatggattt tttaggtaat tgttggaaag aataataaag tttttttgaa 2460
   atttttattt tttttggtaa gagatttttg tattagtaaa a 2501

<210> 170
<211> 2500
<212> DNA
<213> Artificial Sequence

<220>
<223> chemically treated genomic DNA (Homo sapiens)

<400> 170

ttttttttat tgttattttt attatttatt ttttgttttg gggttttatt taggatttag 60
gaatgtgttt tgtgttatgg gtttttatta ttttgggatt ttgatttttt taatagtatt 120
tgtagtattt gttttatttt tatttgagtt tttgattgtt ttttttttat ggtttatttt 180
tttttttgtt ttggtttttt tatgattata aagttgatgg tggtgttgta gtttgtgagg 240
agaaggagga tgagtttgag gttttttttt tttggaggtt attgaggttt ttttttttta 300
aaagttatat attattagat tgaggggttt attgaatttg tttgaagttt attggttttg 360
tttgaagttt attggttttt ttttattttt atttatattt tagtttttgg gaggattggt 420
ttgtggattt attagatgag ttttagtaga ggaaaaaagt gtttggatag taggaatgtg 480
attgtatatt tttatagttt tttggtgttt tttatgtggg tgaaggggtt gatgggaagg 540
tatttagtga atatgtgtat tgaggttagt atttaaaggt ggggagggtt gtagttttta 600
gttttatatt ttatattttt gatttttatt tttgtgaaga taatggaatt tgattttttt 660
ttgttgtttt atatgttttg ggtatttaaa tattttaaat agtgtttttt ttaaaattta 720
ttttattttt aattgagttt tttttttttt tagggttttt ttttgttttg gttttgttat 780
agggataagg attatggtgg ttgtttgtga agtgaggtag atgatttgtt aaaatagaaa 840
tagtaatata ttttatagta ttatataaga ttaagagttt tataattaaa tattaaaaat 900
gtttagtata taatttaaaa ttatttagaa tatgaagaat taggaatata gggttattga 960
aatagggaaa gataattagt agatattatt gttaagatag agatgttggg attatgtggg 1020
aaagttttta aagatgttat tttataaatg tttaggttgg gtgtggttgt ttatgtttgt 1080
aattttagta ttttgggagg ttgaggtggg tggatttttt gaggttagga gtttgagatt 1140
agtttgatta atatggtgaa attttgtttt tattaaaaat gtaaaaaatt agttgggtat 1200
ggtggtgtgt atttgtagtt ttagttattt gggagtttga aataggagaa ttgtttgaat 1260
ttaggaggta gaggttgtag tgaattgaga ttatgtgatt gtattttggt ttggataata 1320
gtgagatttt gtttaaaaaa aaaatgttta aataagtaat tatgaatatt gttgaaataa 1380
gtaaaaataa agttttggta tatatataaa aaaagaatat ttaaagaaga agtaaatgga 1440
aattttagaa gtggaaaata tgataatgaa ggaaaataat tttttttaaa tggatttaat 1500
agtagaatgg agataagaaa agagtttgtg aatttaaaga tagagaaata gaaattgttt 1560
tgtgtggtta agagtagtga tttatattaa taattttagt atttttgtgt gttgaagtaa 1620

gaagatagat ggtttgaggt taggagtttt taatttgggt aatatagtga gattttgttt 1680
ttataaaaaa ataattaaaa atagttgagt gtgatgatat atgtttgtag ttttagttat 1740
tgtgaaggtt gaggtaagag gattttttgg gtttaggagt ttaaggttgt agtgagttat 1800
gattatatat tgtagtttag tttgggtaat agagtgagat tttgtgttta aaataaaata 1860
aaaagaaatt gttgaagtgt tttataattt gatatttaaa atgttaagta tataatttaa 1920
aattatttag aatatgaaga agtatgaaag ttaggtattt tggttaggtg tagtggttta 1980
tatttgtaat tttagtattt tgggaggttg aggtaggtag attatgaggt taggagttta 2040
agattagttt ggttaagatg gtgaaatttt atgtttatta aaaatataaa aattagttgg 2100
gtatggtggt gggtatttgt aattttagtt atttgggagg ttgaggtaga gaattttttg 2160
aatttgggag gtagagtttg tagtgagtta agattgtgtt attgtatttt agtttgggtg 2220
atagagtgag gttttatttt aaaaaaaaaa aaggaagtta tggaatttta aatggagaaa 2280
gatagataat tgatattaat gtaaagatag agatgttgag attatttggg aaagttttta 2340
aagatgttag tttaaaaaag ttataaatat ttatgtttag atataagttt ttaatttttt 2400
tgggtagata ttaagaagta taattattgg attgaatagt aagagtttgt ttagttttgt 2460
aagaaatatt gttaaattgt tttttaaatt ggttttatta 2500

<210> 171
<211> 2500
<212> DNA
<213> Artificial Sequence

<220>
<223> chemically treated genomic DNA (Homo sapiens)

<400> 171

tggtaaagtt aatttgaaag gtagtttggt agtatttttt ataaaattaa ataaattttt 60
attatttagt ttagtagttg tattttttgg tatttattta aaggagttga aaatttgtgt 120
ttggatatgg atgtttatag tttttttaaa ttagtatttt taaaggtttt tttagataat 180
tttaatattt ttgtttttgt attggtgtta gttgtttgtt tttttttatt tgaaatttta 240
taattttttt tttttttttt gaaatggagt tttattttgt tatttaggtt ggagtgtagt 300
ggtatgattt tggtttattg taaattttgt tttttgggtt taaggaattt tttgttttag 360
ttttttgagt agttgggatt ataggtgttt gttattatat ttagttaatt tttgtatttt 420
tggtagatat ggggttttat tattttggtt aggttggttt tgaatttttg attttgtgat 480
ttatttgttt tagtttttta aaatgttggg attataggta tgagttattg tatttggtta 540
agatgtttaa tttttatgtt tttttgtatt ttgagtaatt ttgaattata tgtttgatat 600
tttgaatatt aggttatgag atattttaat aatttttttt tgttttgttt tagatatagg 660
gttttatttt gttgtttagg ttggattgta gtgtgtgatt atagtttatt gtagttttga 720
atttttgggt ttaagggatt tttttgtttt agtttttata gtagttagga ttataggtat 780
gtgttattat atttagttat ttttaattgt ttttttgtag agataaggtt ttattatgtt 840
gtttaggttg gaaattttta gttttaagtt atttattttt ttattttagt atgtaaaagt 900
gttaggatta ttggtgtaag ttattgtttt tggttatata gaataatttt tattttttta 960
tttttgagtt tataaatttt ttttttgttt ttattttgtt attgaattta tttggggaga 1020
attatttttt tttgttatta tattttttat ttttaaaatt tttatttgtt ttttttttaa 1080
atattttttt tttgtgtgtg tgttaggatt ttgtttttat ttgttttaat agtgtttatg 1140
attgtttgtt taagtatttt tttttttagat ggagttttat tgttgtttag gttagagtgt 1200
agttgtgtga ttttggttta ttgtaatttt tgttttttgg gtttaagtaa tttttttgtt 1260
ttagattttt gagtagttaa gattataggt gtgtgttatt atgtttagtt aattttttgt 1320
atttttagta aagatgaggt tttattatgt tggttaggtt ggttttgaat ttttgatttt 1380
aagagattta tttgttttgg ttttttaaag tgttgggatt ataggtatga gtaattgtgt 1440
ttagtttaag tgtttataaa atagtatttt taaaggtttt tttatataat tttaatattt 1500
ttattttagt agtgatgttt gttgattgtt tttttttatt ttagtggttt tatgtttttg 1560
gttttttata ttttgagtaa ttttgaatta tatgttagat atttttaata tttagttatg 1620
agatttttag ttttgtataa tgttatgagg tatgttgtta tttttgtttt agtaggttgt 1680
ttattttatt ttataggtag ttattatggt ttttgttttt atggtagggt tagagtaaga 1740
gagggttttg gaggggaggg gggtttagtt gaagatggaa tgagttttga ggggagtatt 1800
atttgaggta tttgagtatt tgaggtatat gaaatagtag agggaggttg gattttatta 1860
tttttatggg gatgaaaatt gggagtgtgg ggtgtggggt tgggaattgt agtttttttt 1920
atttttgggt gttagtttta gtgtgtatgt ttattgggtg tttttttgtt ggtttttttg 1980
tttatgtgga gaatgttagg gagttgtgag agtgtgtagt tgtgtttttg ttgtttggat 2040
attttttttt tttattgaga tttatttggt agatttgtag gttagttttt ttaggggttg 2100
aagtgtgagt gagggtgagg aggagttagt gggttttagg tggagttagt gggttttggg 2160
taggtttggt gggttttttg gtttggtggt gtgtggtttt tgagggagaa gggttttagt 2220
ggtttttaaa agagaagggt tttgagtttg tttttttttt ttttataggt tgtgatgttg 2280

ttattggttt tgtggttgtg aaggggttag gatagggagg aaagtgggtt atgaagggga 2340
agtggttagg ggtttaggtg aaaatgaggt gagtgttgtg ggtgttgttg gaggggttaa 2400
agttttgagg tggtaggaat ttatgatata gggtatattt ttgagttttg aatggggttt 2460
tgaggtggga ggtgggtggt gaagatggtg gtaaagaagg 2500

<210> 172
<211> 2500
<212> DNA
<213> Artificial Sequence

<220>
<223> chemically treated genomic DNA (Homo sapiens)

<400> 172

tttattttat ttggtgtttt atttttattt tttaaatttt attttttgta tttggttttg 60
tttttattta ttttttggtt ttgtttttta ttttttgatt ttgttgtttt tgttttatgg 120
tttttatttt gttattagaa ttttttagaa tagaatagaa taggatataa ttgaagtagg 180
tggttgtttg gttttttggt tttgtagggg tattttttat tttgtgtaga agtggtattg 240
ttatgtggat tttgtggggt gttttttttt ggttttgagg tttttgttgt ttttgtgttt 300
tggggatttt tgttgtttgt ggtatttgtt ttttttgggt tgttagtatt gttttttggg 360
ttattaggaa tgttagggtt ttttgggtta ttagtattgt ttgttgaatt ttttgtgttt 420
tggtttttgg tttgtatggt tttggagttt ttgtttggtt tttagagaga aggttagggt 480
ttatgaggat gtggaggtag agaggttgta ggaagttttg ttttttggtg tgagatgggt 540
agtttgggat ttttagggtg tttgtgtata ggggttttat ttttggtttt gggagatttt 600
gagtgagttt tggagtatgt gattggtttt tattaatttt gttttttttt aagagagttt 660
gggttggaag gtggttgtaa tgttagtttg gattttttat ttgtgagtag ttggttgttt 720
gttggatttt tgttttggga gttttgtagg gagttaggta ttgtggggtt tagtttgttt 780
tattttttgg gtttttttta tattgaggag taagatgggt ttgggaatat ggggttggga 840

ttgtgtggtt attgttttgg attttgtttg ttttgtttgt ttttggggga gtgtttagga 900
tagattttgg ggggtaggtt tttaattggg tttagtagtt tttgtttttg ttttggttgt 960
ttagttggtg gggtagttgg aattgtatgt ttggtgggtg tggttttgtg ttagttaagg 1020
ttttttaggt gttaggttta gttaaggtgg atgggagggt ttagtgggat ttggggttgg 1080
attttatttt tgggtttaat gggttttgtt tttgttttag ttgttttgtt tggtggggga 1140
gttggagttg tgtgtggggt gggttgtggg tggtttttta ttagttaggg gtttttgggt 1200
tttaggtttg gtttaggtgg ataggtggga tttgttggat ttgaggttgg gttgggtttg 1260
gtttttgggt ttggtagtat tagtttttgt tttggttgtt tttgtttggt agggagttgg 1320
gaggtgattt tgtgtttgtt gggtgttggt ttatttttgt gtttggaagg gggttagatt 1380
tataggaaag gtgttgtgag aggtgggtag gggtgggggt ggtatggttt taggagttgg 1440
gtgtttgttt ttatgttttg tttataagtt tgtggggtga gaggttgtaa tattttattt 1500
tgggtgtttg ggttgtttat taggttggtt taggtttttt atattttgta ttttgggttg 1560
ggtatgaaat tgatttaatt gttttatagg ttgttttttt tgatatatat agaaattgat 1620
ttttttggtt tttaagtttg aattttatag ttgttttatt tgaatttttt ttttaggaaa 1680
tgatttttgg gttttttaaa atattaaaga attgaaattt attagataat tatatttaga 1740
taatgagatg ttggattttt taaatttatt aggattgttt tttgggtttt tttgagtttt 1800
tgttttttta tatattggtg tatttttttt tggttatata aatttttagt tttagttagg 1860
gggatggatt tgagatttaa tttttatttt tttggttgta gtatttaatt aaagttgttt 1920
tttttggtaa tgttttttgt tttagttagt tattggtttt ttgtgtggtt agtagtagga 1980
tgtagattaa atttttggta ttttggtaat aggtatattt ttaggaaggg attttggata 2040
tggttattat ttttaggttt aaagtgaatg tattttttgt atttttagtt ttttgtgtag 2100
tagttttaat ttatgtatgt ttgtgttata tttagttttt agatggttga gtgattttaa 2160
gaaaggtttt tttgatattt agatagaggt tgtagggttg gggttgggtg agggtggtgg 2220
gtttgtgggg atattttatt gtgttaaaaa gttattgtag atatagtaat aaaaatatgt 2280
tattttttaa agtaggtttt tgtttttgtt tttgttgttt taagaagggg ttagggtgta 2340
ggaggtaggg ggaatttttt ttagttggag ttgttgtggt gagtaaggtt ttgttttgga 2400
ggtttttgag gttggtattt ttttggggat tgggaagggg gtagggaagg tagtagttta 2460
gggaaagttt tgtttttttg gagttgaggt agttggggag 2500

<210> 173
<211> 2500
<212> DNA
<213> Artificial Sequence

<220>
<223> chemically treated genomic DNA (Homo sapiens)

<400> 173

tttttaatt gttttggttt tgggggatg aggtttttt tgggttgttg tttttttgt 60
ttttttttta gtttttagaa gggtgttagt tttagaggtt tttagagtag agttttgttt 120
attgtagtag ttttagttgg aggaggtttt ttttgttttt tgtattttga ttttttttta 180
gagtagtaga ggtagaagta ggagtttgtt ttggaaaatg atatgttttt attgttatgt 240
ttgtagtggt tttttagtat agtagaatgt ttttgtaggt ttgttatttt tatttagttt 300
tagttttgta gtttttgttt gggtgttggg gaagtttttt ttggggttat ttagttgttt 360
gaggattgga tgtggtatag atatatatag gttaaagttg ttatatggaa gattggagat 420
gtgggaagtg tatttgtttt aagtttagag gtggtagttg tgtttggggt ttttttttgg 480
gggtgtgttt gttattgaaa tattaagggt ttggtttatg ttttgttgtt ggttgtatag 540
aaagttaatg attgattgag atgaagagta ttattaggga agatggtttt aattgggtgt 600
tgtagttggg gagatgggag ttaagtttta aatttatttt tttgattaaa attaggggtt 660
tatatagtta ggaagaaatg tattaatgtg taggaaaata ggaatttggg agggtttaga 720
aagtaatttt gatgaatttg gggggtttag tattttattg tttggatgtg gttatttggt 780
gagttttagt tttttgatat tttgagaggt ttgaaggtta ttttttgagg aaggaattta 840
gataaaataa ttgtaaggtt taagtttgaa gattagaagg gttagttttt atgtgtatta 900
aaaagaatag tttatgggat gattggattg gttttatgtt taatttaggg tataggatgt 960
gggaagtttg agttggtttg gtgagtagtt tagatatttg ggatggagta ttgtagtttt 1020
ttgttttata gatttgtgaa tagggtgtgg ggatagatat ttagttttta gggttatgtt 1080
atttttattt ttgtttgttt tttgtagtat tttttttgtg gatttgattt ttttttagat 1140
atagaagtaa gttagtattt agtagatata gggttatttt ttagtttttt attaggtaag 1200
agtgattagg gtagggattg atattgttga atttaggagt taggtttgat ttagttttag 1260
gtttagtagg ttttgtttgt ttatttgggt taggtttaga gtttgggagt ttttggttgg 1320
tgggaggtta tttgtaattt attttatatg tagttttagt ttttttatta ggtggggtga 1380
ttaggatagg gatagaattt gttgaattta ggagtgagat ttggttttgg gttttgttgg 1440
gtttttttgt ttattttggt tggatttggt gtttgggaga ttttggttgg tgtgaggtta 1500
tgtttattag atatgtagtt ttagttattt tattagttgg gtgattagga tagggatgga 1560
ggttgttgag tttagttaga ggtttgtttt ttggggtttg ttttgggtgt ttttttaagg 1620
atggataggg taggtagggt ttgggatgat ggttgtatag ttttggtttt gtgtttttag 1680
gtttgttttg ttttttgatg tgagggagat ttgggggatg ggataggttg ggttttgtag 1740
tgtttgattt tttgtagggt ttttgggata ggggtttggt ggatagttgg ttgtttatgg 1800
gtgaggggtt taagttggta ttgtggttat tttttggttt gggttttttt ggggaggggt 1860
ggggttggtg agaattggtt atgtgttttg gggtttattt ggggtttttt agggttggaa 1920
gtagggtttt tgtgtgtagg tgttttgagg attttgggtt gtttatttta tgttaggggg 1980
tggaattttt tgtagttttt ttgtttttgt atttttgtgg gttttgattt tttttttgag 2040
agttgggtag aggttttgga gttatgtagg ttgaaggtta gggtataggg ggtttgatgg 2100
gtgatgttga tggtttagga ggttttggta tttttgatgg tttagggggt aatgttggtg 2160
gtttaggaga ggtgggtgtt atgggtggta gaggtttttg gggtgtaggg gtagtaaggg 2220
ttttggggtt gagaggaggt gttttgtggg gtttgtatgg tggtgttgtt tttgtgtagg 2280
atggaaggtg tttttgtggg gttaggaggt tggatagttg tttgttttag ttgtattttg 2340
ttttgttttg ttttaggagg ttttggtggt gaggtggggg ttgtgagatg gagataatag 2400
ggttaagagg tggggggtag ggttaggagg tgggtgaggg tagggttagg tgtggaaggt 2460
ggggtttggg agatgggggt aaggtgttgg gtggggtggg 2500

<210> 174
<211> 2501
<212> DNA
<213> Artificial Sequence

<220>
<223> chemically treated genomic DNA (Homo sapiens)

<400> 174

tgagggtgag gtggtagaag ggttggggtt ggtgttgggg ttggagggtg gtggttaggt 60
gatggagtag gagtagggat gggggtggag gtgtagttga ggagatgggg gttgggtggg 120
gaatgggaat gtgaatgggg tgggagtggg gattggagtg tagttttggg ggtttatagt 180
tttttttttt gggtgttgat tttgatgttt ggtagagttt gtgttttgtt tttttttaat 240
tagttttgtt ttggttatag taatttttga ttttagagtt tggggagaag aatgtggtgt 300
tttttaaggt ttttggagta ggggtttggg ggtagtttag gttgagatgt ttttttgtgg 360

gtgggttagg gttggtagta agggtggtgt tggaatattt ggggtttttt atttggagtg 420
gtggggtttt tagttttttt ttagtttttt attttgtttt tttatagagt ttgggttagg 480
tttttttggt tgaattttat gttgtttttt agaatggaaa ttttgttttt ttttgtttta 540
taatgtgtaa gttagtggtg ttatatttgg gtatttggat gttttttggt gggaattgga 600
ttttgtttgg gtgggggtgg gagtgggtgt gggggtgggg gtgggggtga gggtggaatg 660
gttgggtgga tgggtattag ggttgaggga tggggtgggg gatgtggggt gggggttgga 720
atgataatga agagggggtt gggttatggg agtgagtatt ggagtggggg ttgggttatg 780
ggagtggggt tttagggatt tttagttttt tttgagaatt ttgagtatga tgtttggtag 840
agtttggatg ttgttttttt ttaagtagtt tttttttgat tatatgagtt ttgattttag 900
agtttttagt ggtttaagta gtaagggggt tgggggtggt ttagtttgag aagtttatta 960
ttagatggtt taggattggt atgaagggat tatttggggt tttttatttg gggtaagtgg 1020
gtttttagtt tttttttagt atgttatttt gtttttttat agagtttggg tttgtttttt 1080
tttattgatt ttaagttgtt ttttagattg aaaattttgt ttttttttaa ttattaatgt 1140
ttaagttagt ggtgttatat ttgggtattt gggttttttt gggttgagag taggggtgga 1200
attggatttt gtttggtttg ggttgggggt gagtgtgaag atgtttggat gggtggtgta 1260
aagggaggga ttgggggtgg tagggggagg gatgttagtg gtgttttggg gtttaagttg 1320
ttgtgttggt ggtagtgggg ttgggtgtgg ggttggggga tttttggtta ttttttaggg 1380
ttttgatttt tatgtttggt agaatttgga tatttatatt ttttgttgat tgtgtttgtt 1440
ttttttttat taagtttttg gtttttagtt ttatgaggtg gtaatgggag ggggggtgtg 1500
gttgtggtaa tattgttagg gttttttagg gttgatagga ggggttgagt tgtattttgt 1560
ggtttattta tgttgggaga gtggattttt tgtttttatt taggaaggtt ttttttttag 1620
tttttggtat tttgttgaag ttatgggtgg gagatgttgt ttttgttatt tttgagtatt 1680
tgtttagttg tatttattgt agagaatggt tgtaggtttt aggttaggtg ttttttgtag 1740
agtggtagtt tttgtgattg tagtgatttt tgggagaaga tgtatatttt tttatggggg 1800
ttttttttta gttagagtta tatttggtaa attttttgtt tttaaagtaa tatgttttat 1860
ttttaggtat ggtggtttat gtttgtaatt ttaatagttt gagaggttga ggtgggtaga 1920
ttatttgagg ttaggggttt aagattagtt tggttaatat agtgaaattt tgtttttata 1980
taaaatataa aaattagttg ggtgtggtag tgtttaggag gttgaggtgt gagaattgtt 2040
tgtatttgga ggtggagttt gtagtgaggt gagattgtgt tattgaattt tagtgtgggt 2100
tttgtgatag agtgatattt tgttttaaaa aatttaaaaa atgaaaaaga aatatatttt 2160
ttgtattttt taaggatata tgtaggtagt tgtatatata tatgtattta tttttaggaa 2220
agaatattta ttagtagtat gtatgattta tttttatatt ttattttatt ttttttttat 2280
tttttatgtt ttgttgaaat aatttttttt gaaatataat tttttattta ttatgttggt 2340
gtttggttgt tattagtgga atttttgatt tatattgttt taattagttt tgtttaattg 2400
gtgttttgtt gttgttttag ggttattagt attatgtttt gaggagttgt tttagattgt 2460
gtaatgatgt gaagtggaaa tttgaaaatt tttattgtaa t 2501

<210> 175
<211> 2501
<212> DNA
<213> Artificial Sequence

<220>
<223> chemically treated genomic DNA (Homo sapiens)

<400> 175

gttataatga aagtttttaa atttttattt tatattatta tatagtttga ggtagttttt 60
taaaatatga tgttagtagt tttagggtaa tagtaggata ttagttaaat agggttggtt 120
aagatagtat ggattaagag ttttattaat aataattaga tattaatata gtgggtgaga 180
aattgtgttt taaaaaaaat tattttaata gaatgtaaag aatggaaaag aagtagaata 240
ggatataaga gtgaattatg tatattattg gtaggtattt ttttttagaa ataaatatat 300
atatatatat agttatttat atatattttt ggaagatatg aaaagtgtat ttttttttta 360
ttttttaaat tttttgagat agagtgttat tttgttgtga ggtttatgtt agagtttaat 420
ggtatgattt tgttttattg taaattttgt ttttaggtgt aagtgatttt tatgttttag 480
ttttttgagt attattatat ttagttaatt tttgtatttt gtgtagagat ggggttttat 540
tatgttggtt aggttggttt tgaatttttg attttaagtg atttgtttgt tttggttttt 600
taaattgttg ggattatagg tgtgaattat tgtgtttggg agtaaaatat attattttga 660
ggataaaagg tttgttaagt gtagttttgg ttggagagaa gtttttgtgg gaaggtgtgt 720
gttttttttt agaggttatt ataattgtgg gagttgttat tttgtaggga gtatttggtt 780
tgggatttgt agttattttt tgtaatgggt gtagttgggt aaatgtttag agatggtaga 840
aatagtattt tttgtttatg attttaataa agtgttagga gttgggagga ggattttttt 900
gagtggggat agaggtttta tttttttgat gtagatgggt tatagaatgt agtttagttt 960
tttttgttag ttttggaaga ttttggtaat gttattatga ttatattttt ttttttattg 1020

ttattttatg ggattggggg ttagagattt ggtgggagag aagtagatat aattggtaga 1080
gaatatgggt gtttagattt tgttaggtat gaaagttagg attttgaggg gtgattgagg 1140
gttttttaat tttatgttta attttattat tattaatatg atgatttaag ttttggggta 1200
ttattggtat tttttttttt attattttta attttttttt ttatgttatt tatttaaata 1260
tttttatgtt tatttttagt ttaagttagg tagaatttgg ttttattttt gtttttaatt 1320
tagggaagtt taggtgttta gatgtgatgt tattgatttg agtattagtg gttagagaga 1380
agtgaggttt ttggtttgag gggtggtttg agattggtgg agggaagtgg gtttaggttt 1440
tgtaaggagg taaggtgata tgttgaggga ggattgagga tttatttatt ttagatagag 1500
gattttaaat aattttttta tgttagtttt ggattatttg gtggtggatt ttttaggttg 1560
ggttattttt agtttttttg ttgtttaaat tattggggat tttgaagtta gagtttgtgt 1620
gattagggaa gggttgttta ggagagggta gtgtttaggt tttgttagat attatgttta 1680
ggatttttaa ggagggttga gggtttttaa gattttattt ttgtgattta atttttattt 1740
taatgtttat ttttgtgatt taattttttt tttattgtta ttttaatttt tattttatat 1800
tttttatttt attttttaat tttgatgttt atttgtttag ttattttatt tttattttta 1860
tttttatttt tatgtttatt tttattttta tttaggtagg atttggtttt tgttaggaaa 1920
tatttgggtg tttggatgtg atgttattga tttgtgtatt gtggggtaga gagaagtgag 1980
gtttttattt tgagggatgg tgtagagttt ggttgaagga atttgattta ggttttgtga 2040
ggaggtaagg tgagaggttg agggaggatt gaggattttg ttattttaaa tagagagttt 2100
taaatatttt agtgttgttt ttgttgttag ttttggttta tttgtgggaa gatgttttag 2160
tttgggttgt ttttagattt ttgttttaaa agttttgaga gatattatgt tttttttttt 2220
aagttttgga attagaggtt gttgtgatta gggtaggatt ggttaggaga gggtagggta 2280
taggttttgt taggtattaa gattagtatt taagagggag ggttgtgggt ttttaagatt 2340
gtattttaat ttttattttt attttatttg tatttttatt ttttatttaa tttttatttt 2400
tttagttata tttttatttt tatttttatt tttatttgt tatttgatta ttattttta 2460
gttttagtat tagttttaat ttttttgtta ttttattttt a 2501

<210> 176
<211> 2501
<212> DNA
<213> Artificial Sequence

<220>
<223> chemically treated genomic DNA (Homo sapiens)

<400> 176

gtagggtttg ggtttttttt ttgttttttt gaagttgtat ttttggtatt aatatttttt 60
tttttatgtt agttttagat gttgatgtta gaatttgtgt ttttggttgt tatgattagg 120
gttttttagg gttgatatta ggggtagagt tttttttgag tgttttgata ttgatgttgg 180
tagaatttag gtgtggtttt tgttgaatta gttgggtttt ggttatatta agttttgatt 240
tttagagttt tttgtggttt aagtggtggg agagagtggg ggtgggggtg gggggggggt 300
gatagtttag tttgagaagt ttgtttttag gtggtttaga gttggtagta gaggtagtgt 360
tgtattattt agggtttttt atttgggttg ggggtgtttt tagttttttt ttagtttttt 420
attttgtttt tttatagagt ttgggtttgt tttttttagt tgattttaag tttgtttttt 480
agaatgagaa ttttgttttt ttttgatttt tgaggtgtaa gttagtggtg ttatatttgg 540
tttttggggt ttttttgggt tgatagtagg ggtggagtgg attttgttgg aattggggtg 600
ggagttgggg tgggagtgag ggtgggagtt tgggtggggt ggggtatggg tgaggatggg 660
gttggggtag gggtggtggt gaagatgagt gtggggaggg gtgtggaggt gggaattggg 720
atggggattt tggggttgtg ggttgtggtg ggggtggggg tggggttttt tagttatttt 780
ttagtgtttt gattttgatg tttggaaaag tttggatttt tgttttgtgt tgaggatgtt 840
tttttttttt aaattaggtt ttttgatttg gagtttttta aggtggtagt gaggggaggg 900
ttgtggtagg ggtgataata ttgttagggt tttttagggt tgataagagg ggtttagttg 960
gattttgtgg tttttttgtg aggggtgggt ggatttttgg tttttattta ggagggtttt 1020
ttttttggtt tttgtttttt tgatgaagtg atgggtggga gatgtttagt ttttgttatt 1080
tttgattatt tgtttagttg tattttttgt agagtatggg tgtggttttt aggttagatg 1140
gtttttgggg agttgtaata tttgtgattg taatgattta tgggagaaga tgtatatttt 1200
tttaatgggg ttttttttaa gttataagta gatttggtaa attttttgtt ttaaagggta 1260
tattttttat aagaaagagg ttgagtattg gggtgtggag aggttttgtt gttttgagtt 1320
tagttgatat tttatagttt aatgtagtga tgtttggttt tttaattttt atttttagta 1380
aaaatatgtg ttttaaatgt ttgttaagtg tatattgaat atatatataa tattttatga 1440
aagaatattt attttatgtt gtgtggttta ttgtttattt tatttttggt ttatttttta 1500
tattttatta taataatttt tttttttttt tttgagtaag aaaatttaat ataaagaatt 1560
attaatttgt agtagaggat taattaagat tagttgttat tgtggtggtt ttaggtggtt 1620
agtagtatag attttggagt tatatttttt taattaattt tgtttttttt gttgttttga 1680

tattatttta gagttattgg gttattggta ttgtggtatg aagtgttgtt ttagattgtg 1740
taataaagta gggttagaaa tttaaatatt ttttttattt tgttttttta tttgttgttt 1800
atgtaggttg tggatttttg taaatgtatt gttgtttttt atttttttta ttttaaatat 1860
aaggtttaat ttgaaatttt gtagtttgtt tagggtttta ttatgttttg ttgtgtagaa 1920
tttgttgagt gttgataatg tttttttttt tttttttttt aggattgatt ttggagtgta 1980
agttagtaat atgaatttga tgtttatttt ggtttaaagg taaggtatta attttgtaaa 2040
taatttaagg aaaatttatg tttttaaaat attttgtttt ttattaatgg atatggtata 2100
atgtttattt tatttgggat ttttttattt tgaatttatt agtaaatttt tatagttgat 2160
tttagtaaga ttttgtttat ttttgttagg attattttta aggtaatttt aggagttttt 2220
tgttgttttt aatgggatat tttttattgt atgtttattt tattgttttt ttatgattag 2280
tttttttgatt ttgttataat gagttttttat aaaatatttt tttttgaag tttttttattt 2340
atttaaatat tttatatgtt tatttttttg attatttgtt aaagataata ttgtttttaa 2400
atattttttt ttttaaaatt tatgtatttt atttagtttg atatttatag ttttggttat 2460
gtgttttagt gtagttttga atagtagaga aggtagtata t 2501

<210> 177
<211> 2501
<212> DNA
<213> Artificial Sequence

<220>
<223> chemically treated genomic DNA (Homo sapiens)

<400> 177

gtatgttatt ttttttattg tttaaaattg tattgaaata tatagttaga gttatgggta 60
ttaaattaaa taagatgtat gaattttgaa aagagagatg tttggagata atgttatttt 120
taatgaatag ttaaaggaat aagtatgtgg aatatttaaa tgaataaaag attttagaag 180
aaaaatgttt tatggaagtt tattatagta aaattaagaa attggttata aaaaaatagt 240
aaatagaata tataatagaa aatattttat taaaaatagt aaaaaatttt taaaattatt 300
ttaaagataa ttttaataaa aatgagtagg attttattgg agttaattgt aaaagtttgt 360
tgatagattt aggtgaaaga agttttaaat aaataaagta ttatattata tttattaatg 420
gagaatagaa tattttaaag gtgtgaattt tttttaaatt atttatagaa ttagtgtttt 480
atttttaggt taaaatgagt attaagttta tattgttgat ttatatttta gaattaattt 540
tggaggggag aagggaaaaa atattattaa tatttaataa attttatata gtaaaatatg 600
atagaatttt aagtagattg tagaattttg aattggattt tgtgtttgga gtagggggag 660
tggaggataa tagtgtattt gtggaggttt gtagtttgta tagatggtaa ataagaggat 720
gagtaggaag aaatatttga atttttggtt ttgttttatt gtataatttg aggtaatatt 780
ttatattatg atgttagtag tttagtagtt ttggagtgat attaggatag taggaaagat 840
ggggttgatt gagggagtat gattttggag tttatgttgt taattattta aaattattat 900
agtgatgatt ggttttagtt gattttttgt tataagttaa taatttttta tattaaattt 960
ttttgtttag aaaaaaagag aaaaattatt ataatagaat ataaagaata gattagaaat 1020
agaataagta gtgaattata tagtgtaagg taggtatttt tttatgaaat gttatatatg 1080
tatttaatat atatttggta gatatttaaa atatatattt ttattaggaa tgagggttag 1140
aaaattagat attattgtat tggattataa aatattagtt aaatttaaaa tagtaggatt 1200
tttttatatt ttgatgttta gttttttttt tgtagaaaat atatttttta ggataaaaag 1260
tttgttaagt ttatttgtgg tttgggaaga gttttgttgg gaaggtgtgt gttttttttt 1320
ataggttatt ataattatgg gtgttgtagt tttttaggga ttatttggtt tgggaattgt 1380
atttatgttt tgtaagggat gtagttgggt aaatgattag aggtgatgga aattgagtat 1440
tttttattta ttattttatt aaagagataa aagttaggag gaagattttt ttgagtgagg 1500
attgagggtt tatttatttt ttataaaggg gttatagaat ttagttgagt tttttttgtt 1560
agttttggaa gattttggta atgttgttat ttttattata attttttttt tattgttatt 1620
ttggaagatt ttgagttaag agatttggtt taaggggaga agatattttt agtataaaat 1680
aggggtttag gtttttttag gtattaaggt tgagatattg agggatgatt gaaagatttt 1740
atttttattt ttattatgat ttatagtttt aagattttta ttttagtttt tatttttatg 1800
ttttttttta tatttatttt tattattatt tttgttttaa ttttattttt atttgtattt 1860
tattttattt ggatttttat ttttattttt attttaattt ttattttagt tttggtagaa 1920
tttattttgt ttttgttgtt aatttaggga agttttgggg attggatgtg atgttattaa 1980
tttatgtttt ggggattaga gagaagtgag gtttttgttt tgagggatag gtttgagatt 2040
ggttgaagag agtgggttta ggttttgtga ggaggtaagg tgagaggttg agggaggatt 2100
gaggatgttt ttaatttaga tagaaagttt taaataatat agtgttgttt ttgttgttag 2160
ttttggatta tttgagggta gattttttag gttgggttgt tatttttttt ttatttttgt 2220
ttttattttt ttttgttgtt taagttgtag gggattttgg gagttagagt ttggtgtgat 2280
tagggtttgg ttggtttggt gggagttgtg tttaggtttt gttagtatta atgttgggat 2340

gtttagggag ggttttgttt ttgatgttgg ttttgggaag ttttggttat ggtgattagg 2400
aatgtaaatt ttgatgttgg tatttgaggt tgatgtaggg aaggggatat tggtattaag 2460
aatgtggttt taggggagta gagggaaggt ttgggttttg t 2501

<210> 178
<211> 2501
<212> DNA
<213> Artificial Sequence

<220>
<223> chemically treated genomic DNA (Homo sapiens)

<400> 178

taggggtaaa gttttgattt tgttaggatg ggtggggatg ggaattgggg tttgggggtt 60
ttgttgatgt ttgagtgatg gtggtgggtt tggtgttggg ggatgggggt ggggttggtg 120
ttgggggatg ggaatggtgg tgggggtgtg ggggggttat ttttagtttt tttttggggt 180
tttgattttg atttttggtt gagtttgggt tttgagtatt tttaattagt tttgttttgg 240
ttatataaag tttttatatt agagtttttt gtggttaaag tggtgggggg tggtggggtg 300
aggtggtggt gattgagttt gagaagtttt tttttgtagg gtggtttagg tttggtagta 360
gaggtagtgt tggattattg gggttttttt gtttggggtg aggtttgttt tagttttttt 420
ttggtatttt attttgtttt tttatggagt ttgtgtttgt ttttttttat tgatgttagg 480
ttgttgttta gggtgagaat tttgttgttt tttgattttt aatgtgtaag ttagtggtgt 540
tatatttggt tttgtgattt ttttgggttt atagtagggg tggaattgga ttttttttgg 600
atggggattt agatggaggt ggaggtaagt atgaagatgg tgttgggggt aggggtaggg 660
atagggatgg ggattttgag gttgtagatt ttggtggggt tgggggagtt gggggtggga 720
ggggtttttg gttatttttt aaggttttga tttggatgtt aggttgagtt tggattgtta 780
ttttttgttg attgtgtttg ttttttttag attaagtttt tgattttgag tttttttgaa 840
gtggtattgg gggaggggtg tggttggggt gatattattg tttaagttta ttagggttga 900
taagagggat tgagttggat tttgtcgttt tattatgtgg gggagggtgg atttttagtt 960
tttatttaag agggtttttt tttttatttt tggttgttgg atgaagtgat tggtaggaga 1020
tattttgttt ttgtaatgtt tgtgtatttg tatagttgta ttttttgtag agaatggttg 1080
tgggttttag gttaggagtt ttttgtaggg tgggagtttt tatgattgta ttgatttttg 1140
gaagaagata gatatttttt tatgggagtt tttttttagt tagagttata tttggtaaat 1200
ttttggtttt aaatgattta tttattgaat tgaagaaata tggtttatat attttttaag 1260
tatatatgta ggtagtgata tatatattta tttttatgaa ttaatattta tttttagtat 1320
gtattgttta tttttattat tttaatttat ttttatttta gtttttgtgt tttgttgaag 1380
gaattttttt ttaaatgtaa tttttttttt attatgttgg ttttaggttg ttattagtag 1440
aattttggag ttatgttgtt ttaataaatt taattttttt ggtgttttgt tattatttag 1500
ggtttttggt attgtgtttt taggagttgt tttagattgt gtaatgaagt gaagtggaaa 1560
ttttaaaatt tttattttat ttattttgtt atttgttgtt taggtaggtt ataaattttt 1620
ttaatatttt tgttgttaat tttttttaat tgatatggta agtgttatat ataagtatta 1680
gtttaataag gttttagttt ttattttgga ttggttttgt tttgttgtgt agattttttt 1740
gttaatattg gttttttatt ttttgttttt attttataaa tttatagggt tgatttggga 1800
agtagagtat agtagtttaa atttgtttgt aatgttggtg attataggta aggtattaaa 1860
ttgtaaataa tttaagttga gtttatattt ttatattgtt gtgtttttta tgaatgaata 1920
tgttatattt ttttatgtat gttaagttat ttttttttga atatgttagt atattttgat 1980
agttgttttt ataagtagtt tatatatttt tattagattt ttatttaggt aatttttgga 2040
ttttattgtt attgtaaatg gtatattgtt tattaattgt tgtagttagt tattgttttt 2100
gtgaggtgaa gattttgatt ttgttgtagt aatttgttgt atattttatt ttgaaggttg 2160
atgtatttga atattttata tgtttttttt tttgaatttt taagattgat aatggtatta 2220
ttattaaata ttatatgttt ttttaatatt tatattattt atttattttg atatttatag 2280
ttttttgtgt tttaattttg attttaaatg tagagataat aggttttatt tattttttgt 2340
ttttgattgg aatgatagtt ttttgaatat ttgatattta agtatgtttg ttgttgattt 2400
taggaaatgg aagtgttttt tgaatatagg ttaaataaat attttttttt ttttataaat 2460
ttattaaatg tttttaaaaa gtttttttttg tatttttttga t 2501

<210> 179
<211> 2501
<212> DNA
<213> Artificial Sequence

<220>
<223> chemically treated genomic DNA (Homo sapiens)

<400> 179

gttaaagggt atagaaaaag ttttttaaag atatttaatg agtttatgga aaaaagaaaa 60
tatttattta atttatattt aaagagtatt tttatttttt aaaattaata gtaaatatat 120
ttaaatgtta aatgtttgaa gaattattat tttagttagg aatgagaagt agatgaagtt 180
tattattttt atgtttgaaa ttaggattga gatataaaaa gttataagta ttaaaatgag 240
taagtggtat gaatattgga aaaatatgta atgtttagtg ataatattat tattgatttt 300
gaaaatttaa aggaaaaggt atgtggaata tttaagtgta ttggttttta gaatgaggtg 360
tgtaatagat tattatagta aaattaaaat ttttatttta tgaaagtaat aattaattat 420
agtaattaat agataatatg ttatttgtaa tagtaataaa atttaagaat tatttgaata 480
gaagtttaat aaaaatgtat aagttgttta tagaggtaat tattgaagta tattgatata 540
tttaggaaaa aatagtttga tatatataga ggagtataat atatttattt atggggaata 600
taatagtgta aaaatgtaaa tttagtttaa attatttata atttagtgtt ttatttgtag 660
ttgttgatat tataagtaaa tttgggttgt tgtgttttgt tttttaaatt aattttgtaa 720
atttatagaa tggaaataga aagtaaaaaa ttagtattag taagaaaatt tatataataa 780
aatagggtta gtttagggtg agattgaaga ttttgttgag ttggtatttg tgtgtgatat 840
ttgttgtgtt ggttagaggg ggttagtggt agggatgttg gggaggtttg tagtttgttt 900
agatggtaga taatagaatg ggtagaataa aagttttaaa atttttattt tattttattg 960
tataatttga ggtagttttt gaaaatatga tgttaagagt tttaggtaat agtaggatat 1020
taggaaaatt gggtttgtta aggtagtatg gttttagagt tttgttaata ataatttgaa 1080
attaatatag tgggagagga attatgttta aaaaaaaatt tttttaatag aatataaaga 1140
ttggaataga agtagattag gatagtaaga gtgaatgatg tatattaagg gtaggtgtta 1200
gtttgtagaa ataaatatat atattgttat ttatatatat atttggaaga tatgtaaatt 1260
gtattttttt aatttagtga ataaatta[?]t taggattaaa ggtttgttaa gtgtagtttt 1320
ggttggagag gagtttttgt gggaaagt[?][?] ttgttttttt ttagaggtta atatgattgt 1380
gggagttttt gttttgtagg gagtttttgg tttgggattt gtagttattt tttataaggg 1440
gtgtagttgt gtaaatgtat agatgttata gaaatagagt attttttgtt aattatttta 1500
tttaatagtt aggagtgagg aagaggattt ttttgagtga ggattgaggg tttatttttt 1560
tttatgtagt gggattatag aatttagttt agtttttttt gttagttttg ataaatttag 1620
gtaataatgt tattttgatt gtattttttt tttagtgtta ttttaggggg atttagagtt 1680
agagatttgg tttgagggga gtagatataa ttggtagagg atggtggttt aggtttagtt 1740
tggtatttaa gttaggattt tgagggatga ttaaaggttt tttttatttt taattttttt 1800
aattttatta ggatttatag ttttaggatt tttgttttta tttttatttt tatttttaat 1860
attattttta tgtttatttt tatttttatt tggattttta tttaggaaga atttagtttt 1920
atttttgttg tgaatttagg gaagttatgg ggttggatgt gatgttattg atttgtgtgt 1980
tggaggttag agaatagtga gatttttgtt ttgagtaatg gtttgatgtt ggtggaggga 2040
agtaggtgta ggttttgtga ggaggtaagg taagatgttg agggaggatt gaggtgggtt 2100
ttattttaga tagagggttt ttaataattt agtgttgttt ttgttgttag gtttggatta 2160
ttttgtaggg gaagattttt taggtttagt tgttattatt ttattttgtt attttttgtt 2220
gttttaattg tagggaattt tggtgtaaga gttttgtgtg attagggtag ggttggttag 2280
aagtgtttag ggtttagatt tagttaggaa ttaaggttag gattttaaga ggggattgag 2340
ggtaattttt ttgtattttt attattattt ttatttttta atattaattt tatttttatt 2400
ttttaatatt aaatttatta ttattgttta aatattaatg ggatttttaa attttgattt 2460
ttatttttat ttattttggt agaattggag ttttgttttt g 2501

<210> 180
<211> 2501
<212> DNA
<213> Artificial Sequence

<220>
<223> chemically treated genomic DNA (Homo sapiens)

<400> 180

gttgtatatg tgaatgttag gatttgtgtg tttggttgtt atttttggag tttttgtggg 60
ttgattgtag gggtaaagtt tggattttgt taggatggtg gtgggggagg agatgggaat 120
ggggtttggg ggtggggttg gtgtttgagg gatgggggtg gggttggtgt tgggggatgg 180
gattggtagt gagggtaggg ggtgggttgt ttttagtttt tttttggggt tttgattttg 240
atttttggtt gagtttgggt tttgagtatt tttaattagt tttgttttgg ttatataaag 300
tttttatgtt agagtttttt gtggttaaag tggtgggggg tggtggggtg aggtggtggt 360
gattgagttt gagaagtttt tttttgtagg gtggtttagg tttggtagta gaggtagtgt 420
tggattattg gggggttttt gtttggggtg aggtttgttt tagttttttt ttggtgtttt 480

attttgtttt tttatggagt ttgtgtttgt ttttttttgt tgatgttagg ttgttgttta 540
gggtgagaat tttgttgttt tttgattttt aatgtgtaag ttagtggtgt tatatttggg 600
tttgtgattt ttttgggttt atggtaaggg tggaattgga ttttgtttgg atggggattt 660
ggatgggggg gtgggggtaa gtatgaagat ggtgttggtg taggggtagg gattgggatg 720
gggattttga ggttgtagat tttggtgggg ttgggggagt tgggggtggg aggggttttt 780
ggttattttt taaggttttg atttggatgt tagattgagt ttggattgtt attttttgtt 840
gattgtgttt gtttttttta gattaagttt ttgattttga gttggtttga agtggtattg 900
gggggagggg tgtgtttggg gtgagattat tgttagtgtg tattagggtt gataagaggg 960
gttgagttgg attttgtggt tttattatgt gggtgagggt ggatttttag tttttattta 1020
ggagggtttt tttttttatt tttggttgtt tgatgaagga atgggtggga gatgttttgt 1080
ttttgttatt tttgagtatt tgtgtatttg tgtatttgta ttttttgtag agaatggttg 1140
tgggtgttag gttaggtgtt ttttgtaggg taggagtttt tgtgatgata gtgatttttg 1200
ggagaagatg tatatttttt tatgggggtt tttttttagt tagagttata tttggtaaat 1260
ttttggtttt aaatgattta ttttttgaat tgaagaaata tggtttatat attttttaag 1320
tatatatgta ggtagtaata tatatattta tttttatgaa ttaatattta tttttagtat 1380
gtatgattta ttttgatatt ttagtttatt tttattttag tttttgtgtt ttgttgaaag 1440
aattttttta aaaaatataa tttttttttt attttgttgg ttttaggttg ttattagtag 1500
aattttggag ttatgttgtt ttaataaatt taattttttt ggtggatatt gttattattt 1560
agggtttttg gtattgtgtt tttaggggtt gttttagatt gtgtaatgaa gtgaagtgga 1620
aaatttaaaa tttttatttt atttattttt ttatttgttg tttaggtagg ttataaattt 1680
ttttaatatt tttgttgtta atttttttta atagatattg taagtgttat atataagtat 1740
tagtttaata tagtttttag ttttattttg ggtttttttt gttttgttgt gtagattttt 1800
ttgttgatat tggtgtttta tttttttgttt ttattttata aatttatagg gttgatttgg 1860
gaagtagagt atagtagttt aaatttgttt gtaatgttgg tggttatagg taaggtatta 1920
aattgtaaat aatttaagtt gagtttatat ttttatattg ttgtgttttt tgtgaatgaa 1980
tatgttatat ttttttatgg attttaagtt tttttttttt gaatatgtta gtatattttg 2040
gtagttgttt ttataaataa tttatatatt tttattaggt ttttatttag gtaatttttg 2100
gattttgttg ttattgtaag tggtatattg tttattaatt gttgtagtta gttattgttt 2160
ttgtgaggtg aagattttga ttttgttgta gtaatttgtt gtatattttg ttttgaaggt 2220
tgatgtattt gaatattttg tatatttttt tttttgaatt tttaagattg ataatggtat 2280
tattattaaa tattatatgt ttttttaatg tttatattat ttatttattt tggtatttat 2340
agttttttgt gttttaattt tgattttaaa tgtagagata ataggttata tttatttttt 2400
gtttttgatt tgaatgataa ttttttgaat atttgatatt taagtatgtt tgttgttgat 2460
tttaggaaat ggaagttttt ttttagtata ggttgtttaa a 2501

<210> 181
<211> 2501
<212> DNA
<213> Artificial Sequence

<220>
<223> chemically treated genomic DNA (Homo sapiens)

<400> 181

tttaggtaat ttatattgaa agagaatttt tattttttaa aattaatagt aaatatattt 60
aaatgttaaa tgtttgaaga attattattt aagttaggaa tgagaagtag atgtagttta 120
ttatttttat atttgaaatt aggattgaga tataaagagt tgtaagtatt gaaatgagtg 180
agtggtatga atattggaaa aatatgtagt gtttagtgat aatattatta ttgattttga 240
aaatttaaag gaaaaggtat gtggaatatt taagtgtatt ggtttttaga atgaggtgtg 300
taatagatta ttatagtaaa attaaaattt ttattttatg aaagtaataa ttaattataa 360
taattgatag ataatatgtt atttgtaata gtaataaaat ttaagaatta tttgaataga 420
agtttaataa aaatgtataa gttgtttata gaggtaatta ttgaagtata ttgatatatt 480
taggaaaaaa gagtttgaaa tttatagagg agtataatat atttatttat ggggaatata 540
atagtgtaaa aatgtaaatt tagtttaaat tatttataat ttagtgtttt atttgtagtt 600
gttgatatta taagtaaatt tgggttgttg tgttttgttt tttaaattaa ttttgtaaat 660
ttatagaatg gaaatagaaa gtaaaatatt agtattagta agaaaattta tataataaaa 720
tagggaaagt ttagagtgag attggagatt gtgttgagtt ggtatttgtg tgtgatattt 780
gtagtgtttg ttggaggggg ttagtggtag agatgttggg gaggtttgta gtttgtttag 840
atggtagatg aaagaatggg tagaataaaa gttttgaatt ttttatttta ttttattgta 900
taatttgagg tagtttttga aaatatgatg ttaagagttt taggtaatag tagtgtttat 960
taggaaaatt gggtttgtta aggtagtatg gttttagagt tttgttaata ataatttgaa 1020
attaatagag tgggagagga attatgtttt ttaaaaaaat ttttttaata gaatataaag 1080
attggaatgg aagtagatta ggatattaga gtgaattatg tatattaagg gtaggtgtta 1140

gtttatagaa ataaatatat atattgttat ttatatatat atttagaaga tatgtaaatt 1200
gtattttttt aatttagaga ataaattatt taggattaaa ggtttgttaa gtgtagtttt 1260
ggttggggag gagtttttgt gggaaggtgt gtgttttttt ttagaggtta ttattattgt 1320
gggagttttt gttttgtagg gagtatttgg tttggtattt gtagttattt tttataaggg 1380
gtgtagatgt gtagatgtgt agatgtttag aggtgataga aatagagtat tttttattta 1440
tttttttatt aaatagttag gagtgaggaa gaggattttt ttgagtgagg attgaggatt 1500
tatttttatt tatatagtgg gattatagaa tttagtttag ttttttttgt tagttttggt 1560
atatattggt aatgatttta ttttgagtat attttttttt ttaatgttat tttgggttga 1620
tttagagtta gagatttggt ttgaggggag tagatataat tggtagagga tggtggttta 1680
ggtttagttt ggtatttaag ttaggatttt gagggatgat taaaggtttt ttttattttt 1740
aatttttttg attttattag gatttatagt tttaggattt ttgttttaat ttttattttt 1800
atattaatat tatttttatg tttattttta tttttttatt tagattttta tttgggtaga 1860
atttggtttt atttttgttg tgaatttagg gaagttatgg gtttggatgt gatgttattg 1920
atttgtgtat tggaggttag aggatagtga gatttttgtt ttgagtaatg gtttgatgtt 1980
ggtggaggga agtaggtgta ggttttgtga ggaggtaagg taagatgttg agggaggatt 2040
gaggtgggtt ttattttaga tagagggttt ttaataattt agtgttgttt ttgttgttgg 2100
gtttggatta ttttgtaggg gaagattttt taggtttagt tgttattatt ttattttgtt 2160
attttttgtt gttttaattg tagggaattt tggtgtaaga gttttgtgtg attagggtag 2220
ggttggttag aagtgtttag ggtttagatt tagttaggaa ttaaggttag gattttaaga 2280
ggggattgag ggtaatttat tttttatttt tattattaat tttatttttt aatattaatt 2340
ttatttttat tttttaaata ttaattttat ttttaaattt tatttttatt ttttttttta 2400
ttattatttt ggtagaattt gggttttgtt tttgtaatta atttatggaa gttttgggaa 2460
tggtggttaa gtatgtggat tttgatgttt atatgtatgg t 2501

<210> 182
<211> 2501

<212> DNA
<213> Artificial Sequence

<220>
<223> chemically treated genomic DNA (Homo sapiens)

<400> 182

tatttttgga gtttttgtga tttgatatta ggggtaaagt ttggattttg ttaggatagg 60
ggtgggggtg gggatgggaa tgggggatgg gggatggggg tggggttggt gttgggggat 120
gggaatggtg gtgagggtgg tggttaggtt gtttttagtt tttttttggg gttttgattt 180
tgatttttgt tatagtgtgg gttttgattt tttttaatta gttttgtttt ggttatatta 240
agttttgttt ttagagtttt ttgtggttaa agtggtgggg gttggtgggg tgaggtagtg 300
gtgatttagt ttggaagttt ttttttgtgg ggtggtttag gtttggtagt agaggtagta 360
ttggattatt tgaggttttt tgtttgaggt gaggtttgtt ttagtttttt tttagtgttt 420
tattttgttt ttttattgag tttgggttgg tttttttttg ttgatgttag gttgttgttt 480
gttgtttagg gtgagaattt tgtggttttt tgatttttaa tgtgtaagtt agtggtgtta 540
tattttggta atggtattat tttgggtttt gggtaggggt ggaattggat tttgtttgga 600
tggggattgg atgggggtgg aggtaagtat gaagatggtg atggggtaag gggtaaggat 660
tgggatgggg gttttgaggt tgtagatttt ggtggggttg ggggagttgg gggtgggtgg 720
gtggggtttt tggttatttt ttagggtttt gaagttgatg tttggttgag tttggattgt 780
tattttttgt agattgtttt tgtttttttt agattaagtt tttgattttg agtttttttg 840
aagtggtatt gggggaaggg gtgtggtttg ggtgatatta ttgtttgtgt ttattagggt 900
tgataagagg ggttgagttg gattttgtgg tttttttatg gtgtgtgggg tgggggggtg 960
gtgtggattt ttagttttta tttaggaggg ttgtttttgt tatttttggt tgttggatga 1020
agtgatgggt aggagatgtt ttgtttttgt tatttgtgag tatttgtata gttgtatttt 1080
ttgtagagaa tgtttgtggg ttttaggtta ggtgtttttt gtagggtggg gtggtagttt 1140
ttgtggttgt agtgattttt tattttagtt tttgtgtttt gttgaaagaa tttttttttt 1200
taaaaaatgt aatttttttg ttattatggt ggttttaggt tgttattagt agaattttgg 1260
agttatattg ttttaattaa ttaaattttt ttggtgtttt gttattattt agggtttttg 1320
gtattgtgtt tttagaggtt gttttagatt gtgtaaagaa gtgaagtgga aaattttaaa 1380
tttttatttt atttattttg ttatttattg tgtaggttgg ttataaattt ttttaatatt 1440
tttgttgtta atttttttta attaatattg taagtgttaa atataagtat tagtttaata 1500
tagttttgag ttttattttg ggtttttttt gttttgttgt gtagattttg ttgttgatat 1560
tggtttttta tttattgttt ttattttgta attttttagg tttgatttgg gaagtagagt 1620
atagtagttt aaatttgttg taatggtggt ggttataggt aaggtattaa attgtaaata 1680
atttaagttg agtttatatt tttatagtgt tgtgtttttt atgaatgaat atattatatt 1740

tttttatgta ttttaagttt ttttttattt gaatatgtta gtatattttg atagttgttt 1800
ttataaagat tttatatatt tttattagat ttttttttag gtaatttatg aattttgttg 1860
ttattgtaaa tggtatattg tttattaatt gttgtagtta gttattgttt ttgtgaggtg 1920
aagattttga ttttgttgta gtaatttgtt gtattttttg ttttgaaggt tgatgtattt 1980
gaatatttta tatatttttt tttttgaatt tttaagattg ataatggtat tattattaaa 2040
tattatatat ttttttagta tttatattat ttatttattt tggtatttat agttttttgt 2100
gttttaattt tgattttaat tgtagagata atagtttata tttatttttt gtttttgatg 2160

tgaatgataa ttatttgaat gttttgtatt taagtatgtt tgttgttgat tttaggaagt 2220
gtaatttttt tttgaatata ggttaaatat tttttttttt ttttattatg aatttatatt 2280
aaattttttt aaaaagtttt ttttgtattt gttgatataa ttaaattttt ttttttttta 2340
ttttgtatgt aaggaattat aatgaaattg tttttttttt tttttttaat gttaaattgt 2400
atgttatttt tttgggtaga gtttgtttat ttaatatgtt agtaatttat gtaggatatt 2460
tgttgttatg taagggagtt tatttaatgt gtgttttttt t 2501

<210> 183
<211> 2501
<212> DNA
<213> Artificial Sequence

<220>
<223> chemically treated genomic DNA (Homo sapiens)

<400> 183

aaaaaaagta tatattaagt aagttttttt atatagtagt aaatatttta tgtaaattat 60
tagtatatta aataaatagg ttttgtttaa ggaaataata tatggtttaa tattagggga 120
aaaaaaaaaa atggttttat tgtaattttt tatatataaa atggaggaga aaaaaatttg 180
attgtgttaa tgggtataga aaaagttttt taaagaaatt taatatgagt ttatggtaaa 240
aaaaaaaaaa agtatttaat ttatatttaa agagaaattg tattttttaa aattaatagt 300
aaatatattt aaatgtaaaa tgtttgaata attattattt atgttaggaa tgagaagtag 360
atgtaggtta ttatttttat gattgaaatt aggattgaga tataaagagt tataagtatt 420
aaaatgagta agtggtatga atattggaag aatatgtaat gtttagtgat aatattatta 480
ttgattttga aaatttaaag gaaaaggtat gtggaatatt taagtgtatt ggtttttaga 540
atgaggagtg taatagatta ttatagtaaa attaaaattt ttattttatg aaagtaataa 600
ttaattataa taattaatag ataatatgtt atttgtaata gtaataaaat ttatgaattg 660
tttgaaaaga agtttaataa aaatgtataa ggtttttata gagataattg ttgaagtata 720
ttgatatatt taggtaaaaa aaagtttgaa atatatagag gagtataata tatttattta 780
tggggagtat aatattgtaa aaatgtaaat ttagtttaaa ttatttataa tttagtgttt 840
tatttgtagt tgttgttatt ataataaatt tgggttgttg tgttttgttt tttaaattaa 900
atttggaaaa ttatagaatg gaaatagtaa ataaaaaatt agtattagta ataaaattta 960
tgtaataaaa tagggaaagt ttagggtgag atttaaggtt gtgttgagtt ggtatttgtg 1020
tttgatattt gtagtgttgg ttggaggggg ttagtagtag ggatgttggg gaggtttgta 1080
gttggtttat atggtagatg atagaatggg tagaataaaa gtttgaaatt ttttatttta 1140
tttttttgta taatttgagg tagtttttga aaatatgatg ttaagagttt taggtaatag 1200
taggatatta ggaaaatttg gttggttaag gtagtatgat tttagagttt tgttaataat 1260
aatttgaaat tattatagtg gtagaggaat tatatttttt aaaaaaaaaa atttttttaa 1320
tagaatataa agattgaaat aggaggttat tataattgtg ggagttgttg ttttgttttg 1380
tagggagtat ttggtttggg atttgtaggt attttttata aggggtgtag ttgtgtaaat 1440
gtttataggt gatagaaata gagtattttt tgtttattat tttatttaat agttagaggt 1500
gatgaagatg atttttttga gtgaggattg agggtttata ttgttttttt attttatata 1560
ttatagaggg attatagaat ttagtttagt tttttttgtt agttttggta aatgtaggta 1620
gtgatgttat ttagattata tttttttttt taatgttatt ttagggggat ttagagttag 1680
agatttggtt tgaggggagt agaagtaatt tgtagaggat ggtggtttag gtttagttag 1740
gtattaattt taggattttg agggatgatt gaaggttttg tttatttatt tttaattttt 1800
ttgattttat taggatttat agttttagga tttttgtttt aatttttatt ttttgtttta 1860
ttattatttt tatgtttatt tttattttta tttgattttt atttaggtag aatttagttt 1920
tatttttgtt tggaatttag ggtagtattg ttgttaggat gtgatgttat tgatttgtgt 1980
attggaggtt agaagattgt gagatttttg ttttgagtaa tgagtgatgg tttgatgttg 2040
gtggagggaa gttggtttag gtttggtgag gaggtaaggt aagatgttga gggaggattg 2100
aggtgggttt tattttagat agaggttttt aaataattta gtgttgtttt tgttgttggg 2160
tttgggttat tttgtagggg aagattttta ggttgggttg ttattatttt attttgttga 2220
tttttgttgt tttagttatg gggaattttg gggatagagt ttaatgtggt tagggtaggg 2280
ttggttagaa gaggttaggg tttatgttgt ggtaggaatt aaggttagga ttttgagagg 2340

gaattgaggg tagtttaatt attattttta ttattatttt tgttttttaa tattaatttt 2400
atttttattt tttatttttt atttttattt ttatttttat ttttattttg gtagaatttg 2460
ggttttgttt ttggtattaa gttatggaag ttttgggaat g 2501

<210> 184
<211> 2501
<212> DNA
<213> Artificial Sequence

<220>
<223> chemically treated genomic DNA (Homo sapiens)

<400> 184
   gggaagtggg ttaggttttg tgaggagtta aggtgagatg ttgagggagg atttaggagg 60
   tttttatttt agatagatga ttttaaataa ttttgtatta tttttgttat tagttgtggg 120
   ttatttgtgg gtggattttt gagtttgggg tgtttattat tttattgttt ttgaagttgt 180
   aggggatttt ggagttagag tttgggttga ttagtgtaag attagtgagg gtaggttttg 240
   ttaggtatgg attttagtat tttaagagag ggttaagtgt attttatttt tatttttatt 300
   ttttattatg tttttttttt gattattatt tgtattttta aattatttat gtttttattt 360
   tttattagta tttttttttt ttttaatttt ttattttttt tatattgtta ttttttattt 420
   taataatttg ggttttttta ttaattttat tttttttatt tttatttata ttgaattatg 480
   ttttgttttt gtttttaatt tatttttaaa aatttgtttt ttttattgat tttatttttt 540
   ttatttttat ttatgttgaa tttgattttt gttttttttt ttaatttaag aaagttttag 600
   gggtttggat gtgatgttat tgatttgtgt attgggggtt agagagaagt gagttgtttt 660
   gtttgattag tagtttggga ttggtggagg gaagtgggtt aggttttgtg aggagttaag 720
   gtgagatgtt gagggaggat ttaggaggtt tttattttag atagatgatt ttaaataatt 780
   ttgtattatt tttgttatta gttgtgggtt atttgtgggt ggatttttga gtttggggtg 840
   tttttatttt tattgtgttt gaagttgtat gggattttgg agtttgagtt tggggtggtt 900
   agtgtaagat tagtgagggt aggttttatg aggtattaat tttaggattt taagagaggg 960
   ttaagtgtat tttattttta tttttatttt ttattgtttt ttttttttta ttattatttg 1020
   tatttttaaa tttatttgtg tttttatttt ttattagtat ttttattttt aattttgtat 1080
   tttttttata ttgttatttt ttattttaaa aattggggtt tttttattaa ttttattttt 1140
   tttattttta tggatgttga attgggttgt gtttttgttt ttaatttatt ttttaaaatt 1200
   tgtttttttt attgatttta ttttttttat ttttttttat gttgaattgg gtttttgttt 1260
   ttgtttttaa tttaagaaag ttttaggtgt ttggatgtga tgttattgat ttgtgtattg 1320
   ggggttagag agaagtgagt tgttttgatt agttgtttgg gattggtgga gggaagtggg 1380
   ttaggttttg tgaggagtta aggtgagatg ttgagggagg atttaggagg tttttatttt 1440
   agatagatga ttttaaataa ttttgtatta tttttgttat tagttgtggg ttatttgtgg 1500
   gtggattttt gagtttgggg tgtttattat tttattgttt ttgaagttgt agaggatttt 1560
   ggagttagag tttagggtgt ttagtgtaag attagtgagg ttaggttttg ttaggtatta 1620
   attttaggat tgtaagagag ggttaagtgt attttatttt tatttttatt ttttattatg 1680
   tttttttttt tattattatt tgtattttta aatttatttg tgtttttatt ttttattagt 1740
   attttttttt gattttttat ttttgttata ttgttatttg ttattttaaa aattggggtt 1800
   tttttattaa ttttattttt tttattttta tttatgttga attgggtttt gtttttgttt 1860
   ttaatttatt ttttaaaatt tgtttttttt attgattttt tttttatttt tatttatgtt 1920
   gaattgtgtt tttgtttttg tttttaattt aagaaagttt taggggtttg gatgtgatgt 1980
   tattgatttg tgtattgggg gttagagaga agtgagttgt tgtttgatta gtagtttggg 2040
   attggtggaa ggaagtaggt taggttttgt gaggagttaa ggtgagatgt tgaggaggat 2100
   ttaggaggtt attattttag atagatgatt ttaaataatt ttgtattatt tttatttgtt 2160
   gtgggttatt tgtgggtgga tttttgagtt tggggtgttt attattttat tgtttttgaa 2220
   gttgtagggg attttggagt tagagtttgg ggtgtttagt gtaagattag tgaggttagg 2280
   ttttgttggg tattaatttt aggattttaa gagagggtta agtgtatttt atttttattt 2340
   ttatttttta ttatgttttt ttttttatta ttatttgtat ttttaaatta tttatgtttt 2400
   tattttttat tagtattttt ttttttttta atttttattt ttttttatat tgttattttt 2460
   tattttaata atttgggttt ttgtattaat tttatttttt t 2501

<210> 185
<211> 2501
<212> DNA
<213> Artificial Sequence

<220>
<223> chemically treated genomic DNA (Homo sapiens)

<400> 185
   ggaggggtga ggttggtata agggtttggg ttgttggggt gggagatggt ggtatgagag 60
   aggtgggggg ttgaagagga gaggagtgtt ggtgggggat gggggtgtgg atggtttggg 120
   agtgtaaatg gtaatgggaa aggggatgtg gtgggggatg ggaatagggg tggggtatgt 180
   ttagtttttt tttagggttt tgagattgat gtttggtaga gtttggtttt attagtttta 240
   tattaaatat tttaagtttt gattttagag ttttttgtga ttttagaggt agtggggtgg 300
   tgggtgtttt aagtttagaa gtttgtttat aggtggttta tggtaggtag gagtggtgtg 360
   ggattatttg gggttgttta tttggggtgg tggttttttg agtttttttt agtgttttat 420
   tttgattttt tatagggttt ggtttgtttt tttttattaa ttttaagttg ttggttagat 480
   agtagtttgt ttttttttaa tttttaatgt gtaagttagt ggtattatat ttgggttttt 540
   gaggtttttt tgggttgaaa gtggaagtgg aaatatgatt tagtgtggat gggggtgggg 600
   gggaggttgg tggagggggt gggtttttgg gagtgggttg aaagtggaag tggaatttga 660
   tttagtgtgg atgggggtgg gaggggtgag gttggtggag gggttttggt ttttggggtg 720
   gtagatggtg gtatgataga ggtggggggt tgaggaggag tgttggtggg ggatgggggt 780
   gtggatgggt ttaggagtgt aaatggtaat gggaaagggg atgtgatggg ggatgggaat 840
   aggggtgggg tatgtttagt ttttttttat ggttttgaga ttgatgtttg gtagagtttg 900
   gttttattag ttttatatta aatattttaa gttttgattt tagagttttt tgtgatttta 960
   gaggtagtgg ggtggtgggt gttttagatt tagaagtttg tttataggtg gtttatggtt 1020
   ggtagtagga gtggtgtggg attatttggg gttgtttatt tggggtgggg gttttttgag 1080
   ttttttttta gtattttatt ttgatttttt atagggtttg gtttgttttt ttttgttaat 1140
   tttaagtggt tggttagagt agtttgtttt tttttaattt ttaatgtgta agttagtggt 1200
   attatatttg ggtatttggg atttttttgg gttgaaagtg gaagtggaaa tttgatttag 1260
   tgtggaaggg ggtgggaggg gtgaggttgg tggagggggt gggtttttgg gggtgggttg 1320
   aaagtggaag tgtaatttaa tttagtattt atgggggtgg gaggggtgag gttggtggag 1380
   gggttttagt ttttggggtg ggagatggtg gtgtgagaga ggtgtggggt tgaggatagg 1440
   agtgttggtg ggggataggg gtgtggatgg gtttgggagt gtaaatagta atgggaaagg 1500
   ggaggtggtg ggggatagaa ataggggtgg ggtatgtttg gttttttttt agggttttga 1560
   ggttgatgtt ttgtagagtt tgtttttatt agttttatat taattatttt aagtttggat 1620
   tttagagttt tatgtgattt tagatgtagt ggggtggagg gtgttttaga tttagaagtt 1680
   tgtttatggg tggtttatgg ttggtagtag gagtggtgtg ggattatttg gggttgttta 1740
   tttggggtgg gggttttttg agtttttttt tagtgtttta ttttgatttt ttatagggtt 1800
   tggtttgttt ttttttgtta attttaagtt gttggttaga tagagtagtt tgtttttttt 1860
   taatttttaa tgtgtaagtt agtggtatta tatttgggtt tttggggttt ttttgggttg 1920
   aaagaggaag tggaaattag atttagtgtg gatgggggtg ggaggggtga ggttgatgga 1980
   gggggtgggt ttttgggggt gggttgaaag tggaagtgga atgtgattta gtgtggatgg 2040
   gggtgggagg ggtgaggttg gtagaagggt ttgggttgtt ggggtgggag atggtggtat 2100
   gagagaggtg gggggttgaa gaggagagga gtgttggtgg gggatggggg tgtggatggt 2160
   ttgggagtgt aaatggtaat tggaaagggg atgtggtggg ggatgggaat aggggtgggg 2220
   tatgtttagt ttttttttag ggtgttgagg tttatgtttg gtagagtttg tttttattag 2280
   ttttatatta attaatttaa gttttgattt tagagttttt tgtaatttta gaggtagtgg 2340
   ggtggtgggt gttttagatt tagaagtttg tttatgggtg gtttatggtt ggtagtagga 2400
   gtggtgtggg attatttggg gttatttatt tggggtgggg gttttttgag ttttttttta 2460
   gtgttttatt ttgatttttt atagggtttg gtttgttttt t 2501

<210> 186
<211> 2501
<212> DNA
<213> Artificial Sequence

<220>
<223> chemically treated genomic DNA (Homo sapiens)

<400> 186

ttttatgtta atgttaaaga ttagtgttaa ggatttggag gttttgttga agattatagg 60
aaagtagtgt ttgtaatttt tgatgatgtt ttttagtatt tttgtttttg tgattggttt 120
tttgatttaa tatttgagga gtagaaaatg aattaagtta gttatttttt tattgagtgt 180
tgtttggaat atagattttg ggttagggga ggtgtttggt tttttttttt tttggttgtt 240
ggagttttta atggattgtt tttatagagt gaaattgatg gtagtgggga tggtggaggt 300
tttttgagga tttttgagag gatttggtga tttagtagta ggtatttttt ttagggtgtt 360
tgggattaga ggagaggagg aggatatagt tttttgtttt ttagtagtgg tagtttgtat 420

atttattagg tttagggttt ttttttgggt gttaaggttt tttttaggtt tgtagtgttg 480
atttttttgt ttaagagata tgatgatttt ggttattgta ataggtagga gtgtgggtag 540
gagttgggta atggagattt attggtttgg agagagaggg agtatgtgag aagttttagt 600
taaaaattga attttggagg ttttaataaa ggtttattta tagatttttt tttttggtgt 660
tttttttaggg tttttttgggg atttttttgga gtttttttggt gattttggtt agtttgtttt 720
ttgagaattt gaaggaaaaa gtgagagggt attttagggt atataagtaa gtagaggttg 780
attttgtagg attgatagta gagagggtga ggttaggtgt tttggagttt tatatgtttt 840
ggggtgatgg gggttattag tgtgtattta aggtaaatgt ttattgttgg tattagatgt 900
ttgtattttt tttgttttgt gatttgaggg tatggtttta gattaaggtt ttagtgtttg 960
ttttttggag tttttggaag aggaatgtat gggtttttag ggtgtagatt gtaagtatag 1020
ttttaggttt ttagtgttaa taggagggta ggttggaatt tgtgaggttt ttattttttt 1080
ggggtgaatg gttttttttg tgtttatata gggtttttat tttttttttg gtaaggtttg 1140
gattttattt ttttgttggt ttgagaaatt tagattaaga gtttatattt ttgataggga 1200
ataggatatg aggagattta tatttggtta tatgtgttta ggtttttttg ggaaggatag 1260
gaagagatgt tgaaatgtat tggaatttat gtgttttggg tgaggaattt gttttgtttt 1320
tattttgttt tttggtaggg tttaagatgt tttttttatt gatttgagtg tagatttttt 1380
gataaagatt tttttatttt tgatattaat gattttaggg taagttgggg ggattgtagt 1440
agatagtttt gtttatgttt tttggagtga tagtaggggt agggtagatt tttgtgaggt 1500
ttttatttgt gttttggttt aagggtgttt ttaattgttt tttatgtttt ttattggaat 1560
tattgataga ttttgggatt attttgttta tttattagat gggggttttt gtgttgattt 1620
gagttatttt tggagaataa gttttttatt tttttgagat ttgaaaatag gaatgagatg 1680
gagttatatt ttgttatttt tatatggggt gtttaggtat gatttttttg gttttggggt 1740
gagtgttttt atggtttttt gtgggttatt ttttattgtg atggggtttg ttatttttta 1800
atttgaaatt ttttgagatg agtagatagt ttttttttat tgtaagaatt tattattttg 1860
agggtttttt aatttttttt ttgtggtata aatgaggttg gtatataggg ttatttttga 1920
tttggttttt tttgagtaaa aattagggga tagttagatt ttgtggggtt tttttttttt 1980
gagttaaggt atttttaatt tttatgaatg gggtatatat tggggtttgt agatgttggg 2040
attttttttt ttgttgattt gagttattat tgttttgatt atagtttttt tttttttgtt 2100
atttagaagg tgtagtaagt atgggttata ggtggttatt gtgtattgga ttttttaggg 2160
taaaggtttt tattgttttg gatttaaagg tattttgagt tttttttttt tttttttttt 2220
ttgattttgg tagggttggg ttttatttta tgttgatgtg agtttttatt tttttgattt 2280
ttgaggatga aagaggaggt gttttagttt tagatagggt tggagtggat tttttatttt 2340
gaggatttgg gttttttgag gttttttttt atttttagta gggtttgagg agtagagatt 2400
tttttgaggt tttgtttatg atattaagtt tttttttttt tttagttttg gatgtggatg 2460
ttagaatggg tgtgtttgtt tgttattttt ggggtttttg t 2501

<210> 187
<211> 2501
<212> DNA
<213> Artificial Sequence

<220>
<223> chemically treated genomic DNA (Homo sapiens)

<400> 187

atagaagttt taggaatgat agataggtat atttattttg atgtttatat ttagggttga 60
aggagggaaa gggtttagta ttatgagtag ggttttaggg gagtttttgt tttttaagtt 120
ttgttgggag taaagggagg ttttagggaa tttaggtttt taggataggg ggtttatttt 180
aattttgttt gagattgagg tgtttttttt tttatttttg ggaattagag ggatggagat 240
ttatgttagt atagggtggg gtttaatttt gttaggatta aggagaggaa gaagagggag 300
gatttagggt atttttgagt ttagaataat ggggattttt gttttgggag gtttagtgta 360
tagtggttat ttgtagttta tgtttgttgt attttttggg tgataaagag gagagggttg 420
tggttagagt agtggtgatt taggttagta gagggaggag ttttagtatt tgtaggtttt 480
aatgtgtgtt ttatttatga agattgggga tattttggtt tagaaagaag ggattttata 540
gagtttggtt gttttttgat ttttgtttag aggggattaa attaaggata gttttatgtg 600
ttaattttat ttgtgttata ggaaagaagt tgaagagttt ttagggtgat gggtttttgt 660
agtaaagggg agttatttgt ttattttagg gggttttagg ttgaggaatg gtaggtttta 720
ttatgatgaa gagtaattta taggaggtta tagaaatatt tattttagaa ttaaaggggt 780
tatatttgga tattttatgt gggggtgata ggatgtagtt ttattttatt tttgttttta 840
gattttgggg aggtgaggaa tttgtttttt gaggatgatt taggttaata taggggtttt 900
tatttggtgg atagatagag tggttttagg atttgttagt agttttggtg aggaatatga 960
gggatgattg agggtatttt tgggttagaa tatagatgag gattttatgg aaatttgttt 1020
tgtttttgtt gttattttag agagtatggg tagggttgtt tgttgtagtt tttttgattt 1080

attttgggat tattggtgtt agggatgggg aggtttttgt tgaggggttt gtatttaggt 1140
tagtagaggg agtgttttag gttttgttag gagataaggt aagaatgaag taggtttttt 1200
atttaggata tatgaatttt aatgtatttt agtatttttt tttgtttttt ttaagaggat 1260
ttgggtatgt gtggttagat gtgagttttt ttatgttttg ttttttatta gggatgtgag 1320
tttttaattt gagtttttta ggttagtaaa agggtgggat ttaggttttg ttaggagaaa 1380
ggtgagggtt ttgtgtgagt atagagggga ttatttattt taagagggtg gagattttat 1440
agattttagt ttattttttt gttagtattg ggggtttgag gttgtgtttg tagtttgtat 1500
tttgagggtt tatgtatttt ttttttagga gttttaggaa atagatattg aggttttggt 1560
ttgaggttgt gtttttaggt tatagagtag aggagatgta gatgtttagt gttagtagtg 1620
aatgtttgtt ttgaatgtat attaatggtt tttattgttt tagaatatat gggattttag 1680
agtatttggt tttatttttt ttattgttag ttttgtagaa ttagtttttg tttgtttgtg 1740
tattttgagg tgttttttta tttttttttt taggttttta ggggataggt tgattaggat 1800
tattaggaag ttttagagga tttttaggag gttttagagg agtattaaag gagaagattt 1860
gtaagtaagt ttttgttaga gtttttaagg tttagttttt agttgaggtt ttttatatgt 1920
tttttttttt tttaggttag tgggttttta ttgtttagtt tttgtttata tttttgtttg 1980
ttgtggtgat tagagttgtt atgttttttg agtagaagag ttagtattgt aagtttgagg 2040
aaggttttga tatttaagaa gaggttttgg gtttggtggg tgtgtaggtt gttattattg 2100
aggagtagga ggttgtgttt tttttttttt ttttggtttt aggtattttg ggggaggtgt 2160
ttgttgttgg gttattaggt ttttttaaga gtttttaggg agtttttgtt atttttattg 2220
ttattgattt tattttatgg aggtaattta ttaagggttt tagtaattaa gaagaggagg 2280
ggttaagtat tttttttgat ttagagtttg tgttttgagt agtatttagt aagaaggtgg 2340
ttgatttgat ttattttttg ttttttaagt attaagttaa ggagttggtt ataaaggtag 2400
aaatgttgga gagtgttatt aaaaattata agtgttgttt ttttgtgatt tttggtaaag 2460
tttttgagtt tttggtgttg gtttttggta ttgatgtgaa g 2501

<210> 188
<211> 2501
<212> DNA
<213> Artificial Sequence

<220>
<223> chemically treated genomic DNA (Homo sapiens)

<400> 188

aaaaagtata tattaagtaa gtttttttat atagtagtaa atattttatg taaattatta 60
gtatattaaa taaataggtt ttgtttaagg aaataatata tggtttaata ttaggggaaa 120
aaaaaaaaat ggttttattg taatttttta tatataaaat ggaggagaaa aaaatttgat 180
tgtgttaatg ggtatagaaa aagtttttta aagaaattta atatgagttt atggtaaaaa 240
aaaaaaaaaa gtatttaatt tatatttaaa gagaaattgt attttttaaa attaatagta 300
aatatattta aatgtaaaat gtttgaataa ttattattta tgttaggaat gagaagtaga 360
tgtaggttat tatttttatg attgaaatta ggattgagat ataaagagtt ataagtatta 420
aaatgagtaa gtggtatgaa tattggaaga atatgtaatg tttagtgata atattattat 480
tgattttgaa aatttaaagg aaaaggtatg tggaatattt aagtgtattg gtttttagaa 540
tgaggtgtgt aatagattat tatagtaaaa ttaaaatttt tattttatga aagtaataat 600
taattataat aattaataga taatatgtta tttgtaatag taataaaatt tatgaattgt 660
ttgaaaagaa gtttaataaa aatgtataag gtttttatag agataattgt tgaagtatat 720
tgatatattt aggtaaaaaa aattttgaaa ttatatagag gagtataata tatttattta 780
tggggagtat aatattgtaa aaatgtaaat ttagtttaaa ttatttataa tttagtgttt 840
tatttgtagt tgttgttatt ataataaatt tgggttgttg tgttttgttt tttaaattaa 900
atttggaaaa ttatagaatg gaaatagtaa ataaaaaatt agtattagta ataaaattta 960
tgtaataaaa tagggaaagt ttagggtgag atttaaggtt gtgttgagtt ggtatttgtg 1020
tttgatattt gtagtgttgg ttggaggggg ttagtagtag ggatgttggg gaggtttgta 1080
gttggtttat atggtagatg atagaatggg tagaataaaa gtttgaaatt ttttatttta 1140
tttttttgta taatttgagg tagtttttga aaatatgatg ttaagagttt taggtaatag 1200
taggatatta ggaaaatttg gttggttaag gtagtatgat tttagagttt tgttaataat 1260
aatttgaaat tattatagtg gtagaggaat tatatttttt aaaaaaaaaa atttttttaa 1320
tagaatataa agattgaaat aggaggttat tataattgtg ggagttgttg ttttgttttg 1380
tagggagtat ttggtttggg atttgtaggt attttttata aggggtgtag ttgtgtaaat 1440
gtttataggt gatagaaata gagtattttt tgtttattat tttatttaat agttagaggt 1500
gatgaagatg atttttttga gtgaggattg agggtttata ttgttttttt attttatata 1560
ttatagaggg attatagaat ttagtttagt tttttttgtt agttttggta aatgtaggta 1620
gtgatgttat ttagattata tttttttttt taatgttatt ttagggggat ttagagttag 1680
agatttggtt tgaggggagt agaagtaatt tgtagaggat ggtggtttag gtttagttag 1740

gtattaattt taggattttg agggatgatt gaaggttttg tttatttatt tttaattttt 1800
ttgattttat taggatttat agttttagga tttttgtttt aatttttatt ttttgtttta 1860
ttattatttt tatgtttatt tttattttta tttgattttt atttaggtag aatttagttt 1920
tatttttgtt tggaatttag ggtagtattg ttgttaggat gtgatgttat tgatttgtgt 1980
attggaggtt agaagattgt gagatttttg ttttgagtaa tgagtgatgg tttgatgttg 2040
gtggagggaa gttggtttag gtttggtgag gaggtaaggt aagatgttga gggaggattg 2100
aggtgggttt tattttagat agagggtttt aaataattta gtgttgtttt tgttgttggg 2160
tttgggttat tttgtagggg aagattttta ggttgggttg ttattatttt attttgttga 2220
tttttgttgt tttagttatg gggaattttg gggatagagt ttaatgtggt tagggtaggg 2280
ttggttagaa gaggttaggg tttatgttgt ggtaggaatt aaggttagga ttttgagagg 2340
gaattgaggg tagtttaatt attattttta ttattatttt tgttttttaa tattaatttt 2400
atttttattt tttatttttt atttttattt ttatttttat ttttattttg gtagaatttg 2460
ggttttgttt ttggtattaa gttatggaag ttttgggaat g 2501

<210> 189
<211> 2501
<212> DNA
<213> Artificial Sequence

<220>
<223> chemically treated genomic DNA (Homo sapiens)

<400> 189

tatttttgga gtttttgtga tttgatatta ggggtaaagt ttggattttg ttaggatagg 60
ggtgggggtg gggatgggaa tggggaatgg gggatggggg tggggttggt gttgggggat 120
gggaatggtg gtgagggtgg tggttaggtt gtttttagtt tttttttggg gttttgattt 180
tgatttttgt tatagtgtgg gttttgattt tttttaatta gttttgtttt ggttatatta 240
agttttgttt ttagagtttt ttgtggttaa agtggtgggg gttggtgggg tgaggtagtg 300
gtgatttagt ttggaagttt ttttttgtgg ggtggtttag gtttggtagt agaggtagta 360
ttggattatt tgaggttttt tgtttgaggt gaggtttgtt ttagtttttt tttagtgttt 420
tattttgttt ttttattgag tttgggttgg tttttttttg ttgatgttag gttgttgttt 480
gttgtttagg gtgagaattt tgtggttttt tgatttttaa tgtgtaagtt agtggtgtta 540
tattttggta atggtattat tttgggtttt gggtaggggt ggaattggat tttgtttgga 600
tggggattgg atgggggtgg aggtaagtat gaagatggtg atggggtaag gggtaaggat 660
tgggatgggg gttttgaggt tgtagatttt ggtggggttg ggggagttgg gggtgggtgg 720
gtggggtttt tggttatttt ttagggtttt gaagttgatg tttggttgag tttggattgt 780
tattttttgt agattgtttt tgtttttttt agattaagtt tttgattttg agtttttttg 840
aagtggtatt gggggaaggg gtgtggtttg ggtgatatta ttgtttgtgt ttattagggt 900
tgataagagg ggttgagttg gattttgtgg tttttttatg gtgtgtgggg tgggggggtg 960
gtgtggattt ttagttttta tttaggaggg ttgtttttgt tatttttggt tgttggatga 1020
agtgatgggt aggagatgtt ttgtttttgt tatttgtgag tatttgtata gttgtatttt 1080
ttgtagagaa tgtttgtggg ttttaggtta ggtgtttttt gtagggtggg gtggtagttt 1140
ttgtggttgt agtgattttt tattttagtt tttgtgtttt gttgaaagaa tttttttttt 1200
taaaaaatgt aatttttttg ttattatggt ggttttaggt tgttattagt agaattttgg 1260
agttatattg ttttaattaa ttaaattttt ttggtgtttt gttattattt agggtttttg 1320
gtattgtgtt tttagaggtt gttttagatt gtgtaaagaa gtgaagtgga aaattttaaa 1380
tttttatttt atttattttg ttatttattg tgtaggttgg ttataaattt ttttaatatt 1440
tttgttgtta atttttttta attaatattg taagtgttaa atataagtat tagtttaata 1500
tagttttgag ttttattttg ggtttttttt gttttgttgt gtagattttg ttgttgatat 1560
tggtttttta tttattgttt ttattttgta attttttagg tttgatttgg gaagtagagt 1620
atagtagttt aaatttgttg taatggtggt ggttataggt aaggtattaa attgtaaata 1680
atttaagttg agtttatatt tttatagtgt tgtgtttttt atgaatgaat atattatatt 1740
tttttatgta attttaaggt tttttttatt tgaatatgtt agtatatttt gatagttgtt 1800
tttataaaga ttttatatat ttttattaga ttttttttta ggtaatttat gaattttgtt 1860
gttattgtaa atggtatatt gtttattaat tgttgtagtt agttattgtt tttgtgaggt 1920
gaagattttg attttgttgt agtaatttgt tgtatatttt gttttgaagg ttgatgtatt 1980
tgaatatttt atatattttt ttttttgaat ttttaagatt gataatggta ttattattaa 2040
atattatata tttttttagt atttatatta tttatttatt ttggtattta tagttttttg 2100
tgttttaatt ttgattttaa ttgtagagat aatagtttat atttattttt tgtttttgat 2160
gtgaatgata attatttgaa tgttttgtat ttaagtatgt ttgttgttga ttttaggaag 2220
tgtaattttt ttttgaatat aggttaaata tttttttttt ttttttatta tgaatttata 2280
ttaaattttt ttaaaaagtt ttttttgtat ttgttgatgt aattaaattt tttttttttt 2340
tattttgtat gtaaggaatt ataatgaaat tgtttttttt ttttttttta atgttaaatt 2400

gtatgttatt tttttgggta gagtttgttt atttaatatg ttagtaattt atgtaggata 2460
tttgttgtta tgtaagggag tttatttaat gtgtgttttt t 2501

<210> 190
<211> 2501
<212> DNA
<213> Artificial Sequence

<220>
<223> chemically treated genomic DNA (Homo sapiens)

<400> 190

tttaaattaa tatttttagt aaatgaagat taaaaggtat tttaattttt taaaattgat 60
aggaaatata tttgaatgtg aaatgttaga agtattttta tttttgttag gagtaagtta 120
ttgatgtatg tttttattgt tattgtttat agttgtagtg aatgttgaaa tgaaaaagag 180
agttatggat attaagatga aaaagaggta tgaagattga aaagaaatag attatatttt 240
ttataatatg attattaaga aaatttaaag gaagagatat atggaatatt taaatgaata 300
agagatttta gaagatagta tataaagatt attgtagtga atataagaga tttgttatag 360
atgagtaatt tagaatataa aatgaaaaat atattatttt taatagtatt taaatttaaa 420
atataaagaa gaagaagttt aattaaaata tgtaagatat ttatagaagt aattgtataa 480
gtttgttgat agatttagag aaaaaatttt taaataaatg gagtagtata tgatgtttat 540
tgatgaggaa tagaatatag taaaggtgta aattttattt aaattaaata aatagttaat 600
gtttatttgt agttgttaaa gtgataatta agtttaggtt gttgtgtttt atttttaaaa 660
ttaattgtgt aaaaatatag aagggaaaaa gaaaaataaa ataataatat tagtaataaa 720
gtttaaataa taaaatatag aaaatttggg ggaaaaaatt gtgttgaatt ggtatttgtg 780
ttttgggtag ttggagaggt agggaggttg aggaggtttg tagttattgt agatgatagg 840
gtgagttgta ggaaaagttt taaaggatta ttttatatta atgtaggatt tgagtttgta 900
ttttaaaata tgaagttagt agtttagggg taatattagg atattaggaa aattggattg 960
attgagatat tatggtttta gaattttgtt aataataatt agaaataaag agtaggttag 1020
gattattttt taaaatatta ttattttaat taaatatata taatgaatta gaaatagaat 1080
agtatgagtg aattatatat tgtatgagta gatattgttt gataaaataa gtatgtatag 1140
ttgatatata tatatgtata aatatatgta aagatatata tatatatatt tatataatta 1200
tatatatata atataaatat atatatatat atgtgtttat gtatataaat ataaatataa 1260
tgtatatata taattggtat agatttaaaa tttgtttttt attatatgaa tgtgaattaa 1320
attatgaaaa ttattaaagt ggaataggaa ttgttaggta attttaaaat ttaggatttt 1380
tataaattta ttgtagttat ttagtttttt tagtgtaaaa ataaattatt tagtataaaa 1440
gttttgtaaa attttttttt agttggggaa gagtttttgt gggaaggtgt gttttttttt 1500
ttagaggtta ttataatttt aggtgttgta aatttatagg gagaatttgg tttgggattt 1560
gtagttattt tttgtaaggg gtgtagttgt gtaaatgttt agaggtgata gaaatagagt 1620
attttttaaa agagttaaga gttaagaaga ggattttttt gagtgaggat tgagggttta 1680
tttatattat atagaggggt tatataattt agtttagatt tttatgttag ttttgaaaga 1740
ttttagtagt tttgtttttt tattatattt ttttttttat tgttatttta ggggatttgg 1800
aattagaatt ttggtttgag ggaagtagat attattagta gagtatggag ttttaggttt 1860
tgttagattt taaagtgaga attttgaggg atgattgaat tgtttttttt ttattttttg 1920
ttttattaga atttggtttt gtttttgttg tttatttagg tgagttttgg gtggatggat 1980
gtgatgttat tgatgtggtt gtggggatta gagagaagtg agggtttggt tttgaggggt 2040
tggtttgaga ttggttgagg gaagtgggtt tagggtttgt gaggaggtaa ggtgagattt 2100
tgagggagga ttgaggaggt ttttattttt tatataggat ttaaaataat ttagtgttgt 2160
ttttgttgtt agttttggat tattaggggg tagatttgtt tggttgggtt attttttgtt 2220
agttttgtgt ttaagtttta ggggaatttg gagtgagagt ttggtgtgat tagagtaggg 2280
ttggttagta gagggtaggg tttaggtttt gttaggtatt aagggtagga ttttgaggga 2340
gggttgaggt tttatagagt atatttttgt ttttattgtt agttttggga agttttaggt 2400
atggtggttg ggtatggtgg ttgggtatgg tggttgggta tggtggttgg gtttgttgat 2460
tttgatgttt gtatttgggg ttgatgtagt aaaggggttt t 2501

<210> 191
<211> 2501
<212> DNA
<213> Artificial Sequence

<220>
<223> chemically treated genomic DNA (Homo sapiens)

<400> 191

gaagtttttt tgttgtatta gttttggatg tggatgttag aattagtgag tttggttgtt 60
atgtttggtt gttatgtttg gttgttatgt ttggttgtta tgtttagggt tttttagggt 120
tgatagtagg ggtagagatg tgttttgtgg agttttagtt ttttttaggg gttttgtttt 180
tgatgtttgg tagaatttgg gttttgtttt ttgttaatta gttttgtttt ggttatatta 240
agtttttatt ttagattttt ttggggttta agtgtagggt tggtgagggg tggtttagtt 300
agataagttt gttttttggt ggtttagggt tggtagtagg ggtggtgtta gattattttg 360
ggttttatat gaggggtggg ggttttttta gttttttttt agggttttat tttgtttttt 420
tatagatttt gagtttgttt tttttagttg attttaagtt gattttttag aattaggttt 480
ttgttttttt ttgatttttg tggttatgtt agtggtgtta tatttgttta tttagggttt 540
atttgggtgg ataatagggg tggaattgga ttttagtggg gtgggggatg agggaggagt 600
ggtttagtta ttttttaagg tttttatttt gagatttggt agagtttgga gttttgtatt 660
ttgttgatgg tgtttgtttt ttttagatta aggttttgat tttgagtttt ttgaggtggt 720
agtgggggga agggtgtggt gggggggataa ggttgttggg gtttttttagg gttgatatga 780
gggtttgagt tgaattatgt ggttttttta tgtggtgtgg gtggattttt agtttttatt 840
taggagggtt ttttttttga tttttggttt ttttaggaga tattttgttt ttgttatttt 900
tgagtatttg tatagttgta ttttttgtag agaatggttg tgggttttag gttagatttt 960
ttttgtgggt ttgtagtatt taagattgta gtgatttttg ggagaagagg tatatttttt 1020
tatgggggtt tttttttagt tagaagagga ttttgtaaaa tttttgtatt aaatgattta 1080
tttttatatt gaagaaattg agtagttgta ataaatttgt ggaagttttg agttttgaag 1140
ttatttaata atttttattt tattttagtg atttttataa tttaatttgt atttgtatag 1200
taagaaatga attttaaatt tatgttaatt gtatatgtgt attatattta tgtttatatg 1260
tataaatata tatgtatgta tatgtttata ttatatatgt gtaattatat gaatgtatgt 1320
gtatatattt ttatatatat ttatatatat atgtatgttg attatatgta tttattttat 1380
taaataatat ttatttatat aatgtgtgat ttatttatat tattttattt ttagtttatt 1440
atatatattt gattaaaata atagtatttt ggaaggtagt tttaatttat tttttgtttt 1500
tagttgttat tagtagaatt ttggagttat ggtgttttaa ttaatttaat ttttttggtg 1560
ttttgatatt atttttgggt tattggtttt gtgttttgag gtgtagattt ggattttata 1620
ttgatgtggg gtggtttttt aaaatttttt ttgtaattta ttttgttgtt tgtagtggtt 1680
gtagattttt ttaatttttt tgttttttta attgtttaga atataagtat tagtttaata 1740
tagttttttt tttagggttt tttatgtttt gttgtttaga ttttgttgtt gatattgttg 1800
ttttattttt tttttttttt tttatatttt tatatagttg attttgggag tagagtatag 1860
tagtttgggt ttgattgtta ttttggtagt tataggtagg tattaattgt ttatttaatt 1920
taagtaagat ttatattttt gttgtatttt attttttatt agtgaatatt atatattatt 1980
ttatttattt gggaattttt tttttgaatt tgttagtaaa tttgtatagt tgtttttgta 2040
aatgttttgt atattttggt taggtttttt tttttttata ttttggattt aaatgttgtt 2100
ggggatggta tattttttat tttatgtttt aagttgttta tttgtgataa gttttttgta 2160
tttgttgtag tggtttttgt atgttgtttt ttgaagtttt ttatttattt gaatgtttta 2220
tgtgtttttt tttttgaatt tttttgatgg ttatattgtg aaaaatatga tttatttttt 2280
tttaattttt atattttttt tttattttga tatttatagt tttttttttt attttagtat 2340
ttattgtagt tgtgaatggt agtagtgaaa gtatatatta atgatttgtt tttgatgaga 2400
atgggaatgt ttttaatatt ttatatttaa gtatgttttt tgttagtttt agaaaattaa 2460
agtgtttttt aatttttatt tgttaaaaat gttaatttga a 2501

<210> 192
<211> 2501
<212> DNA
<213> Artificial Sequence

<220>
<223> chemically treated genomic DNA (Homo sapiens)

<400> 192

gtggaattag attttgttgt gggagagttg ggggggtggg gtggagtttg gggtggggtg 60
gggtggggtg gggtggggtg tagagtggag ttgaggatgg ggttggggaa tttggggttg 120
taggttatgg ttgggttggg ggggtttagt tttttttagg gttttggttt tgatgtttgg 180
tagagtttgg gttttgtttt tttttaatta gttttgtttt ggttatatta agtttttatt 240
ttagagtgtt tagtagtttg agtggtggag ggagggggtt ggtttagttt tataagttag 300
ttttagattg gtttttgggt ggtttagggt tgggagtaga gatggtattt ggtttttttg 360
ggttttttat ttggggtggg gatattttta gttttttttt agtattttat tttgattttt 420

tatagagttt tggttagttt atttttattg gtattaagtt gttttttaga aggaggtttt 480
tttttttttt ttgattttta gtgtgtatgt tagtgatgtt atatttggtt tttaagtttg 540
gagttttttt gggttgttag taggggttga attgggtttt ggtggagtag gggtggggat 600
ggggataggg gtttttagtt attttttagg gggttttgat tttgatgttt gggttttgat 660
ttttattttt tgtttattgg gtttgttttt tttggattaa gtttttgatt tttagttata 720
tgaggtggta gtgaggggag gggtgtggtt ggggtgataa tattgttagg gttttttagg 780
gttgatagga agggttgagt tggattttgt ggttttttta tgtggggagg ttggattttt 840
agtttttttt taggagggtt ttttttttgg tttttggttt tttgatgaag tgatgtgtgg 900
gagatgattt gtttatgtta tttttgagta tttgtttagt tgtatgtgtt gtagagaatg 960
gttgtgggtt ttaggttaga tgttttttgg ggggttgtag tatttgtgat tgtaggattt 1020
gtgagagaag atgtatatta ttttatgggg gttttttttt agttagagtt agattttgta 1080
aattttttgt tttaaaagat ttatttttat atggaagagg ttgagtagta gtaagagatt 1140
tggagaagtt atgtgttttg aaggtattga atatagtaga gtttagatta gtgattttta 1200
tattttaatt tttatttata tggtaataaa ttttatattg tatgtgtata ttatatatgg 1260
tattgtatat tttgtttata tttatgttta tatatatatt tgttttagat attatatata 1320
tttatgttta tatgtatata tatatgtata tatattttta tattttatat ttatttatat 1380
gtatatattt ttagttatat atttgtttgt atttttagat tttatattat atatatgtat 1440
atatatttta ttaatattta tttatagtat gtggttttta tatatattta ttattttatt 1500
tttagtttat tttttatatt ttattaaaat aataattttt tggaaggttg gttttttttt 1560
attaagtttg ttttaggtgg ttgttagtag aattttggag ttatagtgtt ttaattaatt 1620
taaatttttt ggtgttttga tattatttta gggttattgg tattagattt tgtggtgttg 1680
ttttagattg tgtgatgatg tggggtggaa tgttaaatat ttttttttat ttattttgta 1740
atttgttatt tatgtagggt atagattttt ttgatttttt tgttgttttt tggatttaaa 1800
atataagtat tagtttagta tagtttttag ttatttttag ggtttttttt gttttgatgg 1860
gttttttttt ttaatttttt tttttttata ttttttttgg ggttgatttg gggagtagag 1920
tatagtaatt tgagtttggt tgttattttg gtaatgatag ttaaggtatt tattttgtat 1980
ttaatttaag tagtatttat atttttgtta tatttgtttt tttattaagg aatatggtat 2040
atagatttat ttatttgaga ttattttttg agtttattag taattttgta tagttgtttt 2100
tataaaggtt ttgttaggtt ttttttattt atattttgga ttttaatgtt gttgtgaatg 2160
gtgtattttt tattttttat tttatatatt aagttgtttt tgtgtggtaa ggtttttgat 2220
tttgttttaa tgattttttg tatgatgttt tttgaagttt tttatttatt tgaatattta 2280
atgtgtttat ttttttgaat tttttatgta gataattata tagttaataa atattttatt 2340
tttttgtagt ttttatattt tatttttatt ttgatattta tagttttggt ttttttttat 2400
tttgatattt attgtagatg taggtggtag tggtgatagt atatgttaat gatttgttat 2460
tgttgtgaat ggtagagttt ttgatatttt atatttatgt a 2501

<210> 193
<211> 2501
<212> DNA
<213> Artificial Sequence

<220>
<223> chemically treated genomic DNA (Homo sapiens)

<400> 193

tatataaatg tgaaatgtta gaagttttgt tatttatggt agtgataagt tgttgatata 60
tgttgttatt attattattt atatttgtag tgaatattaa aatgaaaaag agttagagtt 120
atggatgtta agatgaaaat gagatatgaa gattgtaaag aaatagagta tttgttaatt 180
atatgattat ttatatagaa aatttaaagg aatagatata ttgaatattt aaatgaataa 240
aagattttag aaaatattat atagaaggtt attaaagtaa aattaagagt tttgttatat 300
aaaagtaatt tagtatataa aatagaaaat agaaaatgta ttatttatag tagtattaaa 360
atttaaaatg taaataagaa gagtttgata agatttttat ggaagtaatt gtgtaaagtt 420
gttgatagat ttaggaaata attttaagta aatggatttg tatattatat tttttgatgg 480
ggaatagaat atggtaaaga tgtaaatgtt atttaaatta aatatagagt gagtgtttta 540
attgttattg ttaaagtgat aattaagttt aggttgttgt gttttgtttt ttaaattaat 600
tttggaaaaa atataggagg gaaaagatta aaaaaaaaaa tttattaaaa tagagaaagt 660
tttggggatg attgaagatt gtgttaaatt agtatttgtg ttttgggttt agggagtagt 720
agggaaattg aggaggtttg tattttgtat agatggtaga ttataggatg gataggagaa 780
agtgtttaat attttatttt atattattgt gtaatttgag gtagtattat aaaatttgat 840
gttagtagtt ttggagtaat attaggatat taggaaattt gagttgattg agatattatg 900
gttttagagt tttgttaata attatttgaa ataaatttag taggagaaga ttagtttttt 960
aaaaaattat tattttaatg gaatataaag aatgaattag gaatagaata gtaagtatgt 1020
gtgaaggtta tatattatga gtagatatta ataaaatatg tgtgtatata tatagtataa 1080

agtttagaaa tataaatgaa tatgtagtta aagatatata tatgtaagta aatataagat 1140
ataaagatat atatgtgtgt atgtatatat ataagtatag atgtatataa tgtttaaaat 1200
aggtatgtat ataaatgtag atataaataa aatgtataat attatatata atgtatatat 1260
atagtgtaaa atttattatt atgtgaatga gaattaaaat atgaaaattg ttaatttgga 1320
ttttgttgtg tttgatattt ttaaaatata tggttttttt aaattttttg ttgttgttta 1380
gttttttttg tgtaaaaata aattttttag gataaaaggt ttgtaaagtt tagttttggt 1440
tggggaggag tttttatggg atggtgtgta ttttttttta taggttttat aattataggt 1500
gttgtagttt tttagggagt atttagtttg ggatttgtag ttattttttg taatgtgtgt 1560
agttgggtaa atgtttaaag gtgatataaa tagattattt tttatatatt attttattaa 1620
agagttagga gttaggagga gaattttttt gagaggggat tgaaggttta atttttttat 1680
atagaggggt tatagaattt agtttagttt tttttgttag ttttggaaga ttttggtaat 1740
gttgttattt tgattatatt ttttttttta ttgttatttt atgtgattgg gagttagaga 1800
tttggtttga gaggagtaga tttaatgggt agagaatggg gattgagatt taggtattaa 1860
ggttaggatt ttttgaggga tgattgaggg tttttatttt tatttttatt tttattttgt 1920
tagaatttgg tttagttttt attggtaatt tagggaagtt ttaggtttgg gggttggatg 1980
tgatgttatt gatgtgtgta ttgggggtta gagagaaggg agaggttttt ttttgagggg 2040
tggtttgata ttggtggagg taagttggtt gaggttttgt gaggagttaa ggtgaggtgt 2100
tgagggaaga ttgaggatgt ttttatttta gatagaggat ttgaaagaat taagtgttgt 2160
ttttgttttt agttttggat tatttggggg ttgatttggg gttgatttgt gaggttgggt 2220
tgattttttt tttttgttgt ttaagttgtt gggtattttg gagtgagagt ttggtgtgat 2280
tagggtaggg ttggttagga gagggtaggg tttaggtttt gttgggtatt aaggttagga 2340
ttttgaggga ggttgggttt ttttaattta attatgattt atagttttag gttttttagt 2400
tttattttta gttttatttt atattttatt ttattttatt ttattttatt ttaaatttta 2460
ttttattttt ttaatttttt tgtggtagaa tttggttttg t 2501

<210> 194
<211> 2501
<212> DNA
<213> Artificial Sequence

<220>
<223> chemically treated genomic DNA (Homo sapiens)

<400> 194
   ttttttttat ttgattttag ggaggtgtag gttttttaaa tggagaggtt tttaggttaa 60
   aaaagagggg agttttagga tttgttagga gttaaattaa ggaaattgag tgagaattga 120
   ggggattgtt tatttttgaa tagagagagt aatagagttt atttttttgt ataattttta 180
   aattgttagg gtaatatgta aggttgagga tttattatta tttttttttt ttaataattg 240
   attttttttt ttagaatagt tttatgttta tagtaaaatt gagtagaaag gagagtttta 300
   taggtatttt gtttttttat agatatagtt ttttttatat taatttttta agttagggtg 360
   atatatttgt tataattgag aatatatgga tatattatta ttatttaatg tttgtagttt 420
   aggatttatt tttgttgttg tatattttgt ggttttaata tttaaaaatg tataatgata 480
   tatgtttatt attatagaat tatttagaat tgttttattg ttttaaaagt tttttgtgtt 540
   tgatttattt atttttattt ttttttaatt tttggaaatt attgaatttt gtattgtttt 600
   tatatttttg ttttttttag aatgttatat agatggaatt atgtaatgta tagatttttt 660
   aaattggttt tttttatttt gtaatatgta tttaagtttt atttatgttt ttttgtggtt 720
   tgatagttta ttttttttta gtgttgaata atattttatt ttgttaattt atttattttt 780
   tttttgaagg atatattgtt tgttttttag tttgggtaat tatgaataaa gttattataa 840
   atatttgtgt gtaggttttt tttgtggata tattgttttt aatttatttg gttaaataat 900
   aaggagtgta tttgttagat tatatgatga aagtatgtat agttttgtaa gaaatttata 960
   aattgttttt aaaagtggtt gtattatttt ttatgtttat tagtaatgat agtgtgtttt 1020
   tgttgtttta tatttttatt agtatttggg gttattaggg tttttgattt tgattatttt 1080
   tataggtatg tagtggtatt atattattgt tttaatttgt aagtttttaa tgatatgtga 1140
   tgttgattat tattttatgt atgtatttgt tttttatata tattatttgt tgaggtgttt 1200
   gtttagattt tttgtttatt ttttaatagg ttggttagtt tttattgttt agttttaaga 1260
   tatttttgta tgttttggtt aatagttttt tattagatat gttttttgaa aataattttt 1320
   ttttaatttg ggagttattt tatttttttt gtggtatttt tagtagagta gaagttttaa 1380
   tttagtgaag tttagattat taattatttt tttttataga tgtttattat attttatatt 1440
   agaattagaa aagatgaaat ttttggtttg aagttgtagt tattagttag tagaagagat 1500
   agtttataat tttgtttgga gtttagatga ggattttgag tgtaaatttg ggatttaaag 1560
   agtttaggat agagagagta ttaaatgttt ttaggtaggg gtggtgggtt gaggggtttt 1620
   tagatttttt ttatttgggt tttagaaaat taaagagtta attttataat attaatagag 1680
   ggaggtttag gttttgttaa gagttgatat gataatttta aaggtaatta gagtggattt 1740
   tttttaagtt agaatataga aagttttatt gtgagttttg ttgttattta gttagtttta 1800
   ggtagggttg gtaagttgta gtttaaggta tattgtaatt tttttagttg gttttttagg 1860
   ggatagaatg attaagaata atagtttagt gaatatttag agtagtgttt ttaaggaatt 1920
   ttgtagaggt ggtttttgaa aagttaaggt agtatttgtt tgttgaaggt gtttttagga 1980
   ttttatttgt ttttttttag gaattatatt atttgttttt ttgtttatat ttttgtttgt 2040
   tgtgtttaat tatagttatt atgttttggg gttagaagag taagttttgt gtttgtgaga 2100
   aatgttagtg gatttgtggt tagatttagg attttaaggt tggttagttt attgtagtag 2160
   agaaagaaga gttttttttt ttttttttat ttgttttgag ggatattgtt tttagttttt 2220
   ttgtttttgg tattttttag gagttttaga gagagttatt tattattttt gttgttgtag 2280
   ttatgttatg tattggattt gataaaggtg atgagagtta agatgaggaa aatgtaagtt 2340
   ttttttaggt tttaatattt attgagagat tatttaaaga ttttttaatt aggaagatga 2400
   agatgttagt gtagtttttg ttgtataagt ataaaatgaa agagtttatt ataaaggtag 2460
   aaatgttgaa gattattagt aaaaagtata aggagtattt t 2501

<210> 195
<211> 2501
<212> DNA
<213> Artificial Sequence

<220>
<223> chemically treated genomic DNA (Homo sapiens)

<400> 195
   gaagtgtttt ttgtattttt tgttgatgat ttttagtatt tttgtttttg tagtgggttt 60
   ttttatttta tatttgtata gtaggaattg tattaatatt tttgtttttt tggttagaga 120
   atttttgagt gattttttag tggatgttga ggtttgggag gaatttgtat tttttttatt 180
   ttggtttttg ttgtttttat tagatttagt gtatgatata gttgtagtag tagaggtggt 240
   gggtggtttt ttttgaggtt tttggggaat gttaaaagta agggagttgg aggtagtatt 300
   ttttaaaata gatgaggaag aggaaggaga tttttttttt tttgttgtag taggttgatt 360
   aattttgaga ttttgggttt gattatgggt ttgttggtgt tttttatggg tatggagttt 420
   atttttttga ttttgaggta tgatggttgt ggttaggtat agtaggtagg agtgtaggta 480
   gaagggtagg tgatgtggtt tttggagaag agtaaatgaa attttgagaa tatttttagt 540
   aggtagatat tattttggtt ttttagaagt tgtttttgta ggattttttg agaatattgt 600
   tttaagtatt tattgggtta ttgtttttag ttattttgtt ttttgagaag ttagttgagg 660
   aagttataat gtgttttagg ttgtagtttg ttaattttgt ttggggttga ttgggtgata 720
   ataaggtttg tagtggggtt ttttgtgttt tggtttggag aggatttatt ttgattattt 780
   ttagaattgt tatgttagtt tttggtaggg tttgagtttt tttttattgg tgttgtgaaa 840
   ttgatttttt agtttttggg gatttagatg agggaagttt aggggttttt taatttatta 900
   tttttgttta gaagtattta gtgttttttt tgttttgggt tttttaggtt ttaagtttgt 960
   atttaggatt tttatttgga ttttaagtag ggttgtggat tgtttttttt gttgattgat 1020
   aattgtaatt ttagattaag aattttattt tttttagttt tggtataaaa tgtgatgggt 1080
   atttatgaaa gaaaataatt gataatttgg attttgttaa attaaaattt ttgttttgtt 1140
   aaagatatta taaagagaat aagataattt ttaggttggg aaaaaattat ttttaaaaga 1200
   tatatttgat aagggattgt taattaaaat atataaaaat gttttaaaat tgaataataa 1260
   gaattgatta attttattaa aaatggataa aagatttaaa taggtatttt aataaataat 1320
   atatatggaa ggtaaatatg tatatgaaat gatggttaat attatatatt attagagatt 1380
   tgtaaattaa aataataatg tgatattatt atatatttat aagaatggtt aaaattaaaa 1440
   attttgataa ttttaaatat tgatgaggat gtggagtaat aggagtatat tgttattgtt 1500
   gatggatatg aaaaatgata tagttatttt tgaagatagt ttgtaagttt tttataaagt 1560
   tatgtatatt tttattatat gatttagtaa gtgtgttttt tgttatttag ttagatgagt 1620
   tgaaaatggt atgtttataa aaaaaatttg tatataaatg tttatagtag ttttatttat 1680
   aattgtttaa attgggaagt aagtaatatg ttttttagaa ggagagtgga taaattaata 1740
   gaataaaata ttatttagta ttaaaaggga atgaattatt aagttataaa gagatatgga 1800
   tgaaatttag atatatatta taaagtgaaa gaagttaatt tgaaaagttt atatattgta 1860
   taattttatt tatatgatat tttggaaaag gtaaaagtat ggaaatagta taaagtttag 1920
   tggtttttaa aggttgggga gagtgagaga taaatagatt aggtatagag aatttttaag 1980
   gtagtaaaat aattttggat gattttatgg tggtaaatat atgttattat atatttttaa 2040
   atattaaaat tatagaatgt gtagtaatag gaatgaattt taaattatgg atattgggtg 2100
   ataatgatgt gtttatgtat ttttaattgt aataaatgta ttattttggt ttgggaggtt 2160
   gatatgaagg aggttgtgtt tgtgggagga taggatgttt gtgagatttt tttttttgtt 2220
   tagttttgtt gtgaatgtaa aattatttta aaaaagaagg ttaattatta aaaaaaaaaa 2280
   gtgatggtga gtttttagtt ttatatgttg ttttggtaat ttaagagtta tgtagggggg 2340
   tggattttgt tgtttttttt atttagaggt gagtagtttt tttagttttt atttggtttt 2400
   tttgatttgg tttttagtag attttagggt tttttttttt tttgatttga agattttttt 2460
   atttaaaagg tttgtatttt tttgagatta gataggagga a 2501

<210> 196
<211> 2501
<212> DNA
<213> Artificial Sequence

<220>
<223> chemically treated genomic DNA (Homo sapiens)

<400> 196

ttttatttta tattattttt ggaatatggt tttttttttg taaggtttgt agtagaaaaa 60
ttggttatgt ttgtagggat gtgatatttt ttagaaagtt tttaatgaaa gatgattttt 120
agtttagagg atatgggaaa ttaatatttt agaatatatg aggttatgta tagattgatt 180
tttatttgtt tttagattta gagagtttga gatagaattg ttaggttgag tatttattta 240
tttaattttt agatttttta tggatatggt ttaagaaggt taattttatg ttagtagatg 300
tagtttagtt tttgatagat atgaatgtga ggattttgaa tgaagatgat ggaaggattt 360
agttagaatt gtggggtttt aaaaagttta gtttaggttg ttagtttttt aaagtttaga 420
atagttttgg ttaaatgtag tttattataa tttttagttt agaggttggt agagggtgag 480
tattttgatt tgatgggaat agttttaggt tagtggagga tggagtgttt taagattggt 540
taggtgttat ggtgaggatt ttgaagagaa ataagagaat tgttatagga ttaatgagtt 600
tttgtgttag ttgtaaagta atagatttat tataataaaa tagtaaagtt tgtagtaaag 660
agtttaatga ttgtagggtg ttaagtgagg agatgagaag agatttttaa atttattttt 720
ttaaggagtt ttgggttggg atttgtaagg agattatgga gggtgagggg ttggagaatt 780
gaggttgttg atttgttggg gtaaagggga tgaaattatt agaatgtgga aattgtgttt 840
tttttttttt agttagtttt tgtggggttt tttggattag ttgatgttag tagggttttt 900
tagagtagtt ggtattagtg gggtttttta gattagttga gttattagaa ttattagtat 960
gtaggatttt aaggaatatt ttaaagaaaa aaagttttat tatgttaaaa ttgttattta 1020
tagagtaatt aagggtaatt ataatttagt aataggattt atatgatttt gggataatag 1080
gtattaaatt attatgagga agtaggttag agagtagttg attttatgat taatgttgaa 1140
tgttttgtaa gtttggtgta tttttattta tttttttttt tttttttttg attaatttta 1200
tatagtttat aggggtagtt ttaaaattat ttattttaga atagaggagt ttataaattt 1260
tattaatgtt gttatttttg gggagtttag aagagaatta tgaggtggag aaattttatt 1320
aatatgtttt gtgtgtttta tagaggtgaa gattttggat tagtggaaat ggttttaggt 1380
taaagagata aaatttatgt tttaatagga attaaggtga ggattttaag tgttatgagg 1440
gggttttttt ttttgtaata ttagtagagg gggttaaata gagttttttg ttattgttag 1500
tattgagagg ttttaaaata agtatttttg aatttttttt attttatttg tatagttttg 1560
gggaaggaag ggtttttgtt tgaagagggt gattttagtt gtgtagagag agagttttgg 1620
tttttatggg aattaaggtg aggattttga gggtggatga gaagattttt ttttaaaaaa 1680
ggtatattta tagagttttg ttgttgttgt taggtttgtg aggtttaggt aggggtggtt 1740
tgtttggtat gtttagattt ttatagtggg ggttgaggga ggtgggggta ttgtttggag 1800
gttggtggat ttgggttagt atgtatattt gttttaggtt gttagatatt gaggtgagga 1860
tttttagtgg agatgaaggg attagtaatg ttagaatagt gatgtttggt agtgtttagt 1920
tgttagtttt tagatgttat gggttgttgg atgtgagtta ttttgatttt tgttttgaaa 1980
aaaaagattt gagtggatgt ggtttatttt gatttttgtt ttggaggtga ggatttgagt 2040
gagtgtaggg ggtgtggtgt ttggttagtt aggggtgaat ttttaggatt ggttggtagt 2100
taaggtgagg attttgagtg taaattgaag agattatttt tatttgtaat aaagagggtt 2160
ttattaagtt ttgtttttgt atttggtttt gagaggtttt ggtaaagttg tttggtaatg 2220
ttttatttgg aaagttttag ggtaggggaa gggtgggggg aggggtagtt gtgggggaag 2280
gaggtttgga tgtagggaat aggttttatt ttgtatgtag ggtgggtgta ggttttaatt 2340
gaaattaatt tgaggttttt aaatgtggat tgaagagaat attttttatt tttaataaag 2400
gtggttttat taagttttgt ttttgaagat ggttttggga ggttttagta aagttgtgtt 2460
tggaaagttt tagggagggg agggttttga atttaggaag t 2501

<210> 197
<211> 2501
<212> DNA
<213> Artificial Sequence

<220>
<223> chemically treated genomic DNA (Homo sapiens)

<400> 197

gttttttagg tttaaggttt tttttttttt gagatttttt aggtatagtt ttgttggggt      60
tttttagagt tgtttttagg gatgagattt agtgaggtta tttttgttaa gggtaggaga     120
tgtttttttt agtttatatt tagggttttt aaattgattt tagttagggt ttatatttat     180
tttatgtgta gaatgaggtt tattttttgt gtttaaattt tttttttttg tgattgtttt     240
tttttttatt ttttttttgt tttggaattt tttaggtgga atattgttgg atggttttat     300
tggagttttt taggattaaa tgtagaagtg ggatttagtg gggttttttt tgttataggt     360
gggggtggtt tttttagttt atatttaggg tttttatttt gattgttgat tagttttgag     420
aatttatttt tggttgatta gatgttgtat tttttatatt tgtttgggtt tttgttttta     480
aagtggaagt taggatgagt tatatttgtt tgggtttttt tttttaaagt ggaagttagg     540
atgatttata tttggtagtt tgtggtgttt gagggttgat ggttggatgt tattggatgt     600
tattgtttta gtgttgttgg tttttttgtt tttattaggg gtttttattt tagtatttag     660
tagtttggga tgaatatgtg tgttgattta aatttgttag tttttaaata atatttttat     720
tttttttagt ttttattgtg gaaatttaag tgtgttaaat aggttatttt tgtttgggtt     780
ttataggttt gatagtagtg gtagggtttt gtgaatgtgt tttttttggg gagaggtttt     840
tttatttgtt tttagagttt ttattttgat ttttgtgaaa gttaagattt tttttttgta     900
taattggggt tatttttttt agataaaggt tttttttttt ttgagattat ataggtggaa     960
tgaaggggat ttagagatat ttattttggg gtttttttggt gttaataatg gtgaggggtt    1020
ttgtttggtt ttttttgttg gtgttgtaag ggggagagtt tttttgtaat atttaggatt    1080
tttattttga tttttgttag ggtgtgggtt ttgttttttt gatttagggt tatttttatt    1140
agtttaaggt ttttattttt gtgaaatata tgaggtatat tgatgggatt tttttatttt    1200
ataatttttt tttgggtttt ttaagggtga tagtattggt ggaatttgtg ggtttttttg    1260
ttttggggtg ggtggttttg aaattgtttt tatgaattgt ataaaattaa ttagggaaga    1320
gggaagggag ataaatgaag atatgttaag tttgtagaat atttagtatt aattatgaag    1380
ttagttgttt tttgatttgt ttttttatag tagtttggtg tttattattt tagaattatg    1440
tagattttgt tattagatta tagttgtttt taattgtttt atagataata attttaatat    1500
aatgaaattt tttttttttg agatattttt taaggttttg tatattgatg attttaatga    1560
tttagttggt ttgaaggatt ttattgatgt tagttgtttt aaaggatttt attgatatta    1620
gttggtttaa aagattttat aggagttgat taaaaaaaaa aaaatgtagt ttttatattt    1680
tgatgatttt atttttttta ttttaatgaa ttaataattt tagtttttta gttttttgtt    1740
ttttatgatt tttttgtaaa ttttagttta gaattttttg gggagatgaa tttgagagtt    1800
tttttttatt tttttatttg gtattttgta attattaaat tttttgttgt aaattttgtt    1860
gttttattat agtgggtttg ttattttgta gttgatatag aaatttgttg gttttataat    1920
agttttttta tttttttttta gggtttttat tatgatattt ggttagtttt ggggtatttt    1980
attttttatt gatttgaggt tgtttttatt agattaaggt atttattttt tgttgatttt    2040
tgaattggaa gttgtggtga gttatattta gttagggttg ttttggattt tgaagggttg    2100
atagtttgag ttggattttt tgggatttta tagttttggt tgggtttttt tattattttt    2160
atttagggtt tttatgttta tatttattag aggttgaatt gtatttattg atatgaagtt    2220
gattttttta ggttatgttt atgagaggtt tgaagattaa atgagtggat gtttagtttg    2280
atagttttgt tttgagtttt ttaggtttga gagtagatga gagttagttt atgtataatt    2340
ttatatattt tgagatattg gttttttatg ttttttgaat tggggattat tttttattaa    2400
aggttttttg ggaaatgtta tatttttgta aatatgatta gtttttttat tgtaaatttt    2460
gtagagaagg agttgtgttt tgggagtgat gtgagatagg g                        2501

<210> 198
<211> 2501
<212> DNA
<213> Artificial Sequence

<220>
<223> chemically treated genomic DNA (Homo sapiens)

<400> 198
   attattaatt gagtattagt tttttggaag tttttatgtt agtattggga atgttttttt      60
   taaaataaat taattttttg tttatttaat tttagagttt gggagtagtt gttataaaaa     120
   aaaaaaagat gatgaggtgt tttttaaaat ttaaagaata agtataaagg aggaatgaga     180
   aaaagaggag gtttgagatg agagtaatta agtgtaaatg ttttgaggta aggtagtttg     240
   gagtgttagg aaattttaag ttttttattg ggaggtaatt ttaagggaaa ttatatggtg     300
   ggttggatga gattgtggga ggggttaggt ttttagatgg tgttttttag aggtgtgaga     360
   taaagtttgg aatgggaagt agtttttaat agttattttg tgtttatgga taaattagag     420
   agaatttgta tttggggtag gaatggaatg tgttttgtgt ttttgtttta gtgtagtaga     480
   atatagttat agtgtataaa ttaggtgttt tatatagttg gtgtatttta gtgttgagag 540
   attaagtttg gaatgggaaa ttatttttaa tatttatttt tttgttgtgg gaaaattaga 600
   gagaattttt atttaggata aaagtgaaaa gtgttttatg tttttatttt agtatagttt 660
   aatatagtgt ataatttagg tgttttatgt atattatttt tagtgagttt ttgagaaata 720
   agggagatga tagttttagg ggaggtaaga tgtttagaag ttattgtgtt ggtatttttt 780
   gttttgggtt ggagaattaa gagtttgttt tattaaaagg atttttaata ggggtgtagt 840
   taggtatggt ggtttatgtt tgtaatttta gtattttggg aggtgaggta ggtggatttt 900
   ttgagattag gagtttaaga ttagtttggt taatgtggtg aaatttgttt tttattaaaa 960
   atataaaaat tagttgggtg tggtggtgga tatttgtaat tttagttatt agggaggttg 1020
   aggtatgaga attatttgag ttttgaggta taggttgtaa tgagttgaga tagtgttttt 1080
   gtattttagt ttgggtgata gagtgagatt ttattttaaa aaaaaaaaat aaataaataa 1140
   taaaaattaa aataggagtg gttaatatag aagggtgttt aggagttaga aaaaaatatt 1200
   ggttattgat aaaaattttg agatttgagt tgtatttaat tggagaaggt ttttttataa 1260
   atattttata aattatataa ttttttttgt taagtagtat tttgtttgat tataattttt 1320
   ttgtttggag tttggtaata tgtttaagtt tatagggtga gtaagtgata gggttgggat 1380
   tagatttttg ttgtgaattt ttttagagtt tatatttttt attttgtttt tttttgtttg 1440
   gagtagattt tgtgtttttt aatttatttt tttttttaat atttttaaat atattttagg 1500
   ggttaaaaag aaggattttg tgtttaaaga attggattgg aatatatagt taaagtaata 1560
   aagaatatta aaataaatga aagttataaa taaagaggag aaagagatat tatttagtga 1620
   taatatgatt atttatatat gtatagttag ataattttaa aattagggat tatataatta 1680
   ttttgaatag tttttttttt ttaagaagta aattaattaa aatagaagtt atataagagg 1740
   ttagtgttgg ttattgaaaa gagtaaatta gtattatttt tttttgataa tttattagtt 1800
   attttggatt ttttgttttg tgttataatt tttttatttt atattagttt tggggtatag 1860
   atttgtaagg tttgtagtag gaatgttgtt taagagtttg tagggatgtg gtatttttta 1920
   ggaagttttt atttagagag gtaagagaaa ataaggagga tatttataaa taaatattgg 1980
   aaagataatt atattaaaag ataatggggt aatttattta aatttttttt tagtttagaa 2040
   tgttttaggt agtgtagtta gattgagtat tttttttttt atttttagaa gtttttagtg 2100
   aagtgagagt tttgatttaa ggtgatttta gttaattaga gggaggattt agggtttggt 2160
   agatattagg gaggattttg aatgaagaat aggaggagtt agaatagtga tgttttatag 2220
   aaatttgatt ttgttgtttg tttttatagg atttaaatat ttttggttta atgtggttta 2280
   ttaaaatttt agtttagatt ttggaggatt ttgatttgag taggatttat ggtatggaat 2340
   gtggagtttt aggattggtt agtagtgaag tgaggttttt gagtgagtaa aaaagggaaa 2400
   attaaattta taaataaggg gattttttgg agtttaattt atatttttaa ttttggaaag 2460
   ttttaggtag agttataaaa ttggtttgtt ttttatttta g 2501

<210> 199
<211> 2501
<212> DNA
<213> Artificial Sequence

<220>
<223> chemically treated genomic DNA (Homo sapiens)

<400> 199

ttgaaatgag aggtaagtta gttttatagt tttgtttagg gttttttagg gttaaaaata 60
tggattgggt tttgagaagt ttttttattt gtgggtttag tttttttttt tttatttatt 120
taagaatttt attttgttgt tggttagttt tgggatttta tattttatgt tatgaatttt 180
gtttagatta aggttttttg agatttaggt tgaagttttg atgagttata ttaggttagg 240
gatgtttggg ttttgtgagg gtagatagtg gagttgggtt tttatgggat attattgttt 300
tggttttttt tgttttttat ttaaggtttt ttttgatatt tgttagattt tggatttttt 360
ttttagttaa ttgaggttat tttaggttaa aatttttatt ttattgagaa ttttgaaggg 420
taaaagagag gatgtttagt ttgattgtat tgtttggggt attttaggtt gagaagaggt 480
ttgggtagat tgttttgtta ttttttggtg tggttgtttt tttagtattt atttatgggt 540
gtttttttta tttttttttg tttttttgaa taaaggtttt ttgggaaatg ttatattttt 600
gtaaattttt gggtagtgtt tttattgtaa attttgtaag tttgtgtttt agggttaatg 660
tgaggtaaag aaattataat ataaagtagg gagtttaagg tagttggtgg gttgttagag 720
aaaaatgatg ttgatttatt ttttttaata gttagtatta gttttttatg tgatttttgt 780
tttaattgat ttatttttta aggggaagga gttgtttaag atgattgtgt ggtttttgat 840
tttgaggtta tttgattgtg tatatgtaga tgattatatt gttattgaat aatatttttt 900
tttttttttt atttgtagtt tttatttatt ttgatatttt ttattgtttt ggttatgtat 960
tttaatttaa ttttttgggt atagaatttt tttttttaat ttttgagata tatttggaag 1020
tattgagaag aaaagtgaat taaagaatat aaagtttgtt ttaggtagag aggaatagaa 1080

tgaagagtgt gggttttaga agaatttata ataggagttt agttttagtt ttgttattta 1140
tttattttgt gaatttgagt atattattaa attttaaata aagaaattat aattaaataa 1200
aatgttgttt agtaaggaaa attatataat ttataaagtg tttgtggaag agtttttttt 1260
aattggatat aatttaagtt ttaaaatttt tgttaatgat taatattttt ttttaatttt 1320
taggtatttt tttatgttaa ttatttttgt tttgattttt attatttatt tatttttttt 1380
ttttgagatg gagttttatt ttgttattta ggttggagtg tagaggtatt attttggttt 1440
attgtaattt gtgttttagg gtttaagtga tttttgtgtt ttagtttttt tagtagttgg 1500
gattatagat gtttattatt atgtttaatt aatttttgta tttttggtag agatggggtt 1560
ttattatgtt ggttagatta attttgaatt tttgatttta agagatttat ttgttttgtt 1620
ttttaaagtg ttgggattat aggtatgaat tattatgttt ggttgtattt ttgttgaaag 1680
tttttttaat agagtgggtt tttgattttt taatttagga taaagagtgt tagtatggtg 1740
gtttttgggt attttatttt ttttgaagtt gttatttttt ttatttttta gaaatttatt 1800
agagatgatg tatatagaat atttaggttg tgtattgtat tagattgtgt taggataggg 1860
atatagaata ttttttattt ttgttttagg taggggtttt ttttggtttt tttataatag 1920
gaaagtaaat gttagggata gttttttatt ttgggtttag ttttttaata ttgaaatgta 1980
ttaattgtat aaagtattta gtttgtgtat tgtgattatg ttttgttgta ttgggataag 2040
agtatagggt atattttatt ttttgtttag gtgtagattt tttttggttt atttgtgagt 2100
ataaaataat tgttaagaat tattttttat tttaggtttt gttttatatt tttgagaggt 2160
attatttgag ggtttggttt tttttatagt tttatttagt ttattatgta gtttttttta 2220
aaattatttt ttaatggggg atttgaggtt ttttaatatt ttaaattgtt ttgttttagg 2280
gtatttatat ttgattgttt ttattttaaa tttttttttt tttttatttt ttttttgtat 2340
ttgtttttta agttttagag agtattttat tatttttttt ttttttatga tagttgtttt 2400
tagattttaa aattaaatgg gtaagaaatt gatttgtttt ggggaaaata tttttagtat 2460
taatatgaag atttttaaag agttgatgtt taattaatga t 2501

<210> 200
<211> 2501
<212> DNA
<213> Artificial Sequence

<220>
<223> chemically treated genomic DNA (Homo sapiens)

<400> 200

aaaggagaag ttttaaaaaa tttttaatat tagtgtatga tagtaatttt tagtaaattt 60
atattaggag agaattttta ttttgtaaaa tttataattg atatattaaa tgtgaaatat 120
tataaatatt ttgaatagag tttagaagaa tatatatttg tatagtataa attttattgt 180
tgataattgg atgaaaatat atttatagtt tataattata ataataatat tgagtaaatt 240
agaggtaata ataataaaag gagataattg tgataatata attttttaat ttaaaaataa 300
aataatgtaa ttggtgtaaa aatatttaaa tgtataagga aataaagaaa atgtgtgtat 360
aaaagattat tataggtaag ttaggagtat agttatataa aattaataat taattataat 420
atgtaatgtg aaatatgttt tttataatag taataatatt taaaatatat ttaatagaaa 480
attaaataga aatgtatatg tttattaagg agatagttat aaaaatttat tgaaagattt 540
aggaaaaata aatttttaat aaagtagtgt attatgttta tttattgtgg aggagatatt 600
gtaaagttat tgattttttt taaatttttt atagatttat tgttttagtt atagttgtga 660
agatgataat taaatttggg tggttttttt ttatgattaa atttaattat agaaaagtta 720
aaggaagaat ttttttttaa aaaaaaagta gtattaataa tagtaaaata ataaaaatgt 780
gaattttgag agatggttag tttgtattga ataagaatgg gtgtgtgggt aggtaggggt 840
atgagttgaa gtagagatag gtttgtggtt tataatagat gtagataatg gaattggtta 900
gaggaaaagt tttaaaattt tggtttttgt tttttttatt gtgttattgg ataatgtatt 960
tttttttttg ttttattaaa ggaaatttag ttagaaattt ggattttatg tgttagtttt 1020
atggggtaat attgttatag taaaaatatt tgggtaggtt gaagaagtat gaatttagat 1080
attgtgttaa taattatttg aaattttttt agtggaagtg gattggtttt taaatttgta 1140
ttattttaat ataatataaa gaatggatta aattaggata gtaaatattg tagtgagtta 1200
tataagataa tggtagaaat ttatttatga aggtgttgaa tatagatatt gatgttagtt 1260
aagagagaag ttttagatat agttattagg ttaagaagtt tttttatttt ttttttatga 1320
attttatagg atttagttag aggaagggga tgaatttata ttagaaaatg gagaaaattg 1380
agattttgtt agtagttaat gtgaggattt tgagtgagga ttgaggggtt ttatatatta 1440
gaagagataa gagtgttttt taaatttttt tttatttttt gattttgtat tataatttaa 1500
tagtagtagt taggtttagg aggttttagg tagtagtggt taggtgaggt gtaattgaat 1560
tgtatatgag gagttttagg aagttgaagt ttagtttgag agggtgtttt taagttagta 1620
gaggggatat ggtaagaatg aagtgatgat attgagtgag ggttgagggt aaagtttatt 1680
ttaaaaaggg ggttttatag agttttattt ttattgttag ttttggaagg ttttggtatg 1740

gtttttgagt ttaggttgta ttttattttt aattttggat ttttagggaa tgagagtttt 1800
tatttttggt tgattgattt agtttaatag atagagaagt ttaagggttt gtgaagagtt 1860
tttgaaagaa ttttatgtga gtttttggga atttgttgta tttatagaat ataaggggga 1920
gtaatagagt tttgtagtta ttgttatgta gggagtgtta ggtgggtgtg gttggaagtg 1980
gttttttttg atttttattt aataaagggg tttgagttgg ttgtttgagt ttgaaaagtg 2040
ttgttatgtt agtggaagga ggtgttttag atttttttag ttgttttggt gggtattttg 2100
atttagaatg gggggtattt tttgataatt ttaatttttt ttttttgaag aaaggtggtt 2160
ttatagagtt ttgttttaat ttgttttgat ttggtttttg aggagttttt aaatatggtt 2220
gttaggttta aagtttattt attttttttt gttgtgttga agaatatgaa ggttttggtt 2280
taaggtttgt ggatttaggt tagtagaggg agaaatttta ggttttatat agttaagagg 2340
atttttttta gaattgaggg aggggatttt atgttttgta atgggttgta ttgaattttg 2400
tttttgatgt tagtttgggg gtttgggtag gtttttagag attgaggtgt atttttattt 2460
tgtttttgtg agagttagag aggtaggttt ttggttgtaa g 2501

<210> 201
<211> 2501
<212> DNA
<213> Artificial Sequence

<220>
<223> chemically treated genomic DNA (Homo sapiens)

<400> 201

tttataatta ggggtttatt tttttgattt ttatagggat aaagtgagaa tgtattttag 60

tttttagaga tttgtttgga tttttaggtt aatgttaagg gtagagtttg gtgtaatttg 120
ttatgggata tggaattttt ttttttaatt ttaaggaggg tttttttaat tatgtagggt 180
ttgagatttt tttttttatt gatttgggtt tataagtttt ggattgaggt ttttatgttt 240
tttaatatga tggaaaaaaa tgaatggatt ttgggtttaa tagttatgtt tggggatttt 300
ttagggatta ggttggggta ggttggggta gggttttgtg gagttgtttt tttttggggg 360
aagggggttg aagttgttgg ggaatgtttt ttgttttagg ttaggatatt tattaagata 420
gttgagaaga tttgggatgt ttttttttgt tgatgtgata gtatttttta ggtttaggtg 480
attggtttag atttttttat taggtggaag ttaagaggaa ttatttttgg ttatgtttat 540
ttggtgtttt ttgtgtaata gtagttgtag ggtttgttta tttttttttg tgttttgtgg 600
gtgtagtaga ttttagggaa tttatgtaaa gtttttttag ggtttttttg tggatttttg 660
gattttttta tttgttgagt tgaattagtt aattggagat agaggttttt attttttgag 720
agtttagagt tggaagtaag gtgtagtttg agtttgggag ttatgttggg attttttaag 780
attgatagta ggggtggagt tttgtgagat tttttttttg gaatggattt tgtttttagt 840
ttttatttag tgttgttgtt ttatttttgt tatgtttttt ttattgattt gaggatattt 900
ttttagatta gattttaatt ttttgagatt ttttatgtgt aatttagtta tattttattt 960
ggttattatt gtttggggtt ttttaaattt ggttgttgtt attaagttgt gatgtaagat 1020
tggggaatga agggagattt gggggtattt tttgtttttt ttgatatgtg gagtttttta 1080
gtttttattt agggttttta tattgattgt tggtagggtt ttagtttttt ttattttttg 1140
atgtaaattt attttttttt tttgattaga ttttgtaaaa tttataagga gaaagtgaga 1200
agatttttta gtttgatagt tatgtttggg attttttttt tggttgatat tagtgtttgt 1260
atttaatatt tttatgaatg aatttttatt attattttgt gtgatttatt gtaatatttg 1320
ttattttaat ttagtttatt ttttatatta tattaaaata atatgaattt aaaggttagt 1380
ttatttttat taaagaagtt ttaggtagtt attagtatag tgtttggatt tatatttttt 1440
tagtttattt agatgttttt gttgtggtaa tattatttta tggaattggt atatagggtt 1500
taagtttttg attgaatttt ttttagtgaa gtagaaaaaa aaatgtattg tttaatggta 1560
tagtgaaaga agtaaagatt aaagttttaa agtttttttt ttagttaatt ttgttatttg 1620
tgtttgttgt aggttataga tttgttttta ttttagttta tgtttttatt tgtttatata 1680
tttatttttg tttaatataa attgattatt ttttaaggtt tgtatttttg ttgttttgtt 1740
gttgttaata ttgttttttt tttaaaaaaa aatttttttt ttaatttttt tatggttgag 1800
tttggttatg ggaaagaatt atttgggttt ggttgttatt tttatagtta tggttaaggt 1860
aatgaatttg taaaaaattt aaggagaatt aataatttta taatattttt tttatagtaa 1920
ataaatatag tatattgttt tattggagat ttattttttt taagtttttt agtaaatttt 1980
tatagttgtt tttttaatga atatgtatat ttttatttgg ttttttgtta gatatatttt 2040
ggatattgtt gttgttgtga aaggtatatt ttatattata tattataatt agttattgat 2100
tttgtgtgat tatatttttg atttatttat ggtgattttt tgtatatatg ttttttttat 2160
ttttttatat atttaaatat ttttgtatta attatgttgt tttattttta aattaggaga 2220
ttatattatt ataattattt ttttttgtta ttgttatttt taatttattt ggtattatta 2280
ttatagttgt gaattataga tgtattttta tttagttgtt agtagtaggg tttatattat 2340

ataggtgtat atttttttgg attttatttg ggatgtttgt aatgttttat atttagtatg 2400
ttaattatag attttataag atggaaattt ttttttaatg taagtttgtt aaaagttatt 2460
attatatatt ggtattgagg gtttttaag gtttttttt t 2501

<210> 202
<211> 2501
<212> DNA
<213> Artificial Sequence

<220>
<223> chemically treated genomic DNA (Homo sapiens)

<400> 202

tttattaggg tttttagatt tataaatttt tgatttttat tttgggagtt agttagaggg 60
gagatttttt ttgaggaggg tagttttagt taaatagagg gagtagtttt agggttggtt 120
atttaataag aagagaattt tgttgaagga tagtgatttt tgaatatggg ggaggtatag 180
gttgtatttt gttatttttt gttgttagtt ttggaaaaag gtggggtgtg agtgttaagg 240
attggggagg tgggagggaa gaggttagta gggtttaggg agtgagtaga aaggtttgga 300
gtatattttg gaggtgggaa ggaagggatg gaaaggaggt tgataagtgg gtggtggggg 360
gggggtgttt tttttttttg agaggaggtt ttgttttatg ttatttgggt tgttagtttt 420
gattaatttt gatagagttt ttggatgtgt tttgttggat gtgttttttt ggtggttatt 480
ttgggagttt gaaggatttg aggtattttg tgatgaggat tgttttaggt tagtggaggg 540
aggagattta tagatttatt tagtttttaa ggtgagggtt ttgagggaga agtgagggat 600
aattttatta tagagagaag tagttttggt ttgttttatt tttgtggtgg tatttgtagg 660
tggtggaaat gaagatggta gttgagggtt gagggatgtg tttttatggt agaagaaggg 720
aggaaatgtt ggttttttag agaataaata ggaaggtatt gaggaggatt gggggaattt 780
ttattttaga ggatagattt ttagagattt ttattttgtt ttttaaatta gtttttatag 840
agttttttag gtggttgagg ttgagtggtt atttttttat tgttgggtga ggggtgtagg 900
ggagggggag gaggaggttt tgttttgggg gttttatggt aagttagtat ggggagttgt 960
ttttagttgg tagagggaag atttttaggt tttgttggga ataagagtga ggattgaggg 1020
gttatatgtg tgttagaata gatgtgaggt tatagggatt gtttttgtgg tagagtgttg 1080
ggaggtggtt gttattttat tattttttat tgtttttagt gatgtggagt ttttttgagg 1140
ttttttttta ggttagtaga gtggtggggg tttggttttg tttgagaaaa tgtgataatg 1200
ttaattgaat tttgaggggg ttatgaattt tagaattgtg ggattttggg agtttggtta 1260
gttttagttg ttgttttagt tggaggggtt tttttttttt tttttgtagg tgttttagaa 1320
agtaggagtt gaagatttgg gtgtgaggga tatatatatt ttaaaagtat tatagtagag 1380
gaggtttagg tagtgttagg agttaaggtg agtgtatgat ttgattgtgt attaagggtt 1440
ttatttttag aaatagggta gatttggtag tatttggttt gtagttattt attgttattt 1500
ttggtgtttt aggttttgtt tgttagttgt gttttgaggt gtttttttgt gtttttttat 1560
aggtttttag aagataaatt ttttaggaag ataggtgatt tgtgaggttt tagagtatta 1620
ttttaagaga agaagagttg taagttggtt tttgttagag ttattatggg tgagtttttt 1680
agttgaggtt attggtattg tttttttttt ttttagtttt gtgggatttt attatattta 1740
tttgtgttta tatgtttatt tgttgttttt gaataatatt tattatgttt ttttttttaa 1800
attttttatg ttttagtttt gagtaaggtt tttagaaggt aatttttata ttggagttgg 1860
tagatgtaga ggatttttta gatgaggaag aggaggaagt tttttttatt tttttttttt 1920
ttttttattt tttatttttt ttgttttttt tttgtttttt ttttttattt ttattttttt 1980
ttttgttttt gtgtttttta ggggataagg atatgtttat tgttgggatg ttgagttttt 2040
tttagagttt ttttgagagt ttttagagtt gttttgaggg ggaggatttt tagttttttt 2100
tttagatttt ttagagtttt tttgagagtg atgatatttt gtattttttt tagagttttt 2160
agagttgttt tgagggggag gattttttgg atttttttta gagatttttt gaggggaagg 2220
atttttagtt ttttttttag attttttaga gtttttttga gggtgatgat atttagtttt 2280
ttttttagaa tttttagagt ttttttgagg ggaaggattt tttgtttttt ttagagattt 2340
tttagagttt ttttgagggt gaggatgttt agtttttttt gtagaatttt gtgagttttt 2400
tttttttttt tgttttattg agtatttttt agagtttttt tgagagtatt taaagttttt 2460
ttgagggttt tttttagttt gttttttaga tttttgtgag t 2501

<210> 203
<211> 2501
<212> DNA
<213> Artificial Sequence

<220>
<223> chemically treated genomic DNA (Homo sapiens)

<400> 203

gtttatagga atttggagaa tagattgggg aaaattttta aaaggatttt gagtattttt 60
aggggaattt tggaaaatat ttaataaagt agaggagaag aaggaatttg taggattttg 120
tagaggagat tggatatttt tatttttagg agggttttga gaaattttta gaggagatag 180
ggagtttttt tttttaggag aattttgaga attttggaga ggagattggg tgttgttgtt 240
tttaggagaa ttttggggaa tttggagagg agattgggag tttttttttt taggaggttt 300
ttggagagga tttgaggagt tttttttttt agaatgattt tgaggatttt ggagaggata 360
tagggtgttg ttgtttttag gagaattttg gggaatttgg agaggagatt gggagttttt 420
ttttttagga taattttgag gatttttaga ggaattttgg agaagatttg gtattttagt 480
agtaggtata tttttgtttt ttggagaatg tagaagtaga gaggaggatg aggatgagaa 540
agaaaaatag gaggaggatg aggggaataa aaagtggaaa gaggaagaga aaatggagga 600
agtttttttt ttttttttat ttgggggatt ttttgtattt attaatttta gtatgaaaat 660
tgttttttgg aagttttgtt taaagttggg gtgtggaagg tttagaaaga gaggtatgat 720
gaatattgtt taggagtagt aggtaaatgt gtgaatatga ataggtatga tgggatttta 780
taggattgag ggagagaggg atagtgttag tggttttagt tgagaaattt atttatgatg 840
gttttgataa aggttggttt atagtttttt tttttttaag gtggtgtttt agggttttat 900
aggttgtttg tttttttagg gggtttgttt tttgggaatt tgtagaagga tgtgagaaag 960
tattttgggg tatagttggt aggtagagtt tgaggtatta ggaatgatag tgggtggtta 1020
taggttgggt gttgttaggt ttgttttgtt tttgggggta gggtttttgg tatatagtta 1080
ggttgtgtat ttattttgat ttttggtatt gtttgggttt tttttgttgt ggtgttttta 1140
ggatgtatgt gttttttata tttaggtttt taatttttgt tttttggagt atttgtaaga 1200
ggaagggagg gagttttttt agttaagatg gtagttgggg ttggttagat ttttagagtt 1260
ttataatttt gaggtttgtg atttttttag aatttaatta gtattgttat atttttttag 1320
atagggttag gtttttatta ttttgttggt ttgaggaaaa attttaggaa aattttatat 1380
tattgagagt agtgggaggt agtgaggtgg tagttatttt ttagtatttt attatggggg 1440
tggtttttgt ggttttatgt ttgttttgat gtatatgtga ttttttagtt tttatttttg 1500
tttttagtag ggtttgggaa tttttttttt gttaattgga ggtagttttt tatgttgatt 1560
tgttatggga tttttagaat aaggtttttt tttttttttt ttttatattt tttatttagt 1620
agtgagaggg tggttgttta gttttagtta tttggggagt tttatggggg ttgatttgag 1680
gagtagggtg ggaatttttg ggaatttgtt ttttgaggtg ggggtttttt tggttttttt 1740
taatgttttt ttatttattt tttagagggt tggtattttt tttttttttt tgttgtgagg 1800
atatgttttt tagtttttgg ttattatttt tgtttttgtt atttataagt gttattgtag 1860
ggatagggta ggttagggtt gttttttttt gtggtggggt tgttttttat tttttttttg 1920
gggtttttat tttgaagatt ggataggttt ataagttttt tttttttgtt gatttgagat 1980
gatttttgtt ataaaatgtt ttaggttttt tagattttta agatggttgt tgggaaagta 2040
tatttggtaa agtatatttg ggaattttgt tagggttgat tagggttgat agtttggata 2100
gtgtggggtg gggttttttt ttagaggagg aaggtgtttt ttttttgttg tttgtttgtt 2160
agtttttttt ttattttttt ttttttattt ttaggatgtg ttttaaattt ttttgtttat 2220
tttttaagtt ttgttgattt tttttttttt atttttttag tttttgatat ttatatttta 2280
ttttttttta gggttgatag tagggagtgg tagggtgtag tttgtgtttt ttttgtgttt 2340
ggaggttatt gttttttgat agagtttttt ttttgttgag tggttagttt tggggttatt 2400
ttttttgttt aattgaagtt gtttttttta gagaaaattt tttttttggt tgatttttaa 2460
aatggaagtt agaaatttgt aaatttgggg attttgatgg g 2501

<210> 204
<211> 2501
<212> DNA
<213> Artificial Sequence

<220>
<223> chemically treated genomic DNA (Homo sapiens)

<400> 204
   ttttagtagg gtttgggaat tttttttttg ttaattagag gtagtttttt gtgttgattt 60
   gttatgggat ttttagatta aggtttttgt tttttttatg ttatttattt aggaataaga 120
   tggttgttat tttgtttgag ttgattgggg agttttatga gggttgatat ttgaggagta 180
   gggtgggaat ttttgggaat ttgttttttg aggtgggggt ttttttagtt ttttttagtg 240
   tttttttatt tattttttag agggttggta tttttttttt ttttttgggg ttattttttt 300
   gtttgggatt tattagtttt atggtagtgt agtataggtt ggggtttttt ttttttatgg 360
   tgggaggttt tttagttttt ttttagggtt tttattttga ggattggtta ggtttaagat 420
   tttttttttt gttaatttga tttgtgtttt tttataaatg tttttgattt gttagatttt 480
   taagatggtt gttgggaaag tatatttggg aattttgttg tggttgttta gggttgatgt 540
   agtgttaatg tggggtgggg tttttttttt gaggaggaat tattttttat ttttttgttt 600
   tttttttatt tttttttttt tattttttag ttgtttttta attttttttg tttatttttt 660
   aagttttgtg gattttttta tttttatttt tttagttttt gatattttat tttttttttt 720
   ttaggggtgt tagtagggag tggtttggtg tagtttgtgt tttttttttg tgttgggagg 780
   ttattgtttt tttatatttt ttttgttttt tggttagttt tgggattttt ttttttgttt 840
   aattgaagtt gtttttttta gagataatag tttttttttt tgttgatttt taaaatggaa 900
   gttagaaaat tgtatatttg gggattttga tggggttttt tagagttggt agtagggata 960
   ggggaggggt attattagtt atgggtgttt ttttttagtt tttttgagag gttgtgtttg 1020
   gatttttgat attgtttggg atgtttgatt ttgtgtattt gagtttttta tttttgaata 1080
   agtttttttt atttttgaga tttgtaaggt gtaagagagg gagaattata tttatttata 1140
   gtgggatagg gttttttagt tttaaggtag gtttttattg ttttttgaaa tggggtggga 1200
   atgttttttt gtttttttta aggtatgatt tttaaaagag tttatatttt tttttttttt 1260
   tgtttagata gtaatatttg tttttttgat ttttaaattt tattttaagt ttttttattt 1320
   tattgttgtg agtgaaatta gtgttatttt ttttatattt tgtgttaaag gtttttttat 1380
   tgttgttttt ttttttattt tagtttttga aaaaaattat tgttttttta gtaatttgtg 1440
   attttttaaa aaattaatat gtgattaaag ttttaaatat atatattaga tttaattaga 1500
   atgtgaagtt atatgtattt tggaaaatgt atggattttt atatttatta attaagtatt 1560
   atattgatta gttttatttg tttaaatttt ttttgtgtat tttttttgag gttaattatt 1620
   gtatttttat aaagttttta gtaattgttt tttgttttta gtttttatat ttttaatttt 1680
   tttaatttgt tatatgatta gttttataaa atattgattt aaaagtgtgt ttgtattatt 1740
   ttttattata attagtaagg aatgagaatt tttgttattt tatatttttt tttatattga 1800
   gtattttaga tttttaggtt ttagttattt ttataagtat gtattggaat tttttttggt 1860
   ttttgtttgt ttttttttaa tgatatatga tattggttat ttttttatat gttttttttt 1920
   tgtttgttat attttttttt gtaaagtgtt tatttaaatt atttgtattg ttttatagat 1980
   gaataaattt tatttttaga gtattgttaa tttaaaaata taaagagaaa tttttattag 2040
   aattagagtt tttatataat ttatattatt tgttttgttt tgattgttgt atatatatat 2100
   aaagtttatt tttttattta tattttgtat tattatgata tgtttgttat aattgatgga 2160
   attgtattga tattttatat ttaagtttat ggtttatatt aggatttatt tttaatgatg 2220
   gggatattat atattttgat aagggtgtaa tggtaagtat ttattattgt attgttttat 2280
   agaattgttt tattatttta gagtttttgt gtgttttatt taatgaatta attttttttg 2340
   ttttaatttt agtttttagt ttttggaaat tagtaatatt tttagtgtat ttattattgt 2400
   atgttttaga atgtggtatg ttaggaagtt tttttagatt tttttttttt atttagtaat 2460
   atgtttttat gattttttta ggtttatttt attaataatg a 2501

<210> 205
<211> 2501
<212> DNA
<213> Artificial Sequence

<220>
<223> chemically treated genomic DNA (Homo sapiens)

<400> 205

ttattgttag tgggatagat ttggaggaat tatgaaagta tattgttaaa tgaaagaaga 60
aaatttaaaa aggtttttta gtatattata ttttggaata tgtaatgatg aatatattaa 120
aaatattatt ggtttttagg ggttgagggt tggggttggg gtaaggggaa ttgatttatt 180
aggtggaata tataggagtt ttaagatggt gaaataattt tgtgggataa tataatgatg 240
aatatttgtt attatatttt tattgaaatg tataatattt ttattgttaa gagtgagttt 300
tgatgtaaat tatggattta agtgtgaggt attaatatag ttttattagt tgtagtaaat 360
atgttataat aatataaaat gtaaataaaa agatagattt tgtgtgtgtg tgtagtaatt 420
agggtagggt gggtggtgta gattatgtag gaattttaat tttgatgaag attttttttt 480
gtatttttga attagtaatg ttttaaaaat gaagtttatt tatttataaa atgatgtaaa 540
tgatttgaat agatatttta tgaaagggaa tatagtaaat ggaaaaaaag tatataaaaa 600
gatgattaat gttatatgtt attaagaaga ggtaaataaa aattaaagaa ggttttaata 660
tatgtttatg agaatggtta aaatttgaaa atttaaaata tttaatgtaa gagaggatat 720
agaataatag aaatttttat tttttgttag ttgtgataaa aaatagtata ggtatatttt 780
taagttagta ttttatgaag ttaattatat gataaattgg aaaagttaaa agtataggaa 840
ttgaaaatag aagatagttg ttaggagttt tgtagaagtg tggtgattga ttttaaaagg 900
gatgtatagg agaagtttag gtaggtggaa ttggttagta tgatatttgg ttggtagata 960
tgggagttta tgtatttttt aaaatatata taattttata ttttagttaa atttgatgtg 1020
tgtatttaaa attttaatta tgtattggtt ttttaaaggg ttatgggttg ttgaaaagat 1080
agtggttttt tttagggatt aggataggaa aagaagtaat aataaggaag tttttaatat 1140

agggtatggg agagatgata ttaattttat ttatagtagt aaagtggaaa aatttggaat 1200
ggaatttaga ggttgaagga ataaatgttg ttgtttggat aggagaagga aggagtatag 1260
gtttttttag gagttatatt ttgagaaaga tagaggggta tttttatttt atttagaggg 1320
gtagtggagg tttgttttag ggttaggagg ttttatttta ttgtggatgg atgtgatttt 1380
tttttttttg tattttgtag gttttaggga tgggaggggt ttatttggag gtgaaagatt 1440
tagatgtata gggttaggta ttttaggtag tattaggagt ttaggtatag ttttttagga 1500
aggttggaag ggaatattta tgattggtag tgtttttttt ttgtttttgt tgttagtttt 1560
ggaggatttt attagggttt ttagatgtat aattttttga tttttatttt gagagttagt 1620
agaaggggag gttgttgttt ttgaggaggg tagttttagt taagtagaga aagaagtttt 1680
agaattggtt aaaaaataag agagatgtgg aaggatggtg attttttaat atggagggga 1740
ggtgtagatt gtattaagtt attttttgtt gatatttttg gaaaaaggag agtggggtgt 1800
taaggattgg ggaggtggga gtgaagaggt ttgtaaggtt tagggagtga gtaggaaggg 1860
ttggagggta gttgggaggt ggggaggaag ggatggaaaa aaagtagaga ggtggggggt 1920
ggtttttttt tagagaggag gttttgtttt atattgatgt tgtgttagtt ttggataatt 1980
atggtagggt ttttggatgt gtttttttga tggttatttt gggaatttga tggattgaag 2040
gtatttgtga ggaggtgtga attaagttag tggggggaag agttttagat ttggttagtt 2100
tttagggtga gggttttgag gaagaattga gggatttttt attatagaga gaagaaattt 2160
tggtttgtat tgtgttgttg tgagattggt aggttttaga tagggaaatg gttttagaag 2220
aagggaggag gtgttggttt tttagggaat aaataggaag atattgagga gggttggggg 2280
aatttttatt ttagagggta gatttttaga gatttttatt ttgtttttta agtattagtt 2340
tttgtagagt tttttagtta gtttaggtgg ggtggtagtt attttatttt tgggtgagtg 2400
gtgtagggga ggtggaggtt ttggtttgag ggttttatgg taagttagta tggggagttg 2460
tttttggttg gtagagggaa gatttttagg ttttgttggg g 2501

<210> 206
<211> 2501
<212> DNA
<213> Artificial Sequence

<220>
<223> chemically treated genomic DNA (Homo sapiens)

<400> 206

agtgggatgt ggggtgtagg ggttggtttt aggttttttg agtttaagtt ttgttttatt 60
ttgttttgta agtttagaaa agatgttttt gtttttggtt gggtaatttt tggatttatt 120
tattgtttgt aattttttgg gttgaagaga ttattttttg ggagttaatt aatttatttt 180
ttggagtgtg tgtttttttt tgtttttagg agttggtttt ttaaaatttt ttgaggtttg 240
tgtatgtttt ttttgatttt ttgtgttttt attttttata gaaatttatg tatttatgtt 300
tttttttttt tttgagtttg tagaggattt tgttttgttt tttttatatt tagggttgtt 360
ttttttgttg ttaatataaa tttgttgata ggttatgttt gggaagtggg tggggtgttt 420
tggagtggtg ttgaggtgtt gtaggtttgg tttttggttg tgggggagtt gtattttgaa 480
gttttgttgg aatttgtgtt tggggttagt agggggtatt agagttaata gggattgtat 540
gtaaaattta ttttttttta ggttttttag ggggatggtt aggtttttgt agagatgggg 600
aggatttttt tttggtaagg ggagagggat gggttaggag ttaagtggaa ggattttgtg 660
tttaaggttt tttaagggat gggataggtg aggaattttt ttgagttttt tgaattgttt 720
tattagttag gtttgggaag tattttgttt ttataaattt tgtttttttg aaaggggttg 780
ggggggggat tttaatattt tttgttatag gttatgatgg gtatggtggt taagagtaat 840
aggttgtgtg gtgaggttgg ttttttgtgg ttttgttttg ttattttttg aggtattttt 900
atgttagtga atagagttgt gggtttttag ttgtatagat gaatgtatga ataaaatata 960
ggtttatata aaggaagtta ttgtattgat ttagatttga tttgggattg ttgtataaaa 1020
tagggttgat aaaaattgtt gtgtttttat tttggaaata gagatttagt tttttttttt 1080
ttgtttgagg tatttagtaa aggtttgttt ttgagtggtt gtggatgttt aaggatttat 1140
gatttttgat tttataaagt ttagggtgaa aataaaaaat aaaataagga gattattgtt 1200
ttaaagtggt tgttgtagtt aattatttta agaaagtaaa taggttggta ttgaaagtta 1260
tataggtggg tggttttttg ggagggttgt ttagtttttt ttttttgaga tttgagtagg 1320
gagtagttag aggagtttga agggatggtg tttagggtat gggaataggt tgtatatatt 1380
tttatggagt atggtgtttg tttagtttga gaaggaaaag aaatgggagt agagggttga 1440
tgagaaggta gtagatggat agtgtgggat tgaaaagggg atttatttga agattggtta 1500
ttattattta tttttattat ttgattgttg ttgttttatt gagattgatg tgtggagaat 1560
aagttgggga gtggaataaa tggtagggag gggagagaag tgagtgaata tgattttggt 1620
ttagttttat aggatagagt gtggttatga agtttataga ggaggatagg tagggaggaa 1680
ttatggtatg gttaggttgt agggtaagag gtagagagga gagaggattg tggaggatgt 1740
ttgtggagtg ttgattgtta gttgaggttg gttttttatt tatttatttt ttttttttga 1800

gatggagttt tgtttttgtt taggttggag tgtagtggtg tgattttggt ttattgtaag 1860
ttttgttttt tgggtttatg ttattttttt gtttagtttt tttaagtatt gggattatag 1920
gtgtttgtta ttatgtttgg ttaatttttt tgtttttaat agagataggg ttttatgtgt 1980
tagttaggat ggttttgatt ttttgaattt atgatttgtt tattttggtt ttttaaagtg 2040
ttgggattgt aggtattagt tattgtgttt ggttaaggtt ggtttttttt tttatggtga 2100
tgggtgtttg aaagttgtat atattaaaat aaatttatta tttgttgatt taaagtaaaa 2160
ttttgagggt atgaaaggga agggtttatg tttatatggt tgagataaaa gttgttttag 2220
gaagttgggt gtggtggttt atttttgtaa ttttagtatt ttgggaggtt aaggttggag 2280
gattttttga ggttaggaat tgaagattag tttgagtgat atagtaagat tttatttata 2340
tttttaaaaa gggttggttg ttttgggatt ttttgaaata gttttgtaag atatttttgt 2400
tttggaatta taataattta gataagatgt ttttgaaaga atatttgttt aggtatagtg 2460
tttttattaa tgaattgatg ttaattgtgg ttttgagttt t 2501

<210> 207
<211> 2501
<212> DNA
<213> Artificial Sequence

<220>
<223> chemically treated genomic DNA (Homo sapiens)

<400> 207
   aagatttaaa attatagttg gtattagttt attggtggag atgttgtgtt taggtaggtg 60
   tttttttaaa agtattttat ttaaattgtt gtgattttaa aataggaatg ttttgtaggg 120
   ttattttgaa aagttttgag atagttaatt ttttttaaaa atatagatgg ggttttgtta 180
   tattatttag gttagttttt aatttttggt tttaggggat tttttaattt tggtttttta 240
   aagtgttggg attatagggg tgagttatta tatttagttt tttgagatag tttttatttt 300
   agttatgtaa gtatgagttt ttttttttta tatttttagg gttttatttt gggttaataa 360
   atagtgagtt tattttggta tgtgtaattt ttaaatattt attgttataa aagaaaggat 420
   tagttttggt taggtatagt ggttgatgtt tgtaatttta gtattttggg aggttgaggt 480
   gggtggatta tgagtttagg agattaagat tattttggtt aatatgtgaa attttgtttt 540
   tattaaaaat aaaaaaatta gttaggtatg gtagtgggta tttgtagttt tagtatttgg 600
   gaggttgagg tgagagaatg gtatgaattt gggaggtgga gtttgtagtg agttgagatt 660
   gtgttattgt atttagtttt gggtaagagt gagattttgt ttttaaaaaa aaaaataaat 720
   aaataaagga ttagttttga ttgatagttg gtattttgta ggtatttttt ataatttttt 780
   tttttttttg tttttgtttt tataatttgg ttatgttatg attttttttt atttgttttt 840
   ttttgtggat tttatgatta tgttttgttt tatgaggttg gattagggtt atgtttattt 900
   attttttttt tttttttgtt atttgtttta ttttttaatt tgttttttat gtattgattt 960
   taatggagta atagtaatta gatgatgaaa ataaataatg gtggttagtt tttaaatgga 1020
   tttttttttt aattttgtat tatttatttg ttgttttttt attagttttt tgtttttgtt 1080
   tttttttttt tttaaattgg ataagtattg tattttgtga agatgtgtgt agtttgtttt 1140
   tatgttttga atattatttt tttaagtttt tttggttgtt ttttgtttag gttttagaaa 1200
   aggagagttg ggtaattttt ttagggagtt gtttgtttgt gtaattttta gtattagttt 1260
   gtttgttttt ttgagataat tagttataat ggttatttta aaataatggt ttttttattt 1320
   tgttttttgt ttttatttta aattttataa agttagaaat tgtaagtttt tgagtattta 1380
   tagttattta aggataaatt tttgttgaat gttttaagta aagaggggga ggttgagttt 1440
   ttgtttttag ggtgaaaata tgatggtttt tattagtttt gttttatgtg ataattttag 1500
   gttgaatttg gattaatgta atgatttttt ttgtatggat ttatgttttg tttgtgtatt 1560
   tatttgtgta gttgggaatt tgtagttttg tttattggta tggggatgtt ttggggaatg 1620
   gtagggtaga gttataaaaa gttagtttta ttatgtggtt tgttgttttt agttattatg 1680
   tttattatag tttgtggtag gagatgttgg ggtttttttt ttgatttttt ttaggaaaat 1740
   agagtttgtg gaggtggagt attttttagg tttgattgat aaaataattt aaaagattta 1800
   aagagatttt ttgtttgttt tgttttttga agggttttga atatggggtt tttttgtttg 1860
   gtttttagtt tgtttttttt tttttattag gaaaggattt tttttatttt tgtagaagtt 1920
   tgattatttt tttagagggt ttgggaggaa gtgggttttg tatatagttt ttgttgattt 1980
   tagtgttttt tgttggtttt agatgtgagt tttggtgagg ttttagggta tagttttttt 2040
   gtagttagaa gttgggtttg tagtgtttta gtattgtttt gggatatttt atttgttttt 2100
   taggtgtgat ttgttaatag gtttgtattg gtgataaaag gagtagtttt gaatgtaggg 2160
   aaagtagggt ggagtttttt gtaggtttgg gggaggggag gggtgtgaat gtgtggattt 2220
   ttgtggagag tggaaatatg ggagttgagg gggagtatgt gtgggtttta gaaagttttg 2280
   ggaaattgat ttttgggagt agggaggaat gtgtgtttta gagagtaagt taattgattt 2340
   ttggggggtg gtttttttag tttggggggt tatagatagt aggtaaattt agagattatt 2400
   tagttaaggg taggggtatt ttttttagat ttgtggggtg gggtggggtg agatttgagt 2460
   ttaggggatt tggaattggt ttttgtattt tgtattttat t 2501

<210> 208
<211> 2501
<212> DNA
<213> Artificial Sequence

<220>
<223> chemically treated genomic DNA (Homo sapiens)

<400> 208

tgttattaga gtgtatttta tgtgtatgtt tttttttaag agttattgta aggattttgg 60
gtttgttgtg tggtttaaga atttatgttg tttttgaggt tagatttagt tatagtgatt 120
agggtttttt tgttatggtt ttggtggtgg ttggttttaa attggttata gtttgaagat 180
ttattttgaa agggattaag gttagagata gtgttttttt ttttttattt ttagtttagt 240
atttttatta agtatatttt ttggggatta taaatgtttt tttttttgaa gattttagta 300
gtgatttgtg ggattttggt tgggtgtttg tgatttattt ttaaagagaa tttgtatgtt 360
tggggtttta ggatggttgg tttgtttttt ttgtttgaag atgattttgt tttttttttt 420
tagttttggt gggtttttgg attgttgttg ttgtgttttt tttggtttgg gtatgttggg 480
ttggtgtggt tttggtttgt gtttttgttg ggtggttgtg gtgtgggtgg tggtgggata 540
agttgtgagt gtgtgttggg tgtgtgtttg gttggggtag ttgtggggga ggagtgtttg 600
tttgttgttt gtgtgtttaa tggattaggt tggggttgtg aggtaattgt tgtagttagt 660
ggaggtggga ggtgatattg agtttttagt tttgagaggt gtttgaggga aaaggaggtg 720
gtagttaaat tggttttgga gagaagtttt ttttgttttt ttttttagtt agtatgtttt 780
ggattttgtt gttttttagt ttgtgtggtg gggattttgg gttgtggtgg ttggtgtagt 840
tttgatgggt tgtgaattag ggggtgtttt gaatgtgggg gttggggttt gggagtgtga 900
gtggttgtta tggtatgagt ggggtgtgtt gagttttgtg gttatttttg gttttagtta 960
tgaggaggga tgagtgagat gttaaagtta tgtggttttt gtagttgttg gatggggttg 1020
gtttgttttt tgagagtttg aggaatggtt atgtgaagag ttgtgtgagt tttttgtggt 1080
aggatttttt gtgtggtttt tttttttatt tttgttgttt ttgtaatgtg gagttgtggt 1140
tgttgttggt ttttttttag tagttgtggt gttgtttttt tttttgttgg gtgtgttttt 1200
tgttgggtgt tttggttgtt tttgtttttg ttttgttgtt gggtgtggaa tttgagagtt 1260
gggttgttgg ggttgtttgg ttgtggatgt tgttgagtgt gtgtttgtat agtttgagtt 1320
ttttttttag tattgtttgt gttttttttt tttttatttg tttttttatt tggattaagt 1380
ggggatttgg tttgggttgg agttgtggtt tttggtggtt gttggtgttg tttgtttgtt 1440
gttatttggg ggattttttg gtgtggtagt ggtggttttg gttttggggg gatggtgatg 1500
tagggtttgt ggttttgtat atgtttttgg aggtggtagt gggtaggttg ttgttggtgt 1560
tgagttgtgt agggttgttg ttgatgtttg tgtatttgtt gtggttttgg tattgtgttt 1620
tggtgttgtt tttggttagt tttgtttggt gggttttttt tattagtttt gggttgttgt 1680
tttttgtttt taggttgttg ttttttggtt tggtgggggg tgttggttgt ttgttggttt 1740
tggggttgga ttattttttt taaattaggg aagtgttttt ttatttttga ttgtttagtg 1800
ttgttgaaga aaaagtgttt gtgatttgat tttggaggag gtttagttgt gtgttgttag 1860
gagaaattgt agttagttat tatggtagtt gtaaaatatt taggagattg ttgttgtttt 1920
gtatttttag agaataggta tgttagttgg aaggtgaggt ttgggatgtg agtgggtttg 1980
ggtttattgt tgtagttttt gagttgaatt tgtttatttt aaagtatgtt aatttttaga 2040
atggaaaaag ggtgtgttgt gggggggggg ggggaggaaa taatgtttta ttttggaaag 2100
gatttttaaa atataggaag ttttttaaaa atgtagaagt aagtagtttt tgttattgaa 2160
tagaaattaa atagtttgtt aattatatgg tattttttaa ggattgtata tgtaatagat 2220
gagtttgtag aagaatgagg tgaattattt gtggttgaat agattgatta tttttgtttt 2280
tggtattttt tgatatgtgt ttttttggtg ttatttattt gtttgttgtt ataatgatgt 2340
ttatttaaaa ataatatata gtttttagaa tgagaggata tatttatgag ttttaattag 2400
tgtggttttg ttattgaaaa ttgagaggaa aaatttgaga ttttattgtt ataagatatg 2460
agttttgtaa atgagagtaa tttagatgaa aagatagtag t 2501


<210> 209
<211> 2501
<212> DNA
<213> Artificial Sequence

<220>
<223> chemically treated genomic DNA (Homo sapiens)

<400> 209

attgttgttt ttttatttaa attattttta tttgtaaagt ttatgttttg taatgataaa 60
attttagatt tttttttttg atttttaata atgaaattat attgattaaa atttatagat 120
gtattttttt attttaaagg ttgtatgtta tttttgggta ggtattatta taatagtaaa 180
tagataaatg gtattaaaaa gatatatatt agaggatgtt aaaagtggga gtagttaatt 240
tatttaatta taaatgattt attttatttt tttgtgaatt tatttattgt atatatagtt 300
tttaaaaagt gttatgtgat tagtgggtta tttgattttt atttaatgat aaaagttatt 360
tgtttttgta tttttaaaag gttttttgta ttttaagaat tttttttaga ataaaatatt 420
attttttttt tttttttttt tgtaatatat ttttttttta ttttgaaagt taatatgttt 480
tgaaataagt gaatttaatt tggaaattgt agtggtaaat ttgaatttgt ttgtgtttta 540
gattttgttt tttagttaat atatttgttt tttggaaata taaggtaatg atggtttttt 600
gaatattttg taattgttat agtaattggt tgtagttttt tttaatgata tgtagttgga 660
ttttttttgg ggttggatta taggtatttt ttttttggtg gtattggata gttgaggatg 720
aagaggtgtt tttttgattt gaaagaagtg atttaatttt agggttagta ggtagttagt 780
gttttttatt aggttggaga gtagtggttt gagggtggag ggtagtgatt tgaggttggt 840
gaaggagatt tattagatga agttggttag gagtagtatt agaatgtagt gttggagttg 900
tagtgggtgt gtgagtgtta gtagtagttt tatgtagttt agtattagta gtaatttgtt 960
tgttgttgtt tttgggggtg tgtgtggggt tgtggatttt gtgttgttat ttttttaagg 1020
ttaagattat tattgttata ttaagaagtt ttttaggtaa tagtaggtgg gtagtgttag 1080
tagttattag gagttgtagt tttggtttgg gttgggtttt tgtttggttt gggtgaggaa 1140
gtaggtgagg aagaagaagg tataggtgat gttgaagagg gggtttaagt tgtgtgaata 1200
tatgtttagt agtgtttgta gttaggtggt tttggtagtt tagtttttgg gttttgtgtt 1260
tagtagtagg gtgaggataa aggtagttag ggtgtttagt gagaggtgtg tttggtagaa 1320
gggggagtag tgttgtggtt gttggggaga ggttggtggt ggttgtagtt ttatgttgta 1380
gaagtggtag aggtggaaga agaagttgtg tggagggttt tgttgtaagg ggtttatgta 1440
gttttttatg tagttgtttt ttagattttt ggggggtgag ttggttttat ttggtggttg 1500
tagggattgt atggttttgg tgttttgttt gttttttttt atggttgggg ttgggggtgg 1560
ttatgggatt tagtatattt tgtttgtatt gtagtggttg tttgtgtttt tagattttaa 1620
tttttgtgtt tggggtgttt tttggtttgt aatttgttag ggttgtgttg gttgttatgg 1680
tttggggttt ttattgtgtg gattgaggag tggtggagtt tgggatatgt tggttgagga 1740
gaggggtgga aggggttttt ttttaggatt agtttagttg ttgttttttt tttttttggg 1800
tattttttgg gattggagat ttagtgttgt tttttatttt tgttggttgt aatagttatt 1860
ttatggtttt agtttggttt gttgggtgtg tgagtggtgg gtgggtgttt tttttttgtg 1920
attgttttgg ttgggtgtgt gtttagtgtg tgtttatgat ttgttttgtt gttatttgtg 1980
ttgtggttgt ttaataggga tgtgagttgg gattatgttg atttagtgtg tttaggttga 2040
ggaaagtgtg gtggtggtag tttgaagatt tattgggatt gaaagagaag gatgaggtta 2100
tttttggatg ggaggggtaa gttagttatt ttgggatttt aggtgtgtag gttttttttg 2160
agggtaagtt atggatattt agttagaatt ttataggtta ttattggggt ttttgggaaa 2220
gagggtgttt gtggttttta aggggtgtgt ttagtgggga tgttgagtta agggtgggag 2280
gggaggggtg ttgtttttaa ttttagtttt ttttagggtg ggtttttaga ttgtgattag 2340
tttagggtta gttattatta aggttatggt gaagaaattt taattgttgt ggttagattt 2400
agttttagaa gtaatgtgga tttttgggtt atgtagtagg tttaggattt ttgtagtggt 2460
ttttagggag gaatgtgtat gtggagtgta ttttagtagt a 2501

<210> 210
<211> 2501
<212> DNA
<213> Artificial Sequence

<220>
<223> chemically treated genomic DNA (Homo sapiens)

<400> 210

aagttatatt gttagtggtt tttttgggta tgggggtggg agttgtagag ttaggggttt 60
ttatttgtgt tttgaaggag ttataggtta atgggggtga gaatggagta ttttttttag 120
aggtttttaa ggatagaatt tattggtagt tagtttgata ggtattataa ttttagtttg 180
gatttggagt gtagtttttg agataaaatt aaaggtttta tggtaaatat agtatgagat 240
ttttggtggt gggggtggtg gaagttatgg gttggaagtt ggaggtggta ggtgggtggg 300
tagtaggttt agttttgttt ttgtattttt tttttattag gagttttggt ttttttggtt 360
ttttttattt agtatatata gttttttttt tgtttttgag gagtggggat attttaaaga 420
gtaagagttg tgattttttt attttatttt taatgtatat gttgggtttt ataaatttaa 480
gtatgaataa gatgaaatga tttttgtttt tgaggatgtt tagtgagtgg gagaggggt 540

ggatttttaa aggggtgtat ataatatagt ttaatatatg ttgagttgtg ttatataggg 600
agttatagat tgagtaatta ttgggtgagg ggaggggttt taggagagat tgggattgtg 660
tgtattgtgt tttttttttt tttttttaag atagagtttt gttttgttat taggttggag 720
tgtaatggtg tgattttaat ttattgtaat ttttgttttt taggtttaag tgattttttt 780
gttttagttt tttgagtagt tgggattata ggtgtgtgtt attatattta gttaattttt 840
gtatttttag tagagatggg gttttattat gtaggttagg atggttttga ttttttgatt 900
ttgtgatttg tttgttttgg ttttttaaag tgttgggatt ataggtgtga gttattgtgt 960
ttggtttgta ttggggtttt aagttttaat agttttaagt tagggttggt ttttttaaga 1020
aaaaaattta tatatataga tgtgttatat tttgtattta attttagggg ttttagggat 1080
atattgatgt ttatgtggag tttttaaata attttaatag tttatggggt taggttttaa 1140
aaatttaatt tagggttagg tgtagtggtg tatgtttgta attttagtat tttgggaggt 1200
taaggtgggt gtatagtttg agtttaggag tttgagatta gtttggttaa tatagtgatt 1260
ttatttttta tttttgtttt ttattaatta tttataaaaa aatataaaaa ttaaaggggt 1320
gtggtagtgt atttttgtag ttttagttat tgggaaggtt gaggtgggag gattatttga 1380
gtttaggagg ttgaggttgt agtgtgttgt gattatgtta ttgtatttta gtttgagtaa 1440
tagagtgaga ttttatttta agggaaaaaa taaaataaaa taaaataggg aatgtgggaa 1500
ggaagtgttt tttgatgata ttggttatag tgggtttgaa gttgggttgt ggaattggag 1560
attttgggaa aataatattt gttggagtta tgattttagg agtttattaa gtgagtgttt 1620
attgtaggag ggaggtgttt atggaaagat tgtgaggtag tgaatatttt gttgttggtg 1680
aagtgtaagt gtagggaggt tggggaggaa tttaaagtta tgaagatatt gatttaatgg 1740
gttttaagtt tttttagttt tgggatttta tttttgggtt aattttgttt tttttgagga 1800
gtgtatgtgt aatgggatta tttgtttaaa gaatttattt gatttgagat ggtgtgaatg 1860
tgtagttgag tgttgatttt ggttttttta tgagtttttg gggtaggttt taaggtgagt 1920
gatggaagta gttgttttta gtttttggta atatgatagt ggttgttgag tgatttggaa 1980
gtatttttat tttgtgttgt ttgggtttgg tggtagttgg gtttggagtg gatgtgttat 2040
tatggggttg tttaagaagt attgtggaga gaaggaggtg gttgggatga tggtggtggt 2100
tggtattggg ggtgttattg agtagttgtt gtggtattgg gaatataaaa aatataagta 2160
ttggagtggt ggtagtggtg gtagtggtgg tgaatgatgg aagtggagtt gggaatgtgg 2220
gggtgagtgt gggagtgggt ggtgtggggt tgaagttgag gtttggagta gtatgtatgg 2280
gtgggagtgt agttaggtag agttttttga gtggtgtgtg aagtgggaga agtgtgatga 2340
tggttatgag gttggtgagg aggtggggtt tgtgtagggg gtgggttggg tgggggtttt 2400
ggaatgtgtg ggtttgggtg tgtgtgtagt gttagtgaag ttttttaggt taggaaattt 2460
ttaagttgtt tattagtttt attaaatagt ttttttatgg g 2501

<210> 211
<211> 2501
<212> DNA
<213> Artificial Sequence

<220>
<223> chemically treated genomic DNA (Homo sapiens)

<400> 211

tttgtagaaa gattatttaa tagagttggt aaatagtttg gggttttttt ggtttgaaga 60
gttttgttga tattgtgtgt atatttagat ttatgtgttt tgggattttt gtttgatttg 120
ttttttgtgt aggttttgtt tttttattgg ttttgtagtt gttattgtgt tttttttgtt 180
ttatgtgttg tttggagggt tttgtttggt tgtgtttttg tttgtgtgtg ttgttttggg 240
ttttagtttt ggttttgtgt tgtttgtttt tgtgtttgtt tttatgtttt tggttttgtt 300
tttgttgttt gttattgtta ttgttattgt tgttattttg gtgtttgtgt tttttgtgtt 360
tttggtgttg tggtggttgt ttggtggtat ttttggtgtt ggttgttgtt gttgttttgg 420
ttgttttttt ttttttgtga tgttttttgg atgattttat agtggtatgt ttattttgag 480
tttggttgtt gttgagttta gataatgtaa aatgggaata tttttgggtt gtttggtagt 540
tgttgttgtg ttgttggaaa ttaagagtgg ttgtttttgt tgtttgtttt ggagtttgtt 600
ttagaggttt atgggaaggt tgggattagt gtttgattgt atatttgtgt tgttttgggt 660
tgggtgggtt ttttgagtga gtggttttat tgtgtatgtg ttttttaagg gaagtggaat 720
taatttggag gtgggatttt agaattggaa ggatttagag tttattaagt tagtgttttt 780
gtggttttaa gttttttttt ggtttttttg tgtttgtatt ttattggtaa tggggtgttt 840
attattttat ggtttttttg tagatatttt ttttttataa tgggtgtttg tttagtggat 900
ttttagggtt gtggttttaa tgggtgttgt ttttttgaga tttttaattt tatagtttag 960
ttttagattt gttataatta gtattgttag ggaatgtttt ttttttatgt tttttgtttt 1020
gttttgtttt gttttttttt ttgagatagg attttatttt gttgtttagg ttggagtgta 1080
gtggtgtgat tatagtgtat tgtagttttg attttttggg tttaagtgat ttttttattt 1140
tagttttttt agtagttggg attatgagag tatgttatta tgttttttta atttttgtat 1200

ttttttgtag atggttggtg gggagtgggg gtagggggtg gggttattat gttggttagg 1260
ttggttttga atttttgagt ttaggttata tatttgtttt ggttttttaa agtgttgaga 1320
ttataggtat gtgttattgt gtttggtttt ggattggatt tttaaaattt ggttttgtga 1380

attgttaaga ttgtttagga gttttatatg ggtgttagtg tatttttgga atttttgaaa 1440
ttgaatgtaa aatgtggtat atttgtgtgt atgagttttt tttttgagga gattagtttt 1500
aatttaaaat tattgggatt taaaatttta gtgtaggttg ggtgtggtgg tttatgtttg 1560
taattttagt attttgggag gttaaggtag gtgaattatg aggttaagag attgagatta 1620
ttttggttta tatggtaaaa ttttgttttt attaaaaata taaaaattag ttgggtgtgg 1680
tggtgtatat ttgtagtttt agttatttag gaggttgagg taggagaatt gtttgaattt 1740
gggaggtaga ggttgtagtg agttgagatt atattattgt attttggttt ggtgatagag 1800
tgagattttg ttttaaaaaa aaaaaaaaaa agtgtagtgt atataatttt aatttttttt 1860
gaaatttttt tttttattta gtggttgttt agtttgtggt tttttgtgtg gtataattta 1920
gtatgtgtta ggttatgttg tatgtgtttt tttaaggatt tatttttttt tttatttatt 1980
gggtgttttt agaggtagga gttattttat tttatttatg tttgaattta taggatttaa 2040
tatatatatt ggagatgggg tggagaagtt atagtttttg ttttttgaga tgtttttatt 2100
ttttaggagt agagaagggg ttgtgtgtat tgggtggggg gaattaaaag agttaaagtt 2160
tttggtgggg agggggtgtg gggatagagt taagtttgtt gtttatttat ttgttatttt 2220
taatttttaa tttgtagttt ttattatttt tattattaga aattttatat tgtgtttgtt 2280
ataaagtttt taattttatt ttagggattg tattttgggt ttaggttggg gttgtagtgt 2340
ttgttaggtt ggttgttggt gggttttgtt tttgggggtt tttgaggaga gtgttttatt 2400
tttattttta ttgatttgtg gtttttttaa gatatagata gaggtttttg attttgtagt 2460
ttttattttt atgtttaagg aaattattga taatgtggtt t 2501

<210> 212
<211> 2501
<212> DNA
<213> Artificial Sequence

<220>
<223> chemically treated genomic DNA (Homo sapiens)

<400> 212

ttatttgtat tttttggggg agttttttgt ggaggaagtt atggtgtatt tatttttatt 60
gtttttgttt atgttttttt tttgttgatt ttatgttggt tttatggttt tgtatgttgt 120
agtggttata gtttgtgata atattttttt ttttgtttta gttgttttaa ttttattttt 180
ttttttgtta gttttgggtt tagttttgga tttagttttg ggttttgaag ttgaggtttt 240
ggttgtagtt gttattttgt gtttttaatg atttttgggt ttttttgtgg ttgttgaaga 300
tttgttgagg tagaagttgt ttgtggtgtg tgttttatta aaaatttgtt tttttgttat 360
tgaaagagaa attgggaatt tagataatag gaaaattttt atatatatta aatttatatt 420
attttatatt ttgtaaaagt ttttattaga tataaaaatt tattataatg gtatgtgttt 480
tgttataatt ttggatagaa gttgggtttt tgggtaggaa gtggaagtta tagaggaagt 540
tatggagtga gagtgatgtt tgtagtgaag aatttggtgt tattttgaga aggttaaagt 600
gattttttga ttaaatgttt tatttattgt ttagtgtgat ttggtgtttg ggtttatttt 660
ttttagtaat tgtgttttaa atgtaaagaa aattgtaggt ttttggggaa ggattgattt 720
tattatttaa gagaaaaatt tattttttag attttttttt tttatgtaaa tgtttggtat 780
ttagtagatg ttaaatatat gtgagttttt tggttttata agattatata ttgtaaatta 840
atagtaaatt atattaatat gtattttttt agaaattttt aattgttagg tattgatata 900
taaattgagt ttgttgttgt tttagagaga aaaatagata tatttatttt tttttaatta 960
ggatatggta tttgtttttt ttttaagttt tttatttttg aggtaggaaa gtattggtgt 1020
gtgtttttat ttttattttg tttttttatt agtaggttta atgggtagga attatgtttg 1080
ttttgttttt tgttggattt ttgggtttag tagagtgttt tgatatatag taggtatttg 1140
atatagatgt tggttttatg atgaagtaat tgtttttgaa ttaatataaa agggaagatt 1200
tagatttagt gagttggtat tatttgttta gggatatata gttaggaagg aattgaatta 1260
ggattttaat ttaggattat ttaagtttga attttaattg aatataagtt tttagtgatt 1320
ttggatggat ttattaaatt gttttgaatt ttggtttttt tagttgtaaa atagggttag 1380
gaaaagtgga tttggtgttt ttttgtgttg tatttttgta gtgggatttg tgaaagtgaa 1440
tttgagtttt atattgtaga agttttgagg ttttgtttta attagaattt agtatttagg 1500
ataagttttt tagtgatttt agattttttt gtttttgatt ttgtttgttt ttgagaggtg 1560
tggttaaggt ggggttagaa ggtggatgtg tgtatggagt ggtaaagtgt aggtgtaaag 1620
ttttggtgat gttagagagg tgtgttttta gtttggagtt gatttgtaga tgtgttttgt 1680
tttttggtgt tgttttggat tggtgtaggt gtaagttggt aagatggtgg tggttggggt 1740
tttttgtttg aggtgggtgg tattggtttt gttgttgtgg agtttttgtt tgtttgtttg 1800

gagttgttgg ttttggtttg attttattat aagaaggtag ggataaaaga gggatgtgtg 1860
gaatgttgat ttagtggagg tttgggttgg aggggtggtt gtggggtttt gtgtagttag 1920
gattgggagt tgtttttttt tatgtttttg ttttgatttg tttttttttg ttgtgtagtg 1980
aggtttattg taattgataa ataatagtta ttgtttattg ggtggtgatt tttagggggt 2040
tttgttgttg gttgtgattt tgtgtgtttt aattttaaat ttgttaatag tttaggaggt 2100
agggttttgt tgggatttgt ttttttgagt ttagggatag atatattttt ggagtgggtt 2160
ttggttttag ttattttgtt gttttggtta gatgattttg ggttagttat tgtgtttttt 2220
tgaatttgtt tttttaggtg taaatggggg gtttttagtt gttttttata aaggatattg 2280
tgtgtggagt tttggtatgg tttttggtat ataggtttta gtatgaggag attgtttttg 2340
ttattgtttt aggagtgtat aggggagtta ggtttgtaat taatatgtgt aatgtttttg 2400
ttttgtttgg ggggtttttt ggtggggaag gagggataga tttatagttt gtagtttttt 2460
gttgtttggt ggtataaagt tttttagatg tgagattttt a 2501

<210> 213
<211> 2501
<212> DNA
<213> Artificial Sequence

<220>
<223> chemically treated genomic DNA (Homo sapiens)

<400> 213

tgggagtttt atgtttgggg gattttgtgt tattagatag taaggggttg tagattatga 60
atttattttt ttttttttgt taaaaaattt tttaaataaa ataaaaatat tatgtatgtt 120
ggttataagt ttgatttttt tatgtatttt taaagtagta atagaaatgg tttttttgtg 180
ttggggttta tgtgttagga attatgttag gattttatgt atagtatttt ttgtaaggga 240
tagttgagag ttttttattt atatttgggg aaataggttt agagaggtgt agtgattagt 300
ttaaggttat ttggttaggg tagtggagtg gttgaagttg gagtttgttt tgggggtgtg 360
tttgtttttg gatttagaaa gataagtttt aataggattt tgttttttgg gttgttggtg 420
aatttaaaat tgggatgtat gaagttgtgg ttagtggtgg ggttttttgg aagttgttgt 480
ttgatgagtg gtaattgttg tttattagtt gtagtgagtt ttattgtgta gtaagggaaa 540
gtgagttagg gtaaagatgt gggaggggat agtttttagt tttagttgtg tagaattttg 600
tggttgtttt tttagtttag gtttttgttg agttggtatt ttgtgtgttt tttttttgtt 660
tttatttttt tgtgatagag ttgggttggg gttgatagtt ttgggtgggt aggtgggggt 720
tttgtagtag tagagttgat gttgtttgtt ttagatggaa agttttagtt gttgttattt 780
tgttggtttg tgtttgtgtt agtttagagt gatgttgagg ggtggggtgt gtttgtgagt 840
tggttttgag ttgggagtgt gtttttttga tgttattgaa gttttgtgtt tgtgttttat 900
tgttttgtgt atgtgtttgt tttttggttt tgttttgatt atgttttttg ggggtgggtg 960
gggttgggag tgggaaggtt tgaagttgtt ggagggtttg ttttgggtgt tggattttgg 1020
ttgagatagg attttagggt ttttgtagtg tggggtttaa gtttgttttt ataaattttg 1080
ttgtgggaat gtggtgtaga ggagtattga gtttattttt tttaatttta ttttgtagtt 1140
aaggaagttg agatttaggg tagtttagta agtttgttta aggttgttaa ggatttatgt 1200
ttaattagaa tttgaatttg ggtaattttg ggttagaatt ttggtttggt ttttttttgg 1260
ttgtgtgttt ttagatgaat ggtattggtt tgttgagttt gaattttttt ttttgtatta 1320
atttgaggat aattgtttta ttatgaagtt agtatttgtg ttgagtattt attatgtgtt 1380
aaggtgtttt attagatttg gggatttaat ggaaagtaaa atagatataa tttttgttta 1440
ttaagtttgt taatgaggga gtaaggtaga gatagagata tatattaata tttttttatt 1500
ttaaaaatgg gaagtttaag aaggaaatga atattatgtt ttgattgaaa aaaaatgaat 1560
atgtttattt ttttttttaa aataatagta aatttaattt atatgttagt gtttagtaat 1620
taagaatttt taaagaaatg tgtattaatg taatttattg ttggtttgta gtatgtgatt 1680
ttataaaatt ggaaaattta tgtgtattta atgtttatta ggtattagat gtttatataa 1740
aggggaggaa tttgggaaat ggattttttt tttgaatggt gaaattgatt tttttttgag 1800
gatttgtagt tttttttgtg tttgaggtgt agttgttggg gagagtaaat ttaagtgtta 1860
ggttatatta ggtgataagt aaggtattta attgaagaat tattttgatt tttttaagat 1920
ggtgttaaat tttttattat agatattatt tttgttttat gatttttttt atggttttta 1980
ttttttgttt aaagatttaa tttttgtttg aagttgtgat ggaatgtatg ttattataat 2040
gagtttttat atttggtggg agtttttata gggtataaaa taatgtgaat ttagtgtatg 2100
tggagatttt tttgttattt aagtttttag tttttttttt aatggtaaga aaatgggttt 2160
ttgataaggt gtgtgttgtg ggtagttttt gtttagtggg gttttggtgg gttgtgggaa 2220
gatttgagag ttattagaag tgtaagatgg tgattgtggt tgaaattttg gttttagaat 2280
ttgaggttga gtttaaggtt gggtttaagg ttgatggaga ggaggatgag gttaaggtgg 2340
ttaggataag gaggaaggtg ttattgtggg ttgtggttgt tgtgatatat aagattatgg 2400
ggttagtgtg ggattagtag gaggaaggtg tgagtgagag tgatggggat gagtatgtta 2460

tggtttttt tgtggagagt tttttgggg agtatgagtg g 2501

<210> 214
<211> 2500
<212> DNA
<213> Artificial Sequence

<220>
<223> chemically treated genomic DNA (Homo sapiens)

<400> 214

ttgtgagaaa tattttgttt aattatttgt ttgtaaggtg gataaaggtt taattgaaga 60
aatattttta ttatattttg tttggtagtg ttttaaggaa tattataatg atattttatt 120
agaaaaagga ttaaattatt tgattataag aatattttat taatattttg ttgggtagta 180
agttatattg tttaggtttt ttttattttt atttataagt attttagttt gtaagtggtg 240

gtgggttttg gtattaagtg ggttttttat ttttataatg gtttgtaata tttttgtgtt 300
gttgtttgag ttgtttttgt tttgtgtgtt ttttttttat ttttgttttt attttagaat 360
ttaataattt tattgtttta tttattttgt tggatttata ggggatgttt ttttttattt 420
tgttggtatt ggattgtttg tttattgtat atattggttt tttaatgttt gtggtaggtt 480
tttgtaggtt ttgaattttt gtagtggttt ggagtttttg ttttttttgt atatattttt 540
ttggtgataa ggtaaagtta tatttttgag ttttgtttga ttatataggt agtgttttag 600
ttaatggtag ttttagggtg gttttatatt ataaagtttt attgttgatt attttttggg 660
tttgtgggtg aggggtgata ggggtgtaga gtaaattaaa tgttgttgtt gttttagtat 720
tttttgaaga tgtattttaa attgatttgt tgttgttttt tattttttta tgtttaatgt 780
ttatggttta tatggaagtt agaggatgat ttttttaagt ttttttttat agttattttt 840
attaagtgat ttattttttt gttttttagt ttttattatg atttagtatt tttgatgttt 900
tattttaaat tttttatgtt agtgtggtta tatttattta ttggtggaga tgtgaattat 960
tttatttttt ttttatagtt tttttatatg tattttgaag attatttatt tttgggttat 1020
tgttaggtaa attttaattt atgttttttt atttaatgtt tatgtagttt aagtttttta 1080
tttttaattt gaaataatgg tttttagttt gttaaaattt tagtgaataa atatttttta 1140
ttgtgtattt tagttttgtt ttaaattagg ggtgtatatt tttttttttt tttgaaatgg 1200
agttttattg ttgttgttta ggttggagtg tagtggtatg attttagttt attgtaattt 1260
ttgttttttg ggtttaagtg attttttggt ttagtttttt gagtagttgg gattataggt 1320
ttgtgttatt atgtttggtt aatttttgta tttttaggag agatggggtt ttattatttt 1380
ggttaagttg gttttgaatt tttgattttg tgatttattt gttttggttt ttaaagtgtt 1440
gggattatag gtgtgagtta ttatatttag ttaggatggt tattattaaa attattattg 1500
ataagtaaat tttggttatt tttgtagtat ataataattt aatataaagt ttataattaa 1560
taatatatag aagttatttt agaattataa gagtttttta taattttgga gtatatatta 1620
ataatatttt tataaatata gtttaaagaa agttaaatat tattttatat ttgatgatgt 1680
tttttgtatg atttttatat taaataagat taatatgttt tttttggatt ttagaggatt 1740
tatattaaaa aattaatgag gaattaagtt agaatttgat tttggaaagt ttgttaaaga 1800
taaaaagttt aaaatgtttg atattatgaa atagggttat gggttattgt aaaataagtt 1860
atttatttaa ttaaagtagt aatttaaaga ttttaaaaaa gaaaaaaaaa aggtgaaaat 1920
ttttattttt tgagagagga ggtttaatat tttaaataat aagttttaat taaaaatagg 1980
atgtggtaaa tgaaatttgt ttttttgaaa ttttataata aaagtttaat tattttgagt 2040
ataaaatata atttttataa attttgtaat ttttattatt ttgtattaag gagtgagtta 2100
atgttttaag aaagttttgt taaattgata tagtaattta gatattggtt ttgttttagt 2160
gtgtttttga tattaatggt taatttttag agaaattgag ttattttttt tttaatatta 2220
gtttttataa ttttatatgt ttatttgttt tatgatagtt tttgggtttt gaggagttga 2280
atagttttaa tttttgattt tgtattttat gaatgtagtt tattttgatt agtatttttt 2340
attgtgtttg aagatgaggt tgtaattgat gttagtgttt aagatatagt agaatttggt 2400
gtttttttta gatttagaag ttaaagtttt atagtttaat ggtataagga ttttaaaagt 2460
atatatagaa agttatatag atggaagaaa atattttttt 2500

<210> 215
<211> 2500
<212> DNA
<213> Artificial Sequence

<220>
<223> chemically treated genomic DNA (Homo sapiens)

<400> 215

aaaaaagtgt ttttttttat ttgtgtaatt ttttgtatgt gtttttaaag tttttgtgtt 60
attgagttat agggttttga tttttgggtt taaaaaggat attaagtttt gttatatttt 120
aaatattgat attaattata gttttatttt taggtatagt agaagatgtt aattaaagta 180
aattgtgttt atgagatgta gggttagaaa ttaaaattat ttaatttttt aaggtttaag 240
gattattgtg gaataggtgg gtatgtgaga ttgtaagggt tgatattaag agaaaagtag 300
tttagttttt ttaggaatta attattaata ttaaaggtat attaaagtaa aattagtatt 360
taggttgttg tgttagttta ataaggtttt tttggagtat taatttattt tttaatgtaa 420
aatgataaag gttataaggt ttatagaaat tatattttat atttaagatg attaaatttt 480
tattataaaa ttttaaaaaa ataaatttta tttgttgtat tttgttttta attagggttt 540
attgtttggg atattaagtt ttttttttta aagaataaag atttttattt tttttttttt 600
ttttttgaaa tttttgagtt attgttttgg ttaaatgaat gatttatttt ataatgattt 660
atgattttat tttgtgatat taagtatttt aaatttttta tttttgatga attttttaaa 720
gttaaatttt aatttgattt tttattaatt ttttgatatg agttttttga agtttaaaag 780
agatatattg gttttatttg gtataaaaat tatataagaa gtattgttaa atataaaatg 840
gtgtttgatt ttttttgggt tgtatttata gaaatgttat tgatatgtgt tttaaaatta 900
tgggaaattt ttataatttt gaaataattt ttgtatgtta ttaattataa attttatgtt 960
aaattgttgt atgttataga agtaattaaa atttatttgt taatggtggt tttaataatg 1020
gttgttttgg ttgggtgtgg tggtttatgt ttgtaatttt agtattttgg aggttaaggt 1080
gggtggatta tgaggttagg agtttaagat tagtttggtt aagatggtga gattttgttt 1140
tttttaaaaa tataaaaatt agttgggtgt ggtggtatag gtttgtaatt ttagttattt 1200
aggaagttga gttagagaat tatttaaatt taggaggtag aggttgtagt gagttgagat 1260
tatgttattg tattttagtt tgggtaataa tagtgaaatt ttattttaaa aagagaaaaa 1320
aatatatatt tttgatttga aataagatta ggatatatgg taaagaatgt ttatttatta 1380
aagttttgat agattgaagg ttattatttt agattggaaa taggaaattt gaattatatg 1440
gatattgggt aaaagaatat gggttgaaat ttgtttggta gtagtttaaa agtaaatggt 1500
ttttgaggtg tatgtgaagg aattgtagga gggaaatggg ataatttata tttttgttaa 1560
taaataaatg tagttatatt agtgtggggg atttgaggtg agatattgaa aatattaagt 1620
tatggtgagg gttggaagat aaaagaatga attatttaat aggaatggtt gtggggaagg 1680
gtttgaagga attatttttt gatttttatg tgaattgtga atattaaata tggagaaatg 1740
aggagtggtg gtagattggt ttgggatgta ttttaggggg atgttgaaat aataatagta 1800
tttggtttgt tttatatttt tgttattttt tgtttgtaag tttagggagt ggttagtagt 1860
ggggttttgt gatgtgaagt tattttaggg ttgttattgg ttgggatatt gtttgtatga 1920
ttaaataaag tttaagggtg tggttttgtt ttgttattag gagggtatat atagggaggg 1980
taagagtttt gggttattgt gaagatttaa aatttataaa agtttgttgt agatattgaa 2040
gaattaatat atataatgga taaataattt agtgttggtg gggtgaagag gagtgttttt 2100
tgtgaattta atgaggtgaa tgagatggta agattgttag gttttgaagt gaaggtgaga 2160
gtgaaagaaa gatatataga gtgggggtgg tttaaataat aatataggaa tattgtagat 2220
tattgtggaa gtggggggtt tgtttaatgt tagagtttat tgttgtttat aggttggggt 2280
gtttgtaggt aggggtggga ggggtttggg tagtatggtt tgttgtttgg tgggatattg 2340
ataagatgtt tttatgatta ggtggtttgg tttttttttt ggtggaatat tattgtggtg 2400
ttttttagaa tgttgttaag taagatatga tagggatgtt tttttagttg ggtttttgtt 2460
tgttttgtgg ataggtggtt aggtaggatg ttttttatgg 2500

<210> 216
<211> 2501
<212> DNA
<213> Artificial Sequence

<220>
<223> chemically treated genomic DNA (Homo sapiens)

<400> 216

gatgttttta tatagattag tttgtttttt gatttttatt ttgttttatt aataaatgtt 60
tttatgtgta tttatttttt attgttgttg taataaattt tgaatagttt agatattgtt 120
aggtgtggtg gtttatgttt gtaattttag tattttggga ggttgagata agtggattgt 180
ttaaggagtt taagattagt ttggttaata aggtgaaatt ttatttttat taggggttgg 240
tgatgggtgt ttgaaattgt agttatttgg gagtttagaa tagtttttat taatattatt 300
tttttatata tattagggtt gagtgaaaaa ataaattgta tataaaaagt taattataga 360
ggttatttat ataaaatgtt aaaatatgta aaataagagt atatattttt atggataata 420
agtaaatggt aaagtataaa aatatgaatg agaataaaaa attattaaat tgagaagaat 480
ggttgtaagt ggagaaggag ggagggtagg gattgtatag aaagttttaa ggtgatttgt 540

tgatagtggt tttttttgtt tgtttgtttg tttgtttgtt tgtttgtttg tttttgatgg 600
agttttgttt ttgttgttta ggttggagtg taatagtata atattagttt attgtaattt 660
ttaatttttg ggtttaaatt attttgttgt ttatgttttt tgagtagttg gaattatagg 720
tgtttgattt atatttagtt atttttttta tttttggtag taataaggtt ttattatgtt 780
tgttaggtag gttttaaatt tttttatttt agatgattta tttgttttag ttttttaaag 840
tgtttagatt ataggtatga gttattatat ttagtgtgtt tatagtattt ttaatatttt 900
gaataaataa attagattta atatagttgt ggggtaatgt tgagatttga ttggatttaa 960
tagtatttta tatatttttt tatgtgtttg aaatatttta tttttaaagg atatgttgtt 1020
tttgttaagt attgttaatg aattaaagga tagttaagaa aatgttataa gtgaaaatat 1080
aaaaaaagaa aatattaaaa gtaaagattt gtaaaaaaaa tttagagttt ttataattaa 1140
tagtatgtaa gtattggata agtattttat gagagaggat aatgaatata ttatgtaata 1200
aagatggatg tttttttgta ttttattaaa attaaatagt ttttttattt ttaaataatt 1260
ttaatttttt gtgatgagtt tggaagggat tttgaaagag tgatttttga tttaaaatat 1320
agagagtttt tttatttgtt agtgagtaga gataatatag agtgaaaaaa agataagttt 1380
ttgtgaagag atttttgggt attttaggat tattaatggg atatatgtat ttatttgttt 1440
ttttgttttg ataatataat tataagataa ggttagaaag tttaaattgt ttttaataga 1500
tttattattt gttaattaaa taggttagat tttataattt tttataatta ttataaggga 1560
tttgaaaagt tagtgtttga gtatttgtta agtttttgat gttaaaggat tgtttttata 1620
ttgaaaatat tttagagtta ttggattaaa ttatttatgg tttattatat atttaatagt 1680
ttaatttata tattataaga tttatttatt tgaagtgtat aatgattttt agttgttgta 1740
tattttgagt ggatatagtt tagattttta attaattaat ttaattattt gggaaaaagt 1800
aaaagatatg taatggaata agatgagatt gtgatggggt ttagttttta tgtgataaat 1860
tatgagatgg aaaattttga attgaagtta tagataaatg aattaattat gattaaatat 1920
aatttagaag taaattttaa ataatttttt aataattagt ggatttataa tttttgttgt 1980
agaatgtttt tatgtaatag aagttttttt agagtttgga aatttttatt aataaagaaa 2040
aattttgatg tatttttttt tagttgaata tttttagatg atttttggta ggttttatag 2100
aatttttttg aaggtgagtg ttttttaaat ttaaaggatt agagaatttt tgttttttta 2160
tggatatgaa tattggtaag agtggaagaa tattaaaaat atttttattg tttttttttt 2220
atgtttatta taatatttga tgtagtatta atggtttgat aatgttaata gttgtgattt 2280
ttaatatttt ttttgttttt atagagatgt tatatatttt aaatagttta tatggattaa 2340
tttatttaat ttttaagaat atttttatga tgatgttttt attattattt ttaaataata 2400
atgagttata tattttagtt attatttata taaaagttat gtgagttggt gtaaattttt 2460
taagtttttt gagatttaga tttttggtta atgaaatggt a 2501

<210> 217
<211> 2501
<212> DNA
<213> Artificial Sequence

<220>
<223> chemically treated genomic DNA (Homo sapiens)

<400> 217

tgttatttta ttgattagaa gtttggattt tagagaattt agaagatttg tgttaattta 60
tatggttttt atatggatga taattgaagt gtgtgattta ttattatttg gagatagtaa 120
tagaaatgtt gttgtagagg tatttttaag gattaaataa attaatttat gtaaattgtt 180
tagaatatat ggtattttta tgaaaataaa agaaatatta aggattataa ttgttagtat 240
tattaagtta ttgatgttat attaggtgtt gtgatagata tggggaggag gtagtgaggg 300
tatttttgat atttttttat ttttattagt gtttatattt gtggagggat aaaggttttt 360
tggtttttta gatttgagag atatttattt ttgggaagat tttatggagt ttgttgaaag 420
ttatttgagg atgtttaatt gaaagagaat atattagaat ttttttttgt tggtgaagat 480
ttttaaattt tagagagatt tttgttgtat gagggtattt tgtagtagag gttatgagtt 540
tattgattgt tgaggagtta tttgagattt gtttttaaat tatgtttagt tatggttggt 600
ttatttattt gtggttttaa tttagaattt tttattttat ggtttattat atggggatta 660
aattttatta tagttttatt ttattttatt atatattttt tatttttttt taaataatta 720
aattgattgg ttgggaattt gaattgtatt tatttaagat gtgtaataat tgaaaattat 780
tgtatatttt aaatgggtga attttatggt atgtgaatta agttgttaaa tgtgtgatga 840
attatggatg atttggttta gtggttttga aatattttta gtataaaaat agttttttaa 900
tgttaaaaat ttggtagata tttaagtatt ggttttttag gttttttata gtgattgtgg 960
gagattgtag aatttggttt gtttagttgg tgagtaatag gtttattgga gatagtttgg 1020
attttttgat tttgttttat aattgtgttg ttagagtaga agagtaagta aatatatatg 1080
ttttattaat agttttgaaa tgtttaaaaa tttttttgta agaatttgtt ttttttttat 1140
tttatgttat ttttgtttat tgataagtgg gaaagttttt tgtgttttgg attagggatt 1200

atttttttaa aatttttttt aagtttatta tggagaattg gggttatttg ggaatgagaa 1260
gattatttgg ttttgataaa atatagagaa atatttattt ttattatata atgtgtttat 1320
tatttttttt tataagatat ttatttaata tttatatgtt gttaattata gaaattttgg 1380
attttttttg tagatttttg tttttaatat tttttttttt tatattttta tttgtaatat 1440
ttttttaatt gttttttgat ttattaatag tgtttagtaa gaataatatg ttttttaaaa 1500
atgaaatatt ttaaatatgt ggaaaagtat gtggaatatt attgagttta gttggatttt 1560
aatattattt tatagttatg ttagatttga tttatttatt tagagtatta gaaatattat 1620
aaatgtgttg ggtgtggtag tttatgtttg taatttgagt attttgggag gttgaggtag 1680
gtggattatt tgagatgagg aagtttgaga tttgtttggt aaatatggtg aaattttgtt 1740
gttattaaaa ataaaaaaaa tagttgggtg tgggttgggt gtttgtaatt ttagttattt 1800
gggaggtgtg ggtagtagaa tgatttgaat ttgggagttg aaggttgtag tgagttgata 1860
ttatgttatt gtattttagt ttgggtgata agagtgaaat tttattaaaa ataaataaat 1920
aaataaataa ataaataaat aaataaagaa aattattatt aataagttat tttgaagttt 1980
tttgtgtaat ttttgttttt tttttttttt tatttatagt tatttttttt aatttgatgg 2040
ttttttattt ttatttatgt ttttatattt tattatttat ttattattta taaaaatata 2100
tatttttgtt ttgtatgttt tagtatttta tatgaatggt ttttgtagtt aattttttgt 2160
gtgtaatttg tttttttatt tagttttggt gtgtatgagg gaataatgtt agtgggaatt 2220
gttttgaatt tttaaatagt tgtgatttta ggtgtttatt attaattttt agtagagatg 2280
gggttttatt ttgttggtta ggttggtttt gaattttttg agtgatttat ttgttttggt 2340
tttttaaagt gttggaatta taggtgtgag ttattgtgtt tagtagtgtt taagttattt 2400
aaaatttgtt atagtagtaa taaaaaatga atatgtatag aaatatttat tagtgaaata 2460
aaatagaagt taagaaatag attaatttat atgaaaatat t 2501

<210> 218
<211> 2501
<212> DNA
<213> Artificial Sequence

<220>
<223> chemically treated genomic DNA (Homo sapiens)

<400> 218

ggattatatt aggattgata tttttatgat taatagttag ttatttatta gttttggtta 60
attttatttg tattttatta ataatgaata gaattaagtt gggagtatga gatgggatga 120
gggtaagtaa aaatattata aagtttattg tttttatgga gatttagttg gttttttttt 180
tgtttttttt tttttttttt tttttttttt tagttttgtt ttgttgttta agttggagtg 240
tagtggtgtg attttagttt aatgtaattt ttattttttg ggtttaaatt attgagtgag 300
tttaggaggt taagattaat ttgggaaata tagtaaaagg tagggtggtg tatgtttgta 360
gttttaaggt tgaggtggga gaattatttg agttttagag gtagagttta gtttggataa 420
tatagtgaaa ttgtttttaa tagaaaaatt aaaaatatta gtgggggtgg ggtggtgtga 480
gtttatagtt ttgaggttga ggtgggagga ttgtttgagt ttaggaggtt gaggttagtt 540
tggttaatat agtgaaattt tatttttgtt aataataaga ataatagaaa aatagtgtgg 600
gtggggtggt ttatttttgt agttttgagg ttaaggtggg agtattgttt gagtttagga 660
gtttgatatt agtttggtta ataaagtgaa agtttgtttt ttttaaaaaa taaataaata 720
aataaataaa taaaaatgag tggggtagaa tggtatatgt ttatagtttt gaggttgagg 780
tggaagtatt atttgagttt gagaggttga ggttagtttg ggtaatatat tgaaatttgg 840
tttttattaa aatgaaataa aataaaataa aaaatagttt gggtagggtg gtgtatggtt 900
gtagttttga gatgggagga ttttttgagt ttaggagttt gatgttggtt tggttaatat 960
agggaaatat ggtttttatt aaaaaaaaaa aaaaaaaaaa aagtgtgggt agggtggtgt 1020
atgtttgttg ttttgaggtt atggtggaag gattttttga gtttaggatg atgagattag 1080
tttggttaat atagtaaaat ttggttttta tttaaaaaaa aaaaaaaaaa aaagtgtggg 1140
ttgggtggtg aatgtttgta gttttgaggt tgaggtggga ggattgtttg agtttatgat 1200
gttaagatta gtttggttaa tatagtgaaa ttttttttat taaaaaatta aaaaataaaa 1260
aatattagtg ggggtagggt ggtttgtgtt tgtagtttta agatggaggt tggaggattg 1320
gttaaggtag atgtaattaa tattataatt atattttata agtaaatata tttttttttt 1380
tttagggtta taggtaaagg atggtaattg tgtgtattat atttaagatt tttttttaaa 1440
aatgtaagta gaggttgtgg ggttttggat tgttttttta gtggtaatag atatagaagt 1500
tattgaagaa tgaaagttat gattaagtat agtaaatttt tgtaaatgtg ttagtttgga 1560
aaatattgtg ttttttaagt agaaaaatta tagattgatt attttttttt ttttatagtt 1620
tagattagaa tttagatttt taatttaaga tttaggaatg gtttttagag agtttaaaat 1680
ttgatggtgt ttgaggatta tttatttttt tgtagtggta ataaagtgtg gttggaggag 1740
gagatattat tttgttatgt tattgtttaa ggatgatgga ttaattaggg tagttagtga 1800
attttatttg gttaattaga agttagaata gtaggtggaa taagtgaagt ggatgtggtt 1860

tttattagtt ttggttttag ggatggaatt tttttaaagt gggggtggag attttaattt 1920
ttttgtttaa agtatttttt gggattggtt attttaagtt tagagtatgt atgttttgat 1980
tttttttttt ttttgatttt tttatattta gagtatgtat ggtttgattt tttttttgga 2040
tttttttaaa attagagtaa gtatatgttg attttttttt tttatttttt ttattttttt 2100
ttttttttgt ggtgtatttg ttatttagtt ttaataagga gtgtatatga ggtaggttgt 2160
tattttgaat tttttttgtt agtttttaat ttttttaggt atgggatttt ttttaggaat 2220
tttgtagtaa tttaagaaat ttgggttggg tgtggtggtt tatatttgta atttttgtat 2280
ttttggaagt tatagttggt ggattgtttg aatttaagag gtggaggttg tagtgagtta 2340
tgattatgtt agtgtatttt tgattgggta atagagtgag attttgtttg aaaaaataaa 2400
aattaaaaaa gtttatttaa attttttttt ttgggtttaa gtgagttttt tgtttaggtt 2460
ttggggttat aggtgtgtat tattttgttt ttgttaaatt t 2501

<210> 219
<211> 2501
<212> DNA
<213> Artificial Sequence

<220>
<223> chemically treated genomic DNA (Homo sapiens)

<400> 219

aaatttagta gaagtggggt ggtgtgtgtt tgtagtttta aggtttaggt gagaggttta 60
tttgagttta ggaggaaaga tttgagtgag tttttttggt ttttgttttt ttagataggg 120
ttttgttttg ttgtttagtt gggagtgtat tggtgtgatt atggtttatt gtaatttttg 180
ttttttaggt ttaggtgatt tattagttgt ggtttttaaa agtgtaggga ttataagtgt 240
aagttattat gtttggttta aattttttaa gttattatag agtttttagg aaaaattttg 300
tatttgaaaa agttagaaat tgataggaaa gatttgagat ggtggtttgt tttatatata 360
ttttttatta aaattagata gtaaatgtat tgtggaggag gggaggggta ggaagaatgg 420
aaaaagaaaa ttggtgtatg tttattttga ttttggaaga atttaaagag aaaattagat 480
tgtgtgtatt ttgagtatgg aagaattgaa agagagagaa agttagagta tgtgtatttt 540
gaatttaaag tagttaattt taggggatgt tttaggtggg aaaattagag tttttatttt 600
tattttgaga aggttttgtt tttggagttg ggattgatag aagttatatt tgttttgttt 660
gttttgttta ttgttttgat ttttaattgg ttagatggag tttattaatt gttttgattg 720
gtttattatt tttgggtagt gatatagtag aataatgttt ttttttttag ttatattttg 780
ttgttattgt gaaaaagtgg gtggttttta ggtgttgtta gattttgaat tttttgaaga 840
ttgtttttgg attttgggtt aaaaatttgg atttagtttt gaattgtggg aagaaaaaaa 900
tagttaattt gtgatttttt tatttgaaag atatgatgtt ttttaaatta gtatatttgt 960
ggaggtttgt tatatttagt tgtggttttt attttttagt ggtttttatg tttgttgtta 1020
ttgaaaaaat agtttaaggt tttgtaattt ttgtttatat ttttgaaagg gaattttaag 1080
tatggtgtgt ataattatta ttttttattt gtagttttga agagaggaaa atgtatttat 1140
ttatgggatg tgattgtggt attggttata tttgttttgg ttaatttttt agtttttgtt 1200
ttgggattat aggtgtgaat tattttattt ttattaatat tttttatttt ttgatttttt 1260
aatagagaag gttttgttgt gttggttagg ttggttttga tgttgtgggt ttaagtgatt 1320
tttttatttt ggttttggga ttataggtat ttattattta gtttatgttt tttttttttt 1380
ttttttttaa gtagaaatta ggttttgtta tgttggttag gttggtttta ttgttttggg 1440
tttaagggat ttttttgtta tggttttgag atgataggta tgtattattt tgtttatgtt 1500
tttttttttt tttttttttt tagtagaaat tgtgtttttt tatgttggtt aggttggtat 1560
taagtttttg gatttaaggg atttttttat tttaggatta tagttatgta ttattttgtt 1620
taagttattt tttgttttgt tttgttttgt tttagtggaa attgggtttt gatatgttgt 1680
ttaggttggt tttaattttt taggtttaga tgatgttttt attttggttt tagaattata 1740
ggtgtgtgtt attttatttt gtttattttt atttatttat ttatttattt attttttagg 1800
agaggtaggt ttttgttttg ttggttaggt tggtattaaa tttttgggtt taagtaatgt 1860
ttttattttg gttttaaaat tataggagtg agttattttg tttatgttat ttttttgttg 1920
tttttgttgt tagtagaaat ggggttttgt tatgttggtt aggttggttt tgatttttta 1980
ggtttaagta atttttttgt tttggttttg ggattatggg tttatattat tttgttttta 2040
ttaatatttt taattttttt attagagata gttttgttat gttgtttagg ttgggtttta 2100
tttttgggat ttaagtgatt tttttgtttt ggttttggga ttataggtat gtattatttt 2160
gttttttgtt atgtttttta ggttggtttt gattttttgg gtttatttaa tgatttgaat 2220
ttgggaagtg gaggttgtat tgagttgaga ttatattatt gtattttagt ttgggtgata 2280
gagtaagatt gaaaaagaaa gaaagaaaga aagaaagagt gagagagaga ttagttgaat 2340
ttttgtaaga atagtgagtt ttgtggtatt tttatttgtt tttgttttat tttatgtttt 2400
tagtttggtt ttgtttattg ttgatgaaat atagatagga ttggttagaa ttggtagatg 2460
gttggttgtt gattatgaaa atgttagttt tgatatagtt t 2501

<210> 220
<211> 2501
<212> DNA
<213> Artificial Sequence

<220>
<223> chemically treated genomic DNA (Homo sapiens)

<400> 220
   aaatagtttg agttttttta gttttaggat ttttggtgtt ttgggtgggg tggtgtatgt 60
   ttgtagtttt ggggttgagg tgagaggatt gtttgagttt aggagttttt tttttttttt 120
   tttttttttg agatagggtt ttgttttgtt gtttaggttg tgttgtagtg gtgtgattgt 180
   ggtttattgt aatttttgtt ttttgggttt aagtgattta ttatttgtgg gtttttaaag 240
   tgttgggatt ataagtgtga gttattatgt ttagtttaga ttttttaagt tattatagag 300
   tttttaggaa aaattttata tttgaaaaag ttagaaattg ataggaagaa tttgagatgg 360
   tgatttgttt tatataaata tattattaaa attagataag aagtgtatta tggtggaggg 420
   gaggggtagg aagaatggaa agagaaaatt agtgtatgtt tattttgatt gtggaagaat 480
   taaaagggta aattggtgtg tgtgtatttt gaatttaaag tagttaattt aaggggatgt 540
   ttttggtggg aaaattagag tttttttttt tttttgtttt gggaaggttt tgtttttaga 600
   gtttggattg atagatgtta tattagtttt ttttgttttg tttattgttt tgatttttaa 660
   ttggttagat ggagtttatt aattgttttg attggtttat tatttttggg tagtgatatt 720
   gtagaatgtt gttgtttttt ttagttatat tttgtgtgat aaagtgggtg gtttttaggt 780
   gttattagga gattttgaat tttttgaaga ttgtttttgg attttgggtt aaatatttgg 840
   atttagtttt gaattgtggg aagaaaaaat agttgatttg tgattttttt atttgaaaga 900
   tataatgttt tttaaattag tatatttgtg gaggtttgtt gtatttagtt gtggagttta 960
   ttttttagtg gtttttatat ttgttggaat taaaaaaata gtttaaggtt ttttaatttt 1020
   ttttattttt attttttttt tttttttttt tttttttgta gttttttttt tttttttttt 1080
   tattattata ttttaagttt tagggtatat gtgtatattg tgtaggttag ttatatatgt 1140
   atatatgtgt tatgttggtg tgttgtattt attaatttgt tatttagtat tgggtatatt 1200
   ttttaatgtt attttttttt ttttttttta ttttataata gtttttagag tgtgatgttt 1260
   ttttttttgt gtttatgtga ttttattgtt taatttttat ttatgagtga gaatatttgg 1320
   tgtttggttt tttgtttttg tgatagttta ttgagaatga tgatttttaa ttttatttat 1380
   gtttttataa aggatatgaa tttattaatt tttatggttg tatagtattt tatggtgtat 1440
   atgtgttata tttttttaat ttagtttatt attgttggat atttgggttg gttttaagtt 1500
   tttgttattg tgaatagtgt tgtatttttg gaagggaatg taagtgtggt gtgtataatt 1560
   attatttttt atttgtagtt ttggagagag gaaaatgtat ttatttatgg gatgtgattg 1620
   tggtattggt tatatttgtt ttggttaatt tttttatttt atttttggga ttataggtat 1680
   gtattatttt gtttaggttt gtttttgtta ttattattat tattttttag ttgaaattgg 1740
   gtttttttat gtaggttagg ttggttttaa tttttagggt ttaagggatt tttttgtttt 1800
   tttagtttgg ggattatagg tatgtattat tttgtttatg gtattttttt tgttttttag 1860
   tagaaattgg gttttgttat gttgtataga ttggttttaa ttttttgggt ttaagtaatg 1920
   tttttatttt agttttggga ttataggtgt gtgttatttt gttttgataa ttttttttag 1980
   gagagatagg tttttgttat gttgattagg ttggtattga ttttttgggt ttaagtaatt 2040
   tttttatttt agtttgggaa ttataggggt gagttatttt gtttatgtta tttttggttg 2100
   ttttttttat tagtagaaat ggggttttat tatgttggtt aggttggttt tgattttttg 2160
   ggtttaagta atttttttgt tttagttttg ggattattgg tatatattat ttagttttta 2220
   ttaatatttt taattttttt agtagagata gttttgttat gttgtttagg ttggttttta 2280
   ttttttgggt ttaagtgatt tttttgtttt ggttttggga ttgtaggttt gtattatttt 2340
   gttttttgtt atgtttttta ggttggtttt aattttttgg gtttatttaa tgatttgaat 2400
   ttgggaaggg gaggttgtat tgagttgaga ttttattatt gtattttagt ttgggtgata 2460
   gagaaaaatt gttgaaaaga aaaggaaggg aagggagaag g 2501

<210> 221
<211> 2501
<212> DNA
<213> Artificial Sequence

<220>
<223> chemically treated genomic DNA (Homo sapiens)

<400> 221

tttttttttt tttttttttt ttttttttga tagttttttt ttgttattta ggttggagtg 60
tagtggtggg attttagttt aatgtaattt tttttttttg ggtttaaatt attgagtgaa 120
tttaggaggt tgagattagt ttgggaaata tagtaaaagg tagggtggtg taggtttgta 180
gttttgaggt tgaggtagga ggattatttg aatttaggag gtagagatta gtttggataa 240
tatagtgaaa ttgtttttat tagaaaaatt aaagatatta gtgggggttg ggtggtgtgt 300
gttagtggtt ttgaggttga ggtaggagga ttgtttgagt ttaggaggtt aaggttagtt 360
tggttaatat agtgaaattt tatttttatt aataagaaga ataattaaaa atagtatggg 420
tggggtggtt tatttttgta gtttttaggt tgaggtggga ggattgtttg agtttagaag 480
gttgatatta gtttggttaa tatagtgaaa gtttgttttt tttaaaaaaa attattaggg 540
tagaatggta tatgtttatg gttttgaggt tgaggtggaa gtattgtttg agtttaggag 600
gttgaggtta gtttgtgtaa tatagtaaaa tttggttttt attgaaaaat aaaaaaaata 660
ttgtgggtag ggtggtgtat gtttgtggtt tttaggttga ggaggtggga ggattttttg 720
agttttggag gttgaggtta gtttggttta tatagagaaa tttagtttta attaaaaaat 780
aataataata ataataaaaa taaatttggg tagggtggtg tatgtttgta gttttgagaa 840
tgagatggga ggattgatta aggtggatgt aattaatatt ataattatat tttataagta 900
aatatatttt ttttttttta gggttatagg taaaggatgg taattgtgtg tattatattt 960
atattttttt ttaaaaatgt ggtattattt ataatagtaa agatttggaa ttaatttaaa 1020
tgtttaataa tgatagattg gattaagaaa atgtggtata tatatattat ggaatattat 1080
gtagttataa aaattgatga gtttatgttt tttgtaggga tatggatgaa attggaaatt 1140
attattttta gtaaattatt gtaagaataa aaaattaaat attgaatatt tttatttata 1200
ggtgggaatt gaataatgag attatatgga tataggaagg ggaatattat attttgggga 1260
ttgttgtggg gtggggggag gggggaggga tagtattggg agatatattt aatgttagat 1320
gatgagttag tgggtgtagt gtattagtgt ggtatatgta tatatatgta attaatttgt 1380
ataatgtgta tatgtatttt aaaatttaaa gtataataat aaaagaaaaa aaaaagaaaa 1440
ttataaaaaa aaaaaaaaaa gaaaaaaaaa taagaataaa agaggttgga gggttttgga 1500
ttgttttttt agttttaata gatgtagaag ttattgaaga atgaatttta tgattaagta 1560
tagtaaattt ttgtaaatgt gttagtttgg aaaatattgt gttttttaaa tagaaaaatt 1620
atagattgat tatttttttt ttttatggtt tagattagaa tttagatgtt taatttaaga 1680
tttagggatg gtttttagag agtttaaaat tttttgatgg tgtttgagga ttatttattt 1740
tgttgtataa agtgtggttg gaggaggtga taatattttg taatgttatt gtttaaggat 1800
gatggattaa ttagggtagt tagtgaattt tatttggtta attagaagtt agaatagtag 1860
gtggaataag ggaagttgat gtggtgtttg ttagtttagg ttttagggat agaatttttt 1920
taaggtgggg gggaggggag atttgatttt ttttgttgaa agtgtttttt tggattggtt 1980
attttaaatt tagagtatgt atatgttgat ttgttttttt gattttttta taattagggt 2040
aagtatgtgt tgattttttt tttttatttt ttttattttt tttttttgtt gtggtgtatt 2100
ttttatttaa ttttaataat gtatttatgt gaggtaggtt gttattttaa attttttttg 2160
ttagttttta atttttttag gtatgggatt ttttttagga gttttgtagt aatttaaaaa 2220
atttgggttg ggtgtggtgg tttatgtttg taattttagt attttggagg tttataggtg 2280
gtggattgtt tgaatttggg aggtggaggt tgtagtgagt tatgattgtg ttattgtaat 2340
atagtttggg taatagagtg agattttgtt ttaaaaaaaa aaaaaaaaaa aaaaaatttt 2400
tgggtttaag tgattttttt gttttggttt tgggattata ggtgtgtatt attttgttta 2460
gagtattaaa ggttttgagg ttggaaagat ttaggttgtt t 2501

<210> 222
<211> 2501
<212> DNA
<213> Artificial Sequence

<220>
<223> chemically treated genomic DNA (Homo sapiens)

<400> 222
   tttttttttt tttttttttt ttttttttga tagttttttt ttgttattta ggttggagtg 60
   tagtggtggg attttagttt aatgtaattt tttttttttg ggtttaaatt attgagtgaa 120
   tttaggaggt tgagattagt ttgggaaata tagtaaaagg tagggtggtg taggtttgta 180
   gttttgaggt tgaggtagga ggattatttg aatttaggag gtagagatta gtttggataa 240
   tatagtgaaa ttgtttttat tagaaaaatt aaagatatta gtgggggttg ggtggtgtgt 300
   gttagtggtt ttgaggttga ggtaggagga ttgtttgagt ttaggaggtt aaggttagtt 360
   tggttaatat agtgaaattt tatttttatt aataagaaga ataattaaaa atagtatggg 420
   tggggtggtt tatttttgta gtttttaggt tgaggtggga ggattgtttg agtttagaag 480
   gttgatatta gtttggttaa tatagtgaaa gtttgttttt tttaaaaaaa attattaggg 540
   tagaatggta tatgtttatg gttttgaggt tgaggtggaa gtattgtttg agtttaggag 600
   gttgaggtta gtttgtgtaa tatagtaaaa tttggttttt attgaaaaat aaaaaaaata 660
   ttgtgggtag ggtggtgtat gtttgtggtt tttaggttga ggaggtggga ggattttttg 720
   agttttggag gttgaggtta gtttggttta tatagagaaa tttagtttta attaaaaaat 780
   aataataata ataataaaaa taaatttggg tagggtggtg tatgtttgta gttttgagaa 840
   tgagatggga ggattgatta aggtggatgt aattaatatt ataattatat tttataagta 900
   aatatatttt ttttttttta gggttatagg taaaggatgg taattgtgtg tattatattt 960
   atattttttt ttaaaaatgt ggtattattt ataatagtaa agatttggaa ttaatttaaa 1020
   tgtttaataa tgatagattg gattaagaaa atgtggtata tatatattat ggaatattat 1080
   gtagttataa aaattgatga gtttatgttt tttgtaggga tatggatgaa attggaaatt 1140
   attattttta gtaaattatt gtaagaataa aaaattaaat attgtatatt tttatttata 1200
   ggtgggaatt gaataatgag attatatgga tataggaagg ggaatattat attttgggga 1260
   ttgttgtggg gtggggggag gggggaggga tagtattggg agatatattt aatgttagat 1320
   gatgagttag tgggtgtagt gtattagtgt ggtatatgta tatatatgta attaatttgt 1380
   ataatgtgta tatgtatttt aaaatttaaa gtataataat aaaagaaaaa aaaaagaaaa 1440
   ttataaaaaa aaaaaaaaaa gaaaaaaaaa taagaataaa agaggttgga gggttttgga 1500
   ttgttttttt agttttaata gatgtagaag ttattgaaga atgaatttta tgattaagta 1560
   tagtaaattt ttgtaaatgt gttagtttgg aaaatattgt gttttttaaa tagaaaaatt 1620
   atagattgat tatttttttt ttttatggtt tagattagaa tttagatgtt taatttaaga 1680
   tttagggatg gtttttagag agtttaaaat tttttgatgg tgtttgagga ttatttattt 1740
   tgttgtataa agtgtggttg gaggaggtga taatattttg taatgttatt gtttaaggat 1800
   gatggattaa ttagggtagt tagtgaattt tatttggtta attagaagtt agaatagtag 1860
   gtggaataag tgaagttgat gtggtgtttg ttagtttagg ttttagggat agaatttttt 1920
   taaggtgggg gggaggggag attttgattt ttttgttgaa agtgtttttt tggattggtt 1980
   attttaaatt tagagtatgt atatgttgat ttgttttttt gattttttta taattagggt 2040
   aagtatgtgt tgattttttt tttttatttt ttttattttt ttttttgttt gtggtgtatt 2100
   ttttatttaa ttttaataat gtatttatgt gaggtaggtt gttattttaa attttttttg 2160
   ttagttttta atttttttag gtatgggatt ttttttagga gttttgtagt aatttaaaaa 2220
   atttgggttg ggtgtggtgg tttatgtttg taattttagt attttggagg tttataggtg 2280
   gtggattgtt tgaatttggg aggtggaggt tgtagtgagt tatgattgtg ttattgtaat 2340
   atagtttggg taatagagtg agattttgtt ttaaaaaaaa aaaaaaaaaa aaatttttgg 2400
   gtttaagtga tttttttgtt ttggttttgg gattataggt gtgtattatt ttgtttagag 2460
   tattaaaggt tttgaggttg gaaagattta ggttgttttt t 2501

<210> 223
<211> 2501
<212> DNA
<213> Artificial Sequence

<220>
<223> chemically treated genomic DNA (Homo sapiens)

<400> 223
   gagaaatagt ttgagttttt ttagttttag gatttttggt gttttgggtg gggtggtgta 60
   tgtttgtagt tttggggttg aggtgagagg attgtttgag tttaggagtt tttttttttt 120
   tttttttttg agatagggtt ttgttttgtt gtttaggttg tgttgtagtg gtgtgattgt 180
   ggtttattgt aatttttgtt ttttgggttt aagtgattta ttatttgtgg gtttttaaag 240
   tgttgggatt ataagtgtga gttattatgt ttagtttaga ttttttaagt tattatagag 300
   tttttaggaa aaattttata tttgaaaaag ttagaaattg ataggaagaa tttgagatgg 360
   tgatttgttt tatataaata tattattaaa attagataag aagtgtatta tggtggaggg 420
   gaggggtagg aagaatggaa agagaaaatt agtgtatgtt tattttgatt gtggaagaat 480
   taaaagggta aattggtgtg tgtgtatttt gaatttaaag tagttaattt aaggggatgt 540
   ttttggtggg aaaattagag tttttttttt tttttgtttt gggaaggttt tgtttttaga 600
   gtttggattg atagatgtta tattagtttt gtttgttttg tttattgttt tgatttttaa 660
   ttggttagat ggagtttatt aattgttttg attggtttat tatttttggg tagtgatatt 720
   gtagaatgtt gttgtttttt ttagttatat tttgtgtgat aaagtgggtg gtttttaggt 780
   gttattagga gattttgaat tttttgaaga ttgtttttgg attttgggtt aaatatttgg 840
   attttagttt gaattgtggg aagaaaaaat agttgatttg tgattttttt atttgaaaga 900
   tataatgttt tttaaattag tatatttgtg gaggtttgtt gtatttagtt gtggagttta 960
   ttttttagtg gtttttatat ttgttggaat taaaaaaata gtttaaggtt ttttaatttt 1020
   ttttattttt attttttttt tttttttttt tttttttgta gttttttttt tttttttttt 1080
   tattattata ttttaagttt tagggtatat gtgtatattg tgtaggttag ttatatatgt 1140
   atatatgtgt tatgttggtg tgttgtattt attaatttgt tatttagtat tgggtatatt 1200
   ttttaatgtt attttttttt ttttttttta ttttataata gtttttagag tgtgatgttt 1260
   ttttttttgt gtttatgtga ttttattgtt taatttttat ttatgagtga gaatatgtgg 1320
   tgtttggttt tttgtttttg tgatagttta ttgagaatga tgatttttaa ttttatttat 1380
   gtttttataa aggatatgaa tttattaatt tttatggttg tatagtattt tatggtgtat 1440
   atgtgttata tttttttaat ttagtttatt attgttggat atttgggttg gttttaagtt 1500
   tttgttattg tgaatagtgt tgtatttttg gaagggaatg taagtgtggt gtgtataatt 1560
   attatttttt atttgtagtt ttggagagag gaaaatgtat ttatttatgg gatgtgattg 1620
   tggtattggt tatatttgtt ttggttaatt tttttatttt atttttggga ttataggtat 1680
   gtattatttt gtttaggttt gtttttgtta ttattattat tattttttag ttgaaattgg 1740
   gtttttttat gtaggttagg ttggttttaa tttttagggt ttaagggatt tttttatttt 1800
   tttagtttgg ggattatagg tatgtattat tttgtttatg gtattttttt tgttttttag 1860
   tagaaattgg gttttgttat gttgtataga ttggttttaa ttttttgggt ttaagtaatg 1920
   tttttatttt agttttggga ttataggtgt gtgttatttt gttttgataa ttttttttag 1980
   gagagatagg tttttgttat gttgattagg ttggtattga ttttttgggt ttaagtaatt 2040
   tttttatttt agtttgggaa ttataggggt gagttatttt gtttatgtta tttttggttg 2100
   ttttttttat tagtagaaat ggggttttat tatgttggtt aggttggttt tgattttttg 2160
   ggtttaagta atttttttgt tttggttttg ggattattgg tatatattat ttagttttta 2220
   ttaatatttt taattttttt agtagagata gttttgttat gttgtttagg ttggttttta 2280
   ttttttgggt ttaagtgatt tttttgtttt ggttttggga ttgtaggttt gtattatttt 2340
   gttttttgtt atgtttttta ggttggtttt aattttttgg gtttatttaa tgatttgaat 2400
   ttgggaaggg gaggttgtat tgagttgaga ttttattatt gtattttagt ttgggtgata 2460
   gagaaaaatt gttgaaaaga aaaggaaggg aagggagaag g 2501

<210> 224
<211> 2501
<212> DNA
<213> Artificial Sequence

<220>
<223> chemically treated genomic DNA (Homo sapiens)

<400> 224
   ttgtattatt gtattttagt ttaggtgata gattgagatt ttgatttaaa aaaaaagaaa 60
   aaaaaagaaa gagagatata agttgagaga aggaaggtag ggatggtgtg gaggtgttgg 120
   gatgttatag agatatttgg atttattaga atagaagttt aagggagaga aatgtgtagg 180
   atttaggtat gagttttatt gtggttagtt tttgttttta gttttgatag gatatagaag 240
   agtagaatat ttagaagttg ttttgtgatt tttttttgta taaaaggaaa atgtggggta 300
   ttttttgtag tttaagaagt agttaaagta ggaaaaggga tgtttatgtg tttttagatt 360
   tgtttgtatt tagaattttt tgttatttag tttagttatg gttttatatt ttttttttat 420
   atatatatag atgatttttt ttgaggattt tatttttttt tatttttttt tagagaagta 480
   tttggtaata ttattgaagg tttaggaaaa gataaaaaag gaattttgtt agtgatttag 540
   ttagatatgt ttgttattta gttggagttg ttttttgtgt gttggatttg ggaagtgggg 600
   atgatatttt gttttggttg atgttatggt taattgatag aatatgaggg atttttttta 660
   gttttatttt tgttatatag tagggtttta atgttgttgg ttggtttttt ttttattttt 720
   tagaatggat tgagatttat taaatgtagt gtatggttat agatttgttt ttaaggatgt 780
   tagttgatga tagagtgagg atgggaggtt tttaagggtt tagatttttt ttaagatttt 840
   gttttttgtt tttagtattt ttattttttt ttttttagaa attggttatt ttatattgtt 900
   ttttgggtta ttattttgtt ttttttaggt tattttattt tttggatttt ttttggtatt 960
   tgagaattag ttttaggttt ttttgtaaag gtattgtttt tgtttatggt attgggagta 1020
   gtttgttttg taagagaaat agattgagtt tttttagtta tagtagtttt ggttttgtgg 1080
   agggagaaat tagtgaaggt tggttatttt tagttatttg gaattaggtg ttttattttt 1140
   ttatttgggg tttatttgtt tttgagtaat gatatggggt gaagttagat gaaaataggg 1200
   aattaggggt ttttgggaga agtattgatt tgggatgata ggttttttgt gggtaaagta 1260
   gttttgagtt tttgggaggg ggttggttaa tgttgttagt agtttttttg gggttaggtt 1320
   tattttgaaa gatgtataga tttttgttgg gaaggtagag atgtgattgt gtaattttat 1380
   ttagtggggg tatgttgata tttttattta ataaataaag gaagggaaat gagttttaga 1440
   gataatatgg tttttttggt gatggatttg attaggtaga gggatggggg gttttgtttt 1500
   gttgaagaga aatgagtatg gttaatatga atggatttag aggttattat tgggtgattt 1560
   gtaaattatt agaagaagag agttagaatt tttgagatta taagagtttg ttattattta 1620
   aagagaatgt agggagtttt aaggtatagt aattagttag gtgtagtggt ttatgtttgt 1680
   aattttagta ttttgggagg ataaggaggg tgggttgttt gagtttatga gtttgagatt 1740
   agtttggtta atatattgaa attattttta ttaaaataat aataataata aaatatatat 1800
   atatatatat atatatatat atatattaat agaagtggag tggtgtgtgt ttgtagtttt 1860
   aaggtttatg tgagaggatt atttgagttt aggaggaaag atttgagtga gattttttgt 1920
   ttgtttgttt gtttgtttgt tttgagttat ggtttttttt tgttgtttag gttgggatgt 1980
   agtggtgtga ttatggttta ttgtaatttt tgttttgtgg gtttaagtga tttattagtt 2040
   gtggttttta aaattggagt tataagtgtg agttattgta tttggtttaa atttgttaag 2100
   ttattagagt ttttaggaaa aattttatat ttgaaaaagt tagaaattga taggaaggat 2160
   ttgagatggt gatttgtttt atatatattt tttattaaaa ttagataata aatgtattgt 2220
   ggaggagggg aggggtagga agaatggaaa aagaaaatta gtgtatgttt attttgattt 2280
   tggaagaatt gaaagagaaa attagattgt gtgtattttg agtatggaag aattgaaaga 2340
   gagagaaagt tagagtatgt atattttgaa tttaaagtag ttaattttgg gggatgtttt 2400
   aggtgggaaa attagggttt ttatttttag ttagaaagtt ttgtttttgg aggttggatt 2460
   gatagatgtt atattagttt tgtttatttt gtttattgtt t 2501

<210> 225
<211> 2501
<212> DNA
<213> Artificial Sequence

<220>
<223> chemically treated genomic DNA (Homo sapiens)

<400> 225

gaatagtagg tgggataagt aaagttgatg tggtgtttat tagtttagtt tttagggata 60
gaatttttta attgggggtg gagattttga tttttttgtt taaagtattt tttgggattg 120
gttattttaa gtttagagta tgtatgtttt gatttttttt tttttttgat ttttttatat 180
ttagagtatg tatggtttga tttttttttt tgattttttt aaaattagag taagtatgtg 240
ttgatttttt ttttttattt tttttatttt tttttttttt tgtggtgtat ttgttattta 300
gttttaataa ggagtgtatg tgaggtaggt tgttatttta aatttttttt gttagttttt 360
aattttttta ggtatgggat tttttttggg aattttagta atttaataaa tttgggttgg 420
gtatggtggt ttatgtttgt aattttagtt ttggaggtta tagttggtgg attatttgaa 480
tttatgaggt ggaggttgta gtgagttatg attatattat tgtattttag tttgggtaat 540
agagagagat tatgatttaa aataaataaa taaataaata aataaaaaat tttatttaaa 600
tttttttttt tgggtttaag tgattttttt atgtaggttt tgggattata ggtgtatatt 660
attttgtttt tgttaatgtg tgtgtgtgtg tgtgtgtgtg tgtgtgtgtt ttgttgttgt 720
tgttgtttta gtagagatgg ttttggtatg ttggttaggt tggttttaag tttatggatt 780
taagtgattt gttttttttg tttttttaaa gtgttgggat tataggtatg agttattgtg 840
tttggttgat tgttgtattt tgaaattttt tatatttttt ttaagtgatg gtgggttttt 900
gtagttttag aaattttagt tttttttttt taataattta taaattattt agtaatagtt 960
tttaaatttg tttatattag ttatgtttat ttttttttaa tagaatagaa ttttttattt 1020
ttttgtttga ttagatttat tattagagag attatgttat ttttgggatt tatttttttt 1080
tttttattta ttgagtgggg gtgttagtat gtttttattg aataaaatta tatagttata 1140
tttttgtttt tttaataagg gtttgtatat tttttagggt aagtttggtt ttagggaaat 1200
tattaataat attagttaat ttttttttaa agatttaagg ttgttttgtt tataggaagt 1260
ttgttatttt aaattaatat tttttttaga gatttttggt tttttatttt tatttgattt 1320
tgttttatgt tattatttag ggatagataa gttttagatg gagaaatgaa gtatttgatt 1380
ttaggtgatt gagggtggtt ggtttttatt gatttttttt tttataagat taaagttgtt 1440
gtggttggaa agatttggtt tgtttttttt gtaggataga ttgtttttgg tgttatgaat 1500
ggagatgatg tttttgtaag gagatttagg gttggttttt aaatattaga gaagatttaa 1560
aaggtgaggt gatttggagg gggtaaagta gtggtttagg ggatagtgtg gggtgattgg 1620
tttttgagga ggggaggata gagatattgg agataaggag tagggttttg ggggagattt 1680
ggatttttga gagtttttta tttttgtttt attattaatt agtatttttg gagataagtt 1740
tgtgattgtg tattatattt ggtgaatttt agtttatttt ggaaagtggg aagagagtta 1800
gttagtagta ttaaagtttt attgtgtggt aggggtgaag ttaggaaagg ttttttatgt 1860
tttgttagtt agttatggta ttaattagga tggaatatta tttttatttt ttagatttag 1920
tatataggag gtggttttag ttgaatggta gatgtgttta gttgagttat tgataaaatt 1980
ttttttttat ttttttttag gtttttgatg atattgttaa atattttttt aagaaagagt 2040
gggaaaagat gaaatttttg gagaaaatta tttatgtgta tatgaagaga aagtatgagg 2100
ttatgattaa attaggtaat agaaagtttt aggtatagat aagtttgggg atatatgagt 2160
attttttttt ttgttttggt tattttttag gttgtagaaa gtattttata tttttttttt 2220
gtgtagggaa aaattgtaag gtagtttttg ggtgttttgt ttttttgtat tttgttaggg 2280
ttgagggtag ggattggtta tagtggagtt tatatttgga ttttgtatgt tttttttttt 2340
taggtttttg ttttgatgag tttaagtgtt tttgtggtat tttggtattt ttatattgtt 2400
tttatttttt tttttttggt ttgtattttt tttttttttt tttttttttt ttttgagtta 2460
gagttttagt ttgttattta ggttagagtg tagtagtgta a 2501

<210> 226
<211> 2501
<212> DNA
<213> Artificial Sequence

<220>
<223> chemically treated genomic DNA (Homo sapiens)

<400> 226
   gaaaataaag ttaatggtat atttaaaatg gagtttatta ttttattgtt taaaattaag 60
   taatttagta ttaggatgat gataatagtt agttatttaa gtttgaaatt aggtagttat 120
   tttagattgt tttttttttt ttatttttta ttattaaaaa gttataaaat tttattaatt 180
   ttatttttta aatatttttt atatttgata tttttttttt attttattgt ttgtgttata 240
   gtttaagttt ttatgtttta tttaggttat taaaattgtt ttgttatttt ggttttttaa 300
   gtttaaatta ttttttatat tataagtttt tttttgaatt tgattgtgta attttttttg 360
   tttaaaattt tttaagattt ttgtttataa agttaaaaat ttagatattt ttttaaggtt 420
   tttttataat atttgttttt tttttttttt tttttttttt tttttttttt tgtgagatgg 480
   agttttattt tgtggtttag gttggaatgt aatggggtaa ttttggttta ttgtgatgtt 540
   tgtttttttg ggggtttagg tgattttttt gtttagtttt tttgagttat tgggattata 600
   ggtgtgtgtt atgtgtttgg ttaattttgg atgtttattg gagatagggt tttgttatgt 660
   tggttaggtt agttttaaat ttttgatttt aagtgatttg ttttttttgg ttttttaaag 720
   tgttgggatt atagaagtga gttattgtgt ttggttaata tttgtttttg aaatttttag 780
   tgttattttt tgttattttt tttatttaat ttttatatag aatgtttttt ggatttagtt 840
   gtaataattt tttttataat tttaatgttt ttgtttgttt tgtgttagtt agtaatattt 900
   tttttttatt tttttttttt tttttttttt tttttttttt gttttttttt tttttttttt 960
   tttttttatt tttttttgtt tttttttttt gttttttttt ttttttttgt tttttttttt 1020
   tttttttttg agatagaatt tggttttgtt gtttagattg gagttagtgg taggattatt 1080
   agtttattgt agtttttgtt tttaagattt aagtgatttt tttgttttag ttttttaagt 1140
   agttgaggat ataggtgtgt attattatat ttggttattt taggagaggt agggttttat 1200
   tatgttgttt agattggttt tgaatttttt tttgggttta agtagttttt ttgttttggt 1260
   tttttaaagt gttgggatta taggtgtgag ttattggttt gtttagttaa ttttttattt 1320
   atttattttt atgtgaaata gtaatagaat tatattatat ttgtggagtt ttttttgatt 1380
   ttttaagtta ttttaagagt tattttgttt atgtttttat tataggaatt attttaaatg 1440
   attataggat attttaattg tttgtttatt tgtttttttt ttaagattgt gattttttgg 1500
   agggtagaaa tggatttttt atttttagtt ttagtttgtt atagagtttg atagtttttt 1560
   tagtattttt tgataatagt agaagtttaa agaattttta ttttaaggaa atatagaatt 1620
   tgggaaataa ttgtttttag tgattaggga aattttgtga gatttaggtt ttgggagttt 1680
   ggtaagttta tatagtgatg gttatttagt tgttgaattg ttagttgttt ggggtttatg 1740
   tgttttgggt tgtttaggtt gagtgatttt ttgttttttt tgaggttttg aggtgggagt 1800
   tattggtttt ttttgttgtt tttatagatt gtatgtagta gtttgtgtgg gtatagaatt 1860
   tatggttttt tgttagtttt ttaaaggtga gggtaggtgt tttgagttat ttttttgggg 1920
   attgagttta gagtggggtg atgttgtgtt attgtatttt tttgttgtag ttttttgttg 1980
   tttttttttg ggtagtggtt taggaattta agatagtttt ttttggtgat gtgatggaat 2040
   ttaatgggat aggagaaggg aatgggtttt ttttttaggt tagtgtggta gtgggtggta 2100
   gggtgaaagg gagaaggaaa tgagggttta ttttgttgtt tattttgtgg taagtgatgt 2160
   tgtagggttt tattgtagtg agagttttgt ggattttttt ttttttagtt atttagtttg 2220
   taaatattat tttaaagaat atatagtgta tttatgatat ttggtgaaaa aaaatttttt 2280
   tttttttttt gtttttttgg ggtagtagat atttataatt gtaatagaga aaatggaaaa 2340
   gtaaaagttt tttgtattgt ttgtattatt gagattaagt agtagttagg tgtttgtggt 2400
   gaaattttag attttgggag gtgattttat tttttttaag gtaaattttt atgtgatttt 2460
   taattggatt ttttttaatt ttagggaaat gaagaaaagt g 2501

<210> 227
<211> 2501
<212> DNA
<213> Artificial Sequence

<220>
<223> chemically treated genomic DNA (Homo sapiens)

<400> 227

tatttttttt tattttttta aagttaagaa gagtttaatt aagagttata tggaaattta 60

ttttgaagaa agtggaattg ttttttaggg tttgaggttt tattgtagat atttgattgt 120
tgtttgattt tggtggtatg aataatgtaa agggtttttg tttttttatt ttttttgtta 180
tggttgtaaa tatttattgt tttggggggg tgggagggga aggaaagttt ttttttattg 240
agtgttatga atatattatg tattttttaa agtgatgttt ataaattaaa tggttaaaaa 300
aaaaggaatt tatggggttt ttgttatagt ggagttttat aatgttgttt attgtggagt 360
gggtaatgga ataaattttt gttttttttt tttttttgtt ttattgtttg ttgttatgtt 420

ggtttgaaaa gaaagtttgt tttttttttt tgttttatta aattttatta tattgttagg 480
aggaattgtt ttaagttttt ggattattat ttaaaagaaa atagtggagg gttgtggtgg 540
ggaggtgtag tagtataata ttgttttgtt ttgggtttag tttttaggaa aataatttgg 600
ggtatttgtt tttatttttg aagaattagt gggaaattgt gggttttgtg tttatgtgaa 660
ttgttatata tggtttatga gggtaataga aagaattaat ggtttttgtt ttagaatttt 720
agaaaaggtg ggaaattgtt tagtttgagt ggtttggagt gtgtgggttt tgggtggttg 780
gtagtttaat ggttaaataa ttgttgttgt gtgggtttgt taggttttta ggatttgaat 840
tttgtgagat ttttttaatt gttaggaata attgtttttt ggattttgta tttttttgga 900
gtggaagttt tttaggtttt tgttattgtt aaagaatgtt agggaaattg ttaggtttta 960
tgatagattg aagttgagag tgaaaaattt atttttgttt tttaagaggt tataatttta 1020
ggaaggaaat agatgggtaa ataattaaaa tgttttataa ttatttaggg taatttttat 1080
gataaggatg taggtaaagt ggtttttagg gtggtttggg gagttaggag aagttttata 1140
gatgtggtat agttttgtta ttgttttata taagaatgag tgaatggaga gttggttagg 1200
tgggttggtg gtttatgttt gtaattttaa tattttggga ggttaaggtg ggaggattgt 1260
ttgagtttag gaaggagttt aagattagtt tgggtaatgt agtgagattt tgtttttttt 1320
aaaatagttg ggtgtggtgg tgtgtatttg tgtttttagt tatttaaaag gttgaggtgg 1380
gaagattatt tgagttttgg agatagaggt tgtagtgagt taataatttt gttattggtt 1440
ttagtttggg taatagagtt agattttgtt ttaaagagag agagagagga gatagaggag 1500
agaaaggagg tagagggagg aaatggagga gagtagggaa agaggggaga gaggggagag 1560
tgggggaagg gggagaggga gagggagagg gaatggggaa agagtattgt tggttgatgt 1620
aaggtgagta aaggtattgg agttgtgaga gagattatta taattaagtt taagaagtgt 1680
tttgtgtgag gattaagtgg ggaaaatggt aagagatgat attagagatt ttaagagtaa 1740
gtattagttg agtgtggtgg tttatttttg taattttagt attttgggag gttaagaagg 1800
gtggattatt tgaggttaag agtttgagat tagtttgatt aatatgatga aattttgttt 1860
ttagtaaata tttaaaatta gttaggtatg tggtatatgt ttgtagtttt agtgatttgg 1920
gaggttgagg taggagaatt gtttgaattt ttggggaggt agatgttgta gtgagttgag 1980
attgttttat tgtattttag tttgggttat agagtgagat tttgttttat aaaaaaaaaa 2040
aaaaaaaaaa agagagaaag agaaagtaag tattatagaa aggttttgga aaagtgtttg 2100
gatttttgat tttgtggatg gagattttga agaattttga gtagaaagga ttatatgatt 2160
gagtttagaa gaaaatttgt agtatgaaga atggtttagg tttaagagat tagagtagtg 2220
agatagtttt aatagtttgg gtgagatatg aggatttgag ttatgatatg gatagtggag 2280
tgaagaggag gtgttaaatg taaaagatat ttaagaagta aaattagtag aattttataa 2340
ttttttagta gtaagaagtg aaagaaaggg agtagtttaa gatgattatt tggttttagg 2400
tttgagtaat tagttattat tattatttta gtgttaagtt atttaatttt gagtaataaa 2460
ataatgagtt ttattttgaa tatattattg attttgtttt t 2501

<210> 228
<211> 2501
<212> DNA
<213> Artificial Sequence

<220>
<223> chemically treated genomic DNA (Homo sapiens)

<400> 228

tttgtagtgt gttggttatt gtgttgggta ggtagttgtg tatgttggtg gtgaaggttt 60
tgggggggtt tttgtgggtt ttgtttagta gtgtgaagtt gaaggttagt atgttttttg 120
tgttgtgttt gtatagtttt tgtagtattt gggttattag tttttttagg taggatattt 180
gtgggggaag tgttttgagt tgtttgtgtt gttttttgta tgttgttggt tttttttggt 240
tgtttttaat tttagttgga tgttgatttt ggggaggttt atttggtgga aggagggggt 300
ggtggggggt ggttgtgtgt tttagggtat gtatattagg tattgggtta ttagtgtgtg 360
gaaagttgtt gggtttttgt gttgtattag ttgttagttt tggggtttta ggtgttgtat 420
gaatgtggtt agtggtagta ttttgtggta gtggttgtgt agtagggagt gtatggtttg 480
gtagtgggga gtgtgtggta ttgtgggggt ggttggggtt agggtttttt atgtgtgtag 540
taggatgtag tgttgtttga aatttgtgtt gtgaggagag ggtggggttg tggaaaggaa 600
ggggaggggt tgggagggtt tggagggggt tgggttgggg atttgggagg ggttgggatg 660

gggtggggtt tgtgtggagg aggtggagtt ggaaggtgaa ggggtaggat gggtgtttgg 720
gtttttagtt tttttgttat gtgggaagtg tggttttggg tgtttgtgtt tgtgaattta 780
ttgggagttt ggtttggttt tgatagtgta gttgttttgg gtggatttgg gggtttgggt 840
tgtgtttttt tgtttgtgtg ttgtttgtgt ttttagggtg tagggatgtt agtgagggtt 900
ttagtggaga gaggttgaat tggtttaggt tgtggggtaa tttgagggag gggttatgat 960
gtggaggttt tgggaatagg tgtgtgtggt gattttttgg ttgttggttt gatttggaga 1020
tttagggttg tttttaggtt tggatgtggg gtgttgggtt ttgggtatta tgaatgttgg 1080
atgtgaaggg gaggatggag gtgtgtagat gtggttgggg atgaatttga ggatgtattg 1140
ttttttggat gggtatgtgg gattttttgg agtgtttttt tgtaatattt ttttgtgatt 1200
tgggtttttt gatataggtt tgttattttt ttttgtaggt ttttaggtgg tgaggggttt 1260
ttattatgag taaattattt taaatttgtt aattatttat tggggtggtt ttgttgagaa 1320
agggtgggaa atggagttag gtgtttttgt tggttgtgta ttgggtgttt tatattagtt 1380
ataatggttt tgattttggg ttttggtatt ttgtttggta gatgaggtta atatttggtt 1440
atattttgtt tgtatagggt ggagggtagt tttggggttt aggtatttgg ttttaagttt 1500
tggattgtag agttaggagg tttgattttt agtttagtag tagaagttat atggttattg 1560
gtttttttta gatttggggt tttggtataa ttgtaggata gttgaggatt ttttaggaat 1620
ttagattttg ggttgtttaa gtttggattt aaggggtgag aaatttttgg gttttttgag 1680
gtttgtaggg gatgttgtag ttgaggttgg taaatattga aatgttaata aatgtaattt 1740
taaatgtaat tttttttatt tttagaaatt gttggaggaa attgttttat ttatggagtt 1800
agtatttgaa taggttttgt attttttttg ggttgttatg tttgttggag gttagttttg 1860
ttttgtaaat atttaggatt atgggttgtt tttttagaaa tttttttagt gatttttaag 1920
tttttttaaa gggttgtgtt tgtgaattgt ttttgttatt agggtaaagg ggtggtttgg 1980
aaaatttgtt ttaataaaag ttaagttgta gtttatattg gttttttgta gagaagttaa 2040
tatagaaaat ataattttaa aagagggaag agaagaaatg gaagtagaag attatgttgg 2100
agtaattaat attatgtgta tggtgaggaa atgttttttg gtatttaatg aagattgttg 2160
atatttagaa gatattttag tattatgggt gtagttagtg tgtttttgaa aagttgatga 2220
tgttttagtt attatagtgt aaaatattaa gagtgtttta ataataataa aaaaatttta 2280
ttattggttt aaaatattat aatatttgag tggggtgagt tttttatttt agatttagat 2340
gtttttaaat agagtaaatt ttgagttggg tatggtggtt tatatttgta attttagtat 2400
tttaggaggt agaggtaggt ggattatttg aggttagaag tttgagatta gtttggttaa 2460
tatggtgaat tttattttta tttttgtttt tattaaaaat a 2501

<210> 229
<211> 2501
<212> DNA
<213> Artificial Sequence

<220>
<223> chemically treated genomic DNA (Homo sapiens)

<400> 229

tatttttagt agagatgggg gtgggggtgg ggtttattat gttggttagg ttggttttga 60
atttttgatt ttagatgatt tatttgtttt tgttttttaa agtgttggga ttataggtgt 120
gagttattat gtttagttta gaatttattt tgtttagaaa tatttgggtt tgaggtagga 180
agtttatttt atttaagtgt tgtggtgttt taagttaatg atagaatttt tttattgttg 240
ttagaatatt tttgatgttt tatattgtga tgattaagat attattagtt ttttaaagat 300
atattaattg tatttataat attggggtgt tttttgggta ttagtgattt ttattgaatg 360
ttgggaggtg tttttttgtt atgtatatgg tgttaattat tttagtataa ttttttgttt 420
ttattttttt tttttttttt tttaaaattg tgttttttat gttggttttt ttgtagagaa 480
ttagtgtaag ttataattta atttttgttg gaataaattt tttaaattgt ttttttgttt 540
tagtggtaga gataatttat aaatatagtt ttttaaaaag gtttagggat tattaagggg 600
atttttagaa gagtgatttg taattttaag tatttataag atgaggttaa tttttagtga 660
gtgtgatagt ttagggaggg tgtgaggttt gtttaaatgt tagttttata aataaagtaa 720
ttttttttgg tagtttttga aagtaggaaa ggttatattt aaggttgtgt ttgttagtat 780
tttagtgttt gttgatttta gttatagtat ttttgtaagg ttttgggaga tttagaagtt 840
ttttgttttt tagatttaaa tttgagtaat ttggagtttg gatttttggg aagtttttag 900
ttgttttgtg gttgtgttgg ggttttaggt ttggagggga ttagtggttg tgtggttttt 960
attgttgggt tggaagttgg gttttttagt tttgtagttt gaggtttgga gttaggtgtt 1020
tggattttga ggttgttttt tattttgtgt gggtgggatg tgattagatg ttggttttat 1080
ttgttagata gagtgttggg gtttagggtt aaggttgttg tggttggtgt gaggtgtttg 1140
gtgtgtggtt agtaggagtg tttggtttta ttttttattt ttttttgatg ggattgtttt 1200
ggtgggtgat taatagattt ggggtggttt gtttatggtg ggattttttt gttgtttgag 1260
aatttgtaaa gagaaatgat gggtttgtgt taaggagttt aagttgtggg gaagtgttgt 1320

agggaggtat tttgggaggt tttgtgtgtt tgtttaggga gtaatgtgtt tttgggtttg 1380
tttttagttg tgtttatgtg tttttgtttt tttttttatg tttggtattt gtggtgtttg 1440
gagtttgatg ttttgtgttt ggatttggag gtagttttgg gtttttggat taggttagtg 1500
gttaaagggt tgttgtatgt atttgttttt agggttttta tattatggtt ttttttttgg 1560
gttattttat agtttaggtt gatttgattt ttttttgttg gggtttttgt tggtgttttt 1620
gtattttggg agtgtgagtg gtgtgtgggt ggggaagtgt ggtttagatt tttgggtttg 1680
tttggagtag ttgtgttgtt ggggttaggt tgggttttta gtggatttgt gggtatagat 1740
gtttaggatt gtgtttttta tgtggtggag ggattgggga tttgggtatt tgttttgttt 1800
ttttattttt tagttttgtt ttttttgtgt ggattttgtt ttgttttgat tttttttggg 1860
tttttggttt agtttttttt gggttttttt agtttttttt tttttttttt gtggttttgt 1920
tttttttttg tggtgtgagt tttaggtagt gttgtgtttt gttgtgtatg tgggaagttt 1980
tggttttggt tatttttgtg atgttgtgtg ttttttgttg ttgagttgtg tgttttttgt 2040
tgtgtagtta ttattgtgag gtgttgttgt tggttatgtt tgtgtggtgt ttggggtttt 2100
agggttggtg gttggtgtag tgtggggatt tggtggtttt ttgtgtgttg gtggtttagt 2160
gtttggtgtg tgtgttttgg gatgtatggt tgttttttgt tgtttttttt ttttgttagg 2220
tgggtttttt tggggttggt gtttggttgg ggttgagggt ggttgggggg aattagtgat 2280
atgtggagag tagtgtaggt gatttagggt gttttttttg taggtgtttt gtttgaagga 2340
gttggtggtt tgagtgttgt agaggttgtg tgagtgtggt gtgaagaatg tgttggtttt 2400
tggttttgtg ttgttggatg gggtttgtgg gggttttttt gaggttttta ttattagtgt 2460
gtgtagttat ttgtttaata tggtgattga tgtattgtgg g 2501

<210> 230
<211> 2501
<212> DNA
<213> Artificial Sequence

<220>
<223> chemically treated genomic DNA (Homo sapiens)

<400> 230

agaagaaggt atttaaaggt ttagggagat tgagatggtg gagtggatta gttattttag 60
atttatttat tttagttgag agggtttaga agatatattt ttgattattg ttttgtgaaa 120
taggtttgtg agggtagtat ttgtattttt gaagggtttt gtaattgttt ttttttgtat 180
tttagattta atagtgggaa ttgtagttat taaattataa aatttaaata tattgggaat 240
aattggattt taaggtggta ggggttaagt ggtggtattt aattattaaa ggtaaggtgg 300
gtatagttat tgtaatgggt agtagaggta aagtagtaat tagaatagtt tgatttgtgt 360
agagttttgg tattgggtaa ttaattaagg tgtttttaga aatgaaattg ataggaagtt 420
tattgtattt ttatttaatt tatataagta gaaaattttt agattggatg gataatagat 480
taatttgaat tataaatata gagaattatg gttttttagt taatttttaa atttgagtta 540
gtttatagat ttagaatttt ttgaatgaag gggaggttgg gtttttttga ggaaggattt 600
tattatatta ttgataattt atgttgttaa tatttttttt attttttttt aaggagattt 660
atggtttttt attagggtaa ttgtgtattg gggaaaggga aatggttaga tattttgggg 720
attattggat attggttttg agttgatatt gattttaggg tatttaaaat gttattgtgg 780
ttttttagtt aaagtaggga tttatggagg ttaggtaatt aatagagttt tagtttaggt 840
ttgattttgg tttagtgggt ttttggattt gttttgtggt tattttttta gtgttagaat 900
gtataattgg tatatatata tttagtagtt ggtagaattt ttatattggt tttttgattg 960
gtagggtgag ggttattatg gtgggaaagg ttaaatagaa gtttttagag ttgtttttat 1020
gtagaaaaat agtaaattaa aaataatatt gtattttggg agggattata gagattagtg 1080
ttattattaa ggatttgaag atgtaggggt ggtgattttt attatatttt tgtttaattt 1140
ttttatttgg tttgtgtaga aggtagatgg atttaggaga atgatagtgg attattataa 1200
gtttaattaa gtggtgattt taattgtagt tgttgtatta gtttttttag ttttagatgg 1260
aaataaattt tatttgtttt aggtttttgg tataaaaggt gtaattttag ttattttata 1320
ggtttgggtg aggaaaatgt taagagttgt aaaagggttt ggaaaagtaa aaaaggtgat 1380
gtataaatgt aatgattatt attttgtgtt taagaataag ttttggaata taaaatagga 1440
aagtaagttt atagttttaa aatatatgta tataaaattt taaaataatg tagttttaat 1500
tttatagatg gtatttgggg attgtttatt gttttagagt tttaatgttt ttttttttat 1560
gtgggtgttg atttttaaaa aggtagtagt gttgttttta atgtgggtgt tgatttttaa 1620
aaaggtagta gtattgtttt taatgtagtg gttagtaatg ttaagtgtaa tatgtatgtt 1680
aaagagtttt aatttttatt gtaaataagt aagggaagga gattttaaag ttttggttgg 1740
aggaagtttt gttgtagagt agtggttgta tttttgtgtg aataaagggt tagatagttg 1800
ttattttgag gtgagggttt taggttagga gatgttgagt gtttgggagg tttttgatga 1860
ggtggagggt tttgagttta tggagagttg tgaagagaga gaggatttat ttgagttagg 1920
attgtatggt gtgattttgt ttaggttttg ttttttggat ggtttggtgg ttttgttttg 1980

tttttttgtt ttttttgggt gttttaagtt gttgttagaa tttatggttg gtgtggaaat 2040
tgttgtgagg tggtgttatg atggaggttg aggttgttgg tagtatttat ggtgttttag 2100
agtggtagtt ttttggggtt tttggtttgg tttttgtttt tttagttatg gtttgaagag 2160
gtttgggtag gtagggtggg tgtgtaggga ggtgtagtat tgtttttttt ttttgtgttt 2220
tttttagggt taggtggtta ggattttgga gtgagtggat gggagttgtt atttgtaggg 2280
gttttaggat ttttagtggt tttttagttt agggggaatg tagtgtgttt tgttttggtg 2340
ttattttttt taggtgagtt tgatttttat taggttttgg taattttata tatattatta 2400
ttaatatgga ttaaagggat agtagtatta aatgttaggt gatgtttttg ggttatgttt 2460
agttgtagag aattggtttg gtttgagggg gaggggattt g 2501

<210> 231
<211> 2501
<212> DNA
<213> Artificial Sequence

<220>
<223> chemically treated genomic DNA (Homo sapiens)

<400> 231

taggtttttt tttttttaaa ttaggttagt tttttgtggt tgggtatggt ttagaagtgt 60
tatttgatgt ttaatgttat tgtttttttg gtttatgttg atgatagtgt gtgtgggatt 120
gttagagttt ggtaagaatt agatttattt gagggaagta atattgaagt ggggtgtatt 180
gtgttttttt tggattgggg ggttgttggg gattttggag tttttatagg tggtggtttt 240
tatttgtttg ttttggggtt ttggttgttt ggttttgagg ggagtgtgga ggggaagagt 300
agtgttatgt ttttttgtgt atttattttg tttgtttggg tttttttgag ttatggttga 360
agaagtaaga attaggttag gagttttagg gagttgttgt tttgggatgt tatggatatt 420
gttggtgatt tttagttttg ttgtggtgtt gttttgtagt ggtttttatg ttggttatgg 480
gttttggtgg taatttggag tgtttgggag ggatgagggg gtggggtggg gttattaggt 540
tgtttgaggg gtggggtttg ggtggggttg tgttgtgtag ttttgattta gatgagtttt 600
tttttttttt gtagtttttt gtggatttag ggttttttat tttattggga gttttttagg 660
tatttagtgt tttttggttt agggtttttg ttttagagtg gtagttgttt agttttttgt 720
ttatataaaa atgtgattat tattttatgg tagagttttt tttagttagg gttttagagt 780
tttttttttt tatttgtttg taataaaaat taaaattttt taatgtgtat gttgtattta 840
atattattaa ttattgtgtt agagatgata ttgttatttt tttgaaaatt aatatttata 900
ttagagatga tattgttatt tttttggaaa ttaatattta tgtgagaaaa ggggtattga 960
gattttaaag tagtagataa tttttagata ttatttgtag agttggaatt gtattatttt 1020
aaagttttat atgtatatat tttagggttg tagatttatt tttttatttt gtgttttaag 1080
gtttattttt gagtataaaa tgataattat tatatttata tattattttt tttatttttt 1140
taagtttttt tatagttttt ggtatttttt ttatttaggt ttgtgaggta attgggattg 1200
tattttttat attagaaatt tgaggtagat gaaatttatt tttatttagg attagaaaaa 1260
ttggtatagt agttgtaatt ggagttatta tttggttaaa tttatgataa tttattgtta 1320
tttttttaga tttatttgtt ttttgtatag gttgaatggg agagttgaat agggatgtgg 1380
tgggaattat tatttttgta tttttaagtt tttgatggtg gtattgattt ttgtaatttt 1440
ttttgggatg taatattatt tttgatttat tattttttta tatagaggta gttttaaggg 1500
tttttatttg gtttttttta ttataatagt ttttatttta ttagttaggg agttaatgtg 1560
ggagttttgt tagttgttaa gtatgtatat gttaattatg tattttggta ttggggaaat 1620
gattatagga tgagtttagg gatttattgg attgaggtta gatttgagtt aaaattttat 1680
taattatttg atttttataa gtttttattt taattggaag attatagtga tattttgggt 1740
attttggaat taatgttagt ttagaattag tgtttagtag tttttaaaat atttgattat 1800
tttttttttt ttagtgtata gttattttgg taaaaggtta taggtttttt tggggaagga 1860
tgggagaaat attaatagta taagttgttg gtagtgtaat ggggtttttt tttaagggga 1920
tttagttttt tttttattta agaggtttta ggtttgtaaa ttggtttaag tttggaaatt 1980
gattgaggag ttgtgatttt ttatgtttat aatttaaatt agtttgttgt ttatttgatt 2040
tagaagtttt ttgtttatat aaattaagta ggaatgtagt gggtttttta ttaattttat 2100
ttttagaaat attttgatta attatttaat gttagagttt tatatgagtt agattatttt 2160
gattgttgtt ttgtttttgt tgtttattat agtagttatg tttattttgt ttttgatggt 2220
tgagtgttat tatttggttt ttgttatttt gggatttaat tatttttaat gtatttaaat 2280
tttgtagttt agtgattgta gtttttattg ttagatttga aatatagaga agaataatta 2340
taggattttt taaagatgta ggtattgttt ttataaattt attttataag gtagtgatta 2400
agggtatatt ttttggattt ttttagttgg gatgagtaga tttaaagtga ttaatttatt 2460
ttattatttt aatttttttta agttttgga tgtttttttt t 2501

<210> 232

<211> 2500
<212> DNA
<213> Artificial Sequence

<220>
<223> chemically treated genomic DNA (Homo sapiens)

<400> 232

tattggtgtt tattgtgttg tgttgtttat aggtagggta tagttgttgt tgttatttag 60
ggaggggatg gtgggtagta gtgtgtttag gttttgtatg ttggagttta tttgttgtgg 120
tttagatttg gatgttttgt ggtttttttg gttttggaga tgtttagtgt tggttttggt 180
ggtgtttaat ttggtttaga tgttgtttgg gttttggatt ttttgttgtt ttggttgttg 240
taggtattta ggtttggagt ggagtgtgtg ttgagatgtt ggttttggga tatatgtgga 300
agttgggttt tgtagtaaga ggaagtttag gattgtggtg aggagatggt ggggtttggg 360
tgtttgggtt gttgttttgg ttttgggtgg gtgggtgggt gaatgagtag gtgggaggga 420
gggtgggaag tgggggagtg gataggtggt tgggttgtgg agagtttttg ggtgtgggtg 480
ttgttttgaa ttttttattt tagttgtttg gttgttttta gttttttaaa gtttaaataa 540
gaggggttgg tggtgtgggg aagaggagga gttaggaggt ttggttgttt tatttattta 600
gtagtttaag tttggttagg tagtgtgtgt tgttttttgg gggtgttttt gtagttgggg 660
tgagggtggg tttgagggag tgtttttttt ggagatattt ttttttttaa tttaatatgt 720
gtttttagtt ggttggtgtg gtataatttt ttttttattt tttttttttg gtttttgtta 780
gtgtgggagt tttgttttat tggagagttt gtttaggtaa gtagtgagtt tgggtagggt 840
tgaatgtggt gggagtagag atggggttgt taatttaaaa gagtggtttg gagggagggg 900
taggtaggtt ttaagagggt gggaatttta ggaaagtgta gaaagaattt agttaagttt 960
taaagaagtg tatatttttg ttttgtattt ttgagtttgg tttttgtttt tagaattgtt 1020
gggtagggga gtggttatga ttttggggtt attggggaaa gaggtggttt tttgtgggtt 1080
ttagaggttg ggattgaaag tgaatgtttt ttagttgggt ggttgggagt tgtattagtt 1140
ataagttggg tgtattttga ttggttagta gttgtttatt tggtgaggtt tgtgttaggt 1200
ggaaggtggt tgttgtttat ttaaaattgg agttagtagt atttagtttt tttttttagt 1260
gaggaatatt taattttttt atggttaagt tgggaaaata gtggttagtt taggagggaa 1320
gaaaattttt gggtaaatgt ttaaagtttg tgtatatttt ggttggagta ttttggtgtt 1380
tagtttgggg aggaggggtg aaggggaggg tttggatatg aagtttggta gggagtgggt 1440
aatttttgga gttattttgt tgagtattgt ttgtggtttt ggttgaggat attattttgg 1500
ttgtttagtg attgattagg aggtggaagt tagttaaggg gattaaggag tggagtaggg 1560
agaattttta ttggtttatg ggtgtgagtt agtgtttgaa tttttgggag tttgaagagt 1620
tttgttaaat ttgatttttt ttgtttagtt tttgtttggt agtttttgtg ttttagagta 1680
ggtgtttggg gtgggtaggg ggtgttttgg tttggttttt ggatttttta aaggagttag 1740
tatttattgg gaaataattt ttaggtaagg agaagaattg ggggttggag tggagtgtgt 1800
tttagaggtg tgtataaaat tttaggggtg ttaggaggta gtagtgatgg tgtagtgtgg 1860
gatggttagg tgtgatggga gggaagtaat tggttgtagt ttggtttttt gtatttggtt 1920
ggtttgggtt ggggttgtaa gttttttagt taaatttatg tgaagtaaag tagttgtagt 1980
ttgataaaga atggtgtttt aataaatgat atagtaattt tttatgtttt ggtttttagt 2040
ttgtattgta taaatgttgt tgttattaaa ttttggttgt ggatttgaag tgtgtgtggg 2100
tttaggattt atttgaaggg tgtatttttt gggaatgtgt atttgtaggg tgtaggagat 2160
tttgagggta aagatgtggt atttgagttt tttgtttgtt ttttaggtta tttttgttgt 2220
ttgattttgt gttgttttta tttagaagtt tttaaaatta ttattaagaa aagaaagatt 2280
ttataagata ttgtttttat gttttttgtt tagttttttt ttatatgatt tatttttaaa 2340
ttttttgtgg ggtttgtgtg tttggttttt tgagtggaga aaggattttt gagataaaat 2400
ttattgttag ttgttttaag aatttttag attttgtat ttttttttt ttttttgtt 2460
tattattttt atttggaagt tgtttttttt aattaggatt 2500

<210> 233
<211> 2500
<212> DNA
<213> Artificial Sequence

<220>
<223> chemically treated genomic DNA (Homo sapiens)

<400> 233

agttttagtt aagaaaaatg atttttaagt agaaataata ggtggggaga aggaagggag 60
atatagggat ttgggggtt tttaggtaa ttggtagtga atttgtttt gagagttttt 120

tttttattta aaaaattaaa tgtgtgagtt ttgtgaaagg tttagggata gattgtgtgg 180
gagaggattg agtagagagt gtgggggtag tgttttgtag aatttttttt tttttaataa 240
taattttaaa agtttttgag tggagatgat gtaaagttaa gtagtaaagg tggtttggga 300
ggtaagtgga gggtttaagt gttgtatttt tatttttagg gttttttgtg tttatgggat 360
gtgtattttt aagaagtgtg ttttttgagt aagttttggg tttgtatata ttttgggttt 420
gtagttagaa tttaatggtg ataatgttta tgtaatgtaa gttaaaaatt aaagtgtaaa 480
aaattattat gttatttatt gaaatgttat tttttgttaa attgtaatta ttttgtttta 540
tataagtttg gttggaaagt ttgtagtttt agtttgggtt agttaggtat aggaggttgg 600
attgtaattg gttgtttttt ttttgttgtg tttggttgtt ttatgttgtg ttgttgttgt 660
tgttttttgg tgtttttggg attttatatg tatttttgaa atatgttttg ttttggtttt 720
tggttttttt ttttgtttag gggttgtttt ttaatagata ttgatttttt tagaagattt 780
aaaaattaaa ttaaaatatt ttttatttgt tttaaatatt tgttttgggg tgtgggggtt 840
gttaaataga gattagatga agggagttag atttagtgaa gttttttgag tttttaaaga 900
tttgaatatt aatttgtgtt tgtgggttga tggaggtttt ttttatttta ttttttggtt 960
tttttaattg gtttttgttt tttggttaat tattgagtaa ttagaatggt atttttgatt 1020
agggttatag gtagtgtttg gtggagtggt tttaggagtt atttgttttt tgttgggttt 1080
tgtatttaaa tttttttttt tatttttttt ttttaaattg ggtgttagga tgttttggtt 1140
ggaatatatg taggttttgg gtgtttgttt aagggttttt ttttttttta aattagttgt 1200
tgtttttttg gtttaattgt agaagaatta gatatttttt attggaaagg gaaattaagt 1260
gttgttgatt ttaattttag gtaggtggta attgtttttt gtttggtgta aattttatta 1320
agtaaataat tattagttga ttgaaatatg tttggtttat aattggtgta atttttggtt 1380
atttaattga gggatgtttg tttttagttt tgatttttgg aatttataaa gggttatttt 1440
tttttttagt gattttaaga ttatggttat ttttttattt gatagtttta gaagtaagag 1500
ttagatttaa gggtgtaaag taagggtata tgtttttttg aagtttgatt gagttttttt 1560
tgtgtttttt tgaagttttt gtttttttgg agtttatttg tttttttttt taaattattt 1620
ttttagatta ataattttat ttttattttt attgtatttg attttgtttg gatttattgt 1680
ttatttgaat ggatttttta gtgagatgag gtttttatat tggtgaaggt taagaagggg 1740
aggtgggggg agggttgtgt tatattggtt agttgagagt gtgtgttggg ttgaagagga 1800
gggtgttttt gagagggatg tttttttgga tttgttttta ttttagttgt gagggtgttt 1860
ttaaggagta gtgtgtgttg tttggttggg tttgggttgt tgagtgaatg gagtggttga 1920
gttttttggt tttttttttt ttttgtgttg ttggtttttt ttatttgagt tttgggaagt 1980
tgagggtagt taggtagttg gggtaaggag tttaaggtag tgtttatatt tgggggtttt 2040
ttgtaatttg attgtttgtt tgttttttta tttttttgttt tttttttttat ttatttattt 2100
atttatttat ttatttagag ttgggatggt agtttaggtg tttgggtttt gttgtttttt 2160
tgttgtgatt ttggattttt ttttgttgta ggatttggtt tttatgtgtg ttttggagtt 2220
ggtgttttag tatatgtttt gttttgggtt tgggtgttta tagtagttag agtagtaggg 2280
agtttgggat ttgggtggta tttgggttaa gttaggtgtt gttgaggtta gtgttgaatg 2340
tttttagggt tggaggagtt gtggggtgtt tgggtttgag ttgtagtaaa tgggttttga 2400
tgtgtgggat ttgaatgtgt tgttgtttgt tgtttttttt ttgggtggtg gtggtggttg 2460
tgttttgttt gtgagtggtg tggtgtagtg ggtgttggtg 2500

<210> 234
<211> 2500
<212> DNA
<213> Artificial Sequence

<220>
<223> chemically treated genomic DNA (Homo sapiens)

<400> 234

tggtagaatt tttttttgtt aattttttga ttattggtat aaaaatatag tatttaaata 60
tgtgaagtaa aagataattt aggggttagt tgattatggt ttgagattta aatatagttt 120
atggtttgtt ttgtatagtt attgagataa ggattttttt ttatttaaaa gtattattgt 180
taaaaaatat aatattatgt tatagaaatt atatgattta taaagattga aataattatg 240
attagatttg ttaaagtaaa atttggtgat gttgtattaa ttaaatattt gttattttgt 300
tttgtattat tgtatatatt tttttattta ttaattaatt tatttatata tggattgaaa 360
atatggatgt tattttattt ttttaagaaa gtgtttagtt ttatgatatt atgattttag 420
tttgggaaat ttgttttagg gattaggtta atatataaat atatatttga ggtaggagat 480
tggtaggatt tgttttttgg ttataattga gtttagagtt tatagttaga atttatggtt 540
atttttgaaa tatatattgt ttgaatgatt tataggtata tttagggaaa ttagttatta 600
gtttagagat ttaaatgtga agtaatataa tttaaaaaga aaagagtaaa taggtgtgaa 660
tgtatttatt ttatagaaga atttatttaa ttatgtttat tggaagtatg gtagaaaatt 720
tagaattaga gaataaagtg attaataaaa gtatttataa aattggttaa ggtgggagga 780

ttatttgagt ttaggaaatt gagattagta tgggtaatat agtgagatta ttatttaaaa 840
aa[?][?]aataaa taataaataa attaattatt taggtgtggt gtttgtattt gtagtagtag 900
g[?][?] [?][?]gggga ggttatattg ggaggattgt ttgagtttag gagatggagg ttttagtgag 960
g[?][?] [?][?]ttat gttattgttt tttagtttga gtaatagagt gatattttgt ttggaaaaaa 1020
aa[?] [?][?]ataa aaatggtttt ataaataaag atatgaaaat aatttatttt ttttataaaa 1080
gg[?][?][?][?]tttg agatatattt tttagtaagg gattgtttgt ttttaatgtt tttgaatgta 1140
tgagattgga taaagagaga tgaaagtttt taagtttttt tttttattat aaatgttatg 1200
gggatgtggg tttgggaata gtggaaaatt ttattttgtt ttgaaaagtt tttggtttaa 1260
tgtgtatgtt tttttttatg aaagtttttt gttgtttatg tgttttgttt tttaattttt 1320
gtttttttat aattgtgaaa ggagatgttg tgattttttt tttttgtgtt gattatgatt 1380
tagttagtag gtttgtagat tagttttttt agaaagtgaa gtgtgagtga gtgtgagggg 1440
gagtgtgggg ggttttttgt gggttgtgtt atttggttta tggtttttga ggttgttatt 1500
tttttattaa aatttagtga tattgttatg gttttgttgt tgtggggttt tgggtatggg 1560
atgaaggagg gttatagtgg ggaggagggt taggtgagta tggagtgagt tttttttttt 1620
tttttatgga gttttgtttt gttattaggt tggagtgtag tggtgtaatt ttggtttatt 1680
gtaatttttg ttttttgggt ttaagtgatt tttttgtttt agttttttga gtagttggga 1740
gtataggtgt gtataattat atttagttaa tttttgtatt ttttagtaga gatgggtttt 1800
aattatgttg gtgaggatgg ttttgatttt ttggttatat gatttatttg tttttgtttt 1860
ttaaagtgtt gggattatag gtgtgagtta ttgtgtttgg ttatggagtg agttttgaag 1920
gttgatttta tttgaggtat ttgagttttg gtggggggga atttatatat agagaggagg 1980
gattttaaag ttgttaatgg ggatttggga aggagtatag gatagggtaa ggtgggataa 2040
ggaggggtat tatagttttt aaggtatgag ggaattttat tgtgtatgtt tttttggtgt 2100
ttattttagt gtgtatgttt attgggtgtt tttttattgg tttttttgtt agtgtgggga 2160
atgtggtgga gttgtgagtt ggtgatttgg gtttttgagg tttggatttt ttttttgtta 2220
ttgagatatg gtgggtaggt ttataggtag atttaattgg gagttgaagt gtgagtgaga 2280
gtgaagagga attagtaggt ttttggaggg ttgtgtggtt agtgatttag agtgagaagg 2340
tttttgaagt tgttgttttt tttatgtggt gttatgttta tggatttttt tgttttgtta 2400
tggttataat tagggaggaa ggagggttga ggagtggagg ggtttaggtg aagttggggt 2460
gttgttgggg gtatttgagt tttagaagta tttggaattt 2500

<210> 235
<211> 2500
<212> DNA
<213> Artificial Sequence

<220>
<223> chemically treated genomic DNA (Homo sapiens)

<400> 235

gggttttagg tgtttttggg atttggatat ttttaatagt attttagttt tgtttgagtt 60
tttttatttt ttggtttttt ttttttttta gttatggttg tgatgagata agaaggttta 120
tgggtgtggt attgtatgag agggatgatg attttgaggg tttttttatt ttgagttatt 180
gattatataa ttttttggaa gtttgttggt ttttttttat ttttatttat attttaattt 240
ttagttggat ttgtttgtgg atttatttgt tgtgttttag tagtggagaa agaatttaga 300
ttttagggat ttgagttgtt ggtttatagt tttgttgtgt tttttatatt ggtgaagggg 360
ttgatgggaa gatgtttagt gaatatgtgt attgaggtgg gtattaaagg agtatgtgta 420
gtgaggtttt tttgtgtttt aagggttgtg gtgttttttt ttattttgtt ttgttttgtt 480
ttatgttttt ttttaggttt ttattaatga ttttggaatt tttttttttt gtgtgtgggt 540
ttttttttat taggatttaa atattttaaa taaagttagt ttttaaaatt tattttatga 600
ttgggtgtgg tggtttatgt ttgtaatttt agtattttgg ggggtggagg tgggtggatt 660
atgtggttaa gagattgaga ttatttttgt taatatggtt aaaatttgtt tttattgaaa 720
aatataaaaa ttagttgggt gtggttgtgt gtgtttgtat ttttagttat ttgggaggtt 780
gaggtaggag aattatttga atttgggagg tggaggttat agtgagttga gattgtgtta 840
ttgtatttta gtttggtgat agagtaagat tttgtgaaaa aaaaaaaaaa atttatttta 900
tgtttatttg attttttttt ttgttatggt ttttttttgt tttatgtttg ggattttata 960
atagtaaggt tatggtagta ttgttgagtt ttagtagaaa gatgatgatt ttagaggtta 1020
tggattaagt ggtataattt ataggaagtt ttttatgttt ttttttatat ttatttatat 1080
tttatttttt gggaggattg atttgtagat ttattggtta agttatagtt agtatgaaag 1140
aaggaagtta tgatgttttt ttttatagtt gtggagggat agaagttaga gagtaaggtg 1200
tataggtagt aaggaatttt tatagaaagg gatatgtata ttgagttagg gattttttag 1260
ggtagggtag ggttttttgt tgtttttaga tttgtatttt tatggtgttt gtagtagaaa 1320
aagggatttg agagtttttg tttttttttg tttaatttta tgtatttaga aatgttggga 1380
atagatagtt ttttattgga ggatgtgttt taggaagatt ttttgtggaa gaaataaatt 1440

atttttatat ttttatttat aaagttgttt ttatttattt ttttttttag atagagtgtt 1500
attttgttgt ttaggttgga gggtagtggt atgattatat tttattgaag tttttatttt 1560
ttgggtttaa ataatttttt tgatgtagtt tttttagttt ttgttattat aggtgtagat 1620
attatatttg ggtaattggt ttatttatta tttatttatt ttttagatag tggttttatt 1680
gtgttgttta tgttggtttt aattttttgg gtttaagtga tttttttatt ttggttagtt 1740
ttataaatgt ttttattaat tattttgttt tttggttttg ggttttttgt tatgttttta 1800
ataaatgtaa ttgaataaat ttttttgtag aataaatata tttatatttg tttatttttt 1860
tttttttagg ttgtgttatt ttatatttaa atttttggat tggtaattga tttttttgaa 1920
tatatttgta ggttatttag atagtatatg ttttagaaat agttatagat tttgattgta 1980
ggttttaggt ttagttatga ttagaaaata agttttgtta gttttttatt ttaaatgtat 2040
gtttgtgtat tgatttgatt tttgggataa gttttttaga ttgaaattat ggtattataa 2100
agttggatgt ttttttgagg ggatagagta atatttatat ttttaatttg tgtgtaaata 2160
aattaattaa tggatgaaaa gatgtatgta ataatgtaaa gtagaataat aggtatttga 2220
ttaatatggt attattaaat tttgttttaa tagatttgat tgtaattatt ttagtttttg 2280
tgggttatgt agtttttgtg gtgtagtgtt gtgtttttta gtaataatat ttttaaatgg 2340
aaaaaaattt ttattttaat ggttatgtaa aataagttat gggttgtatt tagattttaa 2400
gttatagtta gttagttttt ggattatttt ttattttata tatttgaatg ttgtattttt 2460
gtattagtag ttaaaagatt aataaaggag agttttatta 2500

## Beschreibung

Die vorliegende Erfindung betrifft Diagnose und Therapie menschlicher Krebserkrankungen. Sie ermöglicht über die Untersuchung von DNA-Methylierungszuständen eine Bestimmung des spezifischen Expressionsprofils Tumor-assoziierter Antigene und erlaubt so eine individualisierte Immuntherapie.

### Hintergrund der Erfindung

Die von Ehrlich und Burnet postulierte Immunüberwachung geht davon aus, dass genetische Veränderungen im Rahmen der Entstehung eines malignen Tumors zur Expression aberranter Genprodukte führt, die von dem Immunsystem als "fremd" erkannt werden. Der Nachweis tumorspezifischer Antigene bei spontanen Tumoren ist bisher nur sporadisch gelungen, während kein Zweifel besteht, dass normale und maligne Zellen des gleichen histogenetischen Ursprungs quantitative Unterschiede in der Expression von tumorspezifischen Antigenen (tumor-associated antigenes - TAA) aufweisen. TAA können reexprimierte fötale Antigene, proliferations-assoziierte Antigene oder Neoantigene darstellen. Fragmente dieser Proteine werden zusammen mit MHC Klasse-I Molekülen auf der Zelloberfläche exprimiert und in vielen Fällen von MHC-restingierten T-Lymphozyten erkannt und beseitigt. Diese immunologischen Effektoren bleiben aber in der Realität häufig ineffizient, wofür es unterschiedliche Erklärungsversuche gibt. So kommt es oft zu einer Herunterregulation der MHC-Expression. Es gibt zudem viele weitere Proteine, die bei dem Prozess des durch MHC-restringierte T-Zellen induzierten Zelltods eine wichtige Rolle spielen. Neue Ansätze in der Tumortherapie versuchen, die körpereigene Abwehr durch eine Vakzinierung gegen Tumor-assoziierte Antigene zu induzieren oder zu verstärken. Als Impfstoffe kommen dabei maligne Tumorzellen, Peptid- oder Proteinbestandteile Tumor-assoziierter Antigene und entsprechende DNA-Sequenzen in Betracht (zur Übersicht: Dermime S, Armstrong A, Hawkins RE, Stern PL: Cancer vaccines and immunotherapy. Br Med Bull 2002;62:149-62).

Für eine erfolgreiche Vakzinierung ist es aus mehreren Gründen erforderlich, das individuelle Expressionsprofil möglichst vieler TAA in den Krebszellen zu kennen. So ist die induzierte Immunantwort nur dann wirksam, wenn die entsprechenden Antigene in ausreichender Anzahl in den Krebszellen exprimiert werden. Zudem ist es sinnvoll, die Vakzinierung gleichzeitig gegen mehrere Antigene zu richten. Denn so ist die Wahrscheinlichkeit gering, dass Krebszellvarianten, die ein bestimmtes Antigen nicht mehr exprimieren, der Immunantwort entgehen. Für eine erfolgreiche Vakzinierungsstrategie spielt aber auch die Expression anderer Proteine als der TAA eine Rolle. So kann die Immunantwort nur dann wirksam sein, wenn in den Tumorzellen ausreichend MHC I -Moleküle hergestellt werden, die Fragmente der TAA an der Zelloberfläche präsentieren. Für eine individuelle Immuntherapie ist es zudem von Bedeutung, welche für die T-Zell-Aktivierung erforderlichen Costimulatoren und welche an dem T-Zell-vermittelten Zelltod beteiligten Proteine in den Krebszellen exprimiert werden.

Ein schnelles, routinemäßig anwendbares und nichtinvasives Verfahren zur Expressionsanalyse von Tumor-assoziierten Antigenen und von für die Vakzinierung bedeutsamen Cofaktoren steht jedoch bisher nicht zur Verfügung. Beschrieben sind mehrere Microarray-basierte Verfahren, die von zellulärer RNA ausgehen (Mathiassen et al.: Tumor-associated antigens identified by mRNA expression profiling induce protective anti-tumor immunity. Eur J Immunol. 2001 Apr;31(4):1239-46; Zammatteo et al.: DNA microarray to monitor the expression of MAGE-A genes. Clin Chem. 2002 Jan; 48(1):25-34; Weinschenk et al.: Integrated functional genomics approach for the design of patient-individual antitumor vaccines. Cancer Res. 2002 Oct 15;62(20):5818-27). Diese dort beschriebenen Verfahren weisen aber mehrere Nachteile auf, die ihre Anwendbarkeit in der Praxis beschränken. Ein prinzipielles Problem ist dabei die extreme Instabilität der RNA. Die RNA wird außerhalb von Zellen sofort degradiert. Für eine herkömmliche Expressionsanalyse ist daher immer eine Gewebeprobe erforderlich; ein einfacher Nachweis in Körperflüssigkeiten ist kaum möglich. Die Entnahme einer Biopsie ist für den Patienten unangenehm und mit einem erhöhten Gesundheitsrisiko verbunden. Zudem ist eine Gewebeprobe oft nicht möglich, etwa wenn die Tumor noch nicht lokalisiert ist (Vgl. Mathiassen et al 2001; a.a.o., S. 1243, rechte Spalte, 2. Absatz). Ein weiterer Nachteil der beschriebenen Verfahren liegt darin, dass die verwendete PCR sowohl RNA wie auch genomische DNA amplifiziert. Es besteht daher die Gefahr falsch-positiver Resultate (Zammatteo et al. 2002, a.a.o., S.33 rechte Spalte, 1.Absatz).

Diese Probleme werden durch das erfindungsgemäße Verfahren gelöst. Dieses untersucht die DNA-Methylierungszustände von TAA und von für die Vakzinierung bedeutsamen methylierungsregulierten Cofaktoren und erlaubt so eine Aussage über das spezifische Expressionsprofil von Krebszellen. Als Ausgangsmaterial wird die im Vergleich zur RNA stabile DNA verwendet, so dass auch ein Nachweis aus Körperflüssigkeiten in Betracht kommt. Daneben ist natürlich auch eine Nachweis in Biopsien möglich.

Der Erfindung liegt die Erkenntnis zugrunde, dass die Expression vieler TAA mit einer Methylierung von Cytosinpositionen im Promotorbereich der Gene in Zusammenhang steht. Dies gilt insbesondere für die große Gruppe der Cancer-Testis Antigene (CTA). Diese sind für eine Immuntherapie besonders interessant, da sie fast ausschließlich in Krebszellen exprimiert werden. In gesundem Gewebe kommen sie nur in Testis und in einigen Fällen in der Placenta vor, also an Orten, die Immunzellen nicht zugänglich sind. Bei einer Vakzinierung gegen Cancer-Testis Antigene ist daher die Gefahr einer Autoimmunreaktion gering (zur Übersicht: Kirkin et al.: Cancer/testis antigens: structural and immunobiological properties. Cancer Invest. 2002; 20(2):222-36). Sigalotte et al. (2002, a.a.o) gehen von einer umfassenden Methylierungsregulation aller Cancer-Testis Antigene aus. Denn demethylierende Agenzien verursachen die Expression einer großen Anzahl von Cancer-Testis Antigenen (zur Übersicht: Kirkin et al. a.a.o., S. 222). Zudem gibt es Methylierungsanalysen für Promotoren mehrerer MAGE-Gene sowie anderer Cancer-Testis Antigene, die eine Expressionsregulation über Methylierung nahelegen. Die Untersuchnungen erfolgten teilweise enzymatisch (so etwa für MAGE 2,3 und 4: Sigalotti et al. 2002, a.a.o.), teilweise über Bisulfitierung (MAGE-A1, MAGE-A3, MAGE-B2: Jang et al.: Activation of melanoma antigen tumor antigens occurs early in lung carcinogenesis. Cancer Res. 2001 Nov 1;61(21):7959-63, MAGE-A1: Suyama et al.: The MAGE-A1 gene expression is not determined solely by methylation status of the promoter region in hematological malignancies. Leuk Res. 2002 Dec;26(12):1113-8; MAGE 3: Sigalotti et al. 2002, a.a.o.).

Neben den CTA sind auf viele andere TAA methylierungsreguliert. Dies ist etwa für Proteinase 3, WT1, MUC-1, CEA, HER2, PSMA, Alpha-Fetoprotein, Survivin und G250 gezeigt (siehe etwa: MUC-1: Zrihan-Licht et al.: MUC1: DNA methylation status of the MUC1 gene coding for a breastcancer-associated protein. Int J Cancer 1995 Jul 28; 62 (3): 245-51; Proteinase3: Lubbert et al.: Cytosine demethylation of the proteinase-3/myeloblastin primary granule protease gene during phagocyte development. Leukemia 1999 Sep; 13(9):1420-7; WT1: Loeb and Sukumar: The role of WT1 in oncogenesis: tumor suppressor or oncogene? J Hematol 2002 Aug; 76(2):117-26; CEA: Tran et al.: Correlation of DNA hypomethylation with expression of carcinoembryonic antigen in human colon carcinioma cells. Cancer Res 1988 Oct 15; 48(20):5674-9).

Neben TAA sind weitere für die Vakzinierung bedeutsame Cofaktoren methylierungsreguliert. So ist bekannt, dass eine Methylierung für ein Herunterregulieren des MHC I in Krebszellen verantwortlich sein kann (siehe etwa: Serrano et al.: Reexpression of HLA class I antigens and restoration of antigen-specific CTL response in melanoma cells following 5-aza-2'desoxycytidine-treatment. Int J. Cancer. 2001 Oct 15, 94(2):243-51; Nie: DNA hypermethylation is a mechanism for loss of expression of the HLA class I genes in human esophageal squamous cell carcinomas. Carcinogenesis. 2001 Oct; 22(10):1615-23; Rubocki et al.: Locus-specific de novo methylation down-regulates MHC class I in S49 lymphomas. Immunogenetics 1996; 43(1-2):63-7).

Zur Bestimmung des Methylierungsstatus der oben genannten Antigene und Cofaktoren gibt es unterschiedliche Möglichkeiten. Die Methoden zur Methylierungsanalyse arbeiten im wesentlichen nach zwei unterschiedlichen Prinzipien. Zum einen werden methylierungsspezifische Restriktionsenzyme benutzt, zum anderen erfolgt eine selektive chemische Umwandlung von nicht-methylierten Cytosinen in Uracil (Bisulfit-Behandlung). Die enzymatisch oder chemisch vorbehandelte DNA wird dann meist amplifiziert und kann auf unterschiedliche Weise detektiert werden (zur Übersicht: WO 02/072880). Ein schlagkräfiges Verfahren ist dabei die sogenannte methylierungssensitive PCR (Herman et al.: Methylation-specific PCR: a novel PCR assay for methylation status of CpG islands. Proc Natl Acad Sci U S A. 1996 Sep 3;93(18):9821-6). Dabei werden Primer verwendet, die spezifisch nur an Positionen der bisulfitierten Sequenz binden, die vorher entweder methyliert (oder bei umgekehrtem Ansatz: unmethyliert) waren. Ein vergleichbar sensitives Verfahren ist die sogenannte "Heavy-Methyl"-Methode. Dabei wird eine spezifische Amplifizierung nur der ursprünglich methylierten (bzw. unmethylierten) DNA durch Einsatz von methylierungsspezifischen Blocker-Oligomeren erreicht (zur Übersicht: WO 02/072880). Beide Verfahren sind als Real-Time-Varianten anwendbar, die es ermöglichen den Methylierungsstatus weniger Positionen direkt im Verlauf der PCR nachzuweisen, ohne dass eine nachfolgende Analyse der Produkte erforderlich wäre ("MethylLight" - WO00/70090). Ausführungsformen dieser Varianten sind Lightcycler und Taqman. Weitere bekannte Verfahren zur Methylierungsanalyse sind MS-SnuPE (Gonzalgo ML, Jones PA: Rapid quantitation of methylation differences at specific sites using methylation-sensitive single nucleotide primer extension (Ms-SNuPE) Nucleic Acids Res 1997 Jun 15;25(12):2529-31) und COBRA (Xiong Z, Laird PW: COBRA: a sensitive and quantitative DNA methylation assay. Nucleic Acids Res 1997 Jun 15;25(12):2532-4).

Mittels dieser Methoden ist es auch möglich, das Expressionsprofil von Tumoren anhand von Körperflüssigkeiten zu bestimmen. Denn anders als die instabile RNA ist DNA oft in Körperflüssigkeiten anzutreffen. Bei destruktiven pathologischen Prozessen wie Krebserkrankungen ist die DNA-Konzentration im Blut sogar erhöht (siehe etwa: Anker et al.: Detection of circulating tumour DNA in the blood (plasma/serum) of cancer patients. Cancer Metastasis Rev. 1999;18(1):65-73; Maebo: Plasma DNA level as a tumor marker in primary lung cancer. Jap J Thoraic Dis 28: 1085-1091, 1990 Aug;28(8):1085-91). Eine Krebsdiagnostik über eine Methylierungsanalyse von in Körperflüssigkeiten befindlicher Tumor-DNA ist also möglich und schon mehrfach beschrieben (siehe etwa: Sputum/Lungenkrebs: Palmisano et al.: Predicting lung cancer by detecting aberrant promoter methylation in sputum. Cancer Res. 2000 Nov 1;60(21):5954-8; Blut/ Leberkrebs: Wong et al.: Frequent p15 promoter methylation in tumor and peripheral blood from hepatocellular carcinoma patients. Clin Cancer Res. 2000 Sep;6(9):3516-21, Serum/ "Head and Neck cancer":
Sanchez-Cespedes et al.: Gene promoter hypermethylation in tumors and serum of head and neck cancer patients. Cancer Res. 2000 Feb 15;60(4):892-5;
Urin/Ejakulat/Prostata-Krebs: Cairns et al.: Molecular detection of prostate cancer in urine by GSTP1 hypermethylation. Clin Cancer Res. 2001 Sep;7(9):2727-30).

Aufgabe der vorliegenden Erfindung ist es, die Nachteiel des Standes der Technik zu überwinden und eien verbesserte Therapiemöglichkeit für Tumore zur Verfügung zu stellen. Die Aufgabe wird durch ein Verfahren zur Bestimmung der Angriffspunkte einer Tumor-Immuntherapie gelöst, wobei man:
a) eine DNA-Probe entnimmt,
b) den Methylierungszustand mindestens jeweils einer CpG-Position innerhalb mehrerer Tumor-assoziierter Antigene identifiziert, wobei die Methylierungszustände zusammen ein Methylierungsmuster bilden,
c) aus dem gewonnenen Methylierungsmuster folgert, welche der Tumor-assoziierten Antigene die geeignetsten Angriffspunkte für eine Immuntherapie sind.

Bevorzugt ist es dabei, dass die zu untersuchenden Tumor-assoziierten Antigene folgender Gruppe entnommen sind:
MAGE-A1 - MAGE-A6, MAGE-A8 - MAGE-A12,
MAGE-B1- MAGE-B6,
MAGE-C1 - MAGE-C3;
GAGE-1 - GAGE-8; PAGE-1 - PAGE-4; XAGE-1 - XAGE-3;
LAGE-1a (1S), LAGE-1b(1L), NY-ESO-1
SSX-1 - SSX-5
BAGE, SCP-1, PRAME, SART-1, SART-3, CTp11, TSP50, CT9/BRDT
Proteinase 3; WT1, MUC-1, CEA; HER2, PSMA; Alpha-Fetoprotein; Survivin; G250; Reverse Transkriptase Telomerase (hTERT)

Dabei ist ferner bevorzugt, dass man neben Tumor-assoziierten Antigenen auch MHC I oder weitere für die Vakzinierung bedeutsame, methylierungsregulierte Cofaktoren untersucht.

Besonders ist es erfindungsgemäß bevorzugt, dass man als Proben Serum oder andere Körperflüssigkeiten verwendet.

Weiterhin ist es erfindungsgemäß bevorzugt, dass zur Untersuchung der Methylierungszustände eine chemische Behandlung der DNA mit einem Bisulfit (=Disulfit, Hydrogensulfit) erfolgt.

Dabei ist ganz besonders bevorzugt, dass nach der chemischen Behandlung eine Amplifikation der DNA erfolgt.

Bevorzugt ist erfindungsgemäß aber auch, dass man zur Amplifikation MSP oder Heavy Methyl oder eine Kombinationen dieser verwendet.

Weiterhin ist bevorzugt dass die Identifizierung des Methylierungsstatus über mindestens ein Reporter-Oligonukleotid oder Peptid-Nukleinsäure (PNA) Oligomer erfolgt, das an ein 5'-CG-3' Dinukleotid oder ein 5'-TG-3' Dinukleotid oder ein 5'-CA-3' Dinukleotid hybridisiert und das die Amplifikation der Zielsequenz anzeigt.

Dabei ist besonders bevorzugt, dass das Reporter-Oligomer eine Fluoreszenzmarkierung trägt. Bevorzugt ist aber auch, dass man zur Detektion ein Taqman-Assay durchführt. Weiterhin bevorzugt ist auch, dass man zusätzlich zu dem Reporter-Oligonukleotid ein weiteres mit einem Fluoreszenzfarbstoff markiertes Oligomer verwendet, das unmittelbar benachbart zu dem Reporteroligonukleotid hybridisiert und diese Hybridisierung mittels Fluoreszenz Resonanz Energietransfer nachweisbar ist. Hierbei ist insbesondere bevorzugt, dass man zur Detektion ein Lightcycler Assay durchführt.

Weiterhin ist ein Verfahren erfindunsggemäß bevorzugt, bei dem die Identifizierung durch Längenmessung der amplifizierten zu untersuchenden DNA erfolgt, wobei Methoden zur Längenmessung Gelelektrophorese, Kapillargelelektrophorese, Chromatographie (z.B. HPLC), Massenspektrometrie und andere geeignete Methoden umfassen.

Dabei ist auch bevorzugt, dass man die Amplifikate selbst für die Detektion mit einer nachweisbaren Markierung versieht.

Besonders bevorzugt ist es erfindungsgemäß auch, dass ggf. die Identifizierung des Methylierungsstatus durch Hybridisierung an ein Oligomer-Array erfolgt, wobei Oligomere Nukleinsäuren oder in ihren Hybridisierungseigenschaften ähnliche Moleküle wie PNAs sind.

Dabei ist besonders bevorzugt, dass die Oligomere über einen 12 bis 22 Basen langen Abschnitt an die zu analysierende DNA hybridisieren und sie mindestens ein CG, TG oder CA Dinukleotid umfassen.

Es ist auch bevorzugt, dass die Amplifikation von mindestens zwei Fragmenten gleichzeitig in einem Reaktionsgefäß erfolgt.

Ein weitere Gegenstand der vorliegenden Erfindung ist ein Nukleinsäuremolekül, umfassend eine Sequenz mit einer Länge von mindestens 18 Basen gemäß einer der Sequenzen ausgewählt aus der Gruppe umfassend SEQ ID Nr. 48-235 oder komplementärer Sequenzen.

Gegenstand der vorliegenden Erfindung sind auch Oligonukleotide oder Peptid-Nukleinsäure(PNA)-Oligomere, die mindestens eine Basensequenz mit einer Länge von 10 Nukleotiden umfasst, die an eine chemisch vorbehandelte DNA, gemäß der SEQ ID Nr. 48 bis SEQ ID Nr. 235 hybridisiert oder mit ihr identisch ist.

Ein weiterer Gegenstand der vorliegenden Erfindung ist eine Anordnung, insbesondere Array von mehreren Oligonukleotiden oder Peptid-Nukleinsäure (PNA)-Oligomeren, dadurch gekennzeichnet, dass mindestens ein Oligomer gemäß Anspruch 19 an eine feste Phase gebunden ist.

Ein weiterer Gegenstand der vorliegenden Erfindung ist ein Kit, der aus einem Bisulfit enthaltenen Reagenz und mindestens einem Oligonukleotid gemäß Anspruch 18 besteht, sowie optional Blocker und Reporteroligomere, Standardreagenzien zur Durchführung von MS-SNuPE, MSP, MethyLight, Heavy-Methyl, COBRA oder Nukleinsäuresequenzierung sowie eine Anleitung zur Durchführung der erfindungsgemäßen Methode enthält.

Schließlich ist ein weitere Gegenstand der vorliegenden Erfindung ein Verfahren zur Behandlung von Tumorerkrankungen, dadurch gekennzeichnet, dass man gemäß den Ansprüchen 1 bis 17 die Angriffspunkte für eine Tumor-Immuntherapie bestimmt und anschließend eine Vakzinierung gegen die geeignetesten Tumor-assoziierten Tumorantigene erfolgt. Dabei ist es erfindungsgemäß besonders bevorzugt, dass man als Impfstoffe Tumorzellen, Peptid- oder Proteinbestandteile Tumor-assoziierter Antigene oder entsprechende DNA-Sequenzen verwendet.

### Beschreibung

Das erfindungsgemäße Verfahren erlaubt die gleichzeitige Bestimmung von DNA-Methylierungszuständen mehrerer Tumorassozierter Antigene und anderer für die Vakzinierung bedeutsamen methylierungsregulierter Cofaktoren. Hierdurch lassen sich Angriffspunkte einer Tumor-Immuntherapie bestimmen. Das erfindungemäße Verfahren läuft dabei in folgenden Schritten ab:
a) es wird eine DNA-Probe entnommen
b) der Methylierungszustand mindestens jeweils einer CpG-Position innerhalb mehrerer Tumor-assoziierter Antigenen wird identifiziert, wobei die Methylierungszustände zusammen ein Methylierungsmuster bilden, und
c) aus dem gewonnenen Methylierungsmuster wird gefolgert, welche der Tumor-assoziierten Antigene die geeignetsten Angriffspunkte für eine Immuntherapie sind.

Als zu untersuchende Antigene eignen sich besonders folgende Cancer-Testis Antigene und überexprimierte Selbst-Antigene (zur Übersicht: Kirkin et al. 2002, a.a.o. S. 223 mit weiteren Nachweisen):
Cancer-Testis-Antigene:
MAGE-A1 - MAGE-A6, MAGE-A8 - MAGE-A12,
MAGE-B1- MAGE-B6,
MAGE-C1 - MAGE-C3;
GAGE-1 - GAGE-8; PAGE-1 - PAGE-4; XAGE-1 - XAGE-3;
LAGE-1a (1S), LAGE-1b(1L), NY-ESO-1
SSX-1 - SSX-5
BAGE, SCP-1, PRAME, SART-1, SART-3, CTp11, TSP50, CT9/BRDT

### Überexprimierte Selbst-Antigene:

### Proteinase 3; WT1, MUC-1, CEA; HER2, PSMA; Alpha-Fetoprotein; Survivin; G250; Reverse Transkriptase Telomerase (TERT)

Die Untersuchung der Cancer-Testis Antigene erfolgt bevorzugt folgendermaßen: Zunächst wird dem Patienten eine Probe entnommen, die genomische DNA enthält. Die DNA wird isoliert, bisulfitiert oder mit methylierungssensitiven Restriktionsenzymen verdaut und schließlich amplifiziert. Danach wird der Methylierungszustand von CpG Positionen in der genomischen DNA der TAA bestimmt und hieraus auf ihre Expression geschlossen. Durch Untersuchung mehrerer Cancer-Testis Antigene kann das spezifische Expressionsprofil des Tumors bestimmt werden, und so ein Impfstoff für eine individuelle Immuntherapie entwickelt werden.

Als Ausgangsmaterial können Gewebeproben, aber auch Körperflüssigkeiten, insbesondere Serum, dienen. Denkbar ist es auch, die DNA aus Sputum, Stuhl, Urin oder Gehirn-Rückenmarks-Flüssigkeit zu isolieren. Die DNA-Extraktion erfolgt nach Standardmethoden, aus Blut etwa unter Verwendung des Qiagen UltraSens DNA Extraktions-Kits. Die isolierte DNA kann dann mit methylierungssensitiven Restriktionsenzymen verdaut werden. In einer bevorzugten Ausführungsform wird die DNA jedoch bisulfitiert, etwa durch Umsatz mit Natriumbisulfit (zur Übersicht: WO 02/072880 S. 1-4). Hierzu stehen unterschiedliche Verfahren zur Verfügung. Geeignet ist insbesondere die "Agarose-Bead"-Methode. Hierbei wird die zu untersuchende DNA in einer Agarose-Matrix eingeschlossen. Diffusion und Renaturierung der DNA (Bisulfit reagiert nur an einzelsträngiger DNA) werden so verhindert, Fällung- und Reinigungsschritte werden durch eine schnelle Dialyse ersetzt (Olek et al.: A modified and improved method for bisulphite based cytosine methylation analysis. Nucleic Acids Res. 1996 DEC 15;24(24):5064-6). Geeignet ist auch ein neue Variante, bei der die Behandlung der DNA in Gegenwart eines Radikalfängers und eines denaturierenden Reagenzes, bevorzugt ein Oligethylenglykoldialkylether oder beipielsweise Dioxan, erfolgt (Vgl. DE 101 54 317 A1; DE 100 29 915). Vor der Amplifikation werden die Reagenzien entweder durch Waschen im Falle der Agarosemethode oder über ein DNA-Aufreinigungsverfahren (etwa Fällung oder Bindung an eine Festphase, Membran) entfernt oder aber einfach durch Verdünnung in einen Konzentrationsbereich gebracht, der die Amplifikation nicht mehr signifikant beeinflußt.

Die nachfolgende Amplifikation der DNA erfolgt bevorzugt mittels PCR, es ist jedoch auch die Verwendung andere Amplifikationsverfahren, etwa isothermer Methoden, denkbar. Die zu untersuchenden CpG-Positionen befinden sich bevorzugt im Promotorbereich der Gene. Die Bestimmung möglicher Primer, Sonden und Reaktionskonditionen erfolgt nach dem Stand der Technik ausgehend von den Bisulfit-Sequenzen der Gene (Seq ID 48-235). MSP-Primer enthalten mindestens ein 5'- CpG -3' Dinukleotid; bevorzugt sind Primer, die mindestens drei 5'- CpG-3'Positionen tragen, von denen mindestens eine am 3' Ende lokalisiert ist (bzw. 5'-TG-3' oder 5'-CA-3'-Dinukleotide für die entsprechenden unmethylierten Sequenzen bzw. die Gegenstränge) . Für die Ausführungsform des Heavy Methyl benutzt man in der Regel methylierungsunspezifische Primer in Gegenwart mindestens eines weiteren Blocker-Oligonukleotids, das an ein 5'-CG-3'(bzw. 5'-TG-3'-Dinukleotid oder 5'-CA-3')-Dinukleotid bindet, und so die Amplifikation der Hintergrund DNA verhindert (WO 02/072880, S.11-13). Die Ausführungsform kann über die Auswahl der Polymerase oder über die Modifikation der Blockeroligomere so ausgestaltet sein, dass ein Abbau oder eine Verlängerung der Blocker minimiert wird. So kann ein Abbau verhindert werden entweder durch die Verwendung einer Polymerase ohne Nukleaseaktivität oder bevorzugt durch Einsatz modifizierter Oligonukleotide, etwa solcher, die am 5'-Ende Thioatbrücken aufweisen und daher gegen einen Abbau resistent sind. Eine Verlängerung der Primer kann durch den Einsatz von 3'-Desoxyoligonukleotiden oder aber an der 3'-Position anderweitig funktionalisierten Oligonukleotiden, beipielsweise 3'-O-Acetyloligonukleotiden, verhindert werden. Eine bevorzugte Variante ist dabei der Einsatz von PNA (Peptide Nucleic Acid) Oligomeren. Diese werden von der Polymerase weder abgebaut und verlängert. Verfahren zum Design und der Synthese von DNA-Oligomeren sind Stand der Technik.

Die Detektion der Amplifikate erfolgt bevorzugt über die bekannten Verfahren, etwa über Methoden der Längenmessung wie Gelelektrophorese, Kapillargelelektrophorese und Chromatographie (z.B. HPLC). Auch Massenspektrometrie, MsSNuPE (Gonzalgo & Jones, Nucleic Acids Res. 25:2529-2531, 1997) und Methoden zur Sequenzierung wie die Sanger-Methode, die Maxam-Gilbert-Methode und Sequencing by Hybridisation (SBH) können verwendet werden. Besonders bevorzugt ist die Verwendung von Real-Time-Varianten (Heid et al.: Real time quantitative PCR. Genome Res. 1996 Oct;6(10):986-94, US Patent No. 6,331,393 "Methyl-Light"). Dabei wird die Amplifikation in Gegenwart eines fluoreszenzmarkierten Reporteroligonukleotid durchgeführt, das an ein 5'-CG-3'-Dinukleotid (bzw. 5'-TG-3'oder 5'-CA-3'-Dinukleotid) hybridisiert. Das Reporteroligonukleotid bindet dabei bevorzugt an die zu untersuchende DNA und zeigt deren Amplifikation durch Zunahme oder Abnahme der Fluoreszenz an. Dabei ist es besonders vorteilhaft, wenn die Fluoreszenzveränderung direkt zur Analyse benutzt wird und aus dem Fluorenzenzsignal auf einen Methylierungszustand geschlossen wird. Eine bevorzugte Variante ist dabei das "Taqman"-Verfahren. In einer anderen bevorzugten Ausführungsform wird ein zusätzliches floureszenzmarkiertes Oligomer verwendet, das in unmittelbarer Nähe zu dem ersten Reporteroligonukleotid hybridisiert und sich diese Hybridisierung mittels Fluoreszenz Resonanz Energietransfer nachweisen lässt ("Lightcycler"-Verfahren).

Ein weiteres Detektionsverfahren besteht in der Analyse der Amplifikate mittels Hybridisierung an Oligomer-Arrays, wobei Oligomere Nukleinsäuren oder in ihren Hybridisierungseigenschaften ähnliche Moleküle wie PNAs sein können (ein Überblick über Array-Technologie befindet sich in der Extraausgabe von: Nature Genetics Supplement, Volume 21, January 1999). Auf einem solchen Array können die verschiedenen Oligomere auf einer Festphase in Form eines rechtwinkligen oder hexagonalen Gitters angeordnet sein. Die Festphasenoberfläche ist bevorzugt aus Silizium, Glas, Polystyrol, Aluminium, Stahl, Eisen, Kupfer, Nickel, Silber oder Gold zusammengesetzt. Jedoch sind auch Nitrocellulose und Kunststoffe wie Nylon, die in Form von Pellets oder auch als Harz-Matrizes existieren können, möglich. Die etwa fluoreszenzmarkierten Amplifikate werden an die gebundenen Oligomers hybridisiert und die nicht gebundenen Fragmente entfernt. Vorteilhaft ist es dabei, wenn die Oligomere über einen 12-22 Basen langen Abschnitt an die zu analysierende DNA hybridisieren und sie mindestens ein CG, TG oder CA Dinukleotid umfassen. Die Fluoreszenz-Signale können gescannt und mit Software-Programmen verarbeitet werden (Siehe etwa: Adorjan et al., Tumour class prediction and discovery by microarray-based DNA methylation analysis. Nucleic Acids Res. 2002 Mar 1;30(5):e21).

Eine bevorzugte Ausführungsform der Erfindung ist es, mehrere Fragmente gleichzeitig mittels einer Multiplex-PCR zu amplifizieren. Bei deren Design muss darauf geachtet werden, dass nicht nur die Primer, sondern auch die weiteren eingesetzten Oligonukleotide nicht zueinander komplementär sein dürfen, so dass eine hochgradige Multiplexierung in diesem Fall schwieriger ist als in üblich. Jedoch hat man bei bisulfit-behandelter DNA den Vorteil, dass aufgrund des unterschiedlichen G und C-Gehaltes der beiden DNA-Stränge ein Forward-Primer niemals auch als Reverse-Primer fungieren kann, was die Multiplexierung wiederum erleichtert und den oben beschriebenen Nachteil im wesentlichen ausgleicht. Die Detektion der Amplifikate ist wiederum über unterschiedliche Verfahren möglich. Denkbar ist dabei etwa die Verwendung von Real-Time-Varianten. Für Amplifikationen von mehr als vier Genen empfiehlt es sich aber, die Amplifikate auf andere Weise zu detektieren. Bevorzugt ist dabei eine Analyse über Arrays (s.o.).

Mit den oben beschriebenen Verfahren kann das Expressionsprofil mehrerer TAA bestimmt werden. Es ist erfindungsgemäß bevorzugt, zudem auch die Expression von MHC-Genen und von weiteren für die Vakzinierung bedeutsamen, methylierungsregulierten Cofaktoren zu untersuchen. Aus den erzeugten Informationen kann geschlossen werden, ob sich ein Patient für eine Vakzinierung mit einem bestimmten Impfstoff eignet. Zudem kann nach dem individuellen Expressionsprofil eines Patienten eine individualisierte Vakzine zusammengestellt werden, die neben den exprimierten TAA auch Faktoren enthalten kann, die eventuell herunterregulierte Cofaktoren ersetzen.

Gegenstand der vorliegenden Erfindung ist auch ein Kit, der aus einem Bisulfit enthaltenden Reagenz und Primern besteht, die unter stringenten Bedingungen an einen 18 bp langes Fragment einer der Sequenzen 48-235 binden, sowie optional Blocker und Reporteroligonukleotide und eine Anleitung zur Durchführung der erfindungsgemäßen Methode enthält. In einer weiteren Ausführungsform enthält der Kit weiterhin Standardreagenzien für die Durchführung einer oder mehrerer der folgenden Techniken: MS-SNuPE, MSP, MethyLight, Heavy-Methyl, COBRA oder Nukleinsäuresequenzierung.

Erfindungsgemäß ist auch ein Verfahren zur Behandlung von Tumorerkrankungen, wobei zunächst nach der oben ausgeführten Methodik Angriffspunkte für eine Tumor-Immuntherapie bestimmt werden und anschließend eine Vakzinierung gegen die geeignetesten Tumor-assoziierten Tumorantigene erfolgt. Als Impfstoffe können Tumorzellen, Peptid- oder Proteinbestandteile Tumor-assoziierter Antigene oder entsprechende DNA-Sequenzen verwendet werden.

Erfindungsgemäß sind auch die aufgeführten Bisulfit-Sequenzen der Tumor-assoziierten Antigene. Über die Sequenzen lassen sich die Expressionsprofile der Gene bestimmen. Unten sind für 47 aufgeführte Tumor-assoziierten Antigene insgesamt 235 Sequenzen angegeben. Dabei beziehen sich die Sequenzen 1-47 auf die genomische DNA im Promotorbereich der Gene. Bei den anderen Sequenzen handelt es sich um "Bisulfitsequenzen". Dabei ist zu beachten, dass die genomische DNA in mindestens zwei unterschiedlichen Methylierungszuständen vorkommen kann. Im ersten Fall sind alle CpG-Positionen methyliert, im entgegengesetzten Fall alle CpG-Positionen unmethyliert. Diese unterschiedlich methylierten DNA-Sequenzen führen zu unterschiedlichen Bisulfitsequenzen. Weiter ist zu berücksichtigen, dass bei der Bisulfitierung eines DNA-Doppelstranges zwei Bisulfitstränge entstehen, die zueinander nicht mehr komplementär sind. Insgesamt ergeben sich also pro genomischer Sequenz vier unterschiedliche, nicht komplementäre Bisulfitsequenzen. Bei den angegebenen Sequenzen 48-141 handelt es sich dabei um die ursprünglich voll methylierten DNA, wobei abwechselnd immer die Plus-Strang- und dann die Minus-Strang-Sequenzen angegeben sind. Bei den Sequenzen 142-235 handelt es sich um die ursprünglich unmethylierte DNA, wiederum mit jeweils abwechselnden Plus-Strang- und Minus-Strang-Sequenzen.

### Sequenz-Auflistung

Die Namen entsprechen den im Sequenz-Protokoll angegebenen Sequenzen.

### Gennomische Sequenzen

- 1: name=brdt
- 2: name=ca9,
- 3: name=ctag1,
- 4: name=erbb2,
- 5: name=gage1,
- 6: name=gage2
- 7: name=gage3
- 8: name=gage4
- 9: name=gage5
- 10: name=gage6
- 11: name=gage7
- 12: name=gage8
- 13: name=gagec1=page4
- 14: name=gaged3=xage2
- 15: name=page1
- 16: name=lage-1a
- 17: name=magea1,
- 18: name=magea10
- 19: name=magea12,
- 20: name=magea2
- 21: name=magea3
- 22: name=magea4
- 23: name=magea5
- 24: name=magea6
- 25: name=magea8
- 26: name=magea9
- 27: name=mageb1
- 28: name=mageb2
- 29: name=mageb3
- 30: name=mageb6
- 31: name=magec1
- 32: name=magee1
- 33: name=prame
- 34: name=psma1
- 35: name=sart1
- 36: name=sart3
- 37: name=CTp11
- 38: name=ssx1
- 39: name=ssx2
- 40: name=ssx3
- 41: name=ssx4
- 42: name=ssx5
- 43: name=sycp1
- 44: name=tert
- 45: name=tsp50
- 46: name=wt1
- 47: name=xage1

### Aufmethylierte Bisulfit-Sequenzen

- 48: name=brdt, strand=1 sense bisulphite up
- 49: name=brdt, strand=1 antisense bisulphite up
- 50: name=ca9, strand=1 sense bisulphite up
- 51: name=ca9, strand=1 antisense bisulphite up
- 52: name=ctag1, strand=1 sense bisulphite up
- 53: name=ctag1, strand=1 antisense bisulphite up
- 54: name=erbb2, strand=1 sense bisulphite up
- 55: name=erbb2, strand=1 antisense bisulphite up
- 56: name=gage1, strand=1 sense bisulphite up
- 57: name=gage1, strand=1 antisense bisulphite up
- 58: name=gage2, strand=1 sense bisulphite up
- 59: name=gage2, strand=1 antisense bisulphite up
- 60: name=gage3, strand=1 sense bisulphite up
- 61: name=gage3, strand=1 antisense bisulphite up
- 62: name=gage4, strand=1 sense bisulphite up
- 63: name=gage4, strand=1 antisense bisulphite up
- 64: name=gage5, strand=1 sense bisulphite up
- 65: name=gage5, strand=1 antisense bisulphite up
- 66: name=gage6, strand=1 sense bisulphite up
- 67: name=gage6, strand=1 antisense bisulphite up
- 68: name=gage7, strand=1 sense bisulphite up
- 69: name=gage7, strand=1 antisense bisulphite up
- 70: name=gage8, strand=1 sense bisulphite up
- 71: name=gage8, strand=1 antisense bisulphite up
- 72: name=gagec1=page4, strand=1 sense bisulphite up
- 73: name=gagec1=page4, strand=1 antisense bisulphite up
- 74: name=gaged3=xage2, strand=1 sense bisulphite up
- 75: name=gaged3=xage2, strand=1 antisense bisulphite up
- 76: name=page1, strand=1 sense bisulphite up
- 77: name=page1, strand=1 antisense bisulphite up
- 78: name=lage-1a, strand=1 sense bisulphite up
- 79: name=lage-1a, strand=1 antisense bisulphite up
- 80: name=magea1, strand=-1 sense bisulphite up
- 81: name=magea1, strand=-1 antisense bisulphite up
- 82: name=magea10, strand=-1 sense bisulphite up
- 83: name=magea10, strand=-1 antisense bisulphite up
- 84: name=magea12, strand=-1 sense bisulphite up
- 85: name=magea12, strand=-1 antisense bisulphite up
- 86: name=magea2, strand=-1 sense bisulphite up
- 87: name=magea2, strand=-1 antisense bisulphite up
- 88: name=magea3, strand=-1 sense bisulphite up
- 89: name=magea3, strand=-1 antisense bisulphite up
- 90: name=magea4, strand=1 sense bisulphite up
- 91: name=magea4, strand=1 antisense bisulphite up
- 92: name=magea5, strand=-1 sense bisulphite up
- 93: name=magea5, strand=-1 antisense bisulphite up
- 94: name=magea6, strand=1 sense bisulphite up
- 95: name=magea6, strand=1 antisense bisulphite up
- 96: name=magea8, strand=1 sense bisulphite up
- 97: name=magea8, strand=1 antisense bisulphite up
- 98: name=magea9, strand=-1 sense bisulphite up
- 99: name=magea9, strand=-1 antisense bisulphite up
- 100: name=mageb1, strand=1 sense bisulphite up
- 101: name=mageb1, strand=1 antisense bisulphite up
- 102: name=mageb2, strand=1 sense bisulphite up
- 103: name=mageb2, strand=1 antisense bisulphite up
- 104: name=mageb3, strand=1 sense bisulphite up
- 105: name=mageb3, strand=1 antisense bisulphite up
- 106: name=mageb6, strand=1 sense bisulphite up
- 107: name=mageb6, strand=1 antisense bisulphite up
- 108: name=magec1, strand=1 sense bisulphite up
- 109: name=magec1, strand=1 antisense bisulphite up
- 110: name=magee1, strand=-1 sense bisulphite up
- 111: name=magee1, strand=-1 antisense bisulphite up
- 112: name=prame, strand=-1 sense bisulphite up
- 113: name=prame, strand=-1 antisense bisulphite up
- 114: name=psma1, strand=-1 sense bisulphite up
- 115: name=psma1, strand=-1 antisense bisulphite up
- 116: name=sart1, strand=1 sense bisulphite up
- 117: name=sart1, strand=1 antisense bisulphite up
- 118: name=sart3, strand=-1 sense bisulphite up
- 119: name=sart3, strand=-1 antisense bisulphite up
- 120: name=CTp11, strand=1 sense bisulphite up
- 121: name=CTp11, strand=1 antisense bisulphite up
- 122: name=ssx1, strand=1 sense bisulphite up
- 123: name=ssx1, strand=1 antisense bisulphite up
- 124: name=ssx2, strand=1 sense bisulphite up
- 125: name=ssx2, strand=1 antisense bisulphite up
- 126: name=ssx3, strand=-1 sense bisulphite up
- 127: name=ssx3, strand=-1 antisense bisulphite up
- 128: name=ssx4, strand=1 sense bisulphite up
- 129: name=ssx4, strand=1 antisense bisulphite up
- 130: name=ssx5, strand=-1 sense bisulphite up
- 131: name=ssx5, strand=-1 antisense bisulphite up
- 132: name=sycp1, strand=1 sense bisulphite up
- 133: name=sycp1, strand=1 antisense bisulphite up
- 134: name=tert, strand=-1 sense bisulphite up
- 135: name=tert, strand=-1 antisense bisulphite up
- 136: name=tsp50, strand=1 sense bisulphite up
- 137: name=tsp50, strand=1 antisense bisulphite up
- 138: name=WT1, strand=1 sense bisulphite up
- 139: name=WT1, strand=1 antisense bisulphite up
- 140: name=xage1, strand=1 sense bisulphite up
- 141: name=xage1, strand=1 antisense bisulphite up

#Heruntermethylierte Bisulfit-Sequenzen
- 142: name=brdt, strand=1 sense bisulphite down
- 143: name=brdt, strand=1 antisense bisulphite down
- 144: name=ca9, strand=1 sense bisulphite down
- 145: name=ca9, strand=1 antisense bisulphite down
- 146: name=ctag1, strand=1 sense bisulphite down
- 147: name=ctag1, strand=1 antisense bisulphite down
- 148: name=erbb2, strand=1 sense bisulphite down
- 149: name=erbb2, strand=1 antisense bisulphite down
- 150: name=gage1, strand=1 sense bisulphite down
- 151: name=gage1, strand=1 antisense bisulphite down
- 152: name=gage2, strand=1 sense bisulphite down
- 153: name=gage2, strand=1 antisense bisulphite down
- 154: name=gage3, strand=1 sense bisulphite down
- 155: name=gage3, strand=1 antisense bisulphite down
- 156: name=gage4, strand=1 sense bisulphite down
- 157: name=gage4, strand=1 antisense bisulphite down
- 158: name=gage5, strand=1 sense bisulphite down
- 159: name=gage5, strand=1 antisense bisulphite down
- 160: name=gage6, strand=1 sense bisulphite down
- 161: name=gage6, strand=1 antisense bisulphite down
- 162: name=gage7, strand=1 sense bisulphite down
- 163: name=gage7, strand=1 antisense bisulphite down
- 164: name=gage8, strand=1 sense bisulphite down
- 165: name=gage8, strand=1 antisense bisulphite down
- 166: name=gagec1=page4, strand=1 sense bisulphite down
- 167: name=gagec1=page4, strand=1 antisense bisulphite down
- 168: name=gaged3=xage2, strand=1 sense bisulphite down
- 169: name=gaged3=xage2, strand=1 antisense bisulphite down
- 170: name=page1, strand=1 sense bisulphite down
- 171: name=page1, strand=1 antisense bisulphite down
- 172: name=lagela, strand=1 sense bisulphite down
- 173: name=lagela, strand=1 antisense bisulphite down
- 174: name=magea1, strand=-1 sense bisulphite down
- 175: name=magea1, strand=-1 antisense bisulphite down
- 176: name=magea10, strand=-1 sense bisulphite down
- 177: name=magea10, strand=-1 antisense bisulphite down
- 178: name=magea12, strand=-1 sense bisulphite down
- 179: name=magea12, strand=-1 antisense bisulphite down
- 180: name=magea2, strand=-1 sense bisulphite down
- 181: name=magea2, strand=-1 antisense bisulphite down
- 182: name=magea3, strand=-1 sense bisulphite down
- 183: name=magea3, strand=-1 antisense bisulphite down
- 184: name=magea4, strand=1 sense bisulphite down
- 185: name=magea4, strand=1 antisense bisulphite down
- 186: name=magea5, strand=-1 sense bisulphite down
- 187: name=magea5, strand=-1 antisense bisulphite down
- 188: name=magea6, strand=1 sense bisulphite down
- 189: name=magea6, strand=1 antisense bisulphite down
- 190: name=magea8, strand=1 sense bisulphite down
- 191: name=magea8, strand=1 antisense bisulphite down
- 192: name=magea9, strand=-1 sense bisulphite down
- 193: name=magea9, strand=-1 antisense bisulphite down
- 194: name=mageb1, strand=1 sense bisulphite down
- 195: name=mageb1, strand=1 antisense bisulphite down
- 196: name=mageb2, strand=1 sense bisulphite down
- 197: name=mageb2, strand=1 antisense bisulphite down
- 198: name=mageb3, strand=1 sense bisulphite down
- 199: name=mageb3, strand=1 antisense bisulphite down
- 200: name=mageb6, strand=1 sense bisulphite down
- 201: name=mageb6, strand=1 antisense bisulphite down
- 202: name=magec1, strand=1 sense bisulphite down
- 203: name=magec1, strand=1 antisense bisulphite down
- 204: name=magee1, strand=-1 sense bisulphite down
- 205: name=magee1, strand=-1 antisense bisulphite down
- 206: name=prame, strand=-1 sense bisulphite down
- 207: name=prame, strand=-1 antisense bisulphite down
- 208: name=psma1, strand=-1 sense bisulphite down
- 209: name=psma1, strand=-1 antisense bisulphite down
- 210: name=sart1, strand=1 sense bisulphite down
- 211: name=sart1, strand=1 antisense bisulphite down
- 212: name=sart3, strand=-1 sense bisulphite down
- 213: name=sart3, strand=-1 antisense bisulphite down
- 214: name=CTp11, strand=1 sense bisulphite down
- 215: name=CTp11, strand=1 antisense bisulphite down
- 216: name=ssx1, strand=1 sense bisulphite down
- 217: name=ssx1, strand=1 antisense bisulphite down
- 218: name=ssx2, strand=1 sense bisulphite down
- 219: name=ssx2, strand=1 antisense bisulphite down
- 220: name=ssx3, strand=-1 sense bisulphite down
- 221: name=ssx3, strand=-1 antisense bisulphite down
- 222: name=ssx4, strand=1 sense bisulphite down
- 223: name=ssx4, strand=1 antisense bisulphite down
- 224: name=ssx5, strand=-1 sense bisulphite down
- 225: name=ssx5, strand=-1 antisense bisulphite down
- 226: name=sycp1, strand=1 sense bisulphite down
- 227: name=sycp1, strand=1 antisense bisulphite down
- 228: name=tert, strand=-1 sense bisulphite down
- 229: name=tert, strand=-1 antisense bisulphite down
- 230: name=tsp50, strand=1 sense bisulphite down
- 231: name=tsp50, strand=1 antisense bisulphite down
- 232: name=WT1, strand=1 sense bisulphite down
- 233: name=WT1, strand=1 antisense bisulphite down
- 234: name=xage1, strand=1 sense bisulphite down
- 235: name=xage1, strand=1 antisense bisulphite down

## Patentansprüche

**1.** Verfahren zur Bestimmung der Angriffspunkte einer Tumor-Immuntherapie, wobei man:
a) eine DNA-Probe entnimmt,
b) den Methylierungszustand mindestens jeweils einer CpG-Position innerhalb mehrerer Tumor-assoziierter Antigene identifiziert, wobei die Methylierungszustände zusammen ein Methylierungsmuster bilden,
c) aus dem gewonnenen Methylierungsmuster folgert, welche der Tumor-assoziierten Antigene die geeignetsten Angriffspunkte für eine Immuntherapie sind.

**2.** Verfahren nach Anspruch 1, **dadurch gekennzeichnet,**
**dass** die zu untersuchenden Tumor-assoziierten Antigene folgender Gruppe entnommen sind:
MAGE-A1 - MAGE-A6, MAGE-A8 - MAGE-A12,
MAGE-B1- MAGE-B6,
MAGE-C1 - MAGE-C3;
GAGE-1 - GAGE-8; PAGE-1 - PAGE-4; XAGE-1 - XAGE-3; LAGE-1a (1S), LAGE-1b(1L), NY-ESO-1
SSX-1 - SSX-5
BAGE, SCP-1, PRAME, SART-1, SART-3, CTp11, TSP50, CT9/BRDT
Proteinase 3; WT1, MUC-1, CEA; HER2, PSMA; Alpha-Fetoprotein; Survivin; G250; Reverse Transkriptase Telomerase (hTERT)

**3.** Verfahren nach einem der Ansprüche 1 bis 2, **dadurch gekennzeichnet, dass** man neben Tumor-assoziierten Antigenen auch MHC I oder weitere für die Vakzinierung bedeutsame, methylierungsregulierte Cofaktoren untersucht.

**4.** Verfahren nach einem der Ansprüche 1 bis 3, **dadurch gekennzeichnet, dass** man als Proben Serum oder andere Körperflüssigkeiten verwendet.

**5.** Verfahren nach einem der Ansprüche 1 bis 4, **dadurch gekennzeichnet, dass** zur Untersuchung der Methylierungszustände eine chemische Behandlung der DNA mit einem Bisulfit (=Disulfit, Hydrogensulfit) erfolgt.

**6.** Verfahren nach Anspruch 5, **dadurch gekennzeichnet, dass** nach der chemischen Behandlung eine Amplifikation der DNA erfolgt.

**7.** Verfahren nach einem der Ansprüche 1 bis 6, **dadurch gekennzeichnet, dass** man zur Amplifikation MSP oder Heavy Methyl oder eine Kombinationen dieser verwendet.

**8.** Verfahren nach einem der Ansprüche 1 bis 7, **dadurch gekennzeichnet, dass** die Identifizierung des Methylierungsstatus über mindestens ein Reporter-Oligonukleotid oder Peptid-Nukleinsäure (PNA) Oligomer erfolgt, das an ein 5'-CG-3' Dinukleotid oder ein 5'-TG-3' Dinukleotid oder ein 5'-CA-3' Dinukleotid hybridisiert und das die Amplifikation der Zielsequenz anzeigt.

**9.** Verfahren nach Anspruch 8, **dadurch gekennzeichnet, dass** das Reporter-Oligomer eine Fluoreszenzmarkierung trägt.

**10.** Verfahren nach 9, **dadurch gekennzeichnet, dass** man zur Detektion ein Taqman-Assay durchführt.

**11.** Verfahren nach Anspruch 9, **dadurch gekennzeichnet, dass** man zusätzlich zu dem Reporter-Oligonukleotid ein weiteres mit einem Fluoreszenzfarbstoff markiertes Oligomer verwendet, das unmittelbar benachbart zu dem Reporteroligonukleotid hybridisiert und diese Hybridisierung mittels Fluoreszenz Resonanz Energietransfer nachweisbar ist.

**12.** Verfahren nach Anspruch 11, **dadurch gekennzeichnet, dass** man zur Detektion ein Lightcycler Assay durchführt.

**13.** Verfahren nach Anspruch 6, **dadurch gekennzeichnet, dass** die Identifizierung durch Längenmessung der amplifizierten zu untersuchenden DNA erfolgt, wobei Methoden zur Längenmessung Gelelektrophorese, Kapillargelelektrophorese, Chromatographie (z.B. HPLC), Massenspektrometrie und andere geeignete Methoden umfassen.

**14.** Verfahren nach Anspruch 6, **dadurch gekennzeichnet, dass** man die Amplifikate selbst für die Detektion mit einer nachweisbaren Markierung versieht.

**15.** Verfahren nach den Ansprüchen 6 und 14, **dadurch gekennzeichnet, dass** die Identifizierung des Methylierungsstatus durch Hybridisierung an ein Oligomer-Array erfolgt, wobei Oligomere Nukleinsäuren oder in ihren Hybridisierungseigenschaften ähnliche Moleküle wie PNAs sind.

**16.** Verfahren nach Anspruch 15, **dadurch gekennzeichnet, dass** die Oligomere über einen 12 bis 22 Basen langen Abschnitt an die zu analysierende DNA hybridisieren und sie mindestens ein CG, TG oder CA Dinukleotid umfassen.

**17.** Verfahren nach einem der Ansprüche 6 bis 15, **dadurch gekennzeichnet, dass** die Amplifikation von mindestens zwei Fragmenten gleichzeitig in einem Reaktionsgefäß erfolgt.

**18.** Nukleinsäuremolekül, umfassend eine Sequenz mit einer Länge von mindestens 18 Basen gemäß einer der Sequenzen ausgewählt aus der Gruppe umfassend SEQ ID Nr. 48-235 oder komplementärer Sequenzen.

**19.** Oligonukleotid oder Peptid-Nukleinsäure(PNA)-Oligomer, das mindestens eine Basensequenz mit einer Länge von 10 Nukleotiden umfasst, die an eine chemisch vorbehandelte DNA, gemäß der SEQ ID Nr. 48 bis SEQ ID Nr. 235 hybridisiert oder mit ihr identisch ist.

**21.** Anordnung, insbesondere Array von mehreren Oligonukleotiden oder Peptid-Nukleinsäure (PNA)-Oligomeren, **dadurch gekennzeichnet, dass** mindestens ein Oligomer gemäß Anspruch 19 an eine feste Phase gebunden ist.

**22.** Kit, der aus einem Bisulfit enthaltenen Reagenz und mindestens einem Oligonukleotid gemäß Anspruch 18 besteht, sowie optional Blocker und Reporteroligomere, Standardreagenzien zur Durchführung von MS-SNuPE, MSP, MethyLight, Heavy-Methyl, COBRA oder Nukleinsäuresequenzierung sowie eine Anleitung zur Durchführung der erfindungsgemäßen Methode enthält.

**23.** Verfahren zur Behandlung von Tumorerkrankungen, **dadurch gekennzeichnet, dass** man gemäß den Ansprüchen 1 bis 17 die Angriffspunkte für eine Tumor-Immuntherapie bestimmt und anschließend eine Vakzinierung gegen die geeignetesten Tumor-assoziierten Tumorantigene erfolgt.

**24.** Verfahren nach Anspruch 23, **dadurch gekennzeichnet, dass** man als Impfstoffe Tumorzellen, Peptid- oder Proteinbestandteile Tumor-assoziierter Antigene oder entsprechende DNA-Sequenzen verwendet.
